# DEMANDE DE BREVET EUROPEEN

(11) **EP 4 286 038 A2**
(43) Date de publication de la demande: **06.12.2023**
(21) Numéro de dépôt: 23201373.0
(22) Date de dépôt: 02.06.2017
(51) Int. Cl.: B01F 33/84

(54) **SYSTEME DE DISTRIBUTION D'UN PRODUIT COSMETIQUE**

(30) Priorité: 02.06.2016 FR 1655053
(62) Demande divisionnaire de: 17732768.1
(71) Demandeur: L'OREAL, 75008 Paris (FR)
(72) Inventeur: GIRON, Franck, LAGNY SUR MARNE (FR); SAMAIN, Henri, BIEVRES (FR); KERGOSIEN, Guillaume, CHAVILLE (FR)
(74) Mandataire: Cabinet Nony

(57) **Abrégé**

La présente invention concerne un système (10) de distribution d'un produit, comportant un distributeur recevant au moins deux cartouches ayant chacune un réservoir contenant un produit de base, un premier produit de base comportant au moins 0,1 % en masse de particules présentant un écart de densité, de préférence d'au moins 0,5 g/cm³ , avec le milieu qui les contient, notamment des particules présentant une densité supérieure ou égale à 2g/cm³, et un épaississant, le distributeur permettant de délivrer au moins deux produits de base dans des proportions réglables, la viscosité du premier produit de base étant de préférence supérieure à 2 Pa.s et de préférence encore supérieure ou égale à 4 Pa.s.

## Description

La présente invention concerne les procédés et systèmes de distribution d'un produit cosmétique, notamment d'un produit de maquillage, de coloration, de protection solaire, de soin ou d'un parfum.

### A) Système de mélangeur pouvant fonctionner avec des compositions comportant avec des particules denses

De nombreuses personnes souhaitent se maquiller pour embellir leur apparence, notamment leur visage.

Les motivations de ces personnes peuvent se ranger en deux catégories :
- Masquer certaines imperfections, comme des taches, des rides ou des pores,
- Embellir le rendu du visage par des changements de couleur.

Dans ces différents cas, l'opération consiste à apporter une matière colorée et à en couvrir la peau ou une zone de peau.

Pour obtenir un effet esthétique, la personne doit réussir le choix de la matière colorée.

Dans le premier cas ci-dessus, l'opération peut être complexe car le visage comporte toute une gamme de couleurs.

Ainsi, si la personne ne veut couvrir que quelques zones du visage, en essayant de faire coïncider la couleur ajoutée à la couleur naturelle de la peau environnante, il lui est nécessaire de chercher la couleur qui convient à chaque zone du visage, ce qui est d'autant plus difficile que la couvrance du produit et l'épaisseur déposée, ainsi que la couleur et l'état de surface de la peau sous-jacente ou son caractère plus ou moins gras, peuvent influencer le résultat.

Compte-tenu de ces difficultés, les personnes qui veulent masquer les imperfections de leur visage, ont l'habitude de couvrir l'ensemble du visage. On contourne alors le problème du choix de la matière colorée en fonction de la zone du visage.

Toutefois, le résultat s'éloigne de l'aspect naturel du visage de par l'homogénéité apportée.

Dans le deuxième cas, l'opération n'est pas simple non plus car il est difficile de trouver la matière colorée qui convient le mieux à l'aspect du visage. En particulier, il est difficile de trouver la couleur de son teint, surtout si l'on veut choisir une couleur marquée, différente de sa couleur naturelle. Certaines personnes aimeraient choisir des couleurs bronzées ou autres nuances de teint différentes, mais elles ne le font pas de peur que la couleur ne leur corresponde pas bien. Et si elles le font, elles abandonnent souvent déçues. Quand elles ne sont pas déçues du résultat, elles n'osent plus changer de couleur.

Il en est de même pour le maquillage des lèvres et celui des joues et des paupières.

Pour résoudre ces problèmes, il n'existe que peu de solutions.

Une première approche consiste à acheter de nombreux produits et à tous les essayer. Cette approche est coûteuse et génère souvent des pertes dans la mesure où l'on ne retient généralement qu'un petit nombre des matières colorées essayées.

Une seconde approche consiste à tester, en magasin, différents produits. Elle ne convient pas toujours, car il est très difficile de se figurer le résultat en quelques instants et dans un lieu dépourvu des repères habituels. En particulier, pour apprécier pleinement, l'effet d'un maquillage dans un magasin, il faudrait pouvoir l'éclairer de la même manière que dans les conditions futures d'utilisation, ce qui est rarement possible. En général, ce n'est qu'en testant un maquillage pendant la journée, qu'on se rend compte s'il convient ou non. De plus, si certains magasins disposent de conseillers et permettent d'effectuer des essais, ce n'est pas le cas d un grand nombre d'autres points de vente et des achats effectués sur internet.

Une autre approche a été testée sans toutefois se développer. Elle consisteà réaliser manuellement ses produits par mélange de plusieurs produits de couleur. Cela peut s'avérer relativement difficile à mettre en oeuvre, car il est peu aisé de reproduire exactement plusieurs fois le même mélange, et l'on a du mal à réaliser rapidement les mélanges dont on a besoin au moment du maquillage.

On connaît aussi des distributeurs permettant de délivrer une composition cosmétique de couleur variable.

La demande US2003069667 concerne des procédés et des appareils permettant de personnaliser les produits cosmétiques utilisés par un consommateur. Ce dernier fournit des critères de choix et une formule de produit cosmétique en est dérivée. Les ingrédients de base sont mélangés conformément à la formule et un produit cosmétique personnalisé est distribué sur une surface intermédiaire, pour une application ultérieure.

Le brevet US5785960 divulgue un procédé d'obtention de fonds de teint adaptés à recouvrir les imperfections de la peau humaine. Les étapes du procédé incluent la mesure par spectrophotomètre d'une peau normale d'un client pour obtenir des valeurs de brillance, de rouge et de jaune de coloration de la peau, respectivement dénotées en tant qu'unités L, a et b. Ensuite ces valeurs sont converties par calcul en des valeurs modifiées déterminées par un programme de correction des valeurs L, a et b. En fonction de ces valeurs modifiées, un fond de teint est formulé. Une machine de formulation à distance convertit les instructions reçues et dose et mélange une série de produits de base. Le mélange délivré par la machine est emballé et expédié au client.

La demande FR2970403 divulgue un dispositif de distribution d'un produit cosmétique, notamment d'un parfum, comportant au moins un réservoir contenant un produit à distribuer, notamment plusieurs réservoirs, et un dispositif de rinçage. Le dispositif peut être piloté à l'aide d'un micro-ordinateur ou similaire. Une interface homme machine, par exemple un clavier ou un écran, notamment tactile, permet à l'utilisateur de commander la distribution d'une formule de son choix. Le dispositif peut être agencé pour communiquer avec un serveur ou d'autres dispositifs similaires pour échanger des recettes ou permettre à l'utilisateur de se faire conseiller. Une mémoire d'un circuit électronique du dispositif peut enregistrer les meilleures formules, afin de les reproduire à la demande et de les échanger. Le dispositif peut aussi être utilisé pour réaliser des mélanges de produits cosmétiques colorés. Une certaine quantité, par exemple une goutte, de composition colorée est alors produite par le dispositif et sert à se maquiller ou est à mélanger à une crème de teint ou toute autre base colorée ou non. Le dispositif permet de générer facilement la couleur voulue par l'utilisateur, lequel peut par exemple réaliser en quelques instants plusieurs mélanges de couleurs différentes.

La demande de brevet FR2818101 concerne un dispositif pour la pulvérisation d'un produit cosmétique, notamment d'un fond de teint. Il est possible de réaliser un mélange extemporané sur le substrat traité.

La demande FR 2877819 décrit un distributeur permettant de faire varier la proportion relative de différents produits de base qui sont distribués. Il est ainsi possible de régler la couleur. Les produits de base sont issus de différents réservoirs et distribués par des canaux distincts qui débouchent côte à côte à une extrémité du distributeur. Un inconvénient qui en résulte est que l'utilisateur doit effectuer le mélange sur la peau ou sur un support intermédiaire. De plus, si la quantité distribuée est excessive, celle-ci est perdue.

US 5 622 692 et US5903465 décrivent d'autres exemples de distributeurs permettant de distribuer une composition cosmétique personnalisée.

Parmi les essais d'automatisation de la fabrication d'une composition cosmétique personnalisée qui ont pu être tentés, nombreux sont ceux dans lesquels les solutions proposées permettent de réaliser des mélanges dans des quantités d'environ 100 g ou parfois moins, mais pas dans les très petites proportions dont la personne qui se maquille a généralement besoin, c'est-à-dire de l'ordre du gramme ou beaucoup moins.

Pour illustrer cette problématique, on peut imaginer le cas d'une personne qui veut cacher deux imperfections de l'ordre du cm² sur son visage. Pour la première zone, elle a besoin de trouver le mélange correspondant, puis d'en délivrer une très petite quantité, par exemple d'environ 10 mg. Pour la seconde, elle a besoin de changer le réglage du distributeur, puis de délivrer une toute petite quantité également.

Par conséquent, le choix des matières colorées conférant les meilleurs résultats reste difficile pour un grand nombre de personnes.

Il existe par conséquent un besoin pour faciliter la recherche d'un produit de maquillage répondant aux attentes d'un consommateur, et permettant à celui-ci de réaliser des mélanges dans des conditions fiables et en très petite quantité.

L'invention vise ainsi, selon certains de ses aspects, à faciliter le maquillage du visage et notamment la recherche des produits les plus adaptés aux différentes zones de celuici.

Il existe encore un besoin pour perfectionner les systèmes de distribution permettant de délivrer des produits de couleur variable, afin notamment de faciliter leur utilisation et améliorer la qualité du maquillage.

L'invention repose selon certains de ses aspects sur un système de distribution qui permet de générer des mélanges à partir de produits de base. Ces produits de base peuvent avoir des couleurs différentes, de telle sorte que l'on puisse faire varier la couleur du mélange. Les produits de base peuvent encore permettre de faire varier la couvrance du mélange, de telle sorte que la couleur qui résulte de l'application du mélange sur les matières kératiniques humaines varie, en étant plus ou moins proche de celle desdites matières. Ainsi, la notion de couleur doit être comprise avec une acceptation large et englobe des mélanges dont la couleur varie après application du fait de leur couvrance plus ou moins élevée et de la couleur de la peau sous-jacente.

La précision de la couleur obtenue n'a de valeur que si la couleur reste constante au fil de l'utilisation du système. Ceci est d'autant plus essentiel lorsque le fabricant fait reposer l'attrait du système sur les résultats de maquillage que permet son système. Ainsi, dans le premier cas de figure, il faut que si on a identifié une couleur particulière pour une zone du visage, le système soit capable de restituer cette couleur identifiée chaque fois qu'on l'utilise. Il en est de même dans le second cas de figure où le traitement à des fins d'ajustement doit être très fidèle à la couleur qui a été choisie.

Les effets de masquage s'appuient sur l'utilisation de particules (pigments, charge). On sait que les particules peuvent décanter naturellement. Ce faisant, ce phénomène peut induire une différence en concentration en particules tout au long de l'utilisation du système, ce qui peut produire de nettes variations d'effet de maquillage. Pour comprendre le phénomène de variation il faut se rappeler que, d'habitude (hors invention), une variation de concentration en pigment cause surtout des variations de couvrance, mais peu de couleur. De plus, si un phénomène de décantation apparait, l'utilisateur identifie le problème en voyant :
1) Que le système délivre un produit sans particules, caractéristique d'un déphasage, voire aqueux ou huileux,
2) ou que le système délivre un produit très chargé en particules, voire épais et difficile à étaler.

Dans un système de distribution à réservoirs multiples où l'on délivre des compositions de couleurs différentes pour créer un mélange, une variation de concentration en particules dans un ou plusieurs des réservoirs risque de ne pas se voir car les autres produits délivrés par les autres réservoirs diluent le mélange. Il s'ensuit que l'utilisateur ne peut pas identifier le problème par l'aspect du mélange et ce faisant applique un mélange de couleur différente de celle attendue.

On pourrait imaginer que les phénomènes de décantation étant équivalents dans chacun des réservoirs, ils vont se compenser et limiter les variations de couleur. Toutefois, l'utilisateur peut très bien utiliser son système de telle façon que les réservoirs se vident à différentes vitesses. Ainsi, comme les compartiments sont utilisés à des rythmes différents, les phénomènes de décantation risquent de s'appliquer très différemment d'un compartiment à l'autre. Ceci est d'autant plus vrai qu'un réservoir peut rester dans le système pendant de très longues durées si peu utilisé.

Un problème existe quand on veut utiliser des produits contenant des particules de densité importantes (allant de 2 g/cm³ à parfois plus de 8 g/cm³), tels que choisis parmi

| | |
|---|---|
| Oxychlorure de bismuth : | 7,7 g/cm³ |
| Oxyde de cérium : | 7,6g/cm³ |
| Oxyde de chrome : | 5,7 g/cm³ |
| Oxyde de zirconium : | 5,6 g/cm³ |
| Oxyde de fer : | 5,2g/cm³ |
| Oxyde de titane : | 4,3 g/cm³ |
| Talc (Silicate de magnésium hydroxylé) : | 2,7 g/cm³ |
| Carbonate de calcium : | 2,7 g/cm³ |
| Silice : | 2,6 g/cm³ |
| Nitrure de bore : | 2,1 g/cm³ |

voire même au delà de 8g/cm³ comme le carbure de tungstène (15 g/cm3).

Une possibilité serait d'utiliser des systèmes d'agitation, mais ils sont compliqués en particulier si on veut réaliser de petites doses. En particulier, les processus d'agitation nécessitent un temps minimal, difficilement limitable à moins de quelques secondes. Or, pour que le système fonctionne, en particulier quand on réalise des petites doses, le temps de réaction doit être très court, moins d'une seconde typiquement, afin de ne pas lasser l'utilisateur par une attente.

De plus, les systèmes d'agitation opèrent surtout sur la masse en contact direct avec eux. Donc si on met une pale d'agitation, par exemple, c'est surtout la partie en contact avec la pale qui sera agitée. Le produit se situant dans d'autres parties telles que des tubulures ne seront pas agitées. Or, il faut que ces dernières soient homogènes en densité de particules.

Les systèmes d'agitation présentent aussi d'autres limites comme la consommation d'énergie, le bruit et aussi :
- La difficulté à homogénéiser certaines compositions (rhéoépaississantes)
- La difficulté de placer un système d'homogénéisation dans un réservoir si celui-ci est à volume variable et destiné à changer de volume (cas des compartiments poussés par un piston)
- Les risques de formation d'aggrégats, en particulier lorsque les produits contiennent des fibres par exemple

Enfin, le fabricant peut vouloir intentionnellement imposer un gradient de particules ou autre ingrédient non particulaire dans un ou plusieurs réservoirs, par exemple, pour créer une variation d'un effet dans le temps tel qu'un effet antiacné diminuant application après application. Dans ce cas, il remplira le réservoir avec un gradient qu'il voudra conserver au fil de l'utilisation. L'homogénéisation empêcherait alors cette application.

L'invention vise selon un premier aspect à permettre de traiter une ou plusieurs zones du visage et d'obtenir des mélanges très précis sur le plan de la fidélité des couleurs. Par la suite, on désigne par « zone » une partie du visage définie et assez réduite en surface, d'étendue comprise entre 1 cm² et 100 cm², mieux allant de 2 cm² à 50 cm².

Elle y parvient grâce à un système de distribution d'un produit, comportant un distributeur recevant au moins deux cartouches ayant chacune un réservoir (encore appelé compartiment) contenant un produit de base, un premier produit de base comportant au moins 0,1 % en masse de particules présentant un écart de densité d'au moins 0,5 g/cm³ avec le milieu qui les contient, des particules présentant une densité supérieure ou égale à 2g/cm³, et un épaississant, le distributeur permettant de délivrer au moins deux produits de base dans des proportions réglables.

Le premier produit de base comporte au moins 0,1 % en masse, par rapport à la masse totale du premier produit de base, de particules présentant un écart de densité d'au moins 0,5 g/cm³ avec le milieu qui les contient.

De préférence, le premier produit de base est contenu dans une des au moins deux cartouches et les au moins deux produits de base délivrés par le distributeur dans des proportions réglables sont le premier produit de base et un deuxième produit de base, contenu dans l'autre des au moins deux cartouches.

L'invention peut présenter une ou plusieurs des caractéristiques suivantes, considérées isolément ou en combinaison :
- le premier produit de base comporte au moins 1% en masse, par rapport à la masse totale du premier produit de base, mieux au moins 2% en masse, par rapport à la masse totale du premier produit de base, encore mieux entre 3% et 10% en masse, par rapport à la masse totale du premier produit de base, encore plus préférentiellement entre 4% et 7% en masse, par rapport à la masse totale du premier produit de base, de particules présentant un écart de densité d'au moins 0,5 g/cm³ , mieux d'au moins 1 g/cm³, encore mieux compris entre 2 g/cm³ et 10 g/cm³, encore plus préférentiellement entre 4 g/cm³ et 8 g/cm³ avec le milieu qui les contient.
- la viscosité du premier produit de base est supérieure ou égale à 2 Pa.s et préférentiellement supérieure ou égale à 4 Pa.s, plus préférentiellement comprise entre 4 Pa.s et 10 Pa.s, encore plus préférentiellement comprise entre 5 Pa.s et 8 Pa.s, la viscosité étant mesurée à 1 atm et à 25°C avec un viscosimètre rotatif CONTRAVES TV équipé d'un mobile MS-r3 ou MS-r4 à la fréquence de 60 Hz, après 10 minutes de rotation du mobile.

- le deuxième produit de base présente une viscosité inférieure à celle du premier produit de base.
- le deuxième produit de base a une viscosité inférieure ou égale à 2 Pa.S, préférentiellement inférieure ou égale à 1 Pa.s, plus préférentiellement inférieure ou égale à 0,8 Pa.s, encore plus préférentiellement comprise entre 0,6 Pa.s et 0,2 Pa.s.
- la densité desdites particules est supérieure ou égale à 5g/cm³, mieux supérieure ou égale à 6 g/cm³, encore mieux comprise entre 6 g/cm³ et 10 g/cm³, préférentiellement entre 7 g/cm³ et 8 g/cm³.
- les particules comportent l'un au moins des matériaux choisis dans la liste suivante : oxychlorure de bismuth, oxyde de cérium, oxyde de chrome, oxyde de zirconium, oxyde de fer, oxyde de titane, talc, carbonate de calcium, silice, nitrure de bore, carbure de tungstène, étant de préférence choisi parmi l'oxychlorure de bismuth et l'oxyde de cérium.
- le deuxième produit est dépourvu de particules de densité supérieure ou égale à 7 g/cm³, mieux de densité supérieure ou égale à 6 g/cm³, encore mieux de densité supérieure ou égale à 5g/cm³, préférentiellement de densité supérieure ou égale à 2g/cm³.
- l'épaississant du premier produit de base est choisi parmi les composés saccharidiques de type gomme, tels que la gomme arabique, la gomme acacia, la gomme guar, la gomme gellane, la gomme keraya, la gomme de carraghénane, les composés cellulosiques tels que CMC, HMC, HPMC ,les polymères synthétiques tels que les polyacides acryliques ou méthacrylique tels que les carbomer (Carbopol), ou polyuréthanes, polyvinylacétate, polyvinylalcool, les émulsions épaisses inverses ou directes, des associations de solvants non aqueux avec des agents épaississants pour huile, des argiles telles que bentonite, attapulgite, des organochélateurs, des protéines telles que caséine ou collagène, des agents de rhéologie rhéofluidifiants ou thixotropes, étant de préférence choisi parmi les composés saccharidiques de type gomme, tels que la gomme arabique, la gomme acacia, la gomme guar, la gomme gellane, la gomme keraya, la gomme de carraghénane.
- l'épaississant du premier produit de base est choisi parmi les composés saccharidiques de type gomme et sa teneur massique dans le premier produit de base est comprise entre 0,1 % et 5 % par rapport à la masse totale du premier produit de base, mieux entre 0,8 à 2,5 % par rapport à la masse totale du premier produit de base, encore mieux entre 1,5 % et 2,3 % par rapport à la masse totale du premier produit de base.
- le système comporte une troisième cartouche avec un troisième produit de base.
- le troisième produit de base comporte un épaississant identique ou différent de celui du premier produit de base, préférentiellement identique de celui du premier produit de base.
- l'épaississant du troisième produit de base différent de l'épaississant du premier produit de base est choisi dans la même liste que l'épaississant du premier produit de base, étant de préférence choisi parmi les composés saccharidiques de type gomme, tels que la gomme arabique, la gomme acacia, la gomme guar, la gomme gellane, la gomme keraya, la gomme de carraghénane.
- les cartouches sont reçues de façon amovible dans le distributeur.
- chaque produit quitte la cartouche par un canal de sortie de la cartouche, le canal de sortie étant défini par un embout de distribution de la cartouche entraîné en rotation relative par rapport à un corps de la cartouche par un mécanisme d'entraînement du distributeur pour distribuer le produit de base contenu dans la cartouche.
- le système comporte un mécanisme d'homogénéisation du premier produit de base, notamment vibratoire ; ce mécanisme peut être intégré à la cartouche, le cas échéant.

### Système de distribution

Le système de distribution peut être constitué par un seul appareil fonctionnant de manière autonome, de préférence manipulable d'une main, ou par un appareil qui fonctionne en interagissant avec d'autres pièces ou appareils. Il peut s'agir par exemple de différentes interfaces de sorties qui sont montées sur le distributeur en fonction du type de maquillage à réaliser, comme cela sera précisé plus loin. Il peut s'agir également d'un système informatique qui échange des informations avec le distributeur pour le piloter, ce système informatique comportant par exemple un terminal portable tel qu'un téléphone intelligent, un photophone, une tablette, un ordinateur personnel, ou un terminal dédié.

De préférence, le distributeur est agencé pour mettre sous pression un ou plusieurs compartiments contenant le ou les produits de base, par le biais de dispositifs de dosage volumétrique, de préférence un moteur entraînant en déplacement un piston dans le compartiment correspondant.

Le distributeur peut être formé d'un boîtier et d'au moins deux ou trois compartiments, et de préférence d'autant de moteurs. Par exemple, la rotation des moteurs entraîne des vis sans fin, lesquelles poussent les pistons de chaque compartiment. L'avance des pistons est par exemple contrôlée par le nombre d'impulsions de commande envoyées aux moteurs et/ou par la durée de fonctionnement de ces derniers. Les moteurs peuvent être alimentés séquentiellement, ou de préférence simultanément.

Par exemple, les moteurs sont alimentés durant un cycle élémentaire de fonctionnement pendant une courte durée les uns après les autres, ou les uns en même temps que les autres, de façon à distribuer des microdoses correspondantes.

Les cycles élémentaires sont répétés, avec éventuellement un temps d'arrêt entre eux, qui laisse le temps aux produits de base de s'écouler hors des compartiments.

Les compartiments peuvent être définis par des cartouches qui se retirent lorsqu'elles sont vides. En variante, les compartiments sont présents à demeure et remplis à nouveau une fois vide.

Chaque cartouche peut être fermée par un bouchon démontable pour permettre le nettoyage de la cartouche.

De préférence, le boîtier du distributeur est de forme allongée selon un axe longitudinal, ce qui peut faciliter sa manipulation, et les cartouches sont disposées autour de cet axe, dans le boîtier.

De préférence, les cartouches sont mises en place par l'arrière, et le mélange est délivré par l'avant. La mise en place des cartouches peut s'effectuer individuellement, ou en variante, les cartouches constituent un ensemble monobloc lors de leur mise en place.

Les cartouches peuvent comporter chacune un mécanisme de dosage volumétrique, comportant un piston mu par un mécanisme d'entraînement du distributeur, dans une direction s'accompagnant d'une diminution du volume intérieur contenant le produit de base et d'une expulsion du produit. Il peut être intéressant que les cartouches aient une région de leur paroi au moins qui soit transparente, afin de permettre de voir la couleur du produit contenu à l'intérieur.

Le mécanisme d'entraînement peut comporter un système de motorisation formé de moteurs couplés à des réducteurs, de forme allongée parallèlement à l'axe longitudinal du distributeur, et disposés entre les cartouches. Ce positionnement des moteurs et cartouches rend le distributeur particulièrement compact.

Le produit de base peut sortir de façon étanche de la cartouche correspondante puis circuler dans un canal prévu à cet effet dans le boîtier du distributeur, avant de sortir de celui-ci.

Les cartouches se terminent avantageusement par un embout réalisé de telle sorte que, une fois la cartouche insérée dans le boîtier du distributeur, l'extrémité de l'embout débouche au ras du boîtier. En variante, l'embout est suffisamment long pour dépasser du boîtier et connecter ainsi différentes interfaces de sortie susceptibles d'être rapportées sur le boîtier du distributeur.

Grâce au mécanisme d'entraînement comportant des moteurs pour faire avancer des pistons, il est possible de délivrer de façon précise des mélanges en de très petites quantités. Ainsi, le mécanisme d'entraînement peut délivrer les produits de base avec à un débit minimal inférieur ou égal à 50 µL/s, mieux inférieur ou égal à 20 µL/s, encore mieux inférieur ou égal 10 µL/s. De préférence, le mécanisme d'entraînement délivre des débits compris entre 20 et 100 µL/s, mieux compris entre 40 µL/s et 60 µL/s. Il est donc possible de réaliser facilement un mélange d'environ 10 mg. Un tel système de distribution est alors idéal pour réaliser de petites touches de maquillage, pour couvrir par exemple une zone de 1 cm², mieux une zone de 0, 5 cm².

Il est aussi possible de réaliser des plus grandes quantités de mélange comme celles nécessaires au maquillage d'une joue, ou d'un visage. Ces quantités restent néanmoins relativement faibles, par exemple une quantité comprise entre 100 et 500 mg, mieux comprise entre 150 et 250 mg.

Le système de distribution peut ainsi comporter un distributeur présentant un boîtier, et au moins une cartouche reçue dans le boîtier du distributeur, cette cartouche comportant un corps et un piston mobile dans le corps, le boîtier comportant un mécanisme d'entraînement motorisé pour déplacer le piston de la cartouche.

De préférence, la cartouche comporte un embout de distribution par lequel s'effectue la sortie du produit, et cet embout de distribution est entraîné en rotation par le mécanisme d'entraînement pour déplacer le piston. L'embout peut comporter au moins un relief d'anti-rotation, mieux deux ergots d'anti-rotation diamétralement opposés.

L'embout peut porter un joint d'étanchéité, notamment un joint torique. Ainsi, au changement de cartouche, le joint est également changé, ce qui permet de s'affranchir de l'usure du joint.

Le distributeur peut comporter une carte électronique de pilotage du mécanisme d'entraînement motorisé, cette carte électronique étant traversée par le ou les embouts. Cela peut permettre de réaliser une carte s'étendant sensiblement sur toute la section du distributeur, donc de regrouper sur une seule carte tous les composants électroniques du distributeur, et ainsi de gagner en compacité et fiabilité. La carte peut s'étendre sensiblement perpendiculairement à l'axe longitudinal du boîtier. La carte peut porter un interrupteur de commande du fonctionnement du distributeur.

Le système de distribution peut être agencé pour fonctionner selon au moins deux modes de distribution.

Dans un premier mode, dit « continu », le mélange est distribué tant qu'une pression est exercée sur l'interrupteur de commande.

Dans un deuxième mode, dit « dose », une quantité prédéfinie du mélange est distribuée à chaque pression sur l'interrupteur.

Le ou les embouts peuvent déboucher à une extrémité du boîtier. Cela peut permettre de réduire le volume mort, comme cela sera détaillé dans la suite.

Le ou les embouts peuvent présenter à leur extrémité un système d'obturation pour éviter le séchage des produits dans le conduit, par exemple une membrane cicatrisante.

La cartouche peut comporter une vis creuse sur laquelle le piston est vissé, le piston pouvant se déplacer axialement sur la vis lorsque celle-ci tourne ; le piston est empêché de tourner dans le corps de la cartouche. Par exemple, la friction du piston sur le corps de la cartouche peut suffire à l'empêcher de tourner quand la vis tourne.

Préférentiellement, la rotation est rendue impossible avec un corps de cartouche de section non circulaire et un piston non déformable.

Le couple des moteurs peut être déterminé électroniquement en fonction du courant absorbé, et servir par exemple à détecter une fin de course du piston. Une information concernant le couple peut être transmise à distance à un système informatique présentant une interface homme machine, pour permettre de surveiller le bon fonctionnement du distributeur.

Afin d'ajuster la teinte, le système de distribution selon l'invention doit permettre à l'utilisateur de faire varier le volume délivré provenant de chaque compartiment.

De façon préférée, le distributeur est piloté par un système informatique, qui est intégré au distributeur, ou externe à celui-ci, le distributeur étant alors apte à échanger avec le système informatique des informations par protocole sans fil ou câblé.

Le distributeur peut ainsi être piloté pour permettre l'ajustement de la teinte par distribution simultanée ou séquentielle, et contrôlée, de plusieurs produits de base de couleurs différentes.

La distribution des produits de base peut être continue ; si dans ce cas, les volumes de chacun des produits de base sont distribués d'une seule traite, simultanément ou successivement.

Dans le cas d'une distribution simultanée, il est utile de pouvoir ajuster les débits respectifs des différents produits de base pour que le mélange distribué corresponde à celui souhaité à chaque instant. Un tel mode de distribution peut convenir en particulier lors de la distribution du mélange par pulvérisation, à l'aide d'un aérographe. Pour ajuster les débits il est possible par exemple de jouer sur la vitesse de déplacement des pistons, par exemple, dans le cas d'un entrainement des pistons par une vis sans fin en faisant varier la vitesse de rotation des moteurs qui entrainent la vis. Les produits peuvent aussi être distribués de façon impulsionnelle avec un temps de distribution et un temps d'arrêt à chaque cycle. En jouant sur le rapport cyclique, il est possible d'agir sur le débit.

Tous les produits de base peuvent être délivrés simultanément lors du temps de distribution ; en variante, les cycles des différents produits sont déphasés, de sorte qu'un produit est distribué pendant un temps d'arrêt des autres produits.

Dans un mode particulier de l'invention, le mélange est délivré dans une cavité d'un contenant, pouvant fermer de façon hermétique ou non, par exemple en forme de coupelle, dans lequel on peut faire glisser un applicateur, en particulier un stylet ou une brosse. Un tel système de distribution est spécialement adapté aux liners, gloss et autres formules appliquées sans contact direct avec les mains. Ce contenant peut être amovible.

Par exemple, il sert de distributeur de rouge à lèvres et dispose d'un système de distribution, à vis par exemple. Lorsqu'il n'est pas amovible, le contenant peut être réalisé avec le corps du distributeur. Lorsqu'il est amovible, il peut constituer une interface de sortie parmi d'autres pouvant être montées sur le distributeur.

Les compartiments, et en particulier les cartouches, peuvent contenir tout ou partie du mécanisme d'entraînement, et par exemple le système de motorisation, ou mieux, une partie du système de motorisation, et ce, afin de réduire le nombre de pièces en mouvement dans le corps du distributeur à l'extérieur des cartouches. Par exemple, les cartouches comportent le rotor du moteur. Une fois les cartouches installées dans le corps du distributeur, les rotors sont mis en interaction avec les stators.

Le système de distribution est avantageusement agencé pour permettre de lancer des séquences préprogrammées dans lesquelles le mélange délivré par celui-ci est modifié de façon continue ou discontinue. Un mode « dégradé » permet par exemple passer progressivement d'un mélange A à un mélange B. Dans le cas d'une application par pulvérisation, notamment à l'aide d'un aérographe, cela permet de réaliser simplement les dégradés. Un mode « Alternatif » permet par exemple de passer rapidement d'un mélange A à un mélange B plusieurs fois de suite. Dans le cas d'une application par pulvérisation, on peut ainsi réaliser un dépôt multicouche, avec des formulations différentes pour deux couches adjacentes superposées. Un autre mode permet par exemple de proposer plusieurs mélanges successifs préprogrammés, le système informatique indiquant à chaque fois l'usage qu'il faut en faire à l'utilisateur, par exemple par affichage sur un écran.

Dans le cas d'une application manuelle, les mélanges sont distribués par exemple dans une coupelle. La personne se maquille à l'endroit recommandé avec un mélange correspondant prélevé dans la coupelle, puis nettoie éventuellement la coupelle et commande la délivrance d'un nouveau mélange ; l'opération est répétée autant de fois que nécessaire pour se maquiller complètement.

L'homogénéisation des mélanges distribués peut se faire de différentes façons selon les cas d'utilisation. Dans le cas d'une application manuelle, elle peut se faire directement sur la zone d'application au moment de l'application ou dans la coupelle avant prélèvement; dans le cas d'une application à l'aérographe, c'est la tuyère de l'aérographe qui sert de chambre de mélange ; dans le cas où le mélange est distribué dans un contenant en vue d'une utilisation ultérieure, l'homogénéisation peut être faite à la main ou en faisant passer les produits distribués dans une chambre de mélange, située entre le distributeur et le contenant ou directement intégrée au contenant, comme détaillé par la suite.

Le produit peut être délivré par le système de distribution et utilisé extemporanément. En variante, le produit délivré par le système de distribution est conditionné et utilisé ultérieurement, par exemple à plusieurs reprises, avec par exemple un jour d'intervalle au moins entre deux utilisations.

### Particules

La densité du milieu qui contient les particules peut être comprise entre 0,7 g/cm³ et 1,3 g/cm³, mieux entre 0,8 g/cm³ et 1,2 g/cm³, encore mieux entre 0,9 g/cm³ et 1,1 g/cm³.

Un ou plusieurs compartiments comportent des particules de densité supérieure ou égale à 2g/cm³, mieux supérieure ou égale à 5 g/cm³, mieux supérieure ou égale à 6 g/cm³, encore mieux comprise entre 6 g/cm³ et 10 g/cm³, préférentiellement entre 7 g/cm³ et 8 g/cm³ à un taux d'au moins 0,1 % en masse par rapport à la masse du produit de base contenant lesdites particules, mieux d'au moins 1 % en masse par rapport à la masse du produit de base contenant lesdites particules, encore mieux d'au moins 2 % en masse par rapport à la masse du produit de base contenant lesdites particules, préférentiellement à un taux compris entre 2 % et 10 % en masse par rapport à la masse du produit de base contenant lesdites particules, encore préférentiellement à un taux compris entre 4% et 7 % en masse par rapport à la masse du produit de base contenant lesdites particules.

Les particules peuvent contenir des atomes métalliques, tels que des atomes de titane, fer, chrome, cobalt, plomb, mercure, cérium, bismuth, zincou cuivre. Elles peuvent être de toutes tailles et formes.

L'invention concerne aussi les produits contenant des particules plus légères que le milieu dans lequel elles sont présentes et risquant donc de s'accumuler vers le haut. En particulier, les particules de l'invention peuvent être des particules minérales ou organiques contenant de l'air ou un gaz comme les aérogels de silice ou les particules de polymère expansé, tels que le polystyrène expansé par les alcanes légers. La densité de telles particules peut être inférieure ou égale à 0,5 g/cm³, mieux inférieure ou égale à 0,2 g/cm³, encore mieux comprise entre 0,05 et 0,2g/cm³.

Les particules peuvent être destinées à apporter de la couleur ou de la couvrance ou d'autres effets optiques (réflexion, matité, photoprotection) (objet principal de l'invention) ou d'autres avantages tels qu'un effet de douceur, absorption du sébum ou humidité ou pollution.

### Viscosité et agents de viscosité

La viscosité des produits est mesurée à 1 atm et à 25°C avec un viscosimètre rotatif CONTRAVES TV équipé d'un mobile MS-r3 ou MS-r4 à la fréquence de 60 Hz, après 10 minutes de rotation du mobile
Les rhéologies sont newtoniennes ou non. En particulier, on préfère les épaississants conférant une rhéologie rhéofluidifiante, c'est-à-dire qui voit sa viscosité ne pas être proportionnelle à la contrainte appliquée, avec ou sans caractère thixotrope, c'est-à-dire qui voit sa viscosité diminuer dans le temps. Elles sont typiquement produites par des composés minéraux tels que la bentonite ou des composés organiques comme l'acide hyaluronique.

Les épaississants de l'invention peuvent être des composés gélifiants, comme des ingrédients naturels tels que saccharidiques comme les gommes arabique ou gomme acacia, gomme guar, gomme gellane, karaya etc... des polymères synthétiques tels que les polyacides acryliques ou méthacryliques tels que les carbomer (carbopol), des émulsions épaisses inverses ou directes, ou des associations de solvants non aqueux avec des agents épaississants pour huile.

Dans le cas des composés saccharidiques de type « gomme », sa teneur massique dans le produit de base est comprise entre 0,1 % et 5 % par rapport à la masse totale du produit de base, mieux entre 0,8 à 2,5 % par rapport à la masse totale du produit de base, encore mieux entre 1% et 2% par rapport à la masse totale du produit de base.

Les gélifiants pouvant être utilisés peuvent être naturels ou artificiels tels que des amidons (E441), pectines (E440), agar (E406), acide alginique (E400), Alginate de sodium (E401) ou de potassium (E402) ou d'ammonium (E403) ou de calcium (E404), Carrageenan (E407) ou des ingrédients issus du monde animal (Gélatine comme le E441).

Pour les formulations riches en solvant, on peut utiliser un organogélateur. Ce sont typiquement des liquides organiques, une huile minérale ou une huile végétale, piégé dans un réseau tridimensionnel issu de l'autoassemblage supramoléculaire de petites molécules organiques (appelés organogélateurs) formant des structures microscopiques ou nanoscopiques. Ils sont utilisés de 1 à 10% et peuvent être, par exemple des dérivés de 4-tertbutyl-1-aryl cyclohexanols, dérivés polymériques tels que polyéthylène glycols, polyesters, polyalkylènes, dérivés du N-lauroyl-L-lysine éthyl ester, des dérivés de peptides, des petits acides gras.

Dans le cas où on utilise un épaississement de rhéologie rhéo fluidifiant, on peut utiliser des produits dont la viscosité dépasse 50 Pa.S, mieux dépasse 100 Pa.s, encore mieux est comprise entre 10 et 100 Pa.S. On définit cette rhéologie rhéo fluidifiant, tel que la viscosité chute d'au moins un facteur 2 lorsque la contrainte est augmentée d'un facteur 10. (passage de 1 à 10 s⁻¹)
Pour mesurer cette viscosité, on réalise un test tel que décrit dans la thèse de Clément Saidou HAL Id : tel-00870761 : https://tel.archives-ouvertes.fr/tel-00870761/document
Plus précisément, on peut utiliser un rhéomètre à contrainte imposée de type ARG2 distribué par TA. Les essais sont réalisés dans une géométrie du module de rotation de type Couette en plexiglas, avec un entrefer e = 1mm. Le couple est appliqué à environ 30ml de produit par l'utilisation d'une cellule de cisaillement dans laquelle l'échantillon est chargé. Ce couple induit par une sollicitation électromécanique est alors contrôlé au moyen d'un capteur fixé à la partie mobile du rhéomètre. Ensuite, la contrainte de cisaillement correspondante et la vitesse de déformation (ou gradient de vitesse) générée sont déterminées comme une fonction caractéristique du Couette utilisé, et permet de relier la viscosité apparente (en Pa.s) avec la vitesse de cisaillement (en s⁻¹).

### Respect des règles de rhéologie sur l'ensemble des compartiments

Si le système de distribution ne comporte qu'un seul produit de base contenant des particules denses, celui-ci respecte de préférence les règles de rhéologies définies plus haut.

Si le système comporte deux produits de base ou plus contenant des particules denses :
- au moins un produit de base contenant des particules denses, peut respecter les règles de rhéologie définies plus haut.
- de préférence, tous les produits de base contenant des particules denses respectent les règles de rhéologie définies plus haut.

De préférence, si le système de distribution comporte au moins un compartiment sans particules denses, alors au moins un produit de base ne contenant pas de particules denses, présente une viscosité inférieure ou égale à 2 Pa.s, mieux inférieure ou égale à 1 Pa.s, plus préférentiellement inférieure ou égale à 0,8 Pa.s, encore plus préférentiellement comprise entre 0,6 Pa.s et 0,2 Pa.s.

### Utilisation d'un produit de base pour réduire la viscosité

Comme évoqué juste au-dessus, la faible viscosité du compartiment sans particules permet, par dilution, de limiter la viscosité du mélange distribué.

Cependant, si ce compartiment est utilisé en proportions minoritaires, par exemple àmoins de 33% en masse de la composition finale obtenue si on parle d'un système de distribution à trois compartiments, cet effet de dilution est relativement faible. Ainsi, on peut adjoindre à ce produit de base sans particules denses, un agent limitant la viscosité, comme de l'éthanol.

### Utilisation optionnelle d'un moyen d'homogénéisation

Le système de distribution peut intégrer un moyen d'homogénéisation comme une pièce vibrante ou un autre système mettant en mouvement le produit de base dans le compartiment correspondant.

Le système de distribution peut aussi être prévu pour être mis sur un socle vibrant, par exemple vibrant entre 10000 Hz et 1 Hz, mieux entre 1000 et 5 Hz, encore mieux entre 400 et 100 Hz.

Ce système d'homogénéisation peut être déclenché par l'utilisateur, ou peut être déclenché au sein d'une séquence déclenchée par l'utilisateur. Par exemple, lorsque l'utilisateur met en marche l'appareil, l'homogénéisation est enclenchée. Ou lorsque le système ressent un mouvement (une accélération), le système produit une action d'homogénéisation.

Le système d'homogénéisation peut être déclenché régulièrement, même lorsque le système n'est pas utilisé.

### Utilisation pour le maquillage d'une ou plusieurs zones précises de peau

Le système de distribution permet de se maquiller jour après jour, en ne traitant que les zones à masquer. Pour ce faire, on délivre de petites doses de maquillage, qu'on applique précisément et séquentiellement sur les zones correspondantes. Chaque petite dose est réalisée selon le mélange qui convient à la zone.

Dans un mode préféré de mise en oeuvre de l'invention, le système de distribution attend qu'on le renseigne sur une zone à traiter puis délivre le mélange correspondant. Il peut faire appel à une table de correspondance préprogrammée pour cela, cette table résultant par exemple d'un procédé d'apprentissage tel que défini plus loin. En variante, le système de distribution renseigne la personne, tout en délivrant un mélange, sur la zone sur laquelle celle-ci doit appliquer le mélange. Ainsi, le système de distribution peut suivre un programme d'application où il délivre dans un ordre donné, les différents mélanges à appliquer.

Dans un mode particulier de mise en oeuvre de l'invention, le système de distribution est renseigné sur les quantités à délivrer. Pour cela, il mémorise la correspondance entre la couleur, la zone du visage et la quantité nécessaire, ce qui permet de réduire les coûts et les pertes de produit, et de ne couvrir que faiblement la peau, évitant ainsi des effets d'occlusion. Ce faisant, il est possible d'utiliser des produits très couvrants et trop couvrants pour être appliqués sur l'ensemble du visage. Ainsi, il est possible d'obtenir des maquillages d'aspects naturels, voire indétectables.

Le système de distribution peut aussi permettre, en facilitant la distribution de petites quantités et leur usage rapide, de réduire la durée de conservation des produits, permettant ainsi de réduire les risques d'évolution et/ou les quantités de conservateurs à employer.

Le système de distribution est adapté à traiter les zones qu'on veut masquer, sans avoir à masquer l'ensemble du visage.

Lorsque l'utilisateur recherche la couleur à appliquer sur une zone du visage, il est intéressant de mémoriser la couleur qui va bien pour chaque zone, et le système de distribution est ainsi avantageusement agencé pour mémoriser cette couleur et la zone correspondante. Ainsi, en utilisant les informations mémorisées, à chaque utilisation, on peut obtenir la délivrance du même mélange pour chaque zone, ou, si l'on traite plusieurs zones, la même série de mélanges pour une même série de zones.

Le système de distribution peut aussi être agencé pour permettre de traiter une zone en faisant varier les couleurs application après application. Ainsi, la personne peut maquiller les lèvres avec différentes couleurs, qu'elle choisit au jour le jour selon ses goûts. Cette approche est aussi adaptée aux paupières ou aux cils, ainsi qu'au maquillage du visage car la personne peut avoir envie de changer de couleur de teint. Par exemple, les jours de la semaine, la personne réalise un fond de teint couleur claire, et le weekend, un fond de teint de couleur hâlée, ou un maquillage des yeux d'une couleur un jour, et d'une autre couleur un autre jour.

Le système de distribution peut être agencé pour permettre à l'utilisateur de changer de couleur selon ses goûts du jour, du moment, de ses tenues, du climat. Ainsi, on prévoit avantageusement un système d'aide à la décision pour guider l'utilisateur dans ses choix de couleur.

On peut prévoir aussi un système d'aide pour équilibrer les couleurs sur un même visage et participer à la réalisation d'un maquillage global réussi.

Il peut être souhaitable que plusieurs personnes d'un même groupe, par exemple une famille, puissent utiliser le système de distribution, réduisant ainsi les coûts et minimisant la place occupée. Cette solution est spécialement adaptée aux voyages ou aux hôtels, campings, avions, campings cars, boutiques, écoles... Pour ce faire, on peut prévoir que le système de distribution puisse être renseigné sur la personne qui l'utilise, afin d'accéder à des données personnelles préalablement mémorisées.

### Utilisation en continu pour un maquillage en dégradé

On peut faire en sorte que le système de distribution, pendant qu'il délivre le produit, change la formulation du mélange. On peut déplacer la sortie des produits de base ou du mélange relativement à un contenant ou un support définissant une surface d'application. Dans un mode particulier de mise en oeuvre de l'invention, le système de distribution est agencé pour calculer l'évolution du mélange en fonction de la couleur C1, d'une zone à traiter et de la couleur C2, d'une autre zone à traiter. Par exemple, sachant que le menton nécessite une couleur C1 et que la joue nécessite une couleur C2, le système de distribution peut faire varier la formulation du mélange pendant qu'il le délivre pour réaliser un dégradé de couleur entre ces deux couleurs. Cela permet par exemple de mieux masquer les imperfections du visage en faisant en sorte que le résultat final soit réaliste, ou de faire des dégradés de couleur à des fins d'embellissement. Le système de distribution peut encore être agencé de telle sorte que l'utilisateur puisse commander une variation de couleur du mélange distribué sans que les couleurs de départ et/ou d'arrivée n'aient été préalablement fixées. Pour ce faire, le système de distribution peut disposer d'un système de localisation, ou d'auto-localisation, et déduire d'une table de correspondance les couleurs C1 et C2 qu'il doit réaliser, et donc les évolutions de mélange qu'il doit réaliser.

Le système de distribution peut comporter une tête de sortie, en particulier dans le cas d'un aérographe, qui est mobile et pilotée. Cette option permet alors, sans bouger le reste du système de distribution, de réaliser des dégradés. Par exemple, on localise le système de distribution près de la joue, puis on déclenche un système de commande qui va piloter automatiquement la variation de la formulation du mélange et le mouvement de la tête de sortie, de façon à par exemple avoir le centre de la joue plus rouge que sa périphérie, avec un dégradé entre les deux.

Le système de distribution peut encore être utilisé pour réaliser des produits sur mesure, que l'on conserve pour réaliser plusieurs applications.

Il est aussi possible de réaliser des produits solides ou semi-solides.

### Fabrication de compacts « à façon » ou autres produits solides ou semi-solides

Le système de distribution peut être agencé pour permettre de choisir un mélange et de le délivrer dans un contenant tel qu'une coupelle. Le mélange comporte de préférence des composés qui sont tels que le mélange puisse prendre en masse.

Plus préférentiellement, on utilise des composés qui rendent la prise en masse spécialement rapide. Ces composés sont soit déposés dans le contenant avant ou après son remplissage avec les autres constituants, soit sont prévus dans les compartiments du distributeur avec les autres constituants des produits de base, soit sont compris dans le distributeur dans un compartiment spécialement prévu pour les contenir.

On peut ainsi distribuer des compositions spécifiques qui peuvent par réaction chimique, biochimique ou physicochimique, durcir de façon accélérée après évacuation.

Ces compositions sont spécialement adaptées à la réalisation des compacts, c'est-à-dire :
- Prennent en masse,
- donnent un matériau qui peut se déliter en cas de frottement, et sont préférentiellement colorées.

De préférence, ces compositions sont très riches en particules solides, avec par exemple plus de 10% en masse de particules solides par rapport à la masse totale de la composition, mieux plus de 20% en masse de particules solides par rapport à la masse totale de la composition, encore mieux plus de 30% en masse de particules solides par rapport à la masse totale de la composition, préférentiellement entre 10 et 40% en masse de particules solides par rapport à la masse totale de la composition.

Ces compositions peuvent contenir des particules absorbantes, ou des composés réactifs, tels que ceux qui réagissent en contact avec l'air, par exemple du cyanoacrylate ou des alphasilanes, ou qui réagissent à la lumière, notamment UV.

Le contenant dans lequel le mélange est distribué peut comporter un composé A et les compositions distribuées un composé B, A et B étant choisis pour réagir entre eux et solidifier le mélange.

Dans un mode particulier de mise en oeuvre de l'invention, le système de distribution intègre un moyen de chauffage, par exemple à résistance électrique, pour la réalisation de rouges à lèvres ou autres produits cireux. Dans ce cas, les produits de base sont chauffés avant d'être délivrés.

Le système de distribution peut aussi comporter un moyen pour apporter de l'énergie, thermique et/ou lumineuse, après distribution du mélange dans un contenant, par exemple une résistance électrique ou une diode LED, notamment UV. Cette énergie peut accélérer la prise en masse du mélange distribué.

Préférentiellement, le mélange est homogénéisé avant la prise en masse, donc homogénéisé après délivrance.

### Réalisation de palettes de couleurs

Le système de distribution peut comporter un support, ayant plusieurs régions, et être agencé pour générer automatiquement plusieurs mélanges déposés dans lesdites régions, par exemple une série de couleurs adaptées à différentes parties du visage.

Le support peut définir plusieurs cavités pour recevoir les mélanges ou porter plusieurs contenants, par exemple sous forme de coupelles, éventuellement séparables du support.

Dans un cas particulier, le support prend la forme d'un visage avec des régions pour recevoir les mélanges pour des zones d'applications ciblées.

Le support être mobile, notamment rotatif, par rapport au corps du distributeur, et par exemple entraîné en déplacement par le distributeur, pour remplir successivement différents logements ou contenants.

### Distributeur à coupelle

Il existe un intérêt pour bénéficier d'un système de distribution capable de délivrer un mélange pouvant être facilement prélevé par l'utilisateur. Par ailleurs, dans le cas où les produits de base délivrés par le système de distribution ne sont pas déjà mélangés, il existe un besoin pour permettre à l'utilisateur d'effectuer aisément le mélange.

Le système de distribution peut ainsi comporter une coupelle, et un distributeur pour remplir la coupelle avec au moins un produit, la coupelle étant solidaire du distributeur au moins pendant son remplissage.

La coupelle est parfois encore appelée « creuset » et ce terme doit être compris avec une acceptation large.

Par « coupelle solidaire du distributeur » il faut comprendre que la coupelle est retenue, notamment immobilisée, au moins temporairement sur le distributeur, étant par exemple fixée sur celui-ci par des vis, par aimantation, par encliquetage, par verrouillage baïonnette, par serrage, ou réalisée avec une partie du corps du distributeur par moulage de matière. La coupelle lorsqu'elle est solidaire du distributeur permet de manipuler celui-ci d'une main, la coupelle restant en place sur le distributeur au cours des mouvements de ce dernier.

Le distributeur peut être proposé à l'utilisateur avec la coupelle déjà en place.

En variante, celle-ci est installée par l'utilisateur à la première utilisation du système de distribution.

De préférence la coupelle est moins profonde que large, ce qui facilite l'accès à celle-ci, et permet de prélever le produit, notamment le mélange avec un applicateur ou le doigt.

De préférence, la coupelle est séparable du distributeur, et constitue une interface de sortie qui peut être choisie parmi un ensemble d'interfaces de sortie pouvant être montées sur le distributeur, au choix de l'utilisateur en fonction du maquillage à réaliser, comme détaillé plus loin.

De préférence, le système de distribution comporte plusieurs orifices de remplissage en des produits de base différents, débouchant dans la coupelle. Ainsi, le mélange de ces produits peut avoir lieu dans la coupelle.

La coupelle a de préférence un fond de forme concave vers l'extérieur, ce qui peut faciliter son nettoyage par l'utilisateur entre deux utilisations.

De plus, cela peut faciliter le prélèvement du produit par l'utilisateur, et le mélange des produits de base.

De préférence, le distributeur permet de délivrer au moins deux produits de base dans la coupelle, dans des proportions réglables, et mieux au moins trois produits.

Dans un exemple de mise en oeuvre, le système de distribution comporte au moins deux coupelles pouvant être sélectivement alimentées par le distributeur. Cela peut permettre à l'utilisateur de remplir rapidement ces deux coupelles avec des mélanges dont les caractéristiques sont différentes. Cela peut faciliter les essais de matières colorées, et/ou permettre de préparer plusieurs mélanges de couleurs différentes destinées au maquillage de zones respectives du visage. Les coupelles peuvent être associées à des identifiants rappelant à l'utilisateur à quelle zone du visage un mélange contenu dans une coupelle donnée est destiné.

Les coupelles peuvent être mobiles relativement au distributeur, étant par exemple portées par un support mobile, tel qu'une tourelle mobile en rotation par rapport au distributeur, ou par un tiroir mobile en translation par rapport au distributeur.

Le système de distribution peut comporter un couvercle de fermeture de la coupelle. Ce couvercle de fermeture est de préférence transparent pour permettre à l'utilisateur de visualiser la couleur du mélange contenu à l'intérieur.

Lorsque la coupelle est séparable du distributeur, elle peut être introduite le cas échéant dans un boîtier permettant de la transporter plus facilement, ce boîtier pouvant comporter le cas échéant un miroir et/ou un applicateur. Le couvercle du boîtier peut dans ce cas servir de couvercle pour la coupelle.

Le volume de la coupelle peut être compris entre 2 et 1000 mm³, mieux entre 100 et 1000 mm³, encore mieux entre 250 et 750 mm³.

Le ou les produits de base délivrés dans la coupelle sont de préférence des fonds de teint, mais en variante il s'agit de produits de maquillage des lèvres ou des paupières.

La coupelle est de préférence de forme symétrique de révolution. En variante, elle est de contour polygonal ou autre. Son plus grand diamètre intérieur, ou celui du cercle inscrit dans le cas d'un contour non circulaire, est de préférence compris entre 2 et 100 mm, préférentiellement entre 5 et 40 mm. Sa profondeur est de préférence comprise entre 1 et 10 mm, mieux entre 3 et 8 mm. De préférence, la taille et la forme de la coupelle permettent soit une application directe du mélange sur la peau, soit une préhension au doigt ou avec un applicateur. La coupelle peut être réalisée avec un matériau élastiquement déformable, ce qui permet par exemple d'inverser la concavité du fond de la coupelle et de vider celle-ci plus facilement ou de l'utiliser pour appliquer le produit.

La coupelle peut être dépourvue de mélangeur ; dans ce cas, les produits de base peuvent arriver du distributeur dans la coupelle à l'état non mélangé, par des orifices de distribution respectifs distincts. En variante, le distributeur intègre un mélangeur et les produits de base arrivent déjà mélangés dans la coupelle.

La coupelle peut aussi intégrer un mélangeur statique, comme détaillé plus loin, qui est alimenté par des orifices de remplissage distincts du distributeur, et qui délivre de préférence le mélange dans une cavité de la coupelle située au-dessus du mélangeur.

L'invention a encore pour objet un procédé de préparation d'un produit de maquillage, comportant l'étape consistant à remplir une coupelle d'un système de distribution tel que défini plus haut avec au moins un produit de base provenant du distributeur.

Plusieurs produits peuvent être délivrés dans le fond de la coupelle, puis mélangés à l'aide du doigt ou d'un applicateur, ou d'un mélangeur statique intégré à la coupelle.

La coupelle est de préférence remplie par le dessous. Il a été proposé dans le passé des systèmes de distribution à l'aide d'une sonotrode.

La coupelle selon l'invention n'est pas destinée à entrer en vibration pour distribuer le ou les produits amenés par le ou les canaux d'alimentation de celle-ci. Elle est différente d'une sonotrode. De préférence, la coupelle est réalisée en matière plastique.

### Mélangeur intégré à l'interface de sortie

Il existe un intérêt pour bénéficier d'un système de distribution capable de délivrer un mélange pouvant être facilement utilisé, notamment prélevé par l'utilisateur, sans nécessiter une action additionnelle de mélange de la part de celui-ci.

Le système de distribution peut ainsi comporter un distributeur ayant des canaux de sortie de produits de base et une interface de sortie séparable du distributeur, cette interface ayant un mélangeur statique, délivrant de préférence le mélange dans une cavité où il peut être prélevé.

Le mélangeur statique peut être situé sous la cavité précitée. Le système de distribution est alors particulièrement adapté à la création de compacts, en utilisant comme interfaces de sortie des coupelles à mélangeur statique intégré. Dans ce cas, on vient remplir la cavité de la coupelle en produit par le dessous. Après passage dans le mélangeur statique, les produits de base mélangés viennent recouvrir le mélangeur.

Il est possible d'utiliser plusieurs interfaces de sortie et de les remplir avec des mélanges respectifs différents, sans avoir à purger le mélangeur, ce qui réduit les pertes de produit. L'interface de sortie peut être à usage unique, le cas échéant.

De préférence, le mélangeur statique comporte une chambre centrale communiquant avec des canaux d'admission des produits de base. Cette chambre centrale peut communiquer avec une chambre périphérique comportant une série de cloisons qui agissent comme des déflecteurs pour le mélange, et créent un cisaillement de celui-ci.

La chambre périphérique peut comporter une cloison annulaire ajourée définissant des ajours par lesquels passe le mélange en circulant dans la chambre périphérique. Les chambres centrale et périphérique peuvent être fermées supérieurement par une paroi qui définit le fond de la cavité recevant le mélange.

Le fond de la chambre périphérique peut être de forme hélicoïdale autour de l'axe de la coupelle et de hauteur diminuant au fur et à mesure que l'on progresse vers la sortie. Cette dernière peut déboucher en avant d'une rampe de liaison entre le fond de la chambre périphérique et la paroi supérieure du mélangeur, cette rampe de liaison étant de préférence une portion d'hélice prolongeant l'hélice formée par la paroi de fond de la chambre périphérique.

De préférence la chambre périphérique comporte la cloison annulaire précitée et des cloisons radiales qui forcent le mélange à circuler alternativement entre des régions supérieure et inférieure de la chambre périphérique et entre des régions radialement intérieure et extérieure, le mélange circulant par exemple d'une région supérieure et radialement extérieure à une région inférieure et radialement extérieure en passant à travers la cloison annulaire précitée.

Le mélangeur peut comporter un corps extérieur dans lequel est reçue une pièce formant le coeur du mélangeur, le corps extérieur fermant radialement à l'extérieur la chambre périphérique et comportant un montant qui sépare les chambres centrale et périphérique.

Le corps extérieur du mélangeur et le coeur du mélangeur peuvent être chacun réalisés d'une seule pièce par moulage par injection.

### Volume mort réduit

Il existe un intérêt pour réduire les pertes de produit lors des changements de formulation du mélange, ainsi que pour permettre de faire varier le plus rapidement possible la couleur du mélange au cours de l'application, notamment en cas de couplage du distributeur avec un aérographe.

Selon l'un de ses aspects, indépendamment ou en combinaison avec les autres aspects de celle-ci, notamment ce qui précède, l'invention a pour objet un système de distribution d'un produit de maquillage, comportant un distributeur recevant au moins deux cartouches ayant chacune un réservoir contenant un produit de base, ce dernier quittant la cartouche par un canal de sortie de la cartouche, ce canal de sortie débouchant à l'extérieur du distributeur ou à proximité de sa surface externe.

Le canal de sortie peut notamment déboucher dans une zone de prélèvement du mélange ou à proximité de celle-ci, notamment à moins de 5 mm, mieux à moins de 3 mm, mieux à moins de 1mm, mieux à fleur.

La section transversale du canal de sortie est par exemple comprise entre 1 et 3 mm².

Ainsi, chaque produit de base provenant d'une cartouche peut quitter le distributeur sans se mélanger avec un produit de base d'une autre cartouche et l'on minimise le volume mort qui ne peut être prélevé et susceptible d'accroître l'inertie du système. Le produit est plus rapidement disponible sans avoir à circuler dans des canaux spécifiques du boîtier du distributeur, évitant une étape fastidieuse de purge si changement de cartouche.

L'extérieur du distributeur peut être la zone de prélèvement du produit, notamment lorsque le distributeur est réalisé avec une coupelle non prévue pour être enlevée, dans laquelle le mélange est distribué, ou une zone du distributeur destinée au montage d'une interface de sortie amovible, qui définit la zone de prélèvement. Cette interface de sortie peut comporter une coupelle telle que définie ci-dessus. Cette zone de montage correspond par exemple à l'extrémité du boîtier du distributeur en l'absence de l'interface de sortie. La zone de montage peut être sensiblement plane et perpendiculaire à l'axe longitudinal du boîtier du distributeur.

Le distributeur peut comporter trois cartouches de produits de base.

Le distributeur peut comporter des logements pour recevoir les cartouches, lesquelles sont de préférence reçues de façon amovible dans le distributeur. Ce dernier peut comporter des passages pour des conduits des cartouches définissant les canaux de sortie.

La longueur de ces conduits est de préférence telle que les conduits se situent légèrement en retrait du fond ou à affleurement de la cavité servant au prélèvement du produit, ou en variante légèrement en retrait ou à affleurement de la face d'extrémité du boîtier du distributeur définissant la zone de montage.

Ces conduits des cartouches peuvent être des embouts servant à entraîner en déplacement des pistons au sein des cartouches, comme détaillé plus haut.

### Interfaces de sortie multiples

Il existe un besoin pour pouvoir, à l'aide d'un même système de distribution, réaliser facilement des maquillages différents, et pouvoir si on le souhaite maquiller des zones aussi différentes que la peau, les lèvres ou les cils ou sourcils.

Le système de distribution peut ainsi comporter un ensemble comportant un distributeur d'au moins un produit cosmétique, notamment de maquillage, et au moins deux interfaces de sortie pouvant se monter chacune de façon amovible sur le distributeur, ces interfaces de sortie permettant de recevoir le ou les produits délivrés par le distributeur, étant de préférence choisies parmi les suivantes :
- interface de sortie comportant un contenant, notamment une coupelle permettant un prélèvement du produit au doigt ou à l'aide d'un applicateur,
- interface de sortie permettant de délivrer le produit à un système de pulvérisation, notamment un aérographe,
- interface de sortie comportant plusieurs régions de réception du produit, mobile par rapport au distributeur,
- interface de sortie permettant de délivrer le produit à un embout de distribution.

De préférence, l'ensemble comporte au moins trois desdites interfaces de sortie, mieux les quatre interfaces de sortie.

Le distributeur peut comporter au moins deux produits de base différents et permettre de délivrer ceux-ci dans des proportions variables, et de préférence, le distributeur comporte trois produits de base différents et permet de délivrer ceux-ci dans des proportions variables.

Chaque interface de sortie peut comporter une base permettant sa fixation sur le distributeur. Cette fixation peut s'effectuer à l'aide de vis par exemple, mais de préférence la base est agencée pour permettre un démontage et un remplacement d'une interface de sortie sans nécessité d'outil. Il s'agit par exemple d'une fixation quart de tour ou à l'aide d'une bague de verrouillage externe.

L'interface de sortie et/ou le boîtier du distributeur peuvent comporter des joints d'étanchéité permettant une communication étanche entre le boîtier du distributeur et l'interface de sortie. Le cas échéant, le distributeur est agencé pour reconnaître l'interface de sortie qui est montée dessus, par exemple grâce à identifiant de l'interface de sortie sous forme de reliefs spécifiques, lesquels sont détectés par le distributeur, ou d'une puce électronique reconnue par le distributeur. Cela peut permettre d'adapter le fonctionnement du distributeur à l'interface de sortie montée dessus. Le distributeur peut communiquer à un système informatique des informations sur l'interface de sortie qu'il porte, et le système informatique peut afficher en fonction de ces informations un écran spécifique et/ou lancer un programme spécifique de commande des paramètres de fonctionnement du distributeur, afin par exemple d'adapter la dose distribuée et/ou le débit à la nature de l'interface de sortie.

Plusieurs interfaces de sortie peuvent être proposées initialement à l'utilisateur avec un distributeur commun au sein d'un même emballage, par exemple un coffret ou une boîte de carton.

L'invention a encore pour objet un procédé de maquillage, comportant l'étape consistant à sélectionner une interface de sortie, à la monter sur le distributeur, et à délivrer le ou les produits contenus dans le distributeur dans celle-ci.

### Cartographie et apprentissage

Le terme « cartographie » s'entend ici comme un procédé d'indexation d'une couleur et d'une zone, avec enregistrement.

La cartographie peut concerner des applications sur des zones plus petites qu'un cm2. Toutefois, l'oeil nu a alors du mal à discerner si le résultat obtenu est adéquat, et il est préférable de substituer l'évaluation à l'oeil nu par une évaluation instrumentée, avec grossissement. L'application de petites quantités de matière colorée peut être faite au doigt, avec des outils classiques tels que des pinceaux, ou avec des applicateurs spécialisés.

La cartographie peut être générée lors d'une période d'apprentissage, pendant laquelle l'utilisateur effectue des tests avec des mélanges sur différentes zones du visage ; une fois renseignée, la cartographie peut ensuite être utilisée pour le maquillage de tous les jours.

Des interfaces graphiques spécifiques peuvent servir pendant la période d'apprentissage et pendant la période d'utilisation de la cartographie.

En particulier, le système de distribution peut être utilisé avec une interface graphique où l'opérateur voit le visage, qui est par exemple schématisé, figuratif ou précis comme une photographie ou une simulation 3D. Dans ce cas, l'opérateur peut pointer une partie du visage sur l'écran pour voir apparaître et/ou délivrer la couleur adéquate.

L'interface graphique peut aussi laisser apparaître les autres zones du visage où l'utilisation de la même couleur est adéquate.

Pour réaliser la cartographie, l'opérateur applique une couleur puis réalise l'évaluation.

Les zones du visage peuvent être traitées les unes après les autres ; par exemple, on réalise l'exercice sur une partie de la joue, puis sur le nez, etc....

Une autre possibilité consiste à réaliser un mélange donné, et appliquer sur plusieurs zones ce même mélange. L'opérateur doit alors chercher la zone du visage pour laquelle la couleur est adaptée. Le mélange est ensuite indexé dans le système informatique en l'attribuant à la ou aux zones du visage pour lesquelles il convient.

Selon l'un de ses aspects, indépendamment ou en combinaison avec les autres aspects de celle-ci, et notamment ce qui précède, l'invention a pour objet un procédé d'apprentissage d'un système de distribution comportant un distributeur permettant de distribuer un mélange de couleur variable, et un système informatique permettant de sélectionner une couleur et de mémoriser des données, comportant les étapes consistant à :
a) Sélectionner au moins une couleur à l'aide d'une interface du système informatique,
b) délivrer à l'aide du distributeur au moins un mélange de la couleur sélectionnée,
c) évaluer le ou les mélanges distribués après application sur au moins une zone du visage,
d) mémoriser les caractéristiques d'au moins un mélange, notamment un mélange que l'utilisateur souhaite pouvoir rappeler, et d'au moins une zone sur laquelle il a été testé.

Cette mémorisation peut s'effectuer notamment en vue d'une distribution ultérieure de ce mélange pour maquiller ladite zone.

De préférence, le système informatique est agencé pour permettre à l'utilisateur de signaler si le résultat du test est satisfaisant ou non, voire de le renseigner sur la comparaison à un test effectué précédemment.

On peut aussi réaliser un mélange donné, et chercher la zone du visage pour laquelle il est adapté. On enregistre alors le mélange en l'attribuant à la ou aux zones du visage pour lesquelles il est adapté, dans une table de correspondance qui servira par la suite, pour, partant d'une zone de la peau, en déduire le mélange à utiliser.

On peut procéder de la même façon avec d'autres mélanges pour réaliser une cartographie de l'ensemble du visage et ainsi avoir une table de correspondance complète du visage.

On peut aussi réaliser un mélange donné, l'appliquer sur une zone donnée, puis faire varier le mélange jusqu'à obtenir le mélange le plus adapté. On enregistre alors le mélange en l'attribuant à la ou aux zones du visage pour lesquelles il convient, dans une table de correspondance qui servira par la suite, pour, partant d'une zone de la peau, en déduire le mélange à utiliser.

De préférence, le système informatique évalue et mémorise les quantités utilisées zone par zone. Un tel procédé, par « touches d'essai », permet d'identifier le ou les produits dont a besoin la personne qui veut se maquiller. Ainsi, le système de distribution peut servir sur les points de vente pour conseiller les personnes qui veulent se maquiller ou à la maison pour bien définir les produits à commander.

L'interface du système informatique comporte de préférence un écran tactile affichant la couleur du mélange lors de sa sélection.

L'interface peut afficher un visage et permettre de renseigner le système informatique en sélectionnant la zone sur le visage affiché.

Le système informatique est de préférence agencé pour permettre d'associer une zone, des paramètres de reconstitution du mélange, et la date de l'essai et/ou tout autre identifiant du mélange.

Le système informatique est de préférence également agencé pour permettre d'associer en outre à ladite zone, aux paramètres de reconstitution du mélange, et à la date ou à l'identifiant du mélange, au moins l'une des données suivantes : le nom de la zone, la période de l'année, le nom d'un évènement, un identifiant de l'utilisateur, l'âge de l'utilisateur.

Les étapes a) à c) peuvent être renouvelées au moins une fois avant la mémorisation des caractéristiques du mélange à l'étape d).

Le système informatique peut être agencé pour rechercher dans une base de données une référence de produit commerciale, sur la base des caractéristiques du mélange identifié comme convenant à au moins une zone donnée, et la communiquer à l'utilisateur.

La sélection à l'étape a) peut s'effectuer avec l'aide d'un système expert externe ou non au système informatique.

Le système expert peut analyser une image de l'utilisateur, pour proposer une couleur de mélange au moins sur la base de l'image analysée.

L'étape a) peut être précédée par la proposition d'une couleur et d'une zone à tester avec un mélange de cette couleur, par le système informatique à l'utilisateur.

Le système informatique peut être agencé pour permettre à l'utilisateur de le renseigner sur l'appréciation du résultat du test de l'étape c) et pour générer une proposition de modification du mélange à sélectionner lors du retour à l'étape a).

Le système informatique peut être agencé pour proposer au moins une couleur de mélange à l'étape a) en fonction d'une zone d'application renseignée par l'utilisateur.

Le système informatique peut être agencé pour proposer au moins une zone d'application à l'étape a), en fonction d'une couleur renseignée par l'utilisateur.

Le distributeur peut délivrer à l'étape b) au moins deux mélanges de couleurs différentes, de préférence séparés, en vue de leur application simultanée sur la zone de test.

Cela peut faire gagner du temps, et faciliter la comparaison des résultats.

L'invention a encore pour objet un procédé de maquillage à l'aide d'un système de distribution selon l'invention, dans lequel :
a) L'utilisateur adresse une requête concernant un besoin de maquillage au système informatique,
b) celui-ci génère en retour une proposition de couleur en vue du maquillage d'une zone associée, sur la base de l'apprentissage effectué préalablement, et
c) le système informatique pilote le distributeur pour la production du mélange de la couleur proposée, notamment si celle-ci est validée par l'utilisateur.

Un tel procédé peut utiliser une cartographie précédemment établie avec l'utilisateur.

L'invention a encore pour objet un produit programme d'ordinateur comportant des instructions de code permettant, lorsque exécutées dans un système informatique, d'amener le système informatique à :
- Permettre à l'utilisateur de sélectionner au moins une couleur et/ou une zone d'application, notamment à l'aide d'une interface telle qu'un écran tactile,
- piloter un distributeur de façon à délivrer un mélange de la couleur sélectionnée par l'utilisateur,
- permettre à l'utilisateur de déclencher la mémorisation de la couleur du mélange et d'une zone d'application associée, notamment en vue d'une distribution ultérieure du même mélange, notamment sur la même zone.

Le produit programme d'ordinateur peut comporter des instructions de code permettant, lorsque exécutées dans un système informatique, d'amener le système informatique à :
- Recevoir une requête de l'utilisateur concernant un besoin de maquillage, notamment à l'aide d'une interface telle qu'un écran tactile,
- proposer sur la base au moins de données générées par le procédé d'apprentissage tel que défini ci-dessus, au moins une couleur et/ou une zone d'application,
- piloter un distributeur pour la production du mélange de la couleur proposée, notamment si celle-ci est validée par l'utilisateur.

### Aide à distance

Il est souhaitable de pouvoir aider l'utilisateur à se maquiller, notamment à choisir les bons coloris.

L'invention, selon l'un de ses aspects, indépendamment ou en combinaison avec ses autres aspects, notamment ce qui précède, a ainsi pour objet un procédé de maquillage comportant les étapes consistant à :
- permettre l'établissement d'une liaison vidéo, par exemple via internet, entre une caméra d'un premier site et un deuxième site,
- permettre au deuxième site de piloter directement ou indirectement un distributeur présent sur le premier site, ce distributeur permettant de faire varier la couleur d'un mélange distribué,
- permettre à une personne présente au premier site d'appliquer le mélange distribué et d'envoyer vers le deuxième site une image correspondante, pour recevoir en retour une information relative au résultat de maquillage.

Le deuxième site peut notamment comporter un écran de visualisation qui permet à un conseiller présent devant cet écran de voir le résultat du maquillage avec le produit distribué par le distributeur et de conseiller la personne qui s'est maquillée. Ce conseiller peut agir en retour sur le distributeur pour modifier la couleur du mélange et l'adapter au mieux au visage de la personne présente sur le premier site. Ainsi, cette personne gouverne le mélange délivré par le distributeur. La première personne peut se maquiller sous les yeux de la seconde. La seconde personne voit sur son écran le résultat de l'essai, et ainsi peut corriger le mélange qu'elle va commander à distance, jusqu'à obtenir le maquillage idéal.

Le cas échéant, l'acquisition vidéo peut être calibrée à l'aide d'une mire, ou avec le mélange distribué par le distributeur sur une surface de référence. Cela permet ensuite une visualisation plus fidèle du maquillage effectué sur le premier site.

De préférence, la liaison vidéo entre les deux sites est une liaison duplex.

Le premier site peut recevoir un tutoriel du deuxième site, le cas échéant.

Des identifiants des produits de base peuvent être communiqués au deuxième site ; cela peut permettre de connaître précisément la couleur de chacun des produits de base.

Le procédé peut comporter la mémorisation des paramètres de réglage du distributeur, une fois qu'un mélange donné est estimé satisfaisant. De préférence, cette mémorisation peut être commandée depuis le deuxième site. La mémorisation peut avoir lieu dans le système informatique présent sur le premier site et/ou sur un serveur externe.

Une variante peut consister à faire travailler une personne pour aider le maquillage de plusieurs personnes. Ce mode de mise en oeuvre permet le développement des « Make-Up Artists » et leur travail soit au sein d'un institut, soit via internet. Cela permet aussi le maquillage de personnes aux moyens limités, telles que les personnes malvoyantes, ou discernant mal les couleurs, ou âgées, ou manquant de confiance en elles.

### Pilotage par interface tactile

Il existe un besoin pour faciliter le pilotage du système de distribution et notamment le choix de la couleur du mélange distribué.

Le système de distribution peut ainsi comporter un distributeur et un système informatique de pilotage du distributeur, ce système informatique comportant un écran tactile sur lequel peut être affichée la couleur du mélange et un moyen de sélection déplaçable sur l'écran, pour faire varier la couleur du mélange distribué.

De préférence, l'écran affiche des couleurs extrêmes entre lesquelles la couleur du mélange peut être sélectionnée, en déplaçant le moyen de sélection entre ces couleurs extrêmes.

L'écran peut afficher une échelle de couleurs entre au moins deux couleurs, ou une surface, notamment de contour triangulaire, au sein de laquelle le moyen de sélection peut se déplacer. Cette surface peut localement faire apparaître la couleur du mélange en fonction par exemple de la distance à chacun des sommets, matérialisant chacun un produit de base pur.

Le système informatique peut effectuer une partie des calculs nécessaires pour déterminer les fractions de chacun des produits de base conduisant à un mélange de la couleur recherchée.

Le système informatique peut être un téléphone intelligent, un photophone, une tablette, un ordinateur individuel. En variante, il est intégré au boîtier du distributeur.

Le système informatique peut disposer d'une caméra. Cette dernière peut être utilisée notamment pour l'acquisition d'une image de l'utilisateur et/ou du mélange.

Le système informatique peut être agencé pour afficher une image d'un visage, afin de faciliter le repérage des zones sur lesquelles le mélange doit être appliqué.

### Couplage du système de distribution à un moyen de pulvérisation

Le système de distribution peut comporter ou être connecté à un moyen de pulvérisation du mélange, de préférence un aérographe.

Le système de distribution peut comporter un système de pulvérisation, de préférence un aérographe, l'ensemble comportant :
- Un moyen de pulvérisation, de préférence un aérographe comportant une chambre de prélèvement soumise à un courant d'air d'entraînement,
- un distributeur comportant au moins deux compartiments contenant des produits de base différents, les produits étant délivrés au moyen de pulvérisation de préférence par des orifices de distribution distincts.

Le distributeur peut comporter trois cartouches contenant des produits de maquillage de couleurs différentes.

L'aérographe peut comporter un stylet définissant la chambre de prélèvement, le stylet se fixant sur le distributeur ou sur une interface de sortie fixée au distributeur, ou faisant partie intégrante de cette interface de sortie.

Le système de distribution peut comporter un circuit de contrôle du fonctionnement du distributeur, permettant de faire varier la proportion des produits de base délivrés dans la chambre de prélèvement, au cours du fonctionnement de l'aérographe. Les proportions peuvent être modifiées en fonction du déplacement de l'aérographe relativement à la surface sur laquelle le mélange est pulvérisé. Ce déplacement peut être mécanisé, le cas échéant.

Ce circuit de contrôle peut comporter ou être constitué par un système informatique tel que défini plus haut.

Le boîtier du distributeur peut servir de poignée lors de la manipulation d l'ensemble pour délivrer le mélange.

Le distributeur peut comporter une caméra et/ou un ou plusieurs capteurs tels que des accéléromètres afin de localiser automatiquement la zone sur laquelle le mélange est appliquée, et pouvoir régler automatiquement la couleur en fonction de la position, le cas échéant.

L'invention a encore pour objet un procédé de maquillage à l'aide d'un ensemble tel que défini ci-dessus, dans lequel un mélange est pulvérisé sur la peau à l'aide du moyen de pulvérisation, notamment de l'aérographe.

La composition du mélange peut être modifiée au cours du déplacement de l'aérographe relativement à la peau. Un dégradé peut être réalisé.

Cet aspect de l'invention repose sur la constatation que le distributeur peut être utilisé pour alimenter le système de pulvérisation, notamment l'aérographe, tout en permettant au système de distribution d'être suffisamment réactif pour autoriser un changement de la couleur du mélange distribué en cours de maquillage du visage, notamment lorsque la zone à maquiller change.

Il peut être avantageux que la distribution des produits se fasse de façon itérative, notamment avec des temps de distribution non déphasés entre les différents produits.

Cela peut rendre plus facile de faire varier la composition du mélange distribué au cours du temps.

Le mélange peut être réalisé directement dans l'aérographe, sans pratiquement aucun volume mort gênant, permettant ainsi de changer le mélange pulvérisé en temps réel.

La dépression créée dans la chambre de prélèvement est suffisante pour entraîner les produits de base, sans pour autant gêner le dosage.

La dépression qui règne dans la chambre de prélèvement est par exemple comprise entre 10 mbar et 200 mbar, mieux entre 50 et 150 mbar, encore mieux entre 75 et 125 mbar.

La section des canaux d'arrivée des produits de base dans la chambre est par exemple comprise entre 1 et 3 mm², mieux entre 2 et 3 mm².

L'alimentation en produit s'effectue de préférence de façon continue.

Il est encore possible d'appliquer simultanément des produits de base non miscibles ou réactifs, comme un gel aqueux et un gel huileux, qui vont se déposer de façon pixellisée directement sur la peau, produisant une sorte de gel/gel in situ, des silicones réactives, ou des produits de coloration réagissant ensemble. Les ratios en produits de base peuvent être ajustés en fonction du résultat particulier souhaité. Par exemple, dans le cas des gels aqueux et gels huileux, on pourra faire varier le ratio correspondant au volume du premier produit de base sur le volume du deuxième produit de base entre 10/1 et 1/10, mieux entre 5/1 et 1/5.

### Système de localisation ou d'auto-localisation

Le système de distribution selon l'invention peut disposer d'un système de localisation ou d'auto-localisation.

On appelle système de localisation, un moyen de saisir, de la part de la personne, la zone qu'elle va traiter. Ceci peut être réalisé notamment par des systèmes laissant libre au moins une main. Ainsi, on peut utiliser une interface d'un système informatique telle qu'un écran tactile, un joystick, ou un système de reconnaissance vocale.

On appelle système d'auto-localisation, un moyen de saisir, sans que la personne n'intervienne, la zone à traiter. Ceci peut être réalisé par un ou plusieurs accéléromètres qui déduisent, par le fait des mouvements, les directions que la personne vise ou par une caméra et un système de reconnaissance d'image.

L'invention pourra être mieux comprise à la lecture de la description détaillée qui va suivre, d'exemples de mise en oeuvre non limitatifs de celle-ci, et à l'examen du dessin annexé, sur lequel :
- la figure 1 est une vue schématique en perspective d'un exemple de système de distribution conforme à l'invention,
- la figure 2 est une vue arrière du système de distribution de la figure 1,
- la figure 3 illustre le prélèvement de produit délivré par le système de distribution,
- la figure 4 est une vue schématique en perspective, avec enlèvement de certains composants, du système de distribution de la figure 1,
- la figure 5 représente isolément et p artiellement une cartouche de produit de base pour le distributeur,
- la figure 6 représente le haut de la cartouche avec la pièce d'entraînement,
- la figure 7 représente isolément la pièce d'entraînement, en perspective,
- la figure 8 représente isolément un support de la cartouche,
- la figure 9 représente le mécanisme d'entraînement du distributeur,
- la figure 10 représente l'un des moteurs isolément, accouplé au reste du mécanisme d'entraînement,
- la figure 11 représente une carte électronique de commande des moteurs,
- la figure 12 est une coupe transversale du distributeur,
- la figure 13 représente le boîtier du distributeur sans l'interface de sortie,
- la figure 14 représente isolément un premier exemple d'interface de sortie, en vue de dessus,
- les figures 15, 15A, 16, 16A et 17 représentent d'autres exemples d'interfaces de sortie,
- les figures 18 et 19 sont deux autres vues de l'interface de sortie de la figure 17,
- la figure 20 représente isolément le mélangeur statique,
- la figure 21 est une coupe axiale de l'interface de sortie de la figure 17,
- la figure 22 représente une autre interface de sortie, destinée à coopérer avec un aérographe,
- la figure 23 représente en transparence les différents canaux de l'interface de sortie de la figure 22,
- la figure 24 illustre l'interface de sortie des figures 22 et 23 reliée à un aérographe,
- les figures 25, 27 à 29, 29A, 29B et 29C représentent d'autres exemples d'interfaces de sortie,
- la figure 30 illustre le pilotage du distributeur à l'aide d'un terminal portable,
- la figure 31 représente un exemple d'interface graphique permettant de piloter le distributeur,
- la figure 32 représente un autre exemple d'interface graphique,
- la figure 33 illustre un exemple d'évolution de l'interface graphique de la figure 32 au cours de l'utilisation du dispositif,
- la figure 34 représente un autre exemple d'interface graphique,
- les figures 35 et 36 représentent d'autres exemples d'interfaces graphiques,
- la figure 37 illustre l'évolution de l'interface de la figure 36 au cours de l'utilisation du dispositif,
- la figure 38 représente une interface graphique d'un exemple de système informatique selon l'invention,
- la figure 39 représente un exemple de table de correspondance,
- la figure 40 est un schéma en blocs illustrant des étapes d'un exemple de procédé selon l'invention,
- les figures 41 à 44, et- 46 sont des vues analogues à la figure 40 d'autres exemples de procédés,
- la figure 45 représente un exemple de support permettant l'application de plusieurs compositions colorées différentes,
- la figure 47 illustre un système permettant l'échange d'informations avec un conseiller distant, et
- la figure 48 illustre un support comportant une pluralité de logements contenant des mélanges différents.

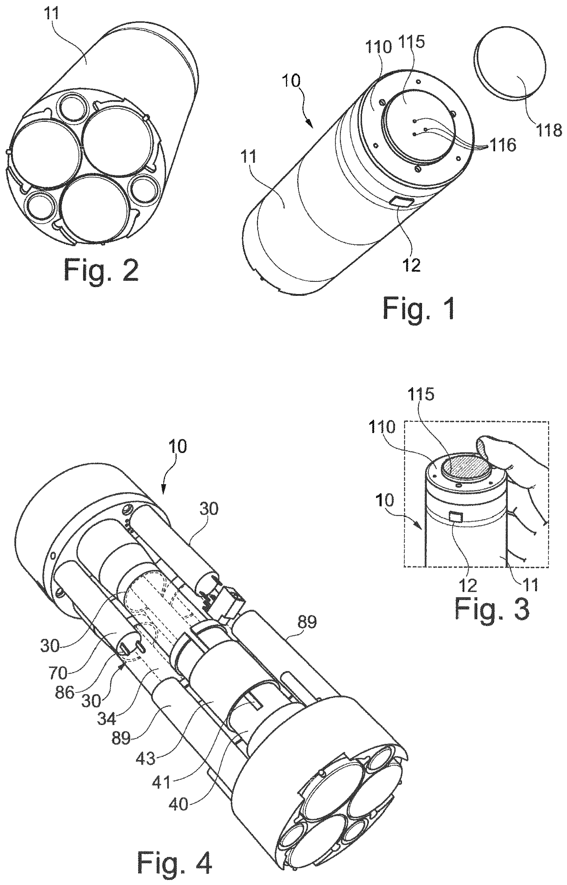

Un exemple de système de distribution 10 pouvant convenir à l'invention est représenté sur les figures 1 et 2, comporte un distributeur 11 qui est muni en partie supérieure d'une interface de sortie 110 par laquelle s'effectue la distribution d'un produit cosmétique de formulation personnalisée.

Le distributeur 11 peut être manipulé d'une main. Sa longueur, hors interface de sortie, est par exemple comprise entre 140 et 160 mm et son diamètre entre 40 et 60 mm.

Le système de distribution 10 peut comporter, comme illustré, un moyen d'actionnement permettant de contrôler la distribution, par exemple un bouton-poussoir 12.

Lorsque l'utilisateur appuie sur le bouton-poussoir 12, le distributeur 11 délivre le produit à partir d'informations qui lui ont été préalablement communiquées par un système informatique, par exemple à l'aide d'une transmission sans fil, comme cela sera détaillé plus loin. Le fonctionnement du bouton-poussoir 12 peut être programmé depuis une interface du système informatique, de façon à délivrer en continu le mélange tant qu'une pression est exercée, ou délivrer uniquement une dose prédéfinie, indépendamment de la durée pendant laquelle l'utilisateur appuie sur le bouton-poussoir.

Comme visible notamment sur la figure 4, le distributeur 11 loge plusieurs
cartouches 30 contenant chacune un produit de base, le distributeur 11 permettant de doser la quantité de chacun des produits de base qui est distribuée de façon à obtenir après un mélange des doses distribuées un produit présentant les propriétés recherchées.

Chacune des cartouches 30 peut être introduite dans le boîtier du distributeur 11 par l'arrière, comme illustré à la figure 2. Dans l'exemple considéré, le distributeur 11 reçoit trois cartouches 30, mais l'invention s'étend également au cas où le nombre de cartouches 30 est différent.

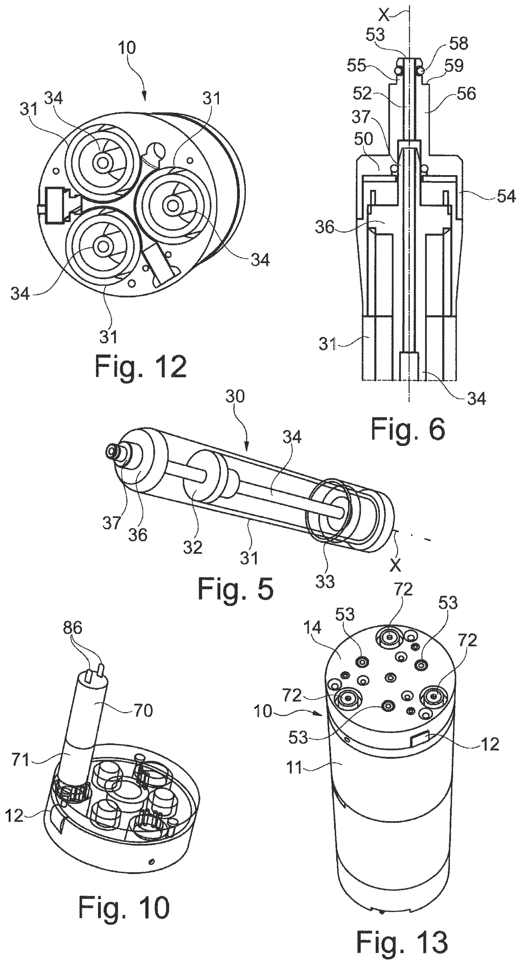

On a représenté isolément à la figure 5 une cartouche 30. Celle-ci comporte un corps 31 dans lequel peut se déplacer selon l'axe longitudinal X de la cartouche un piston 32 de façon à diminuer le volume d'un réservoir 33 situé sous le piston 32, contenant le produit de base correspondant. Le volume du réservoir est de préférence compris entre 2 et 5 mL, étant par exemple de l'ordre de 3 mL.

Le piston 32 est entraîné en déplacement selon l'axe X par une tige creuse 34, filetée extérieurement, venant en prise avec un filetage correspondant traversant le piston 32.

La tige 34 définit un canal par lequel le produit contenu dans le réservoir 33 peut circuler lorsque le piston 32 se déplace dans le corps 31 dans le sens d'une diminution du volume du réservoir 33.

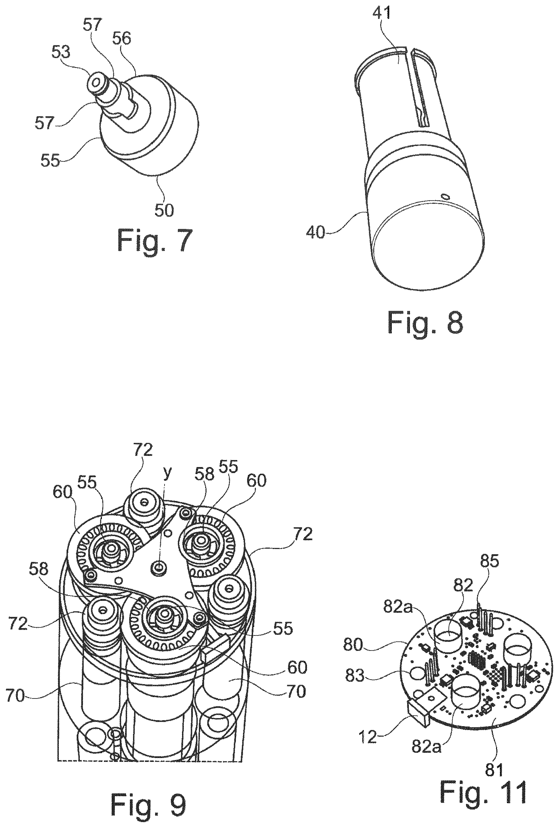

La tige 34 est entraînée en rotation autour de l'axe X par une tête 36 qui peut tourner relativement au corps 31, et communique avec une canule 37. Chaque cartouche 30 est montée dans le distributeur 11 avec une pièce de support 40, que l'on a représentée isolément à la figure 8, qui comporte un manchon de serrage 41 fendu axialement, sur lequel peut coulisser une bague de verrouillage 43, visible sur la figure 4.

Lors de la mise en place d'une cartouche 30, la pièce de support 40 est engagée sur celle-ci, du côté opposé à la canule 37, et la bague de verrouillage 43 déplacée sur le manchon 41 pour serrer la pièce de support 40 sur le corps 31. La pièce de support 40 permet d'immobiliser la cartouche 30 dans le boîtier du distributeur 11.

La tête 36 de la cartouche 30, présentant la canule 37, est coiffée par une pièce d'entraînement 50, représentée isolément à la figure 7, qui vient serrer la tête 36 de façon à pouvoir tourner autour de l'axe X avec elle.

Lorsque la pièce d'entraînement 50 est entraînée en rotation autour de l'axe X, sa rotation est transmise à la tête 36, qui peut tourner relativement au corps 31 et entraîner avec elle en rotation la tige 34.

La force de friction du piston 30 sur la surface intérieure du corps 31 est suffisante pour que le piston 32 ne tourne pas relativement au corps 31, de sorte que la rotation relative de la tige 34 et du piston 32 provoque le déplacement de celui-ci selon l'axe X. Ce déplacement s'accompagne d'une diminution du volume du réservoir 33 et d'une remontée du produit de base contenu dans la cartouche 30 par la tige 34, puis dans la canule 37.

La pièce d'entraînement 50 comporte un canal interne 52 alimenté par la canule 37 et qui débouche à l'extérieur par un orifice de distribution 53. Ce canal 52 est formé par un embout 36. La pièce d'entraînement 50 présente une jupe de montage 54 qui recouvre axialement la tête 36 de la cartouche 30. Cette jupe de montage 54 se raccorde par une paroi transversale 55 à l'embout 56.

L'embout 56 comporte des reliefs 57 permettant son accouplement en rotation avec une roue dentée 60, visible notamment sur la figure 9, appartenant à un mécanisme d'entraînement du boîtier du distributeur 11.

Dans l'exemple considéré, les reliefs 57 se présentent sous la forme de deux ergots diamétralement opposés, formant saillie sur l'embout 56 à sa base, qui s'engagent dans des encoches correspondantes de la roue dentée 60.

L'embout 56 présente une partie rétrécie qui comporte une gorge accueillant un joint torique 58. La partie rétrécie se raccorde par un épaulement 59 au reste de l'embout.

La tête 36 de la cartouche 30 peut porter un joint torique qui assure un couplage étanche entre la canule 37 et la pièce d'entraînement 50.

Le mécanisme d'entraînement comporte des moteurs électriques 70, équipés de réducteurs 71, visibles notamment à la figure 10. L'arbre de sortie de ces réducteurs est accouplé à une roue menante 72 qui engrène avec la roue dentée 60.

Dans l'exemple considéré, les axes X longitudinaux de chacune des cartouches sont disposés à 120° les uns par rapport aux autres, autour de l'axe longitudinal Y du boîtier du distributeur 11.

Les moteurs 70 sont disposés entre les cartouches 30, les axes de rotation des moteurs étant également disposés à 120° les uns des autres autour de l'axe Y du distributeur 11. De cette façon, on obtient une construction compacte du distributeur 11.

Les motoréducteurs présentent avantageusement un couple supérieur à
70nN.m. Par exemple, on utilise un moteur de référence Maxon 118392 associé au réducteur planétaire Maxon 218418. Un tel moteur est de diamètre 10 mm, de puissance 1.5 W, de tension nominale 3 V, de vitesse de ralenti 1300 tours/min et de couple maximum 1.5 mNm. Le réducteur est de diamètre 10 mm, de réduction absolue 256 / 1 et de couple 0,2 Nm.

Un circuit électronique 81, représenté isolément à la figure 11, est présent dansle voisinage de l'extrémité supérieure du boîtier du distributeur 1. Ce circuit électronique 81 comporte une carte 80 qui est traversée par des passages 83 pour les axes de sortie des réducteurs 71, ainsi que des ouvertures 82 pour les parties rétrécies 55 des embouts d'entraînement 56.

Des manchons 82a peuvent être fixés sur la carte 80 pour faire barrière vis-àvis d'éventuelles fuites de produit vers le carte 80. Les embouts 56 traversent les manchons 82a, de préférence avec un faible jeu.

La carte 80 porte le bouton-poussoir 12 précité et supporte un certain nombre de broches de sortie 86 servant à l'alimentation des moteurs 70.

Le circuit électronique 81 comporte un microcontrôleur ou analogue permettant de piloter les différents moteurs 70, afin de distribuer la quantité désirée de chacun des produits de base. La résolution de délivrance des produits de base est par exemple comprise entre 0,001 et 0,003 mL, étant par exemple de l'ordre de 0.0025 mL.

Le boîtier du distributeur 11 loge également une batterie dont les accumulateurs 89, se situent avantageusement comme on le voit sur la figure 4, chacun dans le prolongement d'un moteur 70.

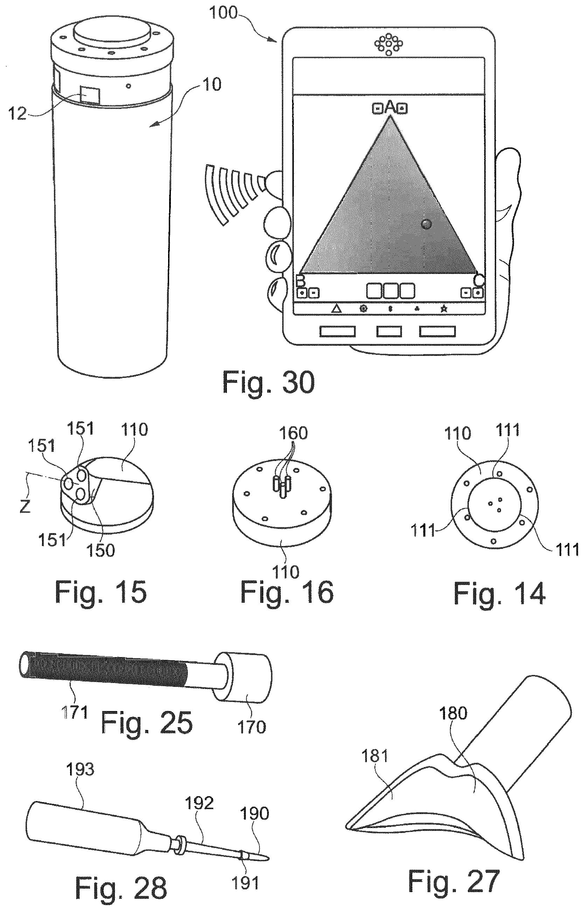

De préférence, comme illustré à la figure 30, le pilotage du distributeur 11 s'effectue à l'aide d'un système informatique 100 tel qu'un terminal portable, par exemple un téléphone intelligent, une tablette par exemple de marque « Ipad » ou un ordinateur personnel.

La transmission des informations de pilotage du distributeur 11 par le terminal 100 s'effectue de préférence sans fil, par exemple par une liaison Bluetooth.

Dans un exemple particulier, la carte électronique 81 permet de gérer les pointd suivants :
- calcul du volume de chaque produit à délivrer en fonction de la consigne de fraction volumique de chaque produit, du mode de fonctionnement (continu, dose ou purge), de la valeur du débit ou du volume,
- mesure des courants d'alimentation des moteurs 70,
- communication Bluetooth avec le système informatique 100,
- gestion du bouton 12 de délivrance des produits,
- gestion de l'interrupteur marche/arrêt,
- gestion de l'affichage de la ou des leds,
- charge de la batterie.

La carte 80 comporte par exemple les composants suivant :
- microcontrôleur CC2541 de Texas Instrument,
- led CMS bleue pour donner des informations de statut à l'utilisateur,
- fusible thermique de protection,
- quartz à 32 MHz,
- interrupteur marche/arrêt

Le microcontrôleur Texas Instrument CC2541 intègre une mémoire flash programmable de 256 ko de RAM ainsi que de nombreuses fonctionnalités. Ce microcontrôleur peut être programmé en C, dans l'environnement IAR Embedded Workbench.

Les orifices de sortie 53 des cartouches 30 débouchent sensiblement à l'extrémité supérieure du boîtier du distributeur 11, comme on peut le voir sur la figure 13 notamment. La face supérieure 14 du boîtier du distributeur 11 définit une surface d'accueil pour le montage d'une interface de sortie qui canalise les produits provenant des cartouches vers une zone de prélèvement ou de distribution.

Dans l'exemple de la figure 1, cette interface de sortie 110 se présente sous la forme d'une pièce rapportée, que l'on a représentée isolément à la figure 14, qui présente à sa périphérie comme illustré des passages 111 pour des vis servant à sa fixation sur le boîtier du distributeur 11.

L'interface de sortie 110 définit dans cet exemple une coupelle 115 dans le fond de laquelle débouchent des orifices d'alimentation 116, en communication chacun par un canal interne à l'interface de sortie 110 avec un orifice de sortie respectif 53.

Ainsi, dans l'exemple considéré, les produits de base contenus dans les cartouches 30 peuvent être distribués jusqu'à la coupelle 115 sans se mélanger.

Lorsqu'il utilise le distributeur 11, l'utilisateur peut remplir la coupelle 115 avec des proportions prédéfinies de chacun des produits de base, comme illustré à la figure 3, puis peut prélever le produit présent dans la coupelle 115 en vue de l'appliquer. Ce prélèvement peut s'effectuer par exemple au doigt, comme illustré, ou à l'aide de tout applicateur cosmétique adapté. La coupelle 115 est de préférence peu profonde, ce qui facilite son nettoyage, et de diamètre suffisamment grand pour ne pas gêner l'accès au produit. La profondeur p de la coupelle 115 est ainsi, de préférence, comprise entre 1 et 5 mm et son diamètre d ou celui du cercle circonscrit lorsque le contour du creuset n'est pas circulaire, est de préférence compris entre 20 et 50 mm. On a de préférence 4 *>*=*d*/*p* <=50.

Les orifices d'alimentation 116 sont de préférence de diamètre inférieur à 3mm par exemple de l'ordre de 1 mm.

L'interface de sortie 110 peut recevoir un couvercle de fermeture 118 de la coupelle 115, pour éviter que le produit ne sèche ou soit exposé à des salissures en l'absence d'utilisation. Ce couvercle 118 est de préférence réalisé en matière plastique transparente et peut se fixer par friction, vissage ou encliquetage sur le montant de la coupelle 115, ou plus généralement à tout endroit adapté sur l'interface de sortie 110.

La contenance maximale de la coupelle 115 est de préférence comprise entre 0.02 et 0.25 ml.

De préférence, le volume défini par le volume des canaux internes de l'interface de sortie 110 entre l'entrée dans ceux-ci depuis les orifices d'alimentation 53 jusqu'aux orifices d'alimentation 116 est inférieur ou égal à 0,4ml.

Le boîtier du distributeur 11, dans sa configuration illustré à la figure 13, c'estàdire sans l'interface de sortie 110 décrite ci-dessus, présente l'avantage de pouvoir être accouplé à diverses formes d'autres interfaces de sortie, en fonction du maquillage que l'on souhaite réaliser et/ou de la zone à traiter.

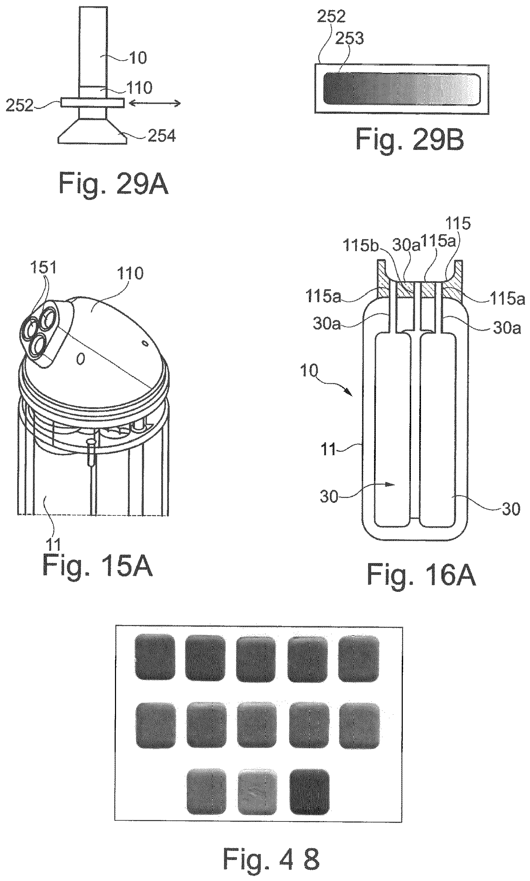

Ainsi, on a représenté sur la figure 15 une variante d'interface de sortie 110 comportant un embout de distribution 150, orienté généralement selon un axe Z qui s'étend obliquement par rapport à l'axe longitudinal Y du distributeur 11. Trois canaux internes communiquent respectivement avec les orifices de sortie 53 des différentes cartouches 30 et débouchent à l'extrémité de l'embout 150. L'interface de sortie 110 peut se monter à une extrémité du boîtier du distributeur 11, comme illustré sur la figure 15A.

Dans la variante de la figure 16, l'interface de sortie 110 comporte trois canules 160 qui communiquent respectivement avec les orifices de sortie 53. Ces canules 160 sont regroupées au centre de l'interface de sortie 110, ce qui permet le montage sur celles-ci par exemple d'un embout 170 d'application du produit sur les cils, tel qu'illustré à la figure 25, d'un embout poreux 180 pour l'application sur les lèvres, tel qu'illustré à la figure 27, ou d'un embout 190 floqué tel qu'une pointe feutre, comme illustré à la figure 28.

Dans le cas de l'embout 170 de la figure 25, celui-ci comporte par exemple, comme illustré, des stries 171 transversales, entre lesquelles débouchent des orifices d'alimentation en produit. Le mélange des différents produits de base peut avoir lieu à l'intérieur de l'embout 170, grâce à un mélangeur statique intégré, par exemple.

L'embout 180 comporte par exemple une partie en mousse à cellules ouvertes, ayant la forme des lèvres. Le mélange des produits de base peut se faire au sein d'un conduit interne de l'embout 180.

L'embout 190 peut comporter un organe d'application poreux 191, à l'extrémité d'une tige 192, se raccordant à une base 193 servant au montage sur le reste de l'interface de sortie de la figure 16.

On a illustré à la figure 16A, de façon schématique, la possibilité d'avoir comme interface de sortie 110 une coupelle 115 ayant des passages 115a pour des conduits de sortie 30a des cartouches, y compris lorsque ces conduits servent à l'entrainement enrotation de tiges filetées de déplacement des pistons. La longueur des conduits 30a est telle que ceux-ci débouchent dans le fond 115b de la coupelle ou à proximité de son fond, sans faire saillie dans celui-ci.

De préférence, la section interne des conduits 30a est faible, pour minimiser le volume mort.

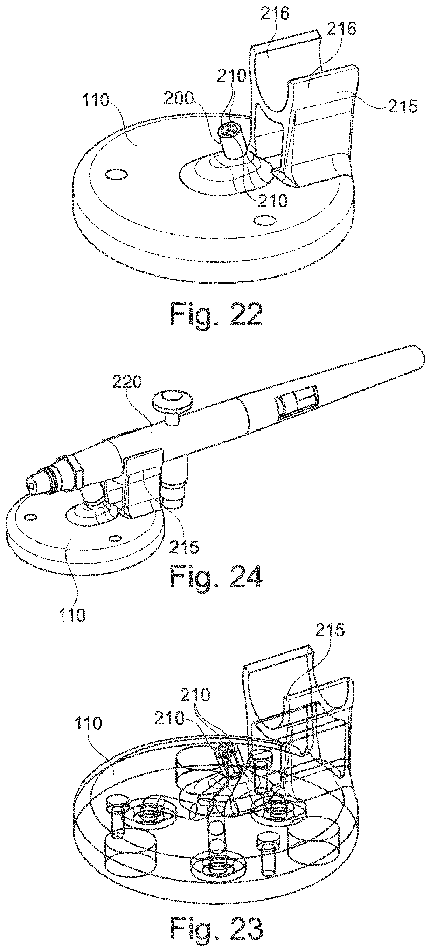

Dans l'exemple des figures 22 à 24, l'interface de sortie 110 comporte un embout 200 qui est orienté généralement obliquement par rapport à l'axe longitudinal Y du distributeur. Cet embout 200 est parcouru par des canaux internes 210, communiquant respectivement avec les orifices de sortie 53 des produits de base provenant des cartouches 30.

L'interface de sortie 110 comporte une partie de montage 215 qui permet la fixation sur l'interface de sortie 110 d'un aérographe 220, comme illustré à la figure 24.

L'embout 200 se fixe à la place du réservoir habituel de l'aérographe et les canaux 210 débouchent dans la tuyère de l'aérographe où ils sont soumis à la dépression créée par la vitesse du flux d'air d'entraînement.

Une pince est formée par deux montants 216, pour recevoir le corps du stylet de l'aérographe 220, et assurer son maintien par encliquetage.

De préférence, l'orientation de l'embout 200 est telle qu'elle permet à l'axe de pulvérisation d'être orienté sensiblement perpendiculairement à l'axe longitudinal du boîtier du distributeur 11. On peut alors se servir de celui-ci comme d'une poignée pour manipuler l'aérographe.

Les orifices de sortie 210 sont avantageusement très rapprochés, étant séparéspar de fines cloisons internes de l'embout 200.

De préférence, la section de chacun des orifices de sortie est inférieure ou égale à 3 mm² sur une longueur d'au moins 5 mm.

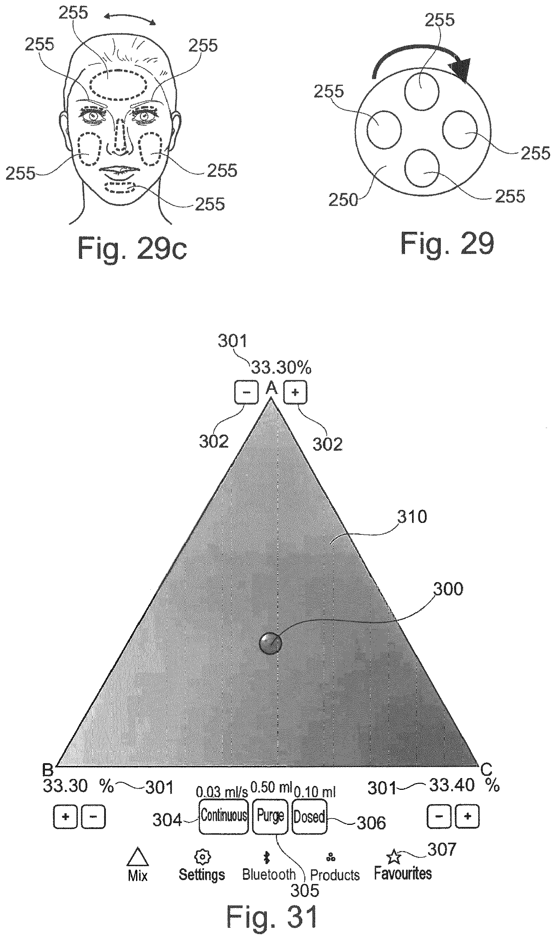

On peut encore munir le boîtier du distributeur 11 en partie supérieure d'un support mobile par rapport au boîtier, par exemple sous forme de tourelle 250, tel qu'illustré à la figure 29.

Cette tourelle 250 tourne par exemple autour d'un axe de rotation qui est confondu avec l'axe longitudinal Y du distributeur.

La tourelle 250 peut comporter plusieurs logements 255 pouvant accueillir chacun les produits délivrés par le distributeur 11 dans une position de remplissagr correspondante. Pour remplir successivement les différents logements, on fait effectuer à la tourelle 250 une rotation, par exemple d'un quart de tour, à chaque fois. La présence de plusieurs logements 255 peut permettre de distribuer des produits de formulations différentes, à partir des différents produits de base, de façon par exemple à faire varier la teinte des produits présents dans les différents logements 255.

On a illustré à la figure 29C un support ayant des logements disposés sur celuici sensiblement comme les différentes zones d'un visage ; chaque logement peut contenir un mélange dont la couleur est adaptée à la partie correspondante du visage. Ainsi, il est facile pour l'utilisateur de savoir où appliquer le mélange prélevé dans un logement donné.

On peut utiliser le distributeur 11 pour délivrer un mélange dont la formulation change au cours du temps et recueillir le mélange dans un contenant mobile par rapport au distributeur, de telle sorte que le mélange soit déposé en un emplacement du contenant qui varie dans le temps, afin de réaliser un dégradé.

Par exemple, comme illustré aux figures 29A et 29B, le système de distribution comporte une interface de sortie 110 comportant une partie fixe relativement au distributeur et une partie mobile 252 présentant un logement 253 pour recevoir le mélange.

Par exemple, le distributeur 11 est disposé dans ce cas avec les orifices de sortie des cartouches vers le bas, et équipé d'un mélangeur, de telle sorte que le mélange tombe sous l'effet de son poids dans le logement 253. Un moteur peut déplacer la partie mobile de l'interface de sortie relativement au distributeur, de façon synchronisée avec la variation des caractéristiques du mélange, de telle sorte que l'on obtienne un dégradé le long dulogement 253, comme illustré à la figure 29B.

Le système de distribution peut comporter un socle 254 qui maintient le distributeur tête en bas.

L'interface de sortie 110, notamment lorsqu'elle comporte une coupelle, peut comporter un mélangeur statique qui assure le mélange des produits de base.

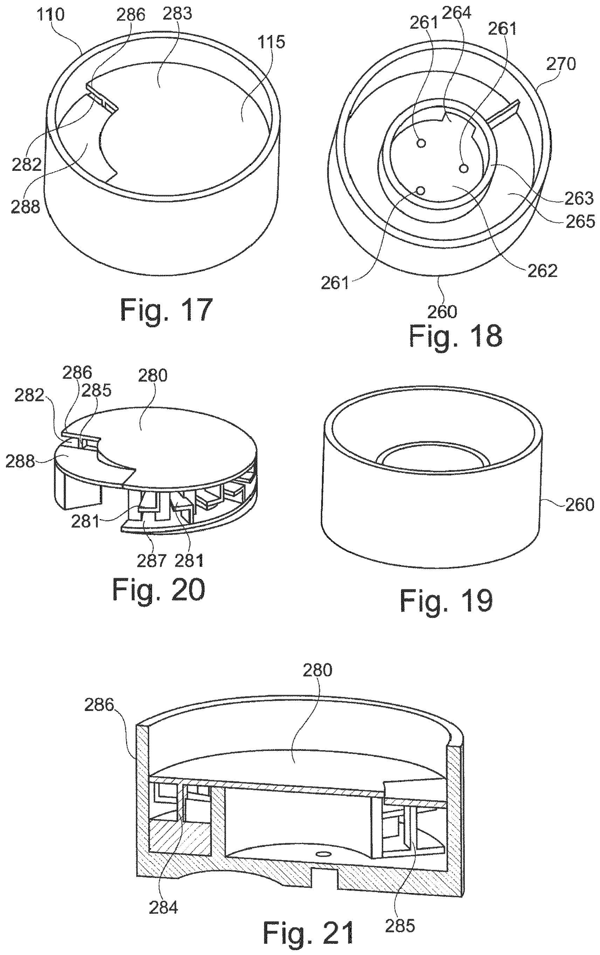

On a représenté sur les figures 17 à 21 une interface de sortie 110 comportant un tel mélangeur statique.

Cette interface de sortie 110 peut comporter un corps extérieur 260 qui se fixe sur le boîtier du distributeur 11 et qui présente un montant tubulaire extérieur 270.

Le corps 260 comporte des canaux 261 pour l'admission des différents produits de base. Ces canaux 261 débouchent dans une chambre centrale 262, délimitée par un montant tubulaire intérieur 263.

Ce montant 263 est traversé par une ouverture 264 qui débouche dans un espace annulaire 265, entre le montant intérieur 263 et le montant extérieur 270.

Un coeur de mélangeur statique 280, représenté isolément à la figure 20, est disposé dans cet espace 265.

La chambre centrale 262 peut communiquer avec une chambre périphérique comportant une série de cloisons formées par le coeur du mélangeur et qui agissent comme des déflecteurs pour le mélange, et créent un cisaillement de celui-ci.

La chambre périphérique peut comporter une cloison annulaire ajourée 284 définissant des ajours 285, dont l'un est visible sur la figure 21, par lesquels passe le mélange en circulant dans la chambre périphérique. Les chambres centrale et périphérique peuvent être fermées supérieurement par une paroi 286 qui définit le fond de la cavité recevant le mélange.

Le fond 287 de la chambre périphérique peut être de forme hélicoïdale et de hauteur diminuant au fur et à mesure que l'on progresse vers la sortie. Cette dernière peut déboucher en avant d'une rampe 288 de liaison entre le fond 287 de la chambre périphérique et la paroi supérieure 286 du mélangeur, cette rampe de liaison étant de préférence une portion d'hélice prolongeant l'hélice formée par la paroi de fond de la chambre périphérique.

De préférence la chambre périphérique comporte la cloison annulaire précitée et des cloisons radiales 281 qui forcent le mélange à circuler alternativement entre des régions supérieure et inférieure de la chambre périphérique et entre des régions radialement intérieure et extérieure, le mélange circulant par exemple d'une région supérieure et radialement extérieure à une région inférieure et radialement extérieure en passant à travers la cloison annulaire précitée.

Le corps 260 ferme radialement à l'extérieur la chambre périphérique.

Le corps extérieur 260 du mélangeur et le coeur 280 du mélangeur peuvent être chacun réalisés d'une seule pièce par moulage par injection.

Le produit gagne le coeur 280 du mélangeur statique par le passage 264, puiscircule entre les montants 263 et 270 sur pratiquement une circonférence complète,jusqu'à la sortie 282.

Les nombreuses chicanes imposées par les cloisons 281 provoquent un mélange intime des composants introduits dans l'interface de sortie 110. Le mélange obtenu peut être prélevé par l'utilisateur dans le logement 283, au-dessus du mélangeur statique.

Comme indiqué précédemment, le système de distribution 10 selon l'invention comporte de préférence une interface homme machine qui permet à l'utilisateur de piloterfacilement et de façon intuitive le distributeur 11. Cette interface peut appartenir à un système informatique 100 qui communique avec le distributeur 11.

On a représenté sur les figures 31 à 37 différents exemples d'interfaces tactiles pouvant permettre à l'utilisateur de choisir la couleur du mélange résultant de la distribution dosée des différents produits de base.

Cette interface peut présenter, comme illustré à la figure 31, une zone de sélection de la couleur, par exemple sous la forme d'un triangle dont les sommetscorrespondent aux couleurs de chacun des produits de base contenus dans les cartouches.

L'utilisateur peut déplacer un curseur 300, par exemple sous la forme d'une balle, relativement aux sommets A, B et C du triangle.

Plus il rapproche le curseur 300 de l'un des sommets, plus la fraction du produit de base correspondant est grande par rapport à la quantité totale des différents produits distribués.

La fraction de chaque produit par rapport à la quantité totale peut être indiquée en 301 par une valeur numérique sur l'interface.

L'interface peut permettre à l'utilisateur d'incrémenter ou de diminuer la quantité de chacun des produits, en agissant par exemple sur des boutons de commande 302, lesquels permettent un réglage précis de la quantité de chacun des produits de base.

La surface du triangle 310 peut avoir une couleur qui varie localement de façon à être représentative en chaque point de la couleur du mélange résultant de la pondération des différents produits de base dans des proportions correspondant aux coordonnées relatives en ce point.

L'interface peut comporter un bouton 305 permettant d'accéder à un menu spécifique de réglage du volume de produit qui est distribué pour purger le distributeur.

L'interface peut aussi avantageusement permettre un réglage du débit de produit grâce à des boutons 304 et 306 renvoyant vers un menu spécifique de réglage du débit.

Dans l'exemple considéré, l'interface offre le choix entre un mode de distribution continu, grâce au bouton 304, où les produits sont distribués tant que l'utilisateur appuie sur le bouton de commande 12.

La dose correspondante peut être transmise à l'interface et affichée.

Le bouton 306 permet de sélectionner un fonctionnement en mode dose, au cours duquel un appui même bref sur le bouton 12 déclenche la distribution d'une dose prédéfinie.

Pour faire varier le débit, le distributeur agit par exemple sur le rapport
cyclique de fonctionnement des moteurs.

L'interface peut être agencée pour permettre à l'utilisateur de programmer ou de mémoriser les réglages qui ont sa préférence, grâce à un menu 307 d'accès à des favoris.

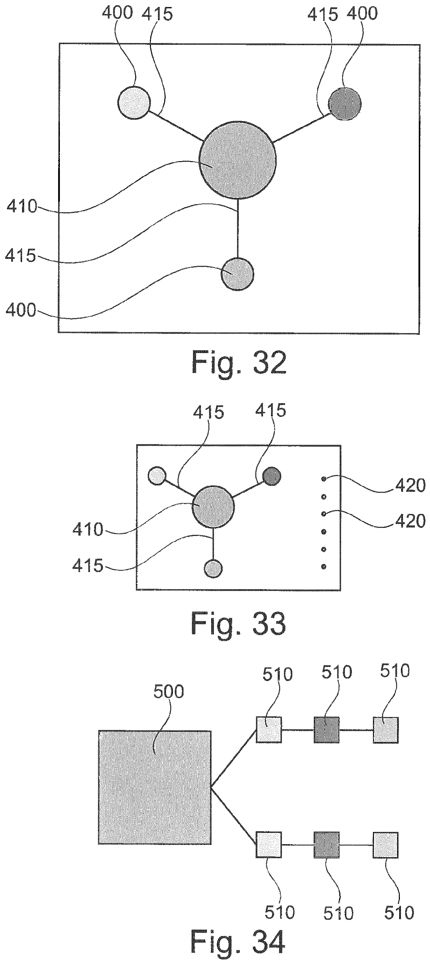

L'interface tactile illustrée à la figure 32 fait apparaître sur l'écran trois zones colorées 400, correspondant chacune à la couleur de l'un des produits de base contenus dans le distributeur 10, et une zone centrale 410 faisant apparaître la couleur du mélange résultant.

La quantité relative de chacun des produits de base peut être ajustée à l'aide de curseurs 415 qui se déplacent par exemple sur des lignes joignant chacune des zones 400 à la zone centrale 410.

Au cours de l'utilisation de l'interface, celle-ci peut mémoriser un réglage donné et faire apparaître sur l'écran un bouton 420 de la couleur du mélange. L'utilisateur peut ensuite, en appuyant simplement sur ce bouton 420, distribuer un mélange de la couleur correspondante.

Dans l'exemple de la figure 34, l'interface affiche dans une zone 500 une teintebdonnée, et propose à l'utilisateur, grâce à des boutons de commande 510 chacun de la couleur du produit de base correspondant, d'accroître ou de diminuer la proportion de ce produit de base dans le mélange final. La couleur de la zone 500 est recalculée en fonction des actions sur les boutons de commande 510.

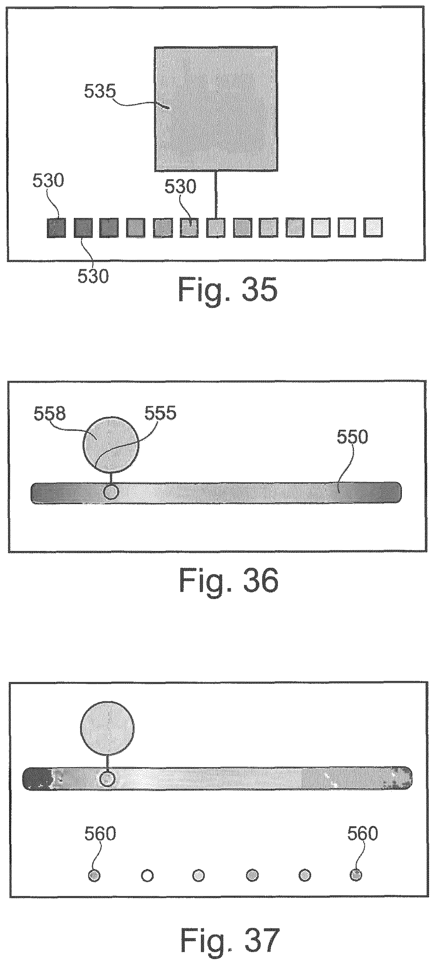

Dans la variante de la figure 35, l'interface fait apparaître un nuancier présentant plusieurs zones 530 correspondants chacune à une proportion particulière des différents produits de base.

L'utilisateur peut sélectionner l'une de ces zones, par exemple en appuyant dessus avec son doigt.

L'interface peut être agencée pour afficher à une échelle plus grande dans une zone 535 la couleur sélectionnée. La programmation du distributeur 11 pour distribuer cette couleur est par exemple déclenchée en appuyant sur la zone.

Dans l'exemple de la figure 36, l'utilisateur peut déplacer sur un nuancier continu 550 un curseur 555, qui fait apparaître dans une zone 558 la couleur sélectionnée.

L'utilisateur peut ensuite, en appuyant par exemple dans la zone 556, déclencher l'envoi au distributeur 11 des instructions nécessaires pour que celui-ci distribue un produit de la couleur sélectionnée.

On voit sur la figure 37 que l'interface peut mémoriser les différentes teintes sélectionnées, et les faire ensuite apparaître sur l'écran de façon à permettre à l'utilisateur, en appuyant sur des boutons correspondants 560, de sélectionner à nouveau très facilement une teinte précédemment choisie.

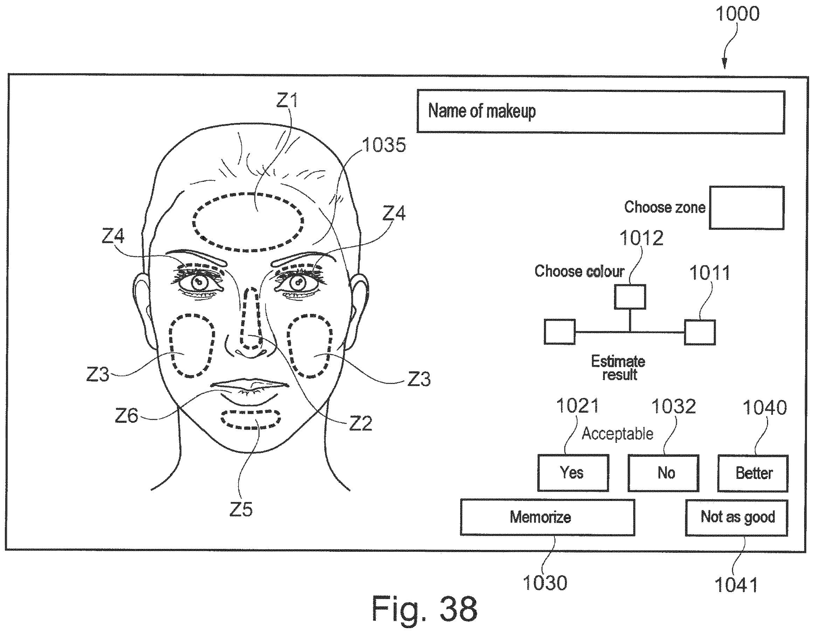

On a représenté à la figure 38 un exemple d'interface utilisateur 1000 d'un système de distribution comportant un distributeur, de préférence tel que décrit précédemment, et un système informatique 100 auquel appartient l'interface.

Le système informatique comporte ici par exemple un appareil tel qu'un ordinateur portable, une tablette ou un téléphone intelligent, fonctionnant de façon autonome ou connecté à un serveur distant.

Dans l'exemple considéré, l'interface 1000 est définie par l'écran tactile d'un tel appareil. Dans une variante non illustrée, le distributeur intègre un écran tactile ou tout autre type d'interface homme machine, et peut être utilisé sans connexion à un autre appareil.

L'appareil exécute une application, qui a par exemple été téléchargée au préalable, et qui affiche sur l'écran un visage 1035 et une série de boutons permettant à l'utilisateur d'entrer des informations.

Le visage peut comporter plusieurs zones Z1 à Z6 sélectionnables tactilement, par exemple le front, le nez, les joues, les paupières, le menton, et les lèvres.

Les boutons présents sur l'écran peuvent permettre par exemple d'entrer le nom du maquillage ou de l'utilisateur, d'afficher la zone sélectionnée, de choisir la couleur, et d'informer le système informatique du résultat acceptable ou non du test effectué, voire comme illustré de donner une information concernant l'évaluation du résultat relativement à un test effectué précédemment, à savoir par exemple mieux ou « moins bien ». L' cran peut également afficher un bouton permettant de mémoriser le choix d'une couleur et d'une zone, après avoir effectué un essai avec cette couleur sur la zone en question.

Le choix de la couleur s'effectue par exemple avec une échelle de couleurs similaire à celle décrite en référence à la figure 36.

Le système informatique est agencé pour mémoriser les données sous forme de table de correspondance par exemple, de manière à associer à une zone du visage des paramètres permettant de reproduire le mélange distribué lors du test. Ces paramètres comportent par exemple les teneurs relatives de chacun des produits de base du distributeur dans le mélange, la quantité Q distribuée, ainsi que des données additionnelles telles que par exemple le nom de la zone, la date de distribution du mélange et/ou tout autre identifiant du mélange, les identifiants des produits de base, la période de l'année, notamment la saison, l'âge de l'utilisateur, son sexe, son nom ou prénom, le nom d'un évènement associé au maquillage, par exemple un anniversaire, entre autres données, la quantité de produit adéquate pour la zone. Les données annexes peuvent permettre à l'utilisateur de reproduire plus facilement un maquillage qu'il a considéré comme adéquat pour une période de l'année, ou rappelant un évènement de la vie, ou pour donner un effet de rajeunissement.

Ces données peuvent être mémorisées dans le système informatique 100, par exemple dans l'appareil précité et/ou sur un serveur distant avec lequel l'appareil communique, ou encore dans une mémoire électronique intégrée au distributeur 11.

Ainsi, selon l'invention, l'utilisateur peut amener le distributeur à délivrer une première matière colorée, à l'appliquer sur une première zone du visage, puis la juger adéquate ou non. Si le résultat est satisfaisant, l'utilisateur peut l'enregistrer en l'indexant à la zone ; si le résultat est non satisfaisant, l'utilisateur peut commander une nouvelle couleur pour refaire les opérations ci-dessus.

Le système informatique peut être utilisé dans ce contexte de diverses façons.

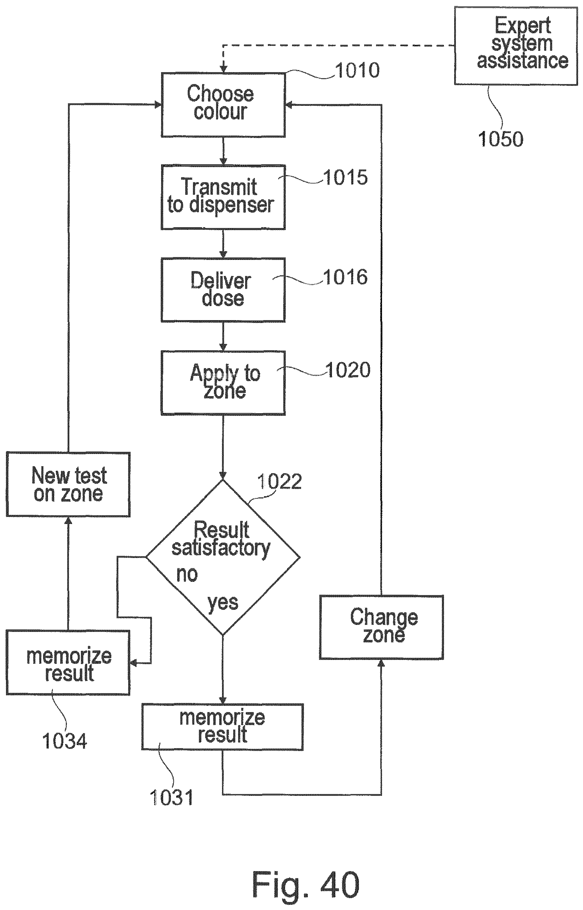

Par exemple, comme illustré à la figure 40, l'utilisateur choisi à une étape 1010 une couleur à essayer, en utilisant par exemple l'échelle de couleurs 1011 apparaissant sur l'écran, en déplaçant le bouton de réglage 1012.

Ensuite, le choix de la couleur est transmis au distributeur 11, à l'étape 1015.

Par exemple, l'appareil transmet les quantités à distribuer de chacun des produits de base, et le circuit électronique 81 se charge de commander les moteurs en conséquence.

A l'étape 1016, l'utilisateur appuie sur le bouton de commande 12 du distributeur 11, ce qui provoque par exemple la distribution d'une dose du mélange, de la couleur sélectionnée par l'utilisateur.

Le mélange est par exemple distribué dans la coupelle 115, puis prélevé et appliqué par l'utilisateur sur les joues ou toute autre zone renseignée sur l'interface, à l'étape 1020.

Dans des variantes, le produit est appliqué à l'aide d'un aérographe ou par tout autre moyen tel que décrit précédemment.

L'utilisateur renseigne ensuite à l'étape 1022 le système informatique à l'aide des boutons 1021, sur le résultat.

Si l'utilisateur indique que le résultat est satisfaisant, le système lui propose par exemple de valider à l'aide d'un bouton les paramètres de l'essai afin de les mémoriser à l'étape 1031.

Si le résultat n'est pas jugé satisfaisant par l'utilisateur et signalé comme tel à l'aide du bouton 1032, le résultat peut néanmoins être automatiquement sauvegardé à l'étape 1034.

On peut ainsi indexer à chaque zone non seulement la ou les couleurs adéquates mais aussi la ou les couleurs qui ne vont pas du tout à cette zone.

L'utilisateur peut alors effectuer un nouvel essai sur la même zone en revenant à l'étape 1010.

Si l'utilisateur était satisfait du résultat, il peut également souhaiter effectuer un nouvel essai, par exemple sur une zone différente du visage.

Le cas échéant, si l'utilisateur n'est pas satisfait, l'interface peut lui proposer d'indiquer si le résultat est estimé mieux ou moins bien que l'essai précédent, à l'aide de boutons correspondants 1040 et 1041.

Dans ce cas, le système informatique peut être agencé pour déterminer si au vu des renseignements entrés par l'utilisateur une proposition peut être automatiquement effectuée quant au choix de la nouvelle couleur à tester.

Le cas échéant, un questionnaire peut s'afficher pour aider le système informatique à proposer une couleur au vu des essais effectués et de l'évaluation qui en a été faite par l'utilisateur ou par un professionnel qui l'assiste.

Par exemple, si la couleur est jugée comme « non adéquate », le système peut recevoir de l'utilisateur une information additionnelle, par exemple « trop clair », qui l'aidera à proposer une nouvelle couleur plus en adéquation avec ce qu'attend l'utilisateur.

Il peut être intéressant que le système informatique puisse recevoir une information de comparaison du résultat par rapports aux essais précédents, par exemple « c'est mieux » ou « c'est moins bien » et que de là, le système soit capable d'en déduire la nouvelle couleur à proposer.

Une autre option est que le système informatique puisse recevoir une information de comparaison par rapport à une cible, par exemple « c'est presqu'idéal » et que de là, le système soit capable d'ajuster automatiquement ses modifications de couleur.

En l'occurrence, s'il reçoit l'information selon laquelle le résultat souhaité est presque atteint, le système peut adopter de petits niveaux de modification de la couleur et revoir l'échelle colorimétrique de réglage en conséquence.

Dans le cas où le système de distribution propose de lui-même les mélanges colorés à tester, il peut se fonder sur des scénarios d'essais préprogrammés et modifier le suivi du scénario selon les réussites ou échecs de l'évaluation. Ainsi, par exemple, si dès la troisième application de produit, il reçoit une information selon laquelle la couleur est presqu'idéale pour l'utilisateur, le système de distribution peut sortir du programme et se laisser guider ensuite par les instructions de l'opérateur.

D'une façon générale, l'utilisateur peut être aidé par un système expert dans le choix des couleurs à essayer.

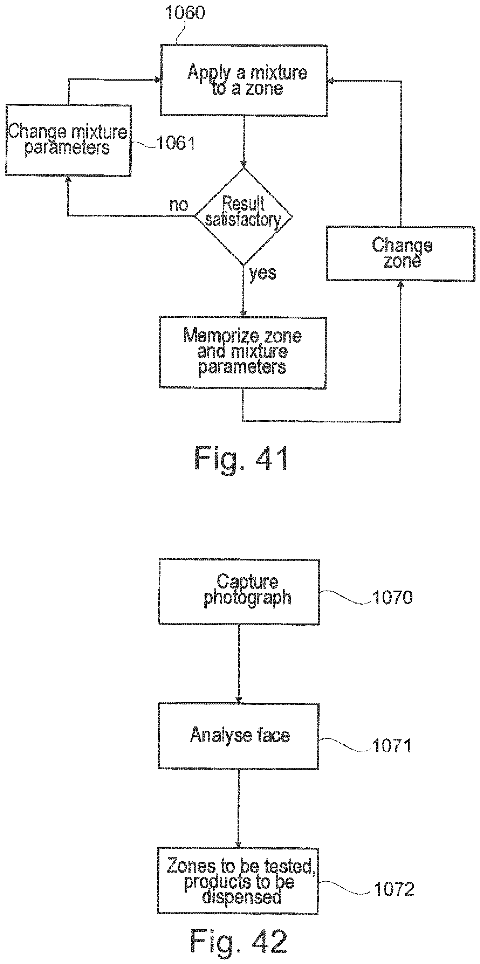

Ce système expert est par exemple un programme exécuté sur l'appareil avec lequel le distributeur communique ou sur le distributeur lui-même, qui se fonde sur des réponses à un questionnaire et/ou sur des mesures, par exemple de la couleur de la peau, effectuées par un capteur spécifique ou une caméra. L'utilisateur peut ainsi se faire aider par une évaluation instrumentée, par exemple un capteur de couleur.Le système expert peut encore être implémenté sur un serveur distant avec lequel l'appareil ou le distributeur échange des informations. L'opérateur peut encore envoyer une image de son visage à un spécialiste qui peut préprogrammer le choix des couleurs de départ. Dans un autre exemple de mise en oeuvre, l'utilisateur présente au système informatique une photo de son visage, et le système informatique est agencé pour l'analyser et créer un programme définissant les zones à tester et les premiers produits à délivrer, à la fois en couleur et quantité. Par exemple, le système informatique peut être agencé pour sélectionner automatiquement des couleurs de maquillage à proposer à l'utilisateur en effectuant l'acquisition d'une photographie à l'étape 1070, comme illustré à la figure 42. Par exemple, l'appareil qui communique avec le distributeur 11 est équipé d'une caméra, et l'utilisateur prend une photographie de son visage. L'image est ensuite analysée à l'étape 1071, et des couleurs sont proposées pour chaque zone du visage à l'étape 1072, selon par exemple des règles prédéfinies d'accord des couleurs.

Le système de distribution peut être orienté par l'utilisateur pour décider la couleur mais aussi la quantité de produit à délivrer. Par exemple, l'utilisateur peut indiquer « nez » ou « tache » et le système de distribution est agencé pour adapter la dose distribuée selon une cartographie en mémoire des doses à distribuer en fonction des zones à traiter.

Le système informatique peut guider l'utilisateur dans le choix des couleurs du mélange à essayer, afin de limiter le nombre d'essais nécessaires jusqu'à l'obtention d'un résultat plaisant pour l'utilisateur.

Il est ainsi possible, comme illustré à la figure 41, qu'après l'application d'un mélange délivré par le distributeur sur une zone donnée du visage à l'étape 1060, le système informatique demande à l'utilisateur si le résultat est satisfaisant ou non, et effectue de lui-même, si le résultat est considéré comme étant non satisfaisant, un changement 1061 des paramètres du distributeur de façon à modifier le mélange distribué.

L'utilisateur n'a alors qu'à effectuer un nouvel essai avec le mélange modifié.

Lorsqu'un mélange est signalé comme étant satisfaisant, le système informatique peut mémoriser les paramètres correspondants afin de permettre de recréerultérieurement le mélange.

Le système peut alors recommencer les étapes précédentes pour une nouvelle zone d'application.

Lors des essais successifs, l'opérateur n'a pas besoin de traiter tout le visage. Il peut par exemple choisir entre 3 et 8 zones de petites surfaces, par exemple 5 zones. Le système de distribution est alors avantageusement prévu pour interpoler et/ou extrapoler les données concernant les couleurs considérées comme adéquates, afin de calculer les couleurs qui devraient être considérées comme adéquates sur des zones sur lesquelles l'exercice n'a pas été fait.

A la fin de l'apprentissage, le système peut généner une visualisation des couleurs adéquates sur les différentes zones, testées ou calculées.

Le système de distribution peut être agencé pour signaler si certaines couleurs apparaissent aberrantes, se fiant en cela à une comparaison par rapport à des cartographies types en mémoire. Ainsi, il peut proposer à l'utilisateur de refaire tout ou partie de l'exercice de cartographie.

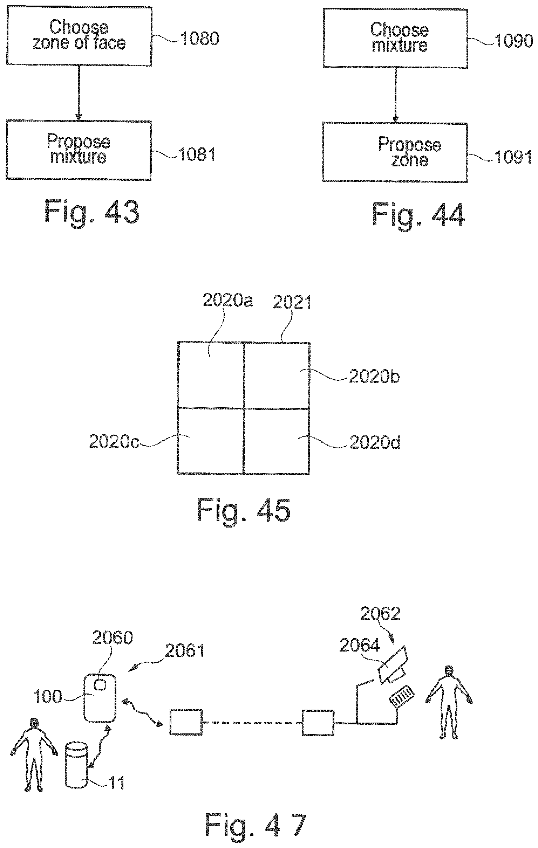

Une fois l'apprentissage du système informatique effectué, c'est-à-dire que des couleurs de mélanges ont été identifiées comme convenant à l'utilisateur pour maquiller certaines zones, l'utilisateur qui souhaite se maquiller n'a qu'à rappeler la zone à maquiller, à l'étape 1080 de la figure 43, et le système peut proposer automatiquement à l'utilisateur à l'étape 1081 une couleur de mélange adéquate.

Dans la variante illustrée à la figure 44, l'utilisateur sélectionne une couleur à l'étape 1090 et le système informatique propose à l'étape 1091 une zone où appliquer un mélange de cette couleur, sur la base d'informations précédemment collectées sur la base des tests effectués.

La zone proposée est par exemple celle où une couleur identique ou proche a déjà été appliquée et le résultat jugé acceptable par l'utilisateur.

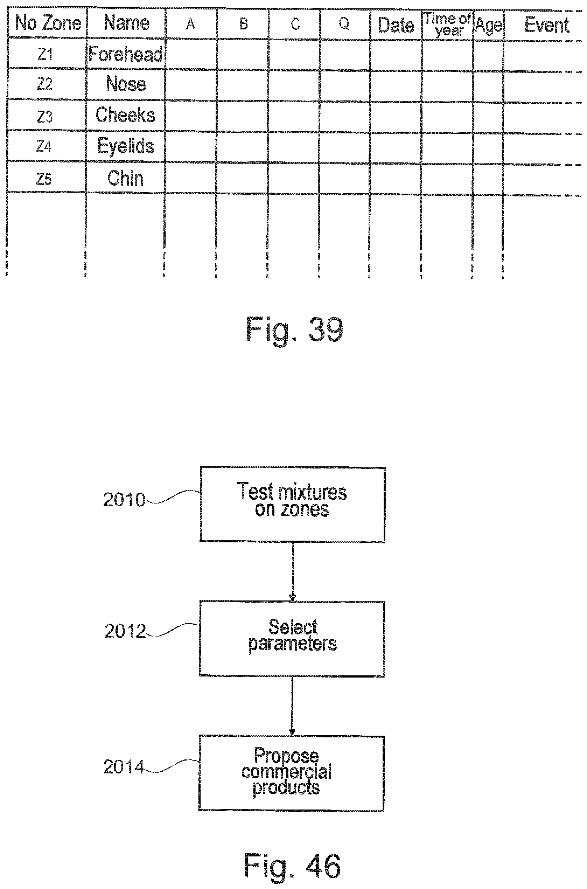

La figure 46 illustre un exemple de mise en oeuvre de l'invention où après avoir effectué des tests sur différentes zones à l'étape 2010, l'utilisateur signale au système le ou les mélanges qu'il estime procurer le meilleur résultat, ce qui permet au système de connaître les paramètres correspondants à l'étape 2012. Ensuite, à l'étape 2014, le système peut proposer à l'utilisateur des références de produits commerciaux ayant les mêmes propriétés ou des propriétés proches.

Dans une variante, le système adresse à un centre de fabrication distant les paramètres permettant de produire une composition de même formulation ou ayant les mêmes propriétés que celle du mélange que l'utilisateur a testé et trouvé satisfaisant.

La figure 45 illustre la possibilité de délivrer à l'aide du distributeur plusieurs doses 2020a à 2020d de mélanges différents, de façon juxtaposée sur un support 2021, de façon à permettre leur application sur des régions distinctes voisines d'une même zone.

L'utilisateur peut d'un seul coup appliquer une série de couleurs afin de cerner rapidement la couleur adéquate. Les matières colorées présentes sur le support 2021 peuvent avoir été choisies par l'opérateur lui-même ou proposées par le système de distribution.

Le support 2021 est par exemple mobile par rapport au boîtier du distributeur et déplacé séquentiellement pour déposer les mélanges correspondants dans les différenteszones 2020a à 2020d, étant par exemple similaire aux supports décrits en référence aux figures 29 ou 29A. L'utilisateur peut alors facilement comparer les résultats entre les différentes régions, et signaler au système le mélange produisant le meilleur effet.

On a illustré à la figure 47 un système qui permet d'aider l'utilisateur à se maquiller, notamment à choisir les bons coloris.

Ce système permet l'établissement d'une liaison vidéo, par exemple via internet, entre une caméra 2060 d'un premier site 2061 et un deuxième site 2062.

La caméra 2060 est par exemple intégrée à une tablette ou un téléphone intelligent qui constitue le système informatique 100.

On permet au deuxième site 2062 de piloter directement ou indirectement le distributeur 11 présent sur le premier site 2061.

Ainsi, la personne présente au premier site peut appliquer le mélange distribué et envoyer vers le deuxième site 2062 une image correspondante, pour recevoir en retour une information relative au résultat de maquillage.

Le deuxième site 2062 peut comporter un écran de visualisation 2064 qui permet à un conseiller présent devant cet écran de voir le résultat du maquillage avec le mélange distribué par le distributeur et de conseiller la personne qui s'est maquillée. Ce conseiller peut agir en retour sur le distributeur 11 pour modifier la couleur du mélange et l'adapter au mieux au visage de la personne présente sur le premier site. Le protocole d'échange des données entre les deux sites permet ainsi l'envoi d'instructions de commande au distributeur 11, soit directement, soit par l'intermédiaire du systèmeinformatique 100 présent sur le premier site. Ainsi, la personne présente sur le deuxième site gouverne le mélange délivré par le distributeur 11. La première personne peut se maquiller sous les yeux de la seconde. La seconde personne voit sur son écran le résultat de l'essai, et ainsi peut corriger le mélange qu'elle va commander à distance, jusqu'à obtenir le maquillage idéal.

De préférence, la liaison vidéo entre les deux sites est une liaison duplex, de sorte que l'utilisateur présent sur le premier site peut voir sur l'écran du système informatique l'image du conseiller. Ce dernier peut adresser à l'utilisateur présent sur le premier site un tutoriel, le cas échéant.

La mémorisation des paramètres de réglage du distributeur 11, une fois qu'un mélange donné est estimé satisfaisant, peut être commandée depuis le deuxième site.

Avantageuseusement, le système de distribution 10 est agencé pour pouvoir modifier de façon esthétique l'ensemble des couleurs pour chaque zone si la personne désire changer la couleur de son visage. Le système de distribution peut être agencé de telle sorte qu'il suffit à l'utilisateur de modifier une seule couleur dans une zone pour qu'il modifie toutes les autres. Le système de distribution peut utiliser pour cela des translations, par exemple en relevant la saturation des couleurs ou en décalant la teinte.

Le système de distribution peut être agencé pour recevoir la cartographie de quelqu'un d'autre, réel ou virtuel. Il peut aussi combiner la cartographie de la personne avec la cartogaphie d'une autre, pour sublimer le maquillage sans perdre les caractéristiques propres.

L'interface peut servir à définir des programmes de maquillage où l'on définit l'ordre des zones à maquiller ou l'ordre des couleurs à proposer.

### Exemples

On réalise un distributeur 11 tel que celui illustré à la figure 3. Le distributeur est agencé pour communiquer avec une tablette 100 telle qu'un Ipad. Ce système informatique exécute une application dénommée « µMix » développée dans l'environnement spécifique d'Apple (XCode 4 et Simulateur iOS) en langage Objective C.

Elle utilise des frameworks de base Foundation, UIKit et CoreGraphics, fournissant les outils de manipulation des structures de données, des outils de calcul et les fonctionnalités liées à l'interface graphique utilisateur.

L'application utilise également le framework CoreBluetooth fournissant un accès aux périphériques Bluetoth 4 Low Energy avec les tâches principales suivantes :
- Recherche des périphériques Bluetooth 4.0 Low Energy,
- Connexion/déconnexion et gestion des paramètres de connexion,• Communication en mode lecture et/ou écriture basée sur l'architecture GATT (Generic Attribute Profile).

L'application propose les fonctionnalités suivantes :
- Définition des fractions de produits de base,
- Choix du mode de fonctionnement lors de l'appui sur le bouton de commande12, à savoir continu, purge ou dose,
- Affichage d'un triangle de fraction volumique tel qu'illustré la figure 30 avec gestion de la fraction volumique par touché tactile sur le triangle ou par les boutons +/associés à chaque produit,
- Connexion/déconnexion Bluetooth et transfert en temps réel des consignes au distributeur,
- Réglages des débits en mode continu, et de la quantité en mode dose,
- Calcul, affichage et transfert au distributeur des fractions volumiques des produits en temps réel, en fonction de la consigne, avec la somme des fractions toujours égale à 100%,
- Récupération et affichage des couples des trois moteurs en temps réel, et
- Sauvegarde des paramètres principaux dans un fichier de configuration.

Le mode continu est un mode de distribution où le mélange des trois produits de base est distribué tant que l'utilisateur appuie sur le bouton de distribution 12. Le produit est délivré avec un débit dont une estimation est affichée au-dessus du bouton 304 « Continu ». Le choix du débit se fait dans un menu « Réglages ».

Le mode « dose » est un mode de distribution du mélange par dose, où la dose est délivrée suite à une impulsion de l'utilisateur sur le bouton de distribution 12. Une impulsion suffit et l'utilisateur peut ensuite relâcher le bouton. La dose globale de produit distribuée est celle indiquée au-dessus du bouton 306 « Dose », 0.1 ml par exemple. Ce volume peut être modifié dans le menu « Réglages ».

Le mode « purge » est un mode de distribution où une dose de mélange à fractions volumiques égales (33%) est délivrée dès que l'utilisateur a appuyé sur le bouton de distribution 12, comme dans le mode « Dose ». Une impulsion suffit et l'utilisateur peut ensuite relâcher le bouton. Quand la dose a été entièrement distribuée, on peut relâcher le bouton. Si l'on relâche avant la fin, la distribution s'arrête, même si le volume spécifié n'est pas atteint. La dose globale de produit distribuée est celle indiquée au-dessus d'un bouton 305 « Purge », 3 ml par exemple. Ce volume peut être modifié dans le menu Réglages.

L'utilisateur détermine la couleur voulue avec l'application qui s'éxécute sur la tablette et qui calcule les fractions des différents produits. La tablette communique cette valeur au distributeur par liaison Bluetooth.

L'électronique intégrée au distributeur 11 récupère l'information et ajuste automatiquement les débits des trois cartouches de façon à obtenir un mélange de la couleur souhaitée.

Quand l'utilisateur veut utiliser le produit, il appuie sur le bouton 12 du distributeur pour faire sortir le produit. Il presse aussi longtemps qu'il veut du produit, en mode « continu ». En mode « dose », l'utilisateur presse une fois le bouton 12 et la dose prédéfinie est délivrée.

La distribution peut se faire en continu, c'est-à-dire les moteurs fonctionnent en continu, tout le volume étant distribué d'une traite, ou de façon itérative, les moteurs fonctionnant alors de façon impulsionnelle ; dans ce cas, l'intervalle de temps entre deux impulsions permet de faire varier le débit. De petits volumes sont délivrés les uns après les autres en plusieurs étapes.

Les impulsions peuvent être séparées par exemple par des intervalles de 50 ms, 100 ms ou 200 ms. La durée d'une impulsion pendant laquelle le moteur tourne va parexemple de 50 à 150 ms.

La page principale de l'application « µMix » comporte dans cet exemple les éléments suivants, comme visible notamment sur la figure 31 :
- Barre de statut en haut de l'écran : indique l'état de la connexion Bluetooth ou µMix s'il n'y a pas de connexion Bluetooth ;
- Barre d'onglets en bas de l'écran : permet de sélectionner la page active :page principale, Réglages, Bluetooth, Produits et Favoris ;
- Bouton Continu 304 pour sélectionner le mode de distribution de produits en continu ;
- Bouton Purge 305 pour sélectionner le mode Purge ;
- Bouton Dose 306 pour sélectionner le mode de distribution par dose avec le volume de dose associé au bouton Dose ;
- Balle bleue 300 que l'utilisateur peut déplacer dans le triangle volumique en la faisant glisser ou avec un double tapotement ;
- Boutons « - » 302 pour chaque produit A, B et C : diminue la fraction de produit sélectionné en se déplaçant le long de la droite reliant le point au sommet du produit sélectionné ;
- Boutons « + » 302 pour chaque produit A, B et C : augmente la fraction de produit sélectionné en se déplaçant le long de la droite reliant le point au sommet du produit sélectionné ;
- Fraction volumique en pourcentage de chaque produit : modifiable par l'utilisateur et mise à jour en temps réel selon la consigne des boutons 302 + et - et de la position de la balle 300.

Lors de la modification des fractions volumiques par déplacement de la balle ou par les boutons + et -, les valeurs des fractions volumiques des produits A, B et C sont mises à jour automatiquement. Lorsque les fractions volumiques sont modifiées avec les boutons + et -, la balle 300 est déplacée automatiquement à la position correspondante sur le triangle.

Au démarrage de l'application exécutée sur la tablette, celle-ci se connecte automatiquement au distributeur 11 s'il est détecté. Lorsque le distributeur s'éteint ou que la connexion Bluetooth est coupée, la tablette se déconnecte. Quand l'utilisateur agit sur les curseurs de réglage des proportions des produits A et B, les valeurs sont transmises en temps réel au distributeur 11.

La page Réglage de l'application comporte les éléments suivants :
- Barre de statut en haut de l'écran : indique l'état de la connexion Bluetooth ou µMix s'il n'y a pas de connexion Bluetooth ;
- Barre d'onglets en bas de l'écran : permet de sélectionner la page active :
   page principale, Réglages, Bluetooth ou Info ;
- Partie « Volumes » avec un champ de texte à remplir par l'utilisateur pour définir le volume de la dose, en ml (2 ml par exemple) et un champ pour le volume de la purge, en ml (3 ml par exemple). Les doses minimales sont dans cet exemple de 0.023 ml et les doses maximales 9.90 ml (3 x 3.3 ml) ;
- Partie « Débit » avec la sélection du débit : rapide (» 0.03 ml/s), moyen (» 0.02 ml/s) ou lent (» 0.01 ml/s) ;
- Partie « Dose » avec le choix du mélange itératif, pour distribuer un mélange de produits avec des petits volumes délivrés les uns après les autres en plusieurs étapes ;

Dans le cas contraire, le volume total de chaque produit est distribué d'une traite ;
- Partie « Image Triangle » pour choisir l'image du triangle qui sera affichée sur la page principale afin de pouvoir afficher un triangle avec les couleurs délivrées par le distributeur 11. En utilisant un bouton « Choix image » de la page « Réglages » on accède à un album.

La page « Produits » de l'application comporte dans l'exemple considéré les éléments suivants :
- Choix de la valeur de chaque produit en unités de pas codeurs, de 0 à 1414.
   Chaque unité correspond à un volume de produit délivré de 2.33 µl, qui est la plus petite quantité que le distributeur dans cet exemple peut délivrer ; lorsque cette feuille est affichée, ce sont les valeurs de produits de cette feuille qui sont transmises en temps réel au distributeur. Dès que la feuille n'est plus affichée, les valeurs envoyées au distributeur sont celles de la feuille principale avec le triangle ;
- Affichage des couples moteur A, B et C en temps réel avec rafraîchissement toutes les 45 valeurs.

Le mode de délivrance des produits est le mode dose directe ou itératif, selon l'option choisie dans la feuille Réglage.

La page « Favoris » permet de sauvegarder des configurations en fichier. Elle donne accès dans l'exemple considéré à 10 fichiers, à savoir « Configuration 1 » à « Configuration 10 » en plus du fichier par défaut. Ces fichiers enregistrent par exemple les paramètres suivants
- fractions des produits A, B et C
- volume de Purge
- volume de Dose,
- débit rapide, moyen ou lent,
- mode Dose, Purge ou Continu,
- distribution continue ou itérative.

### Exemple 1 (premier aspect de l'invention)

On réalise plusieurs produits de base avec un pigment dit de haute densité et un pigment à faible densité.

Les produits de base « A » « B » et « C » sont destinés à des cartouches respectives. Certains produits de base présentent un fort épaississement (A1, B1, C1), d'autres non (A2, B2, C2).

Les proportions sont massiques.

| Formule | Oxychlorure de bismuth (Kolortec co) 7,7 g/cm3 | Aérogel de silice (DOW CORNING VM-2260 AEROGEL BEADS) Densité=0,09 g/cm3 | Jaguar HP 60 (Solvay Novecare) | Red40 (Emerald Performance material) | glycérine | eau |
|---|---|---|---|---|---|---|
| A1 | 5% | | 2% | | 6% | Qs 100% |
| A2 | 5% | | 0,2% | | 6% | Qs 100% |
| B1 | | 0,5% | 2% | | 6% | Qs 100% |
| B2 | | 0,5% | 0,2% | | 6% | Qs 100% |
| C1 | | | 2% | 0,05% | 6% | Qs 100% |
| C2 | | | 0,2% | 0,05% | 6% | Qs 100% |
| | | | | | | |

Les densités des formules sont mesurées (mesurée à 1 atm et à 25°C avec un viscosimètre rotatif CONTRAVES TV équipé d'un mobile MS-r4 à la fréquence de 60 Hz, après 10 minutes de rotation du mobile)
A1 : 2,6 Pa.s
A2 : 0,55 Pa.s
B1 :2,4 Pa.s
B2 : 0,4 Pa.s
C1 :2,6 Pa.s
C2 : 0,6 Pa.s

### Test 1(selon invention)

Le système a été testé avec :
- A1,
- B1,
- C1

On définit grâce au système de distribution un mélange avec les proportions volumiques suivantes dans le mélange final :25%, B :25%, C :50%
On utilise le système à t0 pour délivrer 200 mg de produit. On étale alors le mélange.

On fait de même pendant plusieurs jours (20 jours).

En comparant les résultats, on s'aperçoit alors qu'ils sont constants.

### Test 2 (hors invention)

Le système a été testé avec :
- A2,
- B2,
- C1

Comme dans le cas du test 1, on distribue un mélange avec les proportions volumiques suivantes A :25%, B :25%, C :50%

On fait le même test que dans le test 1.

En comparant les résultats, on s'aperçoit alors que les couleurs obtenues varient d'un jour à l'autre.

### Test 3

Le système a été testé avec :
- A1,
- B2,
- C1

Comme dans le cas du test 1, on réalise un mélange avec les proportions volumiques A :25%, B :25%, C :50%
On fait le même test que dans le test 1.

En comparant les résultats, on s'aperçoit alors que les couleurs obtenues varient d'un jour à l'autre mais moins que dans le test 2.

### Test 4

Le système a été testé avec :
- A1,
- B1,
- C2

Comme dans le cas du test 1, on réalise un mélange A :25%, B :25%, C :50%

On fait le même test que dans le test 1.

En comparant les résultats, on s'aperçoit alors que les couleurs obtenues restent constantes. Cette configuration est préférée au cas du test 1 car plus facile à appliquer.

### Exemple 2 (premier aspect de l'invention)

On réalise les mêmes produits de base mais en remplaçant la glycérine et une partie de l'eau par de l'éthanol dans la formule sans particules denses.

| formule | Oxychlorure de bismuth (Kolortec co) 7,7 g/cm3 | Aérogel de silice (DOW CORNING VM-2260 AEROGEL BEADS) Densité=0,09 g/cm3 | Jaguar HP 60 (Solvay Novecare) | Ethanol | Glycérine | eau |
|---|---|---|---|---|---|---|
| A1 | 5% | | 2% | | 16% | Qs 100% |
| | | | | | | |
| B1 | | 0,5% | 2% | | 16% | Qs 100% |
| | | | | | | |
| C1 | | | 1% | 40% | | Qs 100% |
| | | | | | | |
| | | | | | | |

### Test 1

Le système a été testé avec :
- A1,
- B1,
- C1

On définit grâce au système un mélange A :40%, B :40%, C :20%
On utilise le système à t0 pour délivrer 200 mg de produit. On étale alors le mélange.

On fait de même pendant plusieurs jours (20 jours).

En comparant les résultats, on s'aperçoit alors qu'ils sont constants.

Malgré la forte rhéologie des produits de base des compartiments A et B, on obtient un mélange facile à appliquer.

### B) Système de distribution d'un produit, comportant un distributeur recevant au moins deux cartouches ayant chacune un réservoir contenant un produit de base, un premier produit de base comportant un épaississant, un deuxième produit de base comportant un agent de modulation de la viscosité de l'agent épaississant

La précision de la couleur obtenue est un facteur très important pour obtenir un résultat de qualité. Mais il faut aussi, pour obtenir les plus beaux résultats que le produit ait la meilleure applicabilité possible.

C'est particulièrement vrai dans le cas du masquage des zones à imperfections qui nécessite une application locale pour éviter le réflexe de l'étalement.

Plusieurs autres points de vue sont à considérer.

Verticalité/Horizontalité : Par exemple, sur les zones verticales, telles que le front, les produits risquent de couler (par rapport aux zones horizontales que telles que les cernes).

Grain de peau : Les zones à grain de peau « ouverts » (pores dilatés, ridules) conduit à des problèmes d'applicabilité différentes des zones plus lisses, notamment pour éviter le « marquage » des défauts.

Couvrance : On sait que certaines zones colorées nécessitent des épaisseurs importantes. Il faut donc que l'applicabilité le permette. Idem dans le sens opposé lorsqu'on une couvrance faible pour laisser au visage un aspect naturel, avec la peau visible en transparence.

Bicouche : Dans certains cas, on veut faire réagir ou interagir deux produits sous forme de deux couches (par exemple, pour des effets optiques). Dans ces cas, ls rapport de quantités de produits appliquées est important à respecter. Il faut là aussi que la quantité de chaque couche réalisée soit bien maitrisée.

Enfin, il existe une possibilité que l'utilisateur soit déçu de l'applicabilité des produits, la trouvant peu adaptée à son cas personnel. Ainsi, il est intéressant qu'après certains essais, il puisse modifier l'applicabilité des produits qu'il génère avec son système.

Ceci est d'autant plus essentiel que le fabricant fait reposer l'attrait du système sur les résultats de maquillage que permet son système. Il n'est pas facilement envisageable que les utilisateurs achetent plusieurs systèmes pour couvrir les différents cas de figure ou utilisent le système en substituant les compartiments en fonction de ces cas de figure. L'autre possibilité consistant à prévoir de très nombreux compartiments n'est pas non plus facile à mettre en oeuvre.

Selon un deuxième de ses aspects, l'invention a ainsi pour objet un système de distribution d'un produit, comportant un distributeur recevant au moins deux cartouches ayant chacune un réservoir contenant un produit de base, un premier produit de base comportant un épaississant, un deuxième produit de base comportant un agent de modulation de la viscosité de l'agent épaississant, le distributeur permettant de délivrer au moins ces deux produits de base dans des proportions réglables.

Selon ce deuxième aspect, l'invention peut présenter une ou plusieurs des caractéristiques suivantes :
- Première mise en oeuvre : Le premier produit de base présente une viscosité supérieure ou égale à 2 Pa.s, et préférentiellement supérieure ou égale à 4 Pa.s, plus préférentiellement comprise entre 4 Pa.s et 10 Pa.s, encore plus préférentiellement comprise entre 5 Pa.s et 8 Pa.s, avec un actif épaississant sensible au pH, et le second produit de base contient un agent acide modifiant la viscosité de l'actif du premier produit lors du contact entre les deux produits. L'actif épaississant du premier produit est notamment un gélifiant à base de polymère ou copolymère acrylate ou méthacrylate tel qu'un Carbomer, utilisé entre 0,8 et 2,5% en masse par rapport à la masse du premier produit, mieux entre 1 et 2,3% en masse par rapport à la masse du premier produit, encore mieux entre 1,5 et 2,2% en masse par rapport à la masse du premier produit, et l'agent acidifiant est notamment un acide minéral ou organique tel que l'acide citrique ou lactique, utilisé entre 0,2 et 10% en masse par rapport à la masse du deuxième produit, mieux entre 3 et 10% en masse par rapport à la masse du deuxième produit, encore mieux entre 5 et 8% en masse par rapport à la masse du deuxième produit. Le premier produit peut contenir d'autres épaississants (non sensibles au pH), comme par exemple, des agents épaississants polyosiques. La viscosité du second produit peut être inférieure ou supérieure ou égale à la viscosité du premier produit et être amenée par un agent gélifiant polyosique. De façon préférentielle, la viscosité du second produit est comprise entre 1 et 3Pa.s. Le pH du premier produit est supérieur ou égal à 6, préférentiellement supérieur ou égal à 7, encore préférentiellement supérieur ou égal à 8. Le pH du second produit est inférieur ou égal à 6, mieux inférieur ou égal à 5, préférentiellement compris entre 2 et 5, encore préférentiellement compris entre 3 et 5. De préférence, on associe à l'agent acidifiant du second produit un agent alcalinisant de façon que le pH du second produit ne soit pas inférieur à 2. Dans cette association, on règle l'agent alcalinisant et l'agent acidifiant avec un ratio en normalité alcalin/acide inférieur ou égal à 1, de préférence inférieur ou égal à, de façon que le pH du second produit soit inférieur ou égal à 7 et préférentiellement supérieur ou égal à 2). De préférence, l'agent alcalin est minéral, tel que de la soude, et l'agent acidifiant est organique tel qu'un acide carboxylique comme acide citrique ou l'acide lactique.
- Deuxième mise en oeuvre : Le premier produit de base présente une viscosité supérieure ou égale à 2 Pa.s, et préférentiellement supérieure ou égale à 4 Pa.s, plus préférentiellement comprise entre 4 Pa.s et 10 Pa.s, encore plus préférentiellement comprise entre 5 Pa.s et 8 Pa.s, et le second produit de base présente une viscosité comprise entre 0,01 et 2. Pa.s, mieux entre 0,05 et 1 Pa.s, de préférence entre 0,1 et 0,5 Pa.s. Le premier produit et le second produit contiennent notamment un gélifiant à base de polymère ou copolymère acrylate ou méthacrylate tel qu'un Carbomer ou un gélifiant à base polyosique, avec des concentrations en gélifiant (ensemble des gélifiants) compris pour le premier entre 0,8% et 2,5% en masse par rapport à la masse du premier produit, mieux entre 1 et 2,3% en masse par rapport à la masse du premier produit, encore mieux entre 1,5 et 2,2% en masse par rapport à la masse du premier produit et pour le second produit entre 0 et 2,5% en masse par rapport à la masse du deuxième produit, mieux entre 0 et 2% en masse par rapport à la masse du deuxième produit, encore mieux entre 0 et 1% en masse par rapport à la masse du deuxième produit, de préférence entre 0 et 0,5% en masse par rapport à la masse du deuxième produit. Dans un cas particulier, le premier et le second produit contiennent un actif gélifiant sensible au pH. Dans ce cas, les deux produits de base peuvent contenir cet agent épaississant à des concentrations différentes ou proches. Dans le second cas, (concentrations proches), les pH peuvent être différents tels que supérieur ou égal à 6, préférentiellement supérieur ou égal à 7, encore préférentiellement supérieur ou égal à 8 pour le premier produit et tels qu'inférieur à 6, préférentiellement inférieur à 5 pour le second produit de base.
- Troisième mise en oeuvre : Le premier produit de base présente une viscosité inférieure ou égale à 2Pa.s, mieux inférieure ou égale à 1 Pa.s et contient un actif épaississant sensible au pH, et le second produit de base contient un agent alcalin modifiant la viscosité de l'actif du premier produit lors du contact entre les deux produits. L'actif épaississant du premier produit est notamment un épaississant à base de polymère ou copolymère acrylate ou méthacrylate tel qu'un Carbomer et le second produit est un agent alcalinisant, tel qu'une base minérale ou organique tel qu'une amine, de l'ammoniaque. La viscosité du second produit peut être inférieure ou supérieure ou égale à la viscosité du premier produit. De façon préférentielle, la viscosité du second produit est comprise entre 1 et 3Pa.s. La concentration en agent épaississant du premier produit peut varier de 0,8 à 5% en masse par rapport à la masse du premier produit, mieux entre 1 et 2,3% en masse par rapport à la masse du premier produit, encore mieux entre 1,5 et 2,2% en masse par rapport à la masse du premier produit. Le pH du premier produit est inférieur à 6 et préférentiellement inférieur à 5. Le pH du second produit est supérieur à 6, mieux supérieur à 7, encore mieux supérieur ou égal à 8. De préférence, on associe à l'agent alcalinisant du second produit un agent acidifiant de façon que le pH du second produit soit inférieur ou égal à 10. Dans cette association, on règle l'agent alcalinisant et l'agent acidifiant avec un ratio en normalité alcalin/acide supérieur ou égal à 1, de façon que le pH soit supérieur ou égal à 7, de préférence inférieur ou égal à 10. De préférence, l'agent alcalin est organique, tel qu'une amine organique, et l'agent alcalin est minéral, tel que de l'acide chlorhydrique.
- le deuxième produit de base peut comporter une quantité de tensioactifs anioniques inférieure ou égale à 5%. Une telle quantité de tensioactifs anionique permet de donner au deuxième produit des caractéristiques additionnelles, tels qu'un effet moussant, de détergence ou d'aide au démaquillage, sans qu'il n'y ai d'effet d'épaississement dans le deuxième produit de base, l'effet d'épaississement n'apparaissant qu'au mélange du premier et du deuxième produit de base. Le tensioactif anionique est par exemple choisi parmi le sodium laureth sulfate, le sodium laureth sulfate oxyéthyléné, les tensioactifs à fonction sulfoniques tel que le sodium lauroyl sarcosinate.
- le système de distribution peut comporter une troisième cartouche avec un troisième produit de base,
- le troisième produit de base peut comporter un épaississant, notamment à une concentration différente de celle du premier produit de base,
- l'épaississant du troisième produit de base est choisi parmi les composés saccharidiques de type gomme, tels que la gomme arabique, la gomme acacia, la gomme guar, la gomme gellane, la gomme keraya, la gomme de carraghénane, les composés cellulosiques tels que CMC, HMC, HPMC ,les polymères synthétiques tels que les polyacides acryliques ou méthacrylique tels que les carbomer (Carbopol), ou polyuréthanes, polyvinylacétate, polyvinylalcool, les émulsions épaisses inverses ou directes, des associations de solvants non aqueux avec des agents épaississants pour huile, des argiles telles que bentonite, attapulgite, des organochélateurs, des protéines telles que caséine ou collagène, des agents de rhéologie rhéofluidifiants ou thixotropes, préférentiellement choisi parmi les polymères synthétiques tels que les polyacides acryliques ou méthacrylique partiellement réticulés comme par exemple par des groupes allylether erythreitol, ou allylether sucrose ou allylether de propylène, tels que les carbomer (Carbopol),
- l'épaississant peut être un gel de carbopol, de préférence en milieu neutre ou alcalin, notamment à une teneur comprise entre 0,1 et 2,5 % en masse par rapport à la masse totale du troisième produit de base, mieux entre 0,8 à 2,5 % par rapport à la masse totale du premier produit de base, encore mieux entre 1,5 % et 2,3 % par rapport à la masse totale du premier produit de base.
- les cartouches peuvent être reçues de façon amovible dans le distributeur,
- chaque produit peut quitter la cartouche par un canal de sortie de la cartouche, le canal de sortie étant défini par un embout de distribution de la cartouche entraîné en rotation relative par rapport à un corps de la cartouche par un mécanisme d'entraînement du distributeur pour distribuer le produit de base contenu dans la cartouche,
- le système peut comporter un mécanisme d'homogénéisation du premier produit de base, notamment vibratoire.

Cet aspect de l'invention concerne encore un procédé d'application d'un produit cosmétique, de maquillage et/ou de soin, sur les matières kératiniques humaines, comportant le réglage du distributeur en fonction de la zone à traiter, et la distribution du produit par prélèvement dans les cartouches des produits de base dans les proportions correspondant au réglage du distributeur.

Le réglage peut s'effectuer de façon à avoir la viscosité la plus grande et l'application s'effectuant sur des taches de peau.

Le réglage peut s'effectuer de façon à avoir une viscosité intermédiaire entre les viscosités extrêmes pouvant être obtenues, et l'application s'effectuant sur des taches de peau ou des cernes.
- Ainsi, selon ce deuxième aspect de l'invention, le système de distribution comporte au moins deux compartiments :
   - Un compartiment 1 comporte un agent épaississant E
   - Un autre compartiment 2 comporte un agent réglant R l'épaississement de l'agent épaississant.

Les autres compartiments (3,4...) éventuels comportent d'autres agents pour le maquillage M (ou le soin de la peau (pigments, actifs biologiques, actifs protecteurs, colorants....).
- E est typiquement un épaississant modulable par un agent de pH
- R est typiquement un agent de pH

Ou bien :
- E est un épaississant
- R est un diluant

On peut mettre dans le compartiment 1 et le compartiment 2 des agents M et ce avec une certaine logique.

L'invention permet selon ce deuxième aspect de traiter une ou plusieurs zones du visage et d'obtenir des mélanges très précis sur le plan de la fidélité des couleurs et une applicabilité adaptée permettant d'obtenir des effets particulièrement performants. Par la suite, on désigne par « zone » une partie du visage définie et assez réduite en surface, d'étendue comprise entre 1 cm2 et 100 cm2, mieux allant de 2 cm2 à 50 cm2.

Le système de distribution comporte de préférence un distributeur tel que défini plus haut. Le système de distribution peut ainsi être constitué par un seul appareil fonctionnant de manière autonome, de préférence manipulable d'une main, ou par un appareil qui fonctionne en interagissant avec d'autres pièces ou appareils. Il peut s'agir par exemple de différentes interfaces de sorties qui sont montées sur le distributeur en fonction du type de maquillage à réaliser, comme cela sera précisé plus loin. Il peut s'agir également d'un système informatique qui échange des informations avec le distributeur pour le piloter, ce système informatique comportant par exemple un terminal portable tel qu'un téléphone intelligent, un photophone, une tablette, un ordinateur personnel, ou un terminal dédié.

De préférence, le distributeur est agencé pour mettre sous pression un ou plusieurs compartiments contenant le ou les produits de base, par le biais de dispositifs de dosage volumétrique, de préférence un moteur entraînant en déplacement un piston dans le compartiment correspondant.

Le distributeur peut être formé d'un boîtier et d'au moins deux ou trois compartiments, et de préférence d'autant de moteurs. Par exemple, la rotation des moteurs entraîne des vis sans fin, lesquelles poussent les pistons de chaque compartiment. L'avance des pistons est par exemple contrôlée par le nombre d'impulsions de commande envoyées aux moteurs et/ou par la durée de fonctionnement de ces derniers. Les moteurs peuvent être alimentés séquentiellement, ou de préférence simultanément.

Par exemple, les moteurs sont alimentés durant un cycle élémentaire de fonctionnement pendant une courte durée les uns après les autres, ou les uns en même temps que les autres, de façon à distribuer des microdoses correspondantes.

Les cycles élémentaires sont répétés, avec éventuellement un temps d'arrêt entre eux, qui laisse le temps aux produits de base de s'écouler hors des compartiments.

Les compartiments peuvent être définis par des cartouches qui se retirent lorsqu'elles sont vides. En variante, les compartiments sont présents à demeure et remplis à nouveau une fois vide.

Chaque cartouche peut être fermée par un bouchon démontable pour permettre le nettoyage de la cartouche.

De préférence, le boîtier du distributeur est de forme allongée selon un axe longitudinal, ce qui peut faciliter sa manipulation, et les cartouches sont disposées autour de cet axe, dans le boîtier.

De préférence, les cartouches sont mises en place par l'arrière, et le mélange est délivré par l'avant. La mise en place des cartouches peut s'effectuer individuellement, ou en variante les cartouches constituent un ensemble monobloc lors de leur mise en place.

Les cartouches peuvent comporter chacune un mécanisme de dosage volumétrique, comportant un piston mu par un mécanisme d'entraînement du distributeur, dans une direction s'accompagnant d'une diminution du volume intérieur contenant le produit de base et d'une expulsion du produit. Il peut être intéressant que les cartouches aient une région de leur paroi au moins qui soit transparente, afin de permettre de voir la couleur du produit contenu à l'intérieur.

Le mécanisme d'entraînement peut comporter un système de motorisation formé de moteurs couplés à des réducteurs, de forme allongée parallèlement à l'axe longitudinal du distributeur, et disposés entre les cartouches. Ce positionnement des moteurs et cartouches rend le distributeur particulièrement compact.

Le produit de base peut sortir de façon étanche de la cartouche correspondante puis circuler dans un canal prévu à cet effet dans le boîtier du distributeur, avant de sortir de celui-ci.

Les cartouches se terminent avantageusement par un embout réalisé de telle sorte que, une fois la cartouche insérée dans le boîtier du distributeur, l'extrémité de l'embout débouche au ras du boîtier. En variante, l'embout est suffisamment long pour dépasser du boîtier et connecter ainsi différentes interfaces de sortie susceptibles d'être rapportées sur le boîtier du distributeur.

Grâce au mécanisme d'entraînement comportant des moteurs pour faire avancer des pistons, il est possible de délivrer de façon précise des mélanges en de très petites quantités. Ainsi, le mécanisme d'entraînement peut délivrer les produits de base avec à un débit minimal inférieur ou égal à 50 µL/s, mieux inférieur ou égal à 20 µL/s, encore mieux inférieur ou égal 10 µL/s. De préférence, le mécanisme d'entraînement délivre des débits compris entre 20 et 100 µL/s, mieux compris entre 40 µL/s et 60 µL/s. Il est donc possible de réaliser facilement un mélange d'environ 10 mg. Un tel système de distribution est alors idéal pour réaliser de petites touches de maquillage, pour couvrir par exemple une zone de 1 cm², mieux une zone de 0, 5 cm².

Il est aussi possible de réaliser des plus grandes quantités de mélange comme celles nécessaires au maquillage d'une joue, ou d'un visage. Ces quantités restent néanmoins relativement faibles, par exemple une quantité comprise entre 100 et 500 mg, mieux comprise entre 150 et 250 mg.

L'invention a ainsi pour objet, selon l'un de ses aspects, un système de distribution comportant un distributeur présentant un boîtier, et au moins une cartouche reçue dans le boîtier du ditributeur, cette cartouche comportant un corps et un piston mobile dans le corps, le boîtier comportant un mécanisme d'entraînement motorisé pour déplacer le piston de la cartouche.

De préférence, la cartouche comporte un embout de distribution par lequel s'effectue la sortie du produit, et cet embout de distribution est entraîné en rotation par le mécanisme d'entraînement pour déplacer le piston. L'embout peut comporter au moins un relief d'anti-rotation, mieux deux ergots d'anti-rotation diamétralement opposés.

L'embout peut porter un joint d'étanchéité, notamment un joint torique. Ainsi, au changement de cartouche, le joint est également changé, ce qui permet de s'affranchir de l'usure du joint.

Le distributeur peut comporter une carte électronique de pilotage du mécanisme d'entraînement motorisé, cette carte électronique étant traversée par le ou les embouts. Cela peut permettre de réaliser une carte s'étendant sensiblement sur toute la section du distributeur, donc de regrouper sur une seule carte tous les composants électroniques du distributeur, et ainsi de gagner en compacité et fiabilité. La carte peut s'étendre sensiblement perpendiculairement à l'axe longitudinal du boîtier. La carte peut porter un interrupteur de commande du fonctionnement du distributeur.

Le système de distribution peut être agencé pour fonctionner selon au moins deux modes de distribution.

Dans un premier mode, dit « continu », le mélange est distribué tant qu'une pression est exercée sur l'interrupteur de commande.

Dans un deuxième mode, dit « dose », une quantité prédéfinie du mélange est distribuée à chaque pression sur l'interrupteur.

Le ou les embouts peuvent déboucher à une extrémité du boîtier. Cela peut permettre de réduire le volume mort, comme cela sera détaillé dans la suite.

Le ou les embouts peuvent présenter à leur extrémité un système d'obturation pour éviter le sechage des produits dans le conduit, par exemple une membrane cicatrisante

La cartouche peut comporter une vis creuse sur laquelle le piston est vissé, le piston pouvant se déplacer axialement sur la vis lorsque celle-ci tourne ; le piston est empêché de tourner dans le corps de la cartouche. Par exemple, la friction du piston sur le corps de la cartouche peut suffire à l'empêcher de tourner quand la vis tourne.

Préférentiellement, la rotation est rendue impossible avec un corps de cartouche de section non circulaire et un piston non déformable.

Le couple des moteurs peut être déterminé électroniquement en fonction du courant absorbé, et servir par exemple à détecter une fin de course du piston. Une information concernant le couple peut être transmise à distance à un système informatique présentant une interface homme machine, pour permettre de surveiller le bon fonctionnement du distributeur.

Afin d'ajuster la teinte, le système de distribution selon l'invention doit permettre à l'utilisateur de faire varier le volume délivré provenant de chaque compartiment.

De façon préférée, le distributeur est piloté par un système informatique, qui est intégré au distributeur, ou externe à celui-ci, le distributeur étant alors apte à échanger avec le système informatique des informations par protocole sans fil ou câblé.

Le distributeur peut ainsi être piloté pour permettre l'ajustement de la teinte par distribution simultanée ou séquentielle, et contrôlée, de plusieurs produits de base de couleurs différentes.

La distribution des produits de base peut être continue ; si dans ce cas, les volumes de chacun des produits de base sont distribués d'une seule traite, simultanément ou successivement.

Dans le cas d'une distribution simultanée, il est utile de pouvoir ajuster les débits respectifs des différents produits de base pour que le mélange distribué corresponde à celui souhaité à chaque instant. Un tel mode de distribution peut convenir en particulier lors de la distribution du mélange par pulvérisation, à l'aide d'un aérographe. Pour ajuster les débits il est possible par exemple de jouer sur la vitesse de déplacement des pistons, par exemple, dans le cas d'un entrainement des pistons par une vis sans fin en faisant varier la vitesse de rotation des moteurs qui entrainent la vis. Les produits peuvent aussi être distribués de façon impulsionnelle avec un temps de distribution et un temps d'arrêt à chaque cycle. En jouant sur le rapport cyclique, il est possible d'agir sur le débit.

Tous les produits peuvent être délivrés simultanément lors du temps de distribution ; en variante, les cycles des différents produits sont déphasés, de sorte qu'un produit est distribué pendant un temps d'arrêt des autres produits.

Dans un mode particulier de l'invention, le mélange est délivré dans une cavité d'un contenant, pouvant fermer de façon hermétique ou non, par exemple en forme de coupelle, dans lequel on peut faire glisser un applicateur, en particulier un stylet ou une brosse. Un tel système de distribution est spécialement adapté aux liners, gloss et autres formules appliquées sans contact direct avec les mains. Ce contenant peut être amovible.

Par exemple, il sert de distributeur de rouge à lèvres et dispose d'un système de distribution, à vis par exemple. Lorsqu'il n'est pas amovible, le contenant peut être réalisé avec le corps du distributeur. Lorsqu'il est amovible, il peut constituer une interface de sortie parmi d'autres pouvant être montées sur le distributeur.

Les compartiments, et en particulier les cartouches, peuvent contenir tout ou partie du mécanisme d'entraînement, et par exemple le système de motorisation, ou mieux, une partie du système de motorisation, et ce, afin de réduire le nombre de pièces en mouvement dans le corps du distributeur à l'extérieur des cartouches. Par exemple, les cartouches comportent le rotor du moteur. Une fois les cartouches installées dans le corps du distributeur, les rotors sont mis en interaction avec les stators.

Le système de distribution est avantageusement agencé pour permettre de lancer des séquences préprogrammées dans lesquelles le mélange délivré par celui-ci est modifié de façon continue ou discontinue. Un mode « dégradé » permet par exemple passer progressivement d'un mélange A à un mélange B. Dans le cas d'une application par pulvérisation, notamment à l'aide d'un aérographe, cela permet de réaliser simplement les dégradés. Un mode « Alternatif » permet par exemple de passer rapidement d'un mélange

A à un mélange B plusieurs fois de suite. Dans le cas d'une application par pulvérisation, on peut ainsi réaliser un dépôt multicouche, avec des formulations différentes pour deux couches adjacentes superposées. Un autre mode permet par exemple de proposer plusieursmélanges successifs préprogrammés, le système informatique indiquant à chaque fois l'usage qu'il faut en faire à l'utilisateur, par exemple par affichage sur un écran.

Dans le cas d'une application manuelle, les mélanges sont distribués par exemple dans une coupelle. La personne se maquille à l'endroit recommandé avec un mélange correspondant prélevé dans la coupelle, puis nettoie éventuellement la coupelle et commande la délivrance d'un nouveau mélange ; l'opération est répétée autant de fois que nécessaire pour se maquiller complètement.

L'homogénisation des mélanges distribués peut se faire de différentes façons selon les cas d'utilisation. Dans le cas d'une application manuelle, elle peut se faire directement sur la zone d'application au moment de l'application ou dans la coupelle avant prélèvement ; dans le cas d'une application à l'aérographe, c'est la tuyère de l'aérographe qui sert de chambre de mélange ; dans le cas ou le mélange est distribué dans un contenanten vue d'une utilisation ultérieure, l'homogénéisation peut être faite à la main ou en faisant passer les produits distribués dans une chambre de mélange, située entre le distributeur et le contenant ou directement intégrée au contenant, comme détaillé par la suite.

Le produit peut être délivré par le système de distribution et utilisé extemporanément. En variante, le produit délivré par le système de distribution est conditionné et utilisé ultérieurement, par exemple à plusieurs reprises, avec par exemple un jour d'intervalle au moins entre deux utilisations.

### Réglage de l'applicabilité

Selon un exemple de mise en oeuvre de l'invention, le compartiment 1 comporte une composition de viscosité R1 et le compartiment 2 comporte une composition destinée à modifier la viscosité R1 du produit du compartiment 1 lors du contact.

Cas de figure a : Le compartiment 2 peut contenir un agent capable, lors du contact avec le produit du compartiment 1, de modifier la viscosité du produit de ce dernier. En décidant les rapports des produits à délivrer, on peut ainsi modifier et régler à sa juste valeur, la viscosité finale.

Cas de figure b : Le compartiment 2 peut contenir une composition de viscosité R2 plus faible ou plus forte que R1. Dans ce cas, lors du contact, la viscosité résultante sera modifiée. En décidant les rapports des produits à délivrer, on peut ainsi modifier et régler à sa juste valeur, la viscosité finale.

Cas de figure c : Dans un cas particulier et intéressant, le compartiment 2 peut contenir une composition de viscosité proche de R1 (ou égale), pourvu que le contact modifie la viscosité du produit du compartiment 1.

Pour mettre en oeuvre l'ensemble de ces cas de figures, on peut utiliser divers agents épaississants E et agent de réglage R.

Les épaississants « E » sont typiquement des composés gélifiants, comme des ingrédients naturels tels que saccharidiques comme les gommes arabique ou gomme acacia, gomme guar, gomme gellane, karaya etc... des polymères synthétiques tels que les polyacides acryliques ou méthacryliques tels que les carbomer (carbopol), des émulsions épaisses inverses ou directes, ou des associations de solvants non aqueux avec des agents épaississants pour huile.

Dans le cas des composés saccharidiques type « gomme », on les met entre 0,2% et 5% en masse par rapport à la masse du produit de base les comportant, mieux entre 0,8 % et 2,5% en masse par rapport à la masse du produit de base les comportant, encore mieux entre 1,5% et 2,3% en masse par rapport à la masse du produit de base les comportant.

Les gélifiants pouvant être utilisés peuvent être naturels ou artificiels tels que des amidons (E441), pectines (E440), agar (E406), acide alginique (E400), Alginate de sodium (E401) ou de potassium (E402) ou d'ammonium (E403) ou de calcium (E404), Carrageenan (E407) ou des ingrédients issus du monde animal (Gélatine comme le E441).

Pour les formulations riches en solvant, on peut utiliser un organogélateur. Ce sont typiquement des liquides organiques, une huile minérale ou une huile végétale, piégé dans un réseau tridimensionnel issu de l'autoassemblage supramoléculaire de petites molécules organiques (autrement appelés organogélateurs) formant des structures microscopiques ou nanoscopiques. Ils sont utilisés de 1 à 10% en masse par rapport à la masse du produit de base les comportant et peuvent être, par exemple des dérivés de 4-tertbutyl-1-aryl cyclohexanols, dérivés polymériques tels que polyéthylène glycols, polyesters, polyalkylènes, dérivés du N-lauroyl-L-lysine éthyl ester, des dérivés de peptides, des petits acides gras.

Les agents R sont :
- En particulier pour le cas de figure a, des solvants, tels que l'eau, l'éthanol ou l'huile, des agents de pH, tels que l'ammoniaque, l'amine ou la soude, des sels, tels queNaCl,
- En particulier pour le cas de figure b, des composés épaississants. Le composé épaississant peut :
   ∘ être différent du composé E,
   ∘ ou être le même à concentration différente,
   ∘ ou être le même et à concentration identique mais dans des conditions où son expression épaississante est différente.

Dans le cas particulier du cas de figure c, le produit du compartiment 2, contient un épaississant pour approcher la viscosité R1 et un second agent apte à modifier la viscosité du produit du compartiment 1.

Exemples illustrant le cas de figure a :
- On met dans le compartiment 1, un gel de carbopol à 1% neutralisé (ammoniaque) à 100%. Dans le compartiment 2, on met un agent acidifiant (acide citrique) à 4%.
- On met dans le compartiment 1, un gel de carbopol à 1% neutralisé (ammoniaque) à 10%. Dans le compartiment 2, on met un agent alcalin (ammoniaque 10%).
- On met dans le compartiment 1 une solution de tensioactif anionique (LES 5%). Dans le compartiment 2, on met un agent salin (NaCl) à 10%.

Exemples illustrant le cas de figure b :
- On met dans le compartiment 1, un gel de carbopol à 1% neutralisé (ammoniaque) à 100%. Dans le compartiment 2, on met une gomme de guar (jaguar HP 60), dilué (0,2%).

Exemple illustrant le cas de figure c :
- On met dans le compartiment 1, un gel de carbopol à 1% neutralisé (ammoniaque) à 100%. Dans le compartiment 2, on met un gel de carbopol à 2% neutralisé à 50%.

### Optimisation

Il est très intéressant d'avoir le moins de compartiments possibles.

Ainsi, si on veut faire un réglage de couleur, il faudrait prévoir 2 compartiments de plus que les deux destinés au contrôle de la viscosité.

Si le système ne comporte qu'un seul produit contenant des particules denses, il faut qu'il respecte les règles de rhéologies définies plus haut.

Il est possible de mettre les actifs M dans les compartiments 1 ou 2 ou 1 et 2.

Par exemple, on met les actifs M dans les compartiments 1 et 2 à deux concentrations différentes.

Si on considère que le produit du compartiment 2 augmente la viscosité du compartiment 1, alors on a intérêt à mettre dans le compartiment 2 moins d'actif M que dans le compartiment 1. En effet, en mettant en contact le produit du compartiment 2 avec le produit du compartiment 1, on réduit dans le mélange la concentration en actif M, mais en augmentant l'épaississement du mélange, on augmente l'épaisseur de la couche réalisée, produisant ainsi un effet de compensation.

Il est possible de mettre un actif M1 dans un compartiment 1 et un actif M2 dans le compartiment 2. Par exemple, M1 est un composé couvrant et M2 est un actif anti acné. Ainsi, plus l'utilisateur décide de mettre en contact le produit du compartiment 2 avec le produit du compartiment 1, plus il réduit dans le mélange la concentration en actif M1 et plus il augmente la concentration en actif M2 dans le mélange. Cette configuration est spécialement avantageuse pour le traitement des peaux acnéiques. Dans un mode riche en produit du compartiment 1, le produit s'applique sur l'ensemble du visage. Dans un mode riche en produit du compartiment 2, le produit est épais et s'applique bien sur les boutons : (où il n'y a pas forcément besoin d'une forte concentration en actif couvrant M1 et où il y a besoin d'une forte concentration en actif antiacnée M2).

Il est possible de mettre les actifs M dans un compartiment 3. Ainsi, on peut régler la concentration en actifs M, et régler la viscosité donc l'applicabilité.

### Utilisation pour le maquillage d'une ou plusieurs zones précises de peau

Le système de distribution permet de se maquiller jour après jour, en ne traitant que les zones à masquer. Pour ce faire, on délivre de petites doses de maquillage, qu'on applique précisément et séquentiellement sur les zones correspondantes. Chaque petite dose est réalisée selon le mélange qui convient à la zone.

Dans un mode préféré de mise en oeuvre de l'invention, le système de distribution attend qu'on le renseigne sur une zone à traiter puis délivre le mélange correspondant. Il peut faire appel à une table de correspondance préprogrammée pour cela, cette table résultant par exemple d'un procédé d'apprentissage tel que défini plus loin.

Dans un mode particulièrement intéressant, la programmation tient en compte la viscosité qu'il doit atteindre pour obtenir les résultats optimums. En variante, le système de distribution renseigne la personne, tout en délivrant un mélange, sur la zone sur laquelle celle-ci doit appliquer le mélange. Ainsi, le système de distribution peut suivre un programme d'application où il délivre dans un ordre donné, les différents mélanges à appliquer.

Dans un mode particulier de mise en oeuvre de l'invention, le système de distribution est renseigné sur les quantités à délivrer. Pour cela, il mémorise la correspondance entre la couleur, la zone du visage et la quantité nécessaire, ce qui permet de réduire les coûts et les pertes de produit, et de ne couvrir que faiblement la peau, évitant ainsi des effets d'occlusion. Ce faisant, il est possible d'utiliser des produits très couvrants et trop couvrants pour être appliqués sur l'ensemble du visage. Ainsi, il est possible d'obtenir des maquillages d'aspects naturels, voire indétectables.

Le système de distribution peut aussi permettre, en facilitant la distribution de petites quantités et leur usage rapide, de réduire la durée de conservation des produits, permettant ainsi de réduire les risques d'évolution et/ou les quantités de conservateurs à employer.

Le système de distribution est adapté à traiter les zones qu'on veut masquer, sans avoir à masquer l'ensemble du visage.

Lorsque l'utilisateur recherche la couleur à appliquer sur une zone du visage, il est intéressant de mémoriser la couleur qui va bien pour chaque zone, et le système de distribution est ainsi avantageusement agencé pour mémoriser cette couleur et la zone correspondante. Ainsi, en utilisant les informations mémorisées, à chaque utilisation, on peut obtenir la délivrance du même mélange pour chaque zone, ou, si l'on traite plusieurs zones, la même série de mélanges pour une même série de zones.

Le système de distribution peut aussi être agencé pour permettre de traiter une zone en faisant varier les couleurs application après application. Ainsi, la personne peut maquiller les lèvres avec différentes couleurs, qu'elle choisit au jour le jour selon ses gouts. Cette approche est aussi adaptée aux paupières ou aux cils, ainsi qu'au maquillage du visage car la personne peut avoir envie de changer de couleur de teint. Par exemple, les jours de la semaine, la personne réalise un fond de teint couleur claire, et le weekend, un fond de teint de couleur hâlée, ou un maquillage des yeux d'une couleur un jour, et d'une autre couleur un autre jour.

Le système de distribution peut être agencé pour permettre à l'utilisateur de changer de couleur selon ses goûts du jour, du moment, de ses tenues, du climat. Ainsi, on prévoit avantageusement un système d'aide à la décision pour guider l'utilisateur dans ses choix de couleur.

On peut prévoir aussi un système d'aide pour équilibrer les couleurs sur un même visage et participer à la réalisation d'un maquillage global réussi.

Il peut être souhaitable que plusieurs personnes d'un même groupe, par exemple une famille, puissent utiliser le système de distribution, réduisant ainsi les coûts et minimisant la place occupée. Cette solution est spécialement adaptée aux voyages ou aux hôtels, campings, avions, campings cars, boutiques, écoles... Pour ce faire, on peut prévoir que le système de distribution puisse être renseigné sur la personne qui l'utilise, afin d'accéder à des données personnelles préalablement mémorisées.

L'invention peut faire permettre de réaliser un dégradé, de la même façon que décrit plus haut.

### Utilisation en continu pour un maquillage en dégradé

Dans cette application, le système de distribution, pendant qu'il délivre le produit, change la formulation du mélange. De plus, on déplace la sortie des produits de base ou du mélange relativement à un contenant ou un support définissant une surface d'application. Dans un mode particulier de mise en oeuvre de l'invention, le système de distribution est agencé pour calculer l'évolution du mélange en fonction de la couleur C1, d'une zone à traiter et de la couleur C2, d'une autre zone à traiter. Par exemple, sachant que le menton nécessite une couleur C1 et que la joue nécessite une couleur C2, le système de distribution peut faire varier la formulation du mélange pendant qu'il le délivre pour réaliser un dégradé de couleur entre ces deux couleurs. Cela permet par exemple de mieux masquer les imperfections du visage en faisant en sorte que le résultat final soit réaliste, ou de faire des dégradés de couleur à des fins d'embellissement. Le système de distribution peut encore être agencé de telle sorte que l'utilisateur puisse commander une variation de couleur du mélange distribué sans que les couleurs de départ et/ou d'arrivée n'aient été préalablement fixées. Pour ce faire, le système de distribution peut disposer d'un système de localisation, ou d'auto-localisation, et déduire d'une table de correspondance les couleurs C1 et C2 qu'il doit réaliser, et donc les évolutions de mélange qu'il doit réaliser.

Le système de distribution peut comporter une tête de sortie, en particulier dans le cas d'un aérographe, qui est mobile et pilotée. Cette option permet alors, sans bouger le reste du système de distribution, de réaliser des dégradés. Par exemple, on localise le système de distribution près de la joue, puis on déclenche un système de commande qui va piloter automatiquement la variation de la formulation du mélange et le mouvement de la tête de sortie, de façon à par exemple avoir le centre de la joue plus rouge que sa périphérie, avec un dégradé entre les deux.

Le système de distribution peut encore être utilisé pour réaliser des produits sur mesure, que l'on conserve pour réaliser plusieurs applications.

Il est aussi possible de réaliser des produits solides ou semi-solides.

### Fabrication de compacts « à façon » ou autres produits solides ou semi-solides

Le système de distribution peut être agencé pour permettre de choisir un mélange et de le délivrer dans un contenant tel qu'une coupelle. Le mélange comporte de préférence des composés qui sont tels que le mélange puisse prendre en masse.

Plus préférentiellement, on utilise des composés qui rendent la prise en masse spécialement rapide. Ces composés sont soit déposés dans le contenant avant ou après son remplissage avec les autres constituants, soit sont prévus dans les compartiments du distributeur avec les autres constituants des produits de base, soit sont compris dans le distributeur dans un compartiment spécialement prévu pour les contenir.

On peut ainsi distribuer des compositions spécifiques qui peuvent par réaction chimique, biochimique ou physicochimique, durcir de façon accélérée après évacuation.

Ces compositions sont spécialement adaptées à la réalisation des compacts, c'est-à-dire :
- Prennent en masse,
- donnent un matériau qui peut se déliter en cas de frottement, et sont préférentiellement colorées.

De préférence, ces compositions sont très riches en particules solides, avec par exemple plus de 10% en masse de particules solides par rapport à la masse totale de la composition, mieux plus de 20% en masse de particules solides par rapport à la masse totale de la composition, encore mieux plus de 30% en masse de particules solides par rapport à la masse totale de la composition, préférentiellement entre 10 et 40% en masse de particules solides par rapport à la masse totale de la composition.

Ces compositions peuvent contenir des particules absorbantes, ou des composés réactifs, tels que ceux qui réagissent en contact avec l'air, par exemple du cyanoacrylate ou des alphasilanes, ou qui réagissent à la lumière, notamment UV.

Le contenant dans lequel le mélange est distribué peut comporter un composé A et les compositions distribuées un composé B, A et B étant choisis pour réagir entre eux et solidifier le mélange.

Dans un mode particulier de mise en oeuvre de l'invention, le système de distribution intègre un moyen de chauffage, par exemple à résistance électrique, pour la réalisation de rouges à lèvres ou autres produits cireux. Dans ce cas, les produits de base sont chauffés avant d'être délivrés.

Le système de distribution peut aussi comporter un moyen pour apporter de l'énergie, thermique et/ou lumineuse, après distribution du mélange dans un contenant, par exemple une résistance électrique ou une diode LED, notamment UV. Cette énergie peut accélérer la prise en masse du mélange distribué.

L'utilisation des agents E et R permet de régler les performances rhéologiques du produit solide ou semi-solide.

Préférentiellement, le mélange est homogénéisé avant la prise en masse.

### Réalisation de palettes de couleurs

Le système de distribution peut comporter un support, ayant plusieurs régions, et être agencé pour générer automatiquement plusieurs mélanges déposés dans lesdites régions, par exemple une série de couleurs adaptées à différentes parties du visage.

Le support peut définir plusieurs cavités pour recevoir les mélanges ou porter plusieurs contenants, par exemple sous forme de coupelles, éventuellement séparables du support.

Dans un cas particulier, le support prend la forme d'un visage avec des régions pour recevoir les mélanges pour des zones d'applications ciblées.

Le support être mobile, notamment rotatif, par rapport au corps du distributeur, et par exemple entraîné en déplacement par le distributeur, pour remplir successivement différents logements ou contenants.

### Distributeur à coupelle

Il existe un intérêt pour bénéficier d'un système de distribution capable de délivrer un mélange pouvant être facilement prélevé par l'utilisateur. Par ailleurs, dans le cas où les produits de base délivrés par le système de distribution ne sont pas déjà mélangés, il existe un besoin pour permettre à l'utilisateur d'effectuer aisément le mélange.

Le système de distribution peut comporter une coupelle, et un distributeur pour remplir la coupelle avec au moins un produit, la coupelle étant solidaire du distributeur au moins pendant son remplissage.

La coupelle est parfois encore appelée « creuset » et ce terme doit être compris avec une acceptation large.

Par « coupelle solidaire du distributeur » il faut comprendre que la coupelle est retenue, notamment immobilisée, au moins temporairement sur le distributeur, étant par exemple fixée sur celui-ci par des vis, par aimantation, par encliquetage, par verrouillage baïonnette, par serrage, ou réalisée avec une partie du corps du distributeur par moulage de matière. La coupelle lorsqu'elle est solidaire du distributeur permet de manipuler celui-ci d'une main, la coupelle restant en place sur le distributeur au cours des mouvements de ce dernier.

Le distributeur peut être proposé à l'utilisateur avec la coupelle déjà en place.

En variante, celle-ci est installée par l'utilisateur à la première utilisation du système de distribution.

De préférence la coupelle est moins profonde que large, ce qui facilite l'accès à celle-ci, et permet de prélever le produit, notamment le mélange avec un applicateur ou le doigt.

De préférence, la coupelle est séparable du distributeur, et constitue une interface de sortie qui peut être choisie parmi un ensemble d'interfaces de sortie pouvant être montées sur le distributeur, au choix de l'utilisateur en fonction du maquillage à réaliser, comme détaillé plus loin.

De préférence, le système de distribution comporte plusieurs orifices de remplissage en des produits de base différents, débouchant dans la coupelle. Ainsi, le mélange de ces produits peut avoir lieu dans la coupelle.

La coupelle a de préférence un fond de forme concave vers l'extérieur, ce qui peut faciliter son nettoyage par l'utilisateur entre deux utilisations.

De plus, cela peut faciliter le prélèvement du produit par l'utilisateur, et le mélange des produits de base.

De préférence, le distributeur permet de délivrer au moins deux produits de base dans la coupelle, dans des proportions réglables, et mieux au moins trois produits.

Dans un exemple de mise en oeuvre, le système de distribution comporte au moins deux coupelles pouvant être sélectivement alimentées par le distributeur. Cela peut permettre à l'utilisateur de remplir rapidement ces deux coupelles avec des mélanges dont les caractéristiques sont différentes. Cela peut faciliter les essais de matières colorées, et/ou permettre de préparer plusieurs mélanges de couleurs différentes destinées au maquillage de zones respectives du visage. Les coupelles peuvent être associées à des identifiants rappelant à l'utilisateur à quelle zone du visage un mélange contenu dans une coupelle donnée est destiné.

Les coupelles peuvent être mobiles relativement au distributeur, étant par exemple portées par un support mobile, tel qu'une tourelle mobile en rotation par rapport au distributeur, ou par un tiroir mobile en translation par rapport au distributeur.

Le système de distribution peut comporter un couvercle de fermeture de la coupelle. Ce couvercle de fermeture est de préférence transparent pour permettre à l'utilisateur de visualiser la couleur du mélange contenu à l'intérieur.

Lorsque la coupelle est séparable du distributeur, elle peut être introduite le cas échéant dans un boîtier permettant de la transporter plus facilement, ce boîtier pouvant comporter le cas échéant un miroir et/ou un applicateur. Le couvercle du boîtier peut dans ce cas servir de couvercle pour la coupelle.

Le volume de la coupelle peut être compris entre 2 et 1000 mm³, mieux entre 100 et 1000 mm³, encore mieux entre 250 et 750 mm³.

Le ou les produits de base délivrés dans la coupelle sont de préférence des fonds de teint, mais en variante il s'agit de produits de maquillage des lèvres ou des paupières.

La coupelle est de préférence de forme symétrique de révolution. En variante, elle est de contour polygonal ou autre. Son plus grand diamètre intérieur, ou celui du cercle inscrit dans le cas d'un contour non circulaire, est de préférence compris entre 2 et 100 mm, préférentiellement entre 5 et 40 mm. Sa profondeur est de préférence comprise entre 1 et 10 mm, mieux entre 3 et 8 mm. Sa profondeur est de préférence comprise entre 1 et 10 mm. De préférence, la taille et la forme de la coupelle permettent soit une application directe du mélange sur la peau, soit une préhension au doigt ou avec un applicateur. La coupelle peut être réalisée avec un matériau élastiquement déformable, ce qui permet par exemple d'inverser la concavité du fond de la coupelle et de vider celle-ci plus facilement ou de l'utiliser pour appliquer le produit.

La coupelle peut être dépourvue de mélangeur ; dans ce cas, les produits de base peuvent arriver du distributeur dans la coupelle à l'état non mélangé, par des orifices de distribution respectifs distincts. En variante, le distributeur intègre un mélangeur et les produits de base arrivent déjà mélangés dans la coupelle.

La coupelle peut aussi intégrer un mélangeur statique, comme détaillé plus loin, qui est alimenté par des orifices de remplissage distincts du distributeur, et qui délivre de préférence le mélange dans une cavité de la coupelle située au-dessus du mélangeur.

L'invention a encore pour objet un procédé de préparation d'un produit de maquillage, comportant l'étape consistant à remplir une coupelle d'un système de distribution tel que défini plus haut avec au moins un produit de base provenant du distributeur.

Plusieurs produits peuvent être délivrés dans le fond de la coupelle, puis mélangés à l'aide du doigt ou d'un applicateur, ou d'un mélangeur statique intégré à la coupelle.

La coupelle est de préférence remplie par le dessous. Il a été proposé dans le passé des systèmes de distribution à l'aide d'une sonotrode.

La coupelle selon l'invention n'est pas destinée à entrer en vibration pour distribuer le ou les produits amenés par le ou les canaux d'alimentation de celle-ci. Elle est différente d'une sonotrode. De préférence, la coupelle est réalisée en matière plastique.

### Mélangeur intégré à l'interface de sortie

Il existe un intérêt pour bénéficier d'un système de distribution capable de délivrer un mélange pouvant être facilement utilisé, notamment prélevé par l'utilisateur, sans nécessiter une action additionnelle de mélange de la part de celui-ci.

Le système de distribution comporte de préférence un distributeur ayant des canaux de sortie de produits de base et une interface de sortie séparable du distributeur, cette interface ayant un mélangeur statique, délivrant de préférence le mélange dans une cavité où il peut être prélevé.

Le mélangeur statique peut être situé sous la cavité précitée. Le système de distribution est alors particulièrement adapté à la création de compacts, en utilisant comme interfaces de sortie des coupelles à mélangeur statique intégré. Dans ce cas, on vient remplir la cavité de la coupelle en produit par le dessous. Après passage dans le mélangeur statique, les produits de base mélangés viennent recouvrir le mélangeur.

Il est possible d'utiliser plusieurs interfaces de sortie et de les remplir avec des mélanges respectifs différents, sans avoir à purger le mélangeur, ce qui réduit les pertes de produit. L'interface de sortie peut être à usage unique, le cas échéant.

De préférence, le mélangeur statique comporte une chambre centrale communiquant avec des canaux d'admission des produits de base. Cette chambre centrale peut communiquer avec une chambre périphérique comportant une série de cloisons qui agissent comme des déflecteurs pour le mélange, et créent un cisaillement de celui-ci.

La chambre périphérique peut comporter une cloison annulaire ajourée définissant des ajours par lesquels passe le mélange en circulant dans la chambre périphérique. Les chambres centrale et périphérique peuvent être fermées supérieurement par une paroi qui définit le fond de la cavité recevant le mélange.

Le fond de la chambre périphérique peut être de forme hélicoïdale autour de l'axe de la coupelle et de hauteur diminuant au fur et à mesure que l'on progresse vers la sortie. Cette dernière peut déboucher en avant d'une rampe de liaison entre le fond de la chambre périphérique et la paroi supérieure du mélangeur, cette rampe de liaison étant de préférence une portion d'hélice prolongeant l'hélice formée par la paroi de fond de la chambre périphérique.

De préférence la chambre périphérique comporte la cloison annulaire précitée et des cloisons radiales qui forcent le mélange à circuler alternativement entre des régions supérieure et inférieure de la chambre périphérique et entre des régions radialement intérieure et extérieure, le mélange circulant par exemple d'une région supérieure et radialement extérieure à une région inférieure et radialement extérieure en passant à travers la cloison annulaire précitée.

Le mélangeur peut comporter un corps extérieur dans lequel est reçue une pièce formant le coeur du mélangeur, le corps extérieur fermant radialement à l'extérieur la chambre périphérique et comportant un montant qui sépare les chambres centrale et périphérique.

Le corps extérieur du mélangeur et le coeur du mélangeur peuvent être chacun réalisés d'une seule pièce par moulage par injection.

### Volume mort réduit

Il existe un intérêt pour réduire les pertes de produit lors des changements de formulation du mélange, ainsi que pour permettre de faire varier le plus rapidement possible la couleur du mélange au cours de l'application, notamment en cas de couplage du distributeur avec un aérographe.

Le distributeur recevant au moins deux cartouches ayant chacune un réservoir contenant un produit de base, ce dernier peut quitter la cartouche par un canal de sortie de la cartouche, ce canal de sortie débouchant à l'extérieur du distributeur ou à proximité de sa surface externe.

Le canal de sortie peut notamment déboucher dans une zone de prélèvement du mélange ou à proximité de celle-ci, notamment à moins de 5 mm, mieux à moins de 3 mm, mieux à moins de 1mm, mieux à fleur.

La section transversale du canal de sortie est par exemple comprise entre 1 et3 mm².

Ainsi, chaque produit de base provenant d'une cartouche peut quitter le distributeur sans se mélanger avec un produit de base d'une autre cartouche et l'on minimise le volume mort qui ne peut être prélevé et susceptible d'accroître l'inertie du système. Le produit est plus rapidement disponible sans avoir à circuler dans des canaux spécifiques du boîtier du distributeur, évitant une étape fastidieuse de purge si changement de cartouche.

L'extérieur du distributeur peut être la zone de prélèvement du produit, notamment lorsque le distributeur est réalisé avec une coupelle non prévue pour être enlevée, dans laquelle le mélange est distribué, ou une zone du distributeur destinée au montage d'une interface de sortie amovible, qui définit la zone de prélèvement. Cette interface de sortie peut comporter une coupelle telle que définie ci-dessus. Cette zone de montage correspond par exemple à l'extrémité du boîtier du distributeur en l'absence de l'interface de sortie. La zone de montage peut être sensiblement plane et perpendiculaire à l'axe longitudinal du boîtier du distributeur.

Le distributeur peut comporter trois cartouches de produits de base.

Le distributeur peut comporter des logements pour recevoir les cartouches, lesquelles sont de préférence reçues de façon amovible dans le distributeur. Ce dernier peut comporter des passages pour des conduits des cartouches définissant les canaux de sortie.

La longueur de ces conduits est de préférence telle que les conduits se situent légèrement en retrait du fond ou à affleurement de la cavité servant au prélèvement du produit, ou en variante légèrement en retrait ou à affleurement de la face d'extrémité du boîtier du distributeur définissant la zone de montage.

Ces conduits des cartouches peuvent être des embouts servant à entraîner en déplacement des pistons au sein des cartouches, comme détaillé plus haut.

### Interfaces de sortie multiples

Il existe un besoin pour pouvoir, à l'aide d'un même système de distribution, réaliser facilement des maquillages différents, et pouvoir si on le souhaite maquiller des zones aussi différentes que la peau, les lèvres ou les cils ou sourcils.

Selon l'un de ses aspects, indépendamment ou en combinaison avec les autres aspects de celle-ci, et notamment ce qui précède, l'invention a pour objet un système de distribution comportant un ensemble comportant un distributeur d'au moins un produit cosmétique, notamment de maquillage, et au moins deux interfaces de sortie pouvant se monter chacune de façon amovible sur le distributeur, ces interfaces de sortie permettant de recevoir le ou les produits délivrés par le distributeur, étant de préférence choisies parmi les suivantes :
- interface de sortie comportant un contenant, notamment une coupelle permettant un prélèvement du produit au doigt ou à l'aide d'un applicateur,
- interface de sortie permettant de délivrer le produit à un système de pulvérisation, notamment un aérographe,
- interface de sortie comportant plusieurs régions de réception du produit, mobile par rapport au distributeur,
- interface de sortie permettant de délivrer le produit à un embout de distribution.

De préférence, l'ensemble comporte au moins trois desdites interfaces de sortie, mieux les quatre interfaces de sortie.

Le distributeur peut comporter au moins deux produits de base différents et permettre de délivrer ceux-ci dans des proportions variables, et de préférence, le distributeur comporte trois produits de base différents et permet de délivrer ceux-ci dans des proportions variables.

Chaque interface de sortie peut comporter une base permettant sa fixation sur le distributeur. Cette fixation peut s'effectuer à l'aide de vis par exemple, mais de préférence la base est agencée pour permettre un démontage et un remplacement d'une interface de sortie sans nécessité d'outil. Il s'agit par exemple d'une fixation quart de tour ou à l'aide d'une bague de verrouillage externe.

L'interface de sortie et/ou le boîtier du distributeur peuvent comporter des joints d'étanchéité permettant une communication étanche entre le boîtier du distributeur et l'interface de sortie. Le cas échéant, le distributeur est agencé pour reconnaître l'interface de sortie qui est montée dessus, par exemple grâce à identifiant de l'interface de sortie sous forme de reliefs spécifiques, lesquels sont détectés par le distributeur, ou d'une puce électronique reconnue par le distributeur. Cela peut permettre d'adapter le fonctionnement du distributeur à l'interface de sortie montée dessus. Le distributeur peut communiquer à un système informatique des informations sur l'interface de sortie qu'il porte, et le système informatique peut afficher en fonction de ces informations un écran spécifique et/ou lancer un programme spécifique de commande des paramètres de fonctionnement du distributeur, afin par exemple d'adapter la dose distribuée et/ou le débit à la nature de l'interface de sortie.

Plusieurs interfaces de sortie peuvent être proposées initialement à l'utilisateur avec un distributeur commun au sein d'un même emballage, par exemple un coffret ou une boîte de carton.

L'invention a encore pour objet un procédé de maquillage, comportant l'étape consistant à sélectionner une interface de sortie, à la monter sur le distributeur, et à délivrer le ou les produits contenus dans le distributeur dans celle-ci.

### Cartographie et apprentissage

Le terme « cartographie » s'entend ici comme un procédé d'indexation d'une couleur et d'une zone, avec enregistrement.

La cartographie peut concerner des applications sur des zones plus petites qu'un cm2. Toutefois, l'oeil nu a alors du mal à discerner si le résultat obtenu est adéquat, et il est préférable de substituer l'évaluation à l'oeil nu par une évaluation instrumentée, avec grossissement. L'application de petites quantités de matière colorée peut être faite au doigt, avec des outils classiques tels que des pinceaux, ou avec des applicateurs spécialisés.

La cartographie peut être générée lors d'une période d'apprentissage, pendant laquelle l'utilisateur effectue des tests avec des mélanges sur différentes zones du visage ; une fois renseignée, la cartographie peut ensuite être utilisée pour le maquillage de tous les jours. Cette période d'apprentissage peut tenir compte de la viscosité. Ce faisant, l'utilisateur teste plusieurs rhéologies et renseignent le meilleur réglage obtenu.

Des interfaces graphiques spécifiques peuvent servir pendant la période d'apprentissage et pendant la période d'utilisation de la cartographie.

En particulier, le système de distribution peut être utilisé avec une interface graphique où l'opérateur voit le visage, qui est par exemple schématisé, figuratif ou précis comme une photographie ou une simulation 3D. Dans ce cas, l'opérateur peut pointer une partie du visage sur l'écran pour voir apparaître et/ou délivrer la couleur adéquate.

L'interface graphique peut aussi laisser apparaître les autres zones du visage où l'utilisation de la même couleur est adéquate.

Pour réaliser la cartographie, l'opérateur applique une couleur puis réalise l'évaluation.

Les zones du visage peuvent être traitées les unes après les autres ; par exemple, on réalise l'exercice sur une partie de la joue, puis sur le nez, etc....

Une autre possibilité consiste à réaliser un mélange donné, et appliquer sur plusieurs zones ce même mélange. L'opérateur doit alors chercher la zone du visage pour laquelle la couleur est adaptée. Le mélange est ensuite indexé dans le système informatique en l'attribuant à la ou aux zones du visage pour lesquelles il convient.

Selon l'un de ses aspects, indépendamment ou en combinaison avec les autres aspects de celle-ci, et notamment ce qui précède, l'invention a pour objet un procédé d'apprentissage d'un système de distribution comportant un distributeur permettant de distribuer un mélange de couleur variable, et un système informatique permettant de sélectionner une couleur et de mémoriser des données, comportant les étapes consistant à :
a) Sélectionner au moins une couleur à l'aide d'une interface du système informatique,
b) délivrer à l'aide du distributeur au moins un mélange de la couleur sélectionnée,
c) évaluer le ou les mélanges distribués après application sur au moins une zone du visage,
d) mémoriser les caractéristiques d'au moins un mélange, notamment un mélange que l'utilisateur souhaite pouvoir rappeler, et d'au moins une zone sur laquelle il a été testé.

Cette mémorisation peut s'effectuer notamment en vue d'une distribution ultérieure de ce mélange pour maquiller ladite zone.

De préférence, le système informatique est agencé pour permettre à l'utilisateur de signaler si le résultat du test est satisfaisant ou non, voire de le renseigner sur la comparaison à un test effectué précédemment.

On peut aussi réaliser un mélange donné, et chercher la zone du visage pour laquelle il est adapté. On enregistre alors le mélange en l'attribuant à la ou aux zones du visage pour lesquelles il est adapté, dans une table de correspondance qui servira par la suite, pour, partant d'une zone de la peau, en déduire le mélange à utiliser.

On peut procéder de la même façon avec d'autres mélanges pour réaliser une cartographie de l'ensemble du visage et ainsi avoir une table de correspondance complète du visage.

On peut aussi réaliser un mélange donné, l'appliquer sur une zone donnée, puis faire varier le mélange jusqu'à obtenir le mélange le plus adapté. On enregistre alors le mélange en l'attribuant à la ou aux zones du visage pour lesquelles il convient, dans une table de correspondance qui servira par la suite, pour, partant d'une zone de la peau, en déduire le mélange à utiliser.

De préférence, le système informatique évalue et mémorise les quantités utilisées zone par zone. Un tel procédé, par « touches d'essai », permet d'identifier le ou les produits dont a besoin la personne qui veut se maquiller. Ainsi, le système de distribution peut servir sur les points de vente pour conseiller les personnes qui veulent se maquiller ou à la maison pour bien définir les produits à commander.

L'interface du système informatique comporte de préférence un écran tactile affichant la couleur du mélange lors de sa sélection.

L'interface peut afficher un visage et permettre de renseigner le système informatique en sélectionnant la zone sur le visage affiché.

Le système informatique est de préférence agencé pour permettre d'associer une zone, des paramètres de reconstitution du mélange, et la date de l'essai et/ou tout autre identifiant du mélange.

Le système informatique est de préférence également agencé pour permettre d'associer en outre à ladite zone, aux paramètres de reconstitution du mélange, et à la date ou à l'identifiant du mélange, au moins l'une des données suivantes : le nom de la zone, la période de l'année, le nom d'un évènement, un identifiant de l'utilisateur, l'âge de l'utilisateur.

Les étapes a) à c) peuvent être renouvelées au moins une fois avant la mémorisation des caractéristiques du mélange à l'étape d).

Le système informatique peut être agencé pour rechercher dans une base de données une référence de produit commerciale, sur la base des caractéristiques du mélange identifié comme convenant à au moins une zone donnée, et la communiquer à l'utilisateur.

La sélection à l'étape a) peut s'effectuer avec l'aide d'un système expert externe ou non au système informatique.

Le système expert peut analyser une image de l'utilisateur, pour proposer une couleur de mélange au moins sur la base de l'image analysée.

L'étape a) peut être précédée par la proposition d'une couleur et d'une zone à tester avec un mélange de cette couleur, par le système informatique à l'utilisateur.

Le système informatique peut être agencé pour permettre à l'utilisateur de le renseigner sur l'appréciation du résultat du test de l'étape c) et pour générer une proposition de modification du mélange à sélectionner lors du retour à l'étape a).

Le système informatique peut être agencé pour proposer au moins une couleur de mélange à l'étape a) en fonction d'une zone d'application renseignée par l'utilisateur.

Le système informatique peut être agencé pour proposer au moins une zone d'application à l'étape a), en fonction d'une couleur renseignée par l'utilisateur.

Le distributeur peut délivrer à l'étape b) au moins deux mélanges de couleurs différentes, de préférence séparés, en vue de leur application simultanée sur la zone de test.

Cela peut faire gagner du temps, et faciliter la comparaison des résultats.

L'invention a encore pour objet un procédé de maquillage à l'aide d'un système de distribution selon l'invention, dans lequel :
a) L'utilisateur adresse une requête concernant un besoin de maquillage au système informatique,
b) celui-ci génère en retour une proposition de couleur en vue du maquillage d'une zone associée, sur la base de l'apprentissage effectué préalablement, et
c) le système informatique pilote le distributeur pour la production du mélange de la couleur proposée, notamment si celle-ci est validée par l'utilisateur.

Un tel procédé peut utiliser une cartographie précédemment établie avec l'utilisateur.

L'invention a encore pour objet un produit programme d'ordinateur comportant des instructions de code permettant, lorsque exécutées dans un système informatique, d'amener le système informatique à :
- Permettre à l'utilisateur de sélectionner au moins une couleur et/ou une zone d'application, notamment à l'aide d'une interface telle qu'un écran tactile,
- piloter un distributeur de façon à délivrer un mélange de la couleur sélectionnée par l'utilisateur,
- permettre à l'utilisateur de déclencher la mémorisation de la couleur du mélange et d'une zone d'application associée, notamment en vue d'une distribution ultérieure du même mélange, notamment sur la même zone.

Le produit programme d'ordinateur peut comporter des instructions de code permettant, lorsque exécutées dans un système informatique, d'amener le système informatique à :
- Recevoir une requête de l'utilisateur concernant un besoin de maquillage, notamment à l'aide d'une interface telle qu'un écran tactile,
- proposer sur la base au moins de données générées par le procédé d'apprentissage tel que défini ci-dessus, au moins une couleur et/ou une zone d'application,
- piloter un distributeur pour la production du mélange de la couleur proposée, notamment si celle-ci est validée par l'utilisateur.

### Aide à distance

Il est souhaitable de pouvoir aider l'utilisateur à se maquiller, notamment à choisir les bons coloris.

L'invention, selon l'un de ses aspects, indépendamment ou en combinaison avec ses autres aspects, notamment ce qui précède, a ainsi pour objet un procédé de maquillage comportant les étapes consistant à :
- permettre l'établissement d'une liaison vidéo, par exemple via internet, entre une caméra d'un premier site et un deuxième site,
- permettre au deuxième site de piloter directement ou indirectement un distributeur présent sur le premier site, ce distributeur permettant de faire varier la couleur d'un mélange distribué,
- permettre à une personne présente au premier site d'appliquer le mélange distribué et d'envoyer vers le deuxième site une image correspondante, pour recevoir en retour une information relative au résultat de maquillage.

Le deuxième site peut notamment comporter un écran de visualisation qui permet à un conseiller présent devant cet écran de voir le résultat du maquillage avec le produit distribué par le distributeur et de conseiller la personne qui s'est maquillée. Ce conseiller peut agir en retour sur le distributeur pour modifier la couleur du mélange et l'adapter au mieux au visage de la personne présente sur le premier site. Ainsi, cette personne gouverne le mélange délivré par le distributeur. La première personne peut se maquiller sous les yeux de la seconde. La seconde personne voit sur son écran le résultat de l'essai, et ainsi peut corriger le mélange qu'elle va commander à distance, jusqu'à obtenir le maquillage idéal.

Le cas échéant, l'acquisition vidéo peut être calibrée à l'aide d'une mire, ou avec le mélange distribué par le distributeur sur une surface de référence. Cela permet ensuite une visualisation plus fidèle du maquillage effectué sur le premier site.

De préférence, la liaison vidéo entre les deux sites est une liaison duplex.

Le premier site peut recevoir un tutoriel du deuxième site, le cas échéant.

Des identifiants des produits de base peuvent être communiqués au deuxième site ; cela peut permettre de connaître précisément la couleur de chacun des produits de base.

Le procédé peut comporter la mémorisation des paramètres de réglage du distributeur, une fois qu'un mélange donné est estimé satisfaisant. De préférence, cette mémorisation peut être commandée depuis le deuxième site. La mémorisation peut avoir lieu dans le système informatique présent sur le premier site et/ou sur un serveur externe.

Une variante peut consister à faire travailler une personne pour aider le maquillage de plusieurs personnes. Ce mode de mise en oeuvre permet le développement des « Make-Up Artists » et leur travail soit au sein d'un institut, soit via internet. Cela permet aussi le maquillage de personnes aux moyens limités, telles que les personnes malvoyantes, ou discernant mal les couleurs, ou âgées, ou manquant de confiance en elles.

### Pilotage par interface tactile

Il existe un besoin pour faciliter le pilotage du système de distribution et notamment le choix de la couleur du mélange distribué.

Selon un autre de ses aspects, indépendamment ou en combinaison avec ses autres aspects, et notamment avec ce qui précède, l'invention a ainsi pour objet un système de distribution comportant un distributeur et un système informatique de pilotage du distributeur, ce système informatique comportant un écran tactile sur lequel peut être affichée la couleur du mélange et un moyen de sélection déplaçable sur l'écran, pour faire varier la couleur du mélange distribué.

De préférence, l'écran affiche des couleurs extrêmes entre lesquelles la couleur du mélange peut être sélectionnée, en déplaçant le moyen de sélection entre ces couleurs extrêmes.

L'écran peut afficher une échelle de couleurs entre au moins deux couleurs, ou une surface, notamment de contour triangulaire, au sein de laquelle le moyen de sélection peut se déplacer. Cette surface peut localement faire apparaître la couleur du mélange en fonction par exemple de la distance à chacun des sommets, matérialisant chacun un produit de base pur.

Le système informait que peut effectuer une partie des calculs nécessaires pour déterminer les fractions de chacun des produits de base conduisant à un mélange de la couleur recherchée.

Le système informatique peut être un téléphone intelligent, un photophone, une tablette, un ordinateur individuel. En variante, il est intégré au boîtier du distributeur.

Le système informatique peut disposer d'une caméra. Cette dernière peut être utilisée notamment pour l'acquisition d'une image de l'utilisateur et/ou du mélange.

Le système informatique peut être agencé pour afficher une image d'un visage, afin de faciliter le repérage des zones sur lesquelles le mélange doit être appliqué.

### Couplage du système de distribution à un moyen de pulvérisation

Le système de distribution peut comporter ou être connecté à un moyen de pulvérisation du mélange, de préférence un aérographe.

Le distributeur peut comporter trois cartouches contenant des produits de maquillage de couleurs différentes.

L'aérographe peut comporter un stylet définissant la chambre de prélèvement, le stylet se fixant sur le distributeur ou sur une interface de sortie fixée au distributeur, ou faisant partie intégrante de cette interface de sortie.

Le système de distribution peut comporter un circuit de contrôle du fonctionnement du distributeur, permettant de faire varier la proportion des produits de base délivrés dans la chambre de prélèvement, au cours du fonctionnement de l'aérographe. Les proportions peuvent être modifiées en fonction du déplacement de l'aérographe relativement à la surface sur laquelle le mélange est pulvérisé. Ce déplacement peut être mécanisé, le cas échéant.

Ce circuit de contrôle peut comporter ou être constitué par un système informatique tel que défini plus haut.

Le boîtier du distributeur peut servir de poignée lors de la manipulation d l'ensemble pour délivrer le mélange.

Le distributeur peut comporter une caméra et/ou un ou plusieurs capteurs tels que des accéléromètres afin de localiser automatiquement la zone sur laquelle le mélange est appliqué, et pouvoir régler automatiquement la couleur en fonction de la position, le cas échéant.

L'invention a encore pour objet un procédé de maquillage à l'aide d'un ensemble tel que défini ci-dessus, dans lequel un mélange est pulvérisé sur la peau à l'aide du moyen de pulvérisation, notamment de l'aérographe.

La composition du mélange peut être modifiée au cours du déplacement de l'aérographe relativement à la peau. Un dégradé peut être réalisé.

Cet aspect de l'invention repose sur la constatation que le distributeur peut être utilisé pour alimenter le système de pulvérisation, notamment l'aérographe, tout en permettant au système de distribution d'être suffisamment réactif pour autoriser un changement de la couleur du mélange distribué en cours de maquillage du visage, notamment lorsque la zone à maquiller change.

Il peut être avantageux que la distribution des produits se fasse de façon itérative, notamment avec des temps de distribution non déphasés entre les différents produits.

Cela peut rendre plus facile de faire varier la composition du mélange distribué au cours du temps.

Le mélange peut être réalisé directement dans l'aérographe, sans pratiquement aucun volume mort gênant, permettant ainsi de changer le mélange pulvérisé en temps réel.

La dépression créée dans la chambre de prélèvement est suffisante pour entraîner les produits de base, sans pour autant gêner le dosage.

La dépression qui règne dans la chambre de prélèvement est par exemple comprise entre 10 mbar et 200 mbar, mieux entre 50 et 150 mbar, encore mieux entre 75 et 125 mbar.

La viscosité des produits de base mesurée à 1 atm et à 25°C avec un viscosimètre rotatif CONTRAVES TV équipé d'un mobile MS-r3 ou MS-r4 à la fréquence de 60 Hz, après 10 minutes de rotation du mobile, est par exemple comprise entre 0,05 Pa.S et 50 Pa.S

La section des canaux d'arrivée des produits de base dans la chambre est par exemple comprise entre 1 et 3 mm², mieux entre 2 et 3 mm².

L'alimentation en produit s'effectue de préférence de façon continue.

Il est encore possible d'appliquer simultanément des produits de base non miscibles ou réactifs, comme un gel aqueux et un gel huileux, qui vont se déposer de façon pixellisée directement sur la peau, produisant une sorte de gel/gel in situ, des silicones réactives, ou des produits de coloration réagissant ensemble. Les ratios en produits de base peuvent être ajustés en fonction du résultat particulier souhaité. Par exemple, dans le cas des gels aqueux et gels huileux, on pourra faire varier le ratio correspondant au volume du premier produit de base sur le volume du deuxième produit de base entre 10/1 et 1/10, mieux entre 5/1 et 1/5.

Dans un mode particulier de l'invention, le système choisit une viscosité inférieure ou égale à 10⁻² Pa.S pour les applications sprays et une viscosité importante pour les applications à la main ou avec outil.

### Système de localisation ou d'auto-localisation

Le système de distribution selon l'invention peut disposer d'un système de localisation ou d'auto-localisation.

On appelle système de localisation, un moyen de saisir, de la part de la personne, la zone qu'elle va traiter. Ceci peut être réalisé notamment par des systèmes laissant libre au moins une main. Ainsi, on peut utiliser une interface d'un système informatique telle qu'un écran tactile, un joystick, ou un système de reconnaissance vocale.

On appelle système d'auto-localisation, un moyen de saisir, sans que la personne n'intervienne, la zone à traiter. Ceci peut être réalisé par un ou plusieurs accéléromètres qui déduisent, par le fait des mouvements, les directions que la personne vise ou par une caméra et un système de reconnaissance d'image.

Des exemples de système de distribution ont été décrits précédemment en référence au dessin. La description de ces figures vaut pour cet aspect de l'invention, et cette description ne sera pas reprise ici.

### Exemple 1(deuxième aspect de l'invention)

On réalise plusieurs produits de base (les proportions sont mssiques)

| Produit de base | Oxyde de fer/Oxyd e de titane | Carpobopol 980 Polymer (Lubrizol) | Ammo-niaque 10% avec acide phosphorique qs pH 9,9 | Jaguar HP 60 (Solvay Novecare) | glycérine | eau |
|---|---|---|---|---|---|---|
| A1 | | 2% | | | 6% | Qs 100% |
| B1 | | | 2,44% | 0,2% | 6% | Qs 100% |
| C1 | 5% | | | 0,2% | 6% | Qs 100% |

Les pH et viscosités sont :

| | |
|---|---|
| A1 : pH=2, 84 | Viscosité =0,65 Pa.s |
| B1 : pH=9,90 | Viscosité=0,044 Pa.s |
| C1 : pH=6,80 | Viscosité=0,04 Pa.s |

La viscosité des produits est mesurée à 1 atm et à 25°C avec un viscosimètre rotatif CONTRAVES TV équipé d'un mobile MS-r3 ou MS-r4 à la fréquence de 60 Hz, après 10 minutes de rotation du mobile

Le système de distribution a été testé avec :
- A1→Compartiment 1
- B1 →Compartiment 2
- C1 →Compartiment 3

### Test 1

Un premier mélange a été réalisé pour les proportions volumiques suivantes A :50%, B :0%, C :50%
On utilise le système pour délivrer 200 mg de produit. On étale alors le mélange très facilement (car très fluide, viscosité= 0,45 Pa.s).

### Test 2

Un second mélange a été réalisé pour A :30%, B :20%, C :40%
On utilise le système pour délivrer 200 mg de produit. On étale alors le mélange facilement en couche épaisse sur les zones à couvrir (car épaissi, viscosité= 1,9 Pa.s).

### Test 3

Un troisième mélange a été réalisé pour A :40%, B :40%, C :20%
On utilise le système pour délivrer 200 mg de produit. Le mélange est peu couvrant mais assez visqueux (viscosité=4,7 Pa.s) . Il est idéal pour les zones comme le front. Il ne coule pas à l'application et maintient un aspect naturel.

### Exemple 2(deuxième aspect del'invention)

On réalise la formule suivante Z :

| | |
|---|---|
| Carbopol 980 Polymer (Lubrizol) | 2% |
| Ammoniaque 10% dans l'eau | 2,44% |
| Eau qs | 100% |

| Produit de base | Oxyde de fer | Oxyde de titane | Carpobopol 980 Polymer (Lubrizol) | Ammoniaque 10% dans l'eau | Jaguar HP 60 | glycérine | eau |
|---|---|---|---|---|---|---|---|
| A1 | | 5% | 2% | 2,44 % | | 6% | Qs 100% |
| B1 | | 5% | 0,2% | 0,395 % | | 6% | Qs 100% |
| C1 | 5% | | | | 0,2% | 6% | Qs 100% |

Les pH et viscosités sont :

| | |
|---|---|
| A1 : pH=9,44 | Viscosité =7,8 Pa.s |
| B1 : pH=9,02 | Viscosité=0,047 Pa.s |
| C1 : pH=6,80 | Viscosité=0,04 Pa.s |

La viscosité des produits est mesurée à 1 atm et à 25°C avec un viscosimètre rotatif CONTRAVES TV équipé d'un mobile MS-r3 ou MS-r4 à la fréquence de 60 Hz, après 10 minutes de rotation du mobile

Le système a été testé avec :
- A1→Compartiment 1
- B1 →Compartiment 2
- C1 →Compartiment 3

### Test 1

Un premier mélange a été réalisé pour A :50%, B :0%, C :50%
Le résultat est beige et épais (viscosité=4,701 Pa.s), idéal pour la couvrance forte des zones de peau tachée.

### Test 2

Un second mélange a été réalisé pour A :20%, B :30%, C :50%
Le résultat est beige aussi mais beaucoup plus fluide (viscosité= 1,9 Pa.s) que dans le test 1. On étale alors le mélange facilement en couche épaisse sur les zones à couvrir (car épaissi).

### Test 3

Un troisième mélange a été réalisé pour A :40%, B :40%, C :20%
Le résultat est plus clair et de viscosité intermédiaire (viscosité=3,75 Pa.s) , idéal pour le traitement des cernes.

### C) Système pour obtention de films aux propriétés adaptées aux différents endroits du visage

La précision de la couleur obtenue est un facteur très important pour obtenir un résultat de qualité. Mais il faut aussi, pour obtenir les plus beaux résultats que le produit donne la meilleure qualité de film possible.

On entend par qualité de film, la qualité du dépôt après application sur la peau, puis séchage.

La bonne qualité du film n'est pas une notion simple. En effet, certaines qualités de film sont adaptées à certaines zones du visage et d'autres qualités à d'autres zones du visage.

On veut que les propriétés du film puissent être ajustées aux différentes parties du visage afin de pouvoir avec un même système réaliser le maquillage ou le soin de l'ensemble du visage. Il est connu que le traitement des lèvres exige une certaine souplesse et donc il faut que le film soit de l'ordre du huileux. Il est connu que le traitement du tour de l'oeil demande un film plutôt sec et tenseur. Il est connu que le traitement du fond demande un film non cohésif au sens qu'il ne forme pas un seul et même film après application. D'autres zones, moins mobiles, peuvent bénéficier de films résistants sans provoquer d'inconfort (par exemple, le front).

D'habitude, la solution consiste à utiliser une série de produits réalisés pour telle ou telle partie du visage. Cette approche, très répandue, pose le problème du nombre de références à prévoir pour couvrir tous les besoins en couleur.

Etant donné qu'on veut pouvoir faire varier les concentrations en ingrédients colorés (pour que l'utilisateur puisse avoir la ou les couleurs qui conviennent le mieux à chaque zone), il devient difficile de prévoir une seule qualité de film. Une possibilité est de proposer aux utilisateurs plusieurs systèmes permettant l'ajustement de couleurs montés avec des produits donnant pour certains un type de qualité de films et pour d'autres, d'autres qualités de film.

Mais cette approche n'est pas réaliste car nécessiterait que l'utilisateur ait plusieurs systèmes mélangeur.

Une autre possibilité est de proposer aux utilisateurs un seul système permettant l'ajustement de couleurs montés avec des produits donnant pour certains un type de qualité de films et pour d'autres, d'autres qualités de film. Dans ce cas, selon la zone à traiter, l'utilisateur monterait des produits ou d'autres. Mais cette approche n'est pas réaliste car nécessiterait que l'utilisateur change continuellement les cartouches de produit dans son système.

Une autre possibilité est de proposer aux utilisateurs un seul système permettant l'ajustement de couleurs montés avec toute une série de cartouches de produits de base donnant une variété de qualité de films. Dans ce cas, selon la zone à traiter, le système utiliserait tel ou tel produit sans que l'utilisateur n'ait besoin de changer les cartouches de produit dans les compartiments du système. Mais cette approche n'est pas réaliste car nécessiterait que le système ait de très nombreux compartiments, nécessitant une technologie complexe et onéreuse.

On cherche donc à proposer un système simple permettant de régler la concentration en ingrédients colorés et en qualité de film.

Ce système est spécialement intéressant pour traiter rapidement (sans avoir avoir à changer de systèmes ou de cartouches) toutes les zones du visage, en réglant sa couleur, et assurant les qualités de film adaptées.

Il permet aussi à l'utilisateur de tester ses couleurs et ses qualités de films jusqu'à avoir les meilleures performances en tant qu'effet visuel et confort.

L'invention a ainsi pour objet selon un troisième aspect un système de distribution d'un produit, comportant un distributeur recevant au moins deux cartouches contenant respectivement un premier produit de base et un deuxième produit de base, le distributeur permettant de délivrer au moins ces deux produits de base dans des proportions réglables pour former un film dont une propriété au moins varie en fonction du réglage.

Selon ce troisième aspect, l'invention peut présenter une ou plusieurs des caractéristiques préférentielles suivantes :
- la propriété du film qui varie est sa souplesse,
   la propriété du film qui varie est son caractère huileux,
- la propriété du film qui varie est son caractère tenseur,
- les deux produits de base sont choisis pour réagir ensemble afin de former un film.

le premier produit de base comporte une silicone réactive et le second produit de base un catalyseur provoquant la réticulation de la silicone réactive,
la silicone réactive comporte un mélange de polyorganosiloxane téléchélique portant une fonction vinyl aux deux extrémités de chaîne et de polyhydrogénosiloxane.
   - le catalyseur est à base de platine,
   - le premier produit de base comporte un polymère filmogène et le deuxième produit de base un composé huileux liquide à température ambiante,
le polymère filmogène présente une température de transition vitreuse Tg supérieure ou égale à 30°C, mieux supérieure ou égale à 60°C,
le polymère filmogène est choisi parmi les polymères ou copolymères vinyliques, notamment acryliques,
le polymère filmogène étant liposoluble,
les premier et deuxième produits de base contiennent des polymères filmogènes respectifs différents,
le polymère filmogène du premier produit de base ayant une température de transition vitreuse Tg1 et le polymère du deuxième produit de base ayant une température de transition vitreuse Tg2 différente de Tg1, avec de préférence Tg1>60°C et Tg2<10°C,
   - le premier produit de base comporte un polymère ou copolymère choisi parmi les polymères et copolymères méthacryliques, les polyamides, les alkycelluloses, les polymères et copolymères de la vinylpyrolidone, et les résines silicones,
   - le deuxième produit de base comporte un polymère ou copolymère choisi parmi les polymères et copolymères acryliques, vinyliques et les polycondensats tels que les polyesters et les polyuréthanes,
   - le premier produit de base comporte un composé huileux de température de fusion Tf1>20°C et le deuxième produit de base comportant un composé huileux de température de fusion Tf2<20°C.

Ce troisième aspect de l'invention concerne encore un procédé d'application d'un produit cosmétique, de maquillage et/ou de soin, sur les matières kératiniques humaines, comportant le réglage du distributeur en fonction de la zone à traiter, et la distribution du produit par prélèvement dans les cartouches des produits de base dans les proportions correspondant au réglage du distributeur.

L'invention se base ainsi selon son troisième aspect sur :
- Un système de distribution
- Au moins deux compartiments
- Ces compartiments comprennent des composés formant par contact un film et choisis de telles façon qu'en les associant, on obtient une gamme de qualité de film.

L'invention permet selon ce troisième aspect de traiter une ou plusieurs zones du visage et d'obtenir des mélanges très précis sur le plan de la fidélité des couleurs et un confort et une tenue adaptés permettant d'obtenir des effets particulièrement performants. Par la suite, on désigne par « zone » une partie du visage définie et assez réduite en surface, d'étendue comprise entre 1 cm2 et 100 cm2, mieux allant de 2 cm2 à 50 cm2.

Par la suite, on étend la notion de « maquillage » au-delà de l'apport de couleur ou de pouvoir masquant. Cette notion vaut ici pour tous traitements déposant un film sur la peau, coloré, masquant, ou non. Par exemple, la notion comprend l'application de film protecteur (avec filtres) ou traitant (avec actifs biologiques) ou tenseur.

On entend par qualité de film, les propriétés mécaniques du film. Bien que les propriétés mécaniques forment un continium, on peut découper les films en plusieurs classes de propriétés mécaniques :
1) Film huileux
2) Film souple
3) Film rigide
4) Film tenseur

Le système selon l'invention peut faire varier le film produit. Ainsi, selon les réglages choisis, les propriétés mécaniques du film ne sont pas les mêmes. Dans un mode préféré de l'invention, le système peut, selon les réglages, produire des films d'une catégorie ou d'une autre. (voire sur les trois catégories)
On aborde la qualité du film en appliquant un film de 400 µm d'épaisseur sur un support souple (feuille de papier sulfurisé), puis en le laissant sécher pendant 1 jour. A l'issue, on réalise les tests suivants pour qualifier le film :
Ainsi, on différencie plusieurs cas de figure :
1) Film huileux : Quand on plie le support avec un rayon de courbure de 1 cm, le film reste attaché à son support. Quand le film est frotté par un papier absorbant (Sopalin par ex) quelques secondes, on retrouve tout ou partie du film sur le papier absorbant.
2) Film souple : Quand on plie le support avec un rayon de courbure de 1 cm, le film reste attaché à son support. Quand le film est frotté par un papier absorbant (Sopalin par ex) quelques secondes, on ne note pas de transfert du film sur le papier absorbant.
3) Film rigide : Quand on plie le support avec un rayon de courbure de 1 cm, le film se détache au moins en partie de son support.
4) Les films tenseurs sont des cas particuliers provoquant un effet de rétractation du support lors du séchage.

Le système de distribution est de préférence tel que défini pour les deux premiers aspects de l'invention décrits plus haut.

### Système de distribution

Ainsi, le système de distribution peut être constitué selon le troisième aspect de l'invention, comme pour le premier et deuxième aspect de l'invention, par un seul appareil fonctionnant de manière autonome, de préférence manipulable d'une main, ou par un appareil qui fonctionne en interagissant avec d'autres pièces ou appareils. Il peut s'agir par exemple de différentes interfaces de sorties qui sont montées sur le distributeur en fonction du type de maquillage à réaliser, comme cela sera précisé plus loin. Il peut s'agir également d'un système informatique qui échange des informations avec le distributeur pour le piloter, ce système informatique comportant par exemple un terminal portable tel qu'un téléphone intelligent, un photophone, une tablette, un ordinateur personnel, ou un terminal dédié.

De préférence, le distributeur est agencé pour mettre sous pression un ou plusieurs compartiments contenant le ou les produits de base, par le biais de dispositifs de dosage volumétrique, de préférence un moteur entraînant en déplacement un piston dans le compartiment correspondant.

Le distributeur peut être formé d'un boîtier et d'au moins deux ou trois compartiments, et de préférence d'autant de moteurs. Par exemple, la rotation des moteurs entraîne des vis sans fin, lesquelles poussent les pistons de chaque compartiment. L'avance des pistons est par exemple contrôlée par le nombre d'impulsions de commande envoyées aux moteurs et/ou par la durée de fonctionnement de ces derniers. Les moteurs peuvent être alimentés séquentiellement, ou de préférence simultanément.

Par exemple, les moteurs sont alimentés durant un cycle élémentaire de fonctionnement pendant une courte durée les uns après les autres, ou les uns en même temps que les autres, de façon à distribuer des microdoses correspondantes.

Les cycles élémentaires sont répétés, avec éventuellement un temps d'arrêt entre eux, qui laisse le temps aux produits de base de s'écouler hors des compartiments.

Les compartiments peuvent être définis par des cartouches qui se retirent lorsqu'elles sont vides. En variante, les compartiments sont présents à demeure et remplis à nouveau une fois vide.

Chaque cartouche peut être fermée par un bouchon démontable pour permettre le nettoyage de la cartouche.

De préférence, le boîtier du distributeur est de forme allongée selon un axe longitudinal, ce qui peut faciliter sa manipulation, et les cartouches sont disposées autour de cet axe, dans le boîtier.

De préférence, les cartouches sont mises en place par l'arrière, et le mélange est délivré par l'avant. La mise en place des cartouches peut s'effectuer individuellement, ou en variante les cartouches constituent un ensemble monobloc lors de leur mise en place.

Les cartouches peuvent comporter chacune un mécanisme de dosage volumétrique, comportant un piston mu par un mécanisme d'entraînement du distributeur, dans une direction s'accompagnant d'une diminution du volume intérieur contenant le produit de base et d'une expulsion du produit. Il peut être intéressant que les cartouches aient une région de leur paroi au moins qui soit transparente, afin de permettre de voir la couleur du produit contenu à l'intérieur.

Le mécanisme d'entraînement peut comporter un système de motorisation formé de moteurs couplés à des réducteurs, de forme allongée parallèlement à l'axe longitudinal du distributeur, et disposés entre les cartouches. Ce positionnement des moteurs et cartouches rend le distributeur particulièrement compact.

Le produit de base peut sortir de façon étanche de la cartouche correspondante puis circuler dans un canal prévu à cet effet dans le boîtier du distributeur, avant de sortir de celui-ci.

Les cartouches se terminent avantageusement par un embout réalisé de telle sorte que, une fois la cartouche insérée dans le boîtier du distributeur, l'extrémité de l'embout débouche au ras du boîtier. En variante, l'embout est suffisamment long pour dépasser du boîtier et connecter ainsi différentes interfaces de sortie susceptibles d'être rapportées sur le boîtier du distributeur.

Grâce au mécanisme d'entraînement comportant des moteurs pour faire avancer des pistons, il est possible de délivrer de façon précise des mélanges en de très petites quantités. Ainsi, le mécanisme d'entraînement peut délivrer les produits de base avec à un débit minimal inférieur ou égal à 50 µL/s, mieux inférieur ou égal à 20 µL/s, encore mieux inférieur ou égal 10 µL/s. De préférence, le mécanisme d'entraînement délivre des débits compris entre 20 et 100 µL/s, mieux compris entre 40 µL/s et 60 µL/s. Il est donc possible de réaliser facilement un mélange d'environ 10 mg. Un tel système de distribution est alors idéal pour réaliser de petites touches de maquillage, pour couvrir par exemple une zone de 1 cm², mieux une zone de 0, 5 cm².

Il est aussi possible de réaliser des plus grandes quantités de mélange comme celles nécessaires au maquillage d'une joue, ou d'un visage. Ces quantités restent néanmoins relativement faibles, par exemple une quantité comprise entre 100 et 500 mg, mieux comprise entre 150 et 250 mg.

L'invention a ainsi pour objet, selon l'un de ses aspects, un système de distribution comportant un distributeur présentant un boîtier, et au moins une cartouche reçue dans le boîtier du ditributeur, cette cartouche comportant un corps et un piston mobile dans le corps, le boîtier comportant un mécanisme d'entraînement motorisé pour déplacer le piston de la cartouche.

De préférence, la cartouche comporte un embout de distribution par lequel s'effectue la sortie du produit, et cet embout de distribution est entraîné en rotation par le mécanisme d'entraînement pour déplacer le piston. L'embout peut comporter au moins un relief d' anti-rotation, mieux deux ergots d'anti-rotation diamétralement opposés.

L'embout peut porter un joint d'étanchéité, notamment un joint torique. Ainsi, au changement de cartouche, le joint est également changé, ce qui permet de s'affranchir de l'usure du joint.

Le distributeur peut comporter une carte électronique de pilotage du mécanisme d'entraînement motorisé, cette carte électronique étant traversée par le ou les embouts. Cela peut permettre de réaliser une carte s'étendant sensiblement sur toute la section du distributeur, donc de regrouper sur une seule carte tous les composants électroniques du distributeur, et ainsi de gagner en compacité et fiabilité. La carte peut s'étendre sensiblement perpendiculairement à l'axe longitudinal du boîtier. La carte peut porter un interrupteur de commande du fonctionnement du distributeur.

Le système de distribution peut être agencé pour fonctionner selon au moins deux modes de distribution.

Dans un premier mode, dit « continu », le mélange est distribué tant qu'une pression est exercée sur l'interrupteur de commande.

Dans un deuxième mode, dit « dose », une quantité prédéfinie du mélange est distribuée à chaque pression sur l'interrupteur.

Le ou les embouts peuvent déboucher à une extrémité du boîtier. Cela peut permettre de réduire le volume mort, comme cela sera détaillé dans la suite.

Le ou les embouts peuvent présenter à leur extrémité un système d'obturation pour éviter le séchage des produits dans le conduit, par exemple une membrane cicatrisante

La cartouche peut comporter une vis creuse sur laquelle le piston est vissé, le piston pouvant se déplacer axialement sur la vis lorsque celle-ci tourne ; le piston est empêché de tourner dans le corps de la cartouche. Par exemple, la friction du piston sur le corps de la cartouche peut suffire à l'empêcher de tourner quand la vis tourne.

Préférentiellement, la rotation est rendue impossible avec un corps de cartouche de section non circulaire et un piston non déformable.

Le couple des moteurs peut être déterminé électroniquement en fonction du courant absorbé, et servir par exemple à détecter une fin de course du piston. Une information concernant le couple peut être transmise à distance à un système informatique présentant une interface homme machine, pour permettre de surveiller le bon fonctionnement du distributeur.

Afin d'ajuster la teinte, le système de distribution selon l'invention doit permettre à l'utilisateur de faire varier le volume délivré provenant de chaque compartiment.

De façon préférée, le distributeur est piloté par un système informatique, qui est intégré au distributeur, ou externe à celui-ci, le distributeur étant alors apte à échanger avec le système informatique des informations par protocole sans fil ou câblé.

Le distributeur peut ainsi être piloté pour permettre l'ajustement de la teinte par distribution simultanée ou séquentielle, et contrôlée, de plusieurs produits de base de couleurs différentes.

La distribution des produits de base peut être continue ; si dans ce cas, les volumes de chacun des produits de base sont distribués d'une seule traite, simultanément ou successivement.

Dans le cas d'une distribution simultanée, il est utile de pouvoir ajuster les débits respectifs des différents produits de base pour que le mélange distribué corresponde à celui souhaité à chaque instant. Un tel mode de distribution peut convenir en particulier lors de la distribution du mélange par pulvérisation, à l'aide d'un aérographe. Pour ajuster les débits il est possible par exemple de jouer sur la vitesse de déplacement des pistons, par exemple, dans le cas d'un entrainement des pistons par une vis sans fin en faisant varier la vitesse de rotation des moteurs qui entrainent la vis. Les produits peuvent aussi être distribués de façon impulsionnelle avec un temps de distribution et un temps d'arrêt à chaque cycle. En jouant sur le rapport cyclique, il est possible d'agir sur le débit.

Tous les produits peuvent être délivrés simultanément lors du temps de distribution ; en variante, les cycles des différents produits sont déphasés, de sorte qu'un produit est distribué pendant un temps d'arrêt des autres produits.

Dans un mode particulier de l'invention, le mélange est délivré dans une cavité d'un contenant, pouvant fermer de façon hermétique ou non, par exemple en forme de coupelle, dans lequel on peut faire glisser un applicateur, en particulier un stylet ou une brosse. Un tel système de distribution est spécialement adapté aux liners, gloss et autres formules appliquées sans contact direct avec les mains. Ce contenant peut être amovible.

Par exemple, il sert de distributeur de rouge à lèvres et dispose d'un système de distribution, à vis par exemple. Lorsqu'il n'est pas amovible, le contenant peut être réalisé avec le corps du distributeur. Lorsqu'il est amovible, il peut constituer une interface de sortie parmi d'autres pouvant être montées sur le distributeur.

Les compartiments, et en particulier les cartouches, peuvent contenir tout ou partie du mécanisme d'entraînement, et par exemple le système de motorisation, ou mieux, une partie du système de motorisation, et ce, afin de réduire le nombre de pièces en mouvement dans le corps du distributeur à l'extérieur des cartouches. Par exemple, les cartouches comportent le rotor du moteur. Une fois les cartouches installées dans le corps du distributeur, les rotors sont mis en interaction avec les stators.

Le système de distribution est avantageusement agencé pour permettre de lancer des séquences préprogrammées dans lesquelles le mélange délivré par celui-ci est modifié de façon continue ou discontinue. Un mode « dégradé » permet par exemple passer progressivement d'un mélange A à un mélange B. Dans le cas d'une application par pulvérisation, notamment à l'aide d'un aérographe, cela permet de réaliser simplement les dégradés. Un mode « Alternatif » permet par exemple de passer rapidement d'un mélange

A à un mélange B plusieurs fois de suite. Dans le cas d'une application par pulvérisation, on peut ainsi réaliser un dépôt multicouche, avec des formulations différentes pour deux couches adjacentes superposées. Un autre mode permet par exemple de proposer plusieursmélanges successifs préprogrammés, le système informatique indiquant à chaque fois l'usage qu'il faut en faire à l'utilisateur, par exemple par affichage sur un écran.

Dans le cas d'une application manuelle, les mélanges sont distribués par exemple dans une coupelle. La personne se maquille à l'endroit recommandé avec un mélange correspondant prélevé dans la coupelle, puis nettoie éventuellement la coupelle et commande la délivrance d'un nouveau mélange ; l'opération est répétée autant de fois que nécessaire pour se maquiller complètement.

L'homogénisation des mélanges distribués peut se faire de différentes façons selon les cas d'utilisation. Dans le cas d'une application manuelle, elle peut se faire directement sur la zone d'application au moment de l'application ou dans la coupelle avant prélèvement ; dans le cas d'une application à l'aérographe, c'est la tuyère de l'aérographe qui sert de chambre de mélange ; dans le cas ou le mélange est distribué dans un contenanten vue d'une utilisation ultérieure, l'homogénéisation peut être faite à la main ou en faisant passer les produits distribués dans une chambre de mélange, située entre le distributeur et le contenant ou directement intégrée au contenant, comme détaillé par la suite.

Le produit peut être délivré par le système de distribution et utilisé extemporanément. En variante, le produit délivré par le système de distribution est conditionné et utilisé ultérieurement, par exemple à plusieurs reprises, avec par exemple un jour d'intervalle au moins entre deux utilisations.

### Exemple de mise en oeuvre du réglage de la qualité du film

Le compartiment 1 comporte une composition C1
Le compartiment 2 comporte une composition C2

Le contact entre C1 et C2, à différents ratios, induit différentes qualités de film.

### • Mise en oeuvre a :

On peut utiliser deux ingrédients aptes à réagir ensemble (dans deux compartiments séparés), et régler les proportions relatives.

Typiquement, il y a dans C1 un produit contenant une silicone réactive et dans C2 un produit contenant son catalyseur. Selon les réglages les propriétés du film seront différentes.

La «silicone réactive » peut être typiquement un mélange de polyorganosiloxane téléchélique portant une fonction vinyl aux deux extrémités de chaîne (disponible notamment dans la gamme Andisil VS ^{™} chez AB Specialty Silicones), et de polyhydrogénosiloxane (disponible notamment dans la gamme Andisil XL ^{™} chez AB Specialty Silicones).

Le « catalyseur » peut être typiquement un catalyseur au platine tel que la référence PT-50175F disponible chez Umicore.

### • Mise en œuvre b :

On peut aussi utiliser un polymère filmogène A et un composé huileux liquide à Tamb B. En plaçant A dans un produit destiné au compartiment 1 et B dans un produit destiné au compartiment 2, il est possible, en réglant les mélanges de faire varier la qualité de film finale.

Par « polymère filmogène », on entend un polymère apte à former à lui seul ou en présence d'un agent auxiliaire de filmification, un film macroscopiquement continu et adhérent sur les matières kératiniques, et de préférence un film cohésif, et mieux encore un film dont la cohésion et les propriétés mécaniques sont telles que ledit film peut être isolable et manipulable isolément, par exemple lorsque ledit film est réalisé par coulage sur une surface antiadhérente comme une surface téflonée ou siliconée.

Il est choisi de préférence parmi les polymères présentant une température de transition vitreuse (Tg) supérieure à 30°, de préférence supérieure à 60°C.

Parmi les polymères filmogènes utilisables dans la composition de la présente invention, on peut citer les polymères synthétiques, de type radicalaire ou de type polycondensat, les polymères d'origine naturelle, et leurs mélanges.

Par polymère filmogène radicalaire, on entend un polymère obtenu par polymérisation de monomères à insaturation notamment éthylénique, chaque monomère étant susceptible de s'homopolymériser (à l'inverse des polycondensats).

Les polymères filmogènes de type radicalaire peuvent être notamment des polymères, ou des copolymères, vinyliques, notamment des polymères acryliques.

Les polymères filmogènes vinyliques peuvent résulter de la polymérisation de monomères à insaturation éthylénique ayant au moins un groupement acide et/ou des esters de ces monomères acides et/ou des amides de ces monomères acides.

Comme monomère porteur de groupement acide, on peut utiliser des acides carboxyliques insaturés α,β-éthyléniques tels que l'acide acrylique, l'acide méthacrylique, l'acide crotonique, l'acide maléique, l'acide itaconique. On utilise de préférence l'acide (méth)acrylique et l'acide crotonique, et plus préférentiellement l'acide (méth)acrylique.

Les esters de monomères acides sont avantageusement choisis parmi les esters de l'acide (méth)acrylique (encore appelé les (méth)acrylates), notamment des (méth)acrylates d'alkyle, en particulier d'alkyle en C1-C30, de préférence en C1-C20, des (méth)acrylates d'aryle, en particulier d'aryle en C6-C10, des (méth)acrylates d'hydroxyalkyle, en particulier d'hydroxyalkyle en C2-C6 . Parmi les (méth)acrylates d'alkyle, on peut citer le méthacrylate de méthyle, le méthacrylate d'éthyle, le méthacrylate de butyle, le méthacrylate d'isobutyle, le méthacrylate d'éthyl-2 hexyle, le méthacrylate de lauryle, le méthacrylate de cyclohexyle.

Parmi les (méth)acrylates d'hydroxyalkyle, on peut citer l'acrylate d'hydroxyéthyle, l'acrylate de 2-hydroxypropyle, le méthacrylate d'hydroxyéthyle, le méthacrylate de 2-hydroxypropyle.

Parmi les (méth)acrylates d'aryle, on peut citer l'acrylate de benzyle et l'acrylate de phényle.

Les esters de l'acide (méth)acrylique particulièrement préférés sont les (méth)acrylates d'alkyle.

Comme amides des monomères acides, on peut par exemple citer les (méth)acrylamides, et notamment les N-alkyl (méth)acrylamides, en particulier d'alkyl en C2-C12. Parmi les N- alkyl (méth)acrylamides, on peut citer le N-éthyl acrylamide, le N-t-butyl acrylamide, le N-t- octyl acrylamide et le N-undécylacrylamide.

Les polymères filmogènes vinyliques peuvent également résulter de l'homopolymérisation ou de la copolymérisation de monomères choisis parmi les esters vinyliques et les monomères styrèniques. En particulier, ces monomères peuvent être polymérisés avec des monomères acides et/ou leurs esters et/ou leurs amides, tels que ceux mentionnés précédemment.

Comme exemple d'esters vinyliques, on peut citer l'acétate de vinyle, le néodécanoate de vinyle, le pivalate de vinyle, le benzoate de vinyle et le t-butyl benzoate de vinyle.

Comme monomères styrèniques, on peut citer le styrène et l'alpha-méthyl styrène.

Parmi les polycondensats filmogènes, on peut citer les polyuréthanes, les polyesters, les polyesters amides, les polyamides, et les résines époxyesters, les polyurées. Les polyuréthanes peuvent être choisis parmi les polyuréthanes anioniques, cationiques, nonioniques ou amphotères, les polyuréthanes-acryliques, les polyuréthanes-polyvinylpirrolidones, les polyester-polyuréthanes, les polyéther-polyuréthanes, les polyurées, les polyurée-polyuréthanes, et leurs mélanges.

Les polyesters peuvent être obtenus, de façon connue, par polycondensation d'acides dicarboxyliques avec des polyols, notamment des diols.

L'acide dicarboxylique peut être aliphatique, alicyclique ou aromatique. On peut citer comme exemple de tels acides : l'acide oxalique, l'acide malonique, l'acide diméthylmalonique, l'acide succinique, l'acide glutarique, l'acide adipique, l'acide pimélique, l'acide 2,2- diméthylglutarique, l'acide azélaïque, l'acide subérique, l'acide sébacique, l'acide fumarique, l'acide maléique, l'acide itaconique, l'acide phtalique, l'acide dodécanedioïque, l'acide 1,3- cyclohexanedicarboxylique, l'acide 1 ,4-cyclohexanedicarboxylique, l'acide isophtalique, l'acide téréphtalique, l'acide 2,5-norbornane dicarboxylique, l'acide diglycolique, l'acide thiodipropionique, l'acide 2,5-naphtalènedicarboxylique, l'acide 2,6-naphtalènedicarboxylique. Ces monomères acide dicarboxylique peuvent être utilisés seuls ou en combinaison d'au moins deux monomères acide dicarboxylique. Parmi ces monomères, on choisit préférentiellement l'acide phtalique, l'acide isophtalique, l'acide téréphtalique.

Le diol peut être choisi parmi les diols aliphatiques, alicycliques, aromatiques. On utilise de préférence un diol choisi parmi : l'éthylène glycol, le diéthylène glycol, le triéthylène glycol, le 1,3-propanediol, le cyclohexane diméthanol, le 4-butanediol. Comme autres polyols, on peut utiliser le glycérol, le pentaérythritol, le sorbitol, le triméthylol propane.

Les polyesters amides peuvent être obtenus de manière analogue aux polyesters, par polycondensation de diacides avec des diamines ou des amino alcools. Comme diamine, on peut utiliser l'éthylènediamine, l'hexaméthylènediamine, la meta- ou para-phénylènediamine. Comme aminoalcool, on peut utiliser la monoéthanolamine.

Le polyester peut en outre comprendre au moins un monomère portant au moins un groupement -SO3M, avec M représentant un atome d'hydrogène, un ion ammonium NH4+ ou un ion métallique, comme par exemple un ion Na+, Li+, K+, Mg2+, Ca2+, Cu2+, Fe2+, Fe3+. On peut utiliser notamment un monomère aromatique bifonctionnel comportant un tel groupement -SO3M.

Le noyau aromatique du monomère aromatique bifonctionnel portant en outre un groupement -SO3M tel que décrit ci-dessus peut être choisi par exemple parmi les noyaux benzène, naphtalène, anthracène, diphényl, oxydiphényl, sulfonyldiphényl, méthylènediphényl. On peut citer comme exemple de monomère aromatique bifonctionnel portant en outre un groupement -SO3M : l'acide sulfoisophtalique, l'acide sulfotéréphtalique, l'acide sulfophtalique, l'acide 4-sulfonaphtalène-2,7-dicarboxylique.

Selon un exemple de composition selon l'invention, le polymère filmogène peut être un polymère solubilisé dans une phase grasse liquide comprenant des huiles ou solvants organiques (on dit alors que le polymère filmogène est un polymère liposoluble). De préférence, la phase grasse liquide comprend une huile volatile, éventuellement en mélange avec une huile non volatile.

A titre d'exemple de polymère liposoluble, on peut citer les copolymères d'ester vinylique (le groupe vinylique étant directement relié à l'atome d'oxygène du groupe ester et l'ester vinylique ayant un radical hydrocarboné saturé, linéaire ou ramifié, de 1 à 19 atomes de carbone, lié au carbonyle du groupe ester ) et d'au moins un autre monomère qui peut être un ester vinylique (différent de l'ester vinylique déjà présent), une α-oléfine (ayant de 8 à 28 atomes de carbone), un alkylvinyléther (dont le groupe alkyl comporte de 2 à 18 atomes de carbone), ou un ester allylique ou méthallylique (ayant un radical hydrocarboné saturé, linéaire ou ramifié, de 1 à 19 atomes de carbone, lié au carbonyle du groupe ester).

Ces copolymères peuvent être réticulés à l'aide de réticulants qui peuvent être soit du type vinylique, soit du type allylique ou méthallylique, tels que le tétraallyloxyéthane, le divinylbenzène, l'octanedioate de divinyle, le dodécanedioate de divinyle, et l'octadécanedioate de divinyle.

Comme exemples de ces copolymères, on peut citer les copolymères : acétate de vinyle/stéarate d'allyle, l'acétate de vinyle/laurate de vinyle, acétate de vinyle/stéarate de vinyle, acétate de vinyle/octadécène, acétate de vinyle/octadécylvinyléther, propionate de vinyle/laurate d'allyle, propionate de vinyle/laurate de vinyle, stéarate de vinyle/octadécène- 1, acétate de vinyle/dodécène-1, stéarate de vinyle/éthylvinyléther, propionate de vinyle/cétyl vinyle éther, stéarate de vinyle/acétate d'allyle, diméthyl-2, 2 octanoate de vinyle/laurate de vinyle, diméthyl-2, 2 pentanoate d'allyle/laurate de vinyle, diméthyl propionate de vinyle/stéarate de vinyle, diméthyl propionate d'allyle/stéarate de vinyle, propionate de vinyle/stéarate de vinyle, réticulé avec 0,2 % de divinyl benzène, diméthyl propionate de vinyle/laurate de vinyle, réticulé avec 0,2 % de divinyl benzène, acétate de vinyle/octadécyl vinyl éther, réticulé avec 0,2 % de tétraallyloxyéthane, acétate de vinyle/stéarate d'allyle, réticulé avec 0,2 % de divinyl benzène, acétate de vinyle/octadécène-1 réticulé avec 0,2 % de divinyl benzène et propionate d'allyle/stéarate d'allyle réticulé avec 0,2 % de divinyl benzène.

Comme exemple de polymères filmogènes liposolubles, on peut citer les copolymères d'ester vinylique et au moins un autre monomère qui peut être un ester vinylique, notamment le néodécanoate de vinyle, le benzoate de vinyle et le t-butyl benzoate de vinyle, une α-oléfine, un alkylvinyléther, ou un ester allylique ou méthallylique.

Comme polymères filmogènes liposolubles, on peut également citer les copolymères liposolubles, et en particulier ceux résultant de copolymérisation d'esters vinyliques ayant de 9 à 22 atomes de carbone ou d'acrylates ou de méthacrylates d'alkyle, les radicaux alkyles ayant de 10 à 20 atomes de carbone.

De tels copolymères liposolubles peuvent être choisis parmi les copolymères de polystéarate de vinyle, de polystéarate de vinyle réticulé à l'aide de divinylbenzène, de diallyléther ou de phtalate de diallyle, les copolymères de poly(méth)acrylate de stéaryle, de polylaurate de vinyle, de poly(méth)acrylate de lauryle, ces poly(méth)acrylates pouvant être réticulés à l'aide de diméthacrylate de l'éthylène glycol ou de tétraéthylène glycol.

Les copolymères liposolubles définis précédemment sont connus et notamment décrits dans la demande FR-A-2232303 ; ils peuvent avoir un poids moléculaire moyen en poids allant de 2.000 à 500.000 et de préférence de 4.000 à 200.000.

Comme polymères filmogènes liposolubles utilisables dans l'invention, on peut également citer les polyalkylènes et notamment les copolymères d'alcènes en C2-C20, comme le polybutène, les alkylcelluloses avec un radical alkyle linéaire ou ramifié, saturé ou non en C1 à C8 comme l'éthylcellulose et la propylcellulose, les copolymères de la vinylpyrolidone (VP) et notamment les copolymères de la vinylpyrrolidone et d'alcène en C2 à C40 et mieux en C3 à C20. A titre d'exemple de copolymère de VP utilisable dans l'invention, on peut citer le copolymère de VP/acétate vinyle, VP/méthacrylate d'éthyle, la polyvinylpyrolidone (PVP) butylée, VP/méthacrylate d'éthyle/acide méthacrylique, VP/eicosène, VP/hexadécène, VP/triacontène, VP/styrène, VP/acide acrylique/méthacrylate de lauryle.

On peut également citer les résines de silicone, généralement solubles ou gonflables dans les huiles de silicone. La nomenclature des résines de silicone est connue sous le nom de "MDTQ", la résine étant décrite en fonction des différentes unités monomèriques siloxane qu'elle comprend, chacune des lettres "MDTQ" caractérisant un type d'unité. A titre d'exemples on peut citer les résines polymethylsilsesquioxanes, les résines polypropylsilsesquioxanes, et les résines trimethylsiloxysilicate (TMS).

Par « composé huileux liquide à Tamb », on entend une huile restant sur la peau à température ambiante et pression atmosphérique au moins plusieurs heures et ayant notamment une pression de vapeur inférieure à 0,13 Pa (0,01 mm de Hg).

Ces huiles non volatiles peuvent être des huiles hydrocarbonées notamment d'origine animale ou végétale, des huiles siliconées, ou leurs mélanges. On entend par "huile hydrocarbonée", une huile contenant principalement des atomes d'hydrogène et de carbone et éventuellement des atomes d'oxygène, d'azote, de soufre et/ou de phosphore.

Comme huile hydrocarbonée non volatile, on peut notamment citer :
- les huiles hydrocarbonées d'origine animale,
- les huiles hydrocarbonées d'origine végétale telles que les triglycérides constitués d'esters d'acides gras et de glycérol dont les acides gras peuvent avoir des longueurs de chaînes variées de C4 à C24, ces dernières pouvant être linéaires ou ramifiées, saturées ou insaturées; ces huiles sont notamment des triglycérides d'acide heptanoïque ou d'acide octanoïque, ou bien encore les huiles de germe de blé, de tournesol, de pépins de raisin, de sésame, de maïs, d'abricot, de ricin, de karité, d'avocat, d'olive, de soja, d'amande douce, de palme, de colza, de coton, de noisette, de macadamia, de jojoba, de luzerne, de pavot, de potimarron, de sésame, de courge, de colza, de cassis, d'onagre, de millet, d'orge, de quinoa, de seigle, de carthame, de bancoulier, de passiflore, de rosier muscat ; le beurre de karité ; ou encore les triglycérides des acides caprylique/caprique,
- les éthers de synthèse ayant de 10 à 40 atomes de carbone ;
- les hydrocarbures linéaires ou ramifiés, d'origine minérale ou synthétique tels que la vaseline, les polydécènes, le polyisobutène hydrogéné, le squalane, les huiles de paraffine, et leurs mélanges,
- les esters de synthèse comme les huiles de formule R1COOR2 dans laquelle R1 représente le reste d'un acide gras linéaire ou ramifié comportant de 1 à 40 atomes de carbone et R2 représente une chaîne hydrocarbonée notamment ramifiée contenant de 1 à 40 atomes de carbone à condition que R1 + R2 soit ≥ 10, comme par exemple l'huile de Purcellin (octanoate de cétostéaryle), le myristate d'isopropyle, le palmitate d'isopropyle, les benzoates d'alcools en C12 à C15, le laurate d'hexyle, l'adipate de diisopropyle, l'isononanoate d'isononyle, le néopentanoate d'isodecyl, le palmitate de 2-éthyl-hexyle, l'isostéarate d'isostéaryle, le laurate de 2-hexyl-décyle, le palmitate de 2-octyl-décyle, le myristate de 2-octyl-dodécyle, des heptanoates, octanoates, décanoates ou ricinoléates d'alcools ou de polyalcools comme le dioctanoate de propylène glycol ; les esters hydroxylés comme le lactate d'isostéaryle, le malate de di-isostéaryle, le lactate de 2-octyl-dodécyle ; les esters de polyols et les esters du pentaérythritol,
- les alcools gras liquides à température ambiante à chaîne carbonée ramifiée et/ou insaturée ayant de 12 à 26 atomes de carbone comme l'octyl dodécanol, l'alcool isostéarylique, l'alcool oléique, le 2-hexyldécanol, le 2-butyloctanol, et le 2-undécylpentadécanol,
- les acides gras supérieurs tels que l'acide oléique, l'acide linoléique, l'acide linolénique et leurs mélanges.

Les huiles de silicone non volatiles utilisables dans la composition selon l'invention peuvent être les polydiméthylsiloxanes (PDMS) non volatiles, les polydiméthylsiloxanes comportant des groupements alkyle ou alcoxy pendants et/ou en bouts de chaîne siliconée, groupements ayant chacun de 2 à 24 atomes de carbone, les silicones phénylées comme les phényl triméthicones, les phényl diméthicones, les phényl triméthylsiloxy diphénylsiloxanes, les diphényl diméthicones, les diphényl méthyldiphényl trisiloxanes, et leurs mélanges.

### • Mise en oeuvre c :

On peut aussi utiliser un polymère filmogène A et un polymère filmogène B. En plaçant A dans un produit destiné au compartiment 1 et B dans un produit destiné au compartiment 2, il est possible, en réglant les mélanges de faire varier la qualité de film finale.

Typiquement on utilise un polymère filmogène A de Tg élevée (typiquement >60°C) et un polymère filmogène B de Tg faible (typiquement <10°C), parmi ceux décrits précédemment.

Le polymère filmogène A de Tg élevée est choisi de préférence parmi les polymères ou copolymères méthacryliques, les polyamides, les alkylcelluloses comme l'éthylcellulose et la propylcellulose, les polymères et copolymères de la vinylpyrolidone, et les résines silicones.

Le polymère filmogène B de Tg faible est choisi de préférence parmi les polymères ou copolymères acryliques, les copolymères vinyliques, et les polycondensats tels que les polyesters et les polyuréthanes.

### • Mise en œuvre d :

On peut aussi utiliser un composé huileux de température de fusion > Tamb (cire) A et un composé huileux liquide à Tamb B. En plaçant A dans un produit destiné au compartiment 1 et B dans un produit destiné au compartiment 2, il est possible, en réglant les mélanges de faire varier la qualité de film finale.

Par « composé huileux de température de fusion > Tamb », on entend un corps gras solide à température ambiante.

On peut définir les corps gras pâteux à l'aide d'au moins une des propriétés physico-chimiques suivantes :
- une viscosité de 0,1 à 40 Pa.s (1 à 400 poises), mesurée à 40 °C avec un viscosimètre rotatif CONTRAVES TV équipé d'un mobile MS-r3 ou MS-r4 à la fréquence de 60 Hz,
- un point de fusion de 25-70 °C, de préférence 25-55 °C.

A titre de cires pouvant être utilisées selon l'invention, on peut citer :
- les cires d'origine animale telles que la cire d'abeilles, le spermaceti, la cire de lanoline et les dérivés de lanoline, les cires végétales telles que la cire de Carnauba, de Candellila, d'Ouricury, du Japon, le beurre de cacao ou les cires de fibres de liège ou de canne à sucre,
- les cires minérales, par exemple de paraffine, de vaseline, de lignite ou les cires microcristallines ou les ozokérites,
- les cires synthétiques parmi lesquelles les cires de polyéthylène, et les cires obtenues par synthèse de Fisher-Tropsch,
- les cires de silicone, en particulier les polysiloxanes linéaires substitués; on peut citer, par exemple, les cires de silicone polyéther, les alkyl ou alkoxydiméthicones ayant de 16 à 45 atomes de carbone, les alkyl méthicones,
- les huiles hydrogénées concrètes à 25°C telles que l'huile de ricin hydrogénée, l'huile de jojoba hydrogénée, l'huile de palme hydrogénée, le suif hydrogéné, l'huile de coco hydrogénée et les esters gras concrets à 25°C comme le stéarate d'alkyle en C20-C40,
- et/ou leurs mélanges.

De préférence, on utilisera les cires de polyéthylène, les cires microcristallines, les cires de carnauba, l'huile de jojoba hydrogénée, les cires de candellila, les cires d'abeilles et/ou leurs mélanges.

### Optimisation

Il est très intéressant d'avoir le moins de compartiments possibles.

Ainsi, si on veut que le système puisse faire un réglage de couleur (ce qui est le but principal), il faudrait prévoir 2 compartiments en plus des deux destinés au contrôle de la qualité du film. Ainsi, il faudrait prévoir :
- les compartiments 1 et 2 contiennent les ingrédients permettant de faire varier la qualité du film et
- Et d'autres compartiments (3, 4 par exemple), pour y placer les ingrédients M permettant de faire varier la couleur.

Il est aussi possible de mettre les actifs M dans les compartiments 1 ou 2 ou 1 et 2.

Ainsi, on réduit le nombre de compartiments totaux à 3, voire 2, au lieu de 4.

Par exemple, on met les actifs M dans les produits qu'on place dans les compartiments 1 et 2. Dans le compartiment 1, M est mis à concentration importante et dans le compartiment 2, M est mis à concentration faible.

On met aussi les actifs produisant le film et réglant ses propriétés de film dans les compartiments 1 et 2. On met une cire en émulsion dans le produit du compartiment 1 et une huile dans le produit du compartiment 2.

Si on utilise le système avec un réglage majoritaire en 1, on obtient un produit concentré en cire et en actif M.

Le réglage majoritaire en 1 peut alors être utilisé sur des zones de contour de l'oeil, où on désire une forte couvrance et un film sec.

Le réglage majoritaire en 2 peut être utilisé sur l'ensemble de la surface du visage en « produit de fond » notamment lorsqu'on a la peau sèche. Le peu de pigment assure un aspect naturel et l'aspect huileux du film ne produit pas de gêne.

Il est possible de mettre un actif M1 dans un produit destiné au compartiment 1 et un actif M2 dans un produit destiné au compartiment 2. Par exemple, M1 est un pigment jaune et M2 est un pigment rouge.

On met aussi les actifs produisant le film et réglant ses propriétés de film dans les compartiments 1 et 2. Par exemple, on met une silicone réactive en émulsion dans le produit du compartiment 1 et un catalyseur dans le produit du compartiment 2.

Si on utilise le système avec un réglage majoritaire en 1, on obtient un produit avec un ratio silicone réactive/catalyseur élevé et une forte concentration en pigment jaune

Le réglage majoritaire en 1 peut alors être utilisé sur le visage où la couleur jaune

est adaptée et le film huileux apporte une grande mobilité et donc confort.

Le réglage majoritaire en 2 peut être utilisé sur les lèvres où la couleur rouge est adaptée et la meilleure résistance du film, due à une réticuation plus importante, donnera une meilleure tenue.

Il est possible de mettre les actifs M dans un compartiment 3. Par exemple, on met un pigment jaune.

On met les actifs produisant le film et réglant ses propriétés de film dans les compartiments 1 et 2. Par exemple, on met une cire en émulsion dans le produit du compartiment 1 et une huile dans le produit du compartiment 2.
- Si on utilise le système avec un réglage 70/20/10, on obtiendra un film peu couvrant, mais adapté aux zones sous frottements comme par exemple le cou.
- Si on utilise le système avec un réglage 20/70/10, on obtiendra un résultat peu couvrant et très confortable, adapté aux grandes surfaces du visage.
- Si on utilise le système avec un réglage 30/10/60, on obtiendra un résultat très couvrant, adapté par exemple aux zones très tachées.
- Si on utilise le système avec un réglage 10/30/60, on obtiendra un résultat très couvrant et très confortable, adapté par exemple au maquillage des cils.

L'invention selon ce troisième aspect n'est pas limitée aux actifs amenant des effets coloriels. On peut l'utiliser pour des actifs de soin tels qu'antiâge, antioxydant, antiride, antitranspirant, antitache, photoprotection et hydratation.

On peut aussi mixer des actifs amenant des effets colorielles et des effets de soin.

### Utilisation pour le maquillage d'une ou plusieurs zones précises de peau

Le système de distribution permet de se maquiller jour après jour, en ne traitant que les zones à masquer. Pour ce faire, on délivre de petites doses de maquillage, qu'on applique précisément et séquentiellement sur les zones correspondantes. Chaque petite dose est réalisée selon le mélange qui convient à la zone.

Dans un mode préféré de mise en oeuvre de l'invention, le système de distribution attend qu'on le renseigne sur une zone à traiter puis délivre le mélange correspondant. Il peut faire appel à une table de correspondance préprogrammée pour cela, cette table résultant par exemple d'un procédé d'apprentissage tel que défini plus loin.

Dans un mode particulièrement intéressant, la programmation tient en compte la résistance et le confort qu'il doit atteindre pour obtenir les résultats optimums. En variante, le système de distribution renseigne la personne, tout en délivrant un mélange, sur la zone sur laquelle celle-ci doit appliquer le mélange. Ainsi, le système de distribution peut suivre un programme d'application où il délivre dans un ordre donné, les différentsmélanges à appliquer.

Dans un mode particulier de mise en oeuvre de l'invention, le système de distribution est renseigné sur les quantités à délivrer. Pour cela, il mémorise la correspondance entre la couleur, la zone du visage et la quantité nécessaire, ce qui permet de réduire les coûts et les pertes de produit, et de ne couvrir que faiblement la peau, évitant ainsi des effets d'occlusion. Ce faisant, il est possible d'utiliser des produits très couvrants et trop couvrants pour être appliqués sur l'ensemble du visage. Ainsi, il est possible d'obtenir des maquillages d'aspects naturels, voire indétectables.

Le système de distribution peut aussi permettre, en facilitant la distribution de petites quantités et leur usage rapide, de réduire la durée de conservation des produits, permettant ainsi de réduire les risques d'évolution et/ou les quantités de conservateurs à employer.

Le système de distribution est adapté à traiter les zones qu'on veut masquer, sans avoir à masquer l'ensemble du visage.

Lorsque l'utilisateur recherche la couleur à appliquer sur une zone du visage, il est intéressant de mémoriser la couleur qui va bien pour chaque zone, et le système de distribution est ainsi avantageusement agencé pour mémoriser cette couleur et la zone correspondante. Ainsi, en utilisant les informations mémorisées, à chaque utilisation, on peut obtenir la délivrance du même mélange pour chaque zone, ou, si l'on traite plusieurs zones, la même série de mélanges pour une même série de zones.

Le système de distribution peut aussi être agencé pour permettre de traiter une zone en faisant varier les couleurs application après application. Ainsi, la personne peut maquiller les lèvres avec différentes couleurs, qu'elle choisit au jour le jour selon sesgoûts. Cette approche est aussi adaptée aux paupières ou aux cils, ainsi qu'au maquillage du visage car la personne peut avoir envie de changer de couleur de teint. Par exemple, les jours de la semaine, la personne réalise un fond de teint couleur claire, et le weekend, un fond de teint de couleur hâlée, ou un maquillage des yeux d'une couleur un jour, et d'une autre couleur un autre jour.

Le système de distribution peut être agencé pour permettre à l'utilisateur de changer de couleur selon ses goûts du jour, du moment, de ses tenues, du climat. Ainsi, on prévoit avantageusement un système d'aide à la décision pour guider l'utilisateur dans ses choix de couleur.

On peut prévoir aussi un système d'aide pour équilibrer les couleurs sur un même visage et participer à la réalisation d'un maquillage global réussi.

Il peut être souhaitable que plusieurs personnes d'un même groupe, par exemple une famille, puissent utiliser le système de distribution, réduisant ainsi les coûts et minimisant la place occupée. Cette solution est spécialement adaptée aux voyages ou aux hôtels, campings, avions, campings cars, boutiques, écoles... Pour ce faire, on peut prévoir que le système de distribution puisse être renseigné sur la personne qui l'utilise, afind'accéder à des données personnelles préalablement mémorisées.

### Utilisation en continu pour un maquillage en dégradé

Dans cette application, le système de distribution, pendant qu'il délivre le produit, change la formulation du mélange. De plus, on déplace la sortie des produits de base ou du mélange relativement à un contenant ou un support définissant une surface d'application. Dans un mode particulier de mise en oeuvre de l'invention, le système de distribution est agencé pour calculer l'évolution du mélange en fonction de la couleur C1, d'une zone à traiter et de la couleur C2, d'une autre zone à traiter. Par exemple, sachant que le menton nécessite une couleur C1 et que la joue nécessite une couleur C2, le système de distribution peut faire varier la formulation du mélange pendant qu'il le délivre pouréaliser un dégradé de couleur entre ces deux couleurs. Cela permet par exemple de mieux masquer les imperfections du visage en faisant en sorte que le résultat final soit réaliste, ou de faire des dégradés de couleur à des fins d'embellissement. Le système de distribution peut encore être agencé de telle sorte que l'utilisateur puisse commander une variation de couleur du mélange distribué sans que les couleurs de départ et/ou d'arrivée n'aient été préalablement fixées. Pour ce faire, le système de distribution peut disposer d'un système de localisation, ou d'auto-localisation, et déduire d'une table de correspondance les couleurs C1 et C2 qu'il doit réaliser, et donc les évolutions de mélange qu'il doit réaliser.

Le système de distribution peut comporter une tête de sortie, en particulier dans le cas d'un aérographe, qui est mobile et pilotée. Cette option permet alors, sans bouger le reste du système de distribution, de réaliser des dégradés. Par exemple, on localise le système de distribution près de la joue, puis on déclenche un système de commande qui va piloter automatiquement la variation de la formulation du mélange et le mouvement de la tête de sortie, de façon à par exemple avoir le centre de la joue plus rouge que sa périphérie, avec un dégradé entre les deux.

Le système de distribution peut encore être utilisé pour réaliser des produits sur mesure, que l'on conserve pour réaliser plusieurs applications.

Il est aussi possible de réaliser des produits solides ou semi-solides.

### Fabrication de compacts « à façon » ou autres produits solides ou semi-solides

Le système de distribution peut être agencé pour permettre de choisir un mélange et de le délivrer dans un contenant tel qu'une coupelle. Le mélange comporte de préférence des composés qui sont tels que le mélange puisse prendre en masse.

Plus préférentiellement, on utilise des composés qui rendent la prise en masse spécialement rapide. Ces composés sont soit déposés dans le contenant avant ou après son remplissage avec les autres constituants, soit sont prévus dans les compartiments du distributeur avec les autres constituants des produits de base, soit sont compris dans le distributeur dans un compartiment spécialement prévu pour les contenir.

On peut ainsi distribuer des compositions spécifiques qui peuvent par réaction chimique, biochimique ou physicochimique, durcir de façon accélérée après évacuation.

Ces compositions sont spécialement adaptées à la réalisation des compacts, c'est-à-dire :
- Prennent en masse,
- donnent un matériau qui peut se déliter en cas de frottement, et sont préférentiellement colorées.

De préférence, ces compositions sont très riches en particules solides, avec par exemple plus de 10% en masse de particules solides par rapport à la masse totale de la composition, mieux plus de 20% en masse de particules solides par rapport à la masse totale de la composition, encore mieux plus de 30% en masse de particules solides par rapport à la masse totale de la composition, préférentiellement entre 10 et 40% en masse de particules solides par rapport à la masse totale de la composition.

Ces compositions peuvent contenir des particules absorbantes, ou des composés réactifs, tels que ceux qui réagissent en contact avec l'air, par exemple du cyanoacrylate ou des alphasilanes, ou qui réagissent à la lumière, notamment UV.

Le contenant dans lequel le mélange est distribué peut comporter un composé A et les compositions distribuées un composé B, A et B étant choisis pour réagir entre eux et solidifier le mélange.

Dans un mode particulier de mise en oeuvre de l'invention, le système de distribution intègre un moyen de chauffage, par exemple à résistance électrique, pour la réalisation de rouges à lèvres ou autres produits cireux. Dans ce cas, les produits de base sont chauffés avant d'être délivrés.

Le système de distribution peut aussi comporter un moyen pour apporter de l'énergie, thermique et/ou lumineuse, après distribution du mélange dans un contenant, par exemple une résistance électrique ou une diode LED, notamment UV. Cette énergie peut accélérer la prise en masse du mélange distribué.

Préférentiellement, le mélange est homogénéisé avant la prise en masse.

### Réalisation de palettes de couleurs

Le système de distribution peut comporter un support, ayant plusieurs régions, et être agencé pour générer automatiquement plusieurs mélanges déposés dans lesdites régions, par exemple une série de couleurs adaptées à différentes parties du visage.

Le support peut définir plusieurs cavités pour recevoir les mélanges ou porter plusieurs contenants, par exemple sous forme de coupelles, éventuellement séparables du support.

Dans un cas particulier, le support prend la forme d'un visage avec des régions pour recevoir les mélanges pour des zones d'applications ciblées.

Le support être mobile, notamment rotatif, par rapport au corps du distributeur, et par exemple entraîné en déplacement par le distributeur, pour remplir successivement différents logements ou contenants.

### Distributeur à coupelle

Il existe un intérêt pour bénéficier d'un système de distribution capable de délivrer un mélange pouvant être facilement prélevé par l'utilisateur. Par ailleurs, dans le cas où les produits de base délivrés par le système de distribution ne sont pas déjà mélangés, il existe un besoin pour permettre à l'utilisateur d'effectuer aisément le mélange.

Le système de distribution comporte de préférence une coupelle, et un distributeur pour remplir la coupelle avec au moins un produit, la coupelle étant solidaire du distributeur au moins pendant son remplissage.

La coupelle est parfois encore appelée « creuset » et ce terme doit être compris avec une acceptation large.

Par « coupelle solidaire du distributeur » il faut comprendre que la coupelle est retenue, notamment immobilisée, au moins temporairement sur le distributeur, étant par exemple fixée sur celui-ci par des vis, par aimantation, par encliquetage, par verrouillage baïonnette, par serrage, ou réalisée avec une partie du corps du distributeur par moulage de matière. La coupelle lorsqu'elle est solidaire du distributeur permet de manipuler celui-ci d'une main, la coupelle restant en place sur le distributeur au cours des mouvements de ce dernier.

Le distributeur peut être proposé à l'utilisateur avec la coupelle déjà en place.

En variante, celle-ci est installée par l'utilisateur à la première utilisation du système de distribution.

De préférence la coupelle est moins profonde que large, ce qui facilite l'accès à celle-ci, et permet de prélever le produit, notamment le mélange avec un applicateur ou le doigt.

De préférence, la coupelle est séparable du distributeur, et constitue une interface de sortie qui peut être choisie parmi un ensemble d'interfaces de sortie pouvant être montées sur le distributeur, au choix de l'utilisateur en fonction du maquillage à réaliser, comme détaillé plus loin.

De préférence, le système de distribution comporte plusieurs orifices de remplissage en des produits de base différents, débouchant dans la coupelle. Ainsi, le mélange de ces produits peut avoir lieu dans la coupelle.

La coupelle a de préférence un fond de forme concave vers l'extérieur, ce qui peut faciliter son nettoyage par l'utilisateur entre deux utilisations.

De plus, cela peut faciliter le prélèvement du produit par l'utilisateur, et le mélange des produits de base.

De préférence, le distributeur permet de délivrer au moins deux produits de base dans la coupelle, dans des proportions réglables, et mieux au moins trois produits.

Dans un exemple de mise en oeuvre, le système de distribution comporte au moins deux coupelles pouvant être sélectivement alimentées par le distributeur. Cela peut permettre à l'utilisateur de remplir rapidement ces deux coupelles avec des mélanges dont les caractéristiques sont différentes. Cela peut faciliter les essais de matières colorées, et/ou permettre de préparer plusieurs mélanges de couleurs différentes destinées au maquillage de zones respectives du visage. Les coupelles peuvent être associées à des identifiants rappelant à l'utilisateur à quelle zone du visage un mélange contenu dans une coupelle donnée est destiné.

Les coupelles peuvent être mobiles relativement au distributeur, étant par exemple portées par un support mobile, tel qu'une tourelle mobile en rotation par rapport au distributeur, ou par un tiroir mobile en translation par rapport au distributeur.

Le système de distribution peut comporter un couvercle de fermeture de la coupelle. Ce couvercle de fermeture est de préférence transparent pour permettre à l'utilisateur de visualiser la couleur du mélange contenu à l'intérieur.

Lorsque la coupelle est séparable du distributeur, elle peut être introduite le cas échéant dans un boîtier permettant de la transporter plus facilement, ce boîtier pouvant comporter le cas échéant un miroir et/ou un applicateur. Le couvercle du boîtier peut dans ce cas servir de couvercle pour la coupelle.

Le volume de la coupelle peut être compris entre 2 et 1000 mm³, mieux entre 100 et 1000 mm³, encore mieux entre 250 et 750 mm³.

Le ou les produits de base délivrés dans la coupelle sont de préférence des fonds de teint, mais en variante il s'agit de produits de maquillage des lèvres ou des paupières.

La coupelle est de préférence de forme symétrique de révolution. En variante, elle est de contour polygonal ou autre. Son plus grand diamètre intérieur, ou celui du cercle inscrit dans le cas d'un contour non circulaire, est de préférence compris entre 2 et 100 mm, préférentiellement entre 5 et 40 mm. Sa profondeur est de préférence comprise entre 1 et 10 mm, mieux entre 3 et 8 mm. De préférence, la taille et la forme de la coupelle permettent soit une application directe du mélange sur la peau, soit une préhension au doigt ou avec un applicateur. La coupelle peut être réalisée avec un matériau élastiquement déformable, ce qui permet par exemple d'inverser la concavité du fond de la coupelle et de vider celle-ci plus facilement ou de l'utiliser pour appliquer le produit.

La coupelle peut être dépourvue de mélangeur; dans ce cas, les produits de base peuvent arriver du distributeur dans la coupelle à l'état non mélangé, par des orifices de distribution respectifs distincts. En variante, le distributeur intègre un mélangeur et les produits de base arrivent déjà mélangés dans la coupelle.

La coupelle peut aussi intégrer un mélangeur statique, comme détaillé plus loin, qui est alimenté par des orifices de remplissage distincts du distributeur, et qui délivre de préférence le mélange dans une cavité de la coupelle située au-dessus du mélangeur.

L'invention a encore pour objet un procédé de préparation d'un produit de maquillage, comportant l'étape consistant à remplir une coupelle d'un système de distribution tel que défini plus haut avec au moins un produit de base provenant du distributeur.

Plusieurs produits peuvent être délivrés dans le fond de la coupelle, puis mélangés à l'aide du doigt ou d'un applicateur, ou d'un mélangeur statique intégré à la coupelle.

La coupelle est de préférence remplie par le dessous. Il a été proposé dans le passé des systèmes de distribution à l'aide d'une sonotrode.

La coupelle selon l'invention n'est pas destinée à entrer en vibration pour distribuer le ou les produits amenés par le ou les canaux d'alimentation de celle-ci. Elle est différente d'une sonotrode. De préférence, la coupelle est réalisée en matière plastique.

### Mélangeur intégré à l'interface de sortie

Il existe un intérêt pour bénéficier d'un système de distribution capable de délivrer un mélange pouvant être facilement utilisé, notamment prélevé par l'utilisateur, sans nécessiter une action additionnelle de mélange de la part de celui-ci.

Le système de distribution peut comporter un distributeur ayant des canaux de sortie de produits de base et une interface de sortie séparable du distributeur, cette interface ayant un mélangeur statique, délivrant de préférence le mélange dans une cavité où il peut être prélevé.

Le mélangeur statique peut être situé sous la cavité précitée. Le système de distribution est alors particulièrement adapté à la création de compacts, en utilisant comme interfaces de sortie des coupelles à mélangeur statique intégré. Dans ce cas, on vient remplir la cavité de la coupelle en produit par le dessous. Après passage dans le mélangeur statique, les produits de base mélangés viennent recouvrir le mélangeur.

Il est possible d'utiliser plusieurs interfaces de sortie et de les remplir avec des mélanges respectifs différents, sans avoir à purger le mélangeur, ce qui réduit les pertes de produit. L'interface de sortie peut être à usage unique, le cas échéant.

De préférence, le mélangeur statique comporte une chambre centrale communiquant avec des canaux d'admission des produits de base. Cette chambre centrale peut communiquer avec une chambre périphérique comportant une série de cloisons qui agissent comme des déflecteurs pour le mélange, et créent un cisaillement de celui-ci.

La chambre périphérique peut comporter une cloison annulaire ajourée définissant des ajours par lesquels passe le mélange en circulant dans la chambre périphérique. Les chambres centrale et périphérique peuvent être fermées supérieurement par une paroi qui définit le fond de la cavité recevant le mélange.

Le fond de la chambre périphérique peut être de forme hélicoïdale autour de l'axe de la coupelle et de hauteur diminuant au fur et à mesure que l'on progresse vers la sortie. Cette dernière peut déboucher en avant d'une rampe de liaison entre le fond de la chambre périphérique et la paroi supérieure du mélangeur, cette rampe de liaison étant de préférence une portion d'hélice prolongeant l'hélice formée par la paroi de fond de la chambre périphérique.

De préférence la chambre périphérique comporte la cloison annulaire précitée et des cloisons radiales qui forcent le mélange à circuler alternativement entre des régions supérieure et inférieure de la chambre périphérique et entre des régions radialement intérieure et extérieure, le mélange circulant par exemple d'une région supérieure et radialement extérieure à une région inférieure et radialement extérieure en passant à travers la cloison annulaire précitée.

Le mélangeur peut comporter un corps extérieur dans lequel est reçue une pièce formant le coeur du mélangeur, le corps extérieur fermant radialement à l'extérieur la chambre périphérique et comportant un montant qui sépare les chambres centrale et périphérique.

Le corps extérieur du mélangeur et le coeur du mélangeur peuvent être chacun réalisés d'une seule pièce par moulage par injection.

### Volume mort réduit

Il existe un intérêt pour réduire les pertes de produit lors des changements de formulation du mélange, ainsi que pour permettre de faire varier le plus rapidement possible la couleur du mélange au cours de l'application, notamment en cas de couplage du distributeur avec un aérographe.

Le produit de base peut quitter la cartouche correspondante par un canal de sortie de la cartouche, ce canal de sortie débouchant à l'extérieur du distributeur ou à proximité de sa surface externe.

Le canal de sortie peut notamment déboucher dans une zone de prélèvement du mélange ou à proximité de celle-ci, notamment à moins de 5 mm, mieux à moins de 3 mm, mieux à moins de 1mm, mieux à fleur.

La section transversale du canal de sortie est par exemple comprise entre 1 et 3 mm².

Ainsi, chaque produit de base provenant d'une cartouche peut quitter le distributeur sans se mélanger avec un produit de base d'une autre cartouche et l'on minimise le volume mort qui ne peut être prélevé et susceptible d'accroître l'inertie du système. Le produit est plus rapidement disponible sans avoir à circuler dans des canaux spécifiques du boîtier du distributeur, évitant une étape fastidieuse de purge si changement de cartouche.

L'extérieur du distributeur peut être la zone de prélèvement du produit, notamment lorsque le distributeur est réalisé avec une coupelle non prévue pour être enlevée, dans laquelle le mélange est distribué, ou une zone du distributeur destinée au montage d'une interface de sortie amovible, qui définit la zone de prélèvement. Cette interface de sortie peut comporter une coupelle telle que définie ci-dessus. Cette zone de montage correspond par exemple à l'extrémité du boîtier du distributeur en l'absence de l'interface de sortie. La zone de montage peut être sensiblement plane et perpendiculaire à l'axe longitudinal du boîtier du distributeur.

Le distributeur peut comporter trois cartouches de produits de base.

Le distributeur peut comporter des logements pour recevoir les cartouches, lesquelles sont de préférence reçues de façon amovible dans le distributeur. Ce dernier peut comporter des passages pour des conduits des cartouches définissant les canaux de sortie.

La longueur de ces conduits est de préférence telle que les conduits se situent légèrement en retrait du fond ou à affleurement de la cavité servant au prélèvement du produit, ou en variante légèrement en retrait ou à affleurement de la face d'extrémité du boîtier du distributeur définissant la zone de montage.

Ces conduits des cartouches peuvent être des embouts servant à entraîner en déplacement des pistons au sein des cartocuhes, comme détaillé plus haut.

### Interfaces de sortie multiples

Il existe un besoin pour pouvoir, à l'aide d'un même système de distribution, réaliser facilement des maquillages différents, et pouvoir si on le souhaite maquiller deszones aussi différentes que la peau, les lèvres ou les cils ou sourcils.

Le système de distribution peut comporter un ensemble comportant un distributeur d'au moins un produit cosmétique, notamment de maquillage, et au moins deux interfaces de sortie pouvant se monter chacune de façon amovible sur le distributeur, ces interfaces de sortie permettant de recevoir le ou les produits délivrés par le distributeur, étant de préférence choisies parmiles suivantes :
- interface de sortie comportant un contenant, notamment une coupelle permettant un prélèvement du produit au doigt ou à l'aide d'un applicateur,
- interface de sortie permettant de délivrer le produit à un système de pulvérisation, notamment un aérographe,
- interface de sortie comportant plusieurs régions de réception du produit, mobile par rapport au distributeur,
- interface de sortie permettant de délivrer le produit à un embout de distribution.

De préférence, l'ensemble comporte au moins trois desdites interfaces de sortie, mieux les quatre interfaces de sortie.

Le distributeur peut comporter au moins deux produits de base différents et permettre de délivrer ceux-ci dans des proportions variables, et de préférence, le distributeur comporte trois produits de base différents et permet de délivrer ceux-ci dans des proportions variables.

Chaque interface de sortie peut comporter une base permettant sa fixation sur le distributeur. Cette fixation peut s'effectuer à l'aide de vis par exemple, mais de préférence la base est agencée pour permettre un démontage et un remplacement d'une interface de sortie sans nécessité d'outil. Il s'agit par exemple d'une fixation quart de tour ou à l'aide d'une bague de verrouillage externe.

L'interface de sortie et/ou le boîtier du distributeur peuvent comporter des joints d'étanchéité permettant une communication étanche entre le boîtier du distributeur et l'interface de sortie. Le cas échéant, le distributeur est agencé pour reconnaître l'interface de sortie qui est montée dessus, par exemple grâce à identifiant de l'interface de sortie sous forme de reliefs spécifiques, lesquels sont détectés par le distributeur, ou d'une puce électronique reconnue par le distributeur. Cela peut permettre d'adapter le fonctionnement du distributeur à l'interface de sortie montée dessus. Le distributeur peut communiquer à un système informatique des informations sur l'interface de sortie qu'il porte, et le système informatique peut afficher en fonction de ces informations un écran spécifique et/ou lancer un programme spécifique de commande des paramètres de fonctionnement du distributeur, afin par exemple d'adapter la dose distribuée et/ou le débit à la nature de l'interface de sortie.

Plusieurs interfaces de sortie peuvent être proposées initialement à l'utilisateur avec un distributeur commun au sein d'un même emballage, par exemple un coffret ou une boîte de carton.

L'invention a encore pour objet un procédé de maquillage, comportant l'étape consistant à sélectionner une interface de sortie, à la monter sur le distributeur, et à délivrer le ou les produits contenus dans le distributeur dans celle-ci.

### Cartographie et apprentissage

Le terme « cartographie » s'entend ici comme un procédé d'indexation d'une couleur et d'une zone, avec enregistrement.

La cartographie peut concerner des applications sur des zones plus petites qu'un cm2. Toutefois, l'oeil nu a alors du mal à discerner si le résultat obtenu est adéquat, et il est préférable de substituer l'évaluation à l'oeil nu par une évaluation instrumentée, avec grossissement. L'application de petites quantités de matière colorée peut être faite au doigt, avec des outils classiques tels que des pinceaux, ou avec des applicateurs spécialisés.

La cartographie peut être générée lors d'une période d'apprentissage, pendant laquelle l'utilisateur effectue des tests avec des mélanges sur différentes zones du visage ; une fois renseignée, la cartographie peut ensuite être utilisée pour le maquillage de tous les jours. Cette période d'apprentissage peut tenir compte de la résistance et du confort. Ce faisant, l'utilisateur teste plusieurs rhéologies et renseignent le meilleur réglage obtenu.

Des interfaces graphiques spécifiques peuvent servir pendant la période d'apprentissage et pendant la période d'utilisation de la cartographie.

En particulier, le système de distribution peut être utilisé avec une interface graphique où l'opérateur voit le visage, qui est par exemple schématisé, figuratif ou précis comme une photograhie ou une simulation 3D. Dans ce cas, l'opérateur peut pointer une partie du visage sur l'écran pour voir apparaître et/ou délivrer la couleur adéquate.

L'interface graphique peut aussi laisser apparaître les autres zones du visage où l'utilisation de la même couleur est adéquate.

Pour réaliser la cartographie, l'opérateur applique une couleur puis réalise l'évaluation.

Les zones du visage peuvent être traitées les unes après les autres ; par exemple, on réalise l'exercice sur une partie de la joue, puis sur le nez, etc....

Une autre possibilité consiste à réaliser un mélange donné, et appliquer sur plusieurs zones ce même mélange. L'opérateur doit alors chercher la zone du visage pour laquelle la couleur est adaptée. Le mélange est ensuite indexé dans le système informatique en l'attribuant à la ou aux zones du visage pour lesquelles il convient.

Un procédé d'apprentissage d'un système de distribution selon l'invention, comportant un distributeur permettant de distribuer un mélange de couleur variable, et un système informatique permettant de sélectionner une couleur et de mémoriser des données, comporte les étapes consistant à :
a) Sélectionner au moins une couleur à l'aide d'une interface du système informatique,
b) délivrer à l'aide du distributeur au moins un mélange de la couleur sélectionnée,
c) évaluer le ou les mélanges distribués après application sur au moins une zone du visage,
d) mémoriser les caractéristiques d'au moins un mélange, notamment un mélange que l'utilisateur souhaite pouvoir rappeler, et d'au moins une zone sur laquelle il a été testé.

Cette mémorisation peut s'effectuer notamment en vue d'une distribution ultérieure de ce mélange pour maquiller ladite zone.

De préférence, le système informatique est agencé pour permettre à l'utilisateur de signaler si le résultat du test est satisfaisant ou non, voire de le renseigner sur la comparaison à un test effectué précédemment.

On peut aussi réaliser un mélange donné, et chercher la zone du visage pour laquelle il est adapté. On enregistre alors le mélange en l'attribuant à la ou aux zones du visage pour lesquelles il est adapté, dans une table de correspondance qui servira par la suite, pour, partant d'une zone de la peau, en déduire le mélange à utiliser.

On peut procéder de la même façon avec d'autres mélanges pour réaliser une cartographie de l'ensemble du visage et ainsi avoir une table de correspondance complète du visage.

On peut aussi réaliser un mélange donné, l'appliquer sur une zone donnée, puis faire varier le mélange jusqu'à obtenir le mélange le plus adapté. On enregistre alors le mélange en l'attribuant à la ou aux zones du visage pour lesquelles il convient, dans une table de correspondance qui servira par la suite, pour, partant d'une zone de la peau, en déduire le mélange à utiliser.

De préférence, le système informatique évalue et mémorise les quantités utilisées zone par zone. Un tel procédé, par « touches d'essai », permet d'identifier le ou les produits dont a besoin la personne qui veut se maquiller. Ainsi, le système de distribution peut servir sur les points de vente pour conseiller les personnes qui veulent se maquiller ou à la maison pour bien définir les produits à commander.

L'interface du système informatique comporte de préférence un écran tactile affichant la couleur du mélange lors de sa sélection.

L'interface peut afficher un visage et permettre de renseigner le système informatique en sélectionnant la zone sur le visage affiché.

Le système informatique est de préférence agencé pour permettre d'associer une zone, des paramètres de reconstitution du mélange, et la date de l'essai et/ou tout autre identifiant du mélange.

Le système informatique est de préférence également agencé pour permettre d'associer en outre à ladite zone, aux paramètres de reconstitution du mélange, et à la date ou à l'identifiant du mélange, au moins l'une des données suivantes : le nom de la zone, la période de l'année, le nom d'un évènement, un identifiant de l'utilisateur, l'âge de l'utilisateur.

Les étapes a) à c) peuvent être renouvelées au moins une fois avant la mémorisation des caractéristiques du mélange à l'étape d).

Le système informatique peut être agencé pour rechercher dans une base de données une référence de produit commerciale, sur la base des caractéristiques du mélange identifié comme convenant à au moins une zone donnée, et la communiquer à l'utilisateur.

La sélection à l'étape a) peut s'effectuer avec l'aide d'un système expert externe ou non au système informatique.

Le système expert peut analyser une image de l'utilisateur, pour proposer une couleur de mélange au moins sur la base de l'image analysée.

L'étape a) peut être précédée par la proposition d'une couleur et d'une zone à tester avec un mélange de cette couleur, par le système informatique à l'utilisateur.

Le système informatique peut être agencé pour permettre à l'utilisateur de lerenseigner sur l'appréciation du résultat du test de l'étape c) et pour générer uneproposition de modification du mélange à sélectionner lors du retour à l'étape a).

Le système informatique peut être agencé pour proposer au moins une couleur
de mélange à l'étape a) en fonction d'une zone d'application renseignée par l'utilisateur.

Le système informatique peut être agencé pour proposer au moins une zone d'application à l'étape a), en fonction d'une couleur renseignée par l'utilisateur.

Le distributeur peut délivrer à l'étape b) au moins deux mélanges de couleurs différentes, de préférence séparés, en vue de leur application simultanée sur la zone de test.

Cela peut faire gagner du temps, et faciliter la comparaison des résultats.

L'invention a encore pour objet un procédé de maquillage à l'aide d'un système de distribution selon cet aspect de l'invention, dans lequel :
a) L'utilisateur adresse une requête concernant un besoin de maquillage au système informatique,
b) celui-ci génère en retour une proposition de couleur en vue du maquillage d'une zone associée, sur la base de l'apprentissage effectué préalablement, et
c) le système informatique pilote le distributeur pour la production du mélange de la couleur proposée, notamment si celle-ci est validée par l'utilisateur.

Un tel procédé peut utiliser une cartographie précédemment établie avec l'utilisateur.

L'invention a encore pour objet un produit programme d'ordinateur comportant des instructions de code permettant, lorsque exécutées dans un système informatique, d'amener le système informatique à :
- Permettre à l'utilisateur de sélectionner au moins une couleur et/ou une zone d'application, notamment à l'aide d'une interface telle qu'un écran tactile,
- piloter un distributeur de façon à délivrer un mélange de la couleur sélectionnée par l'utilisateur,
- permettre à l'utilisateur de déclencher la mémorisation de la couleur du mélange et d'une zone d'application associée, notamment en vue d'une distribution ultérieure du même mélange, notamment sur la même zone.

Le produit programme d'ordinateur peut comporter des instructions de code permettant, lorsque exécutées dans un système informatique, d'amener le systèmeinformatique à:
- Recevoir une requête de l'utilisateur concernant un besoin de maquillage, notamment à l'aide d'une interface telle qu'un écran tactile,
- proposer sur la base au moins de données générées par le procédé d'apprentissage tel que défini ci-dessus, au moins une couleur et/ou une zone d'application,
- piloter un distributeur pour la production du mélange de la couleur proposée, notamment si celle-ci est validée par l'utilisateur.

### Aide à distance

Il est souhaitable de pouvoir aider l'utilisateur à se maquiller, notamment à choisir les bons coloris, grâce à un procédé de maquillage comportant les étapes consistant à :
- permettre l'établissement d'une liaison vidéo, par exemple via internet, entre une caméra d'un premier site et un deuxième site,
- permettre au deuxième site de piloter directement ou indirectement un distributeur présent sur le premier site, ce distributeur permettant de faire varier la couleur d'un mélange distribué,
- permettre à une personne présente au premier site d'appliquer le mélange distribué et d'envoyer vers le deuxième site une image correspondante, pour recevoir en retour une information relative au résultat de maquillage.

Le deuxième site peut notamment comporter un écran de visualisation qui permet à un conseiller présent devant cet écran de voir le résultat du maquillage avec le produit distribué par le distributeur et de conseiller la personne qui s'est maquillée. Ce conseiller peut agir en retour sur le distributeur pour modifier la couleur du mélange et l'adapter au mieux au visage de la personne présente sur le premier site. Ainsi, cette personne gouverne le mélange délivré par le distributeur. La première personne peut se maquiller sous les yeux de la seconde. La seconde personne voit sur son écran le résultat de l'essai, et ainsi peut corriger le mélange qu'elle va commander à distance, jusqu'à obtenir le maquillage idéal.

Le cas échéant, l'acquisition vidéo peut être calibrée à l'aide d'une mire, ou avec le mélange distribué par le distributeur sur une surface de référence. Cela permet ensuite une visualisation plus fidèle du maquillage effectué sur le premier site.

De préférence, la liaison vidéo entre les deux sites est une liaison duplex.

Le premier site peut recevoir un tutoriel du deuxième site, le cas échéant.

Des identifiants des produits de base peuvent être communiqués au deuxième site ; cela peut permettre de connaître précisément la couleur de chacun des produits de base.

Le procédé peut comporter la mémorisation des paramètres de réglage du distributeur, une fois qu'un mélange donné est estimé satisfaisant. De préférence, cette mémorisation peut être commandée depuis le deuxième site. La mémorisation peut avoir lieu dans le système informatique présent sur le premier site et/ou sur un serveur externe.

Une variante peut consister à faire travailler une personne pour aider le maquillage de plusieurs personnes. Ce mode de mise en oeuvre permet le développement des « Make-Up Artists » et leur travail soit au sein d'un institut, soit via internet. Cela permet aussi le maquillage de personnes aux moyens limités, telles que les personnes malvoyantes, ou discernant mal les couleurs, ou âgées, ou manquant de confiance en elles.

### Pilotage par interface tactile

Il existe un besoin pour faciliter le pilotage du système de distribution et notamment le choix de la couleur du mélange distribué.

Selon un autre de ses aspects, indépendamment ou en combinaison avec ses autres aspects, et notamment avec ce qui précède, l'invention a ainsi pour objet un système de distribution comportant un distributeur et un système informatique de pilotage du distributeur, ce système informatique comportant un écran tactile sur lequel peut être affichée la couleur du mélange et un moyen de sélection déplaçable sur l'écran, pour faire varier la couleur du mélange distribué.

De préférence, l'écran affiche des couleurs extrêmes entre lesquelles la couleur du mélange peut être sélectionnée, en déplaçant le moyen de sélection entre ces couleurs extrêmes.

L'écran peut afficher une échelle de couleurs entre au moins deux couleurs, ou une surface, notamment de contour triangulaire, au sein de laquelle le moyen de sélectionpeut se déplacer. Cette surface peut localement faire apparaître la couleur du mélange en fonction par exemple de la distance à chacun des sommets, matérialisant chacun un produit de base pur.

Le système informaitque peut effectuer une partie des calculs nécessaires pour déterminer les fractions de chacun des produits de base conduisant à un mélange de la couleur recherchée.

Le système informatique peut être un téléphone intelligent, un photophone, une tablette, un ordinateur individuel. En variante, il est intégré au boîtier du distributeur.

Le système informatique peut disposer d'une caméra. Cette dernière peut être utilisée notamment pour l'acquisition d'une image de l'utilisateur et/ou du mélange.

Le système informatique peut être agencé pour afficher une image d'un visage, afin de faciliter le repérage des zones sur lesquelles le mélange doit être appliqué.

### Couplage du système de distribution à un moyen de pulvérisation

Le système de distribution peut comporter ou être connecté à un moyen de pulvérisation du mélange, de préférence un aérographe.

L'invention a encore pour objet un ensemble comportant :
- Un moyen de pulvérisation, de préférence un aérographe comportant une chambre de prélèvement soumise à un courant d'air d'entraînement,
- un distributeur comportant au moins deux compartiments contenant des produits de base différents, les produits étant délivrés au moyen de pulvérisation de préférence par des orifices de distribution distincts.

Le distributeur peut comporter trois cartouches contenant des produits de maquillage de couleurs différentes.

L'aérographe peut comporter un stylet définissant la chambre de prélèvement, le stylet se fixant sur le distributeur ou sur une interface de sortie fixée au distributeur, ou faisant partie intégrante de cette interface de sortie.

Le système de distribution peut comporter un circuit de contrôle du fonctionnement du distributeur, permettant de faire varier la proportion des produits de base délivrés dans la chambre de prélèvement, au cours du fonctionnement de l'aérographe. Les proportions peuvent être modifiées en fonction du déplacement de l'aérographe relativement à la surface sur laquelle le mélange est pulvérisé. Ce déplacement peut être mécanisé, le cas échéant.

Ce circuit de contrôle peut comporter ou être constitué par un système informatique tel que défini plus haut.

Le boîtier du distributeur peut servir de poignée lors de la manipulation d l'ensemble pour délivrer le mélange.

Le distributeur peut comporter une caméra et/ou un ou plusieurs capteurs tels que des accéléromètres afin de localiser automatiquement la zone sur laquelle le mélange est appliquée, et pouvoir régler automatiquement la couleur en fonction de la position, le cas échéant.

L'invention a encore pour objet un procédé de maquillage à l'aide d'un ensemble tel que défini ci-dessus, dans lequel un mélange est pulvérisé sur la peau à l'aide du moyen de pulvérisation, notamment de l'aérographe.

La composition du mélange peut être modifiée au cours du déplacement de l'aérographe relativement à la peau. Un dégradé peut être réalisé.

Cet aspect de l'invention repose sur la constatation que le distributeur peut être utilisé pour alimenter le système de pulvérisation, notamment l'aérographe, tout en permettant au système de distribution d'être suffisamment réactif pour autoriser un changement de la couleur du mélange distribué en cours de maquillage du visage, notamment lorsque la zone à maquiller change.

Il peut être avantageux que la distribution des produits se fasse de façon itérative, notamment avec des temps de distribution non déphasés entre les différents produits.

Cela peut rendre plus facile de faire varier la composition du mélange distribué au cours du temps.

Le mélange peut être réalisé directement dans l'aérographe, sans pratiquement aucun volume mort gênant, permettant ainsi de changer le mélange pulvérisé en temps réel.

La dépression créée dans la chambre de prélèvement est suffisante pour entraîner les produits de base, sans pour autant gêner le dosage.

La dépression qui règne dans la chambre de prélèvement est par exemple comprise entre 10 mbar et 200 mbar, mieux entre 50 et 150 mbar, encore mieux entre 75 et 125 mbar.

La viscosité des produits de base mesurée à 1 atm et à 25°C avec un viscosimètre rotatif CONTRAVES TV équipé d'un mobile MS-r3 ou MS-r4 à la fréquence de 60 Hz, après 10 minutes de rotation du mobile, est par exemple comprise entre 0,05 Pa.S et 50 Pa.S

La section des canaux d'arrivée des produits de base dans la chambre est par exemple comprise entre 1 et 3 mm², mieux entre 2 et 3 mm².

L'alimention en produit s'effectue de préférence de façon continue.

Il est encore possible d'appliquer simultanément des produits de base non miscibles ou réactifs, comme un gel aqueux et un gel huileux, qui vont se déposer de façon pixellisée directement sur la peau, produisant une sorte de gel/gel in situ, des silicones réactives, ou des produits de coloration réagissant ensemble. Les ratios en produits de base peuvent être ajustés en fonction du résultat particulier souhaité. Par exemple, dans le cas des gels aqueux et gels huileux, on pourra faire varier le ratio correspondant au volume du premier produit de base sur le volume du deuxième produit de base entre 10/1 et 1/10, mieux entre 5/1 et 1/5.

### Système de localisation ou d'auto-localisation

Le système de distribution selon l'invention peut disposer d'un système de localisation ou d'auto-localisation.

On appelle système de localisation, un moyen de saisir, de la part de la personne, la zone qu'elle va traiter. Ceci peut être réalisé notamment par des systèmes laissant libre au moins une main. Ainsi, on peut utiliser une interface d'un système informatique telle qu'un écran tactile, un joystick, ou un système de reconnaissance vocale.

On appelle système d'auto-localisation, un moyen de saisir, sans que la personne n'intervienne, la zone à traiter. Ceci peut être réalisé par un ou plusieurs accéléromètres qui déduisent, par le fait des mouvements, les directions que la personne vise ou par une caméra et un système de reconnaissance d'image.

Un exemple de système de distribution pouvant être utilisé pour mettre en oeuvre l'invention selon ce troisième aspect a déjà été décrit en référence aux figures. Par conséquent, la description des figures n'est pas reprise ici.

### Exemple 1(troisième aspect de l'invention)

On réalise plusieurs produits de base (les proportions sont massiques)
A1 :

| | |
|---|---|
| Ultrahold strong de BASF (copolymère acrylate de Tg=80°C) | 10% |
| Carbopol Ultrez 10 (Lubrizol) * | 1% |
| Monoéthanolamine qs | pH 9 |
| Eau qs | 100% |

B1 :

| | |
|---|---|
| AQ 1350 (Eastman chemicals) (polyester à fonction sulfonique, Tg=0°C) | 10% |
| Carbopol Ultrez 10 (Lubrizol) * | 1% |
| Monoéthanolamine qs | pH 9 |
| Eau qs | 100% |

C1 :

| | |
|---|---|
| Pigments (oxyde de fer, dioxyde de titane 20/80) (polyester à fonction sulfonique, Tg=0°C) | 20% |
| Carbopol (Lubrizol) * | 1% |
| Monoéthanolamine qs | pH 9 |
| Eau qs | 100% |

| | |
|---|---|
| *Homopolymère carboxyvinylique réticulé synthétisé dans le mélange acétate d'éthyle/cyclohexane | |

### Test

### On réalise des mélanges :

On utilise le système pour délivrer des doses de 50 à200 mg de produit. On étale alors les mélanges petit à petit, doses par doses.
Proportions 50/10/40 pour maquiller les paumettes pour donner un effet coloré et brillant (500 mg)
20/40/40 pour les paupières pour leur donner de la couleur, un peu de brillance. La souplesse du film est suffisante pour ne pas créer d'inconfort dans cette partie sensible et souple du corps (100 mg en tout)
20/20/60 pour masquer des imperfections locales comme des taches foncées. (200 mg en tout)

### Exemple 2 (troisième aspect de l'invention)

On réalise plusieurs formules
A1 :

| | |
|---|---|
| Ultrahold strong de BASF * (Tg=80°C) | 10% |
| Jaguar HP 60 (Rhodia) | 1% |
| Monoéthanolamine qs | pH 9 |
| Eau qs | 100% |

| | |
|---|---|
| *Terpolymère acide acrylique/acrylate d'éthyle/n-tertiobutylacrylamide | |

B1 :

| | |
|---|---|
| AQ 1350 (Eastman chemicals) (polyester à fonction sulfonique, Tg=0°C) | 10% |
| Jaguar HP 60 (Rhodia) | 1% |
| Monoéthanolamine qs | pH 9 |
| Eau qs | 100% |

C1 :

| | |
|---|---|
| Mexoryl SX (filtre UVA) | 20% |
| Jaguar HP 60 (Rhodia) | 1% |
| Monoéthanolamine qs | pH 9 |
| Eau qs | 100% |

Le système est testé avec :
- A1→Compartiment 1
- B1 →Compartiment 2
- C1 →Compartiment 3

### Test

On réalise des mélanges :
On utilise le système pour délivrer des doses de 50 à200 mg de produit. On étale alors les mélanges petit à petit, doses par doses.
Proportions 50/10/40 pour protéger les grains de beauté (500 mg)
20/40/40 pour protéger l'ensemble du visage. La souplesse du film est suffisante pour ne pas créer d'inconfort dans cette partie sensible et souple du corps (100 mg en tout)
20/20/60 pour traiter et protéger le tour des yeux, évitant les risques d'apparaition de tache.

### D) Système de distribution permettant de réaliser des mélanges avec des compositions à forte teneur en solvant

Ce quatrième aspect de l'invention concerne plus particulièrement les procédés et systèmes de distribution, notamment d'un produit de maquillage, de soin, de parfumage et d'hygiène.

De nombreuses personnes souhaitent se maquiller pour embellir leur apparence, notamment leur visage. Elles veulent aussi pouvoir protéger leur peau pour en garder la belle apparence ou traiter la peau pour corriger des défauts.

Les motivations de ces personnes peuvent se ranger en deux catégories :
- Des traitements locaux (typiquement sur quelques centimètres carrés). Ce peut être pour masquer des taches, des rides ou des pores, protéger des grains de beauté de la lumière UV, traiter des rides ou des pores, ou amener des composés odorants.
- Des traitements sur de grandes surfaces (100 cm2 ou plus), notamment pour changer le rendu du visage par des changements de couleur ou réaliser une protection contre les rayonnements UV.

Dans ces différents cas, l'opération consiste à appliquer un produit contenant un ou plusieurs ingrédients et à en couvrir la peau ou une zone de peau.

On applique ainsi des compositions contenant des ingrédients insolubles (pigments pour le maquillage, poudres pour les effets antigras). Ces ingrédients peuvent être véhiculés dans de l'eau par exemple.

On applique aussi des ingrédients solubles dans le milieu. Dans ce cas, il y a deux cas de figure :
Dans le cas où les ingrédients sont solubles dans l'eau, on peut utiliser l'eau comme solvant.

Dans le cas où les ingrédients sont solubles dans les solvants organiques mais insolubles ou insuffisamment soluble dans l'eau on formule souvent dans une formulation contenant de l'eau, associant ces ingrédients avec des peptisants, tensioactifs ou molécules compatibilisantes. Les compositions sont à base d'eau ou eau+huile. Cette approche présente la limite de laisser des tensioactifs/peptisants sur la peau, amenant certains défauts comme des touchers collants.

Une autre approche est de les dissoudre dans un solvant organique.

### Approche I : dissolution des ingrédients dans un solvant organique lourd.

On peut les dissoudre dans un solvant organique lourd (à température d'ébullition important (>100°C)), comme de l'huile. Cette approche est souvent insatisfaisante car même si de nombreux travaux ont été réalisés pour trouver des solvants organiques peu impliquants (au sens de se faire oublier une fois appliqués), la limite de cette approche est qu'elle est peu confortable, tant pendant l'application (regraissage des mains) qu'après (impression de sentir une couche grasse sur la peau). De plus les couches réalisées perdent assez vite leur effet (ne serait ce que par usure ou évaporation). Il y a deux situations :
a) Si on applique ces compositions de façon globale, l'inconfort et l'élimination sont des problèmes importants. Pour réduire l'inconfort, le mieux est d'appliquer des quantités limitées de composition. Ceci augmente les risques d'élimination, ce qui induit un besoin d'appliquer à nouveau la composition.
b) Si on applique ces compositions de façon locale, c'est surtout le problème d'usure qui pose problème. En effet, si par un geste incontrôlé, on élimine le film des zones, on perd l'effet. (perte de pouvoir masquant/protecteur... dans le cas de compositions maquillage/photoprotection...).

Ainsi, il existe, dans les deux situations, un besoin d'appliquer de façon successive dans la journée les compositions et ce en petites quantités. En particulier pour le traitement local, comme les doses nécessaires peuvent varier dose par dose, il existe un besoin de pouvoir régler la quantité et/ou la qualité du ou des ingrédient(s). Ce besoin existe aussi pour les traitements globaux car application après application, car il faut pouvoir régler la quantité et/ou la qualité du ou des ingrédient(s). Par exemple, si on a appliqué un maquillage le matin, et qu'à midi, on veut réappliquer le maquillage, il faudra que la concentration en ingrédients masquants soit ajustée à la situation (plus ou moins en fonction de l'élimination).

### Approche II : dissolution des ingrédients dans un solvant organique léger.

On peut aussi utiliser un solvant plus léger (éthanol par exemple). Ces solvants laissent peu ou pas de traces ce qui est un avantage. La vitesse de séchage du solvant organique est un inconvénient, notamment pour les traitements globaux. Pour les traitements locaux, la vitesse de séchage n'est pas trop gênante car on applique la composition par petites touches. Pour les traitements globaux, la vitesse de séchage nécessite qu'on applique le produit par petite touche.

Dans les deux cas, il est intéressant de pouvoir faire varier la qualité et/ou quantité des ingrédients.
a) Pour les traitements globaux, comme la vitesse de séchage rend l'étalement impossible, il faut pouvoir faire varier les quantités d'ingrédients. En effet, on observe, quand on applique une composition de maquillage (donc avec ingrédients masquants), que certaines zones sont plus couvertes que d'autres. Cette irrégularité, gênante pour l'esthétique, doit être corrigée par l'application d'autres touches, dont la concentration en ingrédient devra être ajustée à la situation. Peu d'ingrédient masquant si l'irrégularité est faible. Forte concentration en ingrédient masquant si l'irrégularité est importante.
b) Pour les traitements locaux, il faut pouvoir régler la quantité et/ou qualité d'ingrédient car on veut i) ajuster ces paramètres à la situation de chaque zone du visage, ii) pouvoir appliquer les petites touches petit à petit. Par exemple, pour masquer une tache, on mettra une première touche contenant une forte concentration d'ingrédient masquant. Puis, à l'oeil ou avec un appareil, on estimera la quantité à ajouter. Ainsi, il faudra certainement faire une seconde application au même endroit, en mettant une couche de composition avec moins d'ingrédients masquants. Et ainsi de suite jusqu'à obtention d'un résultat parfait au point que la tache ne se voit plus.

Il existe donc aussi, dans le cas où on véhicule les ingrédients dans une composition à base de solvant organique léger, un besoin de pouvoir régler la quantité et/ou la qualité du ou des ingrédient(s) et d'appliquer par petites touches.

Cas particulier des compositions parfumantes ou rafraichissantes.

Là aussi, l'utilisateur veut pouvoir appliquer les compositions sur son visage. Soit par petites touches localisées (parfums), soit de façon plus globale (compositions rafraichissantes). Dans le premier cas, les ingrédients parfumants sont en général insolubles ou peu solubles dans l'eau. Il faut donc les dissoudre dans une base organique qui peut être un solvant lourd ou léger. Dans le second cas, la composition peut aussi contenir des ingrédients parfumants. Ainsi, les compositions sont en général des ingrédients parfumants dans l'éthanol (eaux de cologne). Il existe un besoin de faire varier les quantités d'ingrédients parfumants pour ajuster l'effet obtenu.

En résumé, il existe plusieurs besoins de mettre à disposition sous forme de petites quantités des compositions huileuses ou base de solvant léger, dont on peut contrôler la qualité et/ou la quantité en ingrédients (solubles ou particulaires ou solubles+particulaires) dans ces compositions.

On comprend que dans l'ensemble de ces situations, il faut que le système puisse délivrer les petites touches de composition (avec le bon réglage) dans des temps réduits. Typiquement quelques secondes ou moins. On comprend aussi que le système doit être facile à utiliser dans la journée et donc préférentiellement transportable. On comprend aussi que le système doit être facilement piloté, avec le moins d'actions humaines. Ainsi, le système doit pouvoir être programmé, en particulier programmé aux besoins personnels de l'utilisateur.

Ceci amène donc à considérer les systèmes formés de :
1) Un système de distribution
2) Plusieurs compartiments dans lesquels on place des cartouches contenant les compositions
3) Les compositions ne contiennent pas les mêmes quantités et/ou qualité d'ingrédients
4) Un système de pilotage

La réalisation d'un tel système pose des problèmes de précision. Sans précision suffisante les quantités délivrées et donc les mélanges sont parfois éloignées des quantités désirées. Plusieurs essais ont été réalisés avec différentes cartouches et on a réalisé des tests pour faire varier les quantités et/ou qualités d'ingrédients. On s'aperçoit que les réglages se font mal et les quantités et/ou qualités d'ingrédients obtenus sont éloignées de cibles.

L'invention a ainsi pour objet, selon un quatrième de ses apects, un système de distribution d'un produit cosmétique, comportant un distributeur recevant au moins deux cartouches ayant chacune un réservoir contenant un produit de base, un premier produit de base comportant au moins un solvant organique, la cartouche le contenant comportant un corps réalisé dans l'une au moins des matières thermoplastiques choisies dans la liste comprenant les polyoléfines simples, le polyvinylchloride (PVC), les polyamides et polyamides semi-aromatiques, le polysulfure de phénylene (PPS), le polybismaléimide, les polyuréthannes, les polyesters, les polyépoxydes, le polyéther bloc amide, le polyacétal, le polyéthercétone, les polyetherimides (PEI), les polyimides, le polyamideimide (PAI), les FEP (ethylène propylène perfluoré), PFA (Polyfluoroalkoxy), ECTFE (éthylène chloro trifluoro éthylène), et ETFE (éthylène tétrafluoroéthylène), et leurs mélanges, et de préférence les polyoléfines simples, le polyvinylchloride (PVC), les polyamides et polyamides semi-aromatiques, le polysulfure de phénylene (PPS), le polybismaléimide, et leurs mélanges.

Selon ce quatrième aspect de l'invention, celle-ci peut en outre présenter une ou plusieurs des caractéristiques suivantes :
- la cartouche comporte le premier produit de base et comporte outre le corps de la cartouche d'autres composants exposés au premier produit de base, l'un au moins de ces composants et de préférence tous ces composants étant choisis dans ladite liste ci-dessus.
   la teneur totale en solvant(s) organique(s) du premier produit de baseest supérieure ou égale à la teneur totale en eau du premier produit de base,
- la teneur totale en solvant(s) organique(s) du premier produit de base est supérieure ou égale à 50% par rapport à l'ensemble des solvants,

les cartouches sont identiques, abstraction faite de leur contenu et d'un éventuel identifiant,
   - l'une au moins des compositions comporte un parfum,
   - les cartouches sont reçues de façon amovible dans le distributeur,
chaque produit quitte la cartouche par un canal de sortie de la cartouche, le canal de sortie est défini par un embout de distribution de la cartouche entraîné en rotation relative par rapport à un corps de la cartouche par un mécanisme d'entraînement du distributeur pour distribuer le produit de base contenu dans la cartouche.

L'invention selon ce quatrième aspect repose ainsi sur l'utilisation d'un système de distribution avec cartouches, produits de base, et pilotage, les cartouches étant formées dans des matériaux particuliers. Ce faisant, les précisions sont bonnes.

L'invention selon cet aspect permet donc d'amener une solution à cette question de délivrance de petites quantités des compositions huileuses ou base de solvant léger, dont on peut contrôler la qualité et/ou la quantité en ingrédients (solubles ou particulaires ou solubles+particulaires). Ce faisant, on peut se maquiller en global ou sur des petites zones, on peut traiter des défauts (rides, pores, taches), appliquer des éléments protecteurs en global ou sur des zones précises et se parfumer. On peut aussi chercher les réglages de formule les plus adaptés. En effet, le système permet de délivrer de petites quantités et ce faisant, après application, on peut identifier si le réglage convient ou en chercher un nouveau. C'est donc un moyen de chercher ses réglages idéaux.

Outre l'avantage d'amener une solution à ces problématiques, on peut aussi profiter du système pour appliquer une huile ou un mélange d'huiles (donc cas où les huiles forment l'ingrédient) en particulier avec des huiles essentielles.

D'une manière générale, l'invention selon ce quatrième aspect sert à tous les cas de figure où le véhicule est riche en solvant organique, lourd et/ou léger, supérieur à 50% par rapport à l'ensemble des solvants et en particulier supérieur à 80% par rapport à l'eau des solvants. Par exemple, une composition parfumante comprenant 20% d'ingrédient odorant + 70% d'éthanol + 10% d'eau, est considérée à 87,5 % de solvant organique (70/80).

L'invention selon ces aspects peut traiter des cas où la composition contient, outre le ou les solvants organiques, des ingrédients solubles dans le solvant. Elle s'applique aussi aux compositions contenant outre le ou les solvants organiques, des ingrédients particulaires. (pigments, poudres, filtres).

On peut se retrouver dans une situation hybride où l'on utilise dans un même système plusieurs cartouches dont certaines seront avec compositions riches en solvant organiques et d'autres non. Ainsi, on peut employer le système :
1) Toutes les cartouches sont selon l'invention (avec matériaux sélectionnés)., qu'elles soient riches en solvant organique ou non.
2) Des cartouches selon l'invention (avec matériaux sélectionnés) pour les compositions riches en solvant organique et des cartouches hors invention pour des compositions qui ne sont pas riches en solvant organique.

La situation 1) est préférée car plus simple sur le plan de la logistique industrielle de fabrication et des récyclages.

L'invention permet de traiter une ou plusieurs zones. Par la suite, on désigne par « zone » une partie du visage définie et assez réduite en surface, d'étendue comprise entre 1 cm2 et 100 cm2, mieux allant de 2 cm2 à 50 cm2.

### Système de distribution

Le système de distribution selon cet aspect de l'invention peut être constitué par un seul appareil fonctionnant de manière autonome, de préférence manipulable d'une main, ou par un appareil qui fonctionne en interagissant avec d'autres pièces ou appareils. Il peut s'agir par exemple de différentes interfaces de sorties qui sont montées sur le distributeur en fonction du type d'effets à réaliser, comme cela sera précisé plus loin. Il peut s'agir également d'un système informatique qui échange des informations avec le distributeur pour le piloter, ce système informatique comportant par exemple un terminal portable tel qu'un téléphone intelligent, un photophone, une tablette, un ordinateur personnel, ou un terminal dédié.

De préférence, le distributeur est agencé pour mettre sous pression un ou plusieurs compartiments contenant le ou les produits de base, par le biais de dispositifs de dosage volumétrique, de préférence un moteur entraînant en déplacement un piston dans le compartiment correspondant.

Le distributeur peut être formé d'un boîtier et d'au moins deux ou trois compartiments, et de préférence d'autant de moteurs. Par exemple, la rotation des moteurs entraîne des vis sans fin, lesquelles poussent les pistons de chaque compartiment. L'avance des pistons est par exemple contrôlée par le nombre d'impulsions de commande envoyées aux moteurs et/ou par la durée de fonctionnement de ces derniers. Les moteurs peuvent être alimentés séquentiellement, ou de préférence simultanément.

Par exemple, les moteurs sont alimentés durant un cycle élémentaire de fonctionnement pendant une courte durée les uns après les autres, ou les uns en même temps que les autres, de façon à distribuer des microdoses correspondantes.

Les cycles élémentaires sont répétés, avec éventuellement un temps d'arrêt entre eux, qui laisse le temps aux produits de base de s'écouler hors des compartiments.

Les compartiments peuvent être définis par des cartouches qui se retirent lorsqu'elles sont vides. En variante, les compartiments sont présents à demeure et remplis à nouveau une fois vide.

Chaque cartouche peut être fermée par un bouchon démontable pour permettre le nettoyage de la cartouche.

De préférence, le boîtier du distributeur est de forme allongée selon un axe longitudinal, ce qui peut faciliter sa manipulation, et les cartouches sont disposées autour de cet axe, dans le boîtier.

De préférence, les cartouches sont mises en place par l'arrière, et le mélange est délivré par l'avant. La mise en place des cartouches peut s'effectuer individuellement, ou en variante les cartouches constituent un ensemble monobloc lors de leur mise en place.

Les cartouches peuvent comporter chacune un mécanisme de dosage volumétrique, comportant un piston mu par un mécanisme d'entraînement du distributeur, dans une direction s'accompagnant d'une diminution du volume intérieur contenant le produit de base et d'une expulsion du produit. Il peut être intéressant que les cartouches aient une région de leur paroi au moins qui soit transparente, afin de permettre de voir la couleur du produit contenu à l'intérieur.

Le mécanisme d'entraînement peut comporter un système de motorisation formé de moteurs couplés à des réducteurs, de forme allongée parallèlement à l'axe longitudinal du distributeur, et disposés entre les cartouches. Ce positionnement des moteurs et cartouches rend le distributeur particulièrement compact.

Le produit de base peut sortir de façon étanche de la cartouche correspondante puis circuler dans un canal prévu à cet effet dans le boîtier du distributeur, avant de sortir de celui-ci.

Les cartouches se terminent avantageusement par un embout réalisé de telle sorte que, une fois la cartouche insérée dans le boîtier du distributeur, l'extrémité de l'embout débouche au ras du boîtier. En variante, l'embout est suffisamment long pour dépasser du boîtier et connecter ainsi différentes interfaces de sortie susceptibles d'être rapportées sur le boîtier du distributeur.

Grâce au mécanisme d'entraînement comportant des moteurs pour faire avancer des pistons, il est possible de délivrer de façon précise des mélanges en de très petites quantités. Ainsi, le mécanisme d'entraînement peut délivrer les produits de base avec à un débit minimal inférieur ou égal à 50 µL/s, mieux inférieur ou égal à 20 µL/s, encore mieux inférieur ou égal 10 µL/s. De préférence, le mécanisme d'entraînement délivre des débits compris entre 20 et 100 µL/s, mieux compris entre 40 µL/s et 60 µL/s. Il est donc possible de réaliser facilement un mélange d'environ 10 mg. Un tel système de distribution est alors idéal pour réaliser de petites touches de maquillage, pour couvrir par exemple une zone de 1 cm², mieux une zone de 0, 5 cm².

Il est aussi possible de réaliser des plus grandes quantités de mélange comme celles nécessaires au maquillage d'une joue, ou d'un visage. Ces quantités restent néanmoins relativement faibles, par exemple une quantité comprise entre 100 et 500 mg, mieux comprise entre 150 et 250 mg.

L'invention a ainsi pour objet, selon l'un de ses aspects, un système de distribution comportant un distributeur présentant un boîtier, et au moins une cartouche reçue dans le boîtier du ditributeur, cette cartouche comportant un corps et un piston mobile dans le corps, le boîtier comportant un mécanisme d'entraînement motorisé pour déplacer le piston de la cartouche.

De préférence, la cartouche comporte un embout de distribution par lequel s'effectue la sortie du produit, et cet embout de distribution est entraîné en rotation par le mécanisme d'entraînement pour déplacer le piston. L'embout peut comporter au moins un relief d'anti-rotation, mieux deux ergots d'anti-rotation diamétralement opposés.

L'embout peut porter un joint d'étanchéité, notamment un joint torique. Ainsi, au changement de cartouche, le joint est également changé, ce qui permet de s'affranchir de l'usure du joint.

Le distributeur peut comporter une carte électronique de pilotage du mécanisme d'entraînement motorisé, cette carte électronique étant traversée par le ou les embouts. Cela peut permettre de réaliser une carte s'étendant sensiblement sur toute la section du distributeur, donc de regrouper sur une seule carte tous les composants électroniques du distributeur, et ainsi de gagner en compacité et fiabilité. La carte peut s'étendre sensiblement perpendiculairement à l'axe longitudinal du boîtier. La carte peut porter un interrupteur de commande du fonctionnement du distributeur.

Le système de distribution peut être agencé pour fonctionner selon au moins deux modes de distribution.

Dans un premier mode, dit « continu », le mélange est distribué tant qu'une pression est exercée sur l'interrupteur de commande.

Dans un deuxième mode, dit « dose », une quantité prédéfinie du mélange est distribuée à chaque pression sur l'interrupteur.

Le ou les embouts peuvent déboucher à une extrémité du boîtier. Cela peut permettre de réduire le volume mort, comme cela sera détaillé dans la suite.

Le ou les embouts peuvent présenter à leur extrémité un système d'obturation pour éviter le sechage des produits dans le conduit, par exemple une membrane cicatrisante
La cartouche peut comporter une vis creuse sur laquelle le piston est vissé, le piston pouvant se déplacer axialement sur la vis lorsque celle-ci tourne ; le piston est empêché de tourner dans le corps de la cartouche. Par exemple, la friction du piston sur le corps de la cartouche peut suffire à l'empêcher de tourner quand la vis tourne.

Préférentiellement, la rotation est rendue impossible avec un corps de cartouche de section non circulaire et un piston non déformable.

Le couple des moteurs peut être déterminé électroniquement en fonction du courant absorbé, et servir par exemple à détecter une fin de course du piston. Une information concernant le couple peut être transmise à distance à un système informatique présentant une interface homme machine, pour permettre de surveiller le bon fonctionnement du distributeur.

Afin d'ajuster l'effet, le système de distribution selon l'invention doit permettre à l'utilisateur de faire varier le volume délivré provenant de chaque compartiment.

De façon préférée, le distributeur est piloté par un système informatique, qui est intégré au distributeur, ou externe à celui-ci, le distributeur étant alors apte à échanger avec le système informatique des informations par protocole sans fil ou câblé.

Le distributeur peut ainsi être piloté pour permettre l'ajustement de l'effet par distribution simultanée ou séquentielle, et contrôlée, de plusieurs produits de base différentes.

La distribution des produits de base peut être continue ; si dans ce cas, les volumes de chacun des produits de base sont distribués d'une seule traite, simultanément ou successivement.

Dans le cas d'une distribution simultanée, il est utile de pouvoir ajuster les débits respectifs des différents produits de base pour que le mélange distribué corresponde à celui souhaité à chaque instant. Un tel mode de distribution peut convenir en particulier lors de la distribution du mélange par pulvérisation, à l'aide d'un aérographe. Pour ajuster les débits il est possible par exemple de jouer sur la vitesse de déplacement des pistons, par exemple, dans le cas d'un entrainement des pistons par une vis sans fin en faisant varier la vitesse de rotation des moteurs qui entrainent la vis. Les produits peuvent aussi être distribués de façon impulsionnelle avec un temps de distribution et un temps d'arrêt à chaque cycle. En jouant sur le rapport cyclique, il est possible d'agir sur le débit.

Tous les produits peuvent être délivrés simultanément lors du temps de distribution ; en variante, les cycles des différents produits sont déphasés, de sorte qu'un produit est distribué pendant un temps d'arrêt des autres produits.

Dans un mode particulier de l'invention, le mélange est délivré dans une cavité d'un contenant, pouvant fermer de façon hermétique ou non, par exemple en forme de coupelle, dans lequel on peut faire glisser un applicateur, en particulier un stylet ou une brosse. Un tel système de distribution est spécialement adapté aux liners, gloss et autres formules appliquées sans contact direct avec les mains. Ce contenant peut être amovible.

Par exemple, il sert de distributeur de rouge à lèvres et dispose d'un système de distribution, à vis par exemple. Lorsqu'il n'est pas amovible, le contenant peut être réalisé avec le corps du distributeur. Lorsqu'il est amovible, il peut constituer une interface de sortie parmi d'autres pouvant être montées sur le distributeur.

Les compartiments, et en particulier les cartouches, peuvent contenir tout ou partie du mécanisme d'entraînement, et par exemple le système de motorisation, ou mieux, une partie du système de motorisation, et ce, afin de réduire le nombre de pièces en mouvement dans le corps du distributeur à l'extérieur des cartouches. Par exemple, les cartouches comportent le rotor du moteur. Une fois les cartouches installées dans le corps du distributeur, les rotors sont mis en interaction avec les stators.

Le système de distribution est avantageusement agencé pour permettre de lancer des séquences préprogrammées dans lesquelles le mélange délivré par celui-ci est modifié de façon continue ou discontinue. Un mode « dégradé » permet par exemple passer progressivement d'un mélange A à un mélange B. Dans le cas d'une application par pulvérisation, notamment à l'aide d'un aérographe, cela permet de réaliser simplement les dégradés. Un mode « Alternatif » permet par exemple de passer rapidement d'un mélange A à un mélange B plusieurs fois de suite. Dans le cas d'une application par pulvérisation, on peut ainsi réaliser un dépôt multicouche, avec des formulations différentes pour deux couches adjacentes superposées. Un autre mode permet par exemple de proposer plusieurs mélanges successifs préprogrammés, le système informatique indiquant à chaque fois l'usage qu'il faut en faire à l'utilisateur, par exemple par affichage sur un écran.

Dans le cas d'une application manuelle, les mélanges sont distribués par exemple dans une coupelle. La personne s'applique le mélange à l'endroit recommandé avec un mélange correspondant prélevé dans la coupelle, puis nettoie éventuellement la coupelle et commande la délivrance d'un nouveau mélange ; l'opération est répétée autant de fois que nécessaire pour traiter son visage complètement.

L'homogénisation des mélanges distribués peut se faire de différentes façons selon les cas d'utilisation. Dans le cas d'une application manuelle, elle peut se faire directement sur la zone d'application au moment de l'application ou dans la coupelle avant prélèvement ; dans le cas d'une application à l'aérographe, c'est la tuyère de l'aérographe qui sert de chambre de mélange ; dans le cas ou le mélange est distribué dans un contenanten vue d'une utilisation ultérieure, l'homogénéisation peut être faite à la main ou en faisant passer les produits distribués dans une chambre de mélange, située entre le distributeur et le contenant ou directement intégrée au contenant, comme détaillé par la suite.

Le produit peut être délivré par le système de distribution et utilisé extemporanément. En variante, le produit délivré par le système de distribution est conditionné et utilisé ultérieurement, par exemple à plusieurs reprises, avec par exemple un jour d'intervalle au moins entre deux utilisations.

### Précisions sur les solvants organiques

On entend par solvants organiques, les composés carbonés liquides ou composés siliconés liquides ou sels liquides (appelés liquides ioniques).

Par exemple : Ethanol ou autres alcools, acétone, acétates d'alkyles, carbonate d'alkylène, pentane, hexane, octane, décane, isododécane, hexadécane et autres alcanes, diméthycones, dialkyl imidazolium acétate, dialkylimidazolium hexafluorophosphate...

Dans un cas particulier, le solvant est gazeux à température ordinaire, et liquide s'il est maintenu sous pression. Dans ce cas, les solvants peuvent être isopentane, butane ou certains composés fluorés dont la température d'ébullition est proche de la température ambiante (0-20°C).

Les solvants peuvent être des mélanges des ingrédients cités ci-dessus.

Les cartouches introduites dans le système peuvent contenir un produit de base contenant des solvants ou mélanges de solvants différents.

### Cartouches et matériaux

Selon ce quatrième aspect de l'invention, les cartouches comportent un corps et des pièces en mouvement destinés à pousser le produit que le corps contient.

Les parties de la cartouche qui n'ont pas d'incidence dans la précision du dosage telles que les pièces conduisant la composition jusqu'à l'orifice de sortie, et d'éventuels habillages extérieurs, ne sont pas soumis aux définitions de l'invention selon ce quatrième aspect sur le plan du choix des matériaux.

Pour obtenir une bonne précision dans la délivrance (et donc dans la réalisation des mélanges), on choisit de préférence les matériaux parmi ceux listés ci-dessous :
Pour le corps de la cartouche et les pièces en mouvement, les plastiques sélectionnés sont de préférence :
   - Polyoléfines simples
   - Polyvinylchloride (PVC)
   - Polyamide et polyamide semi-aromatiques
   - Polysulfure de phénylene (PPS)
   - Polybismaléimide
De façon moins préférée :
   - Poluréthannes
   - Polyester
   - Polyépoxydes
   - Polyéther bloc amide
   - Polyacétal
   - Polyester
   - Polyéthercétone
   - Polyetherimides (PEI)
   - Polyimides
   - Polyamideimide (PAI)
   - Certains polymères fluorés : FEP (ethylène propylène perfluoré) , PFA (Polyfluoroalkoxy), ECTFE (éthylène chloro trifluoro éthylène) , ETFE (éthylène tétrafluoroéthylène)
Sont exclus :
   - Polystyrène et ABS
   - Polyméthacrylate de méthyle (PMMA)
   - Phénoplastes,
   - Aminoplastes,
   - Polycarbonate
   - Polyphenyle oxyde (PPO)
   - Polysulfones
   - PET

### Les polyoléfines

Ce sont des polyéthylènes de différentes densités et réalisés avec différents comonomères (autres alcènes, en particulier alpha-oléphines (butene, hexene, octene ou propylène) et/ou acétate de vinyle, alcool vinylique, esters acryliques). Leur réalisation est classique pour l'homme de l'art, mettant en oeuvre des conditions de polymérisation aboutissant à de fortes teneurs en forme cristallines). Ce sont :
a) Polyéthylène de très basse densité linéaire (PEtbl). (densité de l'ordre de 0,855 à 0,91 g/cm3).
b) Polyéthylène basse-densité-haute pression (PEbd)LDPE (polyéthylène Basse densité) (densité de l'ordre de 0,915 à 0,935 g/cm3).
c) Polyéthylène basse densité linéaire (PEbdl) (densité de l'ordre de 0, 91 à 0,93g/cm3).
d) Polyéthylènes haute et moyenne densité (PEhd et PEmd) (densité de l'ordre de 0,930 à 0,945 pour les premiers et 0,945 à 0,970 g/cm3 pour les seconds).
e) Polyéthylènes UHMW (très haut poids moléculaires) (densité de l'ordre de 0,94 g/cm3).

Les c, d, et e sont les préférés pour l'invention, donnant les meilleures précisions.

Les polyoléfines sont aussi des polypropylènes. Seules les versions « isotactique » sont adaptées (taux d'isotacticité d'au moins 90%). Ils peuvent être réalisés avec ou sans comonomère (éthylène). Les versions copolymérisées (PPCO) sont dites séquencées ou statistiques et mettent en oeuvre environ 1% à 30% d'éthylène.

On peut aussi utiliser des polyoléfines non ou peu cristallisées. Dans ce cas, on utilise un monomère du type chlorure de vinyle. Le polymère obtenu (PVC) est de faible cristallinité (en général <10%) mais de forte densité (1,4 g /cm3). On utilise de préférence la forme « sans plastifiant ».

Les PE et PP sont accessibles chez de nombreux fournisseurs tels que Basell, Exxon Mobil, Arkema, Dow...

Les PVC sont accessibles chez de nombreux fournisseurs tels que Shin Etsu, Solvay, Arkema...

### Polyamides

### Sont utilisables :

Les polyamides PAn (-CO-NH-R)n
   a) PA6 (n=6)
   b) PA 12 (n=12)
Les polyamides PAnm (-CONHR1-NHCOR2) où R1=(CH2)n et R2= (CH2)m
   c) PA66 (n et m=6)
   d) PA610 (n =6 et m= 10)
   e) PA612 (n=6 et m= 10)
Sont préférés, b) , d) et e).
Exemples de PA 6 : Grilon de EMS, Minlon de Dupont
Exemples de PA 66 : Grilon T de EMS, Zytel de Dupont
Exemples de PA 610 : Ultramid S de BASF
Exemples de PA 612: Vestamid D de Degussa
Polysulfures de phénylene (-Ph-S-)n
Exemple de PPS: Ryton de Chevron Phillips, Primet de Solvay
Polybismaléimide
Exemple de PBMI: Kinel de Neopreg

### Mise en œuvre

Plusieurs mises en oeuvre sont possibles pour la réalisation des pièces selon le quatrième aspect de l'invention :
a) Injection dans un moule
b) Usinage

Pour des raisons pratiques, a) est préféré.

### Utilisation pour le traitement d'une ou plusieurs zones précises de peau

Le système de distribution permet de se maquiller, ou traiter la peau pour la soigner et ou la protéger ou se parfumer, jour après jour, en ne traitant que les zones précises. Pour ce faire, on délivre de petites doses de produit, qu'on applique précisément et séquentiellement sur les zones correspondantes. Chaque petite dose est réalisée selon le mélange qui convient à la zone.

Dans un mode préféré de mise en oeuvre de l'invention, le système de distribution attend qu'on le renseigne sur une zone à traiter puis délivre le mélange correspondant. Il peut faire appel à une table de correspondance préprogrammée pour cela, cette table résultant par exemple d'un procédé d'apprentissage tel que défini plus loin. En variante, le système de distribution renseigne la personne, tout en délivrant un mélange, sur la zone sur laquelle celle-ci doit appliquer le mélange. Ainsi, le système de distribution peut suivre un programme d'application où il délivre dans un ordre donné, les différentsmélanges à appliquer.

Dans un mode particulier de mise en oeuvre de l'invention, le système de distribution est renseigné sur les quantités à délivrer. Pour cela, il mémorise la correspondance entre la couleur ou l'effet, la zone du visage et la quantité nécessaire, ce qui permet de réduire les coûts et les pertes de produit, et de ne couvrir que faiblement la peau, évitant ainsi des effets d'occlusion. Ce faisant, il est possible d'utiliser des produits très couvrants et trop couvrants pour être appliqués sur l'ensemble du visage. Ainsi, il est possible d'obtenir des maquillages ou autres traitements d'aspects naturels, voire indétectables.

Le système de distribution peut aussi permettre, en facilitant la distribution de petites quantités et leur usage rapide, de réduire la durée de conservation des produits, permettant ainsi de réduire les risques d'évolution et/ou les quantités de conservateurs à employer.

Dans le cas de produits masquants, le système de distribution est adapté à traiter les zones qu'on veut masquer, sans avoir à masquer l'ensemble du visage.

Lorsque l'utilisateur recherche le produit à appliquer sur une zone du visage, il est intéressant de mémoriser le mélange (ratio entre les produits des compartiments) qui va bien pour chaque zone, et le système de distribution est ainsi avantageusement agencé pour mémoriser l'effet, les ratios et la zone correspondante. Ainsi, en utilisant les informations mémorisées, à chaque utilisation, on peut obtenir la délivrance du même mélange pour chaque zone, ou, si l'on traite plusieurs zones, la même série de mélanges pour une même série de zones.

Le système de distribution peut aussi être agencé pour permettre de traiter une zone en faisant varier les effets application après application. Ainsi, la personne peut maquiller la peau avec différentes couleurs ou parfumer la peau avec différentes notes, qu'elle choisit au jour le jour selon ses goûts. Par exemple, les jours de la semaine, la personne réalise un fond de teint couleur claire, et le weekend, un fond de teint de couleur hâlée, ou un maquillage des yeux d'une couleur un jour, et d'une autre couleur un autre jour. Par exemple, les jours de la semaine, la personne applique un parfum donné, et le weekend, un autre.

Le système est aussi agencé pour délivrer des touches selon le résultat de l'application des touches différentes. Ainsi, si l'utilisateur s'aperçoit qu'après application de plusieurs touches, il manque quelque chose pour parfaire son résultat, le système peut alors délivrer à la demande un mélange permettant de réaliser une touche qui complètera le résultat.

Par exemple, dans le cas de la couleur, si l'utilisateur applique des fonds de teint à base d'éthanol, étant donné la vitesse d'évaporation, il peut arriver que certaines zones du visage soient moins colorées que d'autre. L'utilisateur pourra alors demander au système un mélange moins concentré en agent colorant pour compléter la couleur dans les zones qui en manquait.

Idem avec les parfums. Si la personne applique une touche de parfum à un endroit, puis une autre touche de parfum (différent) sur une autre zone, elle aura peut être avoir envie de compléter l'impression olfactive en appliquant sur les mêmes zones ou sur une autre zone, une autre touche de mélange (différent).

Le système est ainsi prévu pour proposer des variations et une interface pour traduire des ordres simples en réalisation de mélange.

Le système de distribution peut être agencé pour permettre à l'utilisateur de changer les ingrédients selon ses goûts du jour, les besoins du jour, du moment, de ses tenues, du climat. Ainsi, on prévoit avantageusement un système d'aide à la décision pour guider l'utilisateur dans ses choix d'effets.

On peut prévoir aussi un système d'aide pour équilibrer les effets sur un même visage et participer à la réalisation d'un traitement global réussi.

Il peut être souhaitable que plusieurs personnes d'un même groupe, par exemple une famille, puissent utiliser le système de distribution, réduisant ainsi les coûts et minimisant la place occupée. Cette solution est spécialement adaptée aux voyages ou aux hôtels, campings, avions, campings cars, boutiques, écoles... Pour ce faire, on peut prévoir que le système de distribution puisse être renseigné sur la personne qui l'utilise, afind'accéder à des données personnelles préalablement mémorisées.

### Utilisation en continu pour un traitement en dégradé

Dans cette application, le système de distribution, pendant qu'il délivre le produit, change la formulation du mélange.

Cette fonctionnalité est très importante d'autant que la vitesse de séchage est rapide et limite les possibilités d'étaler.

De plus, on déplace la sortie des produits de base ou du mélange relativement à un contenant ou un support définissant une surface d'application. Dans un mode particulier de mise en oeuvre de l'invention, le système de distribution est agencé pour calculer l'évolution du mélange en fonction de la couleur ou de l'effet C1, d'une zone à traiter et de la couleur ou de l'effet C2, d'une autre zone à traiter. Par exemple, sachant que le menton nécessite une couleur C1 et que la joue nécessite une couleur C2, le système de distribution peut faire varier la formulation du mélange pendant qu'il le délivre pouréaliser un dégradé de couleur entre ces deux couleurs. Cela permet par exemple de mieux masquer les imperfections du visage en faisant en sorte que le résultat final soit réaliste, ou de faire des dégradés de couleur à des fins d'embellissement. Le système de distribution peut encore être agencé de telle sorte que l'utilisateur puisse commander une variation d'effet du mélange distribué sans que les effets de départ et/ou d'arrivée n'aient été préalablement fixées. Pour ce faire, le système de distribution peut disposer d'un système de localisation, ou d'auto-localisation, et déduire d'une table de correspondance les effets C1 et C2 qu'il doit réaliser, et donc les évolutions de mélange qu'il doit réaliser.

Le quatrième aspect de l'invention est applicable à la couleur, à la photoprotection. Le même concept est applicable aux produits parfumants où l'utilisateur voudra peut-être réaliser des dégradés de parfums. Par exemple, il peut réaliser un dégradé de parfums le long du cou pour faire partager une richesse d'odeur à une personne qui l'approche.

L'invention est applicable aux compositions protectrices. La personne pourra réaliser des dégradés d'indices de protection, réalisant ainsi, après s'être exposée au soleil, à un résultat de bronzage lui-même en dégradé.

Le système de distribution peut comporter une tête de sortie, en particulier dans le cas d'un aérographe, qui est mobile et pilotée. Cette option permet alors, sans bouger le reste du système de distribution, de réaliser des dégradés. Par exemple, on localise le système de distribution près de la joue, puis on déclenche un système de commande qui va piloter automatiquement la variation de la formulation du mélange et le mouvement de la tête de sortie, de façon à par exemple avoir le centre de la joue plus rouge que sa périphérie, avec un dégradé entre les deux.

Le système de distribution peut encore être utilisé pour réaliser des produits sur mesure, que l'on conserve pour réaliser plusieurs applications.

Il est aussi possible de réaliser des produits solides ou semi-solides. Dans ce cas, on utilise le système avec des formules riches en ingrédients solides et un solvant organique léger ou lourd en relativement faible quantité (moins de 70% et préférentiellement moins de 50%)

### Fabrication de compacts « à façon » ou autres produits solides ou semi-solides

Le système de distribution peut être agencé pour permettre de choisir un mélange et de le délivrer dans un contenant tel qu'une coupelle. Le mélange comporte de préférence des composés qui sont tels que le mélange puisse prendre en masse.

Plus préférentiellement, on utilise des composés qui rendent la prise en masse spécialement rapide. Ces composés sont soit déposés dans le contenant avant ou après son remplissage avec les autres constituants, soit sont prévus dans les compartiments du distributeur avec les autres constituants des produits de base, soit sont compris dans le distributeur dans un compartiment spécialement prévu pour les contenir.

On peut ainsi distribuer des compositions spécifiques qui peuvent par réaction chimique, biochimique ou physicochimique, durcir de façon accélérée après évacuation.

Ces compositions sont spécialement adaptées à la réalisation des compacts, c'est-à-dire :
- Prennent en masse,
- donnent un matériau qui peut se déliter en cas de frottement

De préférence, ces compositions sont très riches en particules solides, avec par exemple plus de 10% en masse de particules solides par rapport à la masse totale de la composition, mieux plus de 20% en masse de particules solides par rapport à la masse totale de la composition, encore mieux plus de 30% en masse de particules solides par rapport à la masse totale de la composition, préférentiellement entre 10 et 40% en masse de particules solides par rapport à la masse totale de la composition.

De préférence, une ou plusieurs des compositions inserrées dans le système contient de l'huile. (au moins 20% en masse).

Ces compositions peuvent contenir des particules absorbantes, ou des composés réactifs, tels que ceux qui réagissent en contact avec l'air, par exemple du cyanoacrylate ou des alphasilanes, ou qui réagissent à la lumière, notamment UV.

Le contenant dans lequel le mélange est distribué peut comporter un composé A et les compositions distribuées un composé B, A et B étant choisis pour réagir entre eux et solidifier le mélange.

Dans un mode particulier de mise en oeuvre de l'invention, le système de distribution intègre un moyen de chauffage, par exemple à résistance électrique, pour la réalisation de rouges à lèvres ou autres produits cireux. Dans ce cas, les produits de base sont chauffés avant d'être délivrés.

Le système de distribution peut aussi comporter un moyen pour apporter de l'énergie, thermique et/ou lumineuse, après distribution du mélange dans un contenant, par exemple une résistance électrique ou une diode LED, notamment UV. Cette énergie peut accélérer la prise en masse du mélange distribué.

Préférentiellement, le mélange est homogénéisé avant la prise en masse.

### Réalisation de palettes de couleurs

Le système de distribution peut comporter un support, ayant plusieurs régions, et être agencé pour générer automatiquement plusieurs mélanges déposés dans lesdites régions, par exemple une série de couleurs adaptées à différentes parties du visage.

Le support peut définir plusieurs cavités pour recevoir les mélanges ou porter plusieurs contenants, par exemple sous forme de coupelles, éventuellement séparables du support.

Dans un cas particulier, le support prend la forme d'un visage avec des régions pour recevoir les mélanges pour des zones d'applications ciblées.

Le support être mobile, notamment rotatif, par rapport au corps du distributeur, et par exemple entraîné en déplacement par le distributeur, pour remplir successivement différents logements ou contenants.

### Réalisation d'autres palettes

L'invention peut être mise en oeuvre pour faire des palettes d'odeur ou de soin (avec actifs de soin) ou de protection (avec filtre UV).

### Distributeur à coupelle

Il existe un intérêt pour bénéficier d'un système de distribution capable de délivrer un mélange pouvant être facilement prélevé par l'utilisateur. Par ailleurs, dans le cas où les produits de base délivrés par le système de distribution ne sont pas déjà mélangés, il existe un besoin pour permettre à l'utilisateur d'effectuer aisément le mélange.

Selon l'un de ses aspects, indépendamment ou en combinaison avec les autres aspects de celle-ci, et notamment avec ce qui précède, l'invention a pour objet un système de distribution d'au moins un produit de maquillage ou soin ou protection ou parfumage, comportant une coupelle, et un distributeur pour remplir la coupelle avec au moins un produit, la coupelle étant solidaire du distributeur au moins pendant son remplissage.

La coupelle est parfois encore appelée « creuset » et ce terme doit être compris avec une acceptation large.

Par « coupelle solidaire du distributeur » il faut comprendre que la coupelle est retenue, notamment immobilisée, au moins temporairement sur le distributeur, étant par exemple fixée sur celui-ci par des vis, par aimantation, par encliquetage, par verrouillage baïonnette, par serrage, ou réalisée avec une partie du corps du distributeur par moulage de matière. La coupelle lorsqu'elle est solidaire du distributeur permet de manipuler celui-ci d'une main, la coupelle restant en place sur le distributeur au cours des mouvements de ce dernier.

Le distributeur peut être proposé à l'utilisateur avec la coupelle déjà en place.

En variante, celle-ci est installée par l'utilisateur à la première utilisation du système de distribution.

De préférence la coupelle est moins profonde que large, ce qui facilite l'accès à celle-ci, et permet de prélever le produit, notamment le mélange avec un applicateur ou le doigt.

De préférence, la coupelle est séparable du distributeur, et constitue une interface de sortie qui peut être choisie parmi un ensemble d'interfaces de sortie pouvant être montées sur le distributeur, au choix de l'utilisateur en fonction de l'effet à réaliser, comme détaillé plus loin.

De préférence, le système de distribution comporte plusieurs orifices de remplissage en des produits de base différents, débouchant dans la coupelle. Ainsi, le mélange de ces produits peut avoir lieu dans la coupelle.

La coupelle a de préférence un fond de forme concave vers l'extérieur, ce qui peut faciliter son nettoyage par l'utilisateur entre deux utilisations.

De plus, cela peut faciliter le prélèvement du produit par l'utilisateur, et le mélange des produits de base.

De préférence, le distributeur permet de délivrer au moins deux produits de base dans la coupelle, dans des proportions réglables, et mieux au moins trois produits.

Dans un exemple de mise en oeuvre, le système de distribution comporte au moins deux coupelles pouvant être sélectivement alimentées par le distributeur. Cela peut permettre à l'utilisateur de remplir rapidement ces deux coupelles avec des mélanges dont les caractéristiques sont différentes. Cela peut faciliter les essais de matières, et/ou permettre de préparer plusieurs mélanges différentes destinées au traitement de zones respectives du visage. Les coupelles peuvent être associées à des identifiants rappelant à l'utilisateur à quelle zone du visage un mélange contenu dans une coupelle donnée est destiné.

Les coupelles peuvent être mobiles relativement au distributeur, étant par exemple portées par un support mobile, tel qu'une tourelle mobile en rotation par rapport au distributeur, ou par un tiroir mobile en translation par rapport au distributeur.

Le système de distribution peut comporter un couvercle de fermeture de la coupelle. Ce couvercle de fermeture est de préférence transparent pour permettre à l'utilisateur de visualiser la couleur du mélange contenu à l'intérieur.

Lorsque la coupelle est séparable du distributeur, elle peut être introduite le cas échéant dans un boîtier permettant de la transporter plus facilement, ce boîtier pouvant comporter le cas échéant un miroir et/ou un applicateur. Le couvercle du boîtier peut dans ce cas servir de couvercle pour la coupelle.

Le volume de la coupelle peut être compris entre 2 et 1000 mm³, mieux entre 100 et 1000 mm³, encore mieux entre 250 et 750 mm³.

Le ou les produits de base délivrés dans la coupelle sont de préférence des fonds de teint, mais en variante il s'agit de produits de maquillage des lèvres ou des paupières.

La coupelle est de préférence de forme symétrique de révolution. En variante, elle est de contour polygonal ou autre. Son plus grand diamètre intérieur, ou celui du cercle inscrit dans le cas d'un contour non circulaire, est de préférence compris entre 2 et 100 mm, préférentiellement entre 5 et 40 mm. Sa profondeur est de préférence comprise entre 1 et 10 mm, mieux entre 3 et 8 mm. De préférence, la taille et la forme de la coupelle permettent soit une application directe du mélange sur la peau, soit une préhension au doigt ou avec un applicateur. La coupelle peut être réalisée avec un matériau élastiquement déformable, ce qui permet par exemple d'inverser la concavité du fond de la coupelle et de vider celle-ci plus facilement ou de l'utiliser pour appliquer le produit.

La coupelle peut être dépourvue de mélangeur ; dans ce cas, les produits de base peuvent arriver du distributeur dans la coupelle à l'état non mélangé, par des orifices de distribution respectifs distincts. En variante, le distributeur intègre un mélangeur et les produits de base arrivent déjà mélangés dans la coupelle.

La coupelle peut aussi intégrer un mélangeur statique, comme détaillé plus loin, qui est alimenté par des orifices de remplissage distincts du distributeur, et qui délivre de préférence le mélange dans une cavité de la coupelle située au-dessus du mélangeur.

L'invention a encore pour objet un procédé de préparation d'un produit de maquillage, de parfumage, de soin, de protection, comportant l'étape consistant à remplir une coupelle d'un système de distribution tel que défini plus haut avec au moins un produit de base provenant du distributeur.

Plusieurs produits peuvent être délivrés dans le fond de la coupelle, puis mélangés à l'aide du doigt ou d'un applicateur, ou d'un mélangeur statique intégré à la coupelle.

La coupelle est de préférence remplie par le dessous. Il a été proposé dans le passé des systèmes de distribution à l'aide d'une sonotrode.

La coupelle selon l'invention n'est pas destinée à entrer en vibration pour distribuer le ou les produits amenés par le ou les canaux d'alimentation de celle-ci. Elle est différente d'une sonotrode. De préférence, la coupelle est réalisée en matière plastique.

### Mélangeur intégré à l'interface de sortie

Il existe un intérêt pour bénéficier d'un système de distribution capable de délivrer un mélange pouvant être facilement utilisé, notamment prélevé par l'utilisateur, sans nécessiter une action additionnelle de mélange de la part de celui-ci.

Selon l'un de ses aspects, indépendamment ou en combinaison avec les autres aspects de celle-ci, et notamment ce qui précède, l'invention a pour objet un système de distribution comportant un distributeur ayant des canaux de sortie de produits de base et une interface de sortie séparable du distributeur, cette interface ayant un mélangeur statique, délivrant de préférence le mélange dans une cavité où il peut être prélevé.

Le mélangeur statique peut être situé sous la cavité précitée. Le système de distribution est alors particulièrement adapté à la création de compacts, en utilisant comme interfaces de sortie des coupelles à mélangeur statique intégré. Dans ce cas, on vient remplir la cavité de la coupelle en produit par le dessous. Après passage dans le mélangeur statique, les produits de base mélangés viennent recouvrir le mélangeur.

Selon cet aspect de l'invention, il est possible d'utiliser plusieurs interfaces de sortie et de les remplir avec des mélanges respectifs différents, sans avoir à purger le mélangeur, ce qui réduit les pertes de produit. L'interface de sortie peut être à usage unique, le cas échéant.

De préférence, le mélangeur statique comporte une chambre centrale communiquant avec des canaux d'admission des produits de base. Cette chambre centrale peut communiquer avec une chambre périphérique comportant une série de cloisons qui agissent comme des déflecteurs pour le mélange, et créent un cisaillement de celui-ci.

La chambre périphérique peut comporter une cloison annulaire ajourée définissant des ajours par lesquels passe le mélange en circulant dans la chambre périphérique. Les chambres centrale et périphérique peuvent être fermées supérieurement par une paroi qui définit le fond de la cavité recevant le mélange.

Le fond de la chambre périphérique peut être de forme hélicoïdale autour de l'axe de la coupelle et de hauteur diminuant au fur et à mesure que l'on progresse vers la sortie. Cette dernière peut déboucher en avant d'une rampe de liaison entre le fond de la chambre périphérique et la paroi supérieure du mélangeur, cette rampe de liaison étant de préférence une portion d'hélice prolongeant l'hélice formée par la paroi de fond de la chambre périphérique.

De préférence la chambre périphérique comporte la cloison annulaire précitée et des cloisons radiales qui forcent le mélange à circuler alternativement entre des régions supérieure et inférieure de la chambre périphérique et entre des régions radialement intérieure et extérieure, le mélange circulant par exemple d'une région supérieure et radialement extérieure à une région inférieure et radialement extérieure en passant à travers la cloison annulaire précitée.

Le mélangeur peut comporter un corps extérieur dans lequel est reçue une pièce formant le coeur du mélangeur, le corps extérieur fermant radialement à l'extérieur la chambre périphérique et comportant un montant qui sépare les chambres centrale et périphérique.

Le corps extérieur du mélangeur et le coeur du mélangeur peuvent être chacun réalisés d'une seule pièce par moulage par injection.

### Volume mort réduit

Il existe un intérêt pour réduire les pertes de produit lors des changements de formulation du mélange, ainsi que pour permettre de faire varier le plus rapidement possible la couleur du mélange au cours de l'application, notamment en cas de couplage du distributeur avec un aérographe.

Selon l'un de ses aspects, indépendamment ou en combinaison avec les autres aspects de celle-ci, notamment ce qui précède, l'invention a pour objet un système de distribution d'un produit de maquillage ou soin ou protection ou parfumage, comportant un distributeur recevant au moins deux cartouches ayant chacune un réservoir contenant un produit de base, ce dernier quittant la cartouche par un canal de sortie de la cartouche, ce canal de sortie débouchant à l'extérieur du distributeur ou à proximité de sa surface externe.

Le canal de sortie peut notamment déboucher dans une zone de prélèvement du mélange ou à proximité de celle-ci, notamment à moins de 5 mm, mieux à moins de 3 mm, mieux à moins de 1mm, mieux à fleur.

La section transversale du canal de sortie est par exemple comprise entre 1 et3 mm².

Ainsi, chaque produit de base provenant d'une cartouche peut quitter le distributeur sans se mélanger avec un produit de base d'une autre cartouche et l'on minimise le volume mort qui ne peut être prélevé et susceptible d'accroître l'inertie du système. Le produit est plus rapidement disponible sans avoir à circuler dans des canaux spécifiques du boîtier du distributeur, évitant une étape fastidieuse de purge si changement de cartouche.

L'extérieur du distributeur peut être la zone de prélèvement du produit, notamment lorsque le distributeur est réalisé avec une coupelle non prévue pour être enlevée, dans laquelle le mélange est distribué, ou une zone du distributeur destinée au montage d'une interface de sortie amovible, qui définit la zone de prélèvement. Cette interface de sortie peut comporter une coupelle telle que définie ci-dessus. Cette zone de montage correspond par exemple à l'extrémité du boîtier du distributeur en l'absence de l'interface de sortie. La zone de montage peut être sensiblement plane et perpendiculaire à l'axe longitudinal du boîtier du distributeur.

Le distributeur peut comporter trois cartouches de produits de base.

Le distributeur peut comporter des logements pour recevoir les cartouches, lesquelles sont de préférence reçues de façon amovible dans le distributeur. Ce dernier peut comporter des passages pour des conduits des cartouches définissant les canaux de sortie.

La longueur de ces conduits est de préférence telle que les conduits se situent légèrement en retrait du fond ou à affleurement de la cavité servant au prélèvement du produit, ou en variante légèrement en retrait ou à affleurement de la face d'extrémité du boîtier du distributeur définissant la zone de montage.

Ces conduits des cartouches peuvent être des embouts servant à entraîner en déplacement des pistons au sein des cartocuhes, comme détaillé plus haut.

### Interfaces de sortie multiples

Il existe un besoin pour pouvoir, à l'aide d'un même système de distribution, réaliser facilement des maquillages ou soin ou protection ou parfumage différents, et pouvoir si on le souhaite traiter des zones aussi différentes que la peau, les lèvres ou les cils ou sourcils, le cou, les cheveux.

L'invention a encore pour objet un système de distribution comportant un ensemble comportant un distributeur d'au moins un produit cosmétique, notamment de maquillage, de soin, de protection ou parfumage, et au moins deux interfaces de sortie pouvant se monter chacune de façon amovible sur le distributeur, ces interfaces de sortie permettant de recevoir le ou les produits délivrés par le distributeur, étant de préférence choisies parmiles suivantes :
- interface de sortie comportant un contenant, notamment une coupelle permettant un prélèvement du produit au doigt ou à l'aide d'un applicateur,
- interface de sortie permettant de délivrer le produit à un système de pulvérisation, notamment un aérographe,
- interface de sortie comportant plusieurs régions de réception du produit, mobile par rapport au distributeur,
- interface de sortie permettant de délivrer le produit à un embout de distribution.

De préférence, l'ensemble comporte au moins trois desdites interfaces de sortie, mieux les quatre interfaces de sortie.

Le distributeur peut comporter au moins deux produits de base différents et permettre de délivrer ceux-ci dans des proportions variables, et de préférence, le distributeur comporte trois produits de base différents et permet de délivrer ceux-ci dans des proportions variables.

Chaque interface de sortie peut comporter une base permettant sa fixation sur le distributeur. Cette fixation peut s'effectuer à l'aide de vis par exemple, mais de préférence la base est agencée pour permettre un démontage et un remplacement d'une interface de sortie sans nécessité d'outil. Il s'agit par exemple d'une fixation quart de tour ou à l'aide d'une bague de verrouillage externe.

L'interface de sortie et/ou le boîtier du distributeur peuvent comporter des joints d'étanchéité permettant une communication étanche entre le boîtier du distributeur et l'interface de sortie. Le cas échéant, le distributeur est agencé pour reconnaître l'interface de sortie qui est montée dessus, par exemple grâce à identifiant de l'interface de sortie sous forme de reliefs spécifiques, lesquels sont détectés par le distributeur, ou d'une puce électronique reconnue par le distributeur. Cela peut permettre d'adapter le fonctionnement du distributeur à l'interface de sortie montée dessus. Le distributeur peut communiquer à un système informatique des informations sur l'interface de sortie qu'il porte, et le système informatique peut afficher en fonction de ces informations un écran spécifique et/ou lancer un programme spécifique de commande des paramètres de fonctionnement du distributeur, afin par exemple d'adapter la dose distribuée et/ou le débit à la nature de l'interface de sortie.

Plusieurs interfaces de sortie peuvent être proposées initialement à l'utilisateur avec un distributeur commun au sein d'un même emballage, par exemple un coffret ou une boîte de carton.

L'invention a encore pour objet un procédé de maquillage ou soin ou protection ou parfumage, comportant l'étape consistant à sélectionner une interface de sortie, à la monter sur le distributeur, et à délivrer le ou les produits contenus dans le distributeur dans celle-ci.

### Cartographie et apprentissage

Le terme « cartographie » s'entend ici comme un procédé d'indexation d'une couleur, odeur, protection, soin et d'une zone, avec enregistrement.

La cartographie peut concerner des applications sur des zones plus petites qu'un cm2. Toutefois, l'oeil nu a alors du mal à discerner si le résultat obtenu est adéquat, et il est préférable de substituer l'évaluation à l'oeil nu par une évaluation instrumentée, avec grossissement. L'application de petites quantités de matière peut être faite au doigt, avec des outils classiques tels que des pinceaux, ou avec des applicateurs spécialisés.

La cartographie peut être générée lors d'une période d'apprentissage, pendant laquelle l'utilisateur effectue des tests avec des mélanges sur différentes zones du visage ; une fois renseignée, la cartographie peut ensuite être utilisée pour le maquillage ou le soin ou la protection ou le parfumage de tous les jours.

Des interfaces graphiques spécifiques peuvent servir pendant la période d'apprentissage et pendant la période d'utilisation de la cartographie.

En particulier, le système de distribution peut être utilisé avec une interface graphique où l'opérateur voit le visage, qui est par exemple schématisé, figuratif ou précis comme une photograhie ou une simulation 3D. Dans ce cas, l'opérateur peut pointer une partie du visage sur l'écran pour voir apparaître et/ou délivrer la couleur etou le soin etou la protection etou l'odeur adéquate.

L'interface graphique peut aussi laisser apparaître les autres zones du visage où l'utilisation de la même couleur ou soin ou protection ou odeur est adéquate.

Pour réaliser la cartographie, l'opérateur applique une couleur ou soin ou protection ou odeur puis réalise l'évaluation.

Les zones du visage peuvent être traitées les unes après les autres ; par exemple, on réalise l'exercice sur une partie de la joue, puis sur le nez, etc....

Une autre possibilité consiste à réaliser un mélange donné, et appliquer sur plusieurs zones ce même mélange. L'opérateur doit alors chercher la zone du visage pour laquelle la couleur ou soin ou protection ou odeur est adaptée. Le mélange est ensuite indexé dans le système informatique en l'attribuant à la ou aux zones du visage pour lesquelles il convient.

L'invention a encore pour objet un procédé d'apprentissage d'un système de distribution comportant un distributeur permettant de distribuer un mélange variable, et un système informatique permettant de sélectionner un effet et de mémoriser des données, comportant les étapes consistant à :
a) Sélectionner au moins un effet à l'aide d'une interface du système informatique,
b) délivrer à l'aide du distributeur au moins un mélange pour approcher l'effet sélectionné,
c) évaluer le ou les mélanges distribués après application sur au moins une zone du visage, (visuel, confort, odeur, toucher)
d) mémoriser les caractéristiques d'au moins un mélange, notamment un mélange que l'utilisateur souhaite pouvoir rappeler, et d'au moins une zone sur laquelle il a été testé.

Cette mémorisation peut s'effectuer notamment en vue d'une distribution ultérieure de ce mélange pour traiter ladite zone.

De préférence, le système informatique est agencé pour permettre à l'utilisateur de signaler si le résultat du test est satisfaisant ou non, voire de le renseigner sur la comparaison à un test effectué précédemment.

On peut aussi réaliser un mélange donné, et chercher la zone du visage pour laquelle il est adapté. On enregistre alors le mélange en l'attribuant à la ou aux zones du visage pour lesquelles il est adapté, dans une table de correspondance qui servira par la suite, pour, partant d'une zone de la peau, en déduire le mélange à utiliser.

On peut procéder de la même façon avec d'autres mélanges pour réaliser une cartographie de l'ensemble du visage et ainsi avoir une table de correspondance complète du visage.

On peut aussi réaliser un mélange donné, l'appliquer sur une zone donnée, puis faire varier le mélange jusqu'à obtenir le mélange le plus adapté. On enregistre alors le mélange en l'attribuant à la ou aux zones du visage pour lesquelles il convient, dans une table de correspondance qui servira par la suite, pour, partant d'une zone de la peau, en déduire le mélange à utiliser.

De préférence, le système informatique évalue et mémorise les quantités utilisées zone par zone. Un tel procédé, par « touches d'essai », permet d'identifier le ou les produits dont a besoin la personne qui veut se maquiller ou soigner sa peau ou protéger sa peau ou se parfumer. Ainsi, le système de distribution peut servir sur les points de vente pour conseiller les personnes qui veulent se maquiller ou soigner sa peau ou protéger sa peau ou se parfumer ou à la maison pour bien définir les produits à commander.

L'interface du système informatique comporte de préférence un écran tactile affichant la couleur du mélange lors de sa sélection.

L'interface peut afficher un visage et permettre de renseigner le système informatique en sélectionnant la zone sur le visage affiché.

Le système informatique est de préférence agencé pour permettre d'associer une zone, des paramètres de reconstitution du mélange, et la date de l'essai et/ou tout autre identifiant du mélange.

Le système informatique est de préférence également agencé pour permettre d'associer en outre à ladite zone, aux paramètres de reconstitution du mélange, et à la date ou à l'identifiant du mélange, au moins l'une des données suivantes : le nom de la zone, la période de l'année, le nom d'un évènement, un identifiant de l'utilisateur, l'âge de l'utilisateur.

Les étapes a) à c) peuvent être renouvelées au moins une fois avant la mémorisation des caractéristiques du mélange à l'étape d).

Le système informatique peut être agencé pour rechercher dans une base de données une référence de produit commerciale, sur la base des caractéristiques du mélange identifié comme convenant à au moins une zone donnée, et la communiquer à l'utilisateur.

La sélection à l'étape a) peut s'effectuer avec l'aide d'un système expert externe ou non au système informatique.

Le système expert peut analyser une image de l'utilisateur, pour proposer un mélange au moins sur la base de l'image analysée.

L'étape a) peut être précédée par la proposition d'une couleur ou autre effet et d'une zone à tester avec un mélange de cette couleur ou de cet effet, par le système informatique à l'utilisateur.

Le système informatique peut être agencé pour permettre à l'utilisateur de lerenseigner sur l'appréciation du résultat du test de l'étape c) et pour générer uneproposition de modification du mélange à sélectionner lors du retour à l'étape a).

Le système informatique peut être agencé pour proposer au moins un effet de mélange à l'étape a) en fonction d'une zone d'application renseignée par l'utilisateur.

Le système informatique peut être agencé pour proposer au moins une zone d'application à l'étape a), en fonction de l'effet renseigné par l'utilisateur.

Le distributeur peut délivrer à l'étape b) au moins deux mélanges différents, de préférence séparés, en vue de leur application simultanée sur la zone de test.

Cela peut faire gagner du temps, et faciliter la comparaison des résultats.

L'invention a encore pour objet un procédé de maquillage ou parfumage ou soin ou protection à l'aide d'un système de distribution selon cet aspect de l'invention, dans lequel :
a) L'utilisateur adresse une requête concernant un besoin au système informatique,
b) celui-ci génère en retour une proposition en vue du traitement d'une zone associée, sur la base de l'apprentissage effectué préalablement, et
c) le système informatique pilote le distributeur pour la production du mélange proposée, notamment si celle-ci est validée par l'utilisateur.

Un tel procédé peut utiliser une cartographie précédemment établie avec l'utilisateur.

L'invention a encore pour objet un produit programme d'ordinateur comportant des instructions de code permettant, lorsque exécutées dans un système informatique, d'amener le système informatique à :
- Permettre à l'utilisateur de sélectionner au moins un effet et/ou une zone d'application, notamment à l'aide d'une interface telle qu'un écran tactile,
- piloter un distributeur de façon à délivrer un mélange sélectionné par l'utilisateur,
- permettre à l'utilisateur de déclencher la mémorisation du mélange et d'une zone d'application associée, notamment en vue d'une distribution ultérieure du même mélange, notamment sur la même zone.

Le produit programme d'ordinateur peut comporter des instructions de code permettant, lorsque exécutées dans un système informatique, d'amener le systèmeinformatique à:
- Recevoir une requête de l'utilisateur concernant un besoin notamment à l'aide d'une interface telle qu'un écran tactile,
- proposer sur la base au moins de données générées par le procédé d'apprentissage tel que défini ci-dessus, au moins et/ou une zone d'application,
- piloter un distributeur pour la production du mélange proposée, notamment si celle-ci est validée par l'utilisateur.

### Aide à distance

Il est souhaitable de pouvoir aider l'utilisateur à se maquiller ou soigner ou protéger sa peau ou se parfumer, notamment à choisir les bons coloris, notes olfactives ou effets de soin ou de protection.

L'invention, selon l'un de ses aspects, indépendamment ou en combinaison avec ses autres aspects, notamment ce qui précède, a ainsi pour objet un procédé de maquillage ou soin ou protection ou parfumage comportant les étapes consistant à :
- permettre l'établissement d'une liaison vidéo, par exemple via internet, entre une caméra d'un premier site et un deuxième site,
- permettre au deuxième site de piloter directement ou indirectement un distributeur présent sur le premier site, ce distributeur permettant de faire varier la couleur, effet protecteur ou soin ou odeur d'un mélange distribué,
- permettre à une personne présente au premier site d'appliquer le mélange distribué et d'envoyer vers le deuxième site une image correspondante ou commentaires, pour recevoir en retour une information relative au résultat de l'application.

Le même concept est applicable au soin ou la protection de la peau.

Le deuxième site peut notamment comporter un écran de visualisation qui permet à un conseiller présent devant cet écran de voir ou entendre ou lire les commentaires sur le résultat du maquillage ou soin ou protection ou parfumage avec le produit distribué par le distributeur et de conseiller la personne qui s'est maquillée. Ce conseiller peut agir en retour sur le distributeur pour modifier la couleur ou soin ou protection ou odeur du mélange et l'adapter au mieux au visage de la personne présente sur le premier site. Ainsi, cette personne gouverne le mélange délivré par le distributeur. La première personne peut se maquiller, ou soigner ou protéger sa peau ou se parfumer sous les yeux de la seconde. La seconde personne voit sur son écran le résultat de l'essai, et ainsi peut corriger le mélange qu'elle va commander à distance, jusqu'à obtenir l'effet idéal.

Le cas échéant, l'acquisition vidéo peut être calibrée à l'aide d'une mire, ou avec le mélange distribué par le distributeur sur une surface de référence. Cela permet ensuite une visualisation plus fidèle du maquillage ou soin ou protection effectué sur le premier site.

De préférence, la liaison vidéo entre les deux sites est une liaison duplex.

Le premier site peut recevoir un tutoriel du deuxième site, le cas échéant.

Des identifiants des produits de base peuvent être communiqués au deuxième site ; cela peut permettre de connaître précisément la couleur de chacun des produits de base.

Le procédé peut comporter la mémorisation des paramètres de réglage du distributeur, une fois qu'un mélange donné est estimé satisfaisant. De préférence, cette mémorisation peut être commandée depuis le deuxième site. La mémorisation peut avoir lieu dans le système informatique présent sur le premier site et/ou sur un serveur externe.

Une variante peut consister à faire travailler une personne pour aider le maquillage, le soin ou la protection de la peau ou le parfumage de plusieurs personnes. Ce mode de mise en oeuvre permet le développement des « Make-Up Artists » ou « coach beauté » ou « coach en parfums » et leur travail soit au sein d'un institut, soit via internet. Cela permet aussi le maquillage ou la protection ou le soin ou le parfumage de personnes aux moyens limités, telles que les personnes malvoyantes, ou discernant mal les couleurs, ou âgées, ou manquant de confiance en elles.

### Pilotage par interface tactile

Il existe un besoin pour faciliter le pilotage du système de distribution et notamment le choix des effets du mélange distribué.

L'invention a encore pour objet un système de distribution comportant un distributeur et un système informatique de pilotage du distributeur, ce système informatique comportant un écran tactile sur lequel peut être affichée la couleur du mélange ou l'effet attendu et un moyen de sélection déplaçable sur l'écran, pour faire varier la couleur ou l'effet protecteur ou soin ou l'odeur du mélange distribué.

De préférence, l'écran affiche les effets extrêmes entre lesquelles l'effet du mélange peut être sélectionné, en déplaçant le moyen de sélection entre ces points extrêmes.

L'écran peut afficher une échelle d'effets en forme de ligne ou surface (contour triangulaire).

Le système informatique peut effectuer une partie des calculs nécessaires pour déterminer les fractions de chacun des produits de base conduisant à un mélange de de l'effet recherché.

Le système informatique peut être un téléphone intelligent, un photophone, une tablette, un ordinateur individuel. En variante, il est intégré au boîtier du distributeur.

Le système informatique peut disposer d'une caméra. Cette dernière peut être utilisée notamment pour l'acquisition d'une image de l'utilisateur et/ou du mélange.

Le système informatique peut être agencé pour afficher une image d'un visage, afin de faciliter le repérage des zones sur lesquelles le mélange doit être appliqué.

### Couplage du système de distribution à un moyen de pulvérisation

Le système de distribution peut comporter ou être connecté à un moyen de pulvérisation du mélange, de préférence un aérographe.

L'invention vise, selon un autre de ses aspects, à perfectionner encore les systèmes de distribution comportant un système de pulvérisation, de préférence un aérographe, et a pour objet un ensemble comportant :
- Un moyen de pulvérisation, de préférence un aérographe comportant une chambre de prélèvement soumise à un courant d'air d'entraînement,
- un distributeur comportant au moins deux compartiments contenant des produits de base différents, les produits étant délivrés au moyen de pulvérisation de préférence par des orifices de distribution distincts.

Le distributeur peut comporter trois cartouches contenant des produits de maquillage de couleurs différentes ou parfumage, soin et protection.

L'aérographe peut comporter un stylet définissant la chambre de prélèvement, le stylet se fixant sur le distributeur ou sur une interface de sortie fixée au distributeur, ou faisant partie intégrante de cette interface de sortie.

Le système de distribution peut comporter un circuit de contrôle du fonctionnement du distributeur, permettant de faire varier la proportion des produits de base délivrés dans la chambre de prélèvement, au cours du fonctionnement de l'aérographe. Les proportions peuvent être modifiées en fonction du déplacement de l'aérographe relativement à la surface sur laquelle le mélange est pulvérisé. Ce déplacement peut être mécanisé, le cas échéant.

Ce circuit de contrôle peut comporter ou être constitué par un système informatique tel que défini plus haut.

Le boîtier du distributeur peut servir de poignée lors de la manipulation d l'ensemble pour délivrer le mélange.

Le distributeur peut comporter une caméra et/ou un ou plusieurs capteurs tels que des accéléromètres afin de localiser automatiquement la zone sur laquelle le mélange est appliqué, et pouvoir régler automatiquement la couleur ou l'effet protecteur ou soin ou parfum en fonction de la position, le cas échéant.

L'invention a encore pour objet un procédé de maquillage ou soin ou protection ou parfumage à l'aide d'un ensemble tel que défini ci-dessus, dans lequel un mélange est pulvérisé sur la peau à l'aide du moyen de pulvérisation, notamment de l'aérographe.

La composition du mélange peut être modifiée au cours du déplacement de l'aérographe relativement à la peau. Un dégradé peut être réalisé.

Cet aspect de l'invention repose sur la constatation que le distributeur peut être utilisé pour alimenter le système de pulvérisation, notamment l'aérographe, tout en permettant au système de distribution d'être suffisamment réactif pour autoriser un changement de la couleur du mélange distribué en cours de traitement du visage, notamment lorsque la zone à traiter change.

Il peut être avantageux que la distribution des produits se fasse de façon itérative, notamment avec des temps de distribution non déphasés entre les différents produits.

Cela peut rendre plus facile de faire varier la composition du mélange distribué au cours du temps.

Le mélange peut être réalisé directement dans l'aérographe, sans pratiquement aucun volume mort gênant, permettant ainsi de changer le mélange pulvérisé en temps réel.

La dépression créée dans la chambre de prélèvement est suffisante pour entraîner les produits de base, sans pour autant gêner le dosage.

La dépression qui règne dans la chambre de prélèvement est par exemple comprise entre 10 mbar et 200 mbar, mieux entre 50 et 150 mbar, encore mieux entre 75 et 125 mbar.

La viscosité des produits de base mesurée à 1 atm et à 25°C avec un viscosimètre rotatif CONTRAVES TV équipé d'un mobile MS-r3 ou MS-r4 à la fréquence de 60 Hz, après 10 minutes de rotation du mobile, est par exemple comprise entre 0,001Pa.S et 50 Pa.S

La section des canaux d'arrivée des produits de base dans la chambre est par exemple comprise entre 1 et 3 mm², mieux entre 2 et 3 mm².

L'alimention en produit s'effectue de préférence de façon continue.

Il est encore possible d'appliquer simultanément des produits de base non miscibles ou réactifs, comme un gel aqueux et un gel huileux, qui vont se déposer de façon pixellisée directement sur la peau, produisant une sorte de gel/gel in situ, des silicones réactives, ou des produits de coloration réagissant ensemble. Les ratios en produits de base peuvent être ajustés en fonction du résultat particulier souhaité. Par exemple, dans le cas des gels aqueux et gels huileux, on pourra faire varier le ratio correspondant au volume du premier produit de base sur le volume du deuxième produit de base entre 10/1 et 1/10, mieux entre 5/1 et 1/5.

### Système de localisation ou d'auto-localisation

Le système de distribution selon l'invention peut disposer d'un système de localisation ou d'auto-localisation.

On appelle système de localisation, un moyen de saisir, de la part de la personne, la zone qu'elle va traiter. Ceci peut être réalisé notamment par des systèmes laissant libre au moins une main. Ainsi, on peut utiliser une interface d'un système informatique telle qu'un écran tactile, un joystick, ou un système de reconnaissance vocale.

On appelle système d'auto-localisation, un moyen de saisir, sans que la personne n'intervienne, la zone à traiter. Ceci peut être réalisé par un ou plusieurs accéléromètres qui déduisent, par le fait des mouvements, les directions que la personne vise ou par une caméra et un système de reconnaissance d'image.

Le système de distribution peut être tel que celui illustré sur les figures décrites précédemment, en respectant le choix des matériaux selon le quatrième aspect de l'invention, notamment pour toutes les pièces rencontrant un mouvement relatif les unes par rapport aux autres.

Les figures ne seront donc pas toutes décrites à nouveau, étant entendu que le système de distribution n'est pas limité à un produit de maquillage et vise la distribution de produits de soin, de protection solaire ou de parfums.

Ainsi, concernant la description de l'interface utilisateur, les boutons présents sur l'écran peuvent permettre par exemple d'entrer le nom du maquillage ou soin ou protection ou parfumage ou de l'utilisateur, d'afficher la zone sélectionnée, de choisir la couleur ou soin ou protection ou odeur, et d'informer le système informatique du résultat acceptable ou non du test effectué, voire comme illustré de donner une information concernant l'évaluation du résultat relativement à un test effectué précédemment, à savoir par exemple mieux ou « moins bien ». L'écran peut également afficher un bouton permettant de mémoriser le choix d'une couleur, protection, soin ou odeur et d'une zone, après avoir effectué un essai sur la zone en question.

Le choix de la couleur ou protection ou soin ou odeur s'effectue par exemple avec une échelle similaire à celle décrite en référence à la figure 36.

Le système informatique est agencé pour mémoriser les données sous forme de table de correspondance par exemple, de manière à associer à une zone du visage des paramètres permettant de reproduire le mélange distribué lors du test. Ces paramètres comportent par exemple les teneurs relatives de chacun des produits de base du distributeur dans le mélange, la quantité Q distribuée, ainsi que des données additionnelles telles que par exemple le nom de la zone, la date de distribution du mélange et/ou tout autre identifiant du mélange, les identifiants des produits de base, la période de l'année, notamment la saison, l'âge de l'utilisateur, son sexe, son nom ou prénom, le nom d'un évènement associé au maquillage protection soi ou parfumage, par exemple un anniversaire, entre autres données, la quantité de produit adéquate pour la zone. Les données annexes peuvent permettre à l'utilisateur de reproduire plus facilement un effet qu'il a considéré comme adéquat pour une période de l'année, ou rappelant un évènement de la vie, ou pour donner un effet de rajeunissement.

Ces données peuvent être mémorisées dans le système informatique 100, par exemple dans l'appareil précité et/ou sur un serveur distant avec lequel l'appareil communique, ou encore dans une mémoire électronique intégrée au distributeur 11.

Ainsi, selon l'invention, l'utilisateur peut amener le distributeur à délivrer une première matière, à l'appliquer sur une première zone du visage, puis la juger adéquate ou non. Si le résultat est satisfaisant, l'utilisateur peut l'enregistrer en l'indexant à la zone ; si le résultat est non satisfaisant, l'utilisateur peut commander une nouvelle matière pour refaire les opérations ci-dessus.

Le système informatique peut être utilisé dans ce contexte de diverses façons.

Par exemple, comme illustré à la figure 40, l'utilisateur choisi à une étape 1010 une couleur ou soin ou protection ou odeur à essayer, en utilisant par exemple l'échelle 1011 apparaissant sur l'écran, en déplaçant le bouton de réglage 1012.

Ensuite, le choix est transmis au distributeur 11, à l'étape 1015.

Par exemple, l'appareil transmet les quantités à distribuer de chacun des produits de base, et le circuit électronique 81 se charge de commander les moteurs en conséquence.

A l'étape 1016, l'utilisateur appuie sur le bouton de commande 12 du distributeur 11, ce qui provoque par exemple la distribution d'une dose du mélange, de la couleur ou effet soin, protecteur ou odeur sélectionnée par l'utilisateur.

Le mélange est par exemple distribué dans la coupelle 115, puis prélevé et appliqué par l'utilisateur sur les joues ou toute autre zone renseignée sur l'interface, à l'étape 1020.

Dans des variantes, le produit est appliqué à l'aide d'un aérographe ou par tout autre moyen tel que décrit précédemment.

L'utilisateur renseigne ensuite à l'étape 1022 le système informatique à l'aide des boutons 1021, sur le résultat.

Si l'utilisateur indique que le résultat est satisfaisant, le système lui propose par exemple de valider à l'aide d'un bouton les paramètres de l'essai afin de les mémoriser à l'étape 1031.

Si le résultat n'est pas jugé satisfaisant par l'utilisateur et signalé comme tel à l'aide du bouton 1032, le résultat peut néanmoins être automatiquement sauvegardé à l'étape 1034.

On peut ainsi indexer à chaque zone non seulement la ou les couleurs ou soins ou protections ou odeurs adéquates mais aussi la ou les couleurs ou soins ou protection ou odeurs qui ne vont pas du tout à cette zone.

L'utilisateur peut alors effectuer un nouvel essai sur la même zone en revenant à l'étape 1010.

Si l'utilisateur était satisfait du résultat, il peut également souhaiter effectuer un nouvel essai, par exemple sur une zone différente du visage.

Le cas échéant, si l'utilisateur n'est pas satisfait, l'interface peut lui proposer d'indiquer si le résultat est estimé mieux ou moins bien que l'essai précédent, à l'aide de boutons correspondants 1040 et 1041.

Dans ce cas, le système informatique peut être agencé pour déterminer si au vu des renseignements entrés par l'utilisateur une proposition peut être automatiquement effectuée quant au choix de la nouvelle couleur ou soin ou protection ou odeur à tester.

Le cas échéant, un questionnaire peut s'afficher pour aider le système informatique à proposer une couleur ou soin ou protection ou odeur au vu des essais effectués et de l'évaluation qui en a été faite par l'utilisateur ou par un professionnel qui l'assiste.

Par exemple, si la couleur est jugée comme « non adéquate », le système peut recevoir de l'utilisateur une information additionnelle, par exemple « trop clair », qui l'aidera à proposer une nouvelle couleur plus en adéquation avec ce qu'attend l'utilisateur.

Il peut être intéressant que le système informatique puisse recevoir une information de comparaison du résultat par rapports aux essais précédents, par exemple « c'est mieux » ou « c'est moins bien » et que de là, le système soit capable d'en déduire la nouvelle couleur ou soin ou protection ou odeur à proposer.

Une autre option est que le système informatique puisse recevoir une information de comparaison par rapport à une cible, par exemple « c'est presqu'idéal » et que de là, le système soit capable d'ajuster automatiquement ses modifications de couleur ou soin ou protection ou odeur.

En l'occurrence, s'il reçoit l'information selon laquelle le résultat souhaité est presque atteint, le système peut adopter de petits niveaux de modification de la couleur couleur ou soin ou protection ou odeur et revoir l'échelle de réglage en conséquence.

Dans le cas où le système de distribution propose de lui-même les mélanges à tester, il peut se fonder sur des scénarios d'essais préprogrammés et modifier le suivi du scénario selon les réussites ou échecs de l'évaluation. Ainsi, par exemple, si dès la troisième application de produit, il reçoit une information selon laquelle la couleur ou soin ou protection ou odeur est presqu'idéale pour l'utilisateur, le système de distribution peut sortir du programme et se laisser guider ensuite par les instructions de l'opérateur.

D'une façon générale, l'utilisateur peut être aidé par un système expert dans le choix des couleurs ou soins ou protections ou odeurs à essayer.

Ce système expert est par exemple un programme exécuté sur l'appareil avec lequel le distributeur communique ou sur le distributeur lui-même, qui se fonde sur des réponses à un questionnaire et/ou sur des mesures, par exemple de la couleur de la peau, effectuées par un capteur spécifique ou une caméra. L'utilisateur peut ainsi se faire aider par une évaluation instrumentée, par exemple un capteur de couleur ou d'image ou d'odeur. Le système expert peut encore être implémenté sur un serveur distant avec lequel l'appareil ou le distributeur échange des informations. L'opérateur peut encore envoyer une image de son visage à un spécialiste qui peut préprogrammer le choix des couleurs ou soin ou protection ou odeur de départ. Dans un autre exemple de mise en oeuvre, l'utilisateur présente au système informatique une photo de son visage, et le système informatique est agencé pour l'analyser et créer un programme définissant les zones à tester et les premiers produits à délivrer, à la fois en couleur, soin, protection et odeur et quantité. Par exemple, le système informatique peut être agencé pour sélectionner automatiquement des couleurs, soins, protection ou odeur à proposer à l'utilisateur en effectuant l'acquisition d'une photographie à l'étape 1070, comme illustré à la figure 42. Par exemple, l'appareil qui communique avec le distributeur 11 est équipé d'une caméra, et l'utilisateur prend une photographie de son visage. L'image est ensuite analysée à l'étape 1071, et des couleurs couleur ou soin ou protection ou odeur sont proposées pour chaque zone du visage à l'étape 1072, selon par exemple des règles prédéfinies d'accord des couleurs.

Le système de distribution peut être orienté par l'utilisateur pour décider la couleur couleur ou soin ou protection ou odeur mais aussi la quantité de produit à délivrer. Par exemple, l'utilisateur peut indiquer « nez » ou « tache » et le système de distribution est agencé pour adapter la dose distribuée selon une cartographie en mémoire des doses à distribuer en fonction des zones à traiter.

Le système informatique peut guider l'utilisateur dans le choix des couleurs ou soin ou protection ou odeur du mélange à essayer, afin de limiter le nombre d'essais nécessaires jusqu'à l'obtention d'un résultat plaisant pour l'utilisateur.

Il est ainsi possible, comme illustré à la figure 41, qu'après l'application d'un mélange délivré par le distributeur sur une zone donnée du visage à l'étape 1060, le système informatique demande à l'utilisateur si le résultat est satisfaisant ou non, et effectue de lui-même, si le résultat est considéré comme étant non satisfaisant, un changement 1061 des paramètres du distributeur de façon à modifier le mélange distribué.

L'utilisateur n'a alors qu'a effectuer un nouvel essai avec le mélange modifié.

Lorsqu'un mélange est signalé comme étant satisfaisant, le système informatique peut mémoriser les paramètres correspondants afin de permettre de recréerultérieurement le mélange.

Le système peut alors recommencer les étapes précédentes pour une nouvelle zone d'application.

Lors des essais successifs, l'opérateur n'a pas besoin de traiter tout le visage. Il peut par exemple choisir entre 3 et 8 zones de petites surfaces, par exemple 5 zones. Le système de distribution est alors avantageusement prévu pour interpoler et/ou extrapoler les données concernant les couleurs ou soins ou protections ou odeurs considérées comme adéquates, afin de calculer les couleurs qui devraient être considérées comme adéquates sur des zones sur lesquelles l'exercice n'a pas été fait.

A la fin de l'apprentissage, le système peut généner une visualisation des couleur ou soin ou protection ou odeur adéquates sur les différentes zones, testées ou calculées.

Le système de distribution peut être agencé pour signaler si certaines couleur ou soin ou protection ou odeur apparaissent aberrantes, se fiant en cela à une comparaison par rapport à des cartographies types en mémoire. Ainsi, il peut proposer à l'utilisateur de refaire tout ou partie de l'exercice de cartographie.

Une fois l'apprentissage du système informatique effectué, c'est-à-dire que des couleur ou soin ou protection ou odeur de mélanges ont été identifiées comme convenant à l'utilisateur pour traiter certaines zones, l'utilisateur qui souhaite se maquiller ou soigner ou protéger ou parfumer n'a qu'à rappeler la zone à traiter, à l'étape 1080 de la figure 43, et le système peut proposer automatiquement à l'utilisateur à l'étape 1081 une couleur ou soin ou protection ou odeur de mélange adéquate.

Dans la variante illustrée à la figure 44, l'utilisateur sélectionne une couleur ou soin ou protection ou odeur à l'étape 1090 et le système informatique propose à l'étape 1091 une zone où appliquer un mélange de cette couleur ou soin ou protection ou odeur, sur la base d'informations précédemment collectées sur la base des tests effectués.

La zone proposée est par exemple celle où une couleur ou soin ou protection ou odeur identique ou proche a déjà été appliquée et le résultat jugé acceptable par l'utilisateur.

La figure 46 illustre un exemple de mise en oeuvre de l'invention où après avoir effectué des tests sur différentes zones à l'étape 2010, l'utilisateur signale au système le ou les mélanges qu'il estime procurer le meilleur résultat, ce qui permet au système de connaître les paramètres correspondants à l'étape 2012. Ensuite, à l'étape 2014, le système peut proposer à l'utilisateur des références de produits commerciaux ayant les mêmes propriétés ou des propriétés proches.

Dans une variante, le système adresse à un centre de fabrication distant les paramètres permettant de produire une composition de même formulation ou ayant les mêmes propriétés que celle du mélange que l'utilisateur a testé et trouvé satisfaisant.

La figure 45 illustre la possibilité de délivrer à l'aide du distributeur plusieurs doses 2020a à 2020d de mélanges différents, de façon juxtaposée sur un support 2021, de façon à permettre leur application sur des régions distinctes voisines d'une même zone.

L'utilisateur peut d'un seul coup appliquer une série de couleur ou soin ou protection ou odeur afin de cerner rapidement couleur ou soin ou protection ou odeur adéquate. Les matières présentes sur le support 2021 peuvent avoir été choisies par l'opérateur lui-même ou proposées par le système de distribution.

Le support 2021 est par exemple mobile par rapport au boîtier du distributeur et déplacé séquentiellement pour déposer les mélanges correspondants dans les différentes zones 2020a à 2020d, étant par exemple similaire aux supports décrits en référence aux figures 29 ou 29A. L'utilisateur peut alors facilement comparer les résultats entre les différentes régions, et signaler au système le mélange produisant le meilleur effet.

On a illustré à la figure 47 un système qui permet d'aider l'utilisateur à se traiter son visage, notamment à choisir les bonnes couleur ou soin ou protection ou odeur.

Ce système permet l'établissement d'une liaison vidéo, par exemple via internet, entre une caméra 2060 d'un premier site 2061 et un deuxième site 2062.

La caméra 2060 est par exemple intégrée à une tablette ou un téléphone intelligent qui constitue le système informatique 100.

On permet au deuxième site 2062 de piloter directement ou indirectement le distributeur 11 présent sur le premier site 2061.

Ainsi, la personne présente au premier site peut appliquer le mélange distribuéet envoyer vers le deuxième site 2062 une image correspondante, pour recevoir en retour une information relative au résultat.

Le deuxième site 2062 peut comporter un écran de visualisation 2064 qui permet à un conseiller présent devant cet écran de voir le résultat ou comprendre via les commentaires le résultat avec le mélange distribué par le distributeur et de conseiller la personne. Ce conseiller peut agir en retour sur le distributeur 11 pour modifier la couleur, soin protection ou odeur du mélange et l'adapter au mieux au visage de la personne présente sur le premier site. Le protocole d'échange des données entre les deux sites permet ainsi l'envoi d'instructions de commande au distributeur 11, soit directement, soit par l'intermédiaire du systèmeinformatique 100 présent sur le premier site. Ainsi, la personne présente sur le deuxième site gouverne le mélange délivré par le distributeur 11. La première personne peut se maquiller ou réaliser le soin ou la protection ou le parfumage sous les yeux de la seconde. La seconde personne voit sur son écran le résultat de l'essai, et ainsi peut corriger le mélange qu'elle va commander à distance, jusqu'à obtenir le traitement idéal.

De préférence, la liaison vidéo entre les deux sites est une liaison duplex, de sorte que l'utilisateur présent sur le premier site peut voir sur l'écran du système informatique l'image du conseiller. Ce dernier peut adresser à l'utilisateur présent sur le premier site un tutoriel, le cas échéant.

La mémorisation des paramètres de réglage du distributeur 11, une fois qu'un mélange donné est estimé satisfaisant, peut être commandée depuis le deuxième site.

L'interface peut servir à définir des programmes de maquillage ou soin ou protection ou odeur où l'on définit l'ordre des zones à traiter ou l'ordre des produits à proposer.

### Exemples

On réalise un distributeur 11 tel que celui illustré à la figure 3 et décrit précédemment en liaison avec les trois premeirs aspects de l'invention.

Pour illustrer le quatrième aspect de l'invention, plusieurs matériaux ont été investigués pour la partie corps et les parties en mouvement.
PEmd (Dow)
PEhd (Dow)
Polyacétal (POM)
PET

A l'exception du polyacétal, réalisé par usinage, les autres sont réalisés par injection.

### Exemple 1 (quatrième aspect de l'invention)

On réalise un set de trois produits de base (les proportions sont massiques) :
F1 :

| | |
|---|---|
| Anéthole | 6% (odeur anisée) |
| Eau | 5% |
| Ethanol absolu qs | 100 |

F2 :

| | |
|---|---|
| Phényléthyl éthanol | 6% (odeur de rose) |
| Eau | 5% |
| Ethanol absolu qs | 100 : |

F3 :

| | |
|---|---|
| Jaguar HP 60 | 2% |
| Eau qs | 100% |

### Test 1

On met les trois produits de base dans trois cartouches avec :
F1 dans une cartouche en PEhd
F2 dans une cartouche en PEhd
F3 dans une cartouche en PET

On met les trois cartouches dans les trois compartiments, C1, C2 et C3
Puis, on réalise à t=0, un premier mélange M0 avec les proportions volumiques suivantes : C1 30%, C2 30%, C3 40%.

Puis on réalise à t= 1j, un second mélange M1 avec les mêmes ratios.

Puis on réalise à t= 30 j, un troisième mélange M30 avec les mêmes ratios.

Enfin on réalise à la main (balance de précision) ,un mélange M avec les mêmes compositions et les mêmes ratios.

Puis, on présente les mélanges à des experts.

Les mélanges réalisés M0, M1 et M30 sont satisfaisants car proche olfactivement du mélange M (un mélange de rose et d'anis).

Le résultat obtenu avec le POM est encore acceptable à t=1j. Le résultat obtenu avec le PS est inacceptable car on ne sent presque pas le parfum (en comparaison à M).

### Test 2

On intervertit les compositions F1 et F3 en les changeant de cartouche.

Ainsi, on met les trois formules dans trois cartouches avec :
F1 dans une cartouche en PET
F2 dans une cartouche en PEhd
F3 dans une cartouche en PEhd

On met les trois cartouches dans les trois compartiments, C1, C2 et C3
Puis, comme précédemment, on réalise à t=0, un premier mélange M0 : C1 30%, C2 30%, C3 40%.

Puis on réalise à t= 1j, un second mélange M1 avec les mêmes ratios.

Puis on réalise à t= 30 j, un troisième mélange M30 avec les mêmes ratios.

Enfin on réalise à la main (balance de précision) ,un mélange M avec les mêmes compositions et les mêmes ratios.

Puis, on présente les mélanges à des experts.

Le mélange réalisé M0 est satisfaisant car proche olfactivement du mélange M.(un mélange de rose et d'anis). Les autres (M1 et M30) ne sont pas satifisants car olfactivement différents (les résultats ne contiennent pas la facette anisée).

### Test 3

On introduit F1 dans une cartouche en POM.

Ainsi, on met les trois formules dans trois cartouches avec :
F1 dans une cartouche en POM
F2 dans une cartouche en PEhd
F3 dans une cartouche en PET

On met les trois cartouches dans les trois compartiments, C1, C2 et C3
Puis, comme précédemment, on réalise à t=0, un premier mélange M0 : C1 30%, C2 30%, C3 40%.

Puis on réalise à t= 1j, un second mélange M1 avec les mêmes ratios.

Puis on réalise à t= 30 j, un troisième mélange M30 avec les mêmes ratios.

Enfin on réalise à la main (balance de précision) ,un mélange M avec les mêmes compositions et les mêmes ratios.

Puis, on présente les mélanges à des experts.

Les mélanges réalisés M0 et M1 sont satisfaisants car proches olfactivement du mélange M.(un mélange de rose et d'anis). Le dernier (M30) n'est pas satifisant car olfactivement différent (les résultats ne contiennent pas la facette anisée).

### Exemple 2 (quatrième aspect de l'invention)

On reprend la configuration de l'exemple 1-test 1

On demande à des experts en parfum de choisir leur mélange idéal.

Pour cela, chacun tente plusieurs mélanges, faisant varier les taux de C1, C2, C3 pour changer l'odeur et la puissance du parfum. A chaque fois, ils réalisent 100 mg de composition, qu'ils testent par appréciation olfactive selon leurs goûts.

Ils sélectionnent trois mélanges qu'ils mémorisent dans le système.

Ils peuvent chaque jour utiliser ces trois mélanges selon leurs envies du moment. Pour cela, ils choisissent les quantités à délivrer. Le système est suffisamment précis pour être capable de libérer des doses spécialement petites tout en respectant l'odeur du mélange qu'ils réalisent. (jusqu'à 20 mg).

### Exemple 3 (quatrième aspect de l'invention)

On réalise un set de trois compositions :
F1 :

| | |
|---|---|
| Curcumine | 0,5% (couleur orange) |
| Huile végétale de tournesol qs | 100 |

F2 :

| | |
|---|---|
| Solvant red 27 | 0,5% (couleur rouge brique) |
| Huile végétale de tournesol qs | 100 : |

F3 :
Huile végétale de tournesol

### Test 1

On met les trois formules dans trois cartouches avec :
F1 dans une cartouche en PEmd
F2 dans une cartouche en PEmd
F3 dans une cartouche en PEmd

On met les trois cartouches dans les trois compartiments, C1, C2 et C3
A t=0, un expert utilise un premier mélange M0 : C1 20%, C2 0%, C3 80%.

Puis, il applique par touches de 100 mg ce mélange sur le visage d'une personne de couleur foncée.

Puis, on complète en délivrant un second mélange P0 : C1 20%, C2 20%, C3 60% pour appliquer sur les joues.

Puis, on complète en délivrant troisième mélange Q0 : C1 20%, C2 60%, C3 20% pour appliquer sur les paupières.

Une fois reparti chez lui avec le système et les instructions, la personne reproduit chez elle une fois par mois les mêmes applications. On obtient un résultat concordant.

### Test 2

Il consiste à réaliser la même opération avec en utilisant d'autres cartouches :
F1 dans une cartouche en PEmd
F2 dans une cartouche en PET
F3 dans une cartouche en PEmd

Le test est le même que dans l'exemple 3-test 1.

Après 1 mois, le résultat concorde mais après 2 mois, le résultat ne concorde pas (couleur orangée dans les trois zones).

### Exemple 4 (quatrième aspect del'invention)

On reprend la configuration de l'exemple 2-test 1

On demande à une personne de rechercher la couleur qui lui sied le mieux pour le maquillage de ses lèvres.

Pour cela, elle tente plusieurs mélanges, faisant varier les taux de C1, C2, C3 pour changer la couleur et la puissance de la couleur. A chaque fois, ils réalisent 100 mg de composition, qu'ils testent par appréciation colorielle sur le dos de leur main.

Ils sélectionnent un mélange qu'ils mémorisent dans le système.

Ils peuvent chaque jour utiliser leur mélange.

### Exemple 5 (quatrième aspect de l'invention)

On réalise un set de trois compositions :
F1 :

| | |
|---|---|
| Curcumine | 2% (couleur orange) |
| Myristate d'isopropyle qs | 100% |

F2 :

| | |
|---|---|
| Ethanol | 100% |

F3 :

| | |
|---|---|
| Myristate d'isopropyle | 100% |

On met les trois formules dans trois cartouches avec :
F1 dans une cartouche en PEhd
F2 dans une cartouche en PEhd
F3 dans une cartouche en PEhd

On met les trois cartouches dans les trois compartiments, C1, C2 et C3
A t=0, un expert utilise un premier mélange M0 : C1 10%, C2 0%, C3 90%.

Puis il l'applique sur le visage d'une personne.

Le lendemain, l'expert demande à la personne le confort qu'elle a ressenti.

Elle remonte le taux de C2 de 10 points au détriment de C3 et ainsi de suite, jusqu'à trouver le mélange produisant le meilleur confort zone par zone.

Puis, elle fait varier le taux de C1 jusqu'à trouver la meilleure intensité colorielle.

Elle finit par trouver le mélange le plus adéquat pour chaque zone.

La personne peut partir avec le système et appliquer ses mélanges zone par zone.

### E) Système permettant d'ajuster le niveau de couvrance d'un fond de teint sans impacter le niveau de matité/brillance

Dans le cas des peaux tachées, la plupart du temps, les zones de peau tachées ne présentent qu'une proportion faible de la surface du visage. Pour traiter ces peaux, on a l'habitude d'utiliser soit un produit très couvrant (mais amenant des défauts d'effet masque) soit un produit peu couvrant (mais avec l'inconvénient de laisser visibles les taches). Il est possible d'utiliser plusieurs produits d'effets couvrant différents, mais généralement, ils présentent des niveaux de brillance/matité différents ce qui rend le maquillage très visible. (et peut faire ressortir les zones tachées).

Il faudrait une solution pour donner accès à toute une gamme de niveau de couvrance et qui puissent être mis sur un même visage, sans qu'on puisse détecter les différences.

Pour résoudre ces problèmes, il n'existe que peu de solutions.

Une première approche consiste à acheter de nombreux produits et à tous les essayer. Cette approche est coûteuse et génère souvent des pertes dans la mesure où l'on ne retient généralement qu'un petit nombre des matières colorées essayées.

Une seconde approche consiste à tester, en magasin, différents produits. Elle ne convient pas toujours, car il est très difficile de se figurer le résultat en quelques instants et dans un lieu dépourvu des repères habituels. En particulier, pour apprécier pleinement, l'effet d'un maquillage dans un magasin, il faudrait pouvoir l'éclairer de la même manière que dans les conditions futures d'utilisation, ce qui est rarement possible. En général, ce n'est qu'en testant un maquillage pendant la journée, qu'on se rend compte s'il convient ou non. De plus, si certains magasins disposent de conseillers et permettent d'effectuer des essais, ce n'est pas le cas d un grand nombre d'autres points de vente et des achats effectués sur internet.

Une autre approche a été testée sans toutefois se développer. Elle consisteà réaliser manuellement ses produits par mélange de plusieurs produits de couleur. Cela peut s'avérer relativement difficile à mettre en oeuvre, car il est peu aisé de reproduire exactement plusieurs fois le même mélange, et l'on a du mal à réaliser rapidement les mélanges dont on a besoin au moment du maquillage.

Parmi les essais d'automatisation de la fabrication d'une composition cosmétique personnalisée qui ont pu être tentés, nombreux sont ceux dans lesquels les solutions proposées permettent de réaliser des mélanges dans des quantités d'environ 100 g ou parfois moins, mais pas dans les très petites proportions dont la personne qui se maquille a généralement besoin, c'est-à-dire de l'ordre du gramme ou beaucoup moins.

Pour illustrer cette problématique, on peut imaginer le cas d'une personne qui veut cacher deux imperfections de l'ordre du cm2 sur son visage. Pour la première zone, elle a besoin de trouver le mélange correspondant, puis d'en délivrer une très petite quantité, par exemple d'environ 10 mg. Pour la seconde, elle a besoin de changer le réglage du distributeur, puis de délivrer une toute petite quantité également.

Par conséquent, le choix des matières colorées conférant les meilleurs résultats reste difficile pour un grand nombre de personnes.

Il existe par conséquent un besoin pour faciliter la recherche d'un produit de maquillage répondant aux attentes d'un consommateur, et permettant à celui-ci de réaliser des mélanges dans des conditions fiables en quantités diverses.

Il existe aussi un besoin d'appliquer sur le même visage plusieurs produits, avec des pouvoirs masquants différents. En particulier, les gens peuvent avoir des zones du visage plus ou moins tachés et donc nécessitant des forces de masquages différentes. Il est donc intéressant de pouvoir disposer d'un moyen de produire des formulations maquantes à différentes forces de masquages.

La réalisation, par mélanges, de produits masquants pose un problème important. Comme ils sont basés sur des ingrédients particulaires (pigments, nacres), on obtient alors des mélanges qui, selon le taux de masquage choisi (donc la quantité d'ingrédients particulaires) donnent sur la peau des aspects de matité/brillance différents.

Il existe donc un besoin pour perfectionner les systèmes de distribution permettant de délivrer des produits de couleur variable tout en gardant le même niveau de matité/brillance, afin notamment de faciliter leur utilisation et améliorer la qualité du maquillage.

L'invention repose ici selon son cinquième aspect sur un système de distribution qui permet de générer des mélanges à partir de produits de base. Ces produits de base peuvent avoir des couleurs différentes, de telle sorte que l'on puisse faire varier la couleur du mélange. Les produits de base peuvent encore permettre de faire varier la couvrance du mélange, de telle sorte que la couleur qui résulte de l'application du mélange sur les matières kératiniques humaines varie, en étant plus ou moins proche de celle desdites matières. Les produits de base peuvent aussi faire varier la couleur et la couvrance. Ainsi, la notion de couleur doit être comprise avec une acceptation large et englobe des mélanges dont la couleur varie après application du fait de leur couvrance plus ou moins élevée et de la couleur de la peau sousjacente.

L'invention a pour objet, selon un cinquième de ses aspects, un système de distribution d'un produit, comportant un distributeur recevant au moins deux cartouches ayant chacune un réservoir et contenant respectivement des premier et deuxième produits de base, le premier produit de base comportant un pigment, le deuxième produit de base comportant une charge distincte du pigment du premier produit de base, le distributeur permettant de délivrer au moins ces deux produits de base dans des proportions réglables.

L'invention selon ce cinquième aspect peut présenter encore une ou plusieurs des caractéristiques suivantes :
- la taille D50 en volume de particule de la charge est comprise entre 100 nm et 1 mm, mieux entre 1 micron et 100 microns, encore mieux entre 2 microns et 50 microns,
- la taille D50 en volume des particules de pigment est comprise entre 100 nm et 25 microns, mieux entre 200 nm et 10 microns,
- le pigment peut-être choisi parmi les pigments minéraux, et de préférence les pigments minéraux modifiés hydrophobes, notamment ceux d'oxyde de fer ou d'oxyde de titane,
- le pigment peut comporter un enrobage comportant au moins un composé lipophile ou hydrophobe,
- la charge peut être choisie parmi le talc, le mica, la silice, le kaolin, les poudres de poly-β-alanine et de polyéthylène, les poudres de polymères de tétrafluoroéthylène, les poudres de nylon, les poudres de polymethacrylate de methyle, la lauroyl-lysine, l'amidon, le nitrure de bore, les microsphères creuses polymériques telles que celles de chlorure de polyvinylidène/acrylonitrile, de copolymères d'acide acrylique, les microbilles de résine de silicone, les particules de polyorganosiloxanes élastomères, le carbonate de calcium précipité, le carbonate et l'hydro-carbonate de magnésium, l'hydroxyapatite, le sulfate de barium, les oxydes d'aluminium, les poudres de polyuréthane, les charges composites, les microsphères de silice creuses, et les microcapsules de verre ou de céramique, les particules qui ont la forme de portions de sphères creuses, et leurs mélanges, préférentiellement étant du talc ou du mica,
- la charge peut comporter un enrobage comportant au moins un composé lipophile ou hydrophobe,
- le premier produit de base et le deuxième produit de base peuvent comporter une émulsion inverse,
- la teneur massique en pigment dans le premier produit de base peut être supérieure ou égale à 5% et de préférence supérieure à 10%
- la teneur massique en charge dans le deuxième produit de base peut être supérieure ou égale à 0,5%%, de préférence supérieure à 1%
- le système peut comporter une troisième cartouche avec un troisième produit de base,
- les cartouches peuvent être reçues de façon amovible dans le distributeur, chaque produit peut quitter la cartouche par un canal de sortie de la cartouche, le canal de sortie étant défini par un embout de distribution de la cartouche entraîné en rotation relative par rapport à un corps de la cartouche par un mécanisme d'entraînement du distributeur pour distribuer le produit de base contenu dans la cartouche.

Selon ce cinquième aspect de l'invention, celle-ci a encore pour objet un procédé d'application d'un produit cosmétique, de maquillage et/ou de soin, sur les matières kératiniques humaines, comportant le réglage du distributeur en fonction de la zone à traiter, et la distribution du produit par prélèvement dans les cartouches des produits de base dans les proportions correspondant au réglage du distributeur.

L'invention repose ainsi selon ce cinquième aspect sur un système de distribution, des compartiments contenant au moins deux produits de base différents lesquels contiennent (les deux) des ingrédients particulaires choisis pour créer un effet de masquage. Ces formulations contiennent de préférence une émulsion inverse concentrée en pigment pour l'une et concentrée pour l'autre en charges. Ainsi, en réalisant les mélanges, la brillance et la matité ne changent pas. On peut donc faire varier le niveau de couvrance tout en gardant un aspect égal. On peut programmer le système pour qu'il délivre pour chaque endroit du visage le meilleur mélange couvrant (ni trop fort, ni trop faible). L'aspect global ne permettra pas de deviner que le visage a été traité avec différents niveaux de couvrance. Le système peut aussi être utilisé pour faire varier le niveau de couvrance selon les envies et les momentspar exemple plus couvrant pour une soirée, plus léger pour la journée).

### Système de distribution

Le système de distribution peut être constitué par un seul appareil fonctionnant de manière autonome, de préférence manipulable d'une main, ou par un appareil qui fonctionne en interagissant avec d'autres pièces ou appareils. Il peut s'agir par exemple de différentes interfaces de sorties qui sont montées sur le distributeur en fonction du type de maquillage à réaliser, comme cela sera précisé plus loin. Il peut s'agir également d'un système informatique qui échange des informations avec le distributeur pour le piloter, ce système informatique comportant par exemple un terminal portable tel qu'un téléphone intelligent, un photophone, une tablette, un ordinateur personnel, ou un terminal dédié.

De préférence, le distributeur est agencé pour mettre sous pression un ou plusieurs compartiments contenant le ou les produits de base, par le biais de dispositifs de dosage volumétrique, de préférence un moteur entraînant en déplacement un piston dans le compartiment correspondant.

Le distributeur peut être formé d'un boîtier et d'au moins deux ou trois compartiments, et de préférence d'autant de moteurs. Par exemple, la rotation des moteurs entraîne des vis sans fin, lesquelles poussent les pistons de chaque compartiment. L'avance des pistons est par exemple contrôlée par le nombre d'impulsions de commande envoyées aux moteurs et/ou par la durée de fonctionnement de ces derniers. Les moteurs peuvent être alimentés séquentiellement, ou de préférence simultanément.

Par exemple, les moteurs sont alimentés durant un cycle élémentaire de fonctionnement pendant une courte durée les uns après les autres, ou les uns en même temps que les autres, de façon à distribuer des microdoses correspondantes.

Les cycles élémentaires sont répétés, avec éventuellement un temps d'arrêt entre eux, qui laisse le temps aux produits de base de s'écouler hors des compartiments.

Les compartiments peuvent être définis par des cartouches qui se retirent lorsqu'elles sont vides. En variante, les compartiments sont présents à demeure et remplis à nouveau une fois vide.

Chaque cartouche peut être fermée par un bouchon démontable pour permettre le nettoyage de la cartouche.

De préférence, le boîtier du distributeur est de forme allongée selon un axe longitudinal, ce qui peut faciliter sa manipulation, et les cartouches sont disposées autour de cet axe, dans le boîtier.

De préférence, les cartouches sont mises en place par l'arrière, et le mélange est délivré par l'avant. La mise en place des cartouches peut s'effectuer individuellement, ou en variante les cartouches constituent un ensemble monobloc lors de leur mise en place.

Les cartouches peuvent comporter chacune un mécanisme de dosage volumétrique, comportant un piston mu par un mécanisme d'entraînement du distributeur, dans une direction s'accompagnant d'une diminution du volume intérieur contenant le produit de base et d'une expulsion du produit. Il peut être intéressant que les cartouches aient une région de leur paroi au moins qui soit transparente, afin de permettre de voir la couleur du produit contenu à l'intérieur.

Le mécanisme d'entraînement peut comporter un système de motorisation formé de moteurs couplés à des réducteurs, de forme allongée parallèlement à l'axe longitudinal du distributeur, et disposés entre les cartouches. Ce positionnement des moteurs et cartouches rend le distributeur particulièrement compact.

Le produit de base peut sortir de façon étanche de la cartouche correspondante puis circuler dans un canal prévu à cet effet dans le boîtier du distributeur, avant de sortir de celui-ci.

Les cartouches se terminent avantageusement par un embout réalisé de telle sorte que, une fois la cartouche insérée dans le boîtier du distributeur, l'extrémité de l'embout débouche au ras du boîtier. En variante, l'embout est suffisamment long pour dépasser du boîtier et connecter ainsi différentes interfaces de sortie susceptibles d'être rapportées sur le boîtier du distributeur.

Grâce au mécanisme d'entraînement comportant des moteurs pour faire avancer des pistons, il est possible de délivrer de façon précise des mélanges en de très petites quantités. Ainsi, le mécanisme d'entraînement peut délivrer les produits de base avec à un débit minimal inférieur ou égal à 50 µL/s, mieux inférieur ou égal à 20 µL/s, encore mieux inférieur ou égal 10 µL/s. De préférence, le mécanisme d'entraînement délivre des débits compris entre 20 et 100 µL/s, mieux compris entre 40 µL/s et 60 µL/s. Il est donc possible de réaliser facilement un mélange d'environ 10 mg. Un tel système de distribution est alors idéal pour réaliser de petites touches de maquillage, pour couvrir par exemple une zone de 1 cm², mieux une zone de 0, 5 cm².

Il est aussi possible de réaliser des plus grandes quantités de mélange comme celles nécessaires au maquillage d'une joue, ou d'un visage. Ces quantités restent néanmoins relativement faibles, par exemple une quantité comprise entre 100 et 500 mg, mieux comprise entre 150 et 250 mg.

L'invention a ainsi pour objet, selon l'un de ses aspects, un système de distribution comportant un distributeur présentant un boîtier, et au moins une cartouche reçue dans le boîtier du distributeur, cette cartouche comportant un corps et un piston mobile dans le corps, le boîtier comportant un mécanisme d'entraînement motorisé pour déplacer le piston de la cartouche.

De préférence, la cartouche comporte un embout de distribution par lequel s'effectue la sortie du produit, et cet embout de distribution est entraîné en rotation par le mécanisme d'entraînement pour déplacer le piston. L'embout peut comporter au moins un relief d'anti-rotation, mieux deux ergots d'anti-rotation diamétralement opposés.

L'embout peut porter un joint d'étanchéité, notamment un joint torique. Ainsi, au changement de cartouche, le joint est également changé, ce qui permet de s'affranchir de l'usure du joint.

Le distributeur peut comporter une carte électronique de pilotage du mécanisme d'entraînement motorisé, cette carte électronique étant traversée par le ou les embouts. Cela peut permettre de réaliser une carte s'étendant sensiblement sur toute la section du distributeur, donc de regrouper sur une seule carte tous les composants électroniques du distributeur, et ainsi de gagner en compacité et fiabilité. La carte peut s'étendre sensiblement perpendiculairement à l'axe longitudinal du boîtier. La carte peut porter un interrupteur de commande du fonctionnement du distributeur.

Le système de distribution peut être agencé pour fonctionner selon au moins deux modes de distribution.

Dans un premier mode, dit « continu », le mélange est distribué tant qu'une pression est exercée sur l'interrupteur de commande.

Dans un deuxième mode, dit « dose », une quantité prédéfinie du mélange est distribuée à chaque pression sur l'interrupteur.

Le ou les embouts peuvent déboucher à une extrémité du boîtier. Cela peut permettre de réduire le volume mort, comme cela sera détaillé dans la suite.

Le ou les embouts peuvent présenter à leur extrémité un système d'obturation pour éviter le séchage des produits dans le conduit, par exemple une membrane cicatrisante

La cartouche peut comporter une vis creuse sur laquelle le piston est vissé, le piston pouvant se déplacer axialement sur la vis lorsque celle-ci tourne ; le piston est empêché de tourner dans le corps de la cartouche. Par exemple, la friction du piston sur le corps de la cartouche peut suffire à l'empêcher de tourner quand la vis tourne.

Préférentiellement, la rotation est rendue impossible avec un corps de cartouche de section non circulaire et un piston non déformable.

Le couple des moteurs peut être déterminé électroniquement en fonction du courant absorbé, et servir par exemple à détecter une fin de course du piston. Une information concernant le couple peut être transmise à distance à un système informatique présentant une interface homme machine, pour permettre de surveiller le bon fonctionnement du distributeur.

Afin d'ajuster la teinte, le système de distribution selon l'invention doit permettre à l'utilisateur de faire varier le volume délivré provenant de chaque compartiment.

De façon préférée, le distributeur est piloté par un système informatique, qui est intégré au distributeur, ou externe à celui-ci, le distributeur étant alors apte à échanger avec le système informatique des informations par protocole sans fil ou câblé.

Le distributeur peut ainsi être piloté pour permettre l'ajustement de la teinte par distribution simultanée ou séquentielle, et contrôlée, de plusieurs produits de base de couleurs différentes.

La distribution des produits de base peut être continue ; si dans ce cas, les volumes de chacun des produits de base sont distribués d'une seule traite, simultanément ou successivement.

Dans le cas d'une distribution simultanée, il est utile de pouvoir ajuster les débits respectifs des différents produits de base pour que le mélange distribué corresponde à celui souhaité à chaque instant. Un tel mode de distribution peut convenir en particulier lors de la distribution du mélange par pulvérisation, à l'aide d'un aérographe. Pour ajuster les débits il est possible par exemple de jouer sur la vitesse de déplacement des pistons, par exemple, dans le cas d'un entrainement des pistons par une vis sans fin en faisant varier la vitesse de rotation des moteurs qui entrainent la vis. Les produits peuvent aussi être distribués de façon impulsionnelle avec un temps de distribution et un temps d'arrêt à chaque cycle. En jouant sur le rapport cyclique, il est possible d'agir sur le débit.

Tous les produits peuvent être délivrés simultanément lors du temps de distribution ; en variante, les cycles des différents produits sont déphasés, de sorte qu'un produit est distribué pendant un temps d'arrêt des autres produits.

Dans un mode particulier de l'invention, le mélange est délivré dans une cavité d'un contenant, pouvant fermer de façon hermétique ou non, par exemple en forme de coupelle, dans lequel on peut faire glisser un applicateur, en particulier un stylet ou une brosse. Un tel système de distribution est spécialement adapté aux liners, gloss et autres formules appliquées sans contact direct avec les mains. Ce contenant peut être amovible.

Par exemple, il sert de distributeur de rouge à lèvres et dispose d'un système de distribution, à vis par exemple. Lorsqu'il n'est pas amovible, le contenant peut être réalisé avec le corps du distributeur. Lorsqu'il est amovible, il peut constituer une interface de sortie parmi d'autres pouvant être montées sur le distributeur.

Les compartiments, et en particulier les cartouches, peuvent contenir tout ou partie du mécanisme d'entraînement, et par exemple le système de motorisation, ou mieux, une partie du système de motorisation, et ce, afin de réduire le nombre de pièces en mouvement dans le corps du distributeur à l'extérieur des cartouches. Par exemple, les cartouches comportent le rotor du moteur. Une fois les cartouches installées dans le corps du distributeur, les rotors sont mis en interaction avec les stators.

Le système de distribution est avantageusement agencé pour permettre de lancer des séquences préprogrammées dans lesquelles le mélange délivré par celui-ci est modifié de façon continue ou discontinue. Un mode « dégradé » permet par exemple passer progressivement d'un mélange A à un mélange B. Dans le cas d'une application par pulvérisation, notamment à l'aide d'un aérographe, cela permet de réaliser simplement les dégradés. Un mode « Alternatif » permet par exemple de passer rapidement d'un mélange

A à un mélange B plusieurs fois de suite. Dans le cas d'une application par pulvérisation, on peut ainsi réaliser un dépôt multicouche, avec des formulations différentes pour deux couches adjacentes superposées. Un autre mode permet par exemple de proposer plusieurs mélanges successifs préprogrammés, le système informatique indiquant à chaque fois l'usage qu'il faut en faire à l'utilisateur, par exemple par affichage sur un écran.

Dans le cas d'une application manuelle, les mélanges sont distribués par exemple dans une coupelle. La personne se maquille à l'endroit recommandé avec un mélange correspondant prélevé dans la coupelle, puis nettoie éventuellement la coupelle et commande la délivrance d'un nouveau mélange ; l'opération est répétée autant de fois que nécessaire pour se maquiller complètement.

L'homogénéisation des mélanges distribués peut se faire de différentes façons selon les cas d'utilisation. Dans le cas d'une application manuelle, elle peut se faire directement sur la zone d'application au moment de l'application ou dans la coupelle avant prélèvement ; dans le cas d'une application à l'aérographe, c'est la tuyère de l'aérographe qui sert de chambre de mélange ; dans le cas où le mélange est distribué dans un contenant en vue d'une utilisation ultérieure, l'homogénéisation peut être faite à la main ou en faisant passer les produits distribués dans une chambre de mélange, située entre le distributeur et le contenant ou directement intégrée au contenant, comme détaillé par la suite.

Le produit peut être délivré par le système de distribution et utilisé extemporanément. En variante, le produit délivré par le système de distribution est conditionné et utilisé ultérieurement, par exemple à plusieurs reprises, avec par exemple un jour d'intervalle au moins entre deux utilisations.

### Ingrédients particulaires

Les ingrédients particulaires sont typiquement des particules de taille comprises entre 100 nm et 1mm, mieux entre 200 nm et 100 microns, encore mieux entre 200 nm et 50 microns
Au sens de l'invention, par "taille" d'une particule, on entend la taille moyenne en volume D50, qui correspond à la taille de particule définie de sorte que 50% en volume des particules ont une taille inférieure à D50.

La taille moyenne en volume peut être appréciée par diffraction de la lumière à l'aide d'un granulomètre laser MasterSizer de Malvern, lesdites particules à évaluer étant dispersées dans un milieu liquide tel que par exemple le néopentanoate d'octyldodécyle.

Les ingrédients particulaires sont des pigments et des charges :

### Pigments

On entend par « *pigments* » des particules blanches ou colorées, minérales ou organiques, insolubles dans un milieu aqueux, destinées à colorer et/ou opacifier la composition et/ou le film résultant. Ces pigments peuvent être blancs ou colorés, minéraux et/ou organiques.

Selon un mode de réalisation, la taille des particules de pigments selon l'invention est comprise entre 100 nm et 25µm, de préférence entre 200 nm et 10µm.

Les pigments utilisés selon l'invention sont choisis préférentiellement parmi les pigments minéraux, et de préférence parmi les pigments minéraux modifiés hydrophobes.

Les pigments minéraux modifiés hydrophobes sont plus particulièrement des pigments modifiés hydrophobes d'oxyde de fer et/ou de dioxyde de titane.

La composition selon l'invention comprend avantageusement au moins un pigment enrobé par au moins un composé lipophile ou hydrophobe.

L'enrobage peut aussi comprendre au moins un composé additionnel non lipophile.

Au sens de l'invention, « *l'enrobage* » d'un pigment selon l'invention désigne de manière générale le traitement en surface total ou partiel du pigment par un agent de surface, absorbé, adsorbé ou greffé sur ledit pigment.

Les pigments traités en surface peuvent être préparés selon des techniques de traitement de surface de nature chimique, électronique, mécano-chimique ou mécanique bien connues de l'homme de l'art. On peut également utiliser des produits commerciaux.

L'agent de surface peut être absorbé, adsorbé ou greffé sur les pigments par évaporation de solvant, réaction chimique et création d'une liaison covalente.

Selon une variante, le traitement de surface consiste en un enrobage des pigments.

L'enrobage peut être réalisé par exemple par adsorption d'un agent de surface liquide à la surface des particules solides par simple mélange sous agitation des particules et dudit agent de surface, éventuellement à chaud, préalablement à l'incorporation des particules dans les autres ingrédients de la composition de maquillage ou de soin.

L'enrobage peut être réalisé par exemple par réaction chimique d'un agent de surface avec la surface des particules solides de pigment et création d'une liaison covalente entre l'agent de surface et les particules. Cette méthode est notamment décrite dans le brevet US 4,578,266.

Le traitement de surface chimique peut consister à diluer l'agent de surface dans un solvant volatile, à disperser les pigments dans ce mélange, puis à évaporer lentement le solvant volatile, de manière à ce que l'agent de surface se dépose à la surface des pigments.

Selon un mode de réalisation particulier de l'invention, les pigments peuvent être enrobés selon l'invention par au moins un composé choisi parmi les agents de surface siliconés ; les agents de surface fluorés ; les agents de surface fluoro-siliconés ; les savons métalliques ; les acides aminés N-acylés ou leurs sels ; la lécithine et ses dérivés ; le trisostéaryle titanate d'isopropyle ; le sébaçate d'isostéaryle ; les cires naturelles végétales ou animales ; les cires synthétiques polaires ; les esters gras ; les phospholipides ; et leurs mélanges.

Selon un mode de réalisation particulier, les pigments peuvent être enrobés par au moins un composé choisi parmi les acides aminés N-acylés ou leurs sels ; le trisostéaryle titanate d'isopropyle ; les agents de surface siliconés ; les cires naturelles végétales ou animales ; la lécithine hydrogénée, les esters gras ; et leurs mélanges.

Selon un mode de réalisation plus particulièrement préféré, les pigments peuvent être enrobés par un acide aminé N-acylé et/ou un de ses sels, en particulier par un dérivé d'acide glutamique et/ou un de ses sels, notamment un stéaroyl glutamate, comme par exemple le stéaroyl glutamate d'aluminium.

Selon un mode de réalisation plus particulièrement préféré, on utilisera des pigments enrobés hydrophobes choisis parmi les dioxydes de titane et les oxydes de fer, enrobés de stéaroyl glutamate d'aluminium, par exemple commercialisé sous la référence NAI^{®} par MIYOSHI KASEI.

### Charges

Ces charges sont des particules incolores ou blanches, solides de toutes formes, qui se présentent sous une forme insoluble et dispersée dans le milieu de la composition.

A titre illustratif de ces charges, peuvent être cités le talc, le mica, la silice, le kaolin, les poudres de poly-β-alanine et de polyéthylène, les poudres de polymères de tétrafluoroéthylène (Téflon^{®}), les poudres de nylon, les poudres de polymethacrylate de methyle, la lauroyl-lysine, l'amidon, le nitrure de bore, les microsphères creuses polymériques telles que celles de chlorure de polyvinylidène/acrylonitrile comme l'Expancel^{®} (Nobel Industrie), de copolymères d'acide acrylique, les microbilles de résine de silicone (Tospearls^{®} de Toshiba, par exemple), les particules de polyorganosiloxanes élastomères, le carbonate de calcium précipité, le carbonate et l'hydro-carbonate de magnésium, l'hydroxyapatite, le sulfate de barium, les oxydes d'aluminium, les poudres de polyuréthane, les charges composites, les microsphères de silice creuses, et les microcapsules de verre ou de céramique. On peut également utiliser des particules, qui ont la forme de portions de sphères creuses, telles que décrites dans les demandes de brevet JP-2003 128 788 et JP-2000 191 789.

Les charges peuvent comporter un enrobage comportant au moins un composé lipophile ou hydrophobe.

L'enrobage peut être réalisé par exemple par adsorption d'un agent de surface liquide à la surface des particules solides par simple mélange sous agitation des particules et dudit agent de surface, éventuellement à chaud, préalablement à l'incorporation des particules dans les autres ingrédients de la composition de maquillage ou de soin.

L'enrobage peut être réalisé par exemple par réaction chimique d'un agent de surface avec la surface des particules solides de charge et création d'une liaison covalente entre l'agent de surface et les particules.

Le traitement de surface chimique peut consister à diluer l'agent de surface dans un solvant volatile, à disperser les pigments dans ce mélange, puis à évaporer lentement le solvant volatile, de manière à ce que l'agent de surface se dépose à la surface des charges.

Selon un mode de réalisation particulier de l'invention, les charges peuvent être enrobées selon l'invention par au moins un composé choisi parmi les agents de surface siliconés ; les agents de surface fluorés ; les agents de surface fluoro-siliconés ; les savons métalliques ; les acides aminés N-acylés ou leurs sels ; la lécithine et ses dérivés ; le trisostéaryle titanate d'isopropyle ; le sébaçate d'isostéaryle ; les cires naturelles végétales ou animales ; les cires synthétiques polaires ; les esters gras ; les phospholipides ; et leurs mélanges.

Selon un mode de réalisation particulier, les charges peuvent être enrobées par au moins un composé choisi parmi les acides aminés N-acylés ou leurs sels ; le trisostéaryle titanate d'isopropyle ; les agents de surface siliconés ; les cires naturelles végétales ou animales ; la lécithine hydrogénée, les esters gras ; et leurs mélanges.

Selon un mode de réalisation plus particulièrement préféré, les charges peuvent être enrobées par un acide aminé N-acylé et/ou un de ses sels, en particulier par un dérivé d'acide glutamique et/ou un de ses sels, notamment un stéaroyl glutamate, comme par exemple le stéaroyl glutamate d'aluminium.

Selon un mode de réalisation plus particulièrement préféré, on utilisera des charges enrobées hydrophobes choisies parmi le talc et le mica, enrobées de stéaroyl glutamate d'aluminium, par exemple commercialisée sous la référence NAI^{®} par MIYOSHI KASEI.

### Emulsions inverses / Compositions anhydre

Selon l'invention, lorsqu'il est précisé qu'une composition est sous la forme d'une émulsion inverse, il est entendu qu'elle peut également être alternativement sous une autre forme selon la composition considérée.

Un produit de base peut se présenter sous la forme d'une émulsion, par exemple une émulsion H/E, E/H, H/E/H ou E/H/E, et de préférence une émulsion inverse E/H, ou sous la forme d'une composition anhydre, comprenant notamment des composés carbonés et/ou siliconés, tels que des huiles hydrocarbonées et/ou siliconées.

Les émulsions selon l'invention sont de préférence des émulsions eau-dans-huile (E/H) encore appelées émulsions inverses, à savoir constituées d'une phase huileuse continue dans laquelle est dispersée la phase aqueuse sous forme de gouttelettes de manière à observer un mélange macroscopiquement homogène à l'oeil nu.

### Utilisation pour le maquillage d'une ou plusieurs zones précises de peau

Le système de distribution permet de se maquiller jour après jour, en ne traitant que les zones à masquer. Pour ce faire, on délivre de petites doses de maquillage, qu'on applique précisément et séquentiellement sur les zones correspondantes. Chaque petite dose est réalisée selon le mélange qui convient à la zone.

Dans un mode préféré de mise en oeuvre de l'invention, le système de distribution attend qu'on le renseigne sur une zone à traiter puis délivre le mélange correspondant. Il peut faire appel à une table de correspondance préprogrammée pour cela, cette table résultant par exemple d'un procédé d'apprentissage tel que défini plus loin. En variante, le système de distribution renseigne la personne, tout en délivrant un mélange, sur la zone sur laquelle celle-ci doit appliquer le mélange. Ainsi, le système de distribution peut suivre un programme d'application où il délivre dans un ordre donné, les différents mélanges à appliquer.

Dans un mode particulier de mise en oeuvre de l'invention, le système de distribution est renseigné sur les quantités à délivrer. Pour cela, il mémorise la correspondance entre la couleur, la zone du visage et la quantité nécessaire, ce qui permet de réduire les coûts et les pertes de produit, et de ne couvrir que faiblement la peau, évitant ainsi des effets d'occlusion. Ce faisant, il est possible d'utiliser des produits très couvrants et trop couvrants pour être appliqués sur l'ensemble du visage. Ainsi, il est possible d'obtenir des maquillages d'aspects naturels, voire indétectables.

Le système permet donc de réaliser des applications localisées et ce en permettant d'obtenir le même niveau de matité/brillance.

Le système de distribution peut aussi permettre, en facilitant la distribution de petites quantités et leur usage rapide, de réduire la durée de conservation des produits, permettant ainsi de réduire les risques d'évolution et/ou les quantités de conservateurs à employer.

Le système de distribution est adapté à traiter les zones qu'on veut masquer, sans avoir à masquer l'ensemble du visage.

Il peut aussi être utilisé pour masquer une ou des taches, avec un seul niveau de matité/brillance.

Dans une variante spécialement avantageuse, le système peut être utilisé pour application sur le fond du visage une formule peu masquante puis une ou deux applications sur des zones précises du visage des formules plus masquantes. On peut aussi commencer par applications sur des zones précises du visage des formules masquantes puis appliquer sur le fond du visage une application d'une formule peu masquantes.

Dans le second cas, on prend le temps, avant d'appliquer la seconde couche, d'attendre que le film produit par l'application sur les zones précises devienne suffisamment cohésif pour que la seconde couche n'emporte pas la première couche.

Ou bien, on applique la seconde couche sans frotter (spray par exemple, ou éponge).

Lorsque l'utilisateur recherche la couleur à appliquer sur une zone du visage, il est intéressant de mémoriser la couleur qui va bien pour chaque zone, et le système de distribution est ainsi avantageusement agencé pour mémoriser cette couleur et la zone correspondante. Ainsi, en utilisant les informations mémorisées, à chaque utilisation, on peut obtenir la délivrance du même mélange pour chaque zone, ou, si l'on traite plusieurs zones, la même série de mélanges pour une même série de zones.

Le système de distribution peut aussi être agencé pour permettre de traiter une zone en faisant varier les couleurs application après application. Ainsi, la personne peut maquiller les lèvres avec différentes couleurs, qu'elle choisit au jour le jour selon ses goûts. Cette approche est aussi adaptée aux paupières ou aux cils, ainsi qu'au maquillage du visage car la personne peut avoir envie de changer de couleur de teint. Par exemple, les jours de la semaine, la personne réalise un fond de teint couleur claire, et le weekend, un fond de teint de couleur hâlée, ou un maquillage des yeux d'une couleur un jour, et d'une autre couleur un autre jour. Idem pour des variations de couvrance.

Le système de distribution peut être agencé pour permettre à l'utilisateur de changer de couleur selon ses goûts du jour, du moment, de ses tenues, du climat. Ainsi, on prévoit avantageusement un système d'aide à la décision pour guider l'utilisateur dans ses choix de couleur (au sens large).

On peut prévoir aussi un système d'aide pour équilibrer les couleurs sur un même visage et participer à la réalisation d'un maquillage global réussi.

Il peut être souhaitable que plusieurs personnes d'un même groupe, par exemple une famille, puissent utiliser le système de distribution, réduisant ainsi les coûts et minimisant la place occupée. Cette solution est spécialement adaptée aux voyages ou aux hôtels, campings, avions, campings cars, boutiques, écoles... Pour ce faire, on peut prévoir que le système de distribution puisse être renseigné sur la personne qui l'utilise, afin d'accéder à des données personnelles préalablement mémorisées.

### Utilisation en continu pour un maquillage en dégradé

Dans cette application, le système de distribution, pendant qu'il délivre le produit, change la formulation du mélange. De plus, on déplace la sortie des produits de base ou du mélange relativement à un contenant ou un support définissant une surface d'application. Dans un mode particulier de mise en oeuvre de l'invention, le système de distribution est agencé pour calculer l'évolution du mélange en fonction de la couleur C1, d'une zone à traiter et de la couleur C2, d'une autre zone à traiter. Par exemple, sachant que le menton nécessite une couleur C1 et que la joue nécessite une couleur C2, le système de distribution peut faire varier la formulation du mélange pendant qu'il le délivre pour réaliser un dégradé de couleur entre ces deux couleurs. Cela permet par exemple de mieux masquer les imperfections du visage en faisant en sorte que le résultat final soit réaliste, ou de faire des dégradés de couleur à des fins d'embellissement. Le système de distribution peut encore être agencé de telle sorte que l'utilisateur puisse commander une variation de couleur du mélange distribué sans que les couleurs de départ et/ou d'arrivée n'aient été préalablement fixées. Pour ce faire, le système de distribution peut disposer d'un système de localisation, ou d'auto-localisation, et déduire d'une table de correspondance les couleurs C1 et C2 qu'il doit réaliser, et donc les évolutions de mélange qu'il doit réaliser.

Ces différents effets pourront être réalisés, avec un effet de matité/brillance constant.

Le système de distribution peut comporter une tête de sortie, en particulier dans le cas d'un aérographe, qui est mobile et pilotée. Cette option permet alors, sans bouger le reste du système de distribution, de réaliser des dégradés. Par exemple, on localise le système de distribution près de la joue, puis on déclenche un système de commande qui va piloter automatiquement la variation de la formulation du mélange et le mouvement de la tête de sortie, de façon à par exemple avoir le centre de la joue plus rouge que sa périphérie, avec un dégradé entre les deux.

Le système de distribution peut encore être utilisé pour réaliser des produits sur mesure, que l'on conserve pour réaliser plusieurs applications.

Il est aussi possible de réaliser des produits solides ou semi-solides.

### Fabrication de compacts « à façon » ou autres produits solides ou semi-solides

Le système de distribution peut être agencé pour permettre de choisir un mélange et de le délivrer dans un contenant tel qu'une coupelle. Le mélange comporte de préférence des composés qui sont tels que le mélange puisse prendre en masse.

Plus préférentiellement, on utilise des composés qui rendent la prise en masse spécialement rapide. Ces composés sont soit déposés dans le contenant avant ou après son remplissage avec les autres constituants, soit sont prévus dans les compartiments du distributeur avec les autres constituants des produits de base, soit sont compris dans le distributeur dans un compartiment spécialement prévu pour les contenir.

On peut ainsi distribuer des compositions spécifiques qui peuvent par réaction chimique, biochimique ou physicochimique, durcir de façon accélérée après évacuation.

Ces compositions sont spécialement adaptées à la réalisation des compacts, c'est-à-dire :
- Prennent en masse,
- donnent un matériau qui peut se déliter en cas de frottement, et sont préférentiellement colorées.

De préférence, ces compositions sont très riches en particules solides, avec par exemple plus de 10% en masse de particules solides par rapport à la masse totale de la composition, mieux plus de 20% en masse de particules solides par rapport à la masse totale de la composition, encore mieux plus de 30% en masse de particules solides par rapport à la masse totale de la composition, préférentiellement entre 10 et 40% en masse de particules solides par rapport à la masse totale de la composition. Ces compositions peuvent contenir des particules absorbantes, ou des composés réactifs, tels que ceux qui réagissent en contact avec l'air, par exemple du cyanoacrylate ou des alphasilanes, ou qui réagissent à la lumière, notamment UV.

Le contenant dans lequel le mélange est distribué peut comporter un composé A et les compositions distribuées un composé B, A et B étant choisis pour réagir entre eux et solidifier le mélange.

Dans un mode particulier de mise en oeuvre de l'invention, le système de distribution intègre un moyen de chauffage, par exemple à résistance électrique, pour la réalisation de rouges à lèvres ou autres produits cireux. Dans ce cas, les produits de base sont chauffés avant d'être délivrés.

Le système de distribution peut aussi comporter un moyen pour apporter de l'énergie, thermique et/ou lumineuse, après distribution du mélange dans un contenant, par exemple une résistance électrique ou une diode LED, notamment UV. Cette énergie peut accélérer la prise en masse du mélange distribué.

Préférentiellement, le mélange est homogénéisé avant la prise en masse.

Les produits, de couvrance différentes, présenteront des effets de matité/brillance équivalents

### Réalisation de palettes de couleurs

Le système de distribution peut comporter un support, ayant plusieurs régions, et être agencé pour générer automatiquement plusieurs mélanges déposés dans lesdites régions, par exemple une série de couleurs adaptées à différentes parties du visage.

Le support peut définir plusieurs cavités pour recevoir les mélanges ou porter plusieurs contenants, par exemple sous forme de coupelles, éventuellement séparables du support.

Dans un cas particulier, le support prend la forme d'un visage avec des régions pour recevoir les mélanges pour des zones d'applications ciblées.

Le support être mobile, notamment rotatif, par rapport au corps du distributeur, et par exemple entraîné en déplacement par le distributeur, pour remplir successivement différents logements ou contenants.

Les produits, sur la palette pourront être de couvrance différentes, tout en présentant des effets de matité/brillance équivalents

### Distributeur à coupelle

Il existe un intérêt pour bénéficier d'un système de distribution capable de délivrer un mélange pouvant être facilement prélevé par l'utilisateur. Par ailleurs, dans le cas où les produits de base délivrés par le système de distribution ne sont pas déjà mélangés, il existe un besoin pour permettre à l'utilisateur d'effectuer aisément le mélange.

L'invention a encore pour objet un système de distribution d'au moins un produit de maquillage, comportant une coupelle, et un distributeur pour remplir la coupelle avec au moins un produit, la coupelle étant solidaire du distributeur au moins pendant son remplissage.

La coupelle est parfois encore appelée « creuset » et ce terme doit être compris avec une acceptation large.

Par « coupelle solidaire du distributeur » il faut comprendre que la coupelle est retenue, notamment immobilisée, au moins temporairement sur le distributeur, étant par exemple fixée sur celui-ci par des vis, par aimantation, par encliquetage, par verrouillage baïonnette, par serrage, ou réalisée avec une partie du corps du distributeur par moulage de matière. La coupelle lorsqu'elle est solidaire du distributeur permet de manipuler celui-ci d'une main, la coupelle restant en place sur le distributeur au cours des mouvements de ce dernier.

Le distributeur peut être proposé à l'utilisateur avec la coupelle déjà en place.

En variante, celle-ci est installée par l'utilisateur à la première utilisation du système de distribution.

De préférence la coupelle est moins profonde que large, ce qui facilite l'accès à celle-ci, et permet de prélever le produit, notamment le mélange avec un applicateur ou le doigt.

De préférence, la coupelle est séparable du distributeur, et constitue une interface de sortie qui peut être choisie parmi un ensemble d'interfaces de sortie pouvant être montées sur le distributeur, au choix de l'utilisateur en fonction du maquillage à réaliser, comme détaillé plus loin.

De préférence, le système de distribution comporte plusieurs orifices de remplissage en des produits de base différents, débouchant dans la coupelle. Ainsi, le mélange de ces produits peut avoir lieu dans la coupelle.

La coupelle a de préférence un fond de forme concave vers l'extérieur, ce qui peut faciliter son nettoyage par l'utilisateur entre deux utilisations.

De plus, cela peut faciliter le prélèvement du produit par l'utilisateur, et le mélange des produits de base.

De préférence, le distributeur permet de délivrer au moins deux produits de base dans la coupelle, dans des proportions réglables, et mieux au moins trois produits.

Dans un exemple de mise en oeuvre, le système de distribution comporte au moins deux coupelles pouvant être sélectivement alimentées par le distributeur. Cela peut permettre à l'utilisateur de remplir rapidement ces deux coupelles avec des mélanges dont les caractéristiques sont différentes. Cela peut faciliter les essais de matières colorées, et/ou permettre de préparer plusieurs mélanges de couleurs différentes destinées au maquillage de zones respectives du visage. Les coupelles peuvent être associées à des identifiants rappelant à l'utilisateur à quelle zone du visage un mélange contenu dans une coupelle donnée est destiné.

Les coupelles peuvent être mobiles relativement au distributeur, étant par exemple portées par un support mobile, tel qu'une tourelle mobile en rotation par rapport au distributeur, ou par un tiroir mobile en translation par rapport au distributeur.

Le système de distribution peut comporter un couvercle de fermeture de la coupelle. Ce couvercle de fermeture est de préférence transparent pour permettre à l'utilisateur de visualiser la couleur du mélange contenu à l'intérieur.

Lorsque la coupelle est séparable du distributeur, elle peut être introduite le cas échéant dans un boîtier permettant de la transporter plus facilement, ce boîtier pouvant comporter le cas échéant un miroir et/ou un applicateur. Le couvercle du boîtier peut dans ce cas servir de couvercle pour la coupelle.

Le volume de la coupelle peut être compris entre 2 et 1000 mm³, mieux entre 100 et 1000 mm³, encore mieux entre 250 et 750 mm³.

Le ou les produits de base délivrés dans la coupelle sont de préférence des fonds de teint, mais en variante il s'agit de produits de maquillage des lèvres ou des paupières.

La coupelle est de préférence de forme symétrique de révolution. En variante, elle est de contour polygonal ou autre. Son plus grand diamètre intérieur, ou celui du cercle inscrit dans le cas d'un contour non circulaire, est de préférence compris entre 2 et 100 mm, préférentiellement entre 5 et 40 mm. Sa profondeur est de préférence comprise entre 1 et 10 mm, mieux entre 3 et 8 mm. De préférence, la taille et la forme de la coupelle permettent soit une application directe du mélange sur la peau, soit une préhension au doigt ou avec un applicateur. La coupelle peut être réalisée avec un matériau élastiquement déformable, ce qui permet par exemple d'inverser la concavité du fond de la coupelle et de vider celle-ci plus facilement ou de l'utiliser pour appliquer le produit.

La coupelle peut être dépourvue de mélangeur ; dans ce cas, les produits de base peuvent arriver du distributeur dans la coupelle à l'état non mélangé, par des orifices de distribution respectifs distincts. En variante, le distributeur intègre un mélangeur et les produits de base arrivent déjà mélangés dans la coupelle.

La coupelle peut aussi intégrer un mélangeur statique, comme détaillé plus loin, qui est alimenté par des orifices de remplissage distincts du distributeur, et qui délivre de préférence le mélange dans une cavité de la coupelle située au-dessus du mélangeur.

L'invention a encore pour objet un procédé de préparation d'un produit de maquillage, comportant l'étape consistant à remplir une coupelle d'un système de distribution tel que défini plus haut avec au moins un produit de base provenant du distributeur.

Les produits, de couvrance différentes, présenteront des effets de matité/brillance équivalents

Plusieurs produits peuvent être délivrés dans le fond de la coupelle, puis mélangés à l'aide du doigt ou d'un applicateur, ou d'un mélangeur statique intégré à la coupelle.

La coupelle est de préférence remplie par le dessous. Il a été proposé dans le passé des systèmes de distribution à l'aide d'une sonotrode.

La coupelle selon l'invention n'est pas destinée à entrer en vibration pour distribuer le ou les produits amenés par le ou les canaux d'alimentation de celle-ci. Elle est différente d'une sonotrode. De préférence, la coupelle est réalisée en matière plastique.

### Mélangeur intégré à l'interface de sortie

Il existe un intérêt pour bénéficier d'un système de distribution capable de délivrer un mélange pouvant être facilement utilisé, notamment prélevé par l'utilisateur, sans nécessiter une action additionnelle de mélange de la part de celui-ci.

Selon l'un de ses aspects, indépendamment ou en combinaison avec les autres aspects de celle-ci, et notamment ce qui précède, l'invention a pour objet un système de distribution comportant un distributeur ayant des canaux de sortie de produits de base et une interface de sortie séparable du distributeur, cette interface ayant un mélangeur statique, délivrant de préférence le mélange dans une cavité où il peut être prélevé.

Le mélangeur statique peut être situé sous la cavité précitée. Le système de distribution est alors particulièrement adapté à la création de compacts, en utilisant comme interfaces de sortie des coupelles à mélangeur statique intégré. Dans ce cas, on vient remplir la cavité de la coupelle en produit par le dessous. Après passage dans le mélangeur statique, les produits de base mélangés viennent recouvrir le mélangeur.

Selon cet aspect de l'invention, il est possible d'utiliser plusieurs interfaces de sortie et de les remplir avec des mélanges respectifs différents, sans avoir à purger le mélangeur, ce qui réduit les pertes de produit. L'interface de sortie peut être à usage unique, le cas échéant.

De préférence, le mélangeur statique comporte une chambre centrale communiquant avec des canaux d'admission des produits de base. Cette chambre centrale peut communiquer avec une chambre périphérique comportant une série de cloisons qui agissent comme des déflecteurs pour le mélange, et créent un cisaillement de celui-ci.

La chambre périphérique peut comporter une cloison annulaire ajourée définissant des ajours par lesquels passe le mélange en circulant dans la chambre périphérique. Les chambres centrale et périphérique peuvent être fermées supérieurement par une paroi qui définit le fond de la cavité recevant le mélange.

Le fond de la chambre périphérique peut être de forme hélicoïdale autour de l'axe de la coupelle et de hauteur diminuant au fur et à mesure que l'on progresse vers la sortie. Cette dernière peut déboucher en avant d'une rampe de liaison entre le fond de la chambre périphérique et la paroi supérieure du mélangeur, cette rampe de liaison étant de préférence une portion d'hélice prolongeant l'hélice formée par la paroi de fond de la chambre périphérique.

De préférence la chambre périphérique comporte la cloison annulaire précitée et des cloisons radiales qui forcent le mélange à circuler alternativement entre des régions supérieure et inférieure de la chambre périphérique et entre des régions radialement intérieure et extérieure, le mélange circulant par exemple d'une région supérieure et radialement extérieure à une région inférieure et radialement extérieure en passant à travers la cloison annulaire précitée.

Le mélangeur peut comporter un corps extérieur dans lequel est reçue une pièce formant le coeur du mélangeur, le corps extérieur fermant radialement à l'extérieur la chambre périphérique et comportant un montant qui sépare les chambres centrale et périphérique.

Le corps extérieur du mélangeur et le coeur du mélangeur peuvent être chacun réalisés d'une seule pièce par moulage par injection.

### Volume mort réduit

Il existe un intérêt pour réduire les pertes de produit lors des changements de formulation du mélange, ainsi que pour permettre de faire varier le plus rapidement possible la couleur du mélange au cours de l'application, notamment en cas de couplage du distributeur avec un aérographe.

Selon l'un de ses aspects, indépendamment ou en combinaison avec les autres aspects de celle-ci, notamment ce qui précède, l'invention a pour objet un système de distribution d'un produit de maquillage, comportant un distributeur recevant au moins deux cartouches ayant chacune un réservoir contenant un produit de base, ce dernier quittant la cartouche par un canal de sortie de la cartouche, ce canal de sortie débouchant à l'extérieur du distributeur ou à proximité de sa surface externe.

Le canal de sortie peut notamment déboucher dans une zone de prélèvement du mélange ou à proximité de celle-ci, notamment à moins de 5 mm, mieux à moins de 3 mm, mieux à moins de 1mm, mieux à fleur.

La section transversale du canal de sortie est par exemple comprise entre 1 et3 mm².

Ainsi, chaque produit de base provenant d'une cartouche peut quitter le distributeur sans se mélanger avec un produit de base d'une autre cartouche et l'on minimise le volume mort qui ne peut être prélevé et susceptible d'accroître l'inertie du système. Le produit est plus rapidement disponible sans avoir à circuler dans des canaux spécifiques du boîtier du distributeur, évitant une étape fastidieuse de purge si changement de cartouche.

L'extérieur du distributeur peut être la zone de prélèvement du produit, notamment lorsque le distributeur est réalisé avec une coupelle non prévue pour être enlevée, dans laquelle le mélange est distribué, ou une zone du distributeur destinée au montage d'une interface de sortie amovible, qui définit la zone de prélèvement. Cette interface de sortie peut comporter une coupelle telle que définie ci-dessus. Cette zone de montage correspond par exemple à l'extrémité du boîtier du distributeur en l'absence de l'interface de sortie. La zone de montage peut être sensiblement plane et perpendiculaire à l'axe longitudinal du boîtier du distributeur.

Le distributeur peut comporter trois cartouches de produits de base.

Le distributeur peut comporter des logements pour recevoir les cartouches, lesquelles sont de préférence reçues de façon amovible dans le distributeur. Ce dernier peut comporter des passages pour des conduits des cartouches définissant les canaux de sortie.

La longueur de ces conduits est de préférence telle que les conduits se situent légèrement en retrait du fond ou à affleurement de la cavité servant au prélèvement du produit, ou en variante légèrement en retrait ou à affleurement de la face d'extrémité du boîtier du distributeur définissant la zone de montage.

Ces conduits des cartouches peuvent être des embouts servant à entraîner en déplacement des pistons au sein des cartouches, comme détaillé plus haut.

### Interfaces de sortie multiples

Il existe un besoin pour pouvoir, à l'aide d'un même système de distribution, réaliser facilement des maquillages différents, et pouvoir si on le souhaite maquiller des zones aussi différentes que la peau, les lèvres ou les cils ou sourcils.

Selon l'un de ses aspects, indépendamment ou en combinaison avec les autres aspects de celle-ci, et notamment ce qui précède, l'invention a pour objet un système de distribution comportant un ensemble comportant un distributeur d'au moins un produit cosmétique, notamment de maquillage, et au moins deux interfaces de sortie pouvant se monter chacune de façon amovible sur le distributeur, ces interfaces de sortie permettant de recevoir le ou les produits délivrés par le distributeur, étant de préférence choisies parmi les suivantes :
- interface de sortie comportant un contenant, notamment une coupelle permettant un prélèvement du produit au doigt ou à l'aide d'un applicateur,
- interface de sortie permettant de délivrer le produit à un système de pulvérisation, notamment un aérographe,
- interface de sortie comportant plusieurs régions de réception du produit, mobile par rapport au distributeur,
- interface de sortie permettant de délivrer le produit à un embout de distribution.

De préférence, l'ensemble comporte au moins trois desdites interfaces de sortie, mieux les quatre interfaces de sortie.

Le distributeur peut comporter au moins deux produits de base différents et permettre de délivrer ceux-ci dans des proportions variables, et de préférence, le distributeur comporte trois produits de base différents et permet de délivrer ceux-ci dans des proportions variables.

Chaque interface de sortie peut comporter une base permettant sa fixation sur le distributeur. Cette fixation peut s'effectuer à l'aide de vis par exemple, mais de préférence la base est agencée pour permettre un démontage et un remplacement d'une interface de sortie sans nécessité d'outil. Il s'agit par exemple d'une fixation quart de tour ou à l'aide d'une bague de verrouillage externe.

L'interface de sortie et/ou le boîtier du distributeur peuvent comporter des joints d'étanchéité permettant une communication étanche entre le boîtier du distributeur et l'interface de sortie. Le cas échéant, le distributeur est agencé pour reconnaître l'interface de sortie qui est montée dessus, par exemple grâce à identifiant de l'interface de sortie sous forme de reliefs spécifiques, lesquels sont détectés par le distributeur, ou d'une puce électronique reconnue par le distributeur. Cela peut permettre d'adapter le fonctionnement du distributeur à l'interface de sortie montée dessus. Le distributeur peut communiquer à un système informatique des informations sur l'interface de sortie qu'il porte, et le système informatique peut afficher en fonction de ces informations un écran spécifique et/ou lancer un programme spécifique de commande des paramètres de fonctionnement du distributeur, afin par exemple d'adapter la dose distribuée et/ou le débit à la nature de l'interface de sortie.

Plusieurs interfaces de sortie peuvent être proposées initialement à l'utilisateur avec un distributeur commun au sein d'un même emballage, par exemple un coffret ou une boîte de carton.

L'invention a encore pour objet un procédé de maquillage, comportant l'étape consistant à sélectionner une interface de sortie, à la monter sur le distributeur, et à délivrer le ou les produits contenus dans le distributeur dans celle-ci.

### Cartographie et apprentissage

Le terme « cartographie » s'entend ici comme un procédé d'indexation d'une couleur et d'une zone, avec enregistrement.

Il se peut qu'une personne nécessite plusieurs niveaux de couvrance avec une seule matité/brillance pour l'ensemble des zones.

Il se peut aussi qu'elle nécessite plusieurs niveaux de couvrance avec un niveau de matité/brillance sur une partie du visage et un autre niveau de matité/brillance pour une autre zone. Par exemple, elle a plusieurs taches sur le front et plusieurs taches sur les joues. Ainsi, elle voudra réaliser plusieurs niveaux de couvrance pour le front avec un niveau de matité/brillance pour cette surface. Elle voudra réaliser plusieurs niveaux de couvrance pour les joues avec un niveau de matité/brillance pour cette surface.

La cartographie peut concerner des applications sur des zones plus petites qu'un cm2. Toutefois, l'oeil nu a alors du mal à discerner si le résultat obtenu est adéquat, et il est préférable de substituer l'évaluation à l'oeil nu par une évaluation instrumentée, avec grossissement. L'application de petites quantités de matière colorée peut être faite au doigt, avec des outils classiques tels que des pinceaux, ou avec des applicateurs spécialisés.

La cartographie peut être générée lors d'une période d'apprentissage, pendant laquelle l'utilisateur effectue des tests avec des mélanges sur différentes zones du visage ; une fois renseignée, la cartographie peut ensuite être utilisée pour le maquillage de tous les jours.

Des interfaces graphiques spécifiques peuvent servir pendant la période d'apprentissage et pendant la période d'utilisation de la cartographie.

En particulier, le système de distribution peut être utilisé avec une interface graphique où l'opérateur voit le visage, qui est par exemple schématisé, figuratif ou précis comme une photographie ou une simulation 3D. Dans ce cas, l'opérateur peut pointer une partie du visage sur l'écran pour voir apparaître et/ou délivrer la couleur adéquate.

L'interface graphique peut aussi laisser apparaître les autres zones du visage où l'utilisation de la même couleur est adéquate.

Pour réaliser la cartographie, l'opérateur applique une couleur puis réalise l'évaluation.

Les zones du visage peuvent être traitées les unes après les autres ; par exemple, on réalise l'exercice sur une partie de la joue, puis sur le nez, etc....

Une autre possibilité consiste à réaliser un mélange donné, et appliquer sur plusieurs zones ce même mélange. L'opérateur doit alors chercher la zone du visage pour laquelle la couleur est adaptée. Le mélange est ensuite indexé dans le système informatique en l'attribuant à la ou aux zones du visage pour lesquelles il convient.

L'invention a encore pour objet un procédé d'apprentissage d'un système de distribution comportant un distributeur permettant de distribuer un mélange de couleur variable, et un système informatique permettant de sélectionner une couleur et de mémoriser des données, comportant les étapes consistant à :
a) Sélectionner au moins une couleur à l'aide d'une interface du système informatique,
b) délivrer à l'aide du distributeur au moins un mélange de la couleur sélectionnée,
c) évaluer le ou les mélanges distribués après application sur au moins une zone du visage,
d) mémoriser les caractéristiques d'au moins un mélange, notamment un mélange que l'utilisateur souhaite pouvoir rappeler, et d'au moins une zone sur laquelle il a été testé.

Cette mémorisation peut s'effectuer notamment en vue d'une distribution ultérieure de ce mélange pour maquiller ladite zone.

De préférence, le système informatique est agencé pour permettre à l'utilisateur de signaler si le résultat du test est satisfaisant ou non, voire de le renseigner sur la comparaison à un test effectué précédemment.

On peut aussi réaliser un mélange donné, et chercher la zone du visage pour laquelle il est adapté. On enregistre alors le mélange en l'attribuant à la ou aux zones du visage pour lesquelles il est adapté, dans une table de correspondance qui servira par la suite, pour, partant d'une zone de la peau, en déduire le mélange à utiliser.

On peut procéder de la même façon avec d'autres mélanges pour réaliser une cartographie de l'ensemble du visage et ainsi avoir une table de correspondance complète du visage.

On peut aussi réaliser un mélange donné, l'appliquer sur une zone donnée, puis faire varier le mélange jusqu'à obtenir le mélange le plus adapté. On enregistre alors le mélange en l'attribuant à la ou aux zones du visage pour lesquelles il convient, dans une table de correspondance qui servira par la suite, pour, partant d'une zone de la peau, en déduire le mélange à utiliser.

De préférence, le système informatique évalue et mémorise les quantités utilisées zone par zone. Un tel procédé, par « touches d'essai », permet d'identifier le ou les produits dont a besoin la personne qui veut se maquiller. Ainsi, le système de distribution peut servir sur les points de vente pour conseiller les personnes qui veulent se maquiller ou à la maison pour bien définir les produits à commander.

L'interface du système informatique comporte de préférence un écran tactile affichant la couleur du mélange lors de sa sélection.

L'interface peut afficher un visage et permettre de renseigner le système informatique en sélectionnant la zone sur le visage affiché.

Le système informatique est de préférence agencé pour permettre d'associer une zone, des paramètres de reconstitution du mélange, et la date de l'essai et/ou tout autre identifiant du mélange.

Le système informatique est de préférence également agencé pour permettre d'associer en outre à ladite zone, aux paramètres de reconstitution du mélange, et à la date ou à l'identifiant du mélange, au moins l'une des données suivantes : le nom de la zone, la période de l'année, le nom d'un évènement, un identifiant de l'utilisateur, l'âge de l'utilisateur.

Les étapes a) à c) peuvent être renouvelées au moins une fois avant la mémorisation des caractéristiques du mélange à l'étape d).

Le système informatique peut être agencé pour rechercher dans une base de données une référence de produit commerciale, sur la base des caractéristiques du mélange identifié comme convenant à au moins une zone donnée, et la communiquer à l'utilisateur.

La sélection à l'étape a) peut s'effectuer avec l'aide d'un système expert externe ou non au système informatique.

Le système expert peut analyser une image de l'utilisateur, pour proposer une couleur de mélange au moins sur la base de l'image analysée.

L'étape a) peut être précédée par la proposition d'une couleur et d'une zone à tester avec un mélange de cette couleur, par le système informatique à l'utilisateur.

Le système informatique peut être agencé pour permettre à l'utilisateur de le renseigner sur l'appréciation du résultat du test de l'étape c) et pour générer une proposition de modification du mélange à sélectionner lors du retour à l'étape a).

Le système informatique peut être agencé pour proposer au moins une couleur de mélange à l'étape a) en fonction d'une zone d'application renseignée par l'utilisateur.

Le système informatique peut être agencé pour proposer au moins une zone d'application à l'étape a), en fonction d'une couleur renseignée par l'utilisateur.

Le distributeur peut délivrer à l'étape b) au moins deux mélanges de couleurs différentes, de préférence séparés, en vue de leur application simultanée sur la zone de test.

Cela peut faire gagner du temps, et faciliter la comparaison des résultats.

L'invention a encore pour objet un procédé de maquillage à l'aide d'un système de distribution selon cet aspect de l'invention, dans lequel :
a) L'utilisateur adresse une requête concernant un besoin de maquillage au système informatique,
b) celui-ci génère en retour une proposition de couleur en vue du maquillage d'une zone associée, sur la base de l'apprentissage effectué préalablement, et
c) le système informatique pilote le distributeur pour la production du mélange de la couleur proposée, notamment si celle-ci est validée par l'utilisateur.

Un tel procédé peut utiliser une cartographie précédemment établie avec l'utilisateur.

L'invention a encore pour objet un produit programme d'ordinateur comportant des instructions de code permettant, lorsque exécutées dans un système informatique, d'amener le système informatique à :
- Permettre à l'utilisateur de sélectionner au moins une couleur et/ou une zone d'application, notamment à l'aide d'une interface telle qu'un écran tactile,
- piloter un distributeur de façon à délivrer un mélange de la couleur sélectionnée par l'utilisateur,
- permettre à l'utilisateur de déclencher la mémorisation de la couleur du mélange et d'une zone d'application associée, notamment en vue d'une distribution ultérieure du même mélange, notamment sur la même zone.

Le produit programme d'ordinateur peut comporter des instructions de code permettant, lorsque exécutées dans un système informatique, d'amener le système informatique à :
- Recevoir une requête de l'utilisateur concernant un besoin de maquillage, notamment à l'aide d'une interface telle qu'un écran tactile,
- proposer sur la base au moins de données générées par le procédé d'apprentissage tel que défini ci-dessus, au moins une couleur et/ou une zone d'application,
- piloter un distributeur pour la production du mélange de la couleur proposée, notamment si celle-ci est validée par l'utilisateur.

La personne peut intégrer dans la cartographie le niveau de matité/brillance qu'elle désire pour chaque zone du visage, zone par zone ou ensemble de zones par ensemble de zones. Le système interprétera alors les mélanges qu'il doit réaliser pour assurer les couleurs, couvrance désirées et la matité/brillance voulu.

La cartographie peut être modifiée par la suite. Ainsi, il est possible de réaliser la cartographie sur le plan des couleurs /couvrance dans un premier temps, puis sophistiquer la cartographie en définissant par la suite les niveaux de matité/brillance.

### Aide à distance

Il est souhaitable de pouvoir aider l'utilisateur à se maquiller, notamment à choisir les bons coloris.

L'invention, selon l'un de ses aspects, indépendamment ou en combinaison avec ses autres aspects, notamment ce qui précède, a ainsi pour objet un procédé de maquillage comportant les étapes consistant à :
- permettre l'établissement d'une liaison vidéo, par exemple via internet, entre une caméra d'un premier site et un deuxième site,
- permettre au deuxième site de piloter directement ou indirectement un distributeur présent sur le premier site, ce distributeur permettant de faire varier la couleur d'un mélange distribué,
- permettre à une personne présente au premier site d'appliquer le mélange distribué et d'envoyer vers le deuxième site une image correspondante, pour recevoir en retour une information relative au résultat de maquillage.

Le deuxième site peut notamment comporter un écran de visualisation qui permet à un conseiller présent devant cet écran de voir le résultat du maquillage avec le produit distribué par le distributeur et de conseiller la personne qui s'est maquillée. Ce conseiller peut agir en retour sur le distributeur pour modifier la couleur du mélange et l'adapter au mieux au visage de la personne présente sur le premier site. Ainsi, cette personne gouverne le mélange délivré par le distributeur. La première personne peut se maquiller sous les yeux de la seconde. La seconde personne voit sur son écran le résultat de l'essai, et ainsi peut corriger le mélange qu'elle va commander à distance, jusqu'à obtenir le maquillage idéal.

Le cas échéant, l'acquisition vidéo peut être calibrée à l'aide d'une mire, ou avec le mélange distribué par le distributeur sur une surface de référence. Cela permet ensuite une visualisation plus fidèle du maquillage effectué sur le premier site.

De préférence, la liaison vidéo entre les deux sites est une liaison duplex.

Le premier site peut recevoir un tutoriel du deuxième site, le cas échéant.

Des identifiants des produits de base peuvent être communiqués au deuxième site ; cela peut permettre de connaître précisément la couleur de chacun des produits de base.

Le procédé peut comporter la mémorisation des paramètres de réglage du distributeur, une fois qu'un mélange donné est estimé satisfaisant. De préférence, cette mémorisation peut être commandée depuis le deuxième site. La mémorisation peut avoir lieu dans le système informatique présent sur le premier site et/ou sur un serveur externe.

Une variante peut consister à faire travailler une personne pour aider le maquillage de plusieurs personnes. Ce mode de mise en oeuvre permet le développement des « Make-Up Artists » et leur travail soit au sein d'un institut, soit via internet. Cela permet aussi le maquillage de personnes aux moyens limités, telles que les personnes malvoyantes, ou discernant mal les couleurs, ou âgées, ou manquant de confiance en elles.

### Pilotage par interface tactile

Il existe un besoin pour faciliter le pilotage du système de distribution et notamment le choix de la couleur du mélange distribué.

L'invention a encore pour objet un système de distribution comportant un distributeur et un système informatique de pilotage du distributeur, ce système informatique comportant un écran tactile sur lequel peut être affichée la couleur du mélange et un moyen de sélection déplaçable sur l'écran, pour faire varier la couleur du mélange distribué.

De préférence, l'écran affiche des couleurs extrêmes entre lesquelles la couleur du mélange peut être sélectionnée, en déplaçant le moyen de sélection entre ces couleurs extrêmes.

L'écran peut afficher une échelle de couleurs entre au moins deux couleurs, ou une surface, notamment de contour triangulaire, au sein de laquelle le moyen de sélection peut se déplacer. Cette surface peut localement faire apparaître la couleur du mélange en fonction par exemple de la distance à chacun des sommets, matérialisant chacun un produit de base pur.

Le système informatique peut effectuer une partie des calculs nécessaires pour déterminer les fractions de chacun des produits de base conduisant à un mélange de la couleur recherchée.

Le système informatique peut être un téléphone intelligent, un photophone, une tablette, un ordinateur individuel. En variante, il est intégré au boîtier du distributeur.

Le système informatique peut disposer d'une caméra. Cette dernière peut être utilisée notamment pour l'acquisition d'une image de l'utilisateur et/ou du mélange.

Le système informatique peut être agencé pour afficher une image d'un visage, afin de faciliter le repérage des zones sur lesquelles le mélange doit être appliqué.

### Couplage du système de distribution à un moyen de pulvérisation

Le système de distribution peut comporter ou être connecté à un moyen de pulvérisation du mélange, de préférence un aérographe.

L'invention vise, selon un autre de ses aspects, à perfectionner encore les systèmes de distribution comportant un système de pulvérisation, de préférence un aérographe, et a pour objet, selon l'un de ses aspects, indépendamment ou en combinaison avec les autres aspects, et notamment ce qui précède, un ensemble comportant :
- Un moyen de pulvérisation, de préférence un aérographe comportant une chambre de prélèvement soumise à un courant d'air d'entraînement,
- un distributeur comportant au moins deux compartiments contenant des produits de base différents, les produits étant délivrés au moyen de pulvérisation de préférence par des orifices de distribution distincts.

Le distributeur peut comporter trois cartouches contenant des produits de maquillage de couleurs différentes.

L'aérographe peut comporter un stylet définissant la chambre de prélèvement, le stylet se fixant sur le distributeur ou sur une interface de sortie fixée au distributeur, ou faisant partie intégrante de cette interface de sortie.

Le système de distribution peut comporter un circuit de contrôle du fonctionnement du distributeur, permettant de faire varier la proportion des produits de base délivrés dans la chambre de prélèvement, au cours du fonctionnement de l'aérographe. Les proportions peuvent être modifiées en fonction du déplacement de l'aérographe relativement à la surface sur laquelle le mélange est pulvérisé. Ce déplacement peut être mécanisé, le cas échéant.

Ce circuit de contrôle peut comporter ou être constitué par un système informatique tel que défini plus haut.

Le boîtier du distributeur peut servir de poignée lors de la manipulation d l'ensemble pour délivrer le mélange.

Le distributeur peut comporter une caméra et/ou un ou plusieurs capteurs tels que des accéléromètres afin de localiser automatiquement la zone sur laquelle le mélange est appliquée, et pouvoir régler automatiquement la couleur en fonction de la position, le cas échéant.

L'invention a encore pour objet un procédé de maquillage à l'aide d'un ensemble tel que défini ci-dessus, dans lequel un mélange est pulvérisé sur la peau à l'aide du moyen de pulvérisation, notamment de l'aérographe.

La composition du mélange peut être modifiée au cours du déplacement de l'aérographe relativement à la peau. Un dégradé peut être réalisé.

Cet aspect de l'invention repose sur la constatation que le distributeur peut être utilisé pour alimenter le système de pulvérisation, notamment l'aérographe, tout en permettant au système de distribution d'être suffisamment réactif pour autoriser un changement de la couleur du mélange distribué en cours de maquillage du visage, notamment lorsque la zone à maquiller change.

Il peut être avantageux que la distribution des produits se fasse de façon itérative, notamment avec des temps de distribution non déphasés entre les différents produits.

Cela peut rendre plus facile de faire varier la composition du mélange distribué au cours du temps.

Le mélange peut être réalisé directement dans l'aérographe, sans pratiquement aucun volume mort gênant, permettant ainsi de changer le mélange pulvérisé en temps réel.

La dépression créée dans la chambre de prélèvement est suffisante pour entraîner les produits de base, sans pour autant gêner le dosage.

La dépression qui règne dans la chambre de prélèvement est par exemple comprise entre 10 mbar et 200 mbar, mieux entre 50 et 150 mbar, encore mieux entre 75 et 125 mbar.

La viscosité des produits de base mesurée à 1 atm et à 25°C avec un viscosimètre rotatif CONTRAVES TV équipé d'un mobile MS-r3 ou MS-r4 à la fréquence de 60 Hz, après 10 minutes de rotation du mobile, est par exemple comprise entre 0,05 Pa.S et 50 Pa.S

La section des canaux d'arrivée des produits de base dans la chambre est par exemple comprise entre 1 et 3 mm², mieux entre 2 et 3 mm².

L'alimentation en produit s'effectue de préférence de façon continue.

Il est encore possible d'appliquer simultanément des produits de base non miscibles ou réactifs, comme un gel aqueux et un gel huileux, qui vont se déposer de façon pixellisée directement sur la peau, produisant une sorte de gel/gel in situ, des silicones réactives, ou des produits de coloration réagissant ensemble. Les ratios en produits de base peuvent être ajustés en fonction du résultat particulier souhaité. Par exemple, dans le cas des gels aqueux et gels huileux, on pourra faire varier le ratio correspondant au volume du premier produit de base sur le volume du deuxième produit de base entre 10/1 et 1/10, mieux entre 5/1 et 1/5.

### Système de localisation ou d'auto-localisation

Le système de distribution selon l'invention peut disposer d'un système de localisation ou d'auto-localisation.

On appelle système de localisation, un moyen de saisir, de la part de la personne, la zone qu'elle va traiter. Ceci peut être réalisé notamment par des systèmes laissant libre au moins une main. Ainsi, on peut utiliser une interface d'un système informatique telle qu'un écran tactile, un joystick, ou un système de reconnaissance vocale.

On appelle système d'auto-localisation, un moyen de saisir, sans que la personne n'intervienne, la zone à traiter. Ceci peut être réalisé par un ou plusieurs accéléromètres qui déduisent, par le fait des mouvements, les directions que la personne vise ou par une caméra et un système de reconnaissance d'image.

L'invention peut être mise en oeuvre selon ce cinqième aspect à l'aide des exemples illustrés sur les figures, précédemment décrites, et dont on ne reprendra pas ici la description.

### Exemple(cinaième aspect de l'invention)

On réalise plusieurs produits de base avec des ingrédients particulaires.

Les produits de base F1 et F2 sont riches en pigment (et de couleurs différentes). Le produit de base F3 est riche en charge.

| | | F1 | F2 | F3 |
|---|---|---|---|---|
| | | % massique | % massique | % massique |
| | Dimethicone copolyol vendu sous la référence KF 6017 par la société Shin Etsu | 2 | 2 | 2 |
| | Etsu Bis PEG/PPG-14/14 dimethicone + Cyclopentasiloxane vendu sous la référence ABIL EM 97 par la société Goldschmidt | 1 | 1 | 1 |
| | Cyclopentasiloxane | 17,65 | 17,65 | 17,65 |
| | Phenyl trimethicone vendu sous la référence DC556 par la société Dow Corning | 2 | 2 | 2 |
| | Ethyl hexyl methoxycinnamate | 3 | 3 | 3 |
| | Squalane | 1 | 1 | 1 |
| | Cyclopentasiloxane | 7 | 7 | 7 |
| | Oxyde de fer faune enrobé de stearoyl glutamate d'aluminium NAI-C33-9001-10 de la société Miyoshi Kasei | 2 | 1,1 | 1,33 |
| | Oxyde de fer rouge enrobé de stearoyl glutamate d'aluminium NAI-C33-8001-10 de la société Miyoshi Kasei | 0,2 | 0,6 | 0,13 |
| | Oxyde de fer noir enrobé de stearoyl glutamate d'aluminium NAI-C33-7001-10 de la société Miyoshi Kasei | 0,15 | 0,15 | 0,1 |
| | Dioxyde de titane (anatase) enrobé de stearoyl glutamate d'aluminium NAI-TAO-77891 de la société Miyoshi Kasei | 9,65 | 10,15 | 6,44 |
| | Talc enrobé de stearoyl glutamate d'aluminium NAI-TA-13R de la société Miyoshi Kasei | 0 | 0 | 1,5 |
| | Talc vendu sous la référence Micro Ace P3 par la société Nippon Talc | 0,5 | 0,5 | 0,5 |
| | Poudre de nylon 12 vendue sous la référence SP 500 par la société Toray Industries | 0,5 | 0,5 | 0,5 |
| | Eau déminéralisée | 36,15 | 36,15 | 38,65 |
| | 1,3-Butylene glycol | 3 | 3 | 3 |
| | Sulfate de magnésium | 0,7 | 0,7 | 0,7 |
| | Solution de maltose hydrogénée | 0,5 | 0,5 | 0,5 |
| | Alcool éthylique 96° dénaturé | 13 | 13 | 13 |
| | TOTAL | 100 | 100 | 100 |

### Mode opératoire

On pèse les constituants de la phase A dans le bêcher principal et l'on agite en maintenant à température ambiante sous Moritz (1000 tr/min). La phase B est préparée séparément en broyant trois fois à la tricylindre, le mélange de pigments et de cyclopentasiloxane. Cette phase B est ensuite ajoutée en maintenant l'agitation, ainsi que les charges (phase C).

La phase aqueuse D est aussi préparée séparément, en pesant dans un bêcher le butylène glycol, le sulfate de magnésium, la solution de maltose hydrogénée et l'eau à température ambiante. La phase aqueuse D est placée sous agitation à l'aide d'un barreau magnétique jusqu'à homogénéisation. C D E L'émulsion se fait à température ambiante : on verse la phase aqueuse D dans la phase grasse en augmentant progressivement la vitesse d'agitation (Moritz) jusqu'à 4000 tours/mn. On maintient l'agitation pendant 7 mn. Puis on ajoute enfin la phase E (alcool éthylique) pendant les 3 dernières minutes de 5 l'émulsification.

### Test

On utilise le système de distribution pour réaliser plusieurs mélanges (les proportions au sein du mélange sont volummiques) :

| | |
|---|---|
| M1 : | A 50%, B 20%, C 30% |
| M2 : | A 50%, B 0%, C 50% |
| M3 : | A 50%, B 40%, C 10% |

Les mélanges M1, M2, M3 sont appliqués sur différentes zones du visage. On obtient des couvrances différentes, des couleurs différentes (plus ou moins rosées) mais avec des niveaux de matités équivalents.

### F) Système de distribution pour personnaliser le niveau de photoprotection d'un fond de teint sans modifier son niveau de matité et brillance

Les femmes ont besoin de pouvoir se maquiller et se protéger du soleil. Le niveau d'UV pouvant varier d'un jour à l'autre et les besoins de photoprotection aussi, il faut pouvoir donner accès à toute une gamme de niveau de photoprotection, en même temps que donner accès à une gamme de couleur et de couvrance.

Dans le cas des peaux tachées, la plupart du temps, les zones de peau tachées ne présentent qu'une proportion faible de la surface du visage. Elles nécessitent une protection spécialement élevée car on sait que ces zones peuvent évoluer sous l'irradiation solaire, générer plus de taches en particulier.

D'autres zones comme les cicatrices nécessitent aussi une forte protection.

A l'inverse, le fond de la peau nécessite moins de protection étant entendu que les personnes à le faire bronzer pour des raisons esthétiques. Il faut donc pouvoir fournir des produits de maquillage contenant peu ou pas de filtre.

Il faudrait une solution pour donner accès à toute une gamme de niveaux de photoprotection et qui puissent être mis sur un même visage, sans qu'on puisse détecter les différences.

Pour résoudre ces problèmes, il n'existe que peu de solutions.

Une première approche consiste à acheter de nombreux produits de maquillage avec filtres et à tous les essayer. Cette approche est coûteuse et génère souvent des pertes dans la mesure où l'on ne retient généralement qu'un petit nombre des produits essayés.

Une seconde approche consiste à tester, en magasin, différents produits. Elle ne convient pas toujours, car il est très difficile de se figurer le résultat en quelques instants et dans un lieu dépourvu des repères habituels. En particulier, pour apprécier pleinement, l'effet d'un maquillage dans un magasin, il faudrait pouvoir l'éclairer de la même manière que dans les conditions futures d'utilisation, ce qui est rarement possible. En général, ce n'est qu'en testant un maquillage pendant la journée, qu'on se rend compte s'il convient ou non. De plus, si certains magasins disposent de conseillers et permettent d'effectuer des essais, ce n'est pas le cas d un grand nombre d'autres points de vente et des achats effectués sur internet.

Une autre approche a été testée sans toutefois se développer. Elle consisteà réaliser manuellement ses produits par mélange de plusieurs produits de couleur. Cela peut s'avérer relativement difficile à mettre en oeuvre, car il est peu aisé de reproduire exactement plusieurs fois le même mélange, et l'on a du mal à réaliser rapidement les mélanges dont on a besoin au moment du maquillage.

On connaît aussi des distributeurs permettant de délivrer une composition cosmétique de couleur variable.

Parmi les essais d'automatisation de la fabrication d'une composition cosmétique personnalisée qui ont pu être tentés, nombreux sont ceux dans lesquels les solutions proposées permettent de réaliser des mélanges dans des quantités d'environ 100 g ou parfois moins, mais pas dans les très petites proportions dont la personne qui se maquille a généralement besoin, c'est-à-dire de l'ordre du gramme ou beaucoup moins.

Pour illustrer cette problématique, on peut imaginer le cas d'une personne qui veut cacher deux imperfections de l'ordre du cm2 sur son visage. Pour la première zone, elle a besoin de trouver le mélange correspondant, puis d'en délivrer une très petite quantité, par exemple d'environ 10 mg. Pour la seconde, elle a besoin de changer le réglage du distributeur, puis de délivrer une toute petite quantité également.

Par conséquent, le choix des matières colorées conférant les meilleurs résultats reste difficile pour un grand nombre de personnes.

Il existe par conséquent un besoin pour faciliter la recherche d'un produit de maquillage filtrant répondant aux attentes d'un consommateur, et permettant à celui-ci de réaliser des mélanges dans des conditions fiables en quantités diverses.

L'invention vise ainsi, selon certains de ses aspects, à faciliter le maquillage photoprotecteur du visage et notamment la recherche des produits les plus adaptés aux différentes zones de celui-ci.

Il existe encore un besoin pour perfectionner les systèmes de distribution permettant de délivrer des produits de couleur variable, afin notamment de faciliter leur utilisation et améliorer la qualité du maquillage.

Il existe aussi un besoin d'appliquer sur le même visage plusieurs produits, avec des pouvoirs photoprotecteurs différents. En particulier, les gens peuvent avoir des zones du visage plus ou moins tachés et donc nécessitant des forces de photoprotection différentes. Il est donc intéressant de pouvoir disposer d'un moyen de produire des formulations photoprotectrices à différentes forces de photoprotection.

La réalisation, par mélanges, de produits masquants et photoprotecteur pose un problème important. Comme ils sont basés sur des ingrédients huileux (filtres organiques) on obtient alors des mélanges qui, selon le taux de photoprotection choisi (donc la quantité d'ingrédients huileux) donnent sur la peau des aspects de matité/brillance différents.

Il existe donc un besoin pour perfectionner les systèmes de distribution permettant de délivrer des produits de photoprotection variable tout en gardant le même niveau de matité/brillance, afin notamment de faciliter leur utilisation et améliorer la qualité du maquillage et photoprotection .

L'invention a ainsi pour objet, selon un sixième de ses aspects, un système de distribution d'un produit, comportant un distributeur recevant au moins deux cartouches contenant respectivement des premier et deuxième produits de base, le premier produit de base comportant, notamment sous forme d'émulsion inverse, un filtre solaire organique et une huile, le deuxième produit de base comportant une huile, le distributeur permettant de délivrer au moins ces deux produits de base dans des proportions réglables, la concentration en filtre solaire dans le premier produit de base étant supérieure à celle du deuxième produit de base et la concentration en huile dans le deuxième produit de base étant supérieure à celle du premier produit de base.

Selon ce sixième aspect, l'invention peut présenter une ou plusieurs des caractéristiques suivantes :
- la teneur massique du filtre solaire organique dans le premier produit de base est supérieure ou égale à 2% de la masse du premier produit de base, mieux à supérieur ou égale à 4% de la masse du premier produit de base, par exemple compris entre 4% et 30%,
- la teneur massique en huile(s) dans le deuxième produit de base est supérieure ou égale à 10% de la masse du deuxième produit de base, préférentiellement à supérieur à 15% de la masse du deuxième produit de base, de préférence supérieure ou égale à 20% , par exemple compris entre 20% et 60%, le deuxième produit de base est dépourvu de filtre solaire organique,
- le deuxième produit de base comporte un filtre solaire organique,
- l'un au moins des premier et deuxième produits de base contient un agent de coloration,
- l'agent de coloration est choisi parmi les pigments, notamment les oxydes de fer,
- chacun des premier et deuxième produits de base comporte un agent de coloration,
- l'un au moins des premier et deuxième produits de base contient une charge incolore,
- chacun des premier et deuxième produits de base comporte une charge incolore
- Le système de distribution comporte une troisième cartouche avec un troisième produit de base,
- les cartouches sont reçues de façon amovible dans le distributeur,
- chaque produit quitte la cartouche par un canal de sortie de la cartouche, le canal de sortie étant défini par un embout de distribution de la cartouche entraîné en rotation relative par rapport à un corps de la cartouche par un mécanisme d'entraînement du distributeur pour distribuer le produit de base contenu dans la cartouche.

L'invention selon ce sixième aspect a encore pour objet un procédé de réglage d'un système tel que défini ce-dessus, comportant le réglage du distributeur en fonction de la zone à traiter avec le produit et/ou l'intensité du rayonnement UV.

L'invention repose selon ce sixième aspect sur un système de distribution qui permet de générer des mélanges à partir de produits de base. Ces produits de base peuvent avoir des couleurs différentes, de telle sorte que l'on puisse faire varier la couleur du mélange. Les produits de base permettent de faire varier la photoprotection du mélange. Les produits de base peuvent aussi faire varier la couleur et la couvrance. Ainsi, la notion de couleur doit être comprise avec une acceptation large et englobe des mélanges dont la couleur varie après application du fait de leur couvrance plus ou moins élevée et de la couleur de la peau sous-jacente.

L'invention repose selon son sixième aspect sur un système de distribution, des compartiments contenant au moins deux formulations différentes lesquelles contiennent des ingrédients filtrants organiques choisis pour créer un effet de filtration. Ces formulations contiennent de préférence une émulsion inverse concentrée en filtre organique pour l'une et concentrée pour l'autre en huile. Ainsi, en réalisant les mélanges, la brillance et la matité ne changent pas. On peut donc faire varier le niveau de filtration tout en gardant un aspect égal.

On peut programmer le système de distribution pour qu'il délivre pour chaque endroit du visage le meilleur mélange filtrant (ni trop fort, ni trop faible). L'aspect global ne permettra pas de deviner que le visage a été traité avec différents niveaux de filtration Le système peut aussi être utilisé pour faire varier le niveau de filtration selon les envies et les moments. (par ex, plus filtrant à midi, plus léger pour l'après midi).

### Système de distribution

Le système de distribution peut être constitué par un seul appareil fonctionnant de manière autonome, de préférence manipulable d'une main, ou par un appareil qui fonctionne en interagissant avec d'autres pièces ou appareils. Il peut s'agir par exemple de différentes interfaces de sorties qui sont montées sur le distributeur en fonction du type de maquillage à réaliser, comme cela sera précisé plus loin. Il peut s'agir également d'un système informatique qui échange des informations avec le distributeur pour le piloter, ce système informatique comportant par exemple un terminal portable tel qu'un téléphone intelligent, un photophone, une tablette, un ordinateur personnel, ou un terminal dédié.

De préférence, le distributeur est agencé pour mettre sous pression un ou plusieurs compartiments contenant le ou les produits de base, par le biais de dispositifs de dosage volumétrique, de préférence un moteur entraînant en déplacement un piston dans le compartiment correspondant.

Le distributeur peut être formé d'un boîtier et d'au moins deux ou trois compartiments, et de préférence d'autant de moteurs. Par exemple, la rotation des moteurs entraîne des vis sans fin, lesquelles poussent les pistons de chaque compartiment. L'avance des pistons est par exemple contrôlée par le nombre d'impulsions de commande envoyées aux moteurs et/ou par la durée de fonctionnement de ces derniers. Les moteurs peuvent être alimentés séquentiellement, ou de préférence simultanément.

Par exemple, les moteurs sont alimentés durant un cycle élémentaire de fonctionnement pendant une courte durée les uns après les autres, ou les uns en même temps que les autres, de façon à distribuer des microdoses correspondantes.

Les cycles élémentaires sont répétés, avec éventuellement un temps d'arrêt entre eux, qui laisse le temps aux produits de base de s'écouler hors des compartiments.

Les compartiments peuvent être définis par des cartouches qui se retirent lorsqu'elles sont vides. En variante, les compartiments sont présents à demeure et remplis à nouveau une fois vide.

Chaque cartouche peut être fermée par un bouchon démontable pour permettre le nettoyage de la cartouche.

De préférence, le boîtier du distributeur est de forme allongée selon un axe longitudinal, ce qui peut faciliter sa manipulation, et les cartouches sont disposées autour de cet axe, dans le boîtier.

De préférence, les cartouches sont mises en place par l'arrière, et le mélange est délivré par l'avant. La mise en place des cartouches peut s'effectuer individuellement, ou en variante les cartouches constituent un ensemble monobloc lors de leur mise en place.

Les cartouches peuvent comporter chacune un mécanisme de dosage volumétrique, comportant un piston mu par un mécanisme d'entraînement du distributeur, dans une direction s'accompagnant d'une diminution du volume intérieur contenant le produit de base et d'une expulsion du produit. Il peut être intéressant que les cartouches aient une région de leur paroi au moins qui soit transparente, afin de permettre de voir la couleur du produit contenu à l'intérieur.

Le mécanisme d'entraînement peut comporter un système de motorisation formé de moteurs couplés à des réducteurs, de forme allongée parallèlement à l'axe longitudinal du distributeur, et disposés entre les cartouches. Ce positionnement des moteurs et cartouches rend le distributeur particulièrement compact.

Le produit de base peut sortir de façon étanche de la cartouche correspondante puis circuler dans un canal prévu à cet effet dans le boîtier du distributeur, avant de sortir de celui-ci.

Les cartouches se terminent avantageusement par un embout réalisé de telle sorte que, une fois la cartouche insérée dans le boîtier du distributeur, l'extrémité de l'embout débouche au ras du boîtier. En variante, l'embout est suffisamment long pour dépasser du boîtier et connecter ainsi différentes interfaces de sortie susceptibles d'être rapportées sur le boîtier du distributeur.

Grâce au mécanisme d'entraînement comportant des moteurs pour faire avancer des pistons, il est possible de délivrer de façon précise des mélanges en de très petites quantités. Ainsi, le mécanisme d'entraînement peut délivrer les produits de base avec à un débit minimal inférieur ou égal à 50 µL/s, mieux inférieur ou égal à 20 µL/s, encore mieux inférieur ou égal 10 µL/s. De préférence, le mécanisme d'entraînement délivre des débits compris entre 20 et 100 µL/s, mieux compris entre 40 µL/s et 60 µL/s. Il est donc possible de réaliser facilement un mélange d'environ 10 mg. Un tel système de distribution est alors idéal pour réaliser de petites touches de maquillage, pour couvrir par exemple une zone de 1 cm², mieux une zone de 0, 5 cm². Il est aussi possible de réaliser des plus grandes quantités de mélange comme celles nécessaires au maquillage d'une joue, ou d'un visage. Ces quantités restent néanmoins relativement faibles, par exemple une quantité comprise entre 100 et 500 mg, mieux comprise entre 150 et 250 mg.

L'invention a ainsi pour objet, selon l'un de ses aspects, un système de distribution comportant un distributeur présentant un boîtier, et au moins une cartouche reçue dans le boîtier du distributeur, cette cartouche comportant un corps et un piston mobile dans le corps, le boîtier comportant un mécanisme d'entraînement motorisé pour déplacer le piston de la cartouche.

De préférence, la cartouche comporte un embout de distribution par lequel s'effectue la sortie du produit, et cet embout de distribution est entraîné en rotation par le mécanisme d'entraînement pour déplacer le piston. L'embout peut comporter au moins un relief d'anti-rotation, mieux deux ergots d'anti-rotation diamétralement opposés.

L'embout peut porter un joint d'étanchéité, notamment un joint torique. Ainsi, au changement de cartouche, le joint est également changé, ce qui permet de s'affranchir de l'usure du joint.

Le distributeur peut comporter une carte électronique de pilotage du mécanisme d'entraînement motorisé, cette carte électronique étant traversée par le ou les embouts. Cela peut permettre de réaliser une carte s'étendant sensiblement sur toute la section du distributeur, donc de regrouper sur une seule carte tous les composants électroniques du distributeur, et ainsi de gagner en compacité et fiabilité. La carte peut s'étendre sensiblement perpendiculairement à l'axe longitudinal du boîtier. La carte peut porter un interrupteur de commande du fonctionnement du distributeur.

Le système de distribution peut être agencé pour fonctionner selon au moins deux modes de distribution.

Dans un premier mode, dit « continu », le mélange est distribué tant qu'une pression est exercée sur l'interrupteur de commande.

Dans un deuxième mode, dit « dose », une quantité prédéfinie du mélange est distribuée à chaque pression sur l'interrupteur.

Le ou les embouts peuvent déboucher à une extrémité du boîtier. Cela peut permettre de réduire le volume mort, comme cela sera détaillé dans la suite.

Le ou les embouts peuvent présenter à leur extrémité un système d'obturation pour éviter le séchage des produits dans le conduit, par exemple une membrane cicatrisante

La cartouche peut comporter une vis creuse sur laquelle le piston est vissé, le piston pouvant se déplacer axialement sur la vis lorsque celle-ci tourne ; le piston est empêché de tourner dans le corps de la cartouche. Par exemple, la friction du piston sur le corps de la cartouche peut suffire à l'empêcher de tourner quand la vis tourne.

Préférentiellement, la rotation est rendue impossible avec un corps de cartouche de section non circulaire et un piston non déformable.

Le couple des moteurs peut être déterminé électroniquement en fonction du courant absorbé, et servir par exemple à détecter une fin de course du piston. Une information concernant le couple peut être transmise à distance à un système informatique présentant une interface homme machine, pour permettre de surveiller le bon fonctionnement du distributeur.

Afin d'ajuster la teinte, le système de distribution selon l'invention doit permettre à l'utilisateur de faire varier le volume délivré provenant de chaque compartiment.

De façon préférée, le distributeur est piloté par un système informatique, qui est intégré au distributeur, ou externe à celui-ci, le distributeur étant alors apte à échanger avec le système informatique des informations par protocole sans fil ou câblé.

Le distributeur peut ainsi être piloté pour permettre l'ajustement de la teinte par distribution simultanée ou séquentielle, et contrôlée, de plusieurs produits de base de couleurs différentes.

La distribution des produits de base peut être continue ; si dans ce cas, les volumes de chacun des produits de base sont distribués d'une seule traite, simultanément ou successivement.

Dans le cas d'une distribution simultanée, il est utile de pouvoir ajuster les débits respectifs des différents produits de base pour que le mélange distribué corresponde à celui souhaité à chaque instant. Un tel mode de distribution peut convenir en particulier lors de la distribution du mélange par pulvérisation, à l'aide d'un aérographe. Pour ajuster les débits il est possible par exemple de jouer sur la vitesse de déplacement des pistons, par exemple, dans le cas d'un entraînement des pistons par une vis sans fin en faisant varier la vitesse de rotation des moteurs qui entrainent la vis. Les produits peuvent aussi être distribués de façon impulsionnelle avec un temps de distribution et un temps d'arrêt à chaque cycle. En jouant sur le rapport cyclique, il est possible d'agir sur le débit.

Tous les produits peuvent être délivrés simultanément lors du temps de distribution ; en variante, les cycles des différents produits sont déphasés, de sorte qu'un produit est distribué pendant un temps d'arrêt des autres produits.

Dans un mode particulier de l'invention, le mélange est délivré dans une cavité d'un contenant, pouvant fermer de façon hermétique ou non, par exemple en forme de coupelle, dans lequel on peut faire glisser un applicateur, en particulier un stylet ou une brosse. Un tel système de distribution est spécialement adapté aux liners, gloss et autres formules appliquées sans contact direct avec les mains. Ce contenant peut être amovible.

Par exemple, il sert de distributeur de rouge à lèvres et dispose d'un système de distribution, à vis par exemple. Lorsqu'il n'est pas amovible, le contenant peut être réalisé avec le corps du distributeur. Lorsqu'il est amovible, il peut constituer une interface de sortie parmi d'autres pouvant être montées sur le distributeur.

Les compartiments, et en particulier les cartouches, peuvent contenir tout ou partie du mécanisme d'entraînement, et par exemple le système de motorisation, ou mieux, une partie du système de motorisation, et ce, afin de réduire le nombre de pièces en mouvement dans le corps du distributeur à l'extérieur des cartouches. Par exemple, les cartouches comportent le rotor du moteur. Une fois les cartouches installées dans le corps du distributeur, les rotors sont mis en interaction avec les stators.

Le système de distribution est avantageusement agencé pour permettre de lancer des séquences préprogrammées dans lesquelles le mélange délivré par celui-ci est modifié de façon continue ou discontinue. Un mode « dégradé » permet par exemple passer progressivement d'un mélange A à un mélange B. Dans le cas d'une application par pulvérisation, notamment à l'aide d'un aérographe, cela permet de réaliser simplement les dégradés. Un mode « Alternatif » permet par exemple de passer rapidement d'un mélange

A à un mélange B plusieurs fois de suite. Dans le cas d'une application par pulvérisation, on peut ainsi réaliser un dépôt multicouche, avec des formulations différentes pour deux couches adjacentes superposées. Un autre mode permet par exemple de proposer plusieurs mélanges successifs préprogrammés, le système informatique indiquant à chaque fois l'usage qu'il faut en faire à l'utilisateur, par exemple par affichage sur un écran.

Dans le cas d'une application manuelle, les mélanges sont distribués par exemple dans une coupelle. La personne se maquille à l'endroit recommandé avec un mélange correspondant prélevé dans la coupelle, puis nettoie éventuellement la coupelle et commande la délivrance d'un nouveau mélange ; l'opération est répétée autant de fois que nécessaire pour se maquiller complètement.

L'homogénéisation des mélanges distribués peut se faire de différentes façons selon les cas d'utilisation. Dans le cas d'une application manuelle, elle peut se faire directement sur la zone d'application au moment de l'application ou dans la coupelle avant prélèvement ; dans le cas d'une application à l'aérographe, c'est la tuyère de l'aérographe qui sert de chambre de mélange ; dans le cas où le mélange est distribué dans un contenant en vue d'une utilisation ultérieure, l'homogénéisation peut être faite à la main ou en faisant passer les produits distribués dans une chambre de mélange, située entre le distributeur et le contenant ou directement intégrée au contenant, comme détaillé par la suite.

Le produit peut être délivré par le système de distribution et utilisé extemporanément. En variante, le produit délivré par le système de distribution est conditionné et utilisé ultérieurement, par exemple à plusieurs reprises, avec par exemple un jour d'intervalle au moins entre deux utilisations.

### Ingrédients filtrants

Les ingrédients filtrants sont choisis parmi les filtres UV organique lipophile liquide. Par « filtres UV organique lipophile liquide », on entend toute molécule chimique organique capable d'absorber au moins les rayonnements UV dans la gamme des longueurs d'onde comprises entre 280 et 400nm ; ladite molécule étant sous forme liquide à température ambiante (20 - 25°C) et à pression atmosphérique (760 mm de Hg) et susceptible d'être miscible dans une phase huileuse.

Les filtres UV liquides organiques utilisables selon l'invention, peuvent être choisis parmi
- les composés β,β-diphénylacrylate lipophiles liquides
- les composés salicylates lipophiles liquides
- les composés cinnamates lipophiles liquides
- et leurs mélanges.

### i) Composés β,β-diphénylacrylate

Parmi les filtres UVB lipophiles liquides organiques utilisables selon l'invention, on peut citer les composés β,β-diphénylacrylate ou α-cyano- β,β-diphénylacrylate d'alkyle lipophiles liquides de formule (I) suivante : 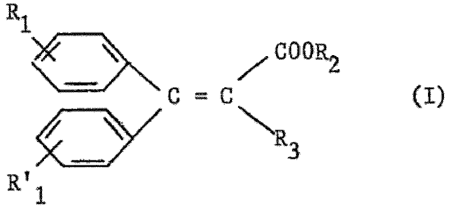 où R₁ à R₃ peuvent prendre les significations suivantes :
- R₁ et R'₁, identiques ou différents, représentent un atome d'hydrogène, un radical alcoxy en Ci-Cs à chaîne droite ou ramifiée ou un radical alkyle en C₁-C₄ à chaîne droite ou ramifiée,
- R₁ et R'₁ étant en position para méta;
- R₂ représente un radical alkyle en C₁-C₁₂ à chaîne droite ou ramifiée,
- R₃ représente un atome d'hydrogène ou le radical CN.

Parmi les radicaux alcoxy en Ci-Cs à chaîne droite ou ramifiée, on peut citer par exemple les radicaux méthoxy, éthoxy, n-propoxy, isopropoxy, n-butoxy, isobutoxy, tert-butoxy, n-amyloxy, isoamyloxy, néopentyloxy, n-hexyloxy, n-heptyloxy, n-octyloxy et 2-éthylhexyloxy.

Parmi les radicaux alkyle en C₁-C₄ à chaîne droite ou ramifiée, on peut citer plus particulièrement les radicaux méthyle, éthyle, n-propyle, isopropyle, n-butyle, isobutyle, tert.-butyle. Pour les radicaux alkyle en C₁-C₁₂, on peut citer à titre d'exemple, en plus de ceux mentionnés ci-dessus, les radicaux n-amyle, isoamyle, néopentyle, n-hexyle, n-heptyle, n-octyle, 2-éthylhexyle, décyle et lauryle.

Parmi les composés de formule générale (I), on préfère plus particulièrement les composés suivants :
le α-cyano-β,β-diphénylacrylate de 2-éthyl hexyl ou Octocrylène, vendu notamment sous le nom commercial « UVINUL N539^{®}» par BASF.
- le α-cyano-β,β-diphénylacrylate d'éthyle comme l'Etocrylene vendu notamment sous le nom commercial « UVINUL N35^{®} » par BASF,
- le β,β-diphénylacrylate de 2-éthyl hexyle,
- le β,β-di(4'-methoxyphenyl)acrylate d'éthyle.

Parmi les composés de formule générale (I), on préfère encore plus particulièrement le composé 2-cyano-3,3-diphénylacrylate de 2-éthyl hexyle ou Octocrylène

### ii) Composés salicylates

Parmi les composés salicylates lipophiles liquides utilisables selon l'invention, on peut citer :
- l'Homosalate vendu sous le nom « Eusolex HMS^{®}» par Rona/EM Industries,
- l'Ethylhexyl Salicylate vendu sous le nom « NEO HELIOPAN OS^{®}» par Symrise.

### iii) Composés cinnamates

Parmi les composés cinnammates lipophiles liquides utilisables selon l'invention, on peut citer :
- l'Ethylhexyl Methoxycinnamate vendu notamment sous le nom commercial PARSOL MCX^{®} par DSM Nutritial Products,
- l'Isopropyl Methoxycinnamate,
- l'Isoamyl Methoxycinnamate vendu sous le nom commercial NEO HELIOPAN E 1000 par Symrise^{®},

Parmi les filtres lipophiles liquides selon l'invention, on utilisera plus particulièrement le composé Ethylhexyl Methoxycinnamate.

Parmi les huiles fluorées et/ou siliconées non volatiles, on peut citer :
- les huiles fluorées éventuellement partiellement hydrocarbonées et/ou siliconées comme les huiles fluorosiliconées, les polyéthers fluorés, les silicones fluorées telles que décrit dans le document EP-A-847752;
- les huiles siliconées comme les polydiméthylsiloxanes (PDMS) non volatiles ; les silicones phénylées comme les phényl triméthicones, les phényl diméthicones, les phényl triméthylsiloxy diphényl siloxanes, les diphényl diméthicones, les diphényl méthyldiphényl trisiloxanes, les 2-phényl éthyl triméthyl-siloxysilicates.

Selon un mode particulier de l'invention, la phase huileuse comprend au moins une huile siliconée telle que définie précédemment et au moins un filtre UV organique lipophile liquide tel que défini ci-dessus.

Selon un mode particulier de l'invention, la phase huileuse comprend au moins l'Ethylhexyl Methoxycinnamate.

### Huiles

Par « huiles », on entend une huile restant sur la peau à température ambiante et pression atmosphérique au moins plusieurs heures et ayant notamment une pression de vapeur inférieure à 0,13 Pa (0,01 mm de Hg).

Ces huiles non volatiles peuvent être des huiles hydrocarbonées notamment d'origine animale ou végétale, des huiles siliconées, ou leurs mélanges. On entend par "huile hydrocarbonée", une huile contenant principalement des atomes d'hydrogène et de carbone et éventuellement des atomes d'oxygène, d'azote, de soufre et/ou de phosphore.

Comme huile hydrocarbonée non volatile, on peut notamment citer :
- les huiles hydrocarbonées d'origine animale,
- les huiles hydrocarbonées d'origine végétale telles que les triglycérides constitués d'esters d'acides gras et de glycérol dont les acides gras peuvent avoir des longueurs de chaînes variées de C4 à C24, ces dernières pouvant être linéaires ou ramifiées, saturées ou insaturées; ces huiles sont notamment des triglycérides d'acide heptanoïque ou d'acide octanoïque, ou bien encore les huiles de germe de blé, de tournesol, de pépins de raisin, de sésame, de maïs, d'abricot, de ricin, de karité, d'avocat, d'olive, de soja, d'amande douce, de palme, de colza, de coton, de noisette, de macadamia, de jojoba, de luzerne, de pavot, de potimarron, de sésame, de courge, de colza, de cassis, d'onagre, de millet, d'orge, de quinoa, de seigle, de carthame, de bancoulier, de passiflore, de rosier muscat ; le beurre de karité ; ou encore les triglycérides des acides caprylique/caprique,
- les éthers de synthèse ayant de 10 à 40 atomes de carbone ;
- les hydrocarbures linéaires ou ramifiés, d'origine minérale ou synthétique tels que la vaseline, les polydécènes, le polyisobutène hydrogéné, le squalane, les huiles de paraffine, et leurs mélanges,
- les esters de synthèse comme les huiles de formule R1COOR2 dans laquelle R1 représente le reste d'un acide gras linéaire ou ramifié comportant de 1 à 40 atomes de carbone et R2 représente une chaîne hydrocarbonée notamment ramifiée contenant de 1 à 40 atomes de carbone à condition que R1 + R2 soit ≥ 10, comme par exemple l'huile de Purcellin (octanoate de cétostéaryle), le myristate d'isopropyle, le palmitate d'isopropyle, les benzoates d'alcools en C12 à C15, le laurate d'hexyle, l'adipate de diisopropyle, l'isononanoate d'isononyle, le néopentanoate d'isodecyl, le palmitate de 2-éthyl-hexyle, l'isostéarate d'isostéaryle, le laurate de 2-hexyl-décyle, le palmitate de 2-octyl-décyle, le myristate de 2-octyl-dodécyle, des heptanoates, octanoates, décanoates ou ricinoléates d'alcools ou de polyalcools comme le dioctanoate de propylène glycol ; les esters hydroxylés comme le lactate d'isostéaryle, le malate de di-isostéaryle, le lactate de 2-octyl-dodécyle ; les esters de polyols et les esters du pentaérythritol,
- les alcools gras liquides à température ambiante à chaîne carbonée ramifiée et/ou insaturée ayant de 12 à 26 atomes de carbone comme l'octyl dodécanol, l'alcool isostéarylique, l'alcool oléique, le 2-hexyldécanol, le 2-butyloctanol, et le 2-undécylpentadécanol,
- les acides gras supérieurs tels que l'acide oléique, l'acide linoléique, l'acide linolénique et leurs mélanges.

Les huiles de silicone non volatiles utilisables dans la composition selon l'invention peuvent être les polydiméthylsiloxanes (PDMS) non volatiles, les polydiméthylsiloxanes comportant des groupements alkyle ou alcoxy pendants et/ou en bouts de chaîne siliconée, groupements ayant chacun de 2 à 24 atomes de carbone, les silicones phénylées comme les phényl triméthicones, les phényl diméthicones, les phényl triméthylsiloxy diphénylsiloxanes, les diphényl diméthicones, les diphényl méthyldiphényl trisiloxanes, et leurs mélanges.

De manière préférentielle, la phase huileuse comprend au moins une huile siliconée et encore plus préférentiellement choisie parmi
- les huiles volatiles siliconées cycliques ayant une viscosité à température ambiante inférieure à 8 cSt et ayant notamment de 4 à 7 atomes de silicium, ces silicones comportant éventuellement des groupes alkyle ou alcoxy ayant de 1 à 10 atomes de carbone, en particulier choisie parmi l'hexamethylcyclotrisiloxane, l'octamethylcylotetrasiloxane, le decamethylcyclopentasiloxane et le dodecamethylcyclohexasiloxane (cyclohexasiloxane), et leurs mélanges ;
- les polydiméthylsiloxanes (PDMS) volatiles ou non volatiles (Nom INCI : DIMETHICONE) ;
- les silicones phénylées ;
- les polydiméthylsiloxanes comprenant des groupes aliphatiques, en particulier alkyle, ou alcoxy, qui sont pendants et/ou à la fin de la chaîne silicone ; ces groupes comprenant chacun de 6 à 24 atomes de carbone, et plus particulièrement le caprylyl methicone tel que le produit commercial DOW CORNING FZ-3196^{®} par la société DOW CORNING ;
- leurs mélanges.

### Emulsions inverses / Composotions anhydres

Selon l'invention, lorsqu'il est précisé qu'une composition est sous la forme d'une émulsion inverse, il est entendu qu'elle peut également être alternativement sous une autre forme selon la composition considérée.

Un produit de base peut se présenter sous la forme d'une émulsion, par exemple une émulsion H/E, E/H, H/E/H ou E/H/E, et de préférence une émulsion inverse E/H, ou sous la forme d'une composition anhydre, comprenant notamment des composés carbonés et/ou siliconés, tels que des huiles hydrocarbonées et/ou siliconées.

Les émulsions selon le sixième aspect de l'invention sont de préférence des émulsions eau-dans-huile (E/H) encore appelées émulsions inverses, à savoir constituées d'une phase huileuse continue dans laquelle est dispersée la phase aqueuse sous forme de gouttelettes de manière à observer un mélange macroscopiquement homogène à l'oeil nu.

### Utilisation pour le maquillage d'une ou plusieurs zones précises de peau

Le système de distribution permet de se maquiller jour après jour, en ne traitant que les zones à masquer. Pour ce faire, on délivre de petites doses de maquillage plus ou moins filtrant, qu'on applique précisément et séquentiellement sur les zones correspondantes. Chaque petite dose est réalisée selon le mélange qui convient à la zone.

Dans un mode préféré de mise en oeuvre de l'invention, le système de distribution attend qu'on le renseigne sur une zone à traiter puis délivre le mélange correspondant. Il peut faire appel à une table de correspondance préprogrammée pour cela, cette table résultant par exemple d'un procédé d'apprentissage tel que défini plus loin. En variante, le système de distribution renseigne la personne, tout en délivrant un mélange, sur la zone sur laquelle celle-ci doit appliquer le mélange. Ainsi, le système de distribution peut suivre un programme d'application où il délivre dans un ordre donné, les différents mélanges à appliquer.

Dans un mode particulier de mise en oeuvre de l'invention, le système de distribution est renseigné sur les quantités à délivrer. Pour cela, il mémorise la correspondance entre la filtration nécessaire la zone du visage et la quantité nécessaire, ce qui permet de réduire les coûts et les pertes de produit, et de ne couvrir que faiblement la peau, évitant ainsi des effets d'occlusion. Ce faisant, il est possible d'utiliser des produits très filtrants et trop filtrants pour être appliqués sur l'ensemble du visage. Ainsi, il est possible d'obtenir des maquillages d'aspects naturels, voire indétectables mais avec un niveau de filtration spécialement efficace.

Le système permet donc de réaliser des applications localisées d'une variété de pouvoir filtrant et ce en permettant d'obtenir le même niveau de matité/brillance.

Le système de distribution peut aussi permettre, en facilitant la distribution de petites quantités et leur usage rapide, de réduire la durée de conservation des produits, permettant ainsi de réduire les risques d'évolution et/ou les quantités de conservateurs à employer.

Le système de distribution est adapté à traiter les zones qu'on veut protéger, sans avoir à filtrer les UV sur l'ensemble du visage.

Il peut aussi être utilisé pour masquer et/ou protéger une ou des taches, avec un seul niveau de matité/brillance.

Dans une variante spécialement avantageuse, le système peut être utilisé pour application sur le fond du visage une formule peu filtrante puis une ou deux applications sur des zones précises du visage des formules plus filtrantes. On peut aussi commencer par applications sur des zones précises du visage des formules filtrantes puis appliquer sur le fond du visage une application d'une formule peu filtrantes.

Dans le second cas, on prend le temps, avant d'appliquer la seconde couche, d'attendre que le film produit par l'application sur les zones précises devienne suffisamment cohésif pour que la seconde couche n'emporte pas la première couche.

Ou bien, on applique la seconde couche sans frotter (spray par exemple, ou éponge).

Lorsque l'utilisateur recherche la filtration à appliquer sur une zone du visage, il est intéressant de mémoriser la filtration qui va bien pour chaque zone, et le système de distribution est ainsi avantageusement agencé pour mémoriser cette filtration et la zone correspondante. Ainsi, en utilisant les informations mémorisées, à chaque utilisation, on peut obtenir la délivrance du même mélange pour chaque zone, ou, si l'on traite plusieurs zones, la même série de mélanges pour une même série de zones.

Le système de distribution peut aussi être agencé pour permettre de traiter une zone en faisant varier les filtrations application après application. Par exemple, les jours de la semaine, la personne réalise un fond de teint couleur claire avec une faible filtration, et le weekend, un fond de teint de couleur hâlée avec une forte filtration.

Le système de distribution peut être agencé pour permettre à l'utilisateur de changer de couleur et/ou filtration selon ses goûts du jour, du moment, de ses tenues, du climat. Ainsi, on prévoit avantageusement un système d'aide à la décision pour guider l'utilisateur dans ses choix de couleur et filtration

On peut prévoir aussi un système d'aide pour équilibrer les couleurs sur un même visage et participer à la réalisation d'un maquillage global réussi.

Il peut être souhaitable que plusieurs personnes d'un même groupe, par exemple une famille, puissent utiliser le système de distribution, réduisant ainsi les coûts et minimisant la place occupée. Cette solution est spécialement adaptée aux voyages ou aux hôtels, campings, avions, campings cars, boutiques, écoles... Pour ce faire, on peut prévoir que le système de distribution puisse être renseigné sur la personne qui l'utilise, afin d'accéder à des données personnelles préalablement mémorisées.

### Utilisation en continu pour une filtration en dégradé

Dans cette application, le système de distribution, pendant qu'il délivre le produit, change la formulation du mélange. De plus, on déplace la sortie des produits de base ou du mélange relativement à un contenant ou un support définissant une surface d'application. Dans un mode particulier de mise en oeuvre de l'invention, le système de distribution est agencé pour calculer l'évolution du mélange en fonction de la filtration F1, d'une zone à traiter et la filtration F2, d'une autre zone à traiter.

Ces différents effets pourront être réalisés, avec un effet de matité/brillance constant.

Le système de distribution peut comporter une tête de sortie, en particulier dans le cas d'un aérographe, qui est mobile et pilotée. Cette option permet alors, sans bouger le reste du système de distribution, de réaliser des dégradés. Par exemple, on localise le système de distribution près de la joue, puis on déclenche un système de commande qui va piloter automatiquement la variation de la formulation du mélange et le mouvement de la tête de sortie, de façon à par exemple avoir le centre de la joue plus rouge que sa périphérie, avec un dégradé entre les deux.

Le système de distribution peut encore être utilisé pour réaliser des produits sur mesure, que l'on conserve pour réaliser plusieurs applications.

Il est aussi possible de réaliser des produits solides ou semi-solides.

### Fabrication de compacts « à façon » ou autres produits solides ou semi-solides

Le système de distribution peut être agencé pour permettre de choisir un mélange et de le délivrer dans un contenant tel qu'une coupelle. Le mélange comporte de préférence des composés qui sont tels que le mélange puisse prendre en masse.

Plus préférentiellement, on utilise des composés qui rendent la prise en masse spécialement rapide. Ces composés sont soit déposés dans le contenant avant ou après son remplissage avec les autres constituants, soit sont prévus dans les compartiments du distributeur avec les autres constituants des produits de base, soit sont compris dans le distributeur dans un compartiment spécialement prévu pour les contenir.

On peut ainsi distribuer des compositions spécifiques qui peuvent par réaction chimique, biochimique ou physicochimique, durcir de façon accélérée après évacuation.

Ces compositions sont spécialement adaptées à la réalisation des compacts, c'est-à-dire :
- Prennent en masse,
- donnent un matériau qui peut se déliter en cas de frottement, et sont préférentiellement colorées.

De préférence, ces compositions sont très riches en particules solides, avec par exemple plus de 10% en masse de particules solides par rapport au poids total de la composition, mieux plus de 20% en masse de particules solides par rapport à la masse totale de la composition, encore mieux plus de 30% en masse de particules solides par rapport à la masse totale de la composition, préférentiellement entre 10 et 40% en masse de particules solides par rapport à la masse totale de la composition.

Ces compositions peuvent contenir des particules absorbantes, ou des composés réactifs, tels que ceux qui réagissent en contact avec l'air, par exemple du cyanoacrylate ou des alphasilanes, ou qui réagissent à la lumière, notamment UV.

Le contenant dans lequel le mélange est distribué peut comporter un composé A et les compositions distribuées un composé B, A et B étant choisis pour réagir entre eux et solidifier le mélange.

Préférentiellement, le mélange est homogénéisé avant la prise en masse.

Les produits, de filtrations différentes, présenteront des effets de matité/brillance équivalents

### Réalisation de palettes de filtration

Le système de distribution peut comporter un support, ayant plusieurs régions, et être agencé pour générer automatiquement plusieurs mélanges déposés dans lesdites régions, par exemple une série de couleurs adaptées à différentes parties du visage.

Le support peut définir plusieurs cavités pour recevoir les mélanges ou porter plusieurs contenants, par exemple sous forme de coupelles, éventuellement séparables du support.

Dans un cas particulier, le support prend la forme d'un visage avec des régions pour recevoir les mélanges pour des zones d'applications ciblées.

Le support être mobile, notamment rotatif, par rapport au corps du distributeur, et par exemple entraîné en déplacement par le distributeur, pour remplir successivement différents logements ou contenants.

Les produits, sur la palette pourront être de couvrance différentes, tout en présentant des effets de matité/brillance équivalents

### Distributeur à coupelle

Il existe un intérêt pour bénéficier d'un système de distribution capable de délivrer un mélange pouvant être facilement prélevé par l'utilisateur. Par ailleurs, dans le cas où les produits de base délivrés par le système de distribution ne sont pas déjà mélangés, il existe un besoin pour permettre à l'utilisateur d'effectuer aisément le mélange.

L'invention a encore pour objet un système de distribution d'au moins un produit de maquillage, comportant une coupelle, et un distributeur pour remplir la coupelle avec au moins un produit, la coupelle étant solidaire du distributeur au moins pendant son remplissage.

La coupelle est parfois encore appelée « creuset » et ce terme doit être compris avec une acceptation large.

Par « coupelle solidaire du distributeur » il faut comprendre que la coupelle est retenue, notamment immobilisée, au moins temporairement sur le distributeur, étant par exemple fixée sur celui-ci par des vis, par aimantation, par encliquetage, par verrouillage baïonnette, par serrage, ou réalisée avec une partie du corps du distributeur par moulage de matière. La coupelle lorsqu'elle est solidaire du distributeur permet de manipuler celui-ci d'une main, la coupelle restant en place sur le distributeur au cours des mouvements de ce dernier.

Le distributeur peut être proposé à l'utilisateur avec la coupelle déjà en place.

En variante, celle-ci est installée par l'utilisateur à la première utilisation du système de distribution.

De préférence la coupelle est moins profonde que large, ce qui facilite l'accès à celle-ci, et permet de prélever le produit, notamment le mélange avec un applicateur ou le doigt.

De préférence, la coupelle est séparable du distributeur, et constitue une interface de sortie qui peut être choisie parmi un ensemble d'interfaces de sortie pouvant être montées sur le distributeur, au choix de l'utilisateur en fonction du maquillage à réaliser, comme détaillé plus loin.

De préférence, le système de distribution comporte plusieurs orifices de remplissage en des produits de base différents, débouchant dans la coupelle. Ainsi, le mélange de ces produits peut avoir lieu dans la coupelle.

La coupelle a de préférence un fond de forme concave vers l'extérieur, ce qui peut faciliter son nettoyage par l'utilisateur entre deux utilisations.

De plus, cela peut faciliter le prélèvement du produit par l'utilisateur, et le mélange des produits de base.

De préférence, le distributeur permet de délivrer au moins deux produits de base dans la coupelle, dans des proportions réglables, et mieux au moins trois produits.

Dans un exemple de mise en oeuvre, le système de distribution comporte au moins deux coupelles pouvant être sélectivement alimentées par le distributeur. Cela peut permettre à l'utilisateur de remplir rapidement ces deux coupelles avec des mélanges dont les caractéristiques sont différentes. Les coupelles peuvent être associées à des identifiants rappelant à l'utilisateur à quelle zone du visage un mélange contenu dans une coupelle donnée est destiné.

Les coupelles peuvent être mobiles relativement au distributeur, étant par exemple portées par un support mobile, tel qu'une tourelle mobile en rotation par rapport au distributeur, ou par un tiroir mobile en translation par rapport au distributeur.

Le système de distribution peut comporter un couvercle de fermeture de la coupelle. Ce couvercle de fermeture est de préférence transparent pour permettre à l'utilisateur de visualiser la couleur du mélange contenu à l'intérieur.

Lorsque la coupelle est séparable du distributeur, elle peut être introduite le cas échéant dans un boîtier permettant de la transporter plus facilement, ce boîtier pouvant comporter le cas échéant un miroir et/ou un applicateur. Le couvercle du boîtier peut dans ce cas servir de couvercle pour la coupelle.

Le volume de la coupelle peut être compris entre 2 et 1000 mm³, mieux entre 100 et 1000 mm³, encore mieux entre 250 et 750 mm³.

Le ou les produits de base délivrés dans la coupelle sont de préférence des fonds de teint, mais en variante il s'agit de produits de maquillage des lèvres ou des paupières.

La coupelle est de préférence de forme symétrique de révolution. En variante, elle est de contour polygonal ou autre. Son plus grand diamètre intérieur, ou celui du cercle inscrit dans le cas d'un contour non circulaire, est de préférence compris entre 2 et 100 mm, préférentiellement entre 5 et 40 mm. Sa profondeur est de préférence comprise entre 1 et 10 mm, mieux entre 3 et 8 mm. Sa profondeur est de préférence comprise entre 1 et 10 mm. De préférence, la taille et la forme de la coupelle permettent soit une application directe du mélange sur la peau, soit une préhension au doigt ou avec un applicateur. La coupelle peut être réalisée avec un matériau élastiquement déformable, ce qui permet par exemple d'inverser la concavité du fond de la coupelle et de vider celle-ci plus facilement ou de l'utiliser pour appliquer le produit.

La coupelle peut être dépourvue de mélangeur ; dans ce cas, les produits de base peuvent arriver du distributeur dans la coupelle à l'état non mélangé, par des orifices de distribution respectifs distincts. En variante, le distributeur intègre un mélangeur et les produits de base arrivent déjà mélangés dans la coupelle.

La coupelle peut aussi intégrer un mélangeur statique, comme détaillé plus loin, qui est alimenté par des orifices de remplissage distincts du distributeur, et qui délivre de préférence le mélange dans une cavité de la coupelle située au-dessus du mélangeur.

L'invention a encore pour objet un procédé de préparation d'un produit de maquillage filtrant, comportant l'étape consistant à remplir une coupelle d'un système de distribution tel que défini plus haut avec au moins un produit de base provenant du distributeur.

Les produits, de couvrance différentes, présenteront des effets de matité/brillance équivalents

Plusieurs produits peuvent être délivrés dans le fond de la coupelle, puis mélangés à l'aide du doigt ou d'un applicateur, ou d'un mélangeur statique intégré à la coupelle.

La coupelle est de préférence remplie par le dessous. Il a été proposé dans le passé des systèmes de distribution à l'aide d'une sonotrode.

La coupelle selon l'invention n'est pas destinée à entrer en vibration pour distribuer le ou les produits amenés par le ou les canaux d'alimentation de celle-ci. Elle est différente d'une sonotrode. De préférence, la coupelle est réalisée en matière plastique.

### Mélangeur intégré à l'interface de sortie

Il existe un intérêt pour bénéficier d'un système de distribution capable de délivrer un mélange pouvant être facilement utilisé, notamment prélevé par l'utilisateur, sans nécessiter une action additionnelle de mélange de la part de celui-ci.

Selon l'un de ses aspects, indépendamment ou en combinaison avec les autres aspects de celle-ci, et notamment ce qui précède, l'invention a pour objet un système de distribution comportant un distributeur ayant des canaux de sortie de produits de base et une interface de sortie séparable du distributeur, cette interface ayant un mélangeur statique, délivrant de préférence le mélange dans une cavité où il peut être prélevé.

Le mélangeur statique peut être situé sous la cavité précitée. Le système de distribution est alors particulièrement adapté à la création de compacts, en utilisant comme interfaces de sortie des coupelles à mélangeur statique intégré. Dans ce cas, on vient remplir la cavité de la coupelle en produit par le dessous. Après passage dans le mélangeur statique, les produits de base mélangés viennent recouvrir le mélangeur.

Selon cet aspect de l'invention, il est possible d'utiliser plusieurs interfaces de sortie et de les remplir avec des mélanges respectifs différents, sans avoir à purger le mélangeur, ce qui réduit les pertes de produit. L'interface de sortie peut être à usage unique, le cas échéant.

De préférence, le mélangeur statique comporte une chambre centrale communiquant avec des canaux d'admission des produits de base. Cette chambre centrale peut communiquer avec une chambre périphérique comportant une série de cloisons qui agissent comme des déflecteurs pour le mélange, et créent un cisaillement de celui-ci.

La chambre périphérique peut comporter une cloison annulaire ajourée définissant des ajours par lesquels passe le mélange en circulant dans la chambre périphérique. Les chambres centrale et périphérique peuvent être fermées supérieurement par une paroi qui définit le fond de la cavité recevant le mélange.

Le fond de la chambre périphérique peut être de forme hélicoïdale autour de l'axe de la coupelle et de hauteur diminuant au fur et à mesure que l'on progresse vers la sortie. Cette dernière peut déboucher en avant d'une rampe de liaison entre le fond de la chambre périphérique et la paroi supérieure du mélangeur, cette rampe de liaison étant de préférence une portion d'hélice prolongeant l'hélice formée par la paroi de fond de la chambre périphérique.

De préférence la chambre périphérique comporte la cloison annulaire précitée et des cloisons radiales qui forcent le mélange à circuler alternativement entre des régions supérieure et inférieure de la chambre périphérique et entre des régions radialement intérieure et extérieure, le mélange circulant par exemple d'une région supérieure et radialement extérieure à une région inférieure et radialement extérieure en passant à travers la cloison annulaire précitée.

Le mélangeur peut comporter un corps extérieur dans lequel est reçue une pièce formant le coeur du mélangeur, le corps extérieur fermant radialement à l'extérieur la chambre périphérique et comportant un montant qui sépare les chambres centrale et périphérique.

Le corps extérieur du mélangeur et le coeur du mélangeur peuvent être chacun réalisés d'une seule pièce par moulage par injection.

### Volume mort réduit

Il existe un intérêt pour réduire les pertes de produit lors des changements de formulation du mélange, ainsi que pour permettre de faire varier le plus rapidement possible la couleur du mélange au cours de l'application, notamment en cas de couplage du distributeur avec un aérographe.

Selon l'un de ses aspects, indépendamment ou en combinaison avec les autres aspects de celle-ci, notamment ce qui précède, l'invention a pour objet un système de distribution d'un produit de maquillage, comportant un distributeur recevant au moins deux cartouches ayant chacune un réservoir contenant un produit de base, ce dernier quittant la cartouche par un canal de sortie de la cartouche, ce canal de sortie débouchant à l'extérieur du distributeur ou à proximité de sa surface externe.

Le canal de sortie peut notamment déboucher dans une zone de prélèvement du mélange ou à proximité de celle-ci, notamment à moins de 5 mm, mieux à moins de 3 mm, mieux à moins de 1mm, mieux à fleur.

La section transversale du canal de sortie est par exemple comprise entre 1 et3 mm².

Ainsi, chaque produit de base provenant d'une cartouche peut quitter le distributeur sans se mélanger avec un produit de base d'une autre cartouche et l'on minimise le volume mort qui ne peut être prélevé et susceptible d'accroître l'inertie du système. Le produit est plus rapidement disponible sans avoir à circuler dans des canaux spécifiques du boîtier du distributeur, évitant une étape fastidieuse de purge si changement de cartouche.

L'extérieur du distributeur peut être la zone de prélèvement du produit, notamment lorsque le distributeur est réalisé avec une coupelle non prévue pour être enlevée, dans laquelle le mélange est distribué, ou une zone du distributeur destinée au montage d'une interface de sortie amovible, qui définit la zone de prélèvement. Cette interface de sortie peut comporter une coupelle telle que définie ci-dessus. Cette zone de montage correspond par exemple à l'extrémité du boîtier du distributeur en l'absence de l'interface de sortie. La zone de montage peut être sensiblement plane et perpendiculaire à l'axe longitudinal du boîtier du distributeur.

Le distributeur peut comporter trois cartouches de produits de base.

Le distributeur peut comporter des logements pour recevoir les cartouches, lesquelles sont de préférence reçues de façon amovible dans le distributeur. Ce dernier peut comporter des passages pour des conduits des cartouches définissant les canaux de sortie.

La longueur de ces conduits est de préférence telle que les conduits se situent légèrement en retrait du fond ou à affleurement de la cavité servant au prélèvement du produit, ou en variante légèrement en retrait ou à affleurement de la face d'extrémité du boîtier du distributeur définissant la zone de montage.

Ces conduits des cartouches peuvent être des embouts servant à entraîner en déplacement des pistons au sein des cartouches, comme détaillé plus haut.

### Interfaces de sortie multiples

Il existe un besoin pour pouvoir, à l'aide d'un même système de distribution, réaliser facilement des maquillages différents, et pouvoir si on le souhaite maquiller et/ou protéger des zones aussi différentes que la peau, les lèvres, le cou....

Selon l'un de ses aspects, indépendamment ou en combinaison avec les autres aspects de celle-ci, et notamment ce qui précède, l'invention a pour objet un système de distribution comportant un ensemble comportant un distributeur d'au moins un produit cosmétique, notamment de maquillage et/ou photoprotecteur, et au moins deux interfaces de sortie pouvant se monter chacune de façon amovible sur le distributeur, ces interfaces de sortie permettant de recevoir le ou les produits délivrés par le distributeur, étant de préférence choisies parmi les suivantes :
- interface de sortie comportant un contenant, notamment une coupelle permettant un prélèvement du produit au doigt ou à l'aide d'un applicateur,
- interface de sortie permettant de délivrer le produit à un système de pulvérisation, notamment un aérographe,
- interface de sortie comportant plusieurs régions de réception du produit, mobile par rapport au distributeur,
- interface de sortie permettant de délivrer le produit à un embout de distribution.

De préférence, l'ensemble comporte au moins trois desdites interfaces de sortie, mieux les quatre interfaces de sortie.

Le distributeur peut comporter au moins deux produits de base différents et permettre de délivrer ceux-ci dans des proportions variables, et de préférence, le distributeur comporte trois produits de base différents et permet de délivrer ceux-ci dans des proportions variables.

Chaque interface de sortie peut comporter une base permettant sa fixation sur le distributeur. Cette fixation peut s'effectuer à l'aide de vis par exemple, mais de préférence la base est agencée pour permettre un démontage et un remplacement d'une interface de sortie sans nécessité d'outil. Il s'agit par exemple d'une fixation quart de tour ou à l'aide d'une bague de verrouillage externe.

L'interface de sortie et/ou le boîtier du distributeur peuvent comporter des joints d'étanchéité permettant une communication étanche entre le boîtier du distributeur et l'interface de sortie. Le cas échéant, le distributeur est agencé pour reconnaître l'interface de sortie qui est montée dessus, par exemple grâce à identifiant de l'interface de sortie sous forme de reliefs spécifiques, lesquels sont détectés par le distributeur, ou d'une puce électronique reconnue par le distributeur. Cela peut permettre d'adapter le fonctionnement du distributeur à l'interface de sortie montée dessus. Le distributeur peut communiquer à un système informatique des informations sur l'interface de sortie qu'il porte, et le système informatique peut afficher en fonction de ces informations un écran spécifique et/ou lancer un programme spécifique de commande des paramètres de fonctionnement du distributeur, afin par exemple d'adapter la dose distribuée et/ou le débit à la nature de l'interface de sortie.

Plusieurs interfaces de sortie peuvent être proposées initialement à l'utilisateur avec un distributeur commun au sein d'un même emballage, par exemple un coffret ou une boîte de carton.

L'invention a encore pour objet un procédé de maquillage, comportant l'étape consistant à sélectionner une interface de sortie, à la monter sur le distributeur, et à délivrer le ou les produits contenus dans le distributeur dans celle-ci.

### Cartographie et apprentissage

Le terme « cartographie » s'entend ici comme un procédé d'indexation d'une couleur et d'une zone, avec enregistrement.

Il se peut qu'une personne nécessite plusieurs niveaux de filtration avec une seule matité/brillance pour l'ensemble des zones.

Il se peut aussi qu'elle nécessite plusieurs niveaux de filtration avec un niveau de matité/brillance sur une partie du visage et un autre niveau de matité/brillance pour une autre zone. Par exemple, elle a plusieurs taches sur le front et plusieurs taches sur les joues. Ainsi, elle voudra réaliser plusieurs niveaux de filtration pour le front avec un niveau de matité/brillance pour cette surface. Elle voudra réaliser plusieurs niveaux de filtration pour les joues avec un niveau de matité/brillance pour cette surface.

La cartographie peut concerner des applications sur des zones plus petites qu'un cm2. Toutefois, l'oeil nu a alors du mal à discerner si le résultat obtenu est adéquat, et il est préférable de substituer l'évaluation à l'oeil nu par une évaluation instrumentée, avec grossissement. L'application de petites quantités de matière colorée peut être faite au doigt, avec des outils classiques tels que des pinceaux, ou avec des applicateurs spécialisés.

La cartographie peut être générée lors d'une période d'apprentissage, pendant laquelle l'utilisateur effectue des tests avec des mélanges sur différentes zones du visage ; une fois renseignée, la cartographie peut ensuite être utilisée pour le maquillage de tous les jours.

Des interfaces graphiques spécifiques peuvent servir pendant la période d'apprentissage et pendant la période d'utilisation de la cartographie.

En particulier, le système de distribution peut être utilisé avec une interface graphique où l'opérateur voit le visage, qui est par exemple schématisé, figuratif ou précis comme une photographie ou une simulation 3D. Dans ce cas, l'opérateur peut pointer une partie du visage sur l'écran pour voir apparaître et/ou délivrer la couleur adéquate.

L'interface graphique peut aussi laisser apparaître les autres zones du visage où l'utilisation de la même couleur est adéquate.

Pour réaliser la cartographie, l'opérateur applique une couleur puis réalise l'évaluation.

Les zones du visage peuvent être traitées les unes après les autres ; par exemple, on réalise l'exercice sur une partie de la joue, puis sur le nez, etc....

Une autre possibilité consiste à réaliser un mélange donné, et appliquer sur plusieurs zones ce même mélange. L'opérateur doit alors chercher la zone du visage pour laquelle la couleur est adaptée. Le mélange est ensuite indexé dans le système informatique en l'attribuant à la ou aux zones du visage pour lesquelles il convient.

Selon l'un de ses aspects, indépendamment ou en combinaison avec les autres aspects de celle-ci, et notamment ce qui précède, l'invention a pour objet un procédé d'apprentissage d'un système de distribution comportant un distributeur permettant de distribuer un mélange de couleur variable, et un système informatique permettant de sélectionner une couleur et de mémoriser des données, comportant les étapes consistant à :
a) Sélectionner au moins une couleur à l'aide d'une interface du système informatique,
b) délivrer à l'aide du distributeur au moins un mélange de la couleur sélectionnée,
c) évaluer le ou les mélanges distribués après application sur au moins une zone du visage,
d) mémoriser les caractéristiques d'au moins un mélange, notamment un mélange que l'utilisateur souhaite pouvoir rappeler, et d'au moins une zone sur laquelle il a été testé.

Cette mémorisation peut s'effectuer notamment en vue d'une distribution ultérieure de ce mélange pour maquiller ladite zone.

De préférence, le système informatique est agencé pour permettre à l'utilisateur de signaler si le résultat du test est satisfaisant ou non, voire de le renseigner sur la comparaison à un test effectué précédemment.

On peut aussi réaliser un mélange donné, et chercher la zone du visage pour laquelle il est adapté. On enregistre alors le mélange en l'attribuant à la ou aux zones du visage pour lesquelles il est adapté, dans une table de correspondance qui servira par la suite, pour, partant d'une zone de la peau, en déduire le mélange à utiliser.

On peut procéder de la même façon avec d'autres mélanges pour réaliser une cartographie de l'ensemble du visage et ainsi avoir une table de correspondance complète du visage.

On peut aussi réaliser un mélange donné, l'appliquer sur une zone donnée, puis faire varier le mélange jusqu'à obtenir le mélange le plus adapté. On enregistre alors le mélange en l'attribuant à la ou aux zones du visage pour lesquelles il convient, dans une table de correspondance qui servira par la suite, pour, partant d'une zone de la peau, en déduire le mélange à utiliser.

De préférence, le système informatique évalue et mémorise les quantités utilisées zone par zone. Un tel procédé, par « touches d'essai », permet d'identifier le ou les produits dont a besoin la personne qui veut se maquiller. Ainsi, le système de distribution peut servir sur les points de vente pour conseiller les personnes qui veulent se maquiller ou à la maison pour bien définir les produits à commander.

L'interface du système informatique comporte de préférence un écran tactile affichant la couleur du mélange lors de sa sélection.

L'interface peut afficher un visage et permettre de renseigner le système informatique en sélectionnant la zone sur le visage affiché.

Le système informatique est de préférence agencé pour permettre d'associer une zone, des paramètres de reconstitution du mélange, et la date de l'essai et/ou tout autre identifiant du mélange.

Le système informatique est de préférence également agencé pour permettre d'associer en outre à ladite zone, aux paramètres de reconstitution du mélange, et à la date ou à l'identifiant du mélange, au moins l'une des données suivantes : le nom de la zone, la période de l'année, le nom d'un évènement, un identifiant de l'utilisateur, l'âge de l'utilisateur.

Les étapes a) à c) peuvent être renouvelées au moins une fois avant la mémorisation des caractéristiques du mélange à l'étape d).

Le système informatique peut être agencé pour rechercher dans une base de données une référence de produit commerciale, sur la base des caractéristiques du mélange identifié comme convenant à au moins une zone donnée, et la communiquer à l'utilisateur.

La sélection à l'étape a) peut s'effectuer avec l'aide d'un système expert externe ou non au système informatique.

Le système expert peut analyser une image de l'utilisateur, pour proposer une couleur de mélange au moins sur la base de l'image analysée.

L'étape a) peut être précédée par la proposition d'une couleur et d'une zone à tester avec un mélange de cette couleur, par le système informatique à l'utilisateur.

Le système informatique peut être agencé pour permettre à l'utilisateur de le renseigner sur l'appréciation du résultat du test de l'étape c) et pour générer une proposition de modification du mélange à sélectionner lors du retour à l'étape a).

Le système informatique peut être agencé pour proposer au moins une couleur de mélange à l'étape a) en fonction d'une zone d'application renseignée par l'utilisateur.

Le système informatique peut être agencé pour proposer au moins une zone d'application à l'étape a), en fonction d'une couleur renseignée par l'utilisateur.

Le distributeur peut délivrer à l'étape b) au moins deux mélanges de couleurs différentes, de préférence séparés, en vue de leur application simultanée sur la zone de test.

Cela peut faire gagner du temps, et faciliter la comparaison des résultats.

L'invention a encore pour objet un procédé de maquillage à l'aide d'un système de distribution selon cet aspect de l'invention, dans lequel :
a) L'utilisateur adresse une requête concernant un besoin de maquillage au système informatique,
b) celui-ci génère en retour une proposition de couleur en vue du maquillage d'une zone associée, sur la base de l'apprentissage effectué préalablement, et
c) le système informatique pilote le distributeur pour la production du mélange de la couleur proposée, notamment si celle-ci est validée par l'utilisateur.

Un tel procédé peut utiliser une cartographie précédemment établie avec l'utilisateur.

L'invention a encore pour objet un produit programme d'ordinateur comportant des instructions de code permettant, lorsque exécutées dans un système informatique, d'amener le système informatique à :
- Permettre à l'utilisateur de sélectionner au moins une couleur et/ou une zone d'application, notamment à l'aide d'une interface telle qu'un écran tactile,
- piloter un distributeur de façon à délivrer un mélange de la couleur sélectionnée par l'utilisateur,
- permettre à l'utilisateur de déclencher la mémorisation de la couleur du mélange et d'une zone d'application associée, notamment en vue d'une distribution ultérieure du même mélange, notamment sur la même zone.

Le produit programme d'ordinateur peut comporter des instructions de code permettant, lorsque exécutées dans un système informatique, d'amener le système informatique à :
- Recevoir une requête de l'utilisateur concernant un besoin de maquillage, notamment à l'aide d'une interface telle qu'un écran tactile,
- proposer sur la base au moins de données générées par le procédé d'apprentissage tel que défini ci-dessus, au moins une couleur et/ou une zone d'application,
- piloter un distributeur pour la production du mélange de la couleur proposée, notamment si celle-ci est validée par l'utilisateur.

La personne peut intégrer dans la cartographie le niveau de matité/brillance qu'elle désire pour chaque zone du visage, zone par zone ou ensemble de zones par ensemble de zones. Le système interprétera alors les mélanges qu'il doit réaliser pour assurer les couleurs, couvrance désirées et la matité/brillance voulu.

La cartographie peut être modifiée par la suite. Ainsi, il est possible de réaliser la cartographie sur le plan des couleurs /couvrance/filtration dans un premier temps, puis sophistiquer la cartographie en définissant par la suite les niveaux de matité/brillance.

### Aide à distance

Il est souhaitable de pouvoir aider l'utilisateur à se maquiller et/ou protéger, notamment à choisir les bons coloris.

L'invention, selon l'un de ses aspects, indépendamment ou en combinaison avec ses autres aspects, notamment ce qui précède, a ainsi pour objet un procédé de maquillage filtrant comportant les étapes consistant à :
- permettre l'établissement d'une liaison vidéo, par exemple via internet, entre une caméra d'un premier site et un deuxième site,
- permettre au deuxième site de piloter directement ou indirectement un distributeur présent sur le premier site, ce distributeur permettant de faire varier la couleur d'un mélange distribué,
- permettre à une personne présente au premier site d'appliquer le mélange distribué et d'envoyer vers le deuxième site une image correspondante, pour recevoir en retour une information relative au résultat de maquillage.

Le deuxième site peut notamment comporter un écran de visualisation qui permet à un conseiller présent devant cet écran de voir le résultat du maquillage avec le produit distribué par le distributeur et de conseiller la personne qui s'est maquillée et/ou protégée. Ce conseiller peut agir en retour sur le distributeur pour modifier la couleur du mélange et l'adapter au mieux au visage de la personne présente sur le premier site. Ainsi, cette personne gouverne le mélange délivré par le distributeur. La première personne peut se maquiller sous les yeux de la seconde. La seconde personne voit sur son écran le résultat de l'essai, et ainsi peut corriger le mélange qu'elle va commander à distance, jusqu'à obtenir le maquillage idéal. Elles peuvent donner des conseils sur le niveau de filtration.

Le cas échéant, l'acquisition vidéo peut être calibrée à l'aide d'une mire, ou avec le mélange distribué par le distributeur sur une surface de référence. Cela permet ensuite une visualisation plus fidèle du maquillage effectué sur le premier site.

De préférence, la liaison vidéo entre les deux sites est une liaison duplex.

Le premier site peut recevoir un tutoriel du deuxième site, le cas échéant.

Des identifiants des produits de base peuvent être communiqués au deuxième site ; cela peut permettre de connaître précisément la couleur de chacun des produits de base.

Le procédé peut comporter la mémorisation des paramètres de réglage du distributeur, une fois qu'un mélange donné est estimé satisfaisant. De préférence, cette mémorisation peut être commandée depuis le deuxième site. La mémorisation peut avoir lieu dans le système informatique présent sur le premier site et/ou sur un serveur externe.

Une variante peut consister à faire travailler une personne pour aider le maquillage de plusieurs personnes. Ce mode de mise en oeuvre permet le développement des « Make-Up Artists » et leur travail soit au sein d'un institut, soit via internet. Cela permet aussi le maquillage de personnes aux moyens limités, telles que les personnes malvoyantes, ou discernant mal les couleurs, ou âgées, ou manquant de confiance en elles.

### Pilotage par interface tactile

Il existe un besoin pour faciliter le pilotage du système de distribution et notamment le choix de la couleur du mélange distribué.

L'invention a encore pour objet un système de distribution comportant un distributeur et un système informatique de pilotage du distributeur, ce système informatique comportant un écran tactile sur lequel peut être affichée la couleur du mélange et un moyen de sélection déplaçable sur l'écran, pour faire varier la couleur du mélange distribué.

De préférence, l'écran affiche des couleurs extrêmes entre lesquelles la couleur du mélange peut être sélectionnée, en déplaçant le moyen de sélection entre ces couleurs extrêmes.

L'écran peut afficher une échelle de couleurs entre au moins deux couleurs, ou une surface, notamment de contour triangulaire, au sein de laquelle le moyen de sélection peut se déplacer. Cette surface peut localement faire apparaître la couleur du mélange en fonction par exemple de la distance à chacun des sommets, matérialisant chacun un produit de base pur.

Le système informatique peut effectuer une partie des calculs nécessaires pour déterminer les fractions de chacun des produits de base conduisant à un mélange de la couleur recherchée.

Le système informatique peut être un téléphone intelligent, un photophone, une tablette, un ordinateur individuel. En variante, il est intégré au boîtier du distributeur.

Le système informatique peut disposer d'une caméra. Cette dernière peut être utilisée notamment pour l'acquisition d'une image de l'utilisateur et/ou du mélange.

Le système informatique peut être agencé pour afficher une image d'un visage, afin de faciliter le repérage des zones sur lesquelles le mélange doit être appliqué.

### Couplage du système de distribution à un moyen de pulvérisation

Le système de distribution peut comporter ou être connecté à un moyen de pulvérisation du mélange, de préférence un aérographe.

L'invention a encore pour objet un ensemble comportant :
- Un moyen de pulvérisation, de préférence un aérographe comportant une chambre de prélèvement soumise à un courant d'air d'entraînement,
- un distributeur comportant au moins deux compartiments contenant des produits de base différents, les produits étant délivrés au moyen de pulvérisation de préférence par des orifices de distribution distincts.

Le distributeur peut comporter trois cartouches contenant des produits de maquillage de couleurs différentes.

L'aérographe peut comporter un stylet définissant la chambre de prélèvement, le stylet se fixant sur le distributeur ou sur une interface de sortie fixée au distributeur, ou faisant partie intégrante de cette interface de sortie.

Le système de distribution peut comporter un circuit de contrôle du fonctionnement du distributeur, permettant de faire varier la proportion des produits de base délivrés dans la chambre de prélèvement, au cours du fonctionnement de l'aérographe. Les proportions peuvent être modifiées en fonction du déplacement de l'aérographe relativement à la surface sur laquelle le mélange est pulvérisé. Ce déplacement peut être mécanisé, le cas échéant.

Ce circuit de contrôle peut comporter ou être constitué par un système informatique tel que défini plus haut.

Le boîtier du distributeur peut servir de poignée lors de la manipulation d l'ensemble pour délivrer le mélange.

Le distributeur peut comporter une caméra et/ou un ou plusieurs capteurs tels que des accéléromètres afin de localiser automatiquement la zone sur laquelle le mélange est appliqué, et pouvoir régler automatiquement la couleur en fonction de la position, le cas échéant.

L'invention a encore pour objet un procédé de maquillage à l'aide d'un ensemble tel que défini ci-dessus, dans lequel un mélange est pulvérisé sur la peau à l'aide du moyen de pulvérisation, notamment de l'aérographe.

La composition du mélange peut être modifiée au cours du déplacement de l'aérographe relativement à la peau. Un dégradé peut être réalisé.

Cet aspect de l'invention repose sur la constatation que le distributeur peut être utilisé pour alimenter le système de pulvérisation, notamment l'aérographe, tout en permettant au système de distribution d'être suffisamment réactif pour autoriser un changement de la couleur du mélange distribué en cours de maquillage du visage, notamment lorsque la zone à maquiller change.

Il peut être avantageux que la distribution des produits se fasse de façon itérative, notamment avec des temps de distribution non déphasés entre les différents produits.

Cela peut rendre plus facile de faire varier la composition du mélange distribué au cours du temps.

Le mélange peut être réalisé directement dans l'aérographe, sans pratiquement aucun volume mort gênant, permettant ainsi de changer le mélange pulvérisé en temps réel.

La dépression créée dans la chambre de prélèvement est suffisante pour entraîner les produits de base, sans pour autant gêner le dosage.

La dépression qui règne dans la chambre de prélèvement est par exemple comprise entre 10 mbar et 200 mbar, mieux entre 50 et 150 mbar, encore mieux entre 75 et 125 mbar.

La viscosité des produits de base mesurée à 1 atm et à 25°C avec un viscosimètre rotatif CONTRAVES TV équipé d'un mobile MS-r3 ou MS-r4 à la fréquence de 60 Hz, après 10 minutes de rotation du mobile, est par exemple comprise entre 0,05 Pa.S et 50 Pa.S
La section des canaux d'arrivée des produits de base dans la chambre est par exemple comprise entre 1 et 3 mm², mieux entre 2 et 3 mm².

L'alimentation en produit s'effectue de préférence de façon continue.

Il est encore possible d'appliquer simultanément des produits de base non miscibles ou réactifs, comme un gel aqueux et un gel huileux, qui vont se déposer de façon pixellisée directement sur la peau, produisant une sorte de gel/gel in situ, des silicones réactives, ou des produits de coloration réagissant ensemble. Les ratios en produits de base peuvent être ajustés en fonction du résultat particulier souhaité. Par exemple, dans le cas des gels aqueux et gels huileux, on pourra faire varier le ratio correspondant au volume du premier produit de base sur le volume du deuxième produit de base entre 10/1 et 1/10, mieux entre 5/1 et 1/5.

### Système de localisation ou d'auto-localisation

Le système de distribution selon l'invention peut disposer d'un système de localisation ou d'auto-localisation.

On appelle système de localisation, un moyen de saisir, de la part de la personne, la zone qu'elle va traiter. Ceci peut être réalisé notamment par des systèmes laissant libre au moins une main. Ainsi, on peut utiliser une interface d'un système informatique telle qu'un écran tactile, un joystick, ou un système de reconnaissance vocale.

On appelle système d'auto-localisation, un moyen de saisir, sans que la personne n'intervienne, la zone à traiter. Ceci peut être réalisé par un ou plusieurs accéléromètres qui déduisent, par le fait des mouvements, les directions que la personne vise ou par une caméra et un système de reconnaissance d'image.

L'invention selon ce sixième aspect peut être mise en oeuvre avec un système de distribution tel que décrit précédemment en référence aux figures, lesquelles ne seront donc pas décrites à nouveau.

### Exemple (sixième aspect de l'invention)

On réalise plusieurs produits de base avec des ingrédients filtrants et huileux.

La formule F1 est riche en filtre UV. La formule F2 est riche en huile.

| | | F1 | F2 |
|---|---|---|---|
| | | % massique | % massique |
| | Dimethicone copolyol vendu sous la référence KF 6017 par la société Shin Etsu | 2 | 2 |
| | Etsu Bis PEG/PPG-14/14 dimethicone + Cyclopentasiloxane vendu sous la référence ABIL EM 97 par la société Goldschmidt | 1 | 1 |
| | Cyclopentasiloxane | 17,65 | 17,65 |
| | Phenyl trimethicone vendu sous la référence DC556 par la société Dow Corning | 0 | 5 |
| | Ethyl hexyl methoxycinnamate | 5 | 0 |
| | Squalane | 1 | 1 |
| | Cyclopentasiloxane | 7 | 7 |
| | Oxyde de fer faune enrobé de stearoyl glutamate d'aluminium NAI-C33-9001-10 de la société Miyoshi Kasei | 1,45 | 1,45 |
| | Oxyde de fer rouge enrobé de stearoyl glutamate d'aluminium NALC33-8001-10 de la société Miyoshi Kasei | 0,4 | 0,4 |
| | Oxyde de fer noir enrobé de stearoyl glutamate d'aluminium NALC33-7001-10 de la société Miyoshi Kasei | 0,15 | 0,15 |
| | Dioxyde de titane (anatase) enrobé de stearoyl glutamate d'aluminium NAI-TAO-77891 de la société Miyoshi Kasei | 10 | 10 |
| | Talc vendu sous la référence Micro Ace P3 par la société Nippon Talc | 0,5 | 0,5 |
| | Poudre de nylon 12 vendue sous la référence SP 500 par la société Toray Industries | 0,5 | 0,5 |
| | Eau déminéralisée | 36,15 | 36,15 |
| | 1,3-Butylene glycol | 3 | 3 |
| | Sulfate de magnésium | 0,7 | 0,7 |
| | Solution de maltose hydrogénée | 0,5 | 0,5 |
| | Alcool éthylique 96° dénaturé | 13 | 13 |
| | TOTAL | 100 | 100 |

### Mode opératoire

On pèse les constituants de la phase A dans le bêcher principal et l'on agite en maintenant à température ambiante sous Moritz (1000 tr/min). La phase B est préparée séparément en broyant trois fois à la tricylindre, le mélange de pigments et de cyclopentasiloxane. Cette phase B est ensuite ajoutée en maintenant l'agitation, ainsi que les charges (phase C).

La phase aqueuse D est aussi préparée séparément, en pesant dans un bêcher le butylène glycol, le sulfate de magnésium, la solution de maltose hydrogénée et l'eau à température ambiante. La phase aqueuse D est placée sous agitation à l'aide d'un barreau magnétique jusqu'à homogénéisation. C D E L'émulsion se fait à température ambiante : on verse la phase aqueuse D dans la phase grasse en augmentant progressivement la vitesse d'agitation (Moritz) jusqu'à 4000 tours/mn. On maintient l'agitation pendant 7 mn. Puis on ajoute enfin la phase E (alcool éthylique) pendant les 3 dernières minutes de 5 l'émulsification.

### Test

Le système de distribution a été testé avec :
- F1 dans le compartiment A
- F2 dans le compartiment B

On utilise le système pour réaliser plusieurs mélanges (proportions volumiques):

| | |
|---|---|
| M1 : | A 30%, B 70% |
| M2 : | A 70%, B 30% |

M1 et M2 sont appliquées sur différentes zones du visage. On obtient des filtrations différentes mais avec des niveaux de matités équivalents.

### G) Système de distribution pour personnaliser les fonds de teint et leur pouvoir matifiant selon les zones du visage

Dans le cas du maquillage global ou le maquillage des peaux tachées, on souhaite avoir une esthétique la plus belle possible. Or, pour parfaire l'esthétique du visage, il ne suffit pas d'appliquer la meilleure couleur au bon endroit. Ainsi, on s'aperçoit que le niveau de matité/brillance idéal peut être différent selon la zone concernée. Certaines personnes aiment que les zones comme les joues soient plutôt brillantes. A l'inverse, elles aiment que d'autres zones comme le front, soient plutôt mates

Pour résoudre ce problème, il n'existe que peu de solutions.

Une première approche consiste à acheter de nombreux produits et à tous les essayer. Cette approche est coûteuse et génère souvent des pertes dans la mesure où l'on ne retient généralement qu'un petit nombre des matières colorées essayées.

Une seconde approche consiste à tester, en magasin, différents produits. Elle ne convient pas toujours, car il est très difficile de se figurer le résultat en quelques instants et dans un lieu dépourvu des repères habituels. En particulier, pour apprécier pleinement, l'effet d'un maquillage dans un magasin, il faudrait pouvoir l'éclairer de la même manière que dans les conditions futures d'utilisation, ce qui est rarement possible. En général, ce n'est qu'en testant un maquillage pendant la journée, qu'on se rend compte s'il convient ou non. De plus, si certains magasins disposent de conseillers et permettent d'effectuer des essais, ce n'est pas le cas d un grand nombre d'autres points de vente et des achats effectués sur internet.

Une autre approche a été testée sans toutefois se développer. Elle consiste à réaliser manuellement ses produits par mélange de plusieurs produits de couleur. Cela peut s'avérer relativement difficile à mettre en oeuvre, car il est peu aisé de reproduire exactement plusieurs fois le même mélange, et l'on a du mal à réaliser rapidement les mélanges dont on a besoin au moment du maquillage.

On connaît aussi des distributeurs permettant de délivrer une composition cosmétique de couleur variable.

Parmi les essais d'automatisation de la fabrication d'une composition cosmétique personnalisée qui ont pu être tentés, nombreux sont ceux dans lesquels les solutions proposées permettent de réaliser des mélanges dans des quantités d'environ 100 g ou parfois moins, mais pas dans les très petites proportions dont la personne qui se maquille a généralement besoin, c'est-à-dire de l'ordre du gramme ou beaucoup moins.

Pour illustrer cette problématique, on peut imaginer le cas d'une personne qui veut maquiller son visage avec un dégradé de matité/brillance allant du mat au brillant parant du nez (mat) jusqu'aux joues (brillantes) puis jusqu'au bord du visage (mat). Pour réaliser cet effet, il faut qu'elle puisse appliquer plusieurs touches de produit, de même couleur mais avec des niveaux de matité/brillance différents et commandables. Deux produits ne suffiront pas car produisant alors des démarcations entre les zones. Ainsi, il y a un besoin de disposer rapidement et de façon sure des petites quantités de produit avec matité/brillance variables. Les touches peuvent être par exemple de l'ordre des 10-50 mg par touche et servir à ne couvrir que des zones d'environ quelques cm2. Le même exercice est encore plus difficile si, pour éviter une sorte d'uniformité, la personne voudra faire varier la couleur (couleur et couvrance) tout en faisant varier le niveau de matité/brillance.

L'invention vise ainsi, selon certains de ses aspects, à faciliter le maquillage du visage et notamment la recherche des produits les plus adaptés en terme de matité/brillance aux différentes zones de celui-ci et l'obtention rapide d'un résultat avec des niveaux de matité/brillance adaptées aux différentes zones.

L'invention vise aussi à obtenir une couleur donnée, avec différents niveaux de matité/brillance.

L'invention repose selon un septième de ses aspects sur un système de distribution qui permet de générer des mélanges à partir de produits de base. Ces produits de base peuvent avoir des couleurs différentes (ou pas de couleur), de telle sorte que l'on puisse faire varier la matité/brillance du mélange. Les produits de base peuvent encore permettre de faire varier la couvrance et/ou couleur du mélange, de telle sorte que la couleur qui résulte de l'application du mélange sur les matières kératiniques humaines varie, en étant plus ou moins proche de celle desdites matières. Ainsi, l'invention selon ce septième aspect permet de fixer une couleur et/ou brillance et faire varier la matité/brillance ou faire varier l'ensemble.. La notion de couleur doit être comprise avec une acceptation large et englobe des mélanges dont la couleur varie après application du fait de leur couvrance plus ou moins élevée et de la couleur de la peau sous-jacente.

L'invention a ainsi pour objet selon un septième de ses aspects un système de distribution d'un produit, comportant un distributeur recevant au moins deux cartouches ayant chacune un réservoir et contenant respectivement des premier et deuxième produits de base, le premier produit de base comportant l'un au moins d'une huile, d'un pigment interférentiel ou à reflet métallique, le deuxième produit de base comportant une charge matifiante, le distributeur permettant de délivrer au moins ces deux produits de base dans des proportions réglables.

L'invention, selon ce septième aspect, peut présenter l'une ou plusieurs des caractéristiques préférentielles suivantes :
- le premier produit de base contient une huile,
- le premier produit de base contient un pigment interférentiel,
- le premier produit de base contient un pigment à reflet métallique,
- la taille D50 en volume de particule du pigment et de la charge est comprise entre 100 nm et 1 mm, mieux entre 200 nm et 100 microns, encore mieux entre 200 nm et 50 microns,
- la charge est choisie parmi le talc, le mica, la silice, le kaolin, les poudres de poly-β-alanine et de polyéthylène, les poudres de polymères de tétrafluoroéthylène, les poudres de nylon, les poudres de polymethacrylate de methyle, la lauroyl-lysine, l'amidon, le nitrure de bore, les microsphères creuses polymériques telles que celles de chlorure de polyvinylidène/acrylonitrile, de copolymères d'acide acrylique, les microbilles de résine de silicone, les particules de polyorganosiloxanes élastomères, le carbonate de calcium précipité, le carbonate et l'hydro-carbonate de magnésium, l'hydroxyapatite, le sulfate de barium, les oxydes d'aluminium, les poudres de polyuréthane, les charges composites, les microsphères de silice creuses, et les microcapsules de verre ou de céramique, les particules qui ont la forme de portions de sphères creuses, et leurs mélanges, préférentiellement est choisie parmi le talc, le mica, la silice, les poudres de nylon, les poudres de polymethacrylate de methyle, et leurs mélanges, encore préférentiellement est du talc,
- la charge peut comporter un enrobage comportant au moins un composé lipophile ou hydrophobe,
- le premier produit de base et le deuxième produit de base comportent une émulsion inverse,
- la teneur massique en charge dans le deuxième produit de base est supérieure ou égale à 0,5% de la masse du premier produit de base, de préférence supérieure à 1% de la masse du premier produit de base, encore de préférence comprise entre 1% et 5% de la masse du premier produit de base,
- le système de distribution comporte une troisième cartouche avec un troisième produit de base,
- les cartouches sont reçues de façon amovible dans le distributeur,
- chaque produit de base quitte la cartouche par un canal de sortie de la cartouche, le canal de sortie étant défini par un embout de distribution de la cartouche entraîné en rotation relative par rapport à un corps de la cartouche par un mécanisme d'entraînement du distributeur pour distribuer le produit de base contenu dans la cartouche.

Selon son septième aspect, l'invention a encore pour objet un procédé d'application d'un produit cosmétique, de maquillage et/ou de soin, sur les matières kératiniques humaines, comportant le réglage du distributeur en fonction de la zone à traiter, et la distribution du produit par prélèvement dans les cartouches des produits de base dans les proportions correspondant au réglage du distributeur.

L'invention repose ainsi selon son septième aspect sur un système de distribution, avec des compartiments contenant au moins deux formulations différentes, lesquelles contiennent de préférence des émulsions inverses concentrées pour l'une en huiles, nacres, ou particules à reflet métallique, et concentrée pour l'autre en charges (matifiantes).

On peut donc faire varier le niveau de matité/brillance tout en gardant un aspect coloriel égal.

On peut programmer le système pour qu'il délivre pour chaque endroit du visage le meilleur mélange matifiant ou brillant (ni trop fort, ni trop faible). L'aspect global ne permettra pas de deviner que le visage a été traité car permettant de se rapprocher de l'aspect d'un visage naturel.

### Système de distribution

Le système de distribution peut être constitué par un seul appareil fonctionnant de manière autonome, de préférence manipulable d'une main, ou par un appareil qui fonctionne en interagissant avec d'autres pièces ou appareils. Il peut s'agir par exemple de différentes interfaces de sorties qui sont montées sur le distributeur en fonction du type de maquillage à réaliser, comme cela sera précisé plus loin. Il peut s'agir également d'un système informatique qui échange des informations avec le distributeur pour le piloter, ce système informatique comportant par exemple un terminal portable tel qu'un téléphone intelligent, un photophone, une tablette, un ordinateur personnel, ou un terminal dédié.

De préférence, le distributeur est agencé pour mettre sous pression un ou plusieurs compartiments contenant le ou les produits de base, par le biais de dispositifs de dosage volumétrique, de préférence un moteur entraînant en déplacement un piston dans le compartiment correspondant.

Le distributeur peut être formé d'un boîtier et d'au moins deux ou trois compartiments, et de préférence d'autant de moteurs. Par exemple, la rotation des moteurs entraîne des vis sans fin, lesquelles poussent les pistons de chaque compartiment. L'avance des pistons est par exemple contrôlée par le nombre d'impulsions de commande envoyées aux moteurs et/ou par la durée de fonctionnement de ces derniers. Les moteurs peuvent être alimentés séquentiellement, ou de préférence simultanément.

Par exemple, les moteurs sont alimentés durant un cycle élémentaire de fonctionnement pendant une courte durée les uns après les autres, ou les uns en même temps que les autres, de façon à distribuer des microdoses correspondantes.

Les cycles élémentaires sont répétés, avec éventuellement un temps d'arrêt entre eux, qui laisse le temps aux produits de base de s'écouler hors des compartiments.

Les compartiments peuvent être définis par des cartouches qui se retirent lorsqu'elles sont vides. En variante, les compartiments sont présents à demeure et remplis à nouveau une fois vide.

Chaque cartouche peut être fermée par un bouchon démontable pour permettre le nettoyage de la cartouche.

De préférence, le boîtier du distributeur est de forme allongée selon un axe longitudinal, ce qui peut faciliter sa manipulation, et les cartouches sont disposées autour de cet axe, dans le boîtier.

De préférence, les cartouches sont mises en place par l'arrière, et le mélange est délivré par l'avant. La mise en place des cartouches peut s'effectuer individuellement, ou en variante les cartouches constituent un ensemble monobloc lors de leur mise en place.

Les cartouches peuvent comporter chacune un mécanisme de dosage volumétrique, comportant un piston mu par un mécanisme d'entraînement du distributeur, dans une direction s'accompagnant d'une diminution du volume intérieur contenant le produit de base et d'une expulsion du produit. Il peut être intéressant que les cartouches aient une région de leur paroi au moins qui soit transparente, afin de permettre de voir la couleur du produit contenu à l'intérieur.

Le mécanisme d'entraînement peut comporter un système de motorisation formé de moteurs couplés à des réducteurs, de forme allongée parallèlement à l'axe longitudinal du distributeur, et disposés entre les cartouches. Ce positionnement des moteurs et cartouches rend le distributeur particulièrement compact.

Le produit de base peut sortir de façon étanche de la cartouche correspondante puis circuler dans un canal prévu à cet effet dans le boîtier du distributeur, avant de sortir de celui-ci.

Les cartouches se terminent avantageusement par un embout réalisé de telle sorte que, une fois la cartouche insérée dans le boîtier du distributeur, l'extrémité de l'embout débouche au ras du boîtier. En variante, l'embout est suffisamment long pour dépasser du boîtier et connecter ainsi différentes interfaces de sortie susceptibles d'être rapportées sur le boîtier du distributeur.

Grâce au mécanisme d'entraînement comportant des moteurs pour faire avancer des pistons, il est possible de délivrer de façon précise des mélanges en de très petites quantités. Ainsi, le mécanisme d'entraînement peut délivrer les produits de base avec à un débit minimal inférieur ou égal à 50 µL/s, mieux inférieur ou égal à 20 µL/s, encore mieux inférieur ou égal 10 µL/s. De préférence, le mécanisme d'entraînement délivre des débits compris entre 20 et 100 µL/s, mieux compris entre 40 µL/s et 60 µL/s. Il est donc possible de réaliser facilement un mélange d'environ 10 mg. Un tel système de distribution est alors idéal pour réaliser de petites touches de maquillage, pour couvrir par exemple une zone de 1 cm², mieux une zone de 0, 5 cm².

Il est aussi possible de réaliser des plus grandes quantités de mélange comme celles nécessaires au maquillage d'une joue, ou d'un visage. Ces quantités restent néanmoins relativement faibles, par exemple une quantité comprise entre 100 et 500 mg, mieux comprise entre 150 et 250 mg.

Le distributeur présente de préférence un boîtier, et au moins une cartouche reçue dans le boîtier du distributeur, cette cartouche comportant un corps et un piston mobile dans le corps, le boîtier comportant un mécanisme d'entraînement motorisé pour déplacer le piston de la cartouche.

De préférence, la cartouche comporte un embout de distribution par lequel s'effectue la sortie du produit, et cet embout de distribution est entraîné en rotation par le mécanisme d'entraînement pour déplacer le piston. L'embout peut comporter au moins un relief d'anti-rotation, mieux deux ergots d'anti-rotation diamétralement opposés.

L'embout peut porter un joint d'étanchéité, notamment un joint torique. Ainsi, au changement de cartouche, le joint est également changé, ce qui permet de s'affranchir de l'usure du joint.

Le distributeur peut comporter une carte électronique de pilotage du mécanisme d'entraînement motorisé, cette carte électronique étant traversée par le ou les embouts. Cela peut permettre de réaliser une carte s'étendant sensiblement sur toute la section du distributeur, donc de regrouper sur une seule carte tous les composants électroniques du distributeur, et ainsi de gagner en compacité et fiabilité. La carte peut s'étendre sensiblement perpendiculairement à l'axe longitudinal du boîtier. La carte peut porter un interrupteur de commande du fonctionnement du distributeur.

Le système de distribution peut être agencé pour fonctionner selon au moins deux modes de distribution.

Dans un premier mode, dit « continu », le mélange est distribué tant qu'une pression est exercée sur l'interrupteur de commande.

Dans un deuxième mode, dit « dose », une quantité prédéfinie du mélange est distribuée à chaque pression sur l'interrupteur.

Le ou les embouts peuvent déboucher à une extrémité du boîtier. Cela peut permettre de réduire le volume mort, comme cela sera détaillé dans la suite.

Le ou les embouts peuvent présenter à leur extrémité un système d'obturation pour éviter le séchage des produits dans le conduit, par exemple une membrane cicatrisante

La cartouche peut comporter une vis creuse sur laquelle le piston est vissé, le piston pouvant se déplacer axialement sur la vis lorsque celle-ci tourne ; le piston est empêché de tourner dans le corps de la cartouche. Par exemple, la friction du piston sur le corps de la cartouche peut suffire à l'empêcher de tourner quand la vis tourne.

Préférentiellement, la rotation est rendue impossible avec un corps de cartouche de section non circulaire et un piston non déformable.

Le couple des moteurs peut être déterminé électroniquement en fonction du courant absorbé, et servir par exemple à détecter une fin de course du piston. Une information concernant le couple peut être transmise à distance à un système informatique présentant une interface homme machine, pour permettre de surveiller le bon fonctionnement du distributeur.

Afin d'ajuster la teinte, le système de distribution selon l'invention doit permettre à l'utilisateur de faire varier le volume délivré provenant de chaque compartiment.

De façon préférée, le distributeur est piloté par un système informatique, qui est intégré au distributeur, ou externe à celui-ci, le distributeur étant alors apte à échanger avec le système informatique des informations par protocole sans fil ou câblé.

Le distributeur peut ainsi être piloté pour permettre l'ajustement de la teinte par distribution simultanée ou séquentielle, et contrôlée, de plusieurs produits de base de couleurs différentes.

La distribution des produits de base peut être continue ; si dans ce cas, les volumes de chacun des produits de base sont distribués d'une seule traite, simultanément ou successivement.

Dans le cas d'une distribution simultanée, il est utile de pouvoir ajuster les débits respectifs des différents produits de base pour que le mélange distribué corresponde à celui souhaité à chaque instant. Un tel mode de distribution peut convenir en particulier lors de la distribution du mélange par pulvérisation, à l'aide d'un aérographe. Pour ajuster les débits il est possible par exemple de jouer sur la vitesse de déplacement des pistons, par exemple, dans le cas d'un entrainement des pistons par une vis sans fin en faisant varier la vitesse de rotation des moteurs qui entrainent la vis. Les produits peuvent aussi être distribués de façon impulsionnelle avec un temps de distribution et un temps d'arrêt à chaque cycle. En jouant sur le rapport cyclique, il est possible d'agir sur le débit.

Tous les produits peuvent être délivrés simultanément lors du temps de distribution ; en variante, les cycles des différents produits sont déphasés, de sorte qu'un produit est distribué pendant un temps d'arrêt des autres produits.

Dans un mode particulier de l'invention, le mélange est délivré dans une cavité d'un contenant, pouvant fermer de façon hermétique ou non, par exemple en forme de coupelle, dans lequel on peut faire glisser un applicateur, en particulier un stylet ou une brosse.
- Lorsqu'il n'est pas amovible, le contenant peut être réalisé avec le corps du distributeur. Lorsqu'il est amovible, il peut constituer une interface de sortie parmi d'autres pouvant être montées sur le distributeur.

Les compartiments, et en particulier les cartouches, peuvent contenir tout ou partie du mécanisme d'entraînement, et par exemple le système de motorisation, ou mieux, une partie du système de motorisation, et ce, afin de réduire le nombre de pièces en mouvement dans le corps du distributeur à l'extérieur des cartouches. Par exemple, les cartouches comportent le rotor du moteur. Une fois les cartouches installées dans le corps du distributeur, les rotors sont mis en interaction avec les stators.

Le système de distribution est avantageusement agencé pour permettre de lancer des séquences préprogrammées dans lesquelles le mélange délivré par celui-ci est modifié de façon continue ou discontinue. Un mode « dégradé » permet par exemple passer progressivement d'un mélange A à un mélange B. Dans le cas d'une application par pulvérisation, notamment à l'aide d'un aérographe, cela permet de réaliser simplement les dégradés. Un mode « Alternatif » permet par exemple de passer rapidement d'un mélange

A à un mélange B plusieurs fois de suite. Dans le cas d'une application par pulvérisation, on peut ainsi réaliser un dépôt multicouche, avec des formulations différentes pour deux couches adjacentes superposées. Un autre mode permet par exemple de proposer plusieurs mélanges successifs préprogrammés, le système informatique indiquant à chaque fois l'usage qu'il faut en faire à l'utilisateur, par exemple par affichage sur un écran.

Dans le cas d'une application manuelle, les mélanges sont distribués par exemple dans une coupelle. La personne se maquille à l'endroit recommandé avec un mélange correspondant prélevé dans la coupelle, puis nettoie éventuellement la coupelle et commande la délivrance d'un nouveau mélange ; l'opération est répétée autant de fois que nécessaire pour se maquiller complètement.

L'homogénéisation des mélanges distribués peut se faire de différentes façons selon les cas d'utilisation. Dans le cas d'une application manuelle, elle peut se faire directement sur la zone d'application au moment de l'application ou dans la coupelle avant prélèvement ; dans le cas d'une application à l'aérographe, c'est la tuyère de l'aérographe qui sert de chambre de mélange ; dans le cas où le mélange est distribué dans un contenant en vue d'une utilisation ultérieure, l'homogénéisation peut être faite à la main ou en faisant passer les produits distribués dans une chambre de mélange, située entre le distributeur et le contenant ou directement intégrée au contenant, comme détaillé par la suite.

Le produit peut être délivré par le système de distribution et utilisé extemporanément. En variante, le produit délivré par le système de distribution est conditionné et utilisé ultérieurement, par exemple à plusieurs reprises, avec par exemple un jour d'intervalle au moins entre deux utilisations.

L'invention repose selon son septième aspect sur un système de distribution, des compartiments contenant au moins deux formulations différentes lesquelles peuvent contenir des émulsions inverses concentrées pour l'une en huiles, nacres, ou particules à reflet métallique, et concentrée pour l'autre en charges (matifiantes).

### Huiles

Par « huiles », on entend une huile restant sur la peau à température ambiante et pression atmosphérique au moins plusieurs heures et ayant notamment une pression de vapeur inférieure à 0,13 Pa (0,01 mm de Hg).

Ces huiles non volatiles peuvent être des huiles hydrocarbonées notamment d'origine animale ou végétale, des huiles siliconées, ou leurs mélanges. On entend par "huile hydrocarbonée", une huile contenant principalement des atomes d'hydrogène et de carbone et éventuellement des atomes d'oxygène, d'azote, de soufre et/ou de phosphore.

Comme huile hydrocarbonée non volatile, on peut notamment citer :
- les huiles hydrocarbonées d'origine animale,
- les huiles hydrocarbonées d'origine végétale telles que les triglycérides constitués d'esters d'acides gras et de glycérol dont les acides gras peuvent avoir des longueurs de chaînes variées de C4 à C24, ces dernières pouvant être linéaires ou ramifiées, saturées ou insaturées; ces huiles sont notamment des triglycérides d'acide heptanoïque ou d'acide octanoïque, ou bien encore les huiles de germe de blé, de tournesol, de pépins de raisin, de sésame, de maïs, d'abricot, de ricin, de karité, d'avocat, d'olive, de soja, d'amande douce, de palme, de colza, de coton, de noisette, de macadamia, de jojoba, de luzerne, de pavot, de potimarron, de sésame, de courge, de colza, de cassis, d'onagre, de millet, d'orge, de quinoa, de seigle, de carthame, de bancoulier, de passiflore, de rosier muscat ; le beurre de karité ; ou encore les triglycérides des acides caprylique/caprique,

- les éthers de synthèse ayant de 10 à 40 atomes de carbone;
- les hydrocarbures linéaires ou ramifiés, d'origine minérale ou synthétique tels que la vaseline, les polydécènes, le polyisobutène hydrogéné, le squalane, les huiles de paraffine, et leurs mélanges,
- les esters de synthèse comme les huiles de formule R1COOR2 dans laquelle R1 représente le reste d'un acide gras linéaire ou ramifié comportant de 1 à 40 atomes de carbone et R2 représente une chaîne hydrocarbonée notamment ramifiée contenant de 1 à 40 atomes de carbone à condition que R1 + R2 soit ≥ 10, comme par exemple l'huile de Purcellin (octanoate de cétostéaryle), le myristate d'isopropyle, le palmitate d'isopropyle, les benzoates d'alcools en C12 à C15, le laurate d'hexyle, l'adipate de diisopropyle, l'isononanoate d'isononyle, le néopentanoate d'isodecyl, le palmitate de 2-éthyl-hexyle, l'isostéarate d'isostéaryle, le laurate de 2-hexyl-décyle, le palmitate de 2-octyl-décyle, le myristate de 2-octyl-dodécyle, des heptanoates, octanoates, décanoates ou ricinoléates d'alcools ou de polyalcools comme le dioctanoate de propylène glycol ; les esters hydroxylés comme le lactate d'isostéaryle, le malate de di-isostéaryle, le lactate de 2-octyl-dodécyle ; les esters de polyols et les esters du pentaérythritol,
- les alcools gras liquides à température ambiante à chaîne carbonée ramifiée et/ou insaturée ayant de 12 à 26 atomes de carbone comme l'octyl dodécanol, l'alcool isostéarylique, l'alcool oléique, le 2-hexyldécanol, le 2-butyloctanol, et le 2-undécylpentadécanol,
- les acides gras supérieurs tels que l'acide oléique, l'acide linoléique, l'acide linolénique et leurs mélanges.

Les huiles de silicone non volatiles utilisables dans la composition selon l'invention peuvent être les polydiméthylsiloxanes (PDMS) non volatiles, les polydiméthylsiloxanes comportant des groupements alkyle ou alcoxy pendants et/ou en bouts de chaîne siliconée, groupements ayant chacun de 2 à 24 atomes de carbone, les silicones phénylées comme les phényl triméthicones, les phényl diméthicones, les phényl triméthylsiloxy diphénylsiloxanes, les diphényl diméthicones, les diphényl méthyldiphényl trisiloxanes, et leurs mélanges.

De manière préférentielle, la phase huileuse comprend au moins une huile siliconée et encore plus préférentiellement choisie parmi
- les huiles volatiles siliconées cycliques ayant une viscosité à température ambiante inférieure à 8 cSt et ayant notamment de 4 à 7 atomes de silicium, ces silicones comportant éventuellement des groupes alkyle ou alcoxy ayant de 1 à 10 atomes de carbone, en particulier choisie parmi l'hexamethylcyclotrisiloxane, l'octamethylcylotetrasiloxane, le decamethylcyclopentasiloxane et le dodecamethylcyclohexasiloxane (cyclohexasiloxane), et leurs mélanges ;
- les polydiméthylsiloxanes (PDMS) volatiles ou non volatiles (Nom INCI : DIMETHICONE) ;
- les silicones phénylées ;
- les polydiméthylsiloxanes comprenant des groupes aliphatiques, en particulier alkyle, ou alcoxy, qui sont pendants et/ou à la fin de la chaîne silicone ; ces groupes comprenant chacun de 6 à 24 atomes de carbone, et plus particulièrement le caprylyl methicone tel que le produit commercial DOW CORNING FZ-3196^{®} par la société DOW CORNING ;
- leurs mélanges.

### Pigment interférentiel (Nacres)

Par « pigment interférentiel » encore appelé « *nacres* », il faut comprendre des particules colorées de toute forme, irisées ou non, notamment produites par certains mollusques dans leur coquille ou bien synthétisées, et qui présentent un effet de couleur par interférence optique.

Les nacres peuvent être choisies parmi les pigments nacrés, tels que le mica titane recouvert avec un oxyde de fer, le mica titane recouvert avec de l'oxychlorure de bismuth, le mica titane recouvert avec de l'oxyde de chrome, le mica titane recouvert avec un colorant organique, ainsi que les pigments nacrés à base d'oxychlorure de bismuth. Il peut également s'agir de particules de mica à la surface desquelles sont superposées au moins deux couches successives d'oxydes métalliques et/ou de matières colorantes organiques.

On peut également citer, à titre d'exemple de nacres, le mica naturel recouvert d'oxyde de titane, d'oxyde de fer, de pigment naturel ou d'oxychlorure de bismuth.

Les nacres peuvent plus particulièrement posséder une couleur ou un reflet jaune, rose, rouge, bronze, orangé, brun, or et/ou cuivré.

De manière avantageuse, les nacres conformes à l'invention sont les micas recouverts de dioxyde de titane ou d'oxyde de fer ainsi que l'oxychlorure de bismuth.

### Particules à reflet métallique

Par « *particules à reflet métallique* », au sens de la présente invention, on entend tout composé dont la nature, la taille, la structure et l'état de surface lui permet de réfléchir la lumière incidente notamment de façon non iridescente.

Les particules à reflet métallique utilisables dans l'invention sont en particulier choisies parmi :
- les particules d'au moins un métal et/ou d'au moins un dérivé métallique ;
- les particules comportant un substrat, organique ou minéral, monomatière ou multimatériaux, recouvert au moins partiellement par au moins une couche à reflet métallique comprenant au moins un métal et/ou au moins un dérivé métallique ; et
- les mélanges desdites particules.

Parmi les métaux pouvant être présents dans lesdites particules, on peut citer par exemple Ag, Au, Cu, Al, Ni, Sn, Mg, Cr, Mo, Ti, Zr, Pt, Va, Rb, W, Zn, Ge, Te, Se et leurs mélanges ou alliages. Ag, Au, Cu, Al, Zn, Ni, Mo, Cr, et leurs mélanges ou alliages (par exemple les bronzes et les laitons) sont des métaux préférés.

Par « *dérivés métalliques* », on désigne des composés dérivés de métaux notamment des oxydes, des fluorures, des chlorures et des sulfures.

### Charges

Ces charges sont des particules incolores ou blanches, solides de toutes formes, qui se présentent sous une forme insoluble et dispersée dans le milieu de la composition.

A titre illustratif de ces charges, peuvent être cités le talc, le mica, la silice, le kaolin, les poudres de poly-β-alanine et de polyéthylène, les poudres de polymères de tétrafluoroéthylène (Téflon^{®}), les poudres de nylon, les poudres de polymethacrylate de methyle, la lauroyl-lysine, l'amidon, le nitrure de bore, les microsphères creuses polymériques telles que celles de chlorure de polyvinylidène/acrylonitrile comme l'Expancel^{®} (Nobel Industrie), de copolymères d'acide acrylique, les microbilles de résine de silicone (Tospearls^{®} de Toshiba, par exemple), les particules de polyorganosiloxanes élastomères, le carbonate de calcium précipité, le carbonate et l'hydro-carbonate de magnésium, l'hydroxyapatite, le sulfate de barium, les oxydes d'aluminium, les poudres de polyuréthane, les charges composites, les microsphères de silice creuses, et les microcapsules de verre ou de céramique. On peut également utiliser des particules, qui ont la forme de portions de sphères creuses, telles que décrites dans les demandes de brevet JP-2003 128 788 et JP-2000 191 789.

Les charges peuvent comporter un enrobage comportant au moins un composé lipophile ou hydrophobe.

### Emulsions inverses / Compositions anhydres

Selon l'invention, lorsqu'il est précisé qu'une composition est sous la forme d'une émulsion inverse, il est entendu qu'elle peut également être alternativement sous une autre forme selon la composition considérée.

Un produit de base peut se présenter sous la forme d'une émulsion, par exemple une émulsion H/E, E/H, H/E/H ou E/H/E, et de préférence une émulsion inverse E/H, ou sous la forme d'une composition anhydre, comprenant notamment des composés carbonés et/ou siliconés, tels que des huiles hydrocarbonées et/ou siliconées.

Les émulsions selon le septième aspect de l'invention sont de préférence des émulsions eau-dans-huile (E/H) encore appelées émulsions inverses, à savoir constituées d'une phase huileuse continue dans laquelle est dispersée la phase aqueuse sous forme de gouttelettes de manière à observer un mélange macroscopiquement homogène à l'oeil nu.

### Utilisation pour le maquillage d'une ou plusieurs zones précises de peau

Le système de distribution permet de se maquiller jour après jour, en ne traitant que les zones à masquer. Pour ce faire, on délivre de petites doses de maquillage, qu'on applique précisément et séquentiellement sur les zones correspondantes. Chaque petite dose est réalisée selon le mélange qui convient à la zone.

Dans un mode préféré de mise en oeuvre de l'invention, le système de distribution attend qu'on le renseigne sur une zone à traiter puis délivre le mélange correspondant. Il peut faire appel à une table de correspondance préprogrammée pour cela, cette table résultant par exemple d'un procédé d'apprentissage tel que défini plus loin. En variante, le système de distribution renseigne la personne, tout en délivrant un mélange, sur la zone sur laquelle celle-ci doit appliquer le mélange. Ainsi, le système de distribution peut suivre un programme d'application où il délivre dans un ordre donné, les différents mélanges à appliquer.

Dans un mode particulier de mise en oeuvre de l'invention, le système de distribution est renseigné sur les quantités à délivrer. Pour cela, il mémorise la correspondance entre la matité/brillance, la zone du visage et la quantité nécessaire, ce qui permet de réduire les coûts et les pertes de produit, et de ne couvrir que faiblement la peau, évitant ainsi des effets d'occlusion. Ce faisant, il est possible d'utiliser des produits très matifiants et trop matifiants ou très brillants ou trop brillants pour être appliqués sur l'ensemble du visage. Ainsi, il est possible d'obtenir des maquillages d'aspects naturels, voire indétectables.

Le système de distribution peut aussi permettre, en facilitant la distribution de petites quantités et leur usage rapide, de réduire la durée de conservation des produits, permettant ainsi de réduire les risques d'évolution et/ou les quantités de conservateurs à employer.

Le système de distribution est adapté à traiter les zones qu'on veut masquer, sans avoir à masquer l'ensemble du visage.

Dans une variante spécialement avantageuse, le système peut être utilisé pour application sur le fond du visage une formule matte puis une ou deux applications sur des zones précises du visage des formules plus brillantes. On peut aussi commencer par applications sur des zones précises du visage des formules brillantes puis appliquer sur le fond du visage une application d'une formule plus matte.

Dans le second cas, on prend le temps, avant d'appliquer la seconde couche, d'attendre que le film produit par l'application sur les zones précises devienne suffisamment cohésif pour que la seconde couche n'emporte pas la première couche.

Ou bien, on applique la seconde couche sans frotter (spray par exemple, ou éponge).

Lorsque l'utilisateur recherche la matité/brillance à appliquer sur une zone du visage, il est intéressant de mémoriser la matité/brillance qui va bien pour chaque zone, et le système de distribution est ainsi avantageusement agencé pour mémoriser cette matité/brillance et la zone correspondante. Ainsi, en utilisant les informations mémorisées, à chaque utilisation, on peut obtenir la délivrance du même mélange pour chaque zone, ou, si l'on traite plusieurs zones, la même série de mélanges pour une même série de zones.

Le système de distribution peut aussi être agencé pour permettre de traiter une zone en faisant varier les matité/brillances application après application. Ainsi, la personne peut maquiller les lèvres avec différentes couleurs, qu'elle choisit au jour le jour selon ses goûts. Cette approche est aussi adaptée aux paupières, ainsi qu'au maquillage du visage car la personne peut avoir envie de changer de couleur de teint. Par exemple, les jours de la semaine, la personne réalise un fond de teint couleur plutôt mat, et le weekend, un fond de teint de plutôt brillant.

Le système de distribution peut être agencé pour permettre à l'utilisateur de changer de matité/brillance en plus des paramètres couleurs/couvrance selon ses goûts du jour, du moment, de ses tenues, du climat. Ainsi, on prévoit avantageusement un système d'aide à la décision pour guider l'utilisateur dans ses choix de matité/brillance et/ou couleur (au sens large).

On peut prévoir aussi un système d'aide pour équilibrer les matité/brillance sur un même visage et participer à la réalisation d'un maquillage global réussi.

Il peut être souhaitable que plusieurs personnes d'un même groupe, par exemple une famille, puissent utiliser le système de distribution, réduisant ainsi les coûts et minimisant la place occupée. Cette solution est spécialement adaptée aux voyages ou aux hôtels, campings, avions, campings cars, boutiques, écoles... Pour ce faire, on peut prévoir que le système de distribution puisse être renseigné sur la personne qui l'utilise, afin d'accéder à des données personnelles préalablement mémorisées.

### Utilisation en continu pour un maquillage en dégradé de matité/brillance

Dans cette application, le système de distribution, pendant qu'il délivre le produit, change la formulation du mélange. De plus, on déplace la sortie des produits de base ou du mélange relativement à un contenant ou un support définissant une surface d'application. Dans un mode particulier de mise en oeuvre de l'invention, le système de distribution est agencé pour calculer l'évolution du mélange en fonction de la matité/brillance C1, d'une zone à traiter et de la matité/brillance C2, d'une autre zone à traiter. Par exemple, sachant que le menton nécessite une matité/brillance C1 et que la joue nécessite une matité/brillance C2, le système de distribution peut faire varier la formulation du mélange pendant qu'il le délivre pour réaliser un dégradé de matité/brillance entre ces deux points. Cela permet par exemple de mieux masquer les imperfections du visage en faisant en sorte que le résultat final soit réaliste, ou de faire des dégradés de matité/brillance à des fins d'embellissement. Le système de distribution peut encore être agencé de telle sorte que l'utilisateur puisse commander une variation de matité/brillance du mélange distribué sans que les matité/brillance de départ et/ou d'arrivée n'aient été préalablement fixées. Pour ce faire, le système de distribution peut disposer d'un système de localisation, ou d'auto-localisation, et déduire d'une table de correspondance les matité/brillance C1 et C2 qu'il doit réaliser, et donc les évolutions de mélange qu'il doit réaliser.

Le système de distribution peut comporter une tête de sortie, en particulier dans le cas d'un aérographe, qui est mobile et pilotée. Cette option permet alors, sans bouger le reste du système de distribution, de réaliser des dégradés. Par exemple, on localise le système de distribution près de la joue, puis on déclenche un système de commande qui va piloter automatiquement la variation de la formulation du mélange et le mouvement de la tête de sortie, de façon à par exemple avoir le centre de la joue plus rouge que sa périphérie, avec un dégradé entre les deux.

Le système de distribution peut encore être utilisé pour réaliser des produits sur mesure, que l'on conserve pour réaliser plusieurs applications.

Il est aussi possible de réaliser des produits solides ou semi-solides.

### Fabrication de compacts « à façon » ou autres produits solides ou semi-solides

Le système de distribution peut être agencé pour permettre de choisir un mélange et de le délivrer dans un contenant tel qu'une coupelle. Le mélange comporte de préférence des composés qui sont tels que le mélange puisse prendre en masse.

Plus préférentiellement, on utilise des composés qui rendent la prise en masse spécialement rapide. Ces composés sont soit déposés dans le contenant avant ou après son remplissage avec les autres constituants, soit sont prévus dans les compartiments du distributeur avec les autres constituants des produits de base, soit sont compris dans le distributeur dans un compartiment spécialement prévu pour les contenir.

On peut ainsi distribuer des compositions spécifiques qui peuvent par réaction chimique, biochimique ou physicochimique, durcir de façon accélérée après évacuation.

Ces compositions sont spécialement adaptées à la réalisation des compacts, c'est-à-dire :
- Prennent en masse,
- donnent un matériau qui peut se déliter en cas de frottement, et sont préférentiellement colorées.

De préférence, ces compositions sont très riches en particules solides, avec par exemple plus de 10% en masse de particules solides par rapport au poids total de la composition, mieux plus de 20% en masse de particules solides par rapport à la masse totale de la composition, encore mieux plus de 30% en masse de particules solides par rapport à la masse totale de la composition, préférentiellement entre 10 et 40% en masse de particules solides par rapport à la masse totale de la composition.

Ces compositions peuvent contenir des particules absorbantes, ou des composés réactifs, tels que ceux qui réagissent en contact avec l'air, par exemple du cyanoacrylate ou des alphasilanes, ou qui réagissent à la lumière, notamment UV.

Le contenant dans lequel le mélange est distribué peut comporter un composé A et les compositions distribuées un composé B, A et B étant choisis pour réagir entre eux et solidifier le mélange.

Dans un mode particulier de mise en oeuvre de l'invention, le système de distribution intègre un moyen de chauffage, par exemple à résistance électrique, pour la réalisation de rouges à lèvres ou autres produits cireux. Dans ce cas, les produits de base sont chauffés avant d'être délivrés.

Le système de distribution peut aussi comporter un moyen pour apporter de l'énergie, thermique et/ou lumineuse, après distribution du mélange dans un contenant, par exemple une résistance électrique ou une diode LED, notamment UV. Cette énergie peut accélérer la prise en masse du mélange distribué.

Préférentiellement, le mélange est homogénéisé avant la prise en masse.

### Réalisation de palettes de matité/brillance

Le système de distribution peut comporter un support, ayant plusieurs régions, et être agencé pour générer automatiquement plusieurs mélanges déposés dans lesdites régions, par exemple une série de matité/brillance adaptées à différentes parties du visage.

Le support peut définir plusieurs cavités pour recevoir les mélanges ou porter plusieurs contenants, par exemple sous forme de coupelles, éventuellement séparables du support.

Dans un cas particulier, le support prend la forme d'un visage avec des régions pour recevoir les mélanges pour des zones d'applications ciblées.

Le support être mobile, notamment rotatif, par rapport au corps du distributeur, et par exemple entraîné en déplacement par le distributeur, pour remplir successivement différents logements ou contenants.

Les produits, sur la palette pourront être de couleurs différentes, tout en présentant des effets de matité/brillance différentes

### Distributeur à coupelle

Il existe un intérêt pour bénéficier d'un système de distribution capable de délivrer un mélange pouvant être facilement prélevé par l'utilisateur. Par ailleurs, dans le cas où les produits de base délivrés par le système de distribution ne sont pas déjà mélangés, il existe un besoin pour permettre à l'utilisateur d'effectuer aisément le mélange.

L'invention a encore pour objet un système de distribution d'au moins un produit de maquillage, comportant une coupelle, et un distributeur pour remplir la coupelle avec au moins un produit, la coupelle étant solidaire du distributeur au moins pendant son remplissage.

La coupelle est parfois encore appelée « creuset » et ce terme doit être compris avec une acceptation large.

Par « coupelle solidaire du distributeur » il faut comprendre que la coupelle est retenue, notamment immobilisée, au moins temporairement sur le distributeur, étant par exemple fixée sur celui-ci par des vis, par aimantation, par encliquetage, par verrouillage baïonnette, par serrage, ou réalisée avec une partie du corps du distributeur par moulage de matière. La coupelle lorsqu'elle est solidaire du distributeur permet de manipuler celui-ci d'une main, la coupelle restant en place sur le distributeur au cours des mouvements de ce dernier.

Le distributeur peut être proposé à l'utilisateur avec la coupelle déjà en place.

En variante, celle-ci est installée par l'utilisateur à la première utilisation du système de distribution.

De préférence la coupelle est moins profonde que large, ce qui facilite l'accès à celle-ci, et permet de prélever le produit, notamment le mélange avec un applicateur ou le doigt.

De préférence, la coupelle est séparable du distributeur, et constitue une interface de sortie qui peut être choisie parmi un ensemble d'interfaces de sortie pouvant être montées sur le distributeur, au choix de l'utilisateur en fonction du maquillage à réaliser, comme détaillé plus loin.

De préférence, le système de distribution comporte plusieurs orifices de remplissage en des produits de base différents, débouchant dans la coupelle. Ainsi, le mélange de ces produits peut avoir lieu dans la coupelle.

La coupelle a de préférence un fond de forme concave vers l'extérieur, ce qui peut faciliter son nettoyage par l'utilisateur entre deux utilisations.

De plus, cela peut faciliter le prélèvement du produit par l'utilisateur, et le mélange des produits de base.

De préférence, le distributeur permet de délivrer au moins deux produits de base dans la coupelle, dans des proportions réglables, et mieux au moins trois produits.

Dans un exemple de mise en oeuvre, le système de distribution comporte au moins deux coupelles pouvant être sélectivement alimentées par le distributeur. Cela peut permettre à l'utilisateur de remplir rapidement ces deux coupelles avec des mélanges dont les caractéristiques sont différentes. Cela peut faciliter les essais de matières colorées, et/ou permettre de préparer plusieurs mélanges de couleurs et matité/brillance différentes destinées au maquillage de zones respectives du visage. Les coupelles peuvent être associées à des identifiants rappelant à l'utilisateur à quelle zone du visage un mélange contenu dans une coupelle donnée est destiné.

Les coupelles peuvent être mobiles relativement au distributeur, étant par exemple portées par un support mobile, tel qu'une tourelle mobile en rotation par rapport au distributeur, ou par un tiroir mobile en translation par rapport au distributeur.

Le système de distribution peut comporter un couvercle de fermeture de la coupelle. Ce couvercle de fermeture est de préférence transparent pour permettre à l'utilisateur de visualiser l'aspect du mélange contenu à l'intérieur.

Lorsque la coupelle est séparable du distributeur, elle peut être introduite le cas échéant dans un boîtier permettant de la transporter plus facilement, ce boîtier pouvant comporter le cas échéant un miroir et/ou un applicateur. Le couvercle du boîtier peut dans ce cas servir de couvercle pour la coupelle.

Le volume de la coupelle peut être compris entre 2 et 1000 mm³, mieux entre 100 et 1000 mm³, encore mieux entre 250 et 750 mm³.

Le ou les produits de base délivrés dans la coupelle sont de préférence des fonds de teint, mais en variante il s'agit de produits de maquillage des lèvres ou des paupières.

La coupelle est de préférence de forme symétrique de révolution. En variante, elle est de contour polygonal ou autre. Son plus grand diamètre intérieur, ou celui du cercle inscrit dans le cas d'un contour non circulaire, est de préférence compris entre 2 et 100 mm, préférentiellement entre 5 et 40 mm. Sa profondeur est de préférence comprise entre 1 et 10 mm, mieux entre 3 et 8 mm. Sa profondeur est de préférence comprise entre 1 et 10 mm. De préférence, la taille et la forme de la coupelle permettent soit une application directe du mélange sur la peau, soit une préhension au doigt ou avec un applicateur. La coupelle peut être réalisée avec un matériau élastiquement déformable, ce qui permet par exemple d'inverser la concavité du fond de la coupelle et de vider celle-ci plus facilement ou de l'utiliser pour appliquer le produit.

La coupelle peut être dépourvue de mélangeur ; dans ce cas, les produits de base peuvent arriver du distributeur dans la coupelle à l'état non mélangé, par des orifices de distribution respectifs distincts. En variante, le distributeur intègre un mélangeur et les produits de base arrivent déjà mélangés dans la coupelle.

La coupelle peut aussi intégrer un mélangeur statique, comme détaillé plus loin, qui est alimenté par des orifices de remplissage distincts du distributeur, et qui délivre de préférence le mélange dans une cavité de la coupelle située au-dessus du mélangeur.

L'invention a encore pour objet un procédé de préparation d'un produit de maquillage, comportant l'étape consistant à remplir une coupelle d'un système de distribution tel que défini plus haut avec au moins un produit de base provenant du distributeur.

Les produits présenteront des effets de matité/brillance différents

Plusieurs produits peuvent être délivrés dans le fond de la coupelle, puis mélangés à l'aide du doigt ou d'un applicateur, ou d'un mélangeur statique intégré à la coupelle.

La coupelle est de préférence remplie par le dessous. Il a été proposé dans le passé des systèmes de distribution à l'aide d'une sonotrode.

La coupelle selon l'invention n'est pas destinée à entrer en vibration pour distribuer le ou les produits amenés par le ou les canaux d'alimentation de celle-ci. Elle est différente d'une sonotrode. De préférence, la coupelle est réalisée en matière plastique.

### Mélangeur intégré à l'interface de sortie

Il existe un intérêt pour bénéficier d'un système de distribution capable de délivrer un mélange pouvant être facilement utilisé, notamment prélevé par l'utilisateur, sans nécessiter une action additionnelle de mélange de la part de celui-ci.

L'invention a encore pour objet un système de distribution comportant un distributeur ayant des canaux de sortie de produits de base et une interface de sortie séparable du distributeur, cette interface ayant un mélangeur statique, délivrant de préférence le mélange dans une cavité où il peut être prélevé.

Le mélangeur statique peut être situé sous la cavité précitée. Le système de distribution est alors particulièrement adapté à la création de compacts, en utilisant comme interfaces de sortie des coupelles à mélangeur statique intégré. Dans ce cas, on vient remplir la cavité de la coupelle en produit par le dessous. Après passage dans le mélangeur statique, les produits de base mélangés viennent recouvrir le mélangeur.

Il est possible d'utiliser plusieurs interfaces de sortie et de les remplir avec des mélanges respectifs différents, sans avoir à purger le mélangeur, ce qui réduit les pertes de produit. L'interface de sortie peut être à usage unique, le cas échéant.

De préférence, le mélangeur statique comporte une chambre centrale communiquant avec des canaux d'admission des produits de base. Cette chambre centrale peut communiquer avec une chambre périphérique comportant une série de cloisons qui agissent comme des déflecteurs pour le mélange, et créent un cisaillement de celui-ci.

La chambre périphérique peut comporter une cloison annulaire ajourée définissant des ajours par lesquels passe le mélange en circulant dans la chambre périphérique. Les chambres centrale et périphérique peuvent être fermées supérieurement par une paroi qui définit le fond de la cavité recevant le mélange.

Le fond de la chambre périphérique peut être de forme hélicoïdale autour de l'axe de la coupelle et de hauteur diminuant au fur et à mesure que l'on progresse vers la sortie. Cette dernière peut déboucher en avant d'une rampe de liaison entre le fond de la chambre périphérique et la paroi supérieure du mélangeur, cette rampe de liaison étant de préférence une portion d'hélice prolongeant l'hélice formée par la paroi de fond de la chambre périphérique.

De préférence la chambre périphérique comporte la cloison annulaire précitée et des cloisons radiales qui forcent le mélange à circuler alternativement entre des régions supérieure et inférieure de la chambre périphérique et entre des régions radialement intérieure et extérieure, le mélange circulant par exemple d'une région supérieure et radialement extérieure à une région inférieure et radialement extérieure en passant à travers la cloison annulaire précitée.

Le mélangeur peut comporter un corps extérieur dans lequel est reçue une pièce formant le coeur du mélangeur, le corps extérieur fermant radialement à l'extérieur la chambre périphérique et comportant un montant qui sépare les chambres centrale et périphérique.

Le corps extérieur du mélangeur et le coeur du mélangeur peuvent être chacun réalisés d'une seule pièce par moulage par injection.

### Volume mort réduit

Il existe un intérêt pour réduire les pertes de produit lors des changements de formulation du mélange, ainsi que pour permettre de faire varier le plus rapidement possible le produit du mélange au cours de l'application, notamment en cas de couplage du distributeur avec un aérographe.

Le produit de base peut quitter la cartouche par un canal de sortie de la cartouche, ce canal de sortie débouchant à l'extérieur du distributeur ou à proximité de sa surface externe.

Le canal de sortie peut notamment déboucher dans une zone de prélèvement du mélange ou à proximité de celle-ci, notamment à moins de 5 mm, mieux à moins de 3 mm, mieux à moins de 1mm, mieux à fleur.

La section transversale du canal de sortie est par exemple comprise entre 1 et3 mm².

Ainsi, chaque produit de base provenant d'une cartouche peut quitter le distributeur sans se mélanger avec un produit de base d'une autre cartouche et l'on minimise le volume mort qui ne peut être prélevé et susceptible d'accroître l'inertie du système. Le produit est plus rapidement disponible sans avoir à circuler dans des canaux spécifiques du boîtier du distributeur, évitant une étape fastidieuse de purge si changement de cartouche.

L'extérieur du distributeur peut être la zone de prélèvement du produit, notamment lorsque le distributeur est réalisé avec une coupelle non prévue pour être enlevée, dans laquelle le mélange est distribué, ou une zone du distributeur destinée au montage d'une interface de sortie amovible, qui définit la zone de prélèvement. Cette interface de sortie peut comporter une coupelle telle que définie ci-dessus. Cette zone de montage correspond par exemple à l'extrémité du boîtier du distributeur en l'absence de l'interface de sortie. La zone de montage peut être sensiblement plane et perpendiculaire à l'axe longitudinal du boîtier du distributeur.

Le distributeur peut comporter trois cartouches de produits de base.

Le distributeur peut comporter des logements pour recevoir les cartouches, lesquelles sont de préférence reçues de façon amovible dans le distributeur. Ce dernier peut comporter des passages pour des conduits des cartouches définissant les canaux de sortie.

La longueur de ces conduits est de préférence telle que les conduits se situent légèrement en retrait du fond ou à affleurement de la cavité servant au prélèvement du produit, ou en variante légèrement en retrait ou à affleurement de la face d'extrémité du boîtier du distributeur définissant la zone de montage.

Ces conduits des cartouches peuvent être des embouts servant à entraîner en déplacement des pistons au sein des cartouches, comme détaillé plus haut.

### Interfaces de sortie multiples

Il existe un besoin pour pouvoir, à l'aide d'un même système de distribution, réaliser facilement des maquillages différents, et pouvoir si on le souhaite maquiller des zones aussi différentes que la peau, les lèvres ou les cils ou sourcils.

Le système de distribution peut comporter un ensemble comportant un distributeur d'au moins un produit cosmétique, notamment de maquillage, et au moins deux interfaces de sortie pouvant se monter chacune de façon amovible sur le distributeur, ces interfaces de sortie permettant de recevoir le ou les produits délivrés par le distributeur, étant de préférence choisies parmi les suivantes :
- interface de sortie comportant un contenant, notamment une coupelle permettant un prélèvement du produit au doigt ou à l'aide d'un applicateur,
- interface de sortie permettant de délivrer le produit à un système de pulvérisation, notamment un aérographe,
- interface de sortie comportant plusieurs régions de réception du produit, mobile par rapport au distributeur,
- interface de sortie permettant de délivrer le produit à un embout de distribution.

De préférence, l'ensemble comporte au moins trois desdites interfaces de sortie, mieux les quatre interfaces de sortie.

Le distributeur peut comporter au moins deux produits de base différents et permettre de délivrer ceux-ci dans des proportions variables, et de préférence, le distributeur comporte trois produits de base différents et permet de délivrer ceux-ci dans des proportions variables.

Chaque interface de sortie peut comporter une base permettant sa fixation sur le distributeur. Cette fixation peut s'effectuer à l'aide de vis par exemple, mais de préférence la base est agencée pour permettre un démontage et un remplacement d'une interface de sortie sans nécessité d'outil. Il s'agit par exemple d'une fixation quart de tour ou à l'aide d'une bague de verrouillage externe.

L'interface de sortie et/ou le boîtier du distributeur peuvent comporter des joints d'étanchéité permettant une communication étanche entre le boîtier du distributeur et l'interface de sortie. Le cas échéant, le distributeur est agencé pour reconnaître l'interface de sortie qui est montée dessus, par exemple grâce à identifiant de l'interface de sortie sous forme de reliefs spécifiques, lesquels sont détectés par le distributeur, ou d'une puce électronique reconnue par le distributeur. Cela peut permettre d'adapter le fonctionnement du distributeur à l'interface de sortie montée dessus. Le distributeur peut communiquer à un système informatique des informations sur l'interface de sortie qu'il porte, et le système informatique peut afficher en fonction de ces informations un écran spécifique et/ou lancer un programme spécifique de commande des paramètres de fonctionnement du distributeur, afin par exemple d'adapter la dose distribuée et/ou le débit à la nature de l'interface de sortie.

Plusieurs interfaces de sortie peuvent être proposées initialement à l'utilisateur avec un distributeur commun au sein d'un même emballage, par exemple un coffret ou une boîte de carton.

L'invention a encore pour objet un procédé de maquillage, comportant l'étape consistant à sélectionner une interface de sortie, à la monter sur le distributeur, et à délivrer le ou les produits contenus dans le distributeur dans celle-ci.

### Cartographie et apprentissage

Le terme « cartographie » s'entend ici comme un procédé d'indexation d'une matité/brillance et d'une zone, avec enregistrement.

Il se peut qu'une personne nécessite plusieurs niveaux de matité/brillance avec une seule couleur et/ou couvrance pour l'ensemble des zones.

La cartographie peut concerner des applications sur des zones plus petites qu'un cm2. Toutefois, l'oeil nu a alors du mal à discerner si le résultat obtenu est adéquat, et il est préférable de substituer l'évaluation à l'oeil nu par une évaluation instrumentée, avec grossissement. L'application de petites quantités de matière colorée peut être faite au doigt, avec des outils classiques tels que des pinceaux, ou avec des applicateurs spécialisés.

La cartographie peut être générée lors d'une période d'apprentissage, pendant laquelle l'utilisateur effectue des tests avec des mélanges sur différentes zones du visage ; une fois renseignée, la cartographie peut ensuite être utilisée pour le maquillage de tous les jours.

Des interfaces graphiques spécifiques peuvent servir pendant la période d'apprentissage et pendant la période d'utilisation de la cartographie.

En particulier, le système de distribution peut être utilisé avec une interface graphique où l'opérateur voit le visage, qui est par exemple schématisé, figuratif ou précis comme une photographie ou une simulation 3D. Dans ce cas, l'opérateur peut pointer une partie du visage sur l'écran pour voir apparaître et/ou délivrer la matité/brillance adéquate.

L'interface graphique peut aussi laisser apparaître les autres zones du visage où l'utilisation de la même matité/brillance est adéquate.

Pour réaliser la cartographie, l'opérateur applique une couleur (avec une matité/brillance donnée) puis réalise l'évaluation.

Les zones du visage peuvent être traitées les unes après les autres ; par exemple, on réalise l'exercice sur une partie de la joue, puis sur le nez, etc....

Une autre possibilité consiste à réaliser un mélange de matité/brillance donnée, et appliquer sur plusieurs zones ce même mélange. L'opérateur doit alors chercher la zone du visage pour laquelle la matité/brillance est adaptée. Le mélange est ensuite indexé dans le système informatique en l'attribuant à la ou aux zones du visage pour lesquelles il convient.

Selon l'un de ses aspects, indépendamment ou en combinaison avec les autres aspects de celle-ci, et notamment ce qui précède, l'invention a pour objet un procédé d'apprentissage d'un système de distribution comportant un distributeur permettant de distribuer un mélange de matité/brillance variable, et un système informatique permettant de sélectionner une matité/brillance et de mémoriser des données, comportant les étapes consistant à :
a) Sélectionner au moins une matité/brillance à l'aide d'une interface du système informatique,
b) délivrer à l'aide du distributeur au moins un mélange de la matité/brillance sélectionnée,
c) évaluer le ou les mélanges distribués après application sur au moins une zone du visage,
d) mémoriser les caractéristiques d'au moins un mélange, notamment un mélange que l'utilisateur souhaite pouvoir rappeler, et d'au moins une zone sur laquelle il a été testé.

Cette mémorisation peut s'effectuer notamment en vue d'une distribution ultérieure de ce mélange pour maquiller ladite zone.

De préférence, le système informatique est agencé pour permettre à l'utilisateur de signaler si le résultat du test est satisfaisant ou non, voire de le renseigner sur la comparaison à un test effectué précédemment.

On peut aussi réaliser un mélange de matité/brillance donnée, et chercher la zone du visage pour laquelle il est adapté. On enregistre alors le mélange en l'attribuant à la ou aux zones du visage pour lesquelles il est adapté, dans une table de correspondance qui servira par la suite, pour, partant d'une zone de la peau, en déduire le mélange à utiliser.

On peut procéder de la même façon avec d'autres mélanges pour réaliser une cartographie de l'ensemble du visage et ainsi avoir une table de correspondance complète du visage.

On peut aussi réaliser un mélange donné, l'appliquer sur une zone donnée, puis faire varier le mélange jusqu'à obtenir le mélange le plus adapté. On enregistre alors le mélange en l'attribuant à la ou aux zones du visage pour lesquelles il convient, dans une table de correspondance qui servira par la suite, pour, partant d'une zone de la peau, en déduire le mélange à utiliser.

De préférence, le système informatique évalue et mémorise les quantités utilisées zone par zone. Un tel procédé, par « touches d'essai », permet d'identifier le ou les produits dont a besoin la personne qui veut se maquiller. Ainsi, le système de distribution peut servir sur les points de vente pour conseiller les personnes qui veulent se maquiller ou à la maison pour bien définir les produits à commander.

L'interface du système informatique comporte de préférence un écran tactile affichant la matité/brillance du mélange lors de sa sélection.

L'interface peut afficher un visage et permettre de renseigner le système informatique en sélectionnant la zone sur le visage affiché.

Le système informatique est de préférence agencé pour permettre d'associer une zone, des paramètres de reconstitution du mélange, et la date de l'essai et/ou tout autre identifiant du mélange.

Le système informatique est de préférence également agencé pour permettre d'associer en outre à ladite zone, aux paramètres de reconstitution du mélange, et à la date ou à l'identifiant du mélange, au moins l'une des données suivantes : le nom de la zone, la période de l'année, le nom d'un évènement, un identifiant de l'utilisateur, l'âge de l'utilisateur.

Les étapes a) à c) peuvent être renouvelées au moins une fois avant la mémorisation des caractéristiques du mélange à l'étape d).

Le système informatique peut être agencé pour rechercher dans une base de données une référence de produit commerciale, sur la base des caractéristiques du mélange identifié comme convenant à au moins une zone donnée, et la communiquer à l'utilisateur.

La sélection à l'étape a) peut s'effectuer avec l'aide d'un système expert externe ou non au système informatique.

Le système expert peut analyser une image de l'utilisateur, pour proposer une matité/brillance de mélange au moins sur la base de l'image analysée.

L'étape a) peut être précédée par la proposition d'une matité/brillance et d'une zone à tester avec un mélange de cette matité/brillance, par le système informatique à l'utilisateur.

Le système informatique peut être agencé pour permettre à l'utilisateur de le renseigner sur l'appréciation du résultat du test de l'étape c) et pour générer une proposition de modification du mélange à sélectionner lors du retour à l'étape a).

Le système informatique peut être agencé pour proposer au moins une matité/brillance de mélange à l'étape a) en fonction d'une zone d'application renseignée par l'utilisateur.

Le système informatique peut être agencé pour proposer au moins une zone d'application à l'étape a), en fonction d'une matité/brillance renseignée par l'utilisateur.

Le distributeur peut délivrer à l'étape b) au moins deux mélanges de matité/brillance différentes, de préférence séparés, en vue de leur application simultanée sur la zone de test.

Cela peut faire gagner du temps, et faciliter la comparaison des résultats.

L'invention a encore pour objet un procédé de maquillage à l'aide d'un système de distribution selon cet aspect de l'invention, dans lequel :
a) L'utilisateur adresse une requête concernant un besoin de maquillage au système informatique,
b) celui-ci génère en retour une proposition de matité/brillance en vue du maquillage d'une zone associée, sur la base de l'apprentissage effectué préalablement, et
c) le système informatique pilote le distributeur pour la production du mélange de la matité/brillance proposée, notamment si celle-ci est validée par l'utilisateur.

Un tel procédé peut utiliser une cartographie précédemment établie avec l'utilisateur.

L'invention a encore pour objet un produit programme d'ordinateur comportant des instructions de code permettant, lorsque exécutées dans un système informatique, d'amener le système informatique à :
- Permettre à l'utilisateur de sélectionner au moins une matité/brillance et/ou une zone d'application, notamment à l'aide d'une interface telle qu'un écran tactile,
- piloter un distributeur de façon à délivrer un mélange de la matité/brillance sélectionnée par l'utilisateur,
- permettre à l'utilisateur de déclencher la mémorisation de la matité/brillance du mélange et d'une zone d'application associée, notamment en vue d'une distribution ultérieure du même mélange, notamment sur la même zone.

Le produit programme d'ordinateur peut comporter des instructions de code permettant, lorsque exécutées dans un système informatique, d'amener le système informatique à :
- Recevoir une requête de l'utilisateur concernant un besoin de maquillage, notamment à l'aide d'une interface telle qu'un écran tactile,
- proposer sur la base au moins de données générées par le procédé d'apprentissage tel que défini ci-dessus, au moins une matité/brillance et/ou une zone d'application,
- piloter un distributeur pour la production du mélange de la matité/brillance proposée, notamment si celle-ci est validée par l'utilisateur.

La personne peut intégrer dans la cartographie le niveau de matité/brillance qu'elle désire pour chaque zone du visage, zone par zone ou ensemble de zones par ensemble de zones. Le système interprétera alors les mélanges qu'il doit réaliser pour assurer les couleurs, couvrance désirées et la matité/brillance voulu.

La cartographie peut être modifiée par la suite. Ainsi, il est possible de réaliser la cartographie sur le plan des matité/brillances dans un premier temps, puis sophistiquer la cartographie en définissant par la suite les niveaux de couleur et couvrance.

### Aide à distance

Il est souhaitable de pouvoir aider l'utilisateur à se maquiller, notamment à choisir les bonnes matité/brillance.

L'invention, selon l'un de ses aspects, indépendamment ou en combinaison avec ses autres aspects, notamment ce qui précède, a ainsi pour objet un procédé de maquillage comportant les étapes consistant à :
- permettre l'établissement d'une liaison vidéo, par exemple via internet, entre une caméra d'un premier site et un deuxième site,
- permettre au deuxième site de piloter directement ou indirectement un distributeur présent sur le premier site, ce distributeur permettant de faire varier matité/brillance d'un mélange distribué,
- permettre à une personne présente au premier site d'appliquer le mélange distribué et d'envoyer vers le deuxième site une image correspondante, pour recevoir en retour une information relative au résultat de maquillage.

Le deuxième site peut notamment comporter un écran de visualisation qui permet à un conseiller présent devant cet écran de voir le résultat du maquillage avec le produit distribué par le distributeur et de conseiller la personne qui s'est maquillée. Ce conseiller peut agir en retour sur le distributeur pour modifier la matité/brillance du mélange et l'adapter au mieux au visage de la personne présente sur le premier site. Ainsi, cette personne gouverne le mélange délivré par le distributeur. La première personne peut se maquiller sous les yeux de la seconde. La seconde personne voit sur son écran le résultat de l'essai, et ainsi peut corriger le mélange qu'elle va commander à distance, jusqu'à obtenir le maquillage idéal.

Le cas échéant, l'acquisition vidéo peut être calibrée à l'aide d'une mire, ou avec le mélange distribué par le distributeur sur une surface de référence. Cela permet ensuite une visualisation plus fidèle du maquillage effectué sur le premier site.

De préférence, la liaison vidéo entre les deux sites est une liaison duplex.

Le premier site peut recevoir un tutoriel du deuxième site, le cas échéant.

Des identifiants des produits de base peuvent être communiqués au deuxième site ; cela peut permettre de connaître précisément la couleur de chacun des produits de base.

Le procédé peut comporter la mémorisation des paramètres de réglage du distributeur, une fois qu'un mélange donné est estimé satisfaisant. De préférence, cette mémorisation peut être commandée depuis le deuxième site. La mémorisation peut avoir lieu dans le système informatique présent sur le premier site et/ou sur un serveur externe.

Une variante peut consister à faire travailler une personne pour aider le maquillage de plusieurs personnes. Ce mode de mise en oeuvre permet le développement des « Make-Up Artists » et leur travail soit au sein d'un institut, soit via internet. Cela permet aussi le maquillage de personnes aux moyens limités, telles que les personnes malvoyantes, ou discernant mal les couleurs, ou âgées, ou manquant de confiance en elles.

### Pilotage par interface tactile

Il existe un besoin pour faciliter le pilotage du système de distribution et notamment le choix de la matité/brillance du mélange distribué.

Selon un autre de ses aspects, indépendamment ou en combinaison avec ses autres aspects, et notamment avec ce qui précède, l'invention a ainsi pour objet un système de distribution comportant un distributeur et un système informatique de pilotage du distributeur, ce système informatique comportant un écran tactile sur lequel peut être affichée la matité/brillance du mélange et un moyen de sélection déplaçable sur l'écran, pour faire varier la matité/brillance du mélange distribué.

De préférence, l'écran affiche des matité/brillances extrêmes entre lesquelles la matité/brillance du mélange peut être sélectionnée, en déplaçant le moyen de sélection entre ces matité/brillances extrêmes.

L'écran peut afficher une échelle de matité/brillances entre au moins deux matité/brillances, ou une surface, notamment de contour triangulaire, au sein de laquelle le moyen de sélection peut se déplacer. Cette surface peut localement faire apparaître la matité/brillance du mélange en fonction par exemple de la distance à chacun des sommets, matérialisant chacun un produit de base pur.

Le système informatique peut effectuer une partie des calculs nécessaires pour déterminer les fractions de chacun des produits de base conduisant à un mélange de la matité/brillance recherchée.

Le système informatique peut être un téléphone intelligent, un photophone, une tablette, un ordinateur individuel. En variante, il est intégré au boîtier du distributeur.

Le système informatique peut disposer d'une caméra. Cette dernière peut être utilisée notamment pour l'acquisition d'une image de l'utilisateur et/ou du mélange.

Le système informatique peut être agencé pour afficher une image d'un visage, afin de faciliter le repérage des zones sur lesquelles le mélange doit être appliqué.

### Couplage du système de distribution à un moyen de pulvérisation

Le système de distribution peut comporter ou être connecté à un moyen de pulvérisation du mélange, de préférence un aérographe.

L'invention vise, selon un autre de ses aspects, à perfectionner encore les systèmes de distribution comportant un système de pulvérisation, de préférence un aérographe, et a pour objet, selon l'un de ses aspects, indépendamment ou en combinaison avec les autres aspects, et notamment ce qui précède, un ensemble comportant :
- Un moyen de pulvérisation, de préférence un aérographe comportant une chambre de prélèvement soumise à un courant d'air d'entraînement,
- un distributeur comportant au moins deux compartiments contenant des produits de base différents, les produits étant délivrés au moyen de pulvérisation de préférence par des orifices de distribution distincts.

Le distributeur peut comporter trois cartouches contenant des produits de maquillage de matité/brillances différentes.

L'aérographe peut comporter un stylet définissant la chambre de prélèvement, le stylet se fixant sur le distributeur ou sur une interface de sortie fixée au distributeur, ou faisant partie intégrante de cette interface de sortie.

Le système de distribution peut comporter un circuit de contrôle du fonctionnement du distributeur, permettant de faire varier la proportion des produits de base délivrés dans la chambre de prélèvement, au cours du fonctionnement de l'aérographe. Les proportions peuvent être modifiées en fonction du déplacement de l'aérographe relativement à la surface sur laquelle le mélange est pulvérisé. Ce déplacement peut être mécanisé, le cas échéant.

Ce circuit de contrôle peut comporter ou être constitué par un système informatique tel que défini plus haut.

Le boîtier du distributeur peut servir de poignée lors de la manipulation d l'ensemble pour délivrer le mélange.

Le distributeur peut comporter une caméra et/ou un ou plusieurs capteurs tels que des accéléromètres afin de localiser automatiquement la zone sur laquelle le mélange est appliquée, et pouvoir régler automatiquement la matité/brillance en fonction de la position, le cas échéant.

L'invention a encore pour objet un procédé de maquillage à l'aide d'un ensemble tel que défini ci-dessus, dans lequel un mélange est pulvérisé sur la peau à l'aide du moyen de pulvérisation, notamment de l'aérographe.

La composition du mélange peut être modifiée au cours du déplacement de l'aérographe relativement à la peau. Un dégradé peut être réalisé.

Cet aspect de l'invention repose sur la constatation que le distributeur peut être utilisé pour alimenter le système de pulvérisation, notamment l'aérographe, tout en permettant au système de distribution d'être suffisamment réactif pour autoriser un changement de la matité/brillance du mélange distribué en cours de maquillage du visage, notamment lorsque la zone à maquiller change.

Il peut être avantageux que la distribution des produits se fasse de façon itérative, notamment avec des temps de distribution non déphasés entre les différents produits.

Cela peut rendre plus facile de faire varier la composition du mélange distribué au cours du temps.

Le mélange peut être réalisé directement dans l'aérographe, sans pratiquement aucun volume mort gênant, permettant ainsi de changer le mélange pulvérisé en temps réel.

La dépression créée dans la chambre de prélèvement est suffisante pour entraîner les produits de base, sans pour autant gêner le dosage.

La dépression qui règne dans la chambre de prélèvement est par exemple comprise entre 10 mbar et 200 mbar, mieux entre 50 et 150 mbar, encore mieux entre 75 et 125 mbar.

La viscosité des produits de base mesurée à 1 atm et à 25°C avec un viscosimètre rotatif CONTRAVES TV équipé d'un mobile MS-r3 ou MS-r4 à la fréquence de 60 Hz, après 10 minutes de rotation du mobile, est par exemple comprise entre 0,05 Pa.S et 50 Pa.S

La section des canaux d'arrivée des produits de base dans la chambre est par exemple comprise entre 1 et 3 mm², mieux entre 2 et 3 mm².

L'alimentation en produit s'effectue de préférence de façon continue.

Il est encore possible d'appliquer simultanément des produits de base non miscibles ou réactifs, comme un gel aqueux et un gel huileux, qui vont se déposer de façon pixellisée directement sur la peau, produisant une sorte de gel/gel in situ, des silicones réactives, ou des produits de coloration réagissant ensemble. Les ratios en produits de base peuvent être ajustés en fonction du résultat particulier souhaité. Par exemple, dans le cas des gels aqueux et gels huileux, on pourra faire varier le ratio correspondant au volume du premier produit de base sur le volume du deuxième produit de base entre 10/1 et 1/10, mieux entre 5/1 et 1/5.

### Système de localisation ou d'auto-localisation

Le système de distribution selon l'invention peut disposer d'un système de localisation ou d'auto-localisation.

On appelle système de localisation, un moyen de saisir, de la part de la personne, la zone qu'elle va traiter. Ceci peut être réalisé notamment par des systèmes laissant libre au moins une main. Ainsi, on peut utiliser une interface d'un système informatique telle qu'un écran tactile, un joystick, ou un système de reconnaissance vocale.

On appelle système d'auto-localisation, un moyen de saisir, sans que la personne n'intervienne, la zone à traiter. Ceci peut être réalisé par un ou plusieurs accéléromètres qui déduisent, par le fait des mouvements, les directions que la personne vise ou par une caméra et un système de reconnaissance d'image.

Le système de distribution peut être tel que celui illustré sur les figures décrites précédemmentLes figures ne seront donc pas toutes décrites à nouveau.

On a illustré à la figure 29C un support ayant des logements disposés sur celuici sensiblement comme les différentes zones d'un visage ; chaque logement peut contenir un mélange dont la matité/brillance est adaptée à la partie correspondante du visage. Ainsi, il est facile pour l'utilisateur de savoir où appliquer le mélange prélevé dans un logement donné.

On peut utiliser le distributeur 11 pour délivrer un mélange dont la formulation change au cours du temps et recueillir le mélange dans un contenant mobile par rapport au distributeur, de telle sorte que le mélange soit déposé en un emplacement du contenant qui varie dans le temps, afin de réaliser un dégradé.

Par exemple, comme illustré aux figures 29A et 29B, le système de distribution comporte une interface de sortie 110 comportant une partie fixe relativement au distributeur et une partie mobile 252 présentant un logement 253 pour recevoir le mélange.

Par exemple, le distributeur 11 est disposé dans ce cas avec les orifices de sortie des cartouches vers le bas, et équipé d'un mélangeur, de telle sorte que le mélange tombe sous l'effet de son poids dans le logement 253. Un moteur peut déplacer la partie mobile de l'interface de sortie relativement au distributeur, de façon synchronisée avec la variation des caractéristiques du mélange, de telle sorte que l'on obtienne un dégradé le long dulogement 253, comme illustré à la figure 29B.

Comme indiqué précédemment, le système de distribution 10 selon l'invention comporte de préférence une interface homme machine qui permet à l'utilisateur de piloterfacilement et de façon intuitive le distributeur 11. Cette interface peut appartenir à un système informatique 100 qui communique avec le distributeur 11.

On a représenté sur les figures 31 à 37 différents exemples d'interfaces tactiles pouvant permettre à l'utilisateur de choisir la matité/brillance du mélange résultant de la distribution dosée des différents produits de base.

Cette interface peut présenter, comme illustré à la figure 31, une zone de sélection de la matité/brillance, par exemple sous la forme d'un triangle dont les sommetscorrespondent aux matité/brillances de chacun des produits de base contenus dans les cartouches.

L'utilisateur peut déplacer un curseur 300, par exemple sous la forme d'une balle, relativement aux sommets A, B et C du triangle.

Plus il rapproche le curseur 300 de l'un des sommets, plus la fraction du produit de base correspondant est grande par rapport à la quantité totale des différents produits distribués.

La fraction de chaque produit par rapport à la quantité totale peut être indiquée en 301 par une valeur numérique sur l'interface.

L'interface peut permettre à l'utilisateur d'incrémenter ou de diminuer la quantité de chacun des produits, en agissant par exemple sur des boutons de commande 302, lesquels permettent un réglage précis de la quantité de chacun des produits de base.

La surface du triangle 310 peut avoir une matité/brillance qui varie localement de façon à être représentative en chaque point de la matité/brillance du mélange résultant de la pondération des différents produits de base dans des proportions correspondant aux coordonnées relatives en ce point.

L'interface peut comporter un bouton 305 permettant d'accéder à un menu spécifique de réglage du volume de produit qui est distribué pour purger le distributeur.

L'interface peut aussi avantageusement permettre un réglage du débit de produit grâce à des boutons 304 et 306 renvoyant vers un menu spécifique de réglage du débit.

Dans l'exemple considéré, l'interface offre le choix entre un mode de distribution continu, grâce au bouton 304, où les produits sont distribués tant que l'utilisateur appuie sur le bouton de commande 12.

La dose correspondante peut être transmise à l'interface et affichée.

Le bouton 306 permet de sélectionner un fonctionnement en mode dose, au cours duquel un appui même bref sur le bouton 12 déclenche la distribution d'une dose prédéfinie.

Pour faire varier le débit, le distributeur agit par exemple sur le rapport cyclique de fonctionnement des moteurs.

L'interface peut être agencée pour permettre à l'utilisateur de programmer ou de mémoriser les réglages qui ont sa préférence, grâce à un menu 307 d'accès à des favoris.

L'interface tactile illustrée à la figure 32 fait apparaître sur l'écran trois zones colorées 400, correspondant chacune à la matité/brillance de l'un des produits de base contenus dans le distributeur 10, et une zone centrale 410 faisant apparaître la matité/brillance du mélange résultant.

La quantité relative de chacun des produits de base peut être ajustée à l'aide de curseurs 415 qui se déplacent par exemple sur des lignes joignant chacune des zones 400 à la zone centrale 410.

Au cours de l'utilisation de l'interface, celle-ci peut mémoriser un réglage donné et faire apparaître sur l'écran un bouton 420 de la matité/brillance du mélange. L'utilisateur peut ensuite, en appuyant simplement sur ce bouton 420, distribuer un mélange de la matité/brillance correspondante.

Dans l'exemple de la figure 34, l'interface affiche dans une zone 500 une teintebdonnée, et propose à l'utilisateur, grâce à des boutons de commande 510 chacun de la matité/brillance du produit de base correspondant, d'accroître ou de diminuer la proportion de ce produit de base dans le mélange final. La matité/brillance de la zone 500 est recalculée en fonction des actions sur les boutons de commande 510.

Dans la variante de la figure 35, l'interface fait apparaître un nuancier présentant plusieurs zones 530 correspondants chacune à une proportion particulière des différents produits de base.

L'utilisateur peut sélectionner l'une de ces zones, par exemple en appuyant dessus avec son doigt.

L'interface peut être agencée pour afficher à une échelle plus grande dans une zone 535 la matité/brillance sélectionnée. La programmation du distributeur 11 pour distribuer cette matité/brillance est par exemple déclenchée en appuyant sur la zone.

Dans l'exemple de la figure 36, l'utilisateur peut déplacer sur un nuancier continu 550 un curseur 555, qui fait apparaître dans une zone 558 la matité/brillance sélectionnée.

L'utilisateur peut ensuite, en appuyant par exemple dans la zone 556, déclencher l'envoi au distributeur 11 des instructions nécessaires pour que celui-ci distribue un produit de la matité/brillance sélectionnée.

On voit sur la figure 37 que l'interface peut mémoriser les différentes teintes sélectionnées, et les faire ensuite apparaître sur l'écran de façon à permettre à l'utilisateur, en appuyant sur des boutons correspondants 560, de sélectionner à nouveau très facilement une teinte précédemment choisie.

On a représenté à la figure 38 un exemple d'interface utilisateur 1000 d'un système de distribution comportant un distributeur, de préférence tel que décrit précédemment, et un système informatique 100 auquel appartient l'interface.

Le système informatique comporte ici par exemple un appareil tel qu'un ordinateur portable, une tablette ou un téléphone intelligent, fonctionnant de façon autonome ou connecté à un serveur distant.

Dans l'exemple considéré, l'interface 1000 est définie par l'écran tactile d'un tel appareil. Dans une variante non illustrée, le distributeur intègre un écran tactile ou tout autre type d'interface homme machine, et peut être utilisé sans connexion à un autre appareil.

L'appareil exécute une application, qui a par exemple été téléchargée au préalable, et qui affiche sur l'écran un visage 1035 et une série de boutons permettant à l'utilisateur d'entrer des informations.

Le visage peut comporter plusieurs zones Zl à Z6 sélectionnables tactilement, par exemple le front, le nez, les joues, les paupières, le menton, et les lèvres.

Les boutons présents sur l'écran peuvent permettre par exemple d'entrer le nom du maquillage ou de l'utilisateur, d'afficher la zone sélectionnée, de choisir la matité/brillance, et d'informer le système informatique du résultat acceptable ou non du test effectué, voire comme illustré de donner une information concernant l'évaluation du résultat relativement à un test effectué précédemment, à savoir par exemple mieux ou « moins bien ». L'écran peut également afficher un bouton permettant de mémoriser le choix d'une matité/brillance et d'une zone, après avoir effectué un essai avec cette matité/brillance sur la zone en question.

Le choix de la matité/brillance s'effectue par exemple avec une échelle de matité/brillances similaire à celle décrite en référence à la figure 36.

Le système informatique est agencé pour mémoriser les données sous forme de table de correspondance par exemple, de manière à associer à une zone du visage des paramètres permettant de reproduire le mélange distribué lors du test. Ces paramètres comportent par exemple les teneurs relatives de chacun des produits de base du distributeur dans le mélange, la quantité Q distribuée, ainsi que des données additionnelles telles que par exemple le nom de la zone, la date de distribution du mélange et/ou tout autre identifiant du mélange, les identifiants des produits de base, la période de l'année, notamment la saison, l'âge de l'utilisateur, son sexe, son nom ou prénom, le nom d'un évènement associé au maquillage, par exemple un anniversaire, entre autres données, la quantité de produit adéquate pour la zone. Les données annexes peuvent permettre à l'utilisateur de reproduire plus facilement un maquillage qu'il a considéré comme adéquat pour une période de l'année, ou rappelant un évènement de la vie, ou pour donner un effet de rajeunissement.

Ces données peuvent être mémorisées dans le système informatique 100, par exemple dans l'appareil précité et/ou sur un serveur distant avec lequel l'appareil communique, ou encore dans une mémoire électronique intégrée au distributeur 11.

Ainsi, selon l'invention, l'utilisateur peut amener le distributeur à délivrer une première matière colorée, à l'appliquer sur une première zone du visage, puis la juger adéquate ou non. Si le résultat est satisfaisant, l'utilisateur peut l'enregistrer en l'indexant à la zone ; si le résultat est non satisfaisant, l'utilisateur peut commander une nouvelle matité/brillance pour refaire les opérations ci-dessus.

Le système informatique peut être utilisé dans ce contexte de diverses façons.

Par exemple, comme illustré à la figure 40, l'utilisateur choisi à une étape 1010 une matité/brillance à essayer, en utilisant par exemple l'échelle de matité/brillances 1011 apparaissant sur l'écran, en déplaçant le bouton de réglage 1012.

Ensuite, le choix de la matité/brillance est transmis au distributeur 11, à l'étape 1015.

Par exemple, l'appareil transmet les quantités à distribuer de chacun des produits de base, et le circuit électronique 81 se charge de commander les moteurs en conséquence.

A l'étape 1016, l'utilisateur appuie sur le bouton de commande 12 du distributeur 11, ce qui provoque par exemple la distribution d'une dose du mélange, de la matité/brillance sélectionnée par l'utilisateur.

Le mélange est par exemple distribué dans la coupelle 115, puis prélevé et appliqué par l'utilisateur sur les joues ou toute autre zone renseignée sur l'interface, à l'étape 1020.

Dans des variantes, le produit est appliqué à l'aide d'un aérographe ou par tout autre moyen tel que décrit précédemment.

L'utilisateur renseigne ensuite à l'étape 1022 le système informatique à l'aide des boutons 1021, sur le résultat.

Si l'utilisateur indique que le résultat est satisfaisant, le système lui propose par exemple de valider à l'aide d'un bouton les paramètres de l'essai afin de les mémoriser à l'étape 1031.

Si le résultat n'est pas jugé satisfaisant par l'utilisateur et signalé comme tel à l'aide du bouton 1032, le résultat peut néanmoins être automatiquement sauvegardé à l'étape 1034.

On peut ainsi indexer à chaque zone non seulement la ou les matité/brillances adéquates mais aussi la ou les matité/brillances qui ne vont pas du tout à cette zone.

L'utilisateur peut alors effectuer un nouvel essai sur la même zone en revenant à l'étape 1010.

Si l'utilisateur était satisfait du résultat, il peut également souhaiter effectuer un nouvel essai, par exemple sur une zone différente du visage.

Le cas échéant, si l'utilisateur n'est pas satisfait, l'interface peut lui proposer d'indiquer si le résultat est estimé mieux ou moins bien que l'essai précédent, à l'aide de boutons correspondants 1040 et 1041.

Dans ce cas, le système informatique peut être agencé pour déterminer si au vu des renseignements entrés par l'utilisateur une proposition peut être automatiquement effectuée quant au choix de la nouvelle matité/brillance à tester.

Le cas échéant, un questionnaire peut s'afficher pour aider le système informatique à proposer une matité/brillance au vu des essais effectués et de l'évaluation qui en a été faite par l'utilisateur ou par un professionnel qui l'assiste.

Par exemple, si la matité/brillance est jugée comme « non adéquate », le système peut recevoir de l'utilisateur une information additionnelle, par exemple « trop mat », « trop brillant » qui l'aidera à proposer une nouvelle matité/brillance plus en adéquation avec ce qu'attend l'utilisateur.

Il peut être intéressant que le système informatique puisse recevoir une information de comparaison du résultat par rapports aux essais précédents, par exemple « c'est mieux » ou « c'est moins bien » et que de là, le système soit capable d'en déduire la nouvelle matité/brillance à proposer.

Une autre option est que le système informatique puisse recevoir une information de comparaison par rapport à une cible, par exemple « c'est presqu'idéal » et que de là, le système soit capable d'ajuster automatiquement ses modifications de matité/brillance.

En l'occurrence, s'il reçoit l'information selon laquelle le résultat souhaité est presque atteint, le système peut adopter de petits niveaux de modification de la matité/brillance et revoir l'échelle de réflexion de réglage en conséquence.

Dans le cas où le système de distribution propose de lui-même les mélanges colorés à tester, il peut se fonder sur des scénarios d'essais préprogrammés et modifier le suivi du scénario selon les réussites ou échecs de l'évaluation. Ainsi, par exemple, si dès la troisième application de produit, il reçoit une information selon laquelle la matité/brillance est presqu'idéale pour l'utilisateur, le système de distribution peut sortir du programme et se laisser guider ensuite par les instructions de l'opérateur.

D'une façon générale, l'utilisateur peut être aidé par un système expert dans le choix des matité/brillances à essayer.

Ce système expert est par exemple un programme exécuté sur l'appareil avec lequel le distributeur communique ou sur le distributeur lui-même, qui se fonde sur des réponses à un questionnaire et/ou sur des mesures, par exemple de la matité/brillance de la peau, effectuées par un capteur spécifique ou une caméra. L'utilisateur peut ainsi se faire aider par une évaluation instrumentée, par exemple un capteur de matité/brillance. Le système expert peut encore être implémenté sur un serveur distant avec lequel l'appareil ou le distributeur échange des informations. L'opérateur peut encore envoyer une image de son visage à un spécialiste qui peut préprogrammer le choix des matité/brillances de départ. Dans un autre exemple de mise en oeuvre, l'utilisateur présente au système informatique une photo de son visage,
et le système informatique est agencé pour l'analyser et créer un programme définissant les zones à tester et les premiers produits à délivrer, à la fois en matité/brillance et quantité. Par exemple, le système informatique peut être agencé pour sélectionner automatiquement des matité/brillances de maquillage à proposer à l'utilisateur en effectuant l'acquisition d'une photographie à l'étape 1070, comme illustré à la figure 42. Par exemple, l'appareil qui communique avec le distributeur 11 est équipé d'une caméra, et l'utilisateur prend une photographie de son visage. L'image est ensuite analysée à l'étape 1071, et des matité/brillances sont proposées pour chaque zone du visage à l'étape 1072, selon par exemple des règles prédéfinies d'accord des matité/brillances.

Le système de distribution peut être orienté par l'utilisateur pour décider la matité/brillance mais aussi la quantité de produit à délivrer. Par exemple, l'utilisateur peut indiquer « nez » ou « tache » et le système de distribution est agencé pour adapter la dose distribuée selon une cartographie en mémoire des doses à distribuer en fonction des zones à traiter.

Le système informatique peut guider l'utilisateur dans le choix des matité/brillances du mélange à essayer, afin de limiter le nombre d'essais nécessaires jusqu'à l'obtention d'un résultat plaisant pour l'utilisateur.

Il est ainsi possible, comme illustré à la figure 41, qu'après l'application d'un mélange délivré par le distributeur sur une zone donnée du visage à l'étape 1060, le système informatique demande à l'utilisateur si le résultat est satisfaisant ou non, et effectue de lui-même, si le résultat est considéré comme étant non satisfaisant, un changement 1061 des paramètres du distributeur de façon à modifier le mélange distribué.

L'utilisateur n'a alors qu'a effectuer un nouvel essai avec le mélange modifié.

Lorsqu'un mélange est signalé comme étant satisfaisant, le système informatique peut mémoriser les paramètres correspondants afin de permettre de recréerultérieurement le mélange.

Le système peut alors recommencer les étapes précédentes pour une nouvelle zone d'application.

Lors des essais successifs, l'opérateur n'a pas besoin de traiter tout le visage. Il peut par exemple choisir entre 3 et 8 zones de petites surfaces, par exemple 5 zones. Le système de distribution est alors avantageusement prévu pour interpoler et/ou extrapoler les données concernant les matité/brillances considérées comme adéquates, afin de calculer les matité/brillances qui devraient être considérées comme adéquates sur des zones sur lesquelles l'exercice n'a pas été fait.

A la fin de l'apprentissage, le système peut généner une visualisation des matité/brillances adéquates sur les différentes zones, testées ou calculées.

Le système de distribution peut être agencé pour signaler si certaines matité/brillances apparaissent aberrantes, se fiant en cela à une comparaison par rapport à des cartographies types en mémoire. Ainsi, il peut proposer à l'utilisateur de refaire tout ou partie de l'exercice de cartographie.

Une fois l'apprentissage du système informatique effectué, c'est-à-dire que des matité/brillances de mélanges ont été identifiées comme convenant à l'utilisateur pour maquiller certaines zones, l'utilisateur qui souhaite se maquiller n'a qu'à rappeler la zone à maquiller, à l'étape 1080 de la figure 43, et le système peut proposer automatiquement à l'utilisateur à l'étape 1081 une matité/brillance de mélange adéquate.

Dans la variante illustrée à la figure 44, l'utilisateur sélectionne une matité/brillance à l'étape 1090 et le système informatique propose à l'étape 1091 une zone où appliquer un mélange de cette matité/brillance, sur la base d'informations précédemment collectées sur la base des tests effectués.

La zone proposée est par exemple celle où une matité/brillance identique ou proche a déjà été appliquée et le résultat jugé acceptable par l'utilisateur.

La figure 46 illustre un exemple de mise en oeuvre de l'invention où après avoir effectué des tests sur différentes zones à l'étape 2010, l'utilisateur signale au système le ou les mélanges qu'il estime procurer le meilleur résultat, ce qui permet au système de connaître les paramètres correspondants à l'étape 2012. Ensuite, à l'étape 2014, le système peut proposer à l'utilisateur des références de produits commerciaux ayant les mêmes propriétés ou des propriétés proches.

Dans une variante, le système adresse à un centre de fabrication distant les paramètres permettant de produire une composition de même formulation ou ayant les mêmes propriétés que celle du mélange que l'utilisateur a testé et trouvé satisfaisant.

La figure 45 illustre la possibilité de délivrer à l'aide du distributeur plusieurs doses 2020a à 2020d de mélanges différents, de façon juxtaposée sur un support 2021, de façon à permettre leur application sur des régions distinctes voisines d'une même zone.

L'utilisateur peut d'un seul coup appliquer une série de matité/brillances afin de cerner rapidement la matité/brillance adéquate. Les matières colorées présentes sur le support 2021 peuvent avoir été choisies par l'opérateur lui-même ou proposées par le système de distribution.

Le support 2021 est par exemple mobile par rapport au boîtier du distributeur et déplacé séquentiellement pour déposer les mélanges correspondants dans les différenteszones 2020a à 2020d, étant par exemple similaire aux supports décrits en référence aux figures 29 ou 29A. L'utilisateur peut alors facilement comparer les résultats entre les différentes régions, et signaler au système le mélange produisant le meilleur effet.

On a illustré à la figure 47 un système qui permet d'aider l'utilisateur à se maquiller, notamment à choisir les bons coloris.

Ce système permet l'établissement d'une liaison vidéo, par exemple via internet, entre une caméra 2060 d'un premier site 2061 et un deuxième site 2062.

La caméra 2060 est par exemple intégrée à une tablette ou un téléphone intelligent qui constitue le système informatique 100.

On permet au deuxième site 2062 de piloter directement ou indirectement le distributeur 11 présent sur le premier site 2061.

Ainsi, la personne présente au premier site peut appliquer le mélange distribué et envoyer vers le deuxième site 2062 une image correspondante, pour recevoir en retour une information relative au résultat de maquillage.

Le deuxième site 2062 peut comporter un écran de visualisation 2064 qui permet à un conseiller présent devant cet écran de voir le résultat du maquillage avec le mélange distribué par le distributeur et de conseiller la personne qui s'est maquillée. Ce conseiller peut agir en retour sur le distributeur 11 pour modifier la matité/brillance du mélange et l'adapter au mieux au visage de la personne présente sur le premier site. Le protocole d'échange des données entre les deux sites permet ainsi l'envoi d'instructions de commande au distributeur 11, soit directement, soit par l'intermédiaire du systèmeinformatique 100 présent sur le premier site. Ainsi, la personne présente sur le deuxième site gouverne le mélange délivré par le distributeur 11. La première personne peut se maquiller sous les yeux de la seconde. La seconde personne voit sur son écran le résultat de l'essai, et ainsi peut corriger le mélange qu'elle va commander à distance, jusqu'à obtenir le maquillage idéal.

De préférence, la liaison vidéo entre les deux sites est une liaison duplex, de sorte que l'utilisateur présent sur le premier site peut voir sur l'écran du système informatique l'image du conseiller. Ce dernier peut adresser à l'utilisateur présent sur le premier site un tutoriel, le cas échéant.

La mémorisation des paramètres de réglage du distributeur 11, une fois qu'un mélange donné est estimé satisfaisant, peut être commandée depuis le deuxième site.

Avantageuseusement, le système de distribution 10 est agencé pour pouvoir modifier de façon esthétique l'ensemble des matité/brillances pour chaque zone si la personne désire changer la matité/brillance de son visage. Le système de distribution peut être agencé de telle sorte qu'il suffit à l'utilisateur de modifier une seule matité/brillance dans une zone pour qu'il modifie toutes les autres. Le système de distribution peut utiliser pour cela des translations, par exemple en relevant la saturation des matité/brillances ou en décalant la teinte.

Le système de distribution peut être agencé pour recevoir la cartographie de quelqu'un d'autre, réel ou virtuel. Il peut aussi combiner la cartographie de la personne avec la cartogaphie d'une autre, pour sublimer le maquillage sans perdre les caractéristiques propres.

L'interface peut servir à définir des programmes de maquillage où l'on définit l'ordre des zones à maquiller ou l'ordre des matité/brillances à proposer.

### Exemples

On réalise un distributeur 11 tel que celui illustré à la figure 3 et précédemment décrit. Le distributeur est agencé pour communiquer avec une tablette 100 telle qu'un Ipad. Ce système informatique exécute une application dénommée « µMix » développée dans l'environnement spécifique d'Apple (XCode 4 et Simulateur iOS) en langage Objective C.

Elle utilise des frameworks de base Foundation, UIKit et CoreGraphics, fournissant les outils de manipulation des structures de données, des outils de calcul et les fonctionnalités liées à l'interface graphique utilisateur.

L'application utilise également le framework CoreBluetooth fournissant un accès aux périphériques Bluetoth 4 Low Energy avec les tâches principales suivantes :
- Recherche des périphériques Bluetooth 4.0 Low Energy,
- Connexion/déconnexion et gestion des paramètres de connexion,
- Communication en mode lecture et/ou écriture basée sur l'architecture GATT (Generic Attribute Profile).

L'application propose les fonctionnalités suivantes :
- Définition des fractions de produits de base,
- Choix du mode de fonctionnement lors de l'appui sur le bouton de commande12, à savoir continu, purge ou dose,
- Affichage d'un triangle de fraction volumique tel qu'illustré la figure 30 avec gestion de la fraction volumique par touché tactile sur le triangle ou par les boutons +/associés à chaque produit,
- Connexion/déconnexion Bluetooth et transfert en temps réel des consignes au distributeur,
- Réglages des débits en mode continu, et de la quantité en mode dose,
- Calcul, affichage et transfert au distributeur des fractions volumiques des produits en temps réel, en fonction de la consigne, avec la somme des fractions toujours égale à 100%,
- Récupération et affichage des couples des trois moteurs en temps réel, et
- Sauvegarde des paramètres principaux dans un fichier de configuration.

Le mode continu est un mode de distribution où le mélange des trois produits de base est distribué tant que l'utilisateur appuie sur le bouton de distribution 12. Le produit est délivré avec un débit dont une estimation est affichée au-dessus du bouton 304 « Continu ». Le choix du débit se fait dans un menu « Réglages ».

Le mode « dose » est un mode de distribution du mélange par dose, où la dose est délivrée suite à une impulsion de l'utilisateur sur le bouton de distribution 12. Une impulsion suffit et l'utilisateur peut ensuite relâcher le bouton. La dose globale de produit distribuée est celle indiquée au-dessus du bouton 306 « Dose », 0.1 ml par exemple. Ce volume peut être modifié dans le menu « Réglages ».

Le mode « purge » est un mode de distribution où une dose de mélange à fractions volumiques égales (33%) est délivrée dès que l'utilisateur a appuyé sur le bouton de distribution 12, comme dans le mode « Dose ». Une impulsion suffit et l'utilisateur peut ensuite relâcher le bouton. Quand la dose a été entièrement distribuée, on peut relâcher le bouton. Si l'on relâche avant la fin, la distribution s'arrête, même si le volume spécifié n'est pas atteint. La dose globale de produit distribuée est celle indiquée au-dessus d'un bouton 305 « Purge », 3 ml par exemple. Ce volume peut être modifié dans le menu Réglages.

L'utilisateur détermine la matité/brillance voulue avec l'application qui s'éxécute sur la tablette et qui calcule les fractions des différents produits. La tablette communique cette valeur au distributeur par liaison Bluetooth.

L'électronique intégrée au distributeur 11 récupère l'information et ajuste automatiquement les débits des trois cartouches de façon à obtenir un mélange de la matité/brillance souhaitée.

Quand l'utilisateur veut utiliser le produit, il appuie sur le bouton 12 du distributeur pour faire sortir le produit. Il presse aussi longtemps qu'il veut du produit, en mode « continu ». En mode « dose », l'utilisateur presse une fois le bouton 12 et la dose prédéfinie est délivrée.

La distribution peut se faire en continu, c'est-à-dire les moteurs fonctionnent en continu, tout le volume étant distribué d'une traite, ou de façon itérative, les moteurs fonctionnant alors de façon impulsionnelle ; dans ce cas, l'intervalle de temps entre deux impulsions permet de faire varier le débit. De petits volumes sont délivrés les uns après les autres en plusieurs étapes.

Les impulsions peuvent être séparées par exemple par des intervalles de 50 ms, 100 ms ou 200 ms. La durée d'une impulsion pendant laquelle le moteur tourne va parexemple de 50 à 150 ms.

La page principale de l'application « µMix » comporte dans cet exemple les éléments suivants, comme visible notamment sur la figure 31 :
- Barre de statut en haut de l'écran : indique l'état de la connexion Bluetooth ou µMix s'il n'y a pas de connexion Bluetooth ;
- Barre d'onglets en bas de l'écran : permet de sélectionner la page active :page principale, Réglages, Bluetooth, Produits et Favoris ;
- Bouton Continu 304 pour sélectionner le mode de distribution de produits en continu ;
- Bouton Purge 305 pour sélectionner le mode Purge ;
- Bouton Dose 306 pour sélectionner le mode de distribution par dose avec le volume de dose associé au bouton Dose ;
- Balle bleue 300 que l'utilisateur peut déplacer dans le triangle volumique en la faisant glisser ou avec un double tapotement ;
- Boutons « - » 302 pour chaque produit A, B et C : diminue la fraction de produit sélectionné en se déplaçant le long de la droite reliant le point au sommet du produit sélectionné ;
- Boutons « + » 302 pour chaque produit A, B et C : augmente la fraction de produit sélectionné en se déplaçant le long de la droite reliant le point au sommet du produit sélectionné ;
- Fraction volumique en pourcentage de chaque produit : modifiable par l'utilisateur et mise à jour en temps réel selon la consigne des boutons 302 + et - et de la position de la balle 300.

Lors de la modification des fractions volumiques par déplacement de la balle ou par les boutons + et -, les valeurs des fractions volumiques des produits A, B et C sont mises à jour automatiquement. Lorsque les fractions volumiques sont modifiées avec les boutons + et -, la balle 300 est déplacée automatiquement à la position correspondante sur le triangle.

Au démarrage de l'application exécutée sur la tablette, celle-ci se connecte automatiquement au distributeur 11 s'il est détecté. Lorsque le distributeur s'éteint ou que la connexion Bluetooth est coupée, la tablette se déconnecte. Quand l'utilisateur agit sur les curseurs de réglage des proportions des produits A et B, les valeurs sont transmises en temps réel au distributeur 11.

La page Réglage de l'application comporte les éléments suivants :
- Barre de statut en haut de l'écran : indique l'état de la connexion Bluetooth ou µMix s'il n'y a pas de connexion Bluetooth ;
- Barre d'onglets en bas de l'écran : permet de sélectionner la page active :
   page principale, Réglages, Bluetooth ou Info ;
- Partie « Volumes » avec un champ de texte à remplir par l'utilisateur pour définir le volume de la dose, en ml (2 ml par exemple) et un champ pour le volume de la purge, en ml (3 ml par exemple). Les doses minimales sont dans cet exemple de 0.023 ml et les doses maximales 9.90 ml (3 x 3.3 ml) ;
- Partie « Débit » avec la sélection du débit : rapide (» 0.03 ml/s), moyen (» 0.02 ml/s) ou lent (» 0.01 ml/s) ;
- Partie « Dose » avec le choix du mélange itératif, pour distribuer un mélange de produits avec des petits volumes délivrés les uns après les autres en plusieurs étapes ;
   Dans le cas contraire, le volume total de chaque produit est distribué d'une traite ;
- Partie « Image Triangle » pour choisir l'image du triangle qui sera affichée sur la page principale afin de pouvoir afficher un triangle avec les matité/brillances délivrées par le distributeur 11. En utilisant un bouton « Choix image » de la page « Réglages » on accède à un album.

La page « Produits » de l'application comporte dans l'exemple considéré les éléments suivants :
- Choix de la valeur de chaque produit en unités de pas codeurs, de 0 à 1414.

Chaque unité correspond à un volume de produit délivré de 2.33 µl, qui est la plus petite quantité que le distributeur dans cet exemple peut délivrer ; lorsque cette feuille est affichée, ce sont les valeurs de produits de cette feuille qui sont transmises en temps réel au distributeur. Dès que la feuille n'est plus affichée, les valeurs envoyées au distributeur sont celles de la feuille principale avec le triangle ;
- Affichage des couples moteur A, B et C en temps réel avec rafraîchissement toutes les 45 valeurs.

Le mode de délivrance des produits est le mode dose directe ou itératif, selon l'option choisie dans la feuille Réglage.

La page « Favoris » permet de sauvegarder des configurations en fichier. Elle donne accès dans l'exemple considéré à 10 fichiers, à savoir « Configuration 1 » à « Configuration 10 » en plus du fichier par défaut. Ces fichiers enregistrent par exemple les paramètres suivants
- fractions des produits A, B et C
- volume de Purge
- volume de Dose,
- débit rapide, moyen ou lent,
- mode Dose, Purge ou Continu,
- distribution continue ou itérative.

### Exemple (septième aspect de l'invention)

On réalise plusieurs produits de base (les proportions sont massiques).

Les formules F1 et F2 sont riches en charges (et de couleurs différentes). La formule F3 est riche en nacre (oxychlorure de bismuth).

| | | F1 | F2 | F3 |
|---|---|---|---|---|
| | | % massique | % massique | % massique |
| | Dimethicone copolyol vendu sous la référence KF 6017 par la société Shin Etsu | 2 | 2 | 1,8 |
| | Etsu Bis PEG/PPG-14/14 dimethicone + Cyclopentasiloxane vendu sous la référence ABIL EM 97 par la société Goldschmidt | 1 | 1 | 0,9 |
| | Cyclopentasiloxane | 17,65 | 17,65 | 15,92 |
| | Phenyl trimethicone vendu sous la référence DC556 par la société Dow Corning | 2 | 2 | 1,8 |
| | Ethyl hexyl methoxycinnamate | 3 | 3 | 2,7 |
| | Squalane | 1 | 1 | 0,9 |
| | Dispersion d'oxychlorure de bismuth dans hydroxystéarate d'éthyl (2) hexyle 70 :30 (Timiron Liquid Silver^{®} de Merck) | 0 | 0 | 10 |
| | Cyclopentasiloxane | 7 | 7 | 6,29 |
| | Oxyde de fer faune enrobé de stearoyl glutamate d'aluminium NAI-C33-9001-10 de la société Miyoshi Kasei | 1,62 | 1,1 | 1,45 |
| | Oxyde de fer rouge enrobé de stearoyl glutamate d'aluminium NAI-C33-8001-10 de la société Miyoshi Kasei | 0,29 | 0,6 | 0,26 |
| | Oxyde de fer noir enrobé de stearoyl glutamate d'aluminium NAI-C33-7001-10 de la société Miyoshi Kasei | 0,13 | 0,13 | 0,12 |
| | Dioxyde de titane (anatase) enrobé de stearoyl glutamate d'aluminium NAI-TAO-77891 de la société Miyoshi Kasei | 9,96 | 10,17 | 8,95 |
| | Talc vendu sous la référence Micro Ace P3 par la société Nippon Talc | 0,5 | 0,5 | 0,45 |
| | Poudre de nylon 12 vendue sous la référence SP 500 par la société Toray Industries | 0,5 | 0,5 | 0,45 |
| | Eau déminéralisée | 36,15 | 36,15 | 32,53 |
| | 1,3-Butylene glycol | 3 | 3 | 2,7 |
| | Sulfate de magnésium | 0,7 | 0,7 | 0,63 |
| | Solution de maltose hydrogénée | 0,5 | 0,5 | 0,45 |
| | Alcool éthylique 96° dénaturé | 13 | 13 | 11,7 |
| | TOTAL | 100 | 100 | 100 |

### Mode opératoire

On pèse les constituants de la phase A dans le bêcher principal et l'on agite en maintenant à température ambiante sous Moritz (1000 tr/min). La phase B est préparée séparément en broyant trois fois à la tricylindre, le mélange de pigments et de cyclopentasiloxane. Cette phase B est ensuite ajoutée en maintenant l'agitation, ainsi que les charges (phase C).

La phase aqueuse D est aussi préparée séparément, en pesant dans un bêcher le butylène glycol, le sulfate de magnésium, la solution de maltose hydrogénée et l'eau à température ambiante. La phase aqueuse D est placée sous agitation à l'aide d'un barreau magnétique jusqu'à homogénéisation. C D E L'émulsion se fait à température ambiante : on verse la phase aqueuse D dans la phase grasse en augmentant progressivement la vitesse d'agitation (Moritz) jusqu'à 4000 tours/mn. On maintient l'agitation pendant 7 mn. Puis on ajoute enfin la phase E (alcool éthylique) pendant les 3 dernières minutes de 5 l'émulsification.

### Test

Le système a été testé avec :
- F1 dans le compartiment A
- F2 dans le compartiment B
- F3 dans le compartiment C

| | |
|---|---|
| M1 (les proportions dans le mélange distribué sont volumiques): | A 30%, B 20%, C 50% |
| M2 : | A 30%, B 50%, C 20% |
| M3 : | A 30%, B 30%, C 40% |

M1, M2, M3 sont appliquées sur différentes zones du visage. On obtient des couleurs différentes, et des résultats de matités brillance différentes

### G) Système pour personnaliser son fond de teint en fonction de l'état de sa peau

La présente invention concerne les procédés et systèmes de distribution, notamment d'un produit de maquillage.

De nombreuses personnes souhaitent se maquiller pour embellir leur apparence, notamment leur visage.

Les motivations de ces personnes peuvent se ranger en deux catégories :
- Masquer certaines imperfections, comme des taches, des rides ou des pores,
- Embellir le rendu du visage par des changements de couleur.

Dans ces différents cas, l'opération consiste à apporter une matière colorée et à en couvrir la peau ou une zone de peau.

Pour obtenir un effet esthétique, la personne doit réussir le choix de la matière colorée.

Dans le premier cas ci-dessus, l'opération peut être complexe car le visage comporte toute une gamme de couleurs.

Ainsi, si la personne ne veut couvrir que quelques zones du visage, en essayant de faire coïncider la couleur ajoutée à la couleur naturelle de la peau environnante, il lui est nécessaire de chercher la couleur qui convient à chaque zone du visage, ce qui est d'autant plus difficile que la couvrance du produit et l'épaisseur déposée, ainsi que la couleur et l'état de surface de la peau sous-jacente ou son caractère plus ou moins gras, peuvent influencer le résultat.

Compte-tenu de ces difficultés, les personnes qui veulent masquer les imperfections de leur visage, ont l'habitude de couvrir l'ensemble du visage. On contourne alors le problème du choix de la matière colorée en fonction de la zone du visage.

Toutefois, le résultat s'éloigne de l'aspect naturel du visage de par l'homogénéité apportée.

Dans le deuxième cas, l'opération n'est pas simple non plus car il est difficile de trouver la matière colorée qui convient le mieux à l'aspect du visage. En particulier, il est difficile de trouver la couleur de son teint, surtout si l'on veut choisir une couleur marquée, différente de sa couleur naturelle. Certaines personnes aimeraient choisir des couleurs bronzées ou autres nuances de teint différentes, mais elles ne le font pas de peur que la couleur ne leur corresponde pas bien. Et si elles le font, elles abandonnent souvent déçues. Quand elles ne sont pas déçues du résultat, elles n'osent plus changer de couleur.

Il en est de même pour le maquillage des lèvres et celui des joues et des paupières.

Les utilisateurs ont aussi besoin de pouvoir ajuster la qualité, la tenue, et le confort de leurs maquillages en fonction du moment du climat ou saison, mais aussi de l'endroit du visage. D'habitude, en utilisant un produit à forte teneur en charges, le maquillage s'adapte bien aux zones plutôt grasses pour apporter de la matité tout au long de la journée. Toutefois, cette solution n'est pas satisfaisante car elle peut conduire à des problèmes d'inconfort sur les autres zones de la peau, et elle a tendance à générer un maquillage inesthétique sur les zones sèches. L'utilisation de composition riche en agent hydratant tel que des glycols comme la glycérine par exemple permet de maquiller l'ensemble du visage dont les zones les plus sèches. Mais cette solution n'est pas très satisfaisante pour d'autres zones du visage. L'exemple du tour du nez est un exemple caractéristique. La zone du dessous du nez est souvent sèche et nécessiterait ainsi l'application d'une formulation contenant un agent hydratant. A quelques centimètres, les ailes du nez sont souvent grasses et supportent mal l'application d'un produit hydratant, produisant brillance et inconfort, et nécessiterait l'application d'une formulation contenant des charges pour apporter de la matité tout au long de la journée.

Les changements climatiques (au jour le jour et au cours de l'année), complique le problème.

On cherche une solution la plus simple possible pour résoudre ce problème.

De plus, on veut pouvoir contrôler la couleur ou la couvrance des produits.

Ceci rend la situation compliquée.

Il est possible d'utiliser plusieurs produits de couleurs et/ou couvrance différentes mais généralement, ils présentent des niveaux de confort et de qualité du maquillage inadaptés. Ainsi, même si une seule zone devient inconfortable, à cause d'une mauvaise adaptation du confort à la zone, le confort entier devient une source de souci, poussant même à l'abandon du maquillage. De la même manière si une seule zone présente un défaut de qualité du maquillage, à cause d'une mauvaise adaptation à la zone, la qualité du maquillage en entier devient une source de souci, poussant même à l'abandon du maquillage.

Il faudrait une solution pour donner accès à toute une gamme de niveau de couleur et couvrance et qui puissent être mis sur un même visage, sans qu'on puisse causer de problème de confort et de qualité du maquillage.

Pour résoudre ces problèmes, il n'existe que peu de solutions.

Une première approche consiste à acheter de nombreux produits et à tous les essayer. Cette approche est coûteuse et génère souvent des pertes dans la mesure où l'on ne retient généralement qu'un petit nombre des matières colorées essayées.

Une seconde approche consiste à tester, en magasin, différents produits. Elle ne convient pas toujours, car il est très difficile de se figurer le résultat en quelques instants et dans un lieu dépourvu des repères habituels. En particulier, pour apprécier pleinement, l'effet d'un maquillage dans un magasin, il faudrait pouvoir l'éclairer de la même manière que dans les conditions futures d'utilisation, ce qui est rarement possible. En général, ce n'est qu'en testant un maquillage pendant la journée, qu'on se rend compte s'il convient ou non sur le plan du résultat et du confort. De plus, si certains magasins disposent de conseillers et permettent d'effectuer des essais, ce n'est pas le cas d un grand nombre d'autres points de vente et des achats effectués sur internet.

Une autre approche a été testée sans toutefois se développer. Elle consiste à réaliser manuellement ses produits par mélange de plusieurs produits de couleur. Cela peut s'avérer relativement difficile à mettre en oeuvre, car il est peu aisé de reproduire exactement plusieurs fois le même mélange, et l'on a du mal à réaliser rapidement les mélanges dont on a besoin au moment du maquillage.

On connaît aussi des distributeurs permettant de délivrer une composition cosmétique de couleur variable.

Parmi les essais d'automatisation de la fabrication d'une composition cosmétique personnalisée qui ont pu être tentés, nombreux sont ceux dans lesquels les solutions proposées permettent de réaliser des mélanges dans des quantités d'environ 100 g ou parfois moins, mais pas dans les très petites proportions dont la personne qui se maquille a généralement besoin, c'est-à-dire de l'ordre du gramme ou beaucoup moins.

Pour illustrer cette problématique, on peut imaginer le cas d'une personne qui veut cacher deux imperfections de l'ordre du cm2 sur son visage. Pour la première zone, elle a besoin de trouver le mélange correspondant, puis d'en délivrer une très petite quantité, par exemple d'environ 10 mg. Pour la seconde, elle a besoin de changer le réglage du distributeur, puis de délivrer une toute petite quantité également.

Par conséquent, le choix des matières colorées conférant les meilleurs résultats reste difficile pour un grand nombre de personnes.

Il existe par conséquent un besoin pour faciliter la recherche d'un produit de maquillage répondant aux attentes d'un consommateur, et permettant à celui-ci de réaliser des mélanges dans des conditions fiables en quantités diverses.

L'invention vise ainsi, selon certains de ses aspects, à faciliter le maquillage du visage et notamment la recherche des produits les plus adaptés aux différentes zones de celuici sur le plan du confort et de la qualité du maquillage.

Il existe encore un besoin pour perfectionner les systèmes de distribution permettant de délivrer des produits de couleur variable, afin notamment de faciliter leur utilisation et améliorer la qualité du maquillage.

Il existe aussi un besoin d'appliquer sur le même visage plusieurs produits, avec des taux d'ingrédients choisis pour s'adapter à l'état de la peau.

En particulier, les gens peuvent avoir des zones du visage plus ou moins tachés et donc nécessitant des forces de masquages différentes. Il est donc intéressant de pouvoir disposer d'un moyen de produire des formulations avec différents types d'ingrédients pour optimiser le confort et la qualité du maquillage vis-à-vis des spécificités de la zone.

Il existe donc un besoin pour perfectionner les systèmes de distribution permettant de délivrer des produits de couleur variable tout en réglant les ingrédients afin notamment d'optimiser le confort et la qualité du maquillage vis-à-vis de la spécificité de la zone.

L'invention repose selon un huitième de ses aspects sur un système de distribution qui permet de générer des mélanges à partir de produits de base. Ces produits de base peuvent avoir des couleurs différentes, de telle sorte que l'on puisse faire varier la couleur du mélange. Les produits de base peuvent encore permettre de faire varier les proportions de charges et d'agent hydratant du mélange, de telle sorte que le confort et la qualité du maquillage qui résulte de l'application du mélange sur les matières kératiniques humaines varie, en étant plus ou moins proche de celle desdites matières. Les produits de base peuvent aussi faire varier la couleur et la couvrance. Ainsi, la notion de couleur doit être comprise avec une acceptation large et englobe des mélanges dont la couleur varie après application du fait de leur couvrance plus ou moins élevée et de la couleur de la peau sous-jacente.

L'invention a pour objet selon un huitième de ses aspects un système de distribution d'un produit, comportant un distributeur recevant au moins deux cartouches ayant chacune un réservoir et contenant respectivement des premier et deuxième produits de base, le premier produit de base comportant un agent hydratant, le deuxième produit de base comportant une charge, le distributeur permettant de délivrer au moins ces deux produits de base dans des proportions réglables.

L'invention peut présenter selon ce huitième aspect une ou plusieurs des caractéristiques suivantes :
- l'agent hydratant est choisi parmi les polyols, l'urée et ses dérivés tels que notamment les hydroxyalkyle urée en particulier l'hydroxyéthylurée, l'acide hyaluronique, la glycine, la β-alanine, la taurine, la triméthyle glycine, et leurs mélanges, préférentiellement choisi parmi les polyols tels que l'éthylèneglycol, le pentaérythritol, le triméthylolpropane, le propylène glycol, la glycérine, les polyglycérols, les polyéthylènes glycols, et leurs mélanges et plus préférentiellement choisi parmi le propylène glycol et la glycérine,
- la taille D50 en volume des particules de la charge est comprise entre 100 nm et 1 mm, mieux entre 1 micron et 100 microns, encore mieux entre 2 microns et 50 microns,
- la charge est choisie parmi le talc, le mica, la silice, le kaolin, les poudres de poly-β-alanine et de polyéthylène, les poudres de polymères de tétrafluoroéthylène, les poudres de nylon, les poudres de polymethacrylate de methyle, la lauroyl-lysine, l'amidon, le nitrure de bore, les microsphères creuses polymériques telles que celles de chlorure de polyvinylidène/acrylonitrile, de copolymères d'acide acrylique, les microbilles de résine de silicone, les particules de polyorganosiloxanes élastomères, le carbonate de calcium précipité, le carbonate et l'hydro-carbonate de magnésium, l'hydroxyapatite, le sulfate de barium, les oxydes d'aluminium, les poudres de polyuréthane, les charges composites, les microsphères de silice creuses, et les microcapsules de verre ou de céramique, les particules qui ont la forme de portions de sphères creuses, et leurs mélanges, préférentiellement est choisie parmi le talc, le mica, la silice, les poudres de nylon, les poudres de polymethacrylate de methyle, et leurs mélanges, encorepréférentiellement étant du talc,

- la charge peut comporter un enrobage comportant au moins un composé lipophile ou hydrophobe,
- le premier produit de base et le deuxième produit de base comportent une émulsion inverse,
- la teneur massique en charge dans le deuxième produit de base est supérieure ou égale à 0,5% en masse par rapport à la masse du deuxième produit et de préférence supérieure à 1% en masse par rapport à la masse du deuxième produit,
- le système de distribution comporte une troisième cartouche avec un troisième produit de base
- les cartouches sont reçues de façon amovible dans le distributeur,
- chaque produit quitte la cartouche par un canal de sortie de la cartouche, le canal de sortie étant défini par un embout de distribution de la cartouche entraîné en rotation relative par rapport à un corps de la cartouche par un mécanisme d'entraînement du distributeur pour distribuer le produit de base contenu dans la cartouche.

L'invention a encore pour objet selon ce huitième aspect un procédé d'application d'un produit cosmétique, de maquillage et/ou de soin, sur les matières kératiniques humaines, comportant le réglage du distributeur en fonction de la zone à traiter, et la distribution du produit par prélèvement dans les cartouches des produits de base dans les proportions correspondant au réglage du distributeur.

L'invention repose selon son huitième aspect sur un système de distribution, avec des compartiments contenant au moins deux formulations différentes. Elles peuvent contenir des émulsions inverses différentes, l'une concentrée en charges, l'autre concentrée en agent hydratant tels que des glycols comme la glycérine par exemple.

On peut programmer le système pour qu'il délivre pour chaque endroit du visage le meilleur mélange en matité, couleur, couvrance et confort (ne contenant ni trop ou trop peu de charges, ni trop ou trop peu d'agent hydratant). Le système peut aussi être utilisé pour faire varier le niveau de charges et d'agent hydratant selon l'état du moment ou l'état du climat présent ou à venir (par ex, plus hydratant pour l'hiver, comprenant plus de charges pour l'été).

### Système de distribution

Le système de distribution peut être constitué par un seul appareil fonctionnant de manière autonome, de préférence manipulable d'une main, ou par un appareil qui fonctionne en interagissant avec d'autres pièces ou appareils. Il peut s'agir par exemple de différentes interfaces de sorties qui sont montées sur le distributeur en fonction du type de maquillage à réaliser, comme cela sera précisé plus loin. Il peut s'agir également d'un système informatique qui échange des informations avec le distributeur pour le piloter, ce système informatique comportant par exemple un terminal portable tel qu'un téléphone intelligent, un photophone, une tablette, un ordinateur personnel, ou un terminal dédié.

De préférence, le distributeur est agencé pour mettre sous pression un ou plusieurs compartiments contenant le ou les produits de base, par le biais de dispositifs de dosage volumétrique, de préférence un moteur entraînant en déplacement un piston dans le compartiment correspondant.

Le distributeur peut être formé d'un boîtier et d'au moins deux ou trois compartiments, et de préférence d'autant de moteurs. Par exemple, la rotation des moteurs entraîne des vis sans fin, lesquelles poussent les pistons de chaque compartiment. L'avance des pistons est par exemple contrôlée par le nombre d'impulsions de commande envoyées aux moteurs et/ou par la durée de fonctionnement de ces derniers. Les moteurs peuvent être alimentés séquentiellement, ou de préférence simultanément.

Par exemple, les moteurs sont alimentés durant un cycle élémentaire de fonctionnement pendant une courte durée les uns après les autres, ou les uns en même temps que les autres, de façon à distribuer des microdoses correspondantes.

Les cycles élémentaires sont répétés, avec éventuellement un temps d'arrêt entre eux, qui laisse le temps aux produits de base de s'écouler hors des compartiments.

Les compartiments peuvent être définis par des cartouches qui se retirent lorsqu'elles sont vides. En variante, les compartiments sont présents à demeure et remplis à nouveau une fois vide.

Chaque cartouche peut être fermée par un bouchon démontable pour permettre le nettoyage de la cartouche.

De préférence, le boîtier du distributeur est de forme allongée selon un axe longitudinal, ce qui peut faciliter sa manipulation, et les cartouches sont disposées autour de cet axe, dans le boîtier.

De préférence, les cartouches sont mises en place par l'arrière, et le mélange est délivré par l'avant. La mise en place des cartouches peut s'effectuer individuellement, ou en variante les cartouches constituent un ensemble monobloc lors de leur mise en place.

Les cartouches peuvent comporter chacune un mécanisme de dosage volumétrique, comportant un piston mu par un mécanisme d'entraînement du distributeur, dans une direction s'accompagnant d'une diminution du volume intérieur contenant le produit de base et d'une expulsion du produit. Il peut être intéressant que les cartouches aient une région de leur paroi au moins qui soit transparente, afin de permettre de voir la couleur du produit contenu à l'intérieur.

Le mécanisme d'entraînement peut comporter un système de motorisation formé de moteurs couplés à des réducteurs, de forme allongée parallèlement à l'axe longitudinal du distributeur, et disposés entre les cartouches. Ce positionnement des moteurs et cartouches rend le distributeur particulièrement compact.

Le produit de base peut sortir de façon étanche de la cartouche correspondante puis circuler dans un canal prévu à cet effet dans le boîtier du distributeur, avant de sortir de celui-ci.

Les cartouches se terminent avantageusement par un embout réalisé de telle sorte que, une fois la cartouche insérée dans le boîtier du distributeur, l'extrémité de l'embout débouche au ras du boîtier. En variante, l'embout est suffisamment long pour dépasser du boîtier et connecter ainsi différentes interfaces de sortie susceptibles d'être rapportées sur le boîtier du distributeur.

Grâce au mécanisme d'entraînement comportant des moteurs pour faire avancer des pistons, il est possible de délivrer de façon précise des mélanges en de très petites quantités. Ainsi, le mécanisme d'entraînement peut délivrer les produits de base avec à un débit minimal inférieur ou égal à 50 µL/s, mieux inférieur ou égal à 20 µL/s, encore mieux inférieur ou égal 10 µL/s. De préférence, le mécanisme d'entraînement délivre des débits compris entre 20 et 100 µL/s, mieux compris entre 40 µL/s et 60 µL/s. Il est donc possible de réaliser facilement un mélange d'environ 10 mg. Un tel système de distribution est alors idéal pour réaliser de petites touches de maquillage, pour couvrir par exemple une zone de 1 cm², mieux une zone de 0, 5 cm².

Il est aussi possible de réaliser des plus grandes quantités de mélange comme celles nécessaires au maquillage d'une joue, ou d'un visage. Ces quantités restent néanmoins relativement faibles, par exemple une quantité comprise entre 100 et 500 mg, mieux comprise entre 150 et 250 mg.

De préférence, la cartouche comporte un embout de distribution par lequel s'effectue la sortie du produit, et cet embout de distribution est entraîné en rotation par le mécanisme d'entraînement pour déplacer le piston. L'embout peut comporter au moins un relief d'anti-rotation, mieux deux ergots d'anti-rotation diamétralement opposés.

L'embout peut porter un joint d'étanchéité, notamment un joint torique. Ainsi, au changement de cartouche, le joint est également changé, ce qui permet de s'affranchir de l'usure du joint.

Le distributeur peut comporter une carte électronique de pilotage du mécanisme d'entraînement motorisé, cette carte électronique étant traversée par le ou les embouts. Cela peut permettre de réaliser une carte s'étendant sensiblement sur toute la section du distributeur, donc de regrouper sur une seule carte tous les composants électroniques du distributeur, et ainsi de gagner en compacité et fiabilité. La carte peut s'étendre sensiblement perpendiculairement à l'axe longitudinal du boîtier. La carte peut porter un interrupteur de commande du fonctionnement du distributeur.

Le système de distribution peut être agencé pour fonctionner selon au moins deux modes de distribution.

Dans un premier mode, dit « continu », le mélange est distribué tant qu'une pression est exercée sur l'interrupteur de commande.

Dans un deuxième mode, dit « dose », une quantité prédéfinie du mélange est distribuée à chaque pression sur l'interrupteur.

Le ou les embouts peuvent déboucher à une extrémité du boîtier. Cela peut permettre de réduire le volume mort, comme cela sera détaillé dans la suite.

Le ou les embouts peuvent présenter à leur extrémité un système d'obturation pour éviter le séchage des produits dans le conduit, par exemple une membrane cicatrisante

La cartouche peut comporter une vis creuse sur laquelle le piston est vissé, le piston pouvant se déplacer axialement sur la vis lorsque celle-ci tourne ; le piston est empêché de tourner dans le corps de la cartouche. Par exemple, la friction du piston sur le corps de la cartouche peut suffire à l'empêcher de tourner quand la vis tourne.

Préférentiellement, la rotation est rendue impossible avec un corps de cartouche de section non circulaire et un piston non déformable.

Le couple des moteurs peut être déterminé électroniquement en fonction du courant absorbé, et servir par exemple à détecter une fin de course du piston. Une information concernant le couple peut être transmise à distance à un système informatique présentant une interface homme machine, pour permettre de surveiller le bon fonctionnement du distributeur.

Afin d'ajuster la teinte, le système de distribution selon l'invention doit permettre à l'utilisateur de faire varier le volume délivré provenant de chaque compartiment.

De façon préférée, le distributeur est piloté par un système informatique, qui est intégré au distributeur, ou externe à celui-ci, le distributeur étant alors apte à échanger avec le système informatique des informations par protocole sans fil ou câblé.

Le distributeur peut ainsi être piloté pour permettre l'ajustement de la teinte par distribution simultanée ou séquentielle, et contrôlée, de plusieurs produits de base avec des proportions de charges et d'agent hydratant différentes.

La distribution des produits de base peut être continue ; si dans ce cas, les volumes de chacun des produits de base sont distribués d'une seule traite, simultanément ou successivement.

Dans le cas d'une distribution simultanée, il est utile de pouvoir ajuster les débits respectifs des différents produits de base pour que le mélange distribué corresponde à celui souhaité à chaque instant. Un tel mode de distribution peut convenir en particulier lors de la distribution du mélange par pulvérisation, à l'aide d'un aérographe. Pour ajuster les débits il est possible par exemple de jouer sur la vitesse de déplacement des pistons, par exemple, dans le cas d'un entrainement des pistons par une vis sans fin en faisant varier la vitesse de rotation des moteurs qui entrainent la vis. Les produits peuvent aussi être distribués de façon impulsionnelle avec un temps de distribution et un temps d'arrêt à chaque cycle. En jouant sur le rapport cyclique, il est possible d'agir sur le débit.

Tous les produits peuvent être délivrés simultanément lors du temps de distribution ; en variante, les cycles des différents produits sont déphasés, de sorte qu'un produit est distribué pendant un temps d'arrêt des autres produits.

Dans un mode particulier de l'invention, le mélange est délivré dans une cavité d'un contenant, pouvant fermer de façon hermétique ou non, par exemple en forme de coupelle, dans lequel on peut faire glisser un applicateur, en particulier un stylet ou une brosse. Un tel système de distribution est spécialement adapté aux liners, gloss et autres formules appliquées sans contact direct avec les mains. Ce contenant peut être amovible.

Par exemple, il sert de distributeur de rouge à lèvres et dispose d'un système de distribution, à vis par exemple. Lorsqu'il n'est pas amovible, le contenant peut être réalisé avec le corps du distributeur. Lorsqu'il est amovible, il peut constituer une interface de sortie parmi d'autres pouvant être montées sur le distributeur.

Les compartiments, et en particulier les cartouches, peuvent contenir tout ou partie du mécanisme d'entraînement, et par exemple le système de motorisation, ou mieux, une partie du système de motorisation, et ce, afin de réduire le nombre de pièces en mouvement dans le corps du distributeur à l'extérieur des cartouches. Par exemple, les cartouches comportent le rotor du moteur. Une fois les cartouches installées dans le corps du distributeur, les rotors sont mis en interaction avec les stators.

Le système de distribution est avantageusement agencé pour permettre de lancer des séquences préprogrammées dans lesquelles le mélange délivré par celui-ci est modifié de façon continue ou discontinue. Un mode « dégradé » permet par exemple passer progressivement d'un mélange A à un mélange B. Dans le cas d'une application par pulvérisation, notamment à l'aide d'un aérographe, cela permet de réaliser simplement les dégradés. Un mode « Alternatif » permet par exemple de passer rapidement d'un mélange

A à un mélange B plusieurs fois de suite. Dans le cas d'une application par pulvérisation, on peut ainsi réaliser un dépôt multicouche, avec des formulations différentes pour deux couches adjacentes superposées. Un autre mode permet par exemple de proposer plusieurs mélanges successifs préprogrammés, le système informatique indiquant à chaque fois l'usage qu'il faut en faire à l'utilisateur, par exemple par affichage sur un écran.

Dans le cas d'une application manuelle, les mélanges sont distribués par exemple dans une coupelle. La personne se maquille à l'endroit recommandé avec un mélange correspondant prélevé dans la coupelle, puis nettoie éventuellement la coupelle et commande la délivrance d'un nouveau mélange ; l'opération est répétée autant de fois que nécessaire pour se maquiller complètement.

L'homogénéisation des mélanges distribués peut se faire de différentes façons selon les cas d'utilisation. Dans le cas d'une application manuelle, elle peut se faire directement sur la zone d'application au moment de l'application ou dans la coupelle avant prélèvement ; dans le cas d'une application à l'aérographe, c'est la tuyère de l'aérographe qui sert de chambre de mélange ; dans le cas où le mélange est distribué dans un contenant en vue d'une utilisation ultérieure, l'homogénéisation peut être faite à la main ou en faisant passer les produits distribués dans une chambre de mélange, située entre le distributeur et le contenant ou directement intégrée au contenant, comme détaillé par la suite.

Le produit peut être délivré par le système de distribution et utilisé extemporanément. En variante, le produit délivré par le système de distribution est conditionné et utilisé ultérieurement, par exemple à plusieurs reprises, avec par exemple un jour d'intervalle au moins entre deux utilisations.

L'invention repose selon son huitième aspect sur un système de distribution, avec des compartiments contenant au moins deux formulations différentes. Elles peuvent contenir des émulsions inverses différentes. L'une concentrée en charges. L'autre concentrée en agent hydratant.

### Charges

Ces charges sont des particules incolores ou blanches, solides de toutes formes, qui se présentent sous une forme insoluble et dispersée dans le milieu de la composition.

A titre illustratif de ces charges, peuvent être cités le talc, le mica, la silice, le kaolin, les poudres de poly-β-alanine et de polyéthylène, les poudres de polymères de tétrafluoroéthylène (Téflon^{®}), les poudres de nylon, les poudres de polymethacrylate de methyle, la lauroyl-lysine, l'amidon, le nitrure de bore, les microsphères creuses polymériques telles que celles de chlorure de polyvinylidène/acrylonitrile comme l'Expancel^{®} (Nobel Industrie), de copolymères d'acide acrylique, les microbilles de résine de silicone (Tospearls^{®} de Toshiba, par exemple), les particules de polyorganosiloxanes élastomères, le carbonate de calcium précipité, le carbonate et l'hydro-carbonate de magnésium, l'hydroxyapatite, le sulfate de barium, les oxydes d'aluminium, les poudres de polyuréthane, les charges composites, les microsphères de silice creuses, et les microcapsules de verre ou de céramique. On peut également utiliser des particules, qui ont la forme de portions de sphères creuses, telles que décrites dans les demandes de brevet JP-2003 128 788 et JP-2000 191 789.

Les charges peuvent comporter un enrobage comportant au moins un composé lipophile ou hydrophobe.

La charge est autre qu'un pigment coloré apportant une couleur à la composition, tel qu'une nacre ou un oxyde de fer.

### Agent hydratant

Par « agent hydratant », on entend selon la présente invention tout composé susceptible de pénétrer dans le stratum corneum et de maintenir celui-ci hydraté.

Les agents hydratants utilisables selon l'invention sont notamment choisis parmi les polyols, l'urée et ses dérivés tels que notamment les hydroxyalkyle urée en particulier l'hydroxyéthylurée comme le produit vendu sous le nom commercial HYDROVANCE^{®} par la société AKZO NOBEL, l'acide hyaluronique, la glycine, la β-alanine, la taurine, la triméthyle glycine et leurs mélanges.

Par « *polyol* », il faut comprendre, au sens de la présente invention, toute molécule organique comportant au moins deux groupements hydroxyle libres.

Selon une forme particulière, le polyol peut être choisi parmi les sucres tels que le thréhalose, le mannitol, le xylitol, le sorbitol, et leurs mélanges.

De préférence, un polyol conforme à la présente invention est présent sous forme liquide à température ambiante.

Un polyol convenant à l'invention peut être un composé de type alkyle, linéaire, ramifié ou cyclique, saturé ou insaturé, portant sur la chaîne alkyle au moins deux fonctions - OH, en particulier au moins trois fonctions -OH, et plus particulièrement au moins quatre fonctions -OH.

Les polyols convenant avantageusement pour la formulation d'une composition selon la présente invention sont ceux présentant notamment de 2 à 32 atomes de carbone, de préférence 3 à 16 atomes de carbone.

Avantageusement, le polyol peut être par exemple choisi parmi l'éthylèneglycol, le pentaérythritol, le triméthylolpropane, le propylène glycol, le 1,3 propanediol, le butylène glycol, l'isoprène glycol, le pentylène glycol, l'héxylène glycol, la glycérine, les polyglycérols, tels que les oligomères du glycérol comme le diglycérol, les polyéthylènes glycols, et leurs mélanges.

Selon un mode de réalisation préféré de l'invention, ledit polyol est choisi parmi l'éthylèneglycol, le pentaérythritol, le triméthylolpropane, le propylène glycol, la glycérine, les polyglycérols, les polyéthylènes glycols, et leurs mélanges.

Selon un mode particulier, la composition de l'invention peut comprendre au moins du propylène glycol et/ou de la glycérine.

### Emulsions inverses / Compositions anhydres

Selon l'invention, lorsqu'il est précisé qu'une composition est sous la forme d'une émulsion inverse, il est entendu qu'elle peut également être alternativement sous une autre forme selon la composition considérée.

Un produit de base peut se présenter sous la forme d'une émulsion, par exemple une émulsion H/E, E/H, H/E/H ou E/H/E, et de préférence une émulsion inverse E/H, ou sous la forme d'une composition anhydre, comprenant notamment des composés carbonés et/ou siliconés, tels que des huiles hydrocarbonées et/ou siliconées.

Les émulsions selon le huitième aspect de l'invention sont de préférence des émulsions eau-dans-huile (E/H) encore appelées émulsions inverses, à savoir constituées d'une phase huileuse continue dans laquelle est dispersée la phase aqueuse sous forme de gouttelettes de manière à observer un mélange macroscopiquement homogène à l'oeil nu.

### Utilisation pour le maquillage d'une ou plusieurs zones précises de peau

Le système de distribution permet de se maquiller jour après jour, en ne traitant que les zones à masquer. Pour ce faire, on délivre de petites doses de maquillage, qu'on applique précisément et séquentiellement sur les zones correspondantes. Chaque petite dose est réalisée selon le mélange qui convient à la zone.

Dans un mode préféré de mise en oeuvre de l'invention, le système de distribution attend qu'on le renseigne sur une zone à traiter puis délivre le mélange correspondant. Il peut faire appel à une table de correspondance préprogrammée pour cela, cette table résultant par exemple d'un procédé d'apprentissage tel que défini plus loin. En variante, le système de distribution renseigne la personne, tout en délivrant un mélange, sur la zone sur laquelle celle-ci doit appliquer le mélange. Ainsi, le système de distribution peut suivre un programme d'application où il délivre dans un ordre donné, les différents mélanges à appliquer.

Dans un mode particulier de mise en oeuvre de l'invention, le système de distribution est renseigné sur les quantités à délivrer. Pour cela, il mémorise la correspondance entre couleur, la zone du visage et la quantité nécessaire, ce qui permet de réduire les coûts et les pertes de produit, et de ne couvrir que faiblement la peau, évitant ainsi des effets d'occlusion. Ce faisant, il est possible d'utiliser des produits très couvrants et trop couvrants pour être appliqués sur l'ensemble du visage. Ainsi, il est possible d'obtenir des maquillages d'aspects naturels, voire indétectables.

Le système de distribution peut aussi permettre, en facilitant la distribution de petites quantités et leur usage rapide, de réduire la durée de conservation des produits, permettant ainsi de réduire les risques d'évolution et/ou les quantités de conservateurs à employer.

Le système de distribution est adapté à traiter les zones qu'on veut masquer, sans avoir à masquer l'ensemble du visage.

Il peut aussi être utilisé pour masquer une ou des taches tout en adaptant le produit pour le meilleur confort et la meilleure qualité du maquillage vis-à-vis de la zone.

Dans une variante spécialement avantageuse, le système peut être utilisé pour application sur le fond du visage une formule peu masquante puis une ou deux applications sur des zones précises du visage des formules plus masquantes le tout avec le meilleur confort et la meilleure qualité du maquillage vis-à-vis de chaque zone. On peut aussi commencer par applications sur des zones précises du visage des formules masquantes puis appliquer sur le fond du visage une application d'une formule peu masquantes.

Dans le second cas, on prend le temps, avant d'appliquer la seconde couche, d'attendre que le film produit par l'application sur les zones précises devienne suffisamment cohésif pour que la seconde couche n'emporte pas la première couche.

Ou bien, on applique la seconde couche sans frotter (spray par exemple, ou éponge).

Lorsque l'utilisateur recherche la couleur à appliquer sur une zone du visage, il est intéressant de mémoriser la couleur qui va bien pour chaque zone, et le système de distribution est ainsi avantageusement agencé pour mémoriser cette couleur et la zone correspondante. Ainsi, en utilisant les informations mémorisées, à chaque utilisation, on peut obtenir la délivrance du même mélange pour chaque zone, ou, si l'on traite plusieurs zones, la même série de mélanges pour une même série de zones.

Le système de distribution peut aussi être agencé pour permettre de traiter une zone en faisant varier les couleurs application après application. Ainsi, la personne peut maquiller les lèvres avec différentes couleurs, qu'elle choisit au jour le jour selon ses goûts. Cette approche est aussi adaptée aux paupières ou aux cils, ainsi qu'au maquillage du visage car la personne peut avoir envie de changer de couleur de teint. Par exemple, les jours de la semaine, la personne réalise un fond de teint couleur claire, et le weekend, un fond de teint de couleur hâlée, ou un maquillage des yeux d'une couleur un jour, et d'une autre couleur un autre jour. Idem pour des variations des proportions de charges et d'agent hydratant pour avoir le meilleur confort et la meilleure qualité du maquillage pour chaque zone.

Le système de distribution peut être agencé pour permettre à l'utilisateur de changer de couleur selon ses goûts du jour, du moment, de ses tenues, du climat. Ainsi, on prévoit avantageusement un système d'aide à la décision pour guider l'utilisateur dans ses choix de couleur (au sens large) et des proportions de charges et d'agent hydratant.

On peut prévoir aussi un système d'aide pour équilibrer les couleurs sur un même visage et participer à la réalisation d'un maquillage global réussi, le tout avec un résultat maquillage et un confort idéal pour chaque zone et donc un résultat maquillage et un confort optimal sur l'ensemble du visage.

Il peut être souhaitable que plusieurs personnes d'un même groupe, par exemple une famille, puissent utiliser le système de distribution, réduisant ainsi les coûts et minimisant la place occupée. Cette solution est spécialement adaptée aux voyages ou aux hôtels, campings, avions, campings cars, boutiques, écoles... Pour ce faire, on peut prévoir que le système de distribution puisse être renseigné sur la personne qui l'utilise, afin d'accéder à des données personnelles préalablement mémorisées.

### Utilisation en continu pour un maquillage en dégradé d'hydratation/matification

Dans cette application, le système de distribution, pendant qu'il délivre le produit, change la formulation du mélange. De plus, on déplace la sortie des produits de base ou du mélange relativement à un contenant ou un support définissant une surface d'application. Dans un mode particulier de mise en oeuvre de l'invention, le système de distribution est agencé pour calculer l'évolution du mélange en fonction de l'hydratation/matification C1, d'une zone à traiter et de l'hydratation/matification C2, d'une autre zone à traiter. Par exemple, sachant que le menton nécessite une hydratation/matification C1 et que la joue nécessite une hydratation/matification C2, le système de distribution peut faire varier la formulation du mélange pendant qu'il le délivre pour réaliser un traitement entre ces deux points. Cela permet par exemple de mieux masquer les imperfections du visage en faisant en sorte que le résultat final soit confortable et que le maquillage soit de qualité, même si on couvre des zones de peau d'états différents. Le système de distribution peut encore être agencé de telle sorte que l'utilisateur puisse commander une variation d'hydratation/matification du mélange distribué sans que les hydratation/matification de départ et/ou d'arrivée n'aient été préalablement fixées. Pour ce faire, le système de distribution peut disposer d'un système de localisation, ou d'auto-localisation, et déduire d'une table de correspondance l'hydratation/matification C1 et l'hydratation/matification C2 qu'il doit réaliser, et donc les évolutions de mélange qu'il doit réaliser.

Le système de distribution peut comporter une tête de sortie, en particulier dans le cas d'un aérographe, qui est mobile et pilotée. Cette option permet alors, sans bouger le reste du système de distribution, de réaliser des dégradés. Par exemple, on localise le système de distribution près de la joue, puis on déclenche un système de commande qui va piloter automatiquement la variation de la formulation du mélange et le mouvement de la tête de sortie, de façon à par exemple avoir le centre de la joue plus rouge que sa périphérie, avec un dégradé entre les deux.

Le système de distribution peut encore être utilisé pour réaliser des produits sur mesure, que l'on conserve pour réaliser plusieurs applications.

Il est aussi possible de réaliser des produits solides ou semi-solides.

### Fabrication de compacts « à façon » ou autres produits solides ou semi-solides

Le système de distribution peut être agencé pour permettre de choisir un mélange et de le délivrer dans un contenant tel qu'une coupelle. Le mélange comporte de préférence des composés qui sont tels que le mélange puisse prendre en masse.

Plus préférentiellement, on utilise des composés qui rendent la prise en masse spécialement rapide. Ces composés sont soit déposés dans le contenant avant ou après son remplissage avec les autres constituants, soit sont prévus dans les compartiments du distributeur avec les autres constituants des produits de base, soit sont compris dans le distributeur dans un compartiment spécialement prévu pour les contenir.

On peut ainsi distribuer des compositions spécifiques qui peuvent par réaction chimique, biochimique ou physicochimique, durcir de façon accélérée après évacuation.

Ces compositions sont spécialement adaptées à la réalisation des compacts, c'est-à-dire :
- Prennent en masse,
- donnent un matériau qui peut se déliter en cas de frottement, et sont préférentiellement colorées.

De préférence, ces compositions sont très riches en particules solides, avec par exemple plus de 10% en masse de particules solides par rapport au à la masse totale de la composition, mieux plus de 20% en masse de particules solides par rapport à la masse totale de la composition, encore mieux plus de 30% en masse de particules solides par rapport à la masse totale de la composition, préférentiellement entre 10 et 40% en masse de particules solides par rapport à la masse totale de la composition.

Ces compositions peuvent contenir des particules absorbantes, ou des composés réactifs, tels que ceux qui réagissent en contact avec l'air, par exemple du cyanoacrylate ou des alphasilanes, ou qui réagissent à la lumière, notamment UV.

Le contenant dans lequel le mélange est distribué peut comporter un composé A et les compositions distribuées un composé B, A et B étant choisis pour réagir entre eux et solidifier le mélange.

Dans un mode particulier de mise en oeuvre de l'invention, le système de distribution intègre un moyen de chauffage, par exemple à résistance électrique, pour la réalisation de rouges à lèvres ou autres produits cireux. Dans ce cas, les produits de base sont chauffés avant d'être délivrés.

Le système de distribution peut aussi comporter un moyen pour apporter de l'énergie, thermique et/ou lumineuse, après distribution du mélange dans un contenant, par exemple une résistance électrique ou une diode LED, notamment UV. Cette énergie peut accélérer la prise en masse du mélange distribué.

Préférentiellement, le mélange est homogénéisé avant la prise en masse.

Les produits, de couvrance différentes, présenteront de l'hydratation/matification différentes, adaptées à différents états de la peau.

### Réalisation de palettes de couleurs

Le système de distribution peut comporter un support, ayant plusieurs régions, et être agencé pour générer automatiquement plusieurs mélanges déposés dans lesdites régions, par exemple une série de couleurs adaptées à différentes parties du visage.

Le support peut définir plusieurs cavités pour recevoir les mélanges ou porter plusieurs contenants, par exemple sous forme de coupelles, éventuellement séparables du support.

Dans un cas particulier, le support prend la forme d'un visage avec des régions pour recevoir les mélanges pour des zones d'applications ciblées.

Le support être mobile, notamment rotatif, par rapport au corps du distributeur, et par exemple entraîné en déplacement par le distributeur, pour remplir successivement différents logements ou contenants.

Les produits, sur la palette pourront être d'hydratation/matification différentes.

### Distributeur à coupelle

Il existe un intérêt pour bénéficier d'un système de distribution capable de délivrer un mélange pouvant être facilement prélevé par l'utilisateur. Par ailleurs, dans le cas où les produits de base délivrés par le système de distribution ne sont pas déjà mélangés, il existe un besoin pour permettre à l'utilisateur d'effectuer aisément le mélange.

De préférence, le système de distribution comporte une coupelle, et un distributeur pour remplir la coupelle avec au moins un produit, la coupelle étant solidaire du distributeur au moins pendant son remplissage.

La coupelle est parfois encore appelée « creuset » et ce terme doit être compris avec une acceptation large.

Par « coupelle solidaire du distributeur » il faut comprendre que la coupelle est retenue, notamment immobilisée, au moins temporairement sur le distributeur, étant par exemple fixée sur celui-ci par des vis, par aimantation, par encliquetage, par verrouillage baïonnette, par serrage, ou réalisée avec une partie du corps du distributeur par moulage de matière. La coupelle lorsqu'elle est solidaire du distributeur permet de manipuler celui-ci d'une main, la coupelle restant en place sur le distributeur au cours des mouvements de ce dernier.

Le distributeur peut être proposé à l'utilisateur avec la coupelle déjà en place.

En variante, celle-ci est installée par l'utilisateur à la première utilisation du système de distribution.

De préférence la coupelle est moins profonde que large, ce qui facilite l'accès à celle-ci, et permet de prélever le produit, notamment le mélange avec un applicateur ou le doigt.

De préférence, la coupelle est séparable du distributeur, et constitue une interface de sortie qui peut être choisie parmi un ensemble d'interfaces de sortie pouvant être montées sur le distributeur, au choix de l'utilisateur en fonction du maquillage à réaliser, comme détaillé plus loin.

De préférence, le système de distribution comporte plusieurs orifices de remplissage en des produits de base différents, débouchant dans la coupelle. Ainsi, le mélange de ces produits peut avoir lieu dans la coupelle.

La coupelle a de préférence un fond de forme concave vers l'extérieur, ce qui peut faciliter son nettoyage par l'utilisateur entre deux utilisations.

De plus, cela peut faciliter le prélèvement du produit par l'utilisateur, et le mélange des produits de base.

De préférence, le distributeur permet de délivrer au moins deux produits de base dans la coupelle, dans des proportions réglables, et mieux au moins trois produits.

Dans un exemple de mise en oeuvre, le système de distribution comporte au moins deux coupelles pouvant être sélectivement alimentées par le distributeur. Cela peut permettre à l'utilisateur de remplir rapidement ces deux coupelles avec des mélanges dont les caractéristiques sont différentes. Cela peut faciliter les essais de matières colorées, et/ou permettre de préparer plusieurs mélanges d'hydratation/matification différentes destinées au maquillage de zones respectives du visage. Les coupelles peuvent être associées à des identifiants rappelant à l'utilisateur à quelle zone du visage un mélange contenu dans une coupelle donnée est destiné.

Les coupelles peuvent être mobiles relativement au distributeur, étant par exemple portées par un support mobile, tel qu'une tourelle mobile en rotation par rapport au distributeur, ou par un tiroir mobile en translation par rapport au distributeur.

Le système de distribution peut comporter un couvercle de fermeture de la coupelle. Ce couvercle de fermeture est de préférence transparent pour permettre à l'utilisateur de visualiser la couleur du mélange contenu à l'intérieur.

Lorsque la coupelle est séparable du distributeur, elle peut être introduite le cas échéant dans un boîtier permettant de la transporter plus facilement, ce boîtier pouvant comporter le cas échéant un miroir et/ou un applicateur. Le couvercle du boîtier peut dans ce cas servir de couvercle pour la coupelle.

Le volume de la coupelle peut être compris entre 2 et 1000 mm³, mieux entre 100 et 1000 mm³, encore mieux entre 250 et 750 mm³.

Le ou les produits de base délivrés dans la coupelle sont de préférence des fonds de teint, mais en variante il s'agit de produits de maquillage des lèvres ou des paupières.

La coupelle est de préférence de forme symétrique de révolution. En variante, elle est de contour polygonal ou autre. Son plus grand diamètre intérieur, ou celui du cercle inscrit dans le cas d'un contour non circulaire, est de préférence compris entre 2 et 100 mm, préférentiellement entre 5 et 40 mm. Sa profondeur est de préférence comprise entre 1 et 10 mm, mieux entre 3 et 8 mm. De préférence, la taille et la forme de la coupelle permettent soit une application directe du mélange sur la peau, soit une préhension au doigt ou avec un applicateur. La coupelle peut être réalisée avec un matériau élastiquement déformable, ce qui permet par exemple d'inverser la concavité du fond de la coupelle et de vider celle-ci plus facilement ou de l'utiliser pour appliquer le produit.

La coupelle peut être dépourvue de mélangeur ; dans ce cas, les produits de base peuvent arriver du distributeur dans la coupelle à l'état non mélangé, par des orifices de distribution respectifs distincts. En variante, le distributeur intègre un mélangeur et les produits de base arrivent déjà mélangés dans la coupelle.

La coupelle peut aussi intégrer un mélangeur statique, comme détaillé plus loin, qui est alimenté par des orifices de remplissage distincts du distributeur, et qui délivre de préférence le mélange dans une cavité de la coupelle située au-dessus du mélangeur.

L'invention a encore pour objet un procédé de préparation d'un produit de maquillage, comportant l'étape consistant à remplir une coupelle d'un système de distribution tel que défini plus haut avec au moins un produit de base provenant du distributeur.

Les produits, de couvrance différentes, présenteront des effets de matité/brillance équivalents

Plusieurs produits peuvent être délivrés dans le fond de la coupelle, puis mélangés à l'aide du doigt ou d'un applicateur, ou d'un mélangeur statique intégré à la coupelle.

La coupelle est de préférence remplie par le dessous. Il a été proposé dans le passé des systèmes de distribution à l'aide d'une sonotrode.

La coupelle selon l'invention n'est pas destinée à entrer en vibration pour distribuer le ou les produits amenés par le ou les canaux d'alimentation de celle-ci. Elle est différente d'une sonotrode. De préférence, la coupelle est réalisée en matière plastique.

### Mélangeur intégré à l'interface de sortie

Il existe un intérêt pour bénéficier d'un système de distribution capable de délivrer un mélange pouvant être facilement utilisé, notamment prélevé par l'utilisateur, sans nécessiter une action additionnelle de mélange de la part de celui-ci.

L'invention a pour objet un système de distribution comportant un distributeur ayant des canaux de sortie de produits de base et une interface de sortie séparable du distributeur, cette interface ayant un mélangeur statique, délivrant de préférence le mélange dans une cavité où il peut être prélevé.

Le mélangeur statique peut être situé sous la cavité précitée. Le système de distribution est alors particulièrement adapté à la création de compacts, en utilisant comme interfaces de sortie des coupelles à mélangeur statique intégré. Dans ce cas, on vient remplir la cavité de la coupelle en produit par le dessous. Après passage dans le mélangeur statique, les produits de base mélangés viennent recouvrir le mélangeur.

Selon cet aspect de l'invention, il est possible d'utiliser plusieurs interfaces de sortie et de les remplir avec des mélanges respectifs différents, sans avoir à purger le mélangeur, ce qui réduit les pertes de produit. L'interface de sortie peut être à usage unique, le cas échéant.

De préférence, le mélangeur statique comporte une chambre centrale communiquant avec des canaux d'admission des produits de base. Cette chambre centrale peut communiquer avec une chambre périphérique comportant une série de cloisons qui agissent comme des déflecteurs pour le mélange, et créent un cisaillement de celui-ci.

La chambre périphérique peut comporter une cloison annulaire ajourée définissant des ajours par lesquels passe le mélange en circulant dans la chambre périphérique. Les chambres centrale et périphérique peuvent être fermées supérieurement par une paroi qui définit le fond de la cavité recevant le mélange.

Le fond de la chambre périphérique peut être de forme hélicoïdale autour de l'axe de la coupelle et de hauteur diminuant au fur et à mesure que l'on progresse vers la sortie. Cette dernière peut déboucher en avant d'une rampe de liaison entre le fond de la chambre périphérique et la paroi supérieure du mélangeur, cette rampe de liaison étant de préférence une portion d'hélice prolongeant l'hélice formée par la paroi de fond de la chambre périphérique.

De préférence la chambre périphérique comporte la cloison annulaire précitée et des cloisons radiales qui forcent le mélange à circuler alternativement entre des régions supérieure et inférieure de la chambre périphérique et entre des régions radialement intérieure et extérieure, le mélange circulant par exemple d'une région supérieure et radialement extérieure à une région inférieure et radialement extérieure en passant à travers la cloison annulaire précitée.

Le mélangeur peut comporter un corps extérieur dans lequel est reçue une pièce formant le coeur du mélangeur, le corps extérieur fermant radialement à l'extérieur la chambre périphérique et comportant un montant qui sépare les chambres centrale et périphérique.

Le corps extérieur du mélangeur et le coeur du mélangeur peuvent être chacun réalisés d'une seule pièce par moulage par injection.

### Volume mort réduit

Il existe un intérêt pour réduire les pertes de produit lors des changements de formulation du mélange, ainsi que pour permettre de faire varier le plus rapidement possible la couleur ou l'hydratation/matification du mélange au cours de l'application, notamment en cas de couplage du distributeur avec un aérographe.

L'invention a encore pour objet un système de distribution d'un produit de maquillage, comportant un distributeur recevant au moins deux cartouches ayant chacune un réservoir contenant un produit de base, ce dernier quittant la cartouche par un canal de sortie de la cartouche, ce canal de sortie débouchant à l'extérieur du distributeur ou à proximité de sa surface externe.

Le canal de sortie peut notamment déboucher dans une zone de prélèvement du mélange ou à proximité de celle-ci, notamment à moins de 5 mm, mieux à moins de 3 mm, mieux à moins de 1mm, mieux à fleur.

La section transversale du canal de sortie est par exemple comprise entre 1 et3 mm².

Ainsi, chaque produit de base provenant d'une cartouche peut quitter le distributeur sans se mélanger avec un produit de base d'une autre cartouche et l'on minimise le volume mort qui ne peut être prélevé et susceptible d'accroître l'inertie du système. Le produit est plus rapidement disponible sans avoir à circuler dans des canaux spécifiques du boîtier du distributeur, évitant une étape fastidieuse de purge si changement de cartouche.

L'extérieur du distributeur peut être la zone de prélèvement du produit, notamment lorsque le distributeur est réalisé avec une coupelle non prévue pour être enlevée, dans laquelle le mélange est distribué, ou une zone du distributeur destinée au montage d'une interface de sortie amovible, qui définit la zone de prélèvement. Cette interface de sortie peut comporter une coupelle telle que définie ci-dessus. Cette zone de montage correspond par exemple à l'extrémité du boîtier du distributeur en l'absence de l'interface de sortie. La zone de montage peut être sensiblement plane et perpendiculaire à l'axe longitudinal du boîtier du distributeur.

Le distributeur peut comporter trois cartouches de produits de base.

Le distributeur peut comporter des logements pour recevoir les cartouches, lesquelles sont de préférence reçues de façon amovible dans le distributeur. Ce dernier peut comporter des passages pour des conduits des cartouches définissant les canaux de sortie.

La longueur de ces conduits est de préférence telle que les conduits se situent légèrement en retrait du fond ou à affleurement de la cavité servant au prélèvement du produit, ou en variante légèrement en retrait ou à affleurement de la face d'extrémité du boîtier du distributeur définissant la zone de montage.

Ces conduits des cartouches peuvent être des embouts servant à entraîner en déplacement des pistons au sein des cartouches, comme détaillé plus haut.

### Interfaces de sortie multiples

Il existe un besoin pour pouvoir, à l'aide d'un même système de distribution, réaliser facilement des maquillages différents, et pouvoir si on le souhaite maquiller des zones aussi différentes que la peau, les lèvres ou les cils ou sourcils.

Selon l'un de ses aspects, indépendamment ou en combinaison avec les autres aspects de celle-ci, et notamment ce qui précède, l'invention a pour objet un système de distribution comportant un ensemble comportant un distributeur d'au moins un produit cosmétique, notamment de maquillage, et au moins deux interfaces de sortie pouvant se monter chacune de façon amovible sur le distributeur, ces interfaces de sortie permettant de recevoir le ou les produits délivrés par le distributeur, étant de préférence choisies parmi les suivantes :
- interface de sortie comportant un contenant, notamment une coupelle permettant un prélèvement du produit au doigt ou à l'aide d'un applicateur,
- interface de sortie permettant de délivrer le produit à un système de pulvérisation, notamment un aérographe,
- interface de sortie comportant plusieurs régions de réception du produit, mobile par rapport au distributeur,
- interface de sortie permettant de délivrer le produit à un embout de distribution.

De préférence, l'ensemble comporte au moins trois desdites interfaces de sortie, mieux les quatre interfaces de sortie.

Le distributeur peut comporter au moins deux produits de base différents et permettre de délivrer ceux-ci dans des proportions variables, et de préférence, le distributeur comporte trois produits de base différents et permet de délivrer ceux-ci dans des proportions variables.

Chaque interface de sortie peut comporter une base permettant sa fixation sur le distributeur. Cette fixation peut s'effectuer à l'aide de vis par exemple, mais de préférence la base est agencée pour permettre un démontage et un remplacement d'une interface de sortie sans nécessité d'outil. Il s'agit par exemple d'une fixation quart de tour ou à l'aide d'une bague de verrouillage externe.

L'interface de sortie et/ou le boîtier du distributeur peuvent comporter des joints d'étanchéité permettant une communication étanche entre le boîtier du distributeur et l'interface de sortie. Le cas échéant, le distributeur est agencé pour reconnaître l'interface de sortie qui est montée dessus, par exemple grâce à identifiant de l'interface de sortie sous forme de reliefs spécifiques, lesquels sont détectés par le distributeur, ou d'une puce électronique reconnue par le distributeur. Cela peut permettre d'adapter le fonctionnement du distributeur à l'interface de sortie montée dessus. Le distributeur peut communiquer à un système informatique des informations sur l'interface de sortie qu'il porte, et le système informatique peut afficher en fonction de ces informations un écran spécifique et/ou lancer un programme spécifique de commande des paramètres de fonctionnement du distributeur, afin par exemple d'adapter la dose distribuée et/ou le débit à la nature de l'interface de sortie.

Plusieurs interfaces de sortie peuvent être proposées initialement à l'utilisateur avec un distributeur commun au sein d'un même emballage, par exemple un coffret ou une boîte de carton.

L'invention a encore pour objet un procédé de maquillage, comportant l'étape consistant à sélectionner une interface de sortie, à la monter sur le distributeur, et à délivrer le ou les produits contenus dans le distributeur dans celle-ci.

### Cartographie et apprentissage

Le terme « cartographie » s'entend ici comme un procédé d'indexation, d'hydratation/matification et d'une zone, avec enregistrement.

Il se peut qu'une personne nécessite plusieurs niveaux d'hydratation/matification avec une seule couleur ou couvrance pour l'ensemble des zones.

Il se peut aussi qu'elle nécessite plusieurs niveaux de couvrance avec un niveau d'hydratation/matification sur une partie du visage et un autre niveau d'hydratation/matification pour une autre zone. Par exemple, elle a plusieurs taches sur le front et plusieurs taches sur les joues. Ainsi, elle voudra réaliser plusieurs niveaux de couvrance pour le front avec un niveau d'hydratation/matification donné pour cette surface. Elle voudra réaliser plusieurs niveaux de couvrance pour les joues avec un niveau d'hydratation/matification pour cette surface.

La cartographie peut concerner des applications sur des zones plus petites qu'un cm2. Toutefois, l'oeil nu a alors du mal à discerner si le résultat obtenu est adéquat, et il est préférable de substituer l'évaluation à l'oeil nu par une évaluation instrumentée, avec grossissement. L'application de petites quantités de matière colorée peut être faite au doigt, avec des outils classiques tels que des pinceaux, ou avec des applicateurs spécialisés.

La cartographie peut être générée lors d'une période d'apprentissage, pendant laquelle l'utilisateur effectue des tests avec des mélanges sur différentes zones du visage ; une fois renseignée, la cartographie peut ensuite être utilisée pour le maquillage de tous les jours.

Des interfaces graphiques spécifiques peuvent servir pendant la période d'apprentissage et pendant la période d'utilisation de la cartographie.

En particulier, le système de distribution peut être utilisé avec une interface graphique où l'opérateur voit le visage, qui est par exemple schématisé, figuratif ou précis comme une photographie ou une simulation 3D. Dans ce cas, l'opérateur peut pointer une partie du visage sur l'écran pour voir apparaître et/ou délivrer l'hydratation/matification adéquate.

L'interface graphique peut aussi laisser apparaître les autres zones du visage où l'utilisation de la même hydratation/matification est adéquate.

Pour réaliser la cartographie, l'opérateur applique une hydratation/matification puis réalise l'évaluation.

Les zones du visage peuvent être traitées les unes après les autres ; par exemple, on réalise l'exercice sur une partie de la joue, puis sur le nez, etc....

Une autre possibilité consiste à réaliser un mélange donné, et appliquer sur plusieurs zones ce même mélange. L'opérateur doit alors chercher la zone du visage pour laquelle l'hydratation/matification est adaptée. Le mélange est ensuite indexé dans le système informatique en l'attribuant à la ou aux zones du visage pour lesquelles il convient.

L'invention a pour objet un procédé d'apprentissage d'un système de distribution comportant un distributeur permettant de distribuer un mélange d'hydratation/matification variable, et un système informatique permettant de sélectionner une hydratation/matification et de mémoriser des données, comportant les étapes consistant à :
a) Sélectionner au moins une hydratation/matification à l'aide d'une interface du système informatique,
b) délivrer à l'aide du distributeur au moins un mélange d'hydratation/matification sélectionnée,
c) évaluer le ou les mélanges distribués après application sur au moins une zone du visage,
d) mémoriser les caractéristiques d'au moins un mélange, notamment un mélange que l'utilisateur souhaite pouvoir rappeler, et d'au moins une zone sur laquelle il a été testé.

Cette mémorisation peut s'effectuer notamment en vue d'une distribution ultérieure de ce mélange pour maquiller ladite zone.

De préférence, le système informatique est agencé pour permettre à l'utilisateur de signaler si le résultat du test est satisfaisant ou non, voire de le renseigner sur la comparaison à un test effectué précédemment.

On peut aussi réaliser un mélange donné, et chercher la zone du visage pour laquelle il est adapté. On enregistre alors le mélange en l'attribuant à la ou aux zones du visage pour lesquelles il est adapté, dans une table de correspondance qui servira par la suite, pour, partant d'une zone de la peau, en déduire le mélange à utiliser.

On peut procéder de la même façon avec d'autres mélanges pour réaliser une cartographie de l'ensemble du visage et ainsi avoir une table de correspondance complète du visage.

On peut aussi réaliser un mélange donné, l'appliquer sur une zone donnée, puis faire varier le mélange jusqu'à obtenir le mélange le plus adapté. On enregistre alors le mélange en l'attribuant à la ou aux zones du visage pour lesquelles il convient, dans une table de correspondance qui servira par la suite, pour, partant d'une zone de la peau, en déduire le mélange à utiliser.

De préférence, le système informatique évalue et mémorise les quantités utilisées zone par zone. Un tel procédé, par « touches d'essai », permet d'identifier le ou les produits dont a besoin la personne qui veut se maquiller. Ainsi, le système de distribution peut servir sur les points de vente pour conseiller les personnes qui veulent se maquiller ou à la maison pour bien définir les produits à commander.

L'interface du système informatique comporte de préférence un écran tactile affichant l'hydratation/matification du mélange lors de sa sélection.

L'interface peut afficher un visage et permettre de renseigner le système informatique en sélectionnant la zone sur le visage affiché.

Le système informatique est de préférence agencé pour permettre d'associer une zone, des paramètres de reconstitution du mélange, et la date de l'essai et/ou tout autre identifiant du mélange.

Le système informatique est de préférence également agencé pour permettre d'associer en outre à ladite zone, aux paramètres de reconstitution du mélange, et à la date ou à l'identifiant du mélange, au moins l'une des données suivantes : le nom de la zone, la période de l'année, le nom d'un évènement, un identifiant de l'utilisateur, l'âge de l'utilisateur.

Les étapes a) à c) peuvent être renouvelées au moins une fois avant la mémorisation des caractéristiques du mélange à l'étape d).

Le système informatique peut être agencé pour rechercher dans une base de données une référence de produit commerciale, sur la base des caractéristiques du mélange identifié comme convenant à au moins une zone donnée, et la communiquer à l'utilisateur.

La sélection à l'étape a) peut s'effectuer avec l'aide d'un système expert externe ou non au système informatique.

Le système expert peut analyser une image de l'utilisateur, pour proposer une hydratation/matification de mélange au moins sur la base de l'image analysée.

L'étape a) peut être précédée par la proposition d'une hydratation/matification et d'une zone à tester avec un mélange de cette hydratation/matification, par le système informatique à l'utilisateur.

Le système informatique peut être agencé pour permettre à l'utilisateur de le renseigner sur l'appréciation du résultat du test de l'étape c) et pour générer une proposition de modification du mélange à sélectionner lors du retour à l'étape a).

Le système informatique peut être agencé pour proposer au moins une hydratation/matification de mélange à l'étape a) en fonction d'une zone d'application renseignée par l'utilisateur.

Le système informatique peut être agencé pour proposer au moins une zone d'application à l'étape a), en fonction d'une hydratation/matification renseignée par l'utilisateur.

Le distributeur peut délivrer à l'étape b) au moins deux mélanges de hydratation/matification différentes, de préférence séparés, en vue de leur application simultanée sur la zone de test.

Cela peut faire gagner du temps, et faciliter la comparaison des résultats.

L'invention a encore pour objet un procédé de maquillage à l'aide d'un système de distribution selon cet aspect de l'invention, dans lequel :
a) L'utilisateur adresse une requête concernant un besoin de maquillage au système informatique,
b) celui-ci génère en retour une proposition d'hydratation/matification leur en vue du maquillage d'une zone associée, sur la base de l'apprentissage effectué préalablement, et
c) le système informatique pilote le distributeur pour la production du mélange de l'hydratation/matification proposée, notamment si celle-ci est validée par l'utilisateur.

Un tel procédé peut utiliser une cartographie précédemment établie avec l'utilisateur.

L'invention a encore pour objet un produit programme d'ordinateur comportant des instructions de code permettant, lorsque exécutées dans un système informatique, d'amener le système informatique à :
- Permettre à l'utilisateur de sélectionner au moins une hydratation/matification et/ou une zone d'application, notamment à l'aide d'une interface telle qu'un écran tactile,
- piloter un distributeur de façon à délivrer un mélange d'hydratation/matification sélectionnée par l'utilisateur,
- permettre à l'utilisateur de déclencher la mémorisation de l'hydratation/matification du mélange et d'une zone d'application associée, notamment en vue d'une distribution ultérieure du même mélange, notamment sur la même zone.

Le produit programme d'ordinateur peut comporter des instructions de code permettant, lorsque exécutées dans un système informatique, d'amener le système informatique à :
- Recevoir une requête de l'utilisateur concernant un besoin de maquillage, notamment à l'aide d'une interface telle qu'un écran tactile,
- proposer sur la base au moins de données générées par le procédé d'apprentissage tel que défini ci-dessus, au moins une hydratation/matification et/ou une zone d'application,
- piloter un distributeur pour la production du mélange d'hydratation/matification proposée, notamment si celle-ci est validée par l'utilisateur.

La personne peut intégrer dans la cartographie le niveau de matité/brillance qu'elle désire pour chaque zone du visage, zone par zone ou ensemble de zones par ensemble de zones. Le système interprétera alors les mélanges qu'il doit réaliser pour assurer les hydratation/matification, couvrance désirées et la matité/brillance voulu.

La cartographie peut être modifiée par la suite. Ainsi, il est possible de réaliser la cartographie sur le plan des couleurs dans un premier temps, puis sophistiquer la cartographie en définissant par la suite les niveaux d'hydratation/matification.

### Aide à distance

Il est souhaitable de pouvoir aider l'utilisateur à se maquiller, notamment à choisir les bons coloris.

L'invention, selon l'un de ses aspects, indépendamment ou en combinaison avec ses autres aspects, notamment ce qui précède, a ainsi pour objet un procédé de maquillage comportant les étapes consistant à :
- permettre l'établissement d'une liaison vidéo, par exemple via internet, entre une caméra d'un premier site et un deuxième site,
- permettre au deuxième site de piloter directement ou indirectement un distributeur présent sur le premier site, ce distributeur permettant de faire varier hydratation/matification d'un mélange distribué,
- permettre à une personne présente au premier site d'appliquer le mélange distribué et d'envoyer vers le deuxième site une image correspondante, pour recevoir en retour une information relative au résultat de maquillage.

Le deuxième site peut notamment comporter un écran de visualisation qui permet à un conseiller présent devant cet écran de voir le résultat du maquillage avec le produit distribué par le distributeur et de conseiller la personne qui s'est maquillée. Ce conseiller peut agir en retour sur le distributeur pour modifier l'hydratation/matification du mélange et l'adapter au mieux au visage de la personne présente sur le premier site. Ainsi, cette personne gouverne le mélange délivré par le distributeur. La première personne peut se maquiller sous les yeux de la seconde. La seconde personne voit sur son écran le résultat de l'essai, et ainsi peut corriger le mélange qu'elle va commander à distance, jusqu'à obtenir le maquillage idéal.

Le cas échéant, l'acquisition vidéo peut être calibrée à l'aide d'une mire, ou avec le mélange distribué par le distributeur sur une surface de référence. Cela permet ensuite une visualisation plus fidèle du maquillage effectué sur le premier site.

De préférence, la liaison vidéo entre les deux sites est une liaison duplex.

Le premier site peut recevoir un tutoriel du deuxième site, le cas échéant.

Des identifiants des produits de base peuvent être communiqués au deuxième site ; cela peut permettre de connaître précisément l'hydratation/matification de chacun des produits de base.

Le procédé peut comporter la mémorisation des paramètres de réglage du distributeur, une fois qu'un mélange donné est estimé satisfaisant. De préférence, cette mémorisation peut être commandée depuis le deuxième site. La mémorisation peut avoir lieu dans le système informatique présent sur le premier site et/ou sur un serveur externe.

Une variante peut consister à faire travailler une personne pour aider le maquillage de plusieurs personnes. Ce mode de mise en oeuvre permet le développement des « Make-Up Artists » et leur travail soit au sein d'un institut, soit via internet. Cela permet aussi le maquillage de personnes aux moyens limités, telles que les personnes malvoyantes, ou discernant mal les hydratation/matification s, ou âgées, ou manquant de confiance en elles.

### Pilotage par interface tactile

Il existe un besoin pour faciliter le pilotage du système de distribution et notamment le choix de l'hydratation/matification du mélange distribué.

L'invention a encore pour objet un système de distribution comportant un distributeur et un système informatique de pilotage du distributeur, ce système informatique comportant un écran tactile sur lequel peut être affichée l'hydratation/matification du mélange et un moyen de sélection déplaçable sur l'écran, pour faire varier l'hydratation/matification du mélange distribué.

De préférence, l'écran affiche des hydratation/matification extrêmes entre lesquelles l'hydratation/matification du mélange peut être sélectionnée, en déplaçant le moyen de sélection entre ces hydratation/matification extrêmes.

L'écran peut afficher une échelle d'évocation d'hydratation/matification entre au moins deux niveaux, ou une surface, notamment de contour triangulaire, au sein de laquelle le moyen de sélection peut se déplacer. Cette surface peut localement faire apparaître l'hydratation/matification du mélange en fonction par exemple de la distance à chacun des sommets, matérialisant chacun un produit de base pur.

Le système informatique peut effectuer une partie des calculs nécessaires pour déterminer les fractions de chacun des produits de base conduisant à un mélange de l'hydratation/matification recherchée.

Le système informatique peut être un téléphone intelligent, un photophone, une tablette, un ordinateur individuel. En variante, il est intégré au boîtier du distributeur.

Le système informatique peut disposer d'une caméra. Cette dernière peut être utilisée notamment pour l'acquisition d'une image de l'utilisateur et/ou du mélange.

Le système informatique peut être agencé pour afficher une image d'un visage, afin de faciliter le repérage des zones sur lesquelles le mélange doit être appliqué.

### Couplage du système de distribution à un moyen de pulvérisation

Le système de distribution peut comporter ou être connecté à un moyen de pulvérisation du mélange, de préférence un aérographe.

L'invention a encore pour objet un ensemble comportant :
- Un moyen de pulvérisation, de préférence un aérographe comportant une chambre de prélèvement soumise à un courant d'air d'entraînement,
- un distributeur comportant au moins deux compartiments contenant des produits de base différents, les produits étant délivrés au moyen de pulvérisation de préférence par des orifices de distribution distincts.

Le distributeur peut comporter trois cartouches contenant des produits de maquillage d'hydratation/matification s différentes.

L'aérographe peut comporter un stylet définissant la chambre de prélèvement, le stylet se fixant sur le distributeur ou sur une interface de sortie fixée au distributeur, ou faisant partie intégrante de cette interface de sortie.

Le système de distribution peut comporter un circuit de contrôle du fonctionnement du distributeur, permettant de faire varier la proportion des produits de base délivrés dans la chambre de prélèvement, au cours du fonctionnement de l'aérographe. Les proportions peuvent être modifiées en fonction du déplacement de l'aérographe relativement à la surface sur laquelle le mélange est pulvérisé. Ce déplacement peut être mécanisé, le cas échéant.

Ce circuit de contrôle peut comporter ou être constitué par un système informatique tel que défini plus haut.

Le boîtier du distributeur peut servir de poignée lors de la manipulation d l'ensemble pour délivrer le mélange.

Le distributeur peut comporter une caméra et/ou un ou plusieurs capteurs tels que des accéléromètres afin de localiser automatiquement la zone sur laquelle le mélange est appliquée, et pouvoir régler automatiquement l'hydratation/matification en fonction de la position, le cas échéant.

L'invention a encore pour objet un procédé de maquillage à l'aide d'un ensemble tel que défini ci-dessus, dans lequel un mélange est pulvérisé sur la peau à l'aide du moyen de pulvérisation, notamment de l'aérographe.

La composition du mélange peut être modifiée au cours du déplacement de l'aérographe relativement à la peau. Un dégradé peut être réalisé.

Cet aspect de l'invention repose sur la constatation que le distributeur peut être utilisé pour alimenter le système de pulvérisation, notamment l'aérographe, tout en permettant au système de distribution d'être suffisamment réactif pour autoriser un changement de l'hydratation/matification du mélange distribué en cours de maquillage du visage, notamment lorsque la zone à maquiller change.

Il peut être avantageux que la distribution des produits se fasse de façon itérative, notamment avec des temps de distribution non déphasés entre les différents produits.

Cela peut rendre plus facile de faire varier la composition du mélange distribué au cours du temps.

Le mélange peut être réalisé directement dans l'aérographe, sans pratiquement aucun volume mort gênant, permettant ainsi de changer le mélange pulvérisé en temps réel.

La dépression créée dans la chambre de prélèvement est suffisante pour entraîner les produits de base, sans pour autant gêner le dosage.

La dépression qui règne dans la chambre de prélèvement est par exemple comprise entre 10 mbar et 200 mbar, mieux entre 50 et 150 mbar, encore mieux entre 75 et 125 mbar.

La viscosité des produits de base mesurée à 1 atm et à 25°C avec un viscosimètre rotatif CONTRAVES TV équipé d'un mobile MS-r3 ou MS-r4 à la fréquence de 60 Hz, après 10 minutes de rotation du mobile, est par exemple comprise entre 0,05 Pa.S et 50 Pa.S

La section des canaux d'arrivée des produits de base dans la chambre est par exemple comprise entre 1 et 3 mm², mieux entre 2 et 3 mm².

L'alimentation en produit s'effectue de préférence de façon continue.

Il est encore possible d'appliquer simultanément des produits de base non miscibles ou réactifs, comme un gel aqueux et un gel huileux, qui vont se déposer de façon pixellisée directement sur la peau, produisant une sorte de gel/gel in situ, des silicones réactives, ou des produits de coloration réagissant ensemble. Les ratios en produits de base peuvent être ajustés en fonction du résultat particulier souhaité. Par exemple, dans le cas des gels aqueux et gels huileux, on pourra faire varier le ratio correspondant au volume du premier produit de base sur le volume du deuxième produit de base entre 10/1 et 1/10, mieux entre 5/1 et 1/5.

### Système de localisation ou d'auto-localisation

Le système de distribution selon l'invention peut disposer d'un système de localisation ou d'auto-localisation.

On appelle système de localisation, un moyen de saisir, de la part de la personne, la zone qu'elle va traiter. Ceci peut être réalisé notamment par des systèmes laissant libre au moins une main. Ainsi, on peut utiliser une interface d'un système informatique telle qu'un écran tactile, un joystick, ou un système de reconnaissance vocale.

On appelle système d'auto-localisation, un moyen de saisir, sans que la personne n'intervienne, la zone à traiter. Ceci peut être réalisé par un ou plusieurs accéléromètres qui déduisent, par le fait des mouvements, les directions que la personne vise ou par une caméra et un système de reconnaissance d'image.

Des exemples de systèmes de distribution pouvant convenir à la mise en oeuvre de l'invention selon son huitième aspect sont représentés sur les figures précédemment décrites, qui ne seront donc pas toutes décrites à nouveau en détail.

On a illustré à la figure 29C un support ayant des logements disposés sur celuici sensiblement comme les différentes zones d'un visage ; chaque logement peut contenir un mélange dont l'hydratation/matification est adaptée à la partie correspondante du visage. Ainsi, il est facile pour l'utilisateur de savoir où appliquer le mélange prélevé dans un logement donné.

On peut utiliser le distributeur 11 pour délivrer un mélange dont la formulation change au cours du temps et recueillir le mélange dans un contenant mobile par rapport au distributeur, de telle sorte que le mélange soit déposé en un emplacement du contenant qui varie dans le temps, afin de réaliser un dégradé.

Par exemple, comme illustré aux figures 29A et 29B, le système de distribution comporte une interface de sortie 110 comportant une partie fixe relativement au distributeur et une partie mobile 252 présentant un logement 253 pour recevoir le mélange.

Par exemple, le distributeur 11 est disposé dans ce cas avec les orifices de sortie des cartouches vers le bas, et équipé d'un mélangeur, de telle sorte que le mélange tombe sous l'effet de son poids dans le logement 253. Un moteur peut déplacer la partie mobile de l'interface de sortie relativement au distributeur, de façon synchronisée avec la variation des caractéristiques du mélange, de telle sorte que l'on obtienne un dégradé le long dulogement 253, comme illustré à la figure 29B.

Le système de distribution peut comporter un socle 254 qui maintient le distributeur tête en bas.

L'interface de sortie 110, notamment lorsqu'elle comporte une coupelle, peut comporter un mélangeur statique qui assure le mélange des produits de base.

On a représenté sur les figures 17 à 21 une interface de sortie 110 comportant un tel mélangeur statique.

Cette interface de sortie 110 peut comporter un corps extérieur 260 qui se fixe sur le boîtier du distributeur 11 et qui présente un montant tubulaire extérieur 270.

Le corps 260 comporte des canaux 261 pour l'admission des différents produits de base. Ces canaux 261 débouchent dans une chambre centrale 262, délimitée par un montant tubulaire intérieur 263.

Ce montant 263 est traversé par une ouverture 264 qui débouche dans un espace annulaire 265, entre le montant intérieur 263 et le montant extérieur 270.

On a représenté sur les figures 31 à 37 différents exemples d'interfaces tactiles pouvant permettre à l'utilisateur de choisir l' hydratation/matification du mélange résultant de la distribution dosée des différents produits de base.

Cette interface peut présenter, comme illustré à la figure 31, une zone de sélection l'hydratation/matification, par exemple sous la forme d'un triangle dont les sommetscorrespondent à l'hydratation/matification de chacun des produits de base contenus dans les cartouches.

L'utilisateur peut déplacer un curseur 300, par exemple sous la forme d'une balle, relativement aux sommets A, B et C du triangle.

Plus il rapproche le curseur 300 de l'un des sommets, plus la fraction du produit de base correspondant est grande par rapport à la quantité totale des différents produits distribués.

La fraction de chaque produit par rapport à la quantité totale peut être indiquée en 301 par une valeur numérique sur l'interface.

L'interface peut permettre à l'utilisateur d'incrémenter ou de diminuer la quantité de chacun des produits, en agissant par exemple sur des boutons de commande 302, lesquels permettent un réglage précis de la quantité de chacun des produits de base.

La surface du triangle 310 peut avoir une hydratation/matification qui varie localement de façon à être représentative en chaque point de l' hydratation/matification du mélange résultant de la pondération des différents produits de base dans des proportions correspondant aux coordonnées relatives en ce point.

L'interface peut comporter un bouton 305 permettant d'accéder à un menu spécifique de réglage du volume de produit qui est distribué pour purger le distributeur.

L'interface peut aussi avantageusement permettre un réglage du débit de produit grâce à des boutons 304 et 306 renvoyant vers un menu spécifique de réglage du débit.

Dans l'exemple considéré, l'interface offre le choix entre un mode de distribution continu, grâce au bouton 304, où les produits sont distribués tant que l'utilisateur appuie sur le bouton de commande 12.

La dose correspondante peut être transmise à l'interface et affichée.

Le bouton 306 permet de sélectionner un fonctionnement en mode dose, au cours duquel un appui même bref sur le bouton 12 déclenche la distribution d'une dose prédéfinie.

Pour faire varier le débit, le distributeur agit par exemple sur le rapport cyclique de fonctionnement des moteurs.

L'interface peut être agencée pour permettre à l'utilisateur de programmer ou de mémoriser les réglages qui ont sa préférence, grâce à un menu 307 d'accès à des favoris.

L'interface tactile illustrée à la figure 32 fait apparaître sur l'écran trois zones colorées 400, correspondant chacune à l'hydratation/matification de l'un des produits de base contenus dans le distributeur 10, et une zone centrale 410 faisant apparaître l'hydratation/matification du mélange résultant.

La quantité relative de chacun des produits de base peut être ajustée à l'aide de curseurs 415 qui se déplacent par exemple sur des lignes joignant chacune des zones 400 à la zone centrale 410.

Au cours de l'utilisation de l'interface, celle-ci peut mémoriser un réglage donné et faire apparaître sur l'écran un bouton 420 de l'hydratation/matification du mélange. L'utilisateur peut ensuite, en appuyant simplement sur ce bouton 420, distribuer un mélange de l'hydratation/matification correspondante.

Dans l'exemple de la figure 34, l'interface affiche dans une zone 500 une teinte donnée, et propose à l'utilisateur, grâce à des boutons de commande 510 chacun de l'hydratation/matification du produit de base correspondant, d'accroître ou de diminuer la proportion de ce produit de base dans le mélange final. L'hydratation/matification de la zone 500 est recalculée en fonction des actions sur les boutons de commande 510.

Dans la variante de la figure 35, l'interface fait apparaître un nuancier présentant plusieurs zones 530 correspondants chacune à une proportion particulière des différents produits de base.

L'utilisateur peut sélectionner l'une de ces zones, par exemple en appuyant dessus avec son doigt.

L'interface peut être agencée pour afficher à une échelle plus grande dans une zone 535 l'hydratation/matification sélectionnée. La programmation du distributeur 11 pour distribuer cette hydratation/matification est par exemple déclenchée en appuyant sur la zone.

Dans l'exemple de la figure 36, l'utilisateur peut déplacer sur un nuancier d'hydratation/matification continu 550 un curseur 555, qui fait apparaître dans une zone 558 l'hydratation/matification sélectionnée.

L'utilisateur peut ensuite, en appuyant par exemple dans la zone 556, déclencher l'envoi au distributeur 11 des instructions nécessaires pour que celui-ci distribue un produit de l'hydratation/matification sélectionnée.

On voit sur la figure 37 que l'interface peut mémoriser les différentes teintes sélectionnées, et les faire ensuite apparaître sur l'écran de façon à permettre à l'utilisateur, en appuyant sur des boutons correspondants 560, de sélectionner à nouveau très facilement une teinte précédemment choisie.

On a représenté à la figure 38 un exemple d'interface utilisateur 1000 d'un système de distribution comportant un distributeur, de préférence tel que décrit précédemment, et un système informatique 100 auquel appartient l'interface.

Le système informatique comporte ici par exemple un appareil tel qu'un ordinateur portable, une tablette ou un téléphone intelligent, fonctionnant de façon autonome ou connecté à un serveur distant.

Dans l'exemple considéré, l'interface 1000 est définie par l'écran tactile d'un tel appareil. Dans une variante non illustrée, le distributeur intègre un écran tactile ou tout autre type d'interface homme machine, et peut être utilisé sans connexion à un autre appareil.

L'appareil exécute une application, qui a par exemple été téléchargée au préalable, et qui affiche sur l'écran un visage 1035 et une série de boutons permettant à l'utilisateur d'entrer des informations.

Le visage peut comporter plusieurs zones Zl à Z6 sélectionnables tactilement, par exemple le front, le nez, les joues, les paupières, le menton, et les lèvres.

Les boutons présents sur l'écran peuvent permettre par exemple d'entrer le nom du maquillage ou de l'utilisateur, d'afficher la zone sélectionnée, de choisir l'hydratation/matification , et d'informer le système informatique du résultat acceptable ou non du test effectué, voire comme illustré de donner une information concernant l'évaluation du résultat relativement à un test effectué précédemment, à savoir par exemple mieux ou « moins bien ». L'écran peut également afficher un bouton permettant de mémoriser le choix d'une hydratation/matification et d'une zone, après avoir effectué un essai avec cette hydratation/matification sur la zone en question.

Le choix de l'hydratation/matification s'effectue par exemple avec une échelle d'hydratation/matification similaire à celle décrite en référence à la figure 36.

Le système informatique est agencé pour mémoriser les données sous forme de table de correspondance par exemple, de manière à associer à une zone du visage des paramètres permettant de reproduire le mélange distribué lors du test. Ces paramètres comportent par exemple les teneurs relatives de chacun des produits de base du distributeur dans le mélange, la quantité Q distribuée, ainsi que des données additionnelles telles que par exemple le nom de la zone, la date de distribution du mélange et/ou tout autre identifiant du mélange, les identifiants des produits de base, la période de l'année, notamment la saison, l'âge de l'utilisateur, son sexe, son nom ou prénom, le nom d'un évènement associé au maquillage, par exemple un anniversaire, entre autres données, la quantité de produit adéquate pour la zone. Les données annexes peuvent permettre à l'utilisateur de reproduire plus facilement un maquillage qu'il a considéré comme adéquat pour une période de l'année, ou rappelant un évènement de la vie, ou pour donner un effet de rajeunissement.

Ces données peuvent être mémorisées dans le système informatique 100, par exemple dans l'appareil précité et/ou sur un serveur distant avec lequel l'appareil communique, ou encore dans une mémoire électronique intégrée au distributeur 11.

Ainsi, selon l'invention, l'utilisateur peut amener le distributeur à délivrer une première matière colorée, à l'appliquer sur une première zone du visage, puis la juger adéquate ou non. Si le résultat est satisfaisant, l'utilisateur peut l'enregistrer en l'indexant à la zone ; si le résultat est non satisfaisant, l'utilisateur peut commander une nouvelle hydratation/matification pour refaire les opérations ci-dessus.

Le système informatique peut être utilisé dans ce contexte de diverses façons.

Par exemple, comme illustré à la figure 40, l'utilisateur choisi à une étape 1010 une hydratation/matification à essayer, en utilisant par exemple l'échelle de hydratation/matification 1011 apparaissant sur l'écran, en déplaçant le bouton de réglage 1012.

Ensuite, le choix d'hydratation/matification est transmis au distributeur 11, à l'étape 1015.

Par exemple, l'appareil transmet les quantités à distribuer de chacun des produits de base, et le circuit électronique 81 se charge de commander les moteurs en conséquence.

A l'étape 1016, l'utilisateur appuie sur le bouton de commande 12 du distributeur 11, ce qui provoque par exemple la distribution d'une dose du mélange, de l' hydratation/matification sélectionnée par l'utilisateur.

Le mélange est par exemple distribué dans la coupelle 115, puis prélevé et appliqué par l'utilisateur sur les joues ou toute autre zone renseignée sur l'interface, à l'étape 1020.

Dans des variantes, le produit est appliqué à l'aide d'un aérographe ou par tout autre moyen tel que décrit précédemment.

L'utilisateur renseigne ensuite à l'étape 1022 le système informatique à l'aide des boutons 1021, sur le résultat.

Si l'utilisateur indique que le résultat est satisfaisant, le système lui propose par exemple de valider à l'aide d'un bouton les paramètres de l'essai afin de les mémoriser à l'étape 1031.

Si le résultat n'est pas jugé satisfaisant par l'utilisateur et signalé comme tel à l'aide du bouton 1032, le résultat peut néanmoins être automatiquement sauvegardé à l'étape 1034.

On peut ainsi indexer à chaque zone non seulement la ou les hydratation/matification s adéquates mais aussi la ou les hydratation/matification s qui ne vont pas du tout à cette zone.

L'utilisateur peut alors effectuer un nouvel essai sur la même zone en revenant à l'étape 1010.

Si l'utilisateur était satisfait du résultat, il peut également souhaiter effectuer un nouvel essai, par exemple sur une zone différente du visage.

Le cas échéant, si l'utilisateur n'est pas satisfait, l'interface peut lui proposer d'indiquer si le résultat est estimé mieux ou moins bien que l'essai précédent, à l'aide de boutons correspondants 1040 et 1041.

Dans ce cas, le système informatique peut être agencé pour déterminer si au vu des renseignements entrés par l'utilisateur une proposition peut être automatiquement effectuée quant au choix de la nouvelle hydratation/matification à tester.

Le cas échéant, un questionnaire peut s'afficher pour aider le système informatique à proposer une hydratation/matification au vu des essais effectués et de l'évaluation qui en a été faite par l'utilisateur ou par un professionnel qui l'assiste.

Par exemple, si l'hydratation/matification est jugée comme « non adéquate », le système peut recevoir de l'utilisateur une information additionnelle, par exemple « trop gras » ou « trop sec », qui l'aidera à proposer une nouvelle hydratation/matification plus en adéquation avec ce qu'attend l'utilisateur.

Il peut être intéressant que le système informatique puisse recevoir une information de comparaison du résultat par rapports aux essais précédents, par exemple « c'est mieux » ou « c'est moins bien » et que de là, le système soit capable d'en déduire la nouvelle hydratation/matification à proposer.

Une autre option est que le système informatique puisse recevoir une information de comparaison par rapport à une cible, par exemple « c'est presqu'idéal » et que de là, le système soit capable d'ajuster automatiquement ses modifications d'hydratation/matification.

En l'occurrence, s'il reçoit l'information selon laquelle le résultat souhaité est presque atteint, le système peut adopter de petits niveaux de modification de l'hydratation/matification et revoir l'échelle d'hydratation de réglage en conséquence.

Dans le cas où le système de distribution propose de lui-même les mélanges colorés à tester, il peut se fonder sur des scénarios d'essais préprogrammés et modifier le suivi du scénario selon les réussites ou échecs de l'évaluation. Ainsi, par exemple, si dès la troisième application de produit, il reçoit une information selon laquelle l'hydratation/matification est presqu'idéale pour l'utilisateur, le système de distribution peut sortir du programme et se laisser guider ensuite par les instructions de l'opérateur.

D'une façon générale, l'utilisateur peut être aidé par un système expert dans le choix des hydratation/matification s à essayer.

Ce système expert est par exemple un programme exécuté sur l'appareil avec lequel le distributeur communique ou sur le distributeur lui-même, qui se fonde sur des réponses à un questionnaire et/ou sur des mesures, par exemple de l'hydratation/matification de la peau, effectuées par un capteur spécifique ou une caméra. L'utilisateur peut ainsi se faire aider par une évaluation instrumentée, par exemple un capteur d'état de sécheresse de la peau ou de graissage de la peau. Le système expert peut encore être implémenté sur un serveur distant avec lequel l'appareil ou le distributeur échange des informations. L'opérateur peut encore envoyer une image de son visage à un spécialiste qui peut préprogrammer le choix des hydratation/matification s de départ. Dans un autre exemple de mise en oeuvre, l'utilisateur présente au système informatique une photo de son visage, et le système informatique est agencé pour l'analyser et créer un programme définissant les zones à tester et les premiers produits à délivrer, à la fois en hydratation/matification et quantité. Par exemple, le système informatique peut être agencé pour sélectionner automatiquement des hydratation/matification s de maquillage à proposer à l'utilisateur en effectuant l'acquisition d'une photographie à l'étape 1070, comme illustré à la figure 42. Par exemple, l'appareil qui communique avec le distributeur 11 est équipé d'une caméra, et l'utilisateur prend une photographie de son visage. L'image est ensuite analysée à l'étape 1071, et des hydratation/matification s sont proposées pour chaque zone du visage à l'étape 1072, selon par exemple des règles prédéfinies d'accord des hydratation/matifications.

Le système de distribution peut être orienté par l'utilisateur pour décider l'hydratation/matification mais aussi la quantité de produit à délivrer. Par exemple, l'utilisateur peut indiquer « nez » ou « tache » et le système de distribution est agencé pour adapter la dose distribuée selon une cartographie en mémoire des doses à distribuer en fonction des zones à traiter.

Le système informatique peut guider l'utilisateur dans le choix des couleurs du mélange à essayer, afin de limiter le nombre d'essais nécessaires jusqu'à l'obtention d'un résultat plaisant pour l'utilisateur.

Il est ainsi possible, comme illustré à la figure 41, qu'après l'application d'un mélange délivré par le distributeur sur une zone donnée du visage à l'étape 1060, le système informatique demande à l'utilisateur si le résultat est satisfaisant ou non, et effectue de lui-même, si le résultat est considéré comme étant non satisfaisant, un changement 1061 des paramètres du distributeur de façon à modifier le mélange distribué.

L'utilisateur n'a alors qu'a effectuer un nouvel essai avec le mélange modifié.

Lorsqu'un mélange est signalé comme étant satisfaisant, le système informatique peut mémoriser les paramètres correspondants afin de permettre de recréerultérieurement le mélange.

Le système peut alors recommencer les étapes précédentes pour une nouvelle zone d'application.

Lors des essais successifs, l'opérateur n'a pas besoin de traiter tout le visage. Il peut par exemple choisir entre 3 et 8 zones de petites surfaces, par exemple 5 zones. Le système de distribution est alors avantageusement prévu pour interpoler et/ou extrapoler les données concernant les hydratation/matifications considérées comme adéquates, afin de calculer l'hydratation/matification qui devraient être considérées comme adéquates sur des zones sur lesquelles l'exercice n'a pas été fait.

A la fin de l'apprentissage, le système peut généner une visualisation des hydratation/matification s adéquates sur les différentes zones, testées ou calculées.

Le système de distribution peut être agencé pour signaler si certaines hydratation/matification s apparaissent aberrantes, se fiant en cela à une comparaison par rapport à des cartographies types en mémoire. Ainsi, il peut proposer à l'utilisateur de refaire tout ou partie de l'exercice de cartographie.

Une fois l'apprentissage du système informatique effectué, c'est-à-dire que des hydratation/matification s de mélanges ont été identifiées comme convenant à l'utilisateur pour maquiller certaines zones, l'utilisateur qui souhaite se maquiller n'a qu'à rappeler la zone à maquiller, à l'étape 1080 de la figure 43, et le système peut proposer automatiquement à l'utilisateur à l'étape 1081 une hydratation/matification de mélange adéquate.

Dans la variante illustrée à la figure 44, l'utilisateur sélectionne une hydratation/matification à l'étape 1090 et le système informatique propose à l'étape 1091 une zone où appliquer un mélange de cette hydratation/matification, sur la base d'informations précédemment collectées sur la base des tests effectués.

La zone proposée est par exemple celle où une hydratation/matification identique ou proche a déjà été appliquée et le résultat jugé acceptable par l'utilisateur.

La figure 46 illustre un exemple de mise en oeuvre de l'invention où après avoir effectué des tests sur différentes zones à l'étape 2010, l'utilisateur signale au système le ou les mélanges qu'il estime procurer le meilleur résultat, ce qui permet au système de connaître les paramètres correspondants à l'étape 2012. Ensuite, à l'étape 2014, le système peut proposer à l'utilisateur des références de produits commerciaux ayant les mêmes propriétés ou des propriétés proches.

Dans une variante, le système adresse à un centre de fabrication distant les paramètres permettant de produire une composition de même formulation ou ayant les mêmes propriétés que celle du mélange que l'utilisateur a testé et trouvé satisfaisant.

La figure 45 illustre la possibilité de délivrer à l'aide du distributeur plusieurs doses 2020a à 2020d de mélanges différents, de façon juxtaposée sur un support 2021, de façon à permettre leur application sur des régions distinctes voisines d'une même zone.

L'utilisateur peut d'un seul coup appliquer une série de hydratation/matification s afin de cerner rapidement l'hydratation/matification adéquate. Les matières colorées présentes sur le support 2021 peuvent avoir été choisies par l'opérateur lui-même ou proposées par le système de distribution.

Le support 2021 est par exemple mobile par rapport au boîtier du distributeur et déplacé séquentiellement pour déposer les mélanges correspondants dans les différenteszones 2020a à 2020d, étant par exemple similaire aux supports décrits en référence aux figures 29 ou 29A. L'utilisateur peut alors facilement comparer les résultats entre les différentes régions, et signaler au système le mélange produisant le meilleur effet.

On a illustré à la figure 47 un système qui permet d'aider l'utilisateur à se maquiller, notamment à choisir les bons coloris.

Ce système permet l'établissement d'une liaison vidéo, par exemple via internet, entre une caméra 2060 d'un premier site 2061 et un deuxième site 2062.

La caméra 2060 est par exemple intégrée à une tablette ou un téléphone intelligent qui constitue le système informatique 100.

On permet au deuxième site 2062 de piloter directement ou indirectement le distributeur 11 présent sur le premier site 2061.

Ainsi, la personne présente au premier site peut appliquer le mélange distribué et envoyer vers le deuxième site 2062 une image correspondante, pour recevoir en retour une information relative au résultat de maquillage.

Le deuxième site 2062 peut comporter un écran de visualisation 2064 qui permet à un conseiller présent devant cet écran de voir le résultat du maquillage avec le mélange distribué par le distributeur et de conseiller la personne qui s'est maquillée. Ce conseiller peut agir en retour sur le distributeur 11 pour modifier l'hydratation/matification du mélange et l'adapter au mieux au visage de la personne présente sur le premier site. Le protocole d'échange des données entre les deux sites permet ainsi l'envoi d'instructions de commande au distributeur 11, soit directement, soit par l'intermédiaire du systèmeinformatique 100 présent sur le premier site. Ainsi, la personne présente sur le deuxième site gouverne le mélange délivré par le distributeur 11. La première personne peut se maquiller sous les yeux de la seconde. La seconde personne voit sur son écran le résultat de l'essai, et ainsi peut corriger le mélange qu'elle va commander à distance, jusqu'à obtenir le maquillage idéal.

De préférence, la liaison vidéo entre les deux sites est une liaison duplex, de sorte que l'utilisateur présent sur le premier site peut voir sur l'écran du système informatique l'image du conseiller. Ce dernier peut adresser à l'utilisateur présent sur le premier site un tutoriel, le cas échéant.

La mémorisation des paramètres de réglage du distributeur 11, une fois qu'un mélange donné est estimé satisfaisant, peut être commandée depuis le deuxième site.

Le système de distribution peut être agencé pour recevoir la cartographie de quelqu'un d'autre, réel ou virtuel. Il peut aussi combiner la cartographie de la personne avec la cartogaphie d'une autre, pour sublimer le maquillage sans perdre les caractéristiques propres.

L'interface peut servir à définir des programmes de maquillage où l'on définit l'ordre des zones à maquiller ou l'ordre des états d'hydratation/matification à proposer.

### Exemples (huitième aspect de l'invention)

On réalise un distributeur 11 tel que celui illustré à la figure 3. Le distributeur est agencé pour communiquer avec une tablette 100 telle qu'un Ipad. Ce système informatique exécute une application dénommée « µMix » développée dans l'environnement spécifique d'Apple (XCode 4 et Simulateur iOS) en langage Objective C.

Elle utilise des frameworks de base Foundation, UIKit et CoreGraphics, fournissant les outils de manipulation des structures de données, des outils de calcul et les fonctionnalités liées à l'interface graphique utilisateur.

L'application utilise également le framework CoreBluetooth fournissant un accès aux périphériques Bluetoth 4 Low Energy avec les tâches principales suivantes :
- Recherche des périphériques Bluetooth 4.0 Low Energy,
- Connexion/déconnexion et gestion des paramètres de connexion,
- Communication en mode lecture et/ou écriture basée sur l'architecture GATT (Generic Attribute Profile).

L'application propose les fonctionnalités suivantes :
- Définition des fractions de produits de base,
- Choix du mode de fonctionnement lors de l'appui sur le bouton de commande12, à savoir continu, purge ou dose,
- Affichage d'un triangle de fraction volumique tel qu'illustré la figure 30 avec gestion de la fraction volumique par touché tactile sur le triangle ou par les boutons +/associés à chaque produit,
- Connexion/déconnexion Bluetooth et transfert en temps réel des consignes au distributeur,
- Réglages des débits en mode continu, et de la quantité en mode dose,
- Calcul, affichage et transfert au distributeur des fractions volumiques des produits en temps réel, en fonction de la consigne, avec la somme des fractions toujours égale à 100%,
- Récupération et affichage des couples des trois moteurs en temps réel, et
- Sauvegarde des paramètres principaux dans un fichier de configuration.

Le mode continu est un mode de distribution où le mélange des trois produits de base est distribué tant que l'utilisateur appuie sur le bouton de distribution 12. Le produit est délivré avec un débit dont une estimation est affichée au-dessus du bouton 304 « Continu ». Le choix du débit se fait dans un menu « Réglages ».

Le mode « dose » est un mode de distribution du mélange par dose, où la dose est délivrée suite à une impulsion de l'utilisateur sur le bouton de distribution 12. Une impulsion suffit et l'utilisateur peut ensuite relâcher le bouton. La dose globale de produit distribuée est celle indiquée au-dessus du bouton 306 « Dose », 0.1 ml par exemple. Ce volume peut être modifié dans le menu « Réglages ».

Le mode « purge » est un mode de distribution où une dose de mélange à fractions volumiques égales (33%) est délivrée dès que l'utilisateur a appuyé sur le bouton de distribution 12, comme dans le mode « Dose ». Une impulsion suffit et l'utilisateur peut ensuite relâcher le bouton. Quand la dose a été entièrement distribuée, on peut relâcher le bouton. Si l'on relâche avant la fin, la distribution s'arrête, même si le volume spécifié n'est pas atteint. La dose globale de produit distribuée est celle indiquée au-dessus d'un bouton 305 « Purge », 3 ml par exemple. Ce volume peut être modifié dans le menu Réglages.

L'utilisateur détermine l'hydratation/matification voulue avec l'application qui s'éxécute sur la tablette et qui calcule les fractions des différents produits. La tablette communique cette valeur au distributeur par liaison Bluetooth.

L'électronique intégrée au distributeur 11 récupère l'information et ajuste automatiquement les débits des trois cartouches de façon à obtenir un mélange de l'hydratation/matification souhaitée.

Quand l'utilisateur veut utiliser le produit, il appuie sur le bouton 12 du distributeur pour faire sortir le produit. Il presse aussi longtemps qu'il veut du produit, en mode « continu ». En mode « dose », l'utilisateur presse une fois le bouton 12 et la dose prédéfinie est délivrée.

La distribution peut se faire en continu, c'est-à-dire les moteurs fonctionnent en continu, tout le volume étant distribué d'une traite, ou de façon itérative, les moteurs fonctionnant alors de façon impulsionnelle ; dans ce cas, l'intervalle de temps entre deux impulsions permet de faire varier le débit. De petits volumes sont délivrés les uns après les autres en plusieurs étapes.

Les impulsions peuvent être séparées par exemple par des intervalles de 50 ms, 100 ms ou 200 ms. La durée d'une impulsion pendant laquelle le moteur tourne va parexemple de 50 à 150 ms.

La page principale de l'application « µMix » comporte dans cet exemple les éléments suivants, comme visible notamment sur la figure 31 :
- Barre de statut en haut de l'écran : indique l'état de la connexion Bluetooth ou µMix s'il n'y a pas de connexion Bluetooth ;
- Barre d'onglets en bas de l'écran : permet de sélectionner la page active : page principale, Réglages, Bluetooth, Produits et Favoris ;
- Bouton Continu 304 pour sélectionner le mode de distribution de produits en continu ;
- Bouton Purge 305 pour sélectionner le mode Purge ;
- Bouton Dose 306 pour sélectionner le mode de distribution par dose avec le volume de dose associé au bouton Dose ;
- Balle bleue 300 que l'utilisateur peut déplacer dans le triangle volumique en la faisant glisser ou avec un double tapotement ;
- Boutons « - » 302 pour chaque produit A, B et C : diminue la fraction de produit sélectionné en se déplaçant le long de la droite reliant le point au sommet du produit sélectionné ;
- Boutons « + » 302 pour chaque produit A, B et C : augmente la fraction de produit sélectionné en se déplaçant le long de la droite reliant le point au sommet du produit sélectionné ;
- Fraction volumique en pourcentage de chaque produit : modifiable par l'utilisateur et mise à jour en temps réel selon la consigne des boutons 302 + et - et de la position de la balle 300.

Lors de la modification des fractions volumiques par déplacement de la balle ou par les boutons + et -, les valeurs des fractions volumiques des produits A, B et C sont mises à jour automatiquement. Lorsque les fractions volumiques sont modifiées avec les boutons + et -, la balle 300 est déplacée automatiquement à la position correspondante sur le triangle.

Au démarrage de l'application exécutée sur la tablette, celle-ci se connecte automatiquement au distributeur 11 s'il est détecté. Lorsque le distributeur s'éteint ou que la connexion Bluetooth est coupée, la tablette se déconnecte. Quand l'utilisateur agit sur les curseurs de réglage des proportions des produits A et B, les valeurs sont transmises en temps réel au distributeur 11.

La page Réglage de l'application comporte les éléments suivants :
- Barre de statut en haut de l'écran : indique l'état de la connexion Bluetooth ou µMix s'il n'y a pas de connexion Bluetooth ;
- Barre d'onglets en bas de l'écran : permet de sélectionner la page active :
   page principale, Réglages, Bluetooth ou Info ;
- Partie « Volumes » avec un champ de texte à remplir par l'utilisateur pour définir le volume de la dose, en ml (2 ml par exemple) et un champ pour le volume de la purge, en ml (3 ml par exemple). Les doses minimales sont dans cet exemple de 0.023 ml et les doses maximales 9.90 ml (3 x 3.3 ml) ;
- Partie « Débit » avec la sélection du débit : rapide (» 0.03 ml/s), moyen (» 0.02 ml/s) ou lent (» 0.01 ml/s) ;
- Partie « Dose » avec le choix du mélange itératif, pour distribuer un mélange de produits avec des petits volumes délivrés les uns après les autres en plusieurs étapes ;

Dans le cas contraire, le volume total de chaque produit est distribué d'une traite ;
- Partie « Image Triangle » pour choisir l'image du triangle qui sera affichée sur la page principale afin de pouvoir afficher un triangle avec les hydratation/matifications délivrées par le distributeur 11. En utilisant un bouton « Choix image » de la page « Réglages » on accède à un album.

La page « Produits » de l'application comporte dans l'exemple considéré les éléments suivants :
- Choix de la valeur de chaque produit en unités de pas codeurs, de 0 à 1414.
   Chaque unité correspond à un volume de produit délivré de 2.33 µl, qui est la plus petite quantité que le distributeur dans cet exemple peut délivrer ; lorsque cette feuille est affichée, ce sont les valeurs de produits de cette feuille qui sont transmises en temps réel au distributeur. Dès que la feuille n'est plus affichée, les valeurs envoyées au distributeur sont celles de la feuille principale avec le triangle ;
- Affichage des couples moteur A, B et C en temps réel avec rafraîchissement toutes les 45 valeurs.

Le mode de délivrance des produits est le mode dose directe ou itératif, selon l'option choisie dans la feuille Réglage.

La page « Favoris » permet de sauvegarder des configurations en fichier. Elle donne accès dans l'exemple considéré à 10 fichiers, à savoir « Configuration 1 » à « Configuration 10 » en plus du fichier par défaut. Ces fichiers enregistrent par exemple les paramètres suivants
- fractions des produits A, B et C
- volume de Purge
- volume de Dose,
- débit rapide, moyen ou lent,
- mode Dose, Purge ou Continu,
- distribution continue ou itérative.

On réalise plusieurs produits de base (les proportions sont massiques).

Les formules F1 et F2 sont riches en charges (et de couleurs différentes). La formule F3 est riche en agent hydratant.

| | | F1 | F2 | F3 |
|---|---|---|---|---|
| | | % massique | % massique | % massique |
| | Dimethicone copolyol vendu sous la référence KF 6017 par la société Shin Etsu | 2 | 2 | 2 |
| | Etsu Bis PEG/PPG-14/14 dimethicone + Cyclopentasiloxane vendu sous la référence ABIL EM 97 par la société Goldschmidt | 1 | 1 | 1 |
| | Cyclopentasiloxane | 17,65 | 17,65 | 17,65 |
| | Phenyl trimethicone vendu sous la référence DC556 par la société Dow Corning | 2 | 2 | 2 |
| | Ethyl hexyl methoxycinnamate | 3 | 3 | 3 |
| | Squalane | 1 | 1 | 1 |
| | Dispersion d'oxychlorure de bismuth dans hydroxystéarate d'éthyl (2) hexyle 70 :30 (Timiron Liquid Silver^{®} de Merck) | 0 | 0 | 0 |
| | Cyclopentasiloxane | 7 | 7 | 7 |
| | Oxyde de fer faune enrobé de stearoyl glutamate d'aluminium NAI-C33-9001-10 de la société Miyoshi Kasei | 1,65 | 1,25 | 1,45 |
| | Oxyde de fer rouge enrobé de stearoyl glutamate d'aluminium NAI-C33-8001-10 de la société Miyoshi Kasei | 0,3 | 0,5 | 0,4 |
| | Oxyde de fer noir enrobé de stearoyl glutamate d'aluminium NAI-C33-7001-10 de la société Miyoshi Kasei | 0,15 | 0,15 | 0,15 |
| | Dioxyde de titane (anatase) enrobé de stearoyl glutamate d'aluminium NAI-TAO-77891 de la société Miyoshi Kasei | 9,9 | 10,1 | 10 |
| | Talc vendu sous la référence Micro Ace P3 par la société Nippon Talc | 0,5 | 0,5 | 0,25 |
| | Poudre de nylon 12 vendue sous la référence SP 500 par la société Toray Industries | 0,5 | 0,5 | 0,25 |
| | Eau déminéralisée | 36,15 | 36,15 | 34,65 |
| | Glycérine | 0 | 0 | 5 |
| | 1,3-Butylene glycol | 3 | 3 | 3 |
| | Sulfate de magnésium | 0,7 | 0,7 | 0,7 |
| | Solution de maltose hydrogénée | 0,5 | 0,5 | 0,5 |
| | Alcool éthylique 96° dénaturé | 13 | 13 | 10 |
| | TOTAL | 100 | 100 | 100 |

### Mode opératoire

On pèse les constituants de la phase A dans le bêcher principal et l'on agite en maintenant à température ambiante sous Moritz (1000 tr/min). La phase B est préparée séparément en broyant trois fois à la tricylindre, le mélange de pigments et de cyclopentasiloxane. Cette phase B est ensuite ajoutée en maintenant l'agitation, ainsi que les charges (phase C).

La phase aqueuse D est aussi préparée séparément, en pesant dans un bêcher la glycérine, le butylène glycol, le sulfate de magnésium, la solution de maltose hydrogénée et l'eau à température ambiante. La phase aqueuse D est placée sous agitation à l'aide d'un barreau magnétique jusqu'à homogénéisation. C D E L'émulsion se fait à température ambiante : on verse la phase aqueuse D dans la phase grasse en augmentant progressivement la vitesse d'agitation (Moritz) jusqu'à 4000 tours/mn. On maintient l'agitation pendant 7 mn. Puis on ajoute enfin la phase E (alcool éthylique) pendant les 3 dernières minutes de 5 l'émulsification.

### Test 1

Le système a été testé avec :
- F1 dans le compartiment A
- F2 dans le compartiment B
- F3 dans le compartiment C

On utilise le système pour réaliser plusieurs mélanges :

| | |
|---|---|
| M1 : | A 30%, B 0%, C 70% |
| M2 : | A 70%, B 0%, C 30% |
| M3 : | A 50%, B 0%, C 50% |

M1, M2, M3 sont appliquées sur différentes zones du visage : M1 sur les zones sèches telles que les joues, M2 sur les zones grasses comme le front, et M3 sur les zones intermédiaires comme le menton.

### Test 2

On utilise le système pour réaliser plusieurs mélanges :

| | |
|---|---|
| M1 : | A 80%, B 10%, C 10% |
| M2 : | A 35%, B 35%, C 30% |
| M3 : | A 20%, B 20%, C 60% |

M1 et M2 est destinée aux zones grasses. M1 assez jaune, est appliquée sur les poches. M2, plus rosée, est appliquée sur les ailes du nez.

M3 est appliquée sur les zones très sèches telles que le dessus de la bouche.

### I) Système pour réaliser des mélanges sur mesure à base de formulation alcoolique

L'invention s'intéresse plus particulièrement selon ce neuvième aspect aux procédés et systèmes de distribution de produits fortement concentrés en alcool.

Elle concerne plus précisément la réalisation de parfums (concentrés, eaux de parfums, eaux de toilettes...), produits de parfumage et déodorants.

Ces produits servent à se parfumer pour le plaisir de l'odeur ou pour masquer des odeurs désagréables (corporelles).

Les utilisateurs demandent à avoir de nombreux parfums, avec différentes notes et intensités. Pour cela, le professionnel réalise et commercialise des mélanges de différents ingrédients, réalisant ainsi des notes qui plaisent à un grand nombre de gens. Mais, l'évolution de la société pousse chacun à vouloir disposer de son parfum, avec des notes et intensités réglés sur mesure. Les utilisateurs peuvent faire des mélanges sur mesure mais ceci répond mal au besoin de changer régulièrement. De plus, on voudrait pouvoir proposer tout un choix de facettes odorantes. Or, on s'aperçoit que quand on fait un mélange sur mesure, utilisant des compositions alcooliques, il y a un risque que le mélange ne corresponde pas à l'odeur programmée si les différents ingrédients ou préparations d'ingrédients ne sont pas bien dosées. Une erreur dans les proportions peut provoquer un écart olfactif notable, voire inacceptable.

Une erreur est d'autant moins acceptable que la personne a dû payer assez cher l'élaboration de son parfum sur mesure (ou passer du temps s'il a voulu le réaliser lui-même) et se retrouve donc déçu que l'odeur ne corresponde pas à l'odeur prévue. Par exemple, s'il dispose d'une ancienne préparation, il se rendra très vite compte de l'écart en réalisant une comparaison. On sait que ce problème n'apparait pas à chaque fois qu'on réalise un mélange mais le risque est là de se retrouver avec des clients insatisfaits. Des réalisations sur de grands volumes pourraient limiter ces risques mais ils ne sont ni économiques, ni raisonnables étant entendu que les quantités utilisées sont souvent limitées (par exemple, 200 mg par jour) car les utilisateurs apprécient de changer de recette à chaque utilisation ou souvent.

Ce problème est d'autant plus grand que les ingrédients ou bases d'ingrédients utilisés sont différents les uns des autres (sur l'odeur).

On pourrait se limiter au mélange d'ingrédients ou bases d'ingrédients proches (sur le plan olfactif), mais ceci réduit fortement le panorama des parfums possibles.

Ainsi, aucune des solutions existantes n'est satisfaisante, limitant à ce jour le développement des parfums sur mesure.

L'invention vise, selon son neuvième aspect, à répondre à ce problème, et a pour objet un système de distribution d'un produit parfumé, comportant un distributeur recevant au moins deux cartouches contenant respectivement des premier et deuxième produits de base, le premier produit de base comportant une composition contenant au moins un alcool et au moins un dérivé cellulosique jouant le rôle d'un épaississant, le distributeur permettant de délivrer au moins ces deux produits de base dans des proportions réglables.

L'invention selon ce neuvième aspect peut présenter une ou plusieurs des caractéristiques suivantes :
- chaque produit de base comportant une composition contenant au moins un alcool et au moins un dérivé cellulosique,
- le premier produit de base ou chaque produit de base a une concentration massique en alcool par rapport au(x) autre(s) composé(s) non alcoolique(s) du premier produit d'au moins 80/20, mieux 85/15 et encore mieux 90/10,
- le premier produit de base ou chaque produit de base contient au moins 50% en masse d'éthanol dans sa phase solvant, mieux au moins 80% en masse d'éthanol dans sa phase solvant, encore mieux au moins 85% en masse d'éthanol dans sa phase solvant,

- le premier produit de base ou chaque produit de base contient au moins un composé odorant, de préférence choisi parmi les esters, carbonates, acides, anhydrides, aldéhydes, alcools, les composés aliphatiques sans fonctions autres qu'alcanes et alcènes, et leurs mélanges,
- le ou les composés odorants sont choisis parmi l'hexanal, l'octanal, le nonanal, le decanal, l'undecanal, le dodecanal, le tridecanal, le 2-méthyl décanal, 2-méthyl undecanal, le trans-2-hexenal, le cis-4-heptanal, le 2,6 diméthyl 5-hepten 1 al, le E-4-décenal, le 10 undecenal, le 2 dodecenal, le 1,1 diméthoxy 2,2,5 triméthyl 4 hexene, l'acide 2 méthyl 2 pentenoique, l'acide (S)-(+)-2-méthyl butanoïque, l'ethyl formate, cis-3- hexenyl formate, l'ethylacetate, le butylacetate, l'isoamyleacétate, l'hexylacétate, le 3,5,5 triméthyl hexylacétate, le trans 2 hexenyl acetate, le cis -3-hexenylacetate, l'ethylpropionate, l'ethylbutyrate, le butylbutyrate, l'isoamylbutyrate, l'hexylbutyrate, le cis 3 hexenylisobutyrate, l'ethylisovalérate, l'éthyl 2 méthylbutyrate, l'ethyl hexanoate, l'ethyl 2 méthylpentanoate, le 2 propenyl hexanoate, l'ethylheptanoate, le 2 propenyl heptanoate, l'ethyloctanoate, le methyl 2 nonenoate, l'ethyl 2 trans 4 cis decadienoate, le methyl 2 octynoate, le méthyl 2 nonynoate, l'ethyl3 octobutanoate, l'allyl amylglycolate, le Z 3 hexenylmethyl carbonate, le3-octanol ;le 2,6 diméthyl 2 heptanol, le trans 2 hexen-1 ol ; le 3 hexen-1-ol ; le 1-octen-3-ol ; le 9-decen-1-ol ; le 10 undecen-1-ol ; le 2-trans-6-cis Nonadien-1-ol ; le 4-methyl-3-decen-5-ol, le myrcène, l'ocimène, le béta Farnesene, le citral, le citral diethylacetate, le citronellal, le methoxydihydrocitronellal, le 2,6, 10 trimethyl 9 undecanal, l'acide cis-geranique, l'acide citronellique, le geranyl ester (formate, acétate, propionate, isobutyrate, isovalerate), le neryl acetate, le linalyl esters (formate, acétate, propionate, isobutyrate), les Citronellyl esters (formate, acétate, propionate, isobutyrate, isovalerate, tiglate) et les esters du myrcenol, le Géraniol, nérol, linalool, myrcenol, lavendulol, citronelol, trans trans farnesol, trans-nerolidol, tetrahydrogeraniol, tetrahydrolinalool, l'avendulol, le trans trans farnesol, le trans-nerolidol, le tetrahydrogeraniol, le tetrahydrolinalool, le limonène, le terpinene, le terpinolene, le phellandrene, le camphene, le 3-carène, le menthyl ester (acétate, lactate), les alpha-terpinyl esters (acétate), les Noryl esters (acétate), les Bornyl esters (acétate), les isobornyl esters (acétate), les cedryl ester (acétate), le 2,4-diméthyl-3-cyclohexene carboxaldehyde, le 4-(4-hydroxy-4-methylpentyl)-3 cyclohexene carboxaldehyde, le 1-(4 isopropycyclohexyl) ethanol, 2-méthyl-4-(2,2,3 trimethyl-3-cyclopentene-1-yl) butanol, 2-méthyl-4-(2,2,3 trimethyl-3-cyclopentene-1-yl) butenol, 3-méthyl-5-(2,2,3 trimethyl-3-cyclopentene-1-yl) 4-penten-2-ol, 3,3-diméthyl-5-(2,2,3 trimethyl-3-cyclopentene-1-yl) 4-penten-2-ol, l'Indole, le p-cymène, le diphénylméthane, le benzaldehyde, le phenylacetaldehyde, le phénylacetaldehyde dimethyl acetal, Dihydrocinnamaldehyde, le 2-phenylpropanal, le cyclamenaldehyde, le 2-methyl-3-(4-tert-butyl-phenyl) propanal, le cinnamaldehyde, l'heliotropine, le furfuraldehyde, les benzyl esters (acetate, propionate, isovalerate), les phenethyl ester (acetate, isobutyrate, isovalerate), l'alpha-trichloromethylbenzyl ester (acetate), le cinnamylacetate, le benzoate ester (acetate, hexyl, benzyl), le phenylacetate ethyl, le phenylacetate geranyl,le methylcinnamate, le benzylcinnamate, le phenylethylcinnamate, l'eugenol acetate, l'acide phenylacetique, l'alcool benzylique, 2 phenylethyl alcool, styrallyl alcool, , 2,2 dimethyl -3-(3-methylphenyl) propanol, alcool cinnamique, 3 methyl-5-phenylpentanol, thymol, anethole, isoeugenol, eugenol, anise alcool, raspeberry ketone, ethylmaltol, 2,6-dimethoxyphenol_, 2-propyl phénol, 2-(methyl thio) phenol_, ortho-guaiacol_, 4-methyl guai anhydride abietic, l'anhydride citraconic,
- le dérivé cellulosique est choisi parmi les dérivés de cellulose obtenus par réaction de cellulose alcalinisée avec de l'oxyde de propylène ou d'éthylène,
- le dérivé cellulosique est de l'hydroxypropylcellulose,
- la taille en poids moléculaire du dérivé cellulosique est supérieure ou égale à 10000, mieux est comprise entre environ 850 000 et environ 1 150 000,
- la teneur massique totale en composé(s) cellulosique(s) varie entre 0,1 % et 20 %, mieux entre 0,5 % et 5%, encore mieux entre 0.7% et 2%, le pourcentage étant exprimé par rapport à la masse du produit de base,
- Le système comporte une troisième cartouche avec un troisième produit de base,
- les cartouches sont reçues de façon amovible dans le distributeur,
- chaque produit quitte la cartouche par un canal de sortie de la cartouche, le canal de sortie étant défini par un embout de distribution de la cartouche entraîné en rotation relative par rapport à un corps de la cartouche par un mécanisme d'entraînement du distributeur pour distribuer le produit de base contenu dans la cartouche,
- Le système de distribution comporte une mémoire électronique pour enregistrer en association avec un produit distribué les proportions respectives en chacun des produits de base de ce produit, en vue de pouvoir automatiquement redistribuer ce produit ultérieurement.

L'invention a encore pour objet selon son neuvième aspect un procédé pour générer un produit parfumé, à l'aide d'un système selon l'une quelconque des revendications précédentes, dans lequel on sélectionne un ou plusieurs produits de base contenus dans des cartouches respectives du distributeur, et l'on distribue les produits de base sélectionnés dans des quantités choisies.

On peut mémoriser en outre les proportions des différents produits de base composant le produit distribué.
1) L'invention, selon son neuvième aspect, repose ainsi de façon préférentielle sur un système de distribution comportant Un distributeur programmable avec plusieurs compartiments
2) Lequel est apte à délivrer de toutes petites quantités de chaque compartiment avec précision.
3) Au moins une composition contient de l'alcool (en particulier de l'éthanol), avec ou sans composés odorants, et un agent du type dérivé cellulosique du type « Hydroxypropylcellulose »
4) D'autres compartiments peuvent optionnellement exister contenant d'autres compositions qu'alcooliques.

Grâce à l'invention selon ce neuvième aspect, la personne pourra :
a) Concevoir des parfums, les enregistrer
b) Faire réaliser une petite quantité pour son utilisation et ce sans risque d'écart par rapport à l'odeur prévue
c) Et ce en utilisant le panorama complet des ingrédients olfactifs

La personne pourra, même si les réservoirs sont épuisés, voire évantés, recharger les compartiments de nouvelles cartouches puis faire réaliser une petite quantité pour son utilisation.

La personne pourra également partager avec d'autres, même à distance, son parfum.

Ce système pourra par ailleurs être utilisé pour réaliser des quantités plus importantes étant entendu que ces quantités devront être considérées une fois réalisées comme à durée limitée (entre 1 mois et 48 mois d'habitude).

### Système de distribution

Le système de distribution peut être constitué par un seul appareil fonctionnant de manière autonome, de préférence manipulable d'une main, ou par un appareil qui fonctionne en interagissant avec d'autres pièces ou appareils. Il peut s'agir par exemple de différentes interfaces de sorties qui sont montées sur le distributeur en fonction du type de note à réaliser, comme cela sera précisé plus loin. Il peut s'agir également d'un système informatique qui échange des informations avec le distributeur pour le piloter, ce système informatique comportant par exemple un terminal portable tel qu'un téléphone intelligent, un photophone, une tablette, un ordinateur personnel, ou un terminal dédié.

De préférence, le distributeur est agencé pour mettre sous pression un ou plusieurs compartiments contenant le ou les produits de base, par le biais de dispositifs de dosage volumétrique, de préférence un moteur entraînant en déplacement un piston dans le compartiment correspondant.

Le distributeur peut être formé d'un boîtier et d'au moins deux ou trois compartiments, et de préférence d'autant de moteurs. Par exemple, la rotation des moteurs entraîne des vis sans fin, lesquelles poussent les pistons de chaque compartiment. L'avance des pistons est par exemple contrôlée par le nombre d'impulsions de commande envoyées aux moteurs et/ou par la durée de fonctionnement de ces derniers. Les moteurs peuvent être alimentés séquentiellement, ou de préférence simultanément.

Par exemple, les moteurs sont alimentés durant un cycle élémentaire de fonctionnement pendant une courte durée les uns après les autres, ou les uns en même temps que les autres, de façon à distribuer des microdoses correspondantes.

Les cycles élémentaires sont répétés, avec éventuellement un temps d'arrêt entre eux, qui laisse le temps aux produits de base de s'écouler hors des compartiments.

Les compartiments peuvent être définis par des cartouches qui se retirent lorsqu'elles sont vides. En variante, les compartiments sont présents à demeure et remplis à nouveau une fois vide.

Chaque cartouche peut être fermée par un bouchon démontable pour permettre le nettoyage de la cartouche.

De préférence, le boîtier du distributeur est de forme allongée selon un axe longitudinal, ce qui peut faciliter sa manipulation, et les cartouches sont disposées autour de cet axe, dans le boîtier.

De préférence, les cartouches sont mises en place par l'arrière, et le mélange est délivré par l'avant. La mise en place des cartouches peut s'effectuer individuellement, ou en variante les cartouches constituent un ensemble monobloc lors de leur mise en place.

Les cartouches peuvent comporter chacune un mécanisme de dosage volumétrique, comportant un piston mu par un mécanisme d'entraînement du distributeur, dans une direction s'accompagnant d'une diminution du volume intérieur contenant le produit de base et d'une expulsion du produit. Il peut être intéressant que les cartouches aient une région de leur paroi au moins qui soit transparente, afin de permettre de voir la couleur du produit contenu à l'intérieur.

Le mécanisme d'entraînement peut comporter un système de motorisation formé de moteurs couplés à des réducteurs, de forme allongée parallèlement à l'axe longitudinal du distributeur, et disposés entre les cartouches. Ce positionnement des moteurs et cartouches rend le distributeur particulièrement compact.

Le produit de base peut sortir de façon étanche de la cartouche correspondante puis circuler dans un canal prévu à cet effet dans le boîtier du distributeur, avant de sortir de celui-ci.

Les cartouches se terminent avantageusement par un embout réalisé de telle sorte que, une fois la cartouche insérée dans le boîtier du distributeur, l'extrémité de l'embout débouche au ras du boîtier. En variante, l'embout est suffisamment long pour dépasser du boîtier et connecter ainsi différentes interfaces de sortie susceptibles d'être rapportées sur le boîtier du distributeur.

Grâce au mécanisme d'entraînement comportant des moteurs pour faire avancer des pistons, il est possible de délivrer de façon précise des mélanges en de très petites quantités. Ainsi, le mécanisme d'entraînement peut délivrer les produits de base avec à un débit minimal inférieur ou égal à 50 µL/s, mieux inférieur ou égal à 20 µL/s, encore mieux inférieur ou égal 10 µL/s. De préférence, le mécanisme d'entraînement délivre des débits compris entre 20 et 100 µL/s, mieux compris entre 40 µL/s et 60 µL/s. Il est donc possible de réaliser facilement un mélange d'environ 10 mg. Un tel système de distribution est alors idéal pour réaliser de petites touches odorantes
Il est aussi possible de réaliser des plus grandes quantités de mélange comme celles nécessaires au parfumage sur un ou quelques jours. Ces quantités restent néanmoins relativement faibles inférieures à 1000 mg, par exemple une quantité comprise entre 10 et 500 mg, mieux comprise entre 40 et 250 mg.

L'invention a ainsi pour objet, selon l'un de ses aspects, un système de distribution comportant un distributeur présentant un boîtier, et au moins une cartouche reçue dans le boîtier du distributeur, cette cartouche comportant un corps et un piston mobile dans le corps, le boîtier comportant un mécanisme d'entraînement motorisé pour déplacer le piston de la cartouche.

De préférence, la cartouche comporte un embout de distribution par lequel s'effectue la sortie du produit, et cet embout de distribution est entraîné en rotation par le mécanisme d'entraînement pour déplacer le piston. L'embout peut comporter au moins un relief d'anti-rotation, mieux deux ergots d'anti-rotation diamétralement opposés.

L'embout peut porter un joint d'étanchéité, notamment un joint torique. Ainsi, au changement de cartouche, le joint est également changé, ce qui permet de s'affranchir de l'usure du joint.

Le distributeur peut comporter une carte électronique de pilotage du mécanisme d'entraînement motorisé, cette carte électronique étant traversée par le ou les embouts. Cela peut permettre de réaliser une carte s'étendant sensiblement sur toute la section du distributeur, donc de regrouper sur une seule carte tous les composants électroniques du distributeur, et ainsi de gagner en compacité et fiabilité. La carte peut s'étendre sensiblement perpendiculairement à l'axe longitudinal du boîtier. La carte peut porter un interrupteur de commande du fonctionnement du distributeur.

Le système de distribution peut être agencé pour fonctionner selon au moins deux modes de distribution.

Dans un premier mode, dit « continu », le mélange est distribué tant qu'une pression est exercée sur l'interrupteur de commande.

Dans un deuxième mode, dit « dose », une quantité prédéfinie du mélange est distribuée à chaque pression sur l'interrupteur.

Le ou les embouts peuvent déboucher à une extrémité du boîtier. Cela peut permettre de réduire le volume mort, comme cela sera détaillé dans la suite.

Le ou les embouts peuvent présenter à leur extrémité un système d'obturation pour éviter le séchage des produits dans le conduit, par exemple une membrane cicatrisante

La cartouche peut comporter une vis creuse sur laquelle le piston est vissé, le piston pouvant se déplacer axialement sur la vis lorsque celle-ci tourne ; le piston est empêché de tourner dans le corps de la cartouche. Par exemple, la friction du piston sur le corps de la cartouche peut suffire à l'empêcher de tourner quand la vis tourne.

Préférentiellement, la rotation est rendue impossible avec un corps de cartouche de section non circulaire et un piston non déformable.

Le couple des moteurs peut être déterminé électroniquement en fonction du courant absorbé, et servir par exemple à détecter une fin de course du piston. Une information concernant le couple peut être transmise à distance à un système informatique présentant une interface homme machine, pour permettre de surveiller le bon fonctionnement du distributeur.

Afin d'ajuster la note, le système de distribution selon l'invention doit permettre à l'utilisateur de faire varier le volume délivré provenant de chaque compartiment.

De façon préférée, le distributeur est piloté par un système informatique, qui est intégré au distributeur, ou externe à celui-ci, le distributeur étant alors apte à échanger avec le système informatique des informations par protocole sans fil ou câblé.

Le distributeur peut ainsi être piloté pour permettre l'ajustement de la note par distribution simultanée ou séquentielle, et contrôlée, de plusieurs produits de base odorants différentes.

La distribution des produits de base peut être continue ; si dans ce cas, les volumes de chacun des produits de base sont distribués d'une seule traite, simultanément ou successivement.

Dans le cas d'une distribution simultanée, il est utile de pouvoir ajuster les débits respectifs des différents produits de base pour que le mélange distribué corresponde à celui souhaité à chaque instant. Un tel mode de distribution peut convenir en particulier lors de la distribution du mélange par pulvérisation, à l'aide d'un aérographe. Pour ajuster les débits il est possible par exemple de jouer sur la vitesse de déplacement des pistons, par exemple, dans le cas d'un entrainement des pistons par une vis sans fin en faisant varier la vitesse de rotation des moteurs qui entrainent la vis. Les produits peuvent aussi être distribués de façon impulsionnelle avec un temps de distribution et un temps d'arrêt à chaque cycle. En jouant sur le rapport cyclique, il est possible d'agir sur le débit.

Tous les produits peuvent être délivrés simultanément lors du temps de distribution ; en variante, les cycles des différents produits sont déphasés, de sorte qu'un produit est distribué pendant un temps d'arrêt des autres produits.

Dans un mode particulier de l'invention, le mélange est délivré dans une cavité d'un contenant, pouvant fermer de façon hermétique ou non, par exemple en forme de coupelle, dans lequel on peut faire glisser un applicateur, en particulier un stylet ou une brosse. Un tel système de distribution est spécialement adapté aux formules qu'on voudrait appliquer sans contact direct avec les mains. Ce contenant peut être amovible.
- Lorsqu'il n'est pas amovible, le contenant peut être réalisé avec le corps du distributeur. Lorsqu'il est amovible, il peut constituer une interface de sortie parmi d'autres pouvant être montées sur le distributeur.

Les compartiments, et en particulier les cartouches, peuvent contenir tout ou partie du mécanisme d'entraînement, et par exemple le système de motorisation, ou mieux, une partie du système de motorisation, et ce, afin de réduire le nombre de pièces en mouvement dans le corps du distributeur à l'extérieur des cartouches. Par exemple, les cartouches comportent le rotor du moteur. Une fois les cartouches installées dans le corps du distributeur, les rotors sont mis en interaction avec les stators.

Le système de distribution est avantageusement agencé pour permettre de lancer des séquences préprogrammées dans lesquelles le mélange délivré par celui-ci est modifié de façon continue ou discontinue. Un mode « dégradé » permet par exemple passer progressivement d'un mélange A à un mélange B. Dans le cas d'une application par pulvérisation, notamment à l'aide d'un aérographe, cela permet de réaliser simplement les dégradés d'odeurs. Un mode « Alternatif » permet par exemple de passer rapidement d'un mélange A à un mélange B plusieurs fois de suite. Dans le cas d'une application par pulvérisation, on peut ainsi réaliser un dépôt multicouche, avec des formulations différentes pour deux couches adjacentes superposées. Un autre mode permet par exemple de proposer plusieurs mélanges successifs préprogrammés, le système informatique indiquant à chaque fois l'usage qu'il faut en faire à l'utilisateur, par exemple par affichage sur un écran.

Dans le cas d'une application manuelle, les mélanges sont distribués par exemple dans une coupelle. La personne s'applique le mélange à l'endroit recommandé avec un mélange correspondant prélevé dans la coupelle, puis nettoie éventuellement la coupelle et commande la délivrance d'un nouveau mélange ; l'opération est répétée autant de fois que nécessaire pour se parfumer complètement.

L'homogénéisation des mélanges distribués peut se faire de différentes façons selon les cas d'utilisation. Dans le cas d'une application manuelle, elle peut se faire directement sur la zone d'application au moment de l'application ou dans la coupelle avant prélèvement ; dans le cas d'une application à l'aérographe, c'est la tuyère de l'aérographe qui sert de chambre de mélange ; dans le cas où le mélange est distribué dans un contenant en vue d'une utilisation ultérieure, l'homogénéisation peut être faite à la main ou en faisant passer les produits distribués dans une chambre de mélange, située entre le distributeur et le contenant ou directement intégrée au contenant, comme détaillé par la suite.

Le produit peut être délivré par le système de distribution et utilisé extemporanément. En variante, le produit délivré par le système de distribution est conditionné et utilisé ultérieurement, par exemple à plusieurs reprises, avec par exemple un jour d'intervalle au moins entre deux utilisations.

### Solvants des compositions

La composition ou les compositions alcooliques contiennent des alcools primaires ou non, à une concentration par rapport à l'eau ou autres solvants organiques non alcooliques d'au moins 80/20, de préférence d'au moins 85/15, encore de préférence d'au moins 90/10. Les alcools contiennent de préférence au moins 50% en masse d'éthanol par rapport à la masse des aux autres alcools, préférentiellement au moins 80% en masse d'éthanol par rapport à la masse des aux autres alcools, encore mieux au moins 85% en masse d'éthanol par rapport à la masse des aux autres alcools. Les autres alcools sont typiquement des glycols et/ou alcools type C3 ou plus, en particulier de l'isopropanol.

Les autres solvants non alcooliques sont typiquement de l'acétone ou éthers, tels qu'éthers de glycols.

De préférence, la composition contient dans sa phase solvant : Au moins 80% en masse d'éthanol, et entre 0 et 10% en masse d'eau et entre 0% et 20% en masse d'un autre solvant organique non alcoolique.

La composition ou les compositions alcooliques contiennent ou non d'autres composés considérés comme « non solvants ».
- Composés odorants
- Composés de soin, tels que filtres, agents biologiques
- Colorants
- Polymères

### Composés odorants

Dans le sens « composé odorant », on entend les molécules odorantes et celles qui ont un effet (exaltant, modifiant) sur les molécules odorantes. On comprend aussi les stéroïdes qui peuvent aussi jouer sur l'odeur finale du mélange.

On considère deux types de composés odorants.
- Esters
- Carbonates
- Acides
- Anhydrides
- Aldéhydes
- Alcools
- Composés aliphatiques sans fonctions autres qu'alcanes et alcènes

### Plus précisément, les composés sont aliphatiques :

- Alcanes et alcènes

### (E,Z)-1,3,5-undecatriène

- Aldéhydes

Hexanal, Octanal, Nonanal, decanal, undecanal, dodecanal, tridecanal, 2-méthyl décanal, 2-méthyl undecanal, trans-2-hexenal, cis-4-heptanal, 2,6 diméthyl 5-hepten 1 al, E-4-décenal, 10 undecenal, 2 dodecenal, 1,1 diméthoxy 2,2,5 triméthyl 4 hexene.
   - Acides
Acide 2 méthyl 2 pentenoique, Acide (S)-(+)-2-méthyl butanoïque
   - Esters
Ethyl formate, cis-3- hexenyl formate, Ethylacetate, Butylacetate, isoamyleacétate, hexylacétate, 3,5,5 triméthyl hexylacétate, trans 2 hexenyl acetate, cis -3- hexenylacetate, ethylpropionate, ethylbutyrate, butylbutyrate, isoamylbutyrate, hexylbutyrate, cis 3 hexenylisobutyrate, ethylisovalérate, éthyl 2 méthylbutyrate, ethyl hexanoate, ethyl 2 méthylpentanoate, 2 propenyl hexanoate, ethylheptanoate, 2 propenyl heptanoate, ethyloctanoate, methyl 2 nonenoate, ethyl 2 trans 4 cis decadienoate, methyl 2 octynoate, méthyl 2 nonynoate, ethyl3 octobutanoate, allyl amylglycolate
   - Carbonates
Z 3 hexenylmethyl carbonate
   - Alcools
3-octanol ;2,6 diméthyl 2 heptanol, trans 2 hexen-1 ol ; 3 hexen-1-ol ; 1-octen-3-ol ; 9-decen-1-ol ;10 undecen-1-ol ; 2-trans-6-cis Nonadien-1-ol ; 4-methyl-3-decen-5-ol

### Les composés sont des dérivés de terpene non cycliques

- Alcanes et alcènes

Myrcène, ocimène, béta Farnesene
   - Aldéhydes et dérivés acétals
Citral, citral diethylacetate, citronellal, methoxydihydrocitronellal, , 2,6, 10 trimethyl 9 undecanal,
   - Acides
Acide cis-geranique, acide citronellique,
   - Esters
Geranyl ester (formate, acétate, propionate, isobutyrate, isovalerate), neryl acetate, linalyl esters (formate, acétate, propionate, isobutyrate), Citronellyl esters (formate, acétate, propionate, isobutyrate, isovalerate, tiglate) et esters du myrcenol, lavendulol, trans trans farnesol, trans-nerolidol, tetrahydrogeraniol, tetrahydrolinalool,
   - Alcools
Géraniol, nérol, linalool, myrcenol, lavendulol, citronelol, trans trans farnesol, trans-nerolidol, tetrahydrogeraniol, tetrahydrolinalool,

### Les composés sont des dérivés de terpene cycliques

- Alcanes et alcènes

Limonène, terpinene, terpinolene, phellandrene, camphene, 3-carène
   - Esters
Menthyl ester (acétate, lactate), alpha-terpinyl esters (acétate), Noryl esters (acétate), Bornyl esters (acétate), isobornyl esters (acétate), cedryl ester (acétate)
   - Alcools
Menthol et différents diastéréoisomères (neomenthol, isomenthol, néoisomenthol) pulegol, et différents diastéréoisomères, piperitone, terpinéols et différents isomères, borneol,

### Les composés sont des dérivés cycliques hors terpenes

- Alcanes et alcènes
- Aldéhydes et dérivés acétals

2,4-diméthyl-3-cyclohexene carboxaldehyde, 4-(4-hydroxy-4-methylpentyl)-3 cyclohexene carboxaldehyde,
   - Esters
OTBCHA, PTBCHA, allyl 4-cyclohexylpropionate, allyl cyclohexyloxyacetate, , méthyljasmonate, methyldihydrojasmonate,
   - Alcools
1-(4 isopropycyclohexyl) ethanol, 2-méthyl-4-(2,2,3 trimethyl-3-cyclopentene-1-yl) butanol, 2-méthyl-4-(2,2,3 trimethyl-3-cyclopentene-1-yl) butenol, 3-méthyl-5-(2,2,3 trimethyl-3-cyclopentene-1-yl) 4-penten-2-ol, 3,3-diméthyl-5-(2,2,3 trimethyl-3-cyclopentene-1-yl) 4-penten-2-ol

### Les composés sont des dérivés aromatiques (benzenique ou hétérocycliques)

- Cycles aromatiques (benzèniques et hétérocycles) sans fonctions supplémentaires

Indole, p-cymène, diphénylméthane
   - Aldéhydes et dérivés acétals
Benzaldehyde, phenylacetaldehyde, phénylacetaldehyde dimethyl acetal, Dihydrocinnamaldehyde, 2-phenylpropanal, cyclamenaldehyde, 2-methyl-3-(4-tert-butyl-phenyl) propanal, cinnamaldehyde, heliotropine, furfuraldehyde,
   - Esters
Benzyl esters (acetate, propionate, isovalerate), phenethyl ester (acetate, isobutyrate, isovalerate), alpha-trichloromethylbenzyl ester (acetate), cinnamylacetate, benzoate ester (acetate, hexyl, benzyl), phenylacetate ethyl, phenylacetate geranyl,methylcinnamate, benzylcinnamate, phenylethylcinnamate, eugenol acetate,
   - Acides
Acide phenylacetique
   - Alcools
Alcool benzylique, 2 phenylethyl alcool, styrallyl alcool, , 2,2 dimethyl -3-(3-methylphenyl) propanol, alcool cinnamique, 3 methyl-5-phenylpentanol, thymol, anethole, isoeugenol, eugenol, anise alcool , raspeberry ketone, ethylmaltol, 2,6-dimethoxyphenol , 2-propyl phénol, 2-(methyl thio) phénol, ortho-guaiacol , 4-methyl guaiacol

### Autres:

- anhydride abietic
- anhydride citraconic

### Dérivé cellulosique

Ils sont obtenus par réaction de cellulose alcalinisée avec de l'oxyde de propylène ou d'éthylène. Le taux de substitution des oxydes d'alkylène vis-à-vis des hydroxyls de la cellulose est typiquement supérieur à 2, mieux supérieure à 4.

Les tailles en poids moléculaire varient de 10.000 à plusieurs millions, préférentiellement de 70.000 à 1.500.000 déterminées par chromatographie d'exclusion.

Par exemple, le dérivé cellulosique est un Klucel^{®} (Ashland) vendus sous la dénomination :
H →1.150.000
M →850.000
G -*370.000
J →140.000
L →95.000
E -*80.000

Tous les grades de pureté sont utilisables :
- Industrial
- F
- CS
- F pharm

Préférentiellement, on emploie les tailles de plus hautes valeurs telles que M et H.

La teneur massique totale en composé(s) cellulosique(s) peut varier entre 0,1 % et 20 %, mieux entre 0,5 % et 5%, encore mieux entre 0.7% et 2%, le pourcentage étant exprimé par rapport à la masse du produit de base.

### Utilisation pour le traitement d'une ou plusieurs zones précises de peau

Le système de distribution permet de se parfumer, jour après jour, en ne traitant que les zones précises. Pour ce faire, on délivre de petites doses de produit, qu'on applique précisément et séquentiellement sur les zones correspondantes. Chaque petite dose est réalisée selon le mélange qui convient à la zone.

Dans un mode préféré de mise en oeuvre de l'invention, le système de distribution attend qu'on le renseigne sur une zone à traiter puis délivre le mélange correspondant. Il peut faire appel à une table de correspondance préprogrammée pour cela, cette table résultant par exemple d'un procédé d'apprentissage tel que défini plus loin. En variante, le système de distribution renseigne la personne, tout en délivrant un mélange, sur la zone sur laquelle celle-ci doit appliquer le mélange. Ainsi, le système de distribution peut suivre un programme d'application où il délivre dans un ordre donné, les différents mélanges à appliquer.

Dans un mode particulier de mise en oeuvre de l'invention, le système de distribution est renseigné sur les quantités à délivrer. Pour cela, il mémorise la correspondance entre l'odeur, la zone du visage et la quantité nécessaire, ce qui permet de réduire les coûts et les pertes de produit, et de ne couvrir que faiblement la peau, évitant ainsi des effets d'occlusion. Le système de distribution peut aussi permettre de réaliser de façon précise des mélanges, en facilitant la distribution de petites quantités et leur usage rapide.

Lorsque l'utilisateur recherche le produit à appliquer sur une zone du visage, il est intéressant de mémoriser le mélange (ratio entre les produits des compartiments) qui va bien pour chaque zone, et le système de distribution est ainsi avantageusement agencé pour mémoriser l'effet, les ratios et la zone correspondante. Ainsi, en utilisant les informations mémorisées, à chaque utilisation, on peut obtenir la délivrance du même mélange pour chaque zone, ou, si l'on traite plusieurs zones, la même série de mélanges pour une même série de zones.

Le système de distribution peut aussi être agencé pour permettre de traiter une zone en faisant varier les effets application après application. Ainsi, la personne peut parfumer la peau avec différentes notes, qu'elle choisit au jour le jour selon ses goûts. Par exemple, les jours de la semaine, la personne applique un parfum donné, et le weekend, un autre.

Le système est aussi agencé pour délivrer des touches selon le résultat de l'application des touches différentes. Ainsi, si l'utilisateur s'aperçoit qu'après application de plusieurs touches, il manque quelque chose pour parfaire son résultat, le système peut alors délivrer à la demande un mélange permettant de réaliser une touche qui complètera le résultat.

Par exemple, dans le cas des parfums, si la personne applique une touche de parfum à un endroit, puis une touche de parfum différent sur une autre zone, elle aura peut-être envie de compléter l'impression olfactive en appliquant sur les mêmes zones ou sur une autre zone, une touche de parfum encore différent.

Le système est ainsi prévu pour proposer des variations et une interface pour traduire des ordres simples en réalisation de mélange.

Le système de distribution peut être agencé pour permettre à l'utilisateur de changer les ingrédients selon ses goûts du jour, les besoins du jour, du moment, de ses tenues, du climat. Ainsi, on prévoit avantageusement un système d'aide à la décision pour guider l'utilisateur dans ses choix de ses notes parfumées.

Il peut être souhaitable que plusieurs personnes d'un même groupe, par exemple une famille, puissent utiliser le système de distribution, réduisant ainsi les coûts et minimisant la place occupée. Cette solution est spécialement adaptée aux voyages ou aux hôtels, campings, avions, campings cars, boutiques, écoles Pour ce faire, on peut prévoir que le système de distribution puisse être renseigné sur la personne qui l'utilise, afin d'accéder à des données personnelles préalablement mémorisées.

### Utilisation en continu pour un traitement en dégradé

Dans cette application, le système de distribution, pendant qu'il délivre le produit, change la formulation du mélange.

De plus, on déplace la sortie des produits de base ou du mélange relativement à un contenant ou un support définissant une surface d'application. Dans un mode particulier de mise en oeuvre de l'invention, le système de distribution est agencé pour calculer l'évolution du mélange en fonction de l'odeur C1, d'une zone à traiter et de l'odeur C2, d'une autre zone à traiter. Par exemple, sachant que le cou nécessite une odeur C1 et que la chevelure nécessite une odeur C2, le système de distribution peut faire varier la formulation du mélange pendant qu'il le délivre pour réaliser un dégradé d'odeur entre ces deux odeurs. Cela permet par exemple de mieux masquer les imperfections du visage en faisant en sorte que le résultat final soit réaliste, ou de faire des dégradés d'odeur à des fins d'augmentation de l'attractivité. Le système de distribution peut encore être agencé de telle sorte que l'utilisateur puisse commander une variation d'odeur du mélange distribué sans que les odeurs de départ et/ou d'arrivée n'aient été préalablement fixées. Pour ce faire, le système de distribution peut disposer d'un système de localisation, ou d'auto-localisation, et déduire d'une table de correspondance les odeurs C1 et C2 qu'il doit réaliser, et donc les évolutions de mélange qu'il doit réaliser.

Par exemple, il peut réaliser un dégradé de parfums le long du cou pour faire partager une richesse d'odeur à une personne qui l'approche.

Le même concept est applicable aux compositions protectrices. La personne pourra réaliser des dégradés d'indices de protection, réalisant ainsi, après s'être exposée au soleil, à un résultat de bronzage lui-même en dégradé.

Le système de distribution peut comporter une tête de sortie, en particulier dans le cas d'un aérographe, qui est mobile et pilotée. Cette option permet alors, sans bouger le reste du système de distribution, de réaliser des dégradés. Par exemple, on localise le système de distribution près de la joue, puis on déclenche un système de commande qui va piloter automatiquement la variation de la formulation du mélange et le mouvement de la tête de sortie, de façon à par exemple avoir le cou parfumé avec un odeur et l'oreille avec une autre, avec un dégradé entre les deux.

Le système de distribution peut encore être utilisé pour réaliser des produits sur mesure, que l'on conserve pour réaliser plusieurs applications.

### Fabrication de compacts « à façon » ou autres produits solides ou semi-solides

Le système de distribution peut être agencé pour permettre de choisir un mélange et de le délivrer dans un contenant tel qu'une coupelle. Le mélange comporte de préférence des composés qui sont tels que le mélange puisse prendre en masse.

Plus préférentiellement, on utilise des composés qui rendent la prise en masse spécialement rapide. Ces composés sont soit déposés dans le contenant avant ou après son remplissage avec les autres constituants, soit sont prévus dans les compartiments du distributeur avec les autres constituants des produits de base, soit sont compris dans le distributeur dans un compartiment spécialement prévu pour les contenir.

On peut ainsi distribuer des compositions spécifiques qui peuvent par réaction chimique, biochimique ou physicochimique, durcir de façon accélérée après évacuation.

Ces compositions sont spécialement adaptées à la réalisation des compacts, c'est-à-dire :
- Prennent en masse,
- donnent un matériau qui peut se déliter en cas de frottement, et sont préférentiellement colorées.

De préférence, ces compositions sont très riches en particules solides, avec par exemple plus de 10% en masse de particules solides par rapport au poidsà la masse totale de la composition, mieux plus de 20% en masse de particules solides par rapport à la masse totale de la composition, encore mieux plus de 30% en masse de particules solides par rapport à la masse totale de la composition, préférentiellement entre 10 et 40% en masse de particules solides par rapport à la masse totale de la composition.

De préférence, une ou plusieurs des compositions insérées dans le système contient de l'huile, de préférence en une teneur supérieure ou égale à 20%.

Ces compositions peuvent contenir des particules absorbantes, ou des composés réactifs, tels que ceux qui réagissent en contact avec l'air, par exemple du cyanoacrylate ou des alphasilanes, ou qui réagissent à la lumière, notamment UV.

Le contenant dans lequel le mélange est distribué peut comporter un composé A et les compositions distribuées un composé B, A et B étant choisis pour réagir entre eux et solidifier le mélange.

Dans un mode particulier de mise en oeuvre de l'invention, le système de distribution intègre un moyen de chauffage, par exemple à résistance électrique, pour la réalisation de rouges à lèvres ou autres produits cireux. Dans ce cas, les produits de base sont chauffés avant d'être délivrés.

Le système de distribution peut aussi comporter un moyen pour apporter de l'énergie, thermique et/ou lumineuse, après distribution du mélange dans un contenant, par exemple une résistance électrique ou une diode LED, notamment UV. Cette énergie peut accélérer la prise en masse du mélange distribué.

Le système contient de préférence des quantités de dérivés cellulosiques importantes telles que supérieures à 5%, et de préférence supérieures à 10%.

Préférentiellement, le mélange est homogénéisé avant la prise en masse.

### Réalisation de palettes d'odeurs

Le système de distribution peut comporter un support, ayant plusieurs régions, et être agencé pour générer automatiquement plusieurs mélanges d'odeurs déposés dans lesdites régions, adaptées à différentes parties du visage.

Le support peut définir plusieurs cavités pour recevoir les mélanges ou porter plusieurs contenants, par exemple sous forme de coupelles, éventuellement séparables du support.

Dans un cas particulier, le support prend la forme d'un visage avec des régions pour recevoir les mélanges pour des zones d'applications ciblées.

Le support être mobile, notamment rotatif, par rapport au corps du distributeur, et par exemple entraîné en déplacement par le distributeur, pour remplir successivement différents logements ou contenants.

### Distributeur à coupelle

Il existe un intérêt pour bénéficier d'un système de distribution capable de délivrer un mélange pouvant être facilement prélevé par l'utilisateur. Par ailleurs, dans le cas où les produits de base délivrés par le système de distribution ne sont pas déjà mélangés, il existe un besoin pour permettre à l'utilisateur d'effectuer aisément le mélange.

Selon l'un de ses aspects, indépendamment ou en combinaison avec les autres aspects de celle-ci, et notamment avec ce qui précède, l'invention a pour objet un système de distribution d'au moins un produit de parfumage, comportant une coupelle, et un distributeur pour remplir la coupelle avec au moins un produit, la coupelle étant solidaire du distributeur au moins pendant son remplissage.

La coupelle est parfois encore appelée « creuset » et ce terme doit être compris avec une acceptation large.

Par « coupelle solidaire du distributeur » il faut comprendre que la coupelle est retenue, notamment immobilisée, au moins temporairement sur le distributeur, étant par exemple fixée sur celui-ci par des vis, par aimantation, par encliquetage, par verrouillage baïonnette, par serrage, ou réalisée avec une partie du corps du distributeur par moulage de matière. La coupelle lorsqu'elle est solidaire du distributeur permet de manipuler celui-ci d'une main, la coupelle restant en place sur le distributeur au cours des mouvements de ce dernier.

Le distributeur peut être proposé à l'utilisateur avec la coupelle déjà en place.

En variante, celle-ci est installée par l'utilisateur à la première utilisation du système de distribution.

De préférence la coupelle est moins profonde que large, ce qui facilite l'accès à celle-ci, et permet de prélever le produit, notamment le mélange avec un applicateur ou le doigt.

De préférence, la coupelle est séparable du distributeur, et constitue une interface de sortie qui peut être choisie parmi un ensemble d'interfaces de sortie pouvant être montées sur le distributeur, au choix de l'utilisateur en fonction du parfumage à réaliser, comme détaillé plus loin.

De préférence, le système de distribution comporte plusieurs orifices de remplissage en des produits de base différents, débouchant dans la coupelle. Ainsi, le mélange de ces produits peut avoir lieu dans la coupelle.

La coupelle a de préférence un fond de forme concave vers l'extérieur, ce qui peut faciliter son nettoyage par l'utilisateur entre deux utilisations.

De plus, cela peut faciliter le prélèvement du produit par l'utilisateur.

De préférence, le distributeur permet de délivrer au moins deux produits de base dans la coupelle, dans des proportions réglables, et mieux au moins trois produits.

Dans un exemple de mise en oeuvre, le système de distribution comporte au moins deux coupelles pouvant être sélectivement alimentées par le distributeur. Cela peut permettre à l'utilisateur de remplir rapidement ces deux coupelles avec des mélanges dont les caractéristiques sont différentes. Cela peut faciliter les essais d'odeur, et/ou permettre de préparer plusieurs mélanges d'odeurs différentes destinées au parfumage de zones respectives du visage. Les coupelles peuvent être associées à des identifiants rappelant à l'utilisateur à quelle zone du visage un mélange contenu dans une coupelle donnée est destiné.

Les coupelles peuvent être mobiles relativement au distributeur, étant par exemple portées par un support mobile, tel qu'une tourelle mobile en rotation par rapport au distributeur, ou par un tiroir mobile en translation par rapport au distributeur.

Le système de distribution peut comporter un couvercle de fermeture de la coupelle. Ce couvercle de fermeture est de préférence transparent pour permettre à l'utilisateur de visualiser la couleur du mélange contenu à l'intérieur.

Lorsque la coupelle est séparable du distributeur, elle peut être introduite le cas échéant dans un boîtier permettant de la transporter plus facilement, ce boîtier pouvant comporter le cas échéant un miroir et/ou un applicateur. Le couvercle du boîtier peut dans ce cas servir de couvercle pour la coupelle.

Le volume de la coupelle peut être compris entre 2 et 1000 mm³, mieux entre 100 et 1000 mm³, encore mieux entre 250 et 750 mm³.

La coupelle est de préférence de forme symétrique de révolution. En variante, elle est de contour polygonal ou autre. Son plus grand diamètre intérieur, ou celui du cercle inscrit dans le cas d'un contour non circulaire, est de préférence compris entre 2 et 100 mm, préférentiellement entre 5 et 40 mm. Sa profondeur est de préférence comprise entre 1 et 10 mm, mieux entre 3 et 8 mm. De préférence, la taille et la forme de la coupelle permettent soit une application directe du mélange sur la peau, soit une préhension au doigt ou avec un applicateur. La coupelle peut être réalisée avec un matériau élastiquement déformable, ce qui permet par exemple d'inverser la concavité du fond de la coupelle et de vider celle-ci plus facilement ou de l'utiliser pour appliquer le produit.

La coupelle peut être dépourvue de mélangeur ; dans ce cas, les produits de base peuvent arriver du distributeur dans la coupelle à l'état non mélangé, par des orifices de distribution respectifs distincts. En variante, le distributeur intègre un mélangeur et les produits de base arrivent déjà mélangés dans la coupelle.

La coupelle peut aussi intégrer un mélangeur statique, comme détaillé plus loin, qui est alimenté par des orifices de remplissage distincts du distributeur, et qui délivre de préférence le mélange dans une cavité de la coupelle située au-dessus du mélangeur.

L'invention a encore pour objet un procédé de préparation d'un produit de parfumage, comportant l'étape consistant à remplir une coupelle d'un système de distribution tel que défini plus haut avec au moins un produit de base provenant du distributeur.

Plusieurs produits peuvent être délivrés dans le fond de la coupelle, puis mélangés à l'aide du doigt ou d'un applicateur, ou d'un mélangeur statique intégré à la coupelle.

La coupelle est de préférence remplie par le dessous. Il a été proposé dans le passé des systèmes de distribution à l'aide d'une sonotrode.

La coupelle selon l'invention n'est pas destinée à entrer en vibration pour distribuer le ou les produits amenés par le ou les canaux d'alimentation de celle-ci. Elle est différente d'une sonotrode. De préférence, la coupelle est réalisée en matière plastique.

### Mélangeur intégré à l'interface de sortie

Il existe un intérêt pour bénéficier d'un système de distribution capable de délivrer un mélange pouvant être facilement utilisé, notamment prélevé par l'utilisateur, sans nécessiter une action additionnelle de mélange de la part de celui-ci.

Le système de distribution peut comporter un distributeur ayant des canaux de sortie de produits de base et une interface de sortie séparable du distributeur, cette interface ayant un mélangeur statique, délivrant de préférence le mélange dans une cavité où il peut être prélevé.

Le mélangeur statique peut être situé sous la cavité précitée. Le système de distribution est alors particulièrement adapté à la création de compacts, en utilisant comme interfaces de sortie des coupelles à mélangeur statique intégré. Dans ce cas, on vient remplir la cavité de la coupelle en produit par le dessous. Après passage dans le mélangeur statique, les produits de base mélangés viennent recouvrir le mélangeur.

Selon cet aspect de l'invention, il est possible d'utiliser plusieurs interfaces de sortie et de les remplir avec des mélanges respectifs différents, sans avoir à purger le mélangeur, ce qui réduit les pertes de produit. L'interface de sortie peut être à usage unique, le cas échéant.

De préférence, le mélangeur statique comporte une chambre centrale communiquant avec des canaux d'admission des produits de base. Cette chambre centrale peut communiquer avec une chambre périphérique comportant une série de cloisons qui agissent comme des déflecteurs pour le mélange, et créent un cisaillement de celui-ci.

La chambre périphérique peut comporter une cloison annulaire ajourée définissant des ajours par lesquels passe le mélange en circulant dans la chambre périphérique. Les chambres centrale et périphérique peuvent être fermées supérieurement par une paroi qui définit le fond de la cavité recevant le mélange.

Le fond de la chambre périphérique peut être de forme hélicoïdale autour de l'axe de la coupelle et de hauteur diminuant au fur et à mesure que l'on progresse vers la sortie. Cette dernière peut déboucher en avant d'une rampe de liaison entre le fond de la chambre périphérique et la paroi supérieure du mélangeur, cette rampe de liaison étant de préférence une portion d'hélice prolongeant l'hélice formée par la paroi de fond de la chambre périphérique.

De préférence la chambre périphérique comporte la cloison annulaire précitée et des cloisons radiales qui forcent le mélange à circuler alternativement entre des régions supérieure et inférieure de la chambre périphérique et entre des régions radialement intérieure et extérieure, le mélange circulant par exemple d'une région supérieure et radialement extérieure à une région inférieure et radialement extérieure en passant à travers la cloison annulaire précitée.

Le mélangeur peut comporter un corps extérieur dans lequel est reçue une pièce formant le coeur du mélangeur, le corps extérieur fermant radialement à l'extérieur la chambre périphérique et comportant un montant qui sépare les chambres centrale et périphérique.

Le corps extérieur du mélangeur et le coeur du mélangeur peuvent être chacun réalisés d'une seule pièce par moulage par injection.

### Volume mort réduit

Il existe un intérêt pour réduire les pertes de produit lors des changements de formulation du mélange, ainsi que pour permettre de faire varier le plus rapidement possible l'odeur du mélange au cours de l'application, notamment en cas de couplage du distributeur avec un aérographe.

Un système de distribution d'un produit de parfumage selon ce neuvième aspect de l'invention comporte un distributeur recevant au moins deux cartouches ayant chacune un réservoir contenant un produit de base, ce dernier peut quitter la cartouche par un canal de sortie de la cartouche, ce canal de sortie débouchant à l'extérieur du distributeur ou à proximité de sa surface externe.

Le canal de sortie peut notamment déboucher dans une zone de prélèvement du mélange ou à proximité de celle-ci, notamment à moins de 5 mm, mieux à moins de 3 mm, mieux à moins de 1mm, mieux à fleur.

La section transversale du canal de sortie est par exemple comprise entre 1 et3 mm².

Ainsi, chaque produit de base provenant d'une cartouche peut quitter le distributeur sans se mélanger avec un produit de base d'une autre cartouche et l'on minimise le volume mort qui ne peut être prélevé et susceptible d'accroître l'inertie du système. Le produit est plus rapidement disponible sans avoir à circuler dans des canaux spécifiques du boîtier du distributeur, évitant une étape fastidieuse de purge si changement de cartouche.

L'extérieur du distributeur peut être la zone de prélèvement du produit, notamment lorsque le distributeur est réalisé avec une coupelle non prévue pour être enlevée, dans laquelle le mélange est distribué, ou une zone du distributeur destinée au montage d'une interface de sortie amovible, qui définit la zone de prélèvement. Cette interface de sortie peut comporter une coupelle telle que définie ci-dessus. Cette zone de montage correspond par exemple à l'extrémité du boîtier du distributeur en l'absence de l'interface de sortie. La zone de montage peut être sensiblement plane et perpendiculaire à l'axe longitudinal du boîtier du distributeur.

Le distributeur peut comporter trois cartouches de produits de base.

Le distributeur peut comporter des logements pour recevoir les cartouches, lesquelles sont de préférence reçues de façon amovible dans le distributeur. Ce dernier peut comporter des passages pour des conduits des cartouches définissant les canaux de sortie.

La longueur de ces conduits est de préférence telle que les conduits se situent légèrement en retrait du fond ou à affleurement de la cavité servant au prélèvement du produit, ou en variante légèrement en retrait ou à affleurement de la face d'extrémité du boîtier du distributeur définissant la zone de montage.

Ces conduits des cartouches peuvent être des embouts servant à entraîner en déplacement des pistons au sein des cartocuhes, comme détaillé plus haut.

### Interfaces de sortie multiples

Il existe un besoin pour pouvoir, à l'aide d'un même système de distribution, réaliser facilement des parfumages différents, et pouvoir si on le souhaite parfumer des zones aussi différentes que la peau, les lèvres, le cou, les sourcils ou la chevelure.

Le système de distribution peut comporter un ensemble comportant un distributeur d'au moins un produit de parfumage, et au moins deux interfaces de sortie pouvant se monter chacune de façon amovible sur le distributeur, ces interfaces de sortie permettant de recevoir le ou les produits délivrés par le distributeur, étant de préférence choisies parmi les suivantes :
- interface de sortie comportant un contenant, notamment une coupelle permettant un prélèvement du produit au doigt ou à l'aide d'un applicateur,
- interface de sortie permettant de délivrer le produit à un système de pulvérisation, notamment un aérographe,
- interface de sortie comportant plusieurs régions de réception du produit, mobile par rapport au distributeur,
- interface de sortie permettant de délivrer le produit à un embout de distribution.

De préférence, l'ensemble comporte au moins trois desdites interfaces de sortie, mieux les quatre interfaces de sortie.

Le distributeur peut comporter au moins deux produits de base différents et permettre de délivrer ceux-ci dans des proportions variables, et de préférence, le distributeur comporte trois produits de base différents et permet de délivrer ceux-ci dans des proportions variables.

Chaque interface de sortie peut comporter une base permettant sa fixation sur le distributeur. Cette fixation peut s'effectuer à l'aide de vis par exemple, mais de préférence la base est agencée pour permettre un démontage et un remplacement d'une interface de sortie sans nécessité d'outil. Il s'agit par exemple d'une fixation quart de tour ou à l'aide d'une bague de verrouillage externe.

L'interface de sortie et/ou le boîtier du distributeur peuvent comporter des joints d'étanchéité permettant une communication étanche entre le boîtier du distributeur et l'interface de sortie. Le cas échéant, le distributeur est agencé pour reconnaître l'interface de sortie qui est montée dessus, par exemple grâce à identifiant de l'interface de sortie sous forme de reliefs spécifiques, lesquels sont détectés par le distributeur, ou d'une puce électronique reconnue par le distributeur. Cela peut permettre d'adapter le fonctionnement du distributeur à l'interface de sortie montée dessus. Le distributeur peut communiquer à un système informatique des informations sur l'interface de sortie qu'il porte, et le système informatique peut afficher en fonction de ces informations un écran spécifique et/ou lancer un programme spécifique de commande des paramètres de fonctionnement du distributeur, afin par exemple d'adapter la dose distribuée et/ou le débit à la nature de l'interface de sortie.

Plusieurs interfaces de sortie peuvent être proposées initialement à l'utilisateur avec un distributeur commun au sein d'un même emballage, par exemple un coffret ou une boîte de carton.

L'invention a encore pour objet un procédé de parfumage, comportant l'étape consistant à sélectionner une interface de sortie, à la monter sur le distributeur, et à délivrer le ou les produits contenus dans le distributeur dans celle-ci.

### Cartographie et apprentissage

Le terme « cartographie » s'entend ici comme un procédé d'indexation d'une, odeur, et d'une zone, avec enregistrement.

La cartographie peut concerner des applications sur des zones plus petites qu'un cm2. Toutefois, l'oeil nu a alors du mal à discerner si le résultat obtenu est adéquat, et il est préférable de substituer l'évaluation à l'oeil nu par une évaluation instrumentée, avec grossissement. L'application de petites quantités de matière peut être faite au doigt, avec des outils classiques tels que des pinceaux, ou avec des applicateurs spécialisés.

La cartographie peut être générée lors d'une période d'apprentissage, pendant laquelle l'utilisateur effectue des tests avec des mélanges sur différentes zones du visage ; une fois renseignée, la cartographie peut ensuite être utilisée pour le parfumage de tous les jours.

Des interfaces graphiques spécifiques peuvent servir pendant la période d'apprentissage et pendant la période d'utilisation de la cartographie.

En particulier, le système de distribution peut être utilisé avec une interface graphique où l'opérateur voit le visage, qui est par exemple schématisé, figuratif ou précis comme une photographie ou une simulation 3D. Dans ce cas, l'opérateur peut pointer une partie du visage sur l'écran pour voir apparaître et/ou délivrer l'odeur adéquate.

L'interface graphique peut aussi laisser apparaître les autres zones du visage où l'utilisation de la même odeur est adéquate.

Pour réaliser la cartographie, l'opérateur applique une odeur puis réalise l'évaluation.

Les zones du visage peuvent être traitées les unes après les autres ; par exemple, on réalise l'exercice sur une partie du cou puis sur l'oreille, puis les cheveux, etc....

Une autre possibilité consiste à réaliser un mélange donné, et appliquer sur plusieurs zones ce même mélange. L'opérateur doit alors chercher la zone du visage pour laquelle l'odeur est adaptée. Le mélange est ensuite indexé dans le système informatique en l'attribuant à la ou aux zones du visage pour lesquelles il convient.

Selon l'un de ses aspects, indépendamment ou en combinaison avec les autres aspects de celle-ci, et notamment ce qui précède, l'invention a pour objet un procédé d'apprentissage d'un système de distribution comportant un distributeur permettant de distribuer un mélange d'odeur variable, et un système informatique permettant de sélectionner un effet parfumant et de mémoriser des données, comportant les étapes consistant à :
a) Sélectionner au moins une odeur à l'aide d'une interface du système informatique,
b) délivrer à l'aide du distributeur au moins un mélange pour approcher l'odeur sélectionnée,
c) évaluer le ou les mélanges distribués après application sur au moins une zone du visage, (odeur)
d) mémoriser les caractéristiques d'au moins un mélange, notamment un mélange que l'utilisateur souhaite pouvoir rappeler, et d'au moins une zone sur laquelle il a été testé.

Cette mémorisation peut s'effectuer notamment en vue d'une distribution ultérieure de ce mélange pour parfumer ladite zone.

De préférence, le système informatique est agencé pour permettre à l'utilisateur de signaler si le résultat du test est satisfaisant ou non, voire de le renseigner sur la comparaison à un test effectué précédemment.

On peut aussi réaliser un mélange donné, et chercher la zone du visage pour laquelle il est adapté. On enregistre alors le mélange en l'attribuant à la ou aux zones du visage pour lesquelles il est adapté, dans une table de correspondance qui servira par la suite, pour, partant d'une zone de la peau, en déduire le mélange à utiliser.

On peut procéder de la même façon avec d'autres mélanges pour réaliser une cartographie de l'ensemble du visage et ainsi avoir une table de correspondance complète du visage.

On peut aussi réaliser un mélange donné, l'appliquer sur une zone donnée, puis faire varier le mélange jusqu'à obtenir le mélange le plus adapté. On enregistre alors le mélange en l'attribuant à la ou aux zones du visage pour lesquelles il convient, dans une table de correspondance qui servira par la suite, pour, partant d'une zone de la peau, en déduire le mélange à utiliser.

De préférence, le système informatique évalue et mémorise les quantités utilisées zone par zone. Un tel procédé, par « touches d'essai », permet d'identifier le ou les produits dont a besoin la personne qui veut se parfumer. Ainsi, le système de distribution peut servir sur les points de vente pour conseiller les personnes qui veulent se parfumer ou à la maison pour bien définir les produits à commander.

L'interface du système informatique comporte de préférence un écran tactile affichant une évocation de l'odeur du mélange lors de sa sélection.

L'interface peut afficher un visage et permettre de renseigner le système informatique en sélectionnant la zone sur le visage affiché.

Le système informatique est de préférence agencé pour permettre d'associer une zone, des paramètres de reconstitution du mélange, et la date de l'essai et/ou tout autre identifiant du mélange.

Le système informatique est de préférence également agencé pour permettre d'associer en outre à ladite zone, aux paramètres de reconstitution du mélange, et à la date ou à l'identifiant du mélange, au moins l'une des données suivantes : le nom de la zone, la période de l'année, le nom d'un évènement, un identifiant de l'utilisateur, l'âge de l'utilisateur.

Les étapes a) à c) peuvent être renouvelées au moins une fois avant la mémorisation des caractéristiques du mélange à l'étape d).

Le système informatique peut être agencé pour rechercher dans une base de données une référence de produit commerciale, sur la base des caractéristiques du mélange identifié comme convenant à au moins une zone donnée, et la communiquer à l'utilisateur.

La sélection à l'étape a) peut s'effectuer avec l'aide d'un système expert externe ou non au système informatique.

Le système expert peut analyser une image de l'utilisateur, pour proposer une odeur de mélange au moins sur la base de l'image analysée.

Le système informatique peut être agencé pour permettre à l'utilisateur de le renseigner sur l'appréciation du résultat du test de l'étape c) et pour générer une proposition de modification du mélange à sélectionner lors du retour à l'étape a).

Le distributeur peut délivrer à l'étape b) au moins deux mélanges différents, de préférence séparés, en vue de leur application simultanée sur la zone de test.

Cela peut faire gagner du temps, et faciliter la comparaison des résultats.

L'invention a encore pour objet un procédé parfumage à l'aide d'un système de distribution selon cet aspect de l'invention, dans lequel :
a) L'utilisateur adresse une requête concernant un besoin au système informatique,
b) celui-ci génère en retour une proposition en vue du traitement d'une zone associée, sur la base de l'apprentissage effectué préalablement, et
c) le système informatique pilote le distributeur pour la production du mélange proposé, notamment si celle-ci est validée par l'utilisateur.

Un tel procédé peut utiliser une cartographie précédemment établie avec l'utilisateur.

L'invention a encore pour objet un produit programme d'ordinateur comportant des instructions de code permettant, lorsque exécutées dans un système informatique, d'amener le système informatique à :
- Permettre à l'utilisateur de sélectionner au moins une odeur et/ou une zone d'application, notamment à l'aide d'une interface telle qu'un écran tactile,
- piloter un distributeur de façon à délivrer un mélange sélectionné par l'utilisateur,
- permettre à l'utilisateur de déclencher la mémorisation du mélange et d'une zone d'application associée, notamment en vue d'une distribution ultérieure du même mélange, notamment sur la même zone.

Le produit programme d'ordinateur peut comporter des instructions de code permettant, lorsque exécutées dans un système informatique, d'amener le système informatique à :
- Recevoir une requête de l'utilisateur concernant un besoin de parfumage notamment à l'aide d'une interface telle qu'un écran tactile,
- proposer sur la base au moins de données générées par le procédé d'apprentissage tel que défini ci-dessus, au moins une odeur et/ou une zone d'application,
- piloter un distributeur pour la production du mélange proposé, notamment si celle-ci est validée par l'utilisateur.

### Aide à distance

Il est souhaitable de pouvoir aider l'utilisateur à se parfumer, notamment à choisir les bonnes notes olfactives.

L'invention, selon l'un de ses aspects, indépendamment ou en combinaison avec ses autres aspects, notamment ce qui précède, a ainsi pour objet un procédé de parfumage comportant les étapes consistant à :
- permettre l'établissement d'une liaison vidéo, par exemple via internet, entre une caméra d'un premier site et un deuxième site,
- permettre au deuxième site de piloter directement ou indirectement un distributeur présent sur le premier site, ce distributeur permettant de faire varier l'odeur d'un mélange distribué,
- permettre à une personne présente au premier site d'appliquer le mélange distribué et d'envoyer vers le deuxième site une image correspondante ou commentaires, pour recevoir en retour une information relative au résultat de l'application.

Le deuxième site peut notamment comporter un écran de visualisation qui permet à un conseiller présent devant cet écran d'entendre ou lire les commentaires sur le résultat de parfumage avec le produit distribué par le distributeur et de conseiller la personne qui s'est parfumée. Ce conseiller peut agir en retour sur le distributeur pour modifier l'odeur du mélange et l'adapter au mieux au visage de la personne présente sur le premier site. Ainsi, cette personne gouverne le mélange délivré par le distributeur. La première personne peut se parfumer sous les yeux de la seconde. La seconde personne voit sur son écran le résultat de l'essai, et ainsi peut corriger le mélange qu'elle va commander à distance, jusqu'à obtenir le parfumage idéal.

De préférence, la liaison vidéo entre les deux sites est une liaison duplex.

Le premier site peut recevoir un tutoriel du deuxième site, le cas échéant.

Des identifiants des produits de base peuvent être communiqués au deuxième site ; cela peut permettre de connaître précisément le ressenti des odeurs de chacun des produits de base.

Le procédé peut comporter la mémorisation des paramètres de réglage du distributeur, une fois qu'un mélange donné est estimé satisfaisant. De préférence, cette mémorisation peut être commandée depuis le deuxième site. La mémorisation peut avoir lieu dans le système informatique présent sur le premier site et/ou sur un serveur externe.

Une variante peut consister à faire travailler une personne pour aider le parfumage de plusieurs personnes. Ce mode de mise en oeuvre permet le développement des « coach en parfumage » et leur travail soit au sein d'un institut, soit via internet. Cela permet aussi le parfumage de personnes aux moyens limités, telles que celles manquant de confiance en elles.

### Pilotage par interface tactile

Il existe un besoin pour faciliter le pilotage du système de distribution et notamment le choix des effets du mélange distribué.

Le système de distribution peut comporter un distributeur et un système informatique de pilotage du distributeur, ce système informatique comportant un écran tactile sur lequel peut être affichée l'odeur du mélange ou l'effet attendu et un moyen de sélection déplaçable sur l'écran, pour faire varier l'odeur du mélange distribué.

De préférence, l'écran affiche les effets extrêmes entre lesquelles l'effet du mélange peut être sélectionné, en déplaçant le moyen de sélection entre ces points extrêmes.

L'écran peut afficher une échelle d'effets en forme de ligne ou surface (contour triangulaire).

Le système informatique peut effectuer une partie des calculs nécessaires pour déterminer les fractions de chacun des produits de base conduisant à un mélange de de l'effet recherché.

Le système informatique peut être un téléphone intelligent, un photophone, une tablette, un ordinateur individuel. En variante, il est intégré au boîtier du distributeur.

Le système informatique peut disposer d'une caméra. Cette dernière peut être utilisée notamment pour l'acquisition d'une image de l'utilisateur et/ou du mélange.

Le système informatique peut être agencé pour afficher une image d'un visage, afin de faciliter le repérage des zones sur lesquelles le mélange doit être appliqué.

### Couplage du système de distribution à un moyen de pulvérisation

Le système de distribution peut comporter ou être connecté à un moyen de pulvérisation du mélange, de préférence un aérographe.

Le système de distribution peut comporter un système de pulvérisation, de préférence un aérographe, et a pour objet, selon l'un de ses aspects, indépendamment ou en combinaison avec les autres aspects, et notamment ce qui précède, un ensemble comportant :
- Un moyen de pulvérisation, de préférence un aérographe comportant une chambre de prélèvement soumise à un courant d'air d'entraînement,
- un distributeur comportant au moins deux compartiments contenant des produits de base différents, les produits étant délivrés au moyen de pulvérisation de préférence par des orifices de distribution distincts.

Le distributeur peut comporter trois cartouches contenant des produits de parfumage.

L'aérographe peut comporter un stylet définissant la chambre de prélèvement, le stylet se fixant sur le distributeur ou sur une interface de sortie fixée au distributeur, ou faisant partie intégrante de cette interface de sortie.

Le système de distribution peut comporter un circuit de contrôle du fonctionnement du distributeur, permettant de faire varier la proportion des produits de base délivrés dans la chambre de prélèvement, au cours du fonctionnement de l'aérographe. Les proportions peuvent être modifiées en fonction du déplacement de l'aérographe relativement à la surface sur laquelle le mélange est pulvérisé. Ce déplacement peut être mécanisé, le cas échéant.

Ce circuit de contrôle peut comporter ou être constitué par un système informatique tel que défini plus haut.

Le boîtier du distributeur peut servir de poignée lors de la manipulation d l'ensemble pour délivrer le mélange.

Le distributeur peut comporter une caméra et/ou un ou plusieurs capteurs tels que des accéléromètres afin de localiser automatiquement la zone sur laquelle le mélange est appliqué, et pouvoir régler automatiquement l'odeur en fonction de la position, le cas échéant.

L'invention a encore pour objet un procédé de parfumage à l'aide d'un ensemble tel que défini ci-dessus, dans lequel un mélange est pulvérisé sur la peau à l'aide du moyen de pulvérisation, notamment de l'aérographe.

La composition du mélange peut être modifiée au cours du déplacement de l'aérographe relativement à la peau. Un dégradé d'odeur peut être réalisé.

Cet aspect de l'invention repose sur la constatation que le distributeur peut être utilisé pour alimenter le système de pulvérisation, notamment l'aérographe, tout en permettant au système de distribution d'être suffisamment réactif pour autoriser un changement d'odeur du mélange distribué en cours de traitement du visage.

Il peut être avantageux que la distribution des produits se fasse de façon itérative, notamment avec des temps de distribution non déphasés entre les différents produits.

Cela peut rendre plus facile de faire varier la composition du mélange distribué au cours du temps.

Le mélange peut être réalisé directement dans l'aérographe, sans pratiquement aucun volume mort gênant, permettant ainsi de changer le mélange pulvérisé en temps réel.

La dépression créée dans la chambre de prélèvement est suffisante pour entraîner les produits de base, sans pour autant gêner le dosage.

La dépression qui règne dans la chambre de prélèvement est par exemple comprise entre 10 mbar et 200 mbar, mieux entre 50 et 150 mbar, encore mieux entre 75 et 125 mbar.

La viscosité des produits de base mesurée à 1 atm et à 25°C avec un viscosimètre rotatif CONTRAVES TV équipé d'un mobile MS-r3 ou MS-r4 à la fréquence de 60 Hz, après 10 minutes de rotation du mobile, est par exemple comprise entre 0,02 Pa.S et 50 Pa.S, de préférence de 0,2 Pa.s à 5 Pa.s

La section des canaux d'arrivée des produits de base dans la chambre est par exemple comprise entre 1 et 3 mm², mieux entre 2 et 3 mm².

L'alimentation en produit s'effectue de préférence de façon continue.

Il est encore possible d'appliquer simultanément des produits de base non miscibles ou réactifs, comme un gel aqueux et une composition selon l'invention (à base de solvant organique), qui vont se déposer de façon pixellisée directement sur la peau, produisant une sorte de gel/gel in situ, des silicones réactives. Les ratios en produits de base peuvent être ajustés en fonction du résultat particulier souhaité. Par exemple, dans le cas des gels aqueux et gels huileux, on pourra faire varier le ratio correspondant au volume du premier produit de base sur le volume du deuxième produit de base entre 10/1 et 1/10, mieux entre 5/1 et 1/5.

### Système de localisation ou d'auto-localisation

Le système de distribution selon l'invention peut disposer d'un système de localisation ou d'auto-localisation.

On appelle système de localisation, un moyen de saisir, de la part de la personne, la zone qu'elle va traiter. Ceci peut être réalisé notamment par des systèmes laissant libre au moins une main. Ainsi, on peut utiliser une interface d'un système informatique telle qu'un écran tactile, un joystick, ou un système de reconnaissance vocale.

On appelle système d'auto-localisation, un moyen de saisir, sans que la personne n'intervienne, la zone à traiter. Ceci peut être réalisé par un ou plusieurs accéléromètres qui déduisent, par le fait des mouvements, les directions que la personne vise ou par une caméra et un système de reconnaissance d'image.

Des exemples de systèmes de distribution pouvant convenir à l'invention selon son neuvième aspect sont représentéss sur les figures, qui ne seront pas toutes décrites à nouveau de façon détaillée.

Le boîtier du distributeur 11, dans sa configuration illustrée à la figure 13, c'est à-dire sans l'interface de sortie 110, présente l'avantage de pouvoir être accouplé à diverses formes d'autres interfaces de sortie, en fonction du parfumage que l'on souhaite réaliser et/ou de la zone à traiter.

On a illustré à la figure 29C un support ayant des logements disposés sur celuici sensiblement comme les différentes zones d'un visage ; chaque logement peut contenir un mélange dont l'odeur est adaptée à la partie correspondante du visage. Ainsi, il est facile pour l'utilisateur de savoir où appliquer le mélange prélevé dans un logement donné.

On peut utiliser le distributeur 11 pour délivrer un mélange dont la formulation change au cours du temps et recueillir le mélange dans un contenant mobile par rapport au distributeur, de telle sorte que le mélange soit déposé en un emplacement du contenant qui varie dans le temps, afin de réaliser un dégradé.

Par exemple, comme illustré aux figures 29A et 29B, le système de distribution comporte une interface de sortie 110 comportant une partie fixe relativement au distributeur et une partie mobile 252 présentant un logement 253 pour recevoir le mélange.

Par exemple, le distributeur 11 est disposé dans ce cas avec les orifices de sortie des cartouches vers le bas, et équipé d'un mélangeur, de telle sorte que le mélange tombe sous l'effet de son poids dans le logement 253. Un moteur peut déplacer la partie mobile de l'interface de sortie relativement au distributeur, de façon synchronisée avec la variation des caractéristiques du mélange, de telle sorte que l'on obtienne un dégradé le long dulogement 253, comme illustré à la figure 29B.

On a représenté sur les figures 31 à 37 différents exemples d'interfaces tactiles pouvant permettre à l'utilisateur de choisir la l'odeur du mélange résultant de la distribution dosée des différents produits de base.

Cette interface peut présenter, comme illustré à la figure 31, une zone de sélection de l'odeur, par exemple sous la forme d'un triangle dont les sommets correspondent aux odeurs de chacun des produits de base contenus dans les cartouches.

L'utilisateur peut déplacer un curseur 300, par exemple sous la forme d'une balle, relativement aux sommets A, B et C du triangle.

Plus il rapproche le curseur 300 de l'un des sommets, plus la fraction du produit de base correspondant est grande par rapport à la quantité totale des différents produits distribués.

La fraction de chaque produit par rapport à la quantité totale peut être indiquée en 301 par une valeur numérique sur l'interface.

L'interface peut permettre à l'utilisateur d'incrémenter ou de diminuer la quantité de chacun des produits, en agissant par exemple sur des boutons de commande 302, lesquels permettent un réglage précis de la quantité de chacun des produits de base.

La surface du triangle 310 peut avoir une odeur qui varie localement de façon à être représentative en chaque point de l'odeur du mélange résultant de la pondération des différents produits de base dans des proportions correspondant aux coordonnées relatives en ce point.

L'interface peut comporter un bouton 305 permettant d'accéder à un menu spécifique de réglage du volume de produit qui est distribué pour purger le distributeur.

L'interface peut aussi avantageusement permettre un réglage du débit de produit grâce à des boutons 304 et 306 renvoyant vers un menu spécifique de réglage du débit.

Dans l'exemple considéré, l'interface offre le choix entre un mode de distribution continu, grâce au bouton 304, où les produits sont distribués tant que l'utilisateur appuie sur le bouton de commande 12.

La dose correspondante peut être transmise à l'interface et affichée.

Le bouton 306 permet de sélectionner un fonctionnement en mode dose, au cours duquel un appui même bref sur le bouton 12 déclenche la distribution d'une dose prédéfinie.

Pour faire varier le débit, le distributeur agit par exemple sur le rapport cyclique de fonctionnement des moteurs.

L'interface peut être agencée pour permettre à l'utilisateur de programmer ou de mémoriser les réglages qui ont sa préférence, grâce à un menu 307 d'accès à des favoris.

L'interface tactile illustrée à la figure 32 fait apparaître sur l'écran trois zones colorées 400, correspondant chacune à l'odeur de l'un des produits de base contenus dans le distributeur 10, et une zone centrale 410 faisant apparaître l'odeur du mélange résultant.

La quantité relative de chacun des produits de base peut être ajustée à l'aide de curseurs 415 qui se déplacent par exemple sur des lignes joignant chacune des zones 400 à la zone centrale 410.

Au cours de l'utilisation de l'interface, celle-ci peut mémoriser un réglage donné et faire apparaître sur l'écran un bouton 420 de l'odeur du mélange. L'utilisateur peut ensuite, en appuyant simplement sur ce bouton 420, distribuer un mélange de l'odeur correspondante.

Dans l'exemple de la figure 34, l'interface affiche dans une zone 500 une odeur donnée, et propose à l'utilisateur, grâce à des boutons de commande 510 chacun de l'odeur du produit de base correspondant, d'accroître ou de diminuer la proportion de ce produit de base dans le mélange final. L'odeur de la zone 500 est recalculée en fonction des actions sur les boutons de commande 510.

Dans la variante de la figure 35, l'interface fait apparaître un nuancier présentant plusieurs zones 530 correspondants chacune à une proportion particulière des différents produits de base.

L'utilisateur peut sélectionner l'une de ces zones, par exemple en appuyant dessus avec son doigt.

L'interface peut être agencée pour afficher à une échelle plus grande dans une zone 535 l'odeur sélectionnée. La programmation du distributeur 11 pour distribuer cette odeur est par exemple déclenchée en appuyant sur la zone.

Dans l'exemple de la figure 36, l'utilisateur peut déplacer sur un nuancier continu 550 un curseur 555, qui fait apparaître dans une zone 558 l'odeur sélectionnée.

L'utilisateur peut ensuite, en appuyant par exemple dans la zone 556, déclencher l'envoi au distributeur 11 des instructions nécessaires pour que celui-ci distribue un produit de l'odeur sélectionnée.

On voit sur la figure 37 que l'interface peut mémoriser les différentes teintes sélectionnées, et les faire ensuite apparaître sur l'écran de façon à permettre à l'utilisateur, en appuyant sur des boutons correspondants 560, de sélectionner à nouveau très facilement une teinte précédemment choisie.

On a représenté à la figure 38 un exemple d'interface utilisateur 1000 d'un système de distribution comportant un distributeur, de préférence tel que décrit précédemment, et un système informatique 100 auquel appartient l'interface.

Le système informatique comporte ici par exemple un appareil tel qu'un ordinateur portable, une tablette ou un téléphone intelligent, fonctionnant de façon autonome ou connecté à un serveur distant.

Dans l'exemple considéré, l'interface 1000 est définie par l'écran tactile d'un tel appareil. Dans une variante non illustrée, le distributeur intègre un écran tactile ou tout autre type d'interface homme machine, et peut être utilisé sans connexion à un autre appareil.

L'appareil exécute une application, qui a par exemple été téléchargée au préalable, et qui affiche sur l'écran un visage 1035 et une série de boutons permettant à l'utilisateur d'entrer des informations.

Le visage peut comporter plusieurs zones Zl à Z6 sélectionnables tactilement, par exemple le front, le nez, les joues, les paupières, le menton, et les lèvres.

Les boutons présents sur l'écran peuvent permettre par exemple d'entrer le nom du parfumage ou de l'utilisateur, d'afficher la zone sélectionnée, de choisir l'odeur, et d'informer le système informatique du résultat acceptable ou non du test effectué, voire comme illustré de donner une information concernant l'évaluation du résultat relativement à un test effectué précédemment, à savoir par exemple mieux ou « moins bien ». L'écran peut également afficher un bouton permettant de mémoriser le choix d'une odeur et d'une zone, après avoir effectué un essai avec cette odeur sur la zone en question.

Le choix de l'odeur s'effectue par exemple avec une échelle similaire à celle décrite en référence à la figure 36.

Le système informatique est agencé pour mémoriser les données sous forme de table de correspondance par exemple, de manière à associer à une zone du visage des paramètres permettant de reproduire le mélange distribué lors du test. Ces paramètres comportent par exemple les teneurs relatives de chacun des produits de base du distributeur dans le mélange, la quantité Q distribuée, ainsi que des données additionnelles telles que par exemple le nom de la zone, la date de distribution du mélange et/ou tout autre identifiant du mélange, les identifiants des produits de base, la période de l'année, notamment la saison, l'âge de l'utilisateur, son sexe, son nom ou prénom, le nom d'un évènement associé au parfumage, par exemple un anniversaire, entre autres données, la quantité de produit adéquate pour la zone. Les données annexes peuvent permettre à l'utilisateur de reproduire plus facilement un parfumage qu'il a considéré comme adéquat pour une période de l'année, ou rappelant un évènement de la vie, ou pour donner un effet de rajeunissement.

Ces données peuvent être mémorisées dans le système informatique 100, par exemple dans l'appareil précité et/ou sur un serveur distant avec lequel l'appareil communique, ou encore dans une mémoire électronique intégrée au distributeur 11.

Ainsi, selon l'invention, l'utilisateur peut amener le distributeur à délivrer une première matière parfumante à l'appliquer sur une première zone du visage, puis la juger adéquate ou non. Si le résultat est satisfaisant, l'utilisateur peut l'enregistrer en l'indexant à la zone ; si le résultat est non satisfaisant, l'utilisateur peut commander une nouvelle odeur pour refaire les opérations ci-dessus.

Le système informatique peut être utilisé dans ce contexte de diverses façons.

Par exemple, comme illustré à la figure 40, l'utilisateur choisi à une étape 1010 une odeur à essayer, en utilisant par exemple l'échelle 1011 apparaissant sur l'écran, en déplaçant le bouton de réglage 1012.

Ensuite, le choix de l'odeur est transmis au distributeur 11, à l'étape 1015.

Par exemple, l'appareil transmet les quantités à distribuer de chacun des produits de base, et le circuit électronique 81 se charge de commander les moteurs en conséquence.

A l'étape 1016, l'utilisateur appuie sur le bouton de commande 12 du distributeur 11, ce qui provoque par exemple la distribution d'une dose du mélange, de l'odeur sélectionnée par l'utilisateur.

Le mélange est par exemple distribué dans la coupelle 115, puis prélevé et appliqué par l'utilisateur sur les joues ou toute autre zone renseignée sur l'interface, à l'étape 1020.

Dans des variantes, le produit est appliqué à l'aide d'un aérographe ou par tout autre moyen tel que décrit précédemment.

L'utilisateur renseigne ensuite à l'étape 1022 le système informatique à l'aide des boutons 1021, sur le résultat.

Si l'utilisateur indique que le résultat est satisfaisant, le système lui propose par exemple de valider à l'aide d'un bouton les paramètres de l'essai afin de les mémoriser à l'étape 1031.

Si le résultat n'est pas jugé satisfaisant par l'utilisateur et signalé comme tel à l'aide du bouton 1032, le résultat peut néanmoins être automatiquement sauvegardé à l'étape 1034.

On peut ainsi indexer à chaque zone non seulement la ou les odeurs adéquates mais aussi la ou les odeurs qui ne vont pas du tout à cette zone.

L'utilisateur peut alors effectuer un nouvel essai sur la même zone en revenant à l'étape 1010.

Si l'utilisateur était satisfait du résultat, il peut également souhaiter effectuer un nouvel essai, par exemple sur une zone différente du visage.

Le cas échéant, si l'utilisateur n'est pas satisfait, l'interface peut lui proposer d'indiquer si le résultat est estimé mieux ou moins bien que l'essai précédent, à l'aide de boutons correspondants 1040 et 1041.

Dans ce cas, le système informatique peut être agencé pour déterminer si au vu des renseignements entrés par l'utilisateur une proposition peut être automatiquement effectuée quant au choix de la nouvelle odeur à tester.

Le cas échéant, un questionnaire peut s'afficher pour aider le système informatique à proposer une odeur au vu des essais effectués et de l'évaluation qui en a été faite par l'utilisateur ou par un professionnel qui l'assiste.

Par exemple, si l'odeur est jugée comme « non adéquate », le système peut recevoir de l'utilisateur une information additionnelle, par exemple « trop fruité », qui l'aidera à proposer une nouvelle odeur plus en adéquation avec ce qu'attend l'utilisateur.

Il peut être intéressant que le système informatique puisse recevoir une information de comparaison du résultat par rapports aux essais précédents, par exemple « c'est mieux » ou « c'est moins bien » et que de là, le système soit capable d'en déduire la nouvelle odeur à proposer.

Une autre option est que le système informatique puisse recevoir une information de comparaison par rapport à une cible, par exemple « c'est presqu'idéal » et que de là, le système soit capable d'ajuster automatiquement ses modifications d'odeur.

En l'occurrence, s'il reçoit l'information selon laquelle le résultat souhaité est presque atteint, le système peut adopter de petits niveaux de modification de l'odeur et revoir l'échelle de réglage en conséquence.

Dans le cas où le système de distribution propose de lui-même les mélanges odorants à tester, il peut se fonder sur des scénarios d'essais préprogrammés et modifier le suivi du scénario selon les réussites ou échecs de l'évaluation. Ainsi, par exemple, si dès la troisième application de produit, il reçoit une information selon laquelle l'odeur est presqu'idéale pour l'utilisateur, le système de distribution peut sortir du programme et se laisser guider ensuite par les instructions de l'opérateur.

D'une façon générale, l'utilisateur peut être aidé par un système expert dans le choix des odeurs à essayer.

Ce système expert est par exemple un programme exécuté sur l'appareil avec lequel le distributeur communique ou sur le distributeur lui-même, qui se fonde sur des réponses à un questionnaire et/ou sur des mesures, par exemple de l'odeur de la peau ou corps, effectuées par un capteur spécifique. L'utilisateur peut ainsi se faire aider par une évaluation instrumentée, par exemple un capteur de d'odeurs. Le système expert peut encore être implémenté sur un serveur distant avec lequel l'appareil ou le distributeur échange des informations. L'opérateur peut encore envoyer une image de son visage à un spécialiste qui peut préprogrammer le choix des odeurs de départ. Dans un autre exemple de mise en oeuvre, l'utilisateur présente au système informatique une photo de son visage, et le système informatique est agencé pour l'analyser et créer un programme définissant les zones à tester et les premiers produits à délivrer, à la fois en odeur et quantité. Par exemple, le système informatique peut être agencé pour sélectionner automatiquement des odeurs à proposer à l'utilisateur en effectuant l'acquisition d'une photographie à l'étape 1070, comme illustré à la figure 42. Par exemple, l'appareil qui communique avec le distributeur 11 est équipé d'une caméra, et l'utilisateur prend une photographie de son visage. L'image est ensuite analysée à l'étape 1071, et des odeurs sont proposées pour chaque zone du visage à l'étape 1072, selon par exemple des règles prédéfinies d'accord des odeurs.

Le système de distribution peut être orienté par l'utilisateur pour décider l'odeur mais aussi la quantité de produit à délivrer. Par exemple, l'utilisateur peut indiquer « cou » ou « cheveux » et le système de distribution est agencé pour adapter la dose distribuée selon une cartographie en mémoire des doses à distribuer en fonction des zones à traiter.

Le système informatique peut guider l'utilisateur dans le choix des odeurs du mélange à essayer, afin de limiter le nombre d'essais nécessaires jusqu'à l'obtention d'un résultat plaisant pour l'utilisateur.

Il est ainsi possible, comme illustré à la figure 41, qu'après l'application d'un mélange délivré par le distributeur sur une zone donnée du visage à l'étape 1060, le système informatique demande à l'utilisateur si le résultat est satisfaisant ou non, et effectue de lui-même, si le résultat est considéré comme étant non satisfaisant, un changement 1061 des paramètres du distributeur de façon à modifier le mélange distribué.

L'utilisateur n'a alors qu'a effectuer un nouvel essai avec le mélange modifié.

Lorsqu'un mélange est signalé comme étant satisfaisant, le système informatique peut mémoriser les paramètres correspondants afin de permettre de recréer ultérieurement le mélange.

Le système peut alors recommencer les étapes précédentes pour une nouvelle zone d'application.

Lors des essais successifs, l'opérateur n'a pas besoin de traiter tout le visage. Il peut par exemple choisir entre 3 et 8 zones de petites surfaces, par exemple 5 zones. Le système de distribution est alors avantageusement prévu pour interpoler et/ou extrapoler les données concernant les odeurs considérées comme adéquates, afin de calculer les odeurs qui devraient être considérées comme adéquates sur des zones sur lesquelles l'exercice n'a pas été fait.

A la fin de l'apprentissage, le système peut généner une visualisation des odeurs adéquates sur les différentes zones, testées ou calculées.

Le système de distribution peut être agencé pour signaler si certaines odeurs apparaissent aberrantes, se fiant en cela à une comparaison par rapport à des cartographies types en mémoire. Ainsi, il peut proposer à l'utilisateur de refaire tout ou partie de l'exercice de cartographie.

Une fois l'apprentissage du système informatique effectué, c'est-à-dire que des odeurs de mélanges ont été identifiées comme convenant à l'utilisateur pour parfumer certaines zones, l'utilisateur qui souhaite se parfumer n'a qu'à rappeler la zone à traiter, à l'étape 1080 de la figure 43, et le système peut proposer automatiquement à l'utilisateur à l'étape 1081 une odeur de mélange adéquate.

Dans la variante illustrée à la figure 44, l'utilisateur sélectionne une odeur à l'étape 1090 et le système informatique propose à l'étape 1091 une zone où appliquer un mélange de cette odeur sur la base d'informations précédemment collectées sur la base des tests effectués.

La zone proposée est par exemple celle où une odeur identique ou proche a déjà été appliquée et le résultat jugé acceptable par l'utilisateur.

La figure 46 illustre un exemple de mise en oeuvre de l'invention où après avoir effectué des tests sur différentes zones à l'étape 2010, l'utilisateur signale au système le ou les mélanges qu'il estime procurer le meilleur résultat, ce qui permet au système de connaître les paramètres correspondants à l'étape 2012. Ensuite, à l'étape 2014, le système peut proposer à l'utilisateur des références de produits commerciaux ayant les mêmes propriétés ou des propriétés proches.

Dans une variante, le système adresse à un centre de fabrication distant les paramètres permettant de produire une composition de même formulation ou ayant les mêmes propriétés que celle du mélange que l'utilisateur a testé et trouvé satisfaisant.

La figure 45 illustre la possibilité de délivrer à l'aide du distributeur plusieurs doses 2020a à 2020d de mélanges différents, de façon juxtaposée sur un support 2021, de façon à permettre leur application sur des régions distinctes voisines d'une même zone.

L'utilisateur peut d'un seul coup appliquer une série d'odeurs afin de cerner rapidement l'odeur adéquate. Les matières présentes sur le support 2021 peuvent avoir été choisies par l'opérateur lui-même ou proposées par le système de distribution.

Le support 2021 est par exemple mobile par rapport au boîtier du distributeur et déplacé séquentiellement pour déposer les mélanges correspondants dans les différentes zones 2020a à 2020d, étant par exemple similaire aux supports décrits en référence aux figures 29 ou 29A. L'utilisateur peut alors facilement comparer les résultats entre les différentes régions, et signaler au système le mélange produisant le meilleur effet.

On a illustré à la figure 47 un système qui permet d'aider l'utilisateur à se parfumer notamment à choisir les bonnes odeurs.

Ce système permet l'établissement d'une liaison vidéo, par exemple via internet, entre une caméra 2060 d'un premier site 2061 et un deuxième site 2062.

La caméra 2060 est par exemple intégrée à une tablette ou un téléphone intelligent qui constitue le système informatique 100.

On permet au deuxième site 2062 de piloter directement ou indirectement le distributeur 11 présent sur le premier site 2061.

Ainsi, la personne présente au premier site peut appliquer le mélange distribué et envoyer vers le deuxième site 2062 une image correspondante, pour recevoir en retour une information relative au résultat de parfumage.

Le deuxième site 2062 peut comporter un écran de visualisation 2064 qui permet à un conseiller présent devant cet écran de comprendre via les commentaires le résultat avec le mélange distribué par le distributeur et de conseiller la personne qui se parfume. Ce conseiller peut agir en retour sur le distributeur 11 pour modifier l'odeur du mélange et l'adapter au mieux au visage de la personne présente sur le premier site. Le protocole d'échange des données entre les deux sites permet ainsi l'envoi d'instructions de commande au distributeur 11, soit directement, soit par l'intermédiaire du systèmeinformatique 100 présent sur le premier site. Ainsi, la personne présente sur le deuxième site gouverne le mélange délivré par le distributeur 11. La première personne peut se parfumer sous les yeux de la seconde. La seconde personne voit sur son écran le résultat de l'essai, et ainsi peut corriger le mélange qu'elle va commander à distance, jusqu'à obtenir le parfumage idéal.

De préférence, la liaison vidéo entre les deux sites est une liaison duplex, de sorte que l'utilisateur présent sur le premier site peut voir sur l'écran du système informatique l'image du conseiller. Ce dernier peut adresser à l'utilisateur présent sur le premier site un tutoriel, le cas échéant.

La mémorisation des paramètres de réglage du distributeur 11, une fois qu'un mélange donné est estimé satisfaisant, peut être commandée depuis le deuxième site.

L'interface peut servir à définir des programmes de parfumage où l'on définit l'ordre des zones à traiter ou l'ordre des odeurs à proposer.

### Exemples

On réalise un distributeur 11 tel que celui illustré à la figure 3.

L'utilisateur détermine l'odeur voulue avec l'application qui s'éxécute sur la tablette et qui calcule les fractions des différents produits. La tablette communique cette valeur au distributeur par liaison Bluetooth.

L'électronique intégrée au distributeur 11 récupère l'information et ajuste automatiquement les débits des trois cartouches de façon à obtenir un mélange de l'odeur souhaitée.

Quand l'utilisateur veut utiliser le produit, il appuie sur le bouton 12 du distributeur pour faire sortir le produit. Il presse aussi longtemps qu'il veut du produit, en mode « continu ». En mode « dose », l'utilisateur presse une fois le bouton 12 et la dose prédéfinie est délivrée.

La distribution peut se faire en continu, c'est-à-dire les moteurs fonctionnent en continu, tout le volume étant distribué d'une traite, ou de façon itérative, les moteurs fonctionnant alors de façon impulsionnelle ; dans ce cas, l'intervalle de temps entre deux impulsions permet de faire varier le débit. De petits volumes sont délivrés les uns après les autres en plusieurs étapes.

Les impulsions peuvent être séparées par exemple par des intervalles de 50 ms, 100 ms ou 200 ms. La durée d'une impulsion pendant laquelle le moteur tourne va parexemple de 50 à 150 ms.

La page principale de l'application « µMix » comporte dans cet exemple les éléments suivants, comme visible notamment sur la figure 31 :
- Barre de statut en haut de l'écran : indique l'état de la connexion Bluetooth ou µMix s'il n'y a pas de connexion Bluetooth ;
- Barre d'onglets en bas de l'écran : permet de sélectionner la page active : page principale, Réglages, Bluetooth, Produits et Favoris ;
- Bouton Continu 304 pour sélectionner le mode de distribution de produits en continu ;
- Bouton Purge 305 pour sélectionner le mode Purge ;
- Bouton Dose 306 pour sélectionner le mode de distribution par dose avec le volume de dose associé au bouton Dose ;
- Balle bleue 300 que l'utilisateur peut déplacer dans le triangle volumique en la faisant glisser ou avec un double tapotement ;
- Boutons « - » 302 pour chaque produit A, B et C : diminue la fraction de produit sélectionné en se déplaçant le long de la droite reliant le point au sommet du produit sélectionné ;
- Boutons « + » 302 pour chaque produit A, B et C : augmente la fraction de produit sélectionné en se déplaçant le long de la droite reliant le point au sommet du produit sélectionné ;
- Fraction volumique en pourcentage de chaque produit : modifiable par l'utilisateur et mise à jour en temps réel selon la consigne des boutons 302 + et - et de la position de la balle 300.

Lors de la modification des fractions volumiques par déplacement de la balle ou par les boutons + et -, les valeurs des fractions volumiques des produits A, B et C sont mises à jour automatiquement. Lorsque les fractions volumiques sont modifiées avec les boutons + et -, la balle 300 est déplacée automatiquement à la position correspondante sur le triangle.

Au démarrage de l'application exécutée sur la tablette, celle-ci se connecte automatiquement au distributeur 11 s'il est détecté. Lorsque le distributeur s'éteint ou que la connexion Bluetooth est coupée, la tablette se déconnecte. Quand l'utilisateur agit sur les curseurs de réglage des proportions des produits A et B, les valeurs sont transmises en temps réel au distributeur 11.

La page Réglage de l'application comporte les éléments suivants :
- Barre de statut en haut de l'écran : indique l'état de la connexion Bluetooth ou µMix s'il n'y a pas de connexion Bluetooth ;
- Barre d'onglets en bas de l'écran : permet de sélectionner la page active :
   page principale, Réglages, Bluetooth ou Info ;
- Partie « Volumes » avec un champ de texte à remplir par l'utilisateur pour définir le volume de la dose, en ml (2 ml par exemple) et un champ pour le volume de la purge, en ml (3 ml par exemple). Les doses minimales sont dans cet exemple de 0.023 ml et les doses maximales 9.90 ml (3 x 3.3 ml) ;
- Partie « Débit » avec la sélection du débit : rapide (» 0.03 ml/s), moyen (» 0.02 ml/s) ou lent (» 0.01 ml/s) ;
- Partie « Dose » avec le choix du mélange itératif, pour distribuer un mélange de produits avec des petits volumes délivrés les uns après les autres en plusieurs étapes ;
   Dans le cas contraire, le volume total de chaque produit est distribué d'une traite ;
- Partie « Image Triangle » pour choisir l'image du triangle qui sera affichée sur la page principale afin de pouvoir afficher un triangle avec les odeurs délivrées par le distributeur 11. En utilisant un bouton « Choix image » de la page « Réglages » on accède à un album.

La page « Produits » de l'application comporte dans l'exemple considéré les éléments suivants :
- Choix de la valeur de chaque produit en unités de pas codeurs, de 0 à 1414.

Chaque unité correspond à un volume de produit délivré de 2.33 µl, qui est la plus petite quantité que le distributeur dans cet exemple peut délivrer ; lorsque cette feuille est affichée, ce sont les valeurs de produits de cette feuille qui sont transmises en temps réel au distributeur. Dès que la feuille n'est plus affichée, les valeurs envoyées au distributeur sont celles de la feuille principale avec le triangle ;
- Affichage des couples moteur A, B et C en temps réel avec rafraîchissement toutes les 45 valeurs.

Le mode de délivrance des produits est le mode dose directe ou itératif, selon l'option choisie dans la feuille Réglage.

La page « Favoris » permet de sauvegarder des configurations en fichier. Elle donne accès dans l'exemple considéré à 10 fichiers, à savoir « Configuration 1 » à « Configuration 10 » en plus du fichier par défaut. Ces fichiers enregistrent par exemple les paramètres suivants
- fractions des produits A, B et C
- volume de Purge
- volume de Dose,
- débit rapide, moyen ou lent,
- mode Dose, Purge ou Continu,
- distribution continue ou itérative.

On a réalisé plusieurs tests avec le système de distribution.

### Exemple 1(neuvième apsect de l'invention)

On réalise un set de trois produits de base :
F1 : (odeur de peau d'orange)

| | |
|---|---|
| n-décanal | 6% |
| n-octanal | 5% |
| Klucel H CS | 1% |
| Ethanol absolu qs | 100 |

F2 (odeur de rose)

| | |
|---|---|
| Phényléthyl éthanol | 6% |
| Klucel H CS | 1% |
| Ethanol absolu qs | 100 |

F3 (odeur d'anis)

| | |
|---|---|
| Anethole | 2% |
| Klucel H CS | 1% |
| Ethanol absolu qs 100 | |

Chaque composition présente une rhéologie allant de 2 à 2,7 Pa.s.

On met les trois cartouches dans les trois compartiments, C1, C2 et C3

Puis, on réalise plusieurs mélanges (200mg) :
Mélange 1 : C1 0%, C2 50%, C3 50%
Mélange 2 : C1 50%, C2 50%, C3 0%
Mélange 3 : C1 50%, C2 0%, C3 50%
Mélange 4 : C1 34%, C2 33%, C3 33%

On compare les notes olfactives de ces mélanges à des mélanges réalisés par balance de précision sur 9g.

### Exemple 2 (neuvième aspect de l'invention)

On réalise les mêmes mélanges que dans l'exemple 1 avec les formules suivantes :
F1 : (odeur de peau d'orange)

| | |
|---|---|
| n-décanal | 6% |
| n-octanal | 5% |
| Klucel M CS | 1% |
| Ethanol absolu qs 100 | |

F2 (odeur de rose)

| | |
|---|---|
| Phényléthyl éthanol | 6% |
| Klucel M CS | 1% |
| Ethanol absolu qs 100 | |

F3 (odeur d'anis)

| | |
|---|---|
| Anethole | 2% |
| Klucel M CS | 1% |
| Ethanol absolu qs 100 | |

On compare les notes olfactives de ces mélanges à des mélanges réalisés par balance de précision sur 9g.

### Exemple 3 (comparatif)

On réalise les mêmes mélanges que dans l'exemple 1 avec les formules suivantes :
F1 : (odeur de peau d'orange)

| | |
|---|---|
| n-décanal | 6% |
| n-octanal | 5% |
| Ethanol absolu qs | 100 |

F2 (odeur de rose)

| | |
|---|---|
| Phényléthyl éthanol | 6% |
| Klucel M CS | 1% |
| Ethanol absolu qs | 100 |

F3 (odeur d'anis)

| | |
|---|---|
| Anethole | 2% |
| Klucel M CS | 1% |
| Ethanol absolu qs | 100 |

Les viscosités sont autour de 2 à 2,5 Pa.s pour les formules F2 et F3 et <0,02 Pa.s pour la formule F1.

La viscosité des produits est mesurée à 1 atm et à 25°C avec un viscosimètre rotatif CONTRAVES TV équipé d'un mobile MS-r3 ou MS-r4 à la fréquence de 60 Hz, après 10 minutes de rotation du mobile

On compare les notes olfactives de ces mélanges à des mélanges réalisés par balance de précision sur 9g. On note une différence olfactive dans le cas des mélanges 2, 3, et 4.
Mélange 2 : C1 50%, C2 50%, C3 0%
Mélange 3 : C1 50%, C2 0%, C3 50%
Mélange 4 : C1 34%, C2 33%, C3 33%

### Exemple 4(neuvième aspect de l'invention)

Deux personnes, une spécialiste, une débutante dans le monde des parfums, sont en relation téléphonique.

Le débutant dispose de l'appareil avec les compositions de l'exemple 1.

Le débutant souhaite mettre au point une formulation anisée et fleurie.

Dans un premier temps, le spécialiste fait régler l'appareil pour réaliser un mélange (100 mg) M1 : C1 20%, C2 0%, C3 80%, croyant satisfaire le débutant.

Le débutant exprime le fait que le mélange lui apparait trop sucré. Le spécialiste interprète cette explication et propose un mélange (100 mg) M2 : C1 20%, C40%, C3 40%, croyant aller dans le sens du désir du débutant.

Le débutant exprime le fait que le mélange lui apparait trop sucré encore. Le spécialiste interprète cette explication et propose un mélange (100 mg) M3 : C1 30%, C50%, C3 20%, croyant aller dans le sens du désir du débutant.

Le débutant exprime le fait que le mélange lui apparait presque parfait. Le spécialiste interprète cette explication et propose trois mélanges (100 mg)
M4 : C1 35%, C50%, C3 15%,
M5 : C1 25%, C50%, C3 25%,
M6 : C1 25%, C60%, C3 15%,

Le débutant choisit son mélange préféré : M5

Par la suite, on réalise en grande quantité et par pesée (25g) le mélange M5. Le débutant retrouve alors un parfum de la même qualité olfactive que ce qu'il avait déterminé lors de la séance de travail commune avec le spécialiste.

Le même essai réalisé avec les formules de l'exemple 3 est aussi possible.

Mais, il existe un écart entre le mélange sélectionné lors de la séance de travail commune avec le spécialiste et le parfum réalisé en grande quantité par pesée.

### J) Système de distribution avec ensemble de cartouches pour personnaliser les niveaux de tenue / confort

La précision de la couleur obtenue est un facteur très important pour obtenir un résultat de qualité. Mais il faut aussi pouvoir ajuster la tenue et le confort du maquillage en fonction du moment, mais aussi de l'endroit du visage. D'habitude, en utilisant un produit à forte tenue, le maquillage résiste bien, même sur les zones sollicitées. Toutefois, cette solution n'est pas satisfaisante à cause de l'inconfort créé. L'utilisation de composition confortable permet de maquiller l'ensemble du visage dont les zones les plus sensibles. Mais cette solution n'est pas très satisfaisante du fait de la mauvaise tenue sur les zones exposées.

On veut que les propriétés du film puissent être ajustées aux différentes parties du visage ou au moment afin de pouvoir avec un même système réaliser le maquillage d'un ensemble du visage, même si ses différentes parties nécessitent des tenues et conforts différents.

Il est connu que le traitement des lèvres exige une forte tenue. Il est connu que le traitement du tour de l'oeil demande un film plutôt souple et confortable, quitte à ce que la tenue soit plutôt limitée. Il est aussi connu que le traitement du fond demande un film plus confortable que résistant. D'autres zones, moins mobiles, peuvent bénéficier de films résistants sans provoquer d'inconfort (par ex, le nez).

On veut aussi que les propriétés du film puissent être ajustées aux différents moments de la journée ou de l'année afin de pouvoir avec un même système réaliser le maquillage adapté au moment.

Ainsi, il est connu qu'en certaines occasions, on souhaite que son rouge à lèvre présente la meilleure tenue possible, comme par exemple en cas de représentation ou d'un spectacle. A l'inverse, dans d'autres occasions, c'est surtout le confort qui prime, comme par exemple, lors d'un moment de convivialité.

D'habitude, la solution consiste à utiliser une série de produits réalisés pour telle ou telle partie du visage. Cette approche, très répandue, pose le problème du nombre de références à prévoir pour couvrir tous les besoins en couleur.

Dans notre cas, étant donné qu'on veut réaliser plusieurs niveaux de tenue/confort, l'approche consisterait à multiplier les produits.

De plus, dans le cas où on veut pouvoir faire varier les concentrations en ingrédients colorés (pour que l'utilisateur puisse avoir la ou les couleurs qui conviennent le mieux à chaque zone), il devient encore plus difficile de prévoir toute une série de produits, avec différentes couleurs et différents niveaux de tenue/confort.

Une possibilité est de proposer aux utilisateurs un seul système permettant l'ajustement de tenue/confort montés avec toute une série de cartouches de produit donnant une variété de qualité de films. Dans ce cas, selon la zone à traiter, le système utiliserait tel ou tel produit sans que l'utilisateur n'ait besoin de changer les cartouches de produit dans les compartiments du système. Mais cette approche n'est pas réaliste car nécessiterait que le système ait de très nombreux compartiments, et cartouches, nécessitant une technologie complexe et onéreuse.

On cherche donc à proposer un système simple permettant de régler la concentration en ingrédients colorés et en qualité de film.

Ce système est spécialement intéressant pour traiter rapidement (sans avoir à changer de systèmes ou de cartouches) toutes les zones du visage, en réglant sa couleur, et assurant les qualités de film adaptées.

Il permet aussi à l'utilisateur de tester ses couleurs et ses qualités de films jusqu'à avoir les meilleures performances en tant qu'effet visuel et confort.

L'invention vise, selon un dixième aspect, à résoudre ce problème, et elle y parvient grâce à un système de distribution d'un produit, comportant un distributeur recevant au moins deux cartouches contenant respectivement un premier produit de base et un deuxième produit de base, le distributeur permettant de délivrer au moins ces deux produits de base dans des proportions réglables, le premier produit de base comportant un solvant volatil et un polymère filmogène, le deuxième produit de base comportant une huile non volatile.

L'invention, selon ce dixième aspect, présente une ou plusieurs des caractéristiques suivantes :
- le premier produit de base est sous forme de composition anhydre liquide,
- le deuxième produit de base est sous forme de composition liquide anhydre,
- le premier produit de base comporte une émulsion inverse,
- le deuxième produit de base comporte une émulsion inverse,
- le polymère filmogène est hydrophobe,
- la propriété du film qui varie est sa souplesse,
- la propriété du film qui varie étant son caractère huileux,
- la propriété du film qui varie est son caractère tenseur,
- les deux produits de base sont choisis pour réagir ensemble afin de former un film,
- le premier produit de base comporte une silicone réactive et le second produit de base un catalyseur provoquant la réticulation de la silicone réactive,
- la silicone réactive comporte comportant un mélange de polyorganosiloxane téléchélique portant une fonction vinyl aux deux extrémités de chaîne et de polyhydrogénosiloxane,
- le catalyseur est à base de platine,
- le premier produit de base comporte un polymère filmogène et le deuxième produit de base un composé huileux liquide à température ambiante,
- le polymère filmogène présente une température de transition vitreuse Tg supérieure ou égale à 30°C, mieux supérieure ou égale à 60°C,
- le polymère filmogène est choisi parmi les polymères ou copolymères vinyliques, notamment acryliques,
- le polymère filmogène est liposoluble,
- les premier et deuxième produits de base contiennent des polymères filmogènes respectifs différents,
- le polymère filmogène du premier produit de base a une température de transition vitreuse Tg1 et le polymère du deuxième produit de base a une température de transition vitreuse Tg2 différente de Tg1, avec de préférence Tg1>60°C et Tg2<10°C,
- le premier produit de base comporte un polymère ou copolymère choisi parmi les polymères et copolymères méthacryliques, les polyamides, les alkycelluloses, les polymères et copolymères de la vinylpyrolidone, et les résines silicones,
- le deuxième produit de base comporte un polymère ou copolymère choisi parmi les polymères et copolymères acryliques, vinyliques et les polycondensats tels que les polyesters et les polyuréthanes,
- le premier produit de base comporte un composé huileux de température de fusion Tfl>20°C et le deuxième produit de base comporte un composé huileux de température de fusion Tf2<20°C.

L'invention a encore pour objet selon son dixième aspect un procédé d'application d'un produit cosmétique, de maquillage et/ou de soin, sur les matières kératiniques humaines, comportant le réglage du distributeur en fonction de la zone à traiter, et la distribution du produit par prélèvement dans les cartouches des produits de base dans les proportions correspondant au réglage du distributeur.

L'invention se base ainsi selon son dixième aspect sur :
- Un système de distribution

Au moins deux compartiments qui comprennent de préférence des émulsions inverses différentes. L'une avec un solvant volatil et un filmogène. L'autre avec une huile non volatile.
- On peut programmer le système pour qu'il délivre pour chaque endroit du visage le meilleur mélange.

L'invention permet selon ce dixième aspect de traiter une ou plusieurs zones du visage et d'obtenir des mélanges très précis sur le plan de la fidélité des couleurs et un confort et une tenue adaptés permettant d'obtenir des effets particulièrement performants. Par la suite, on désigne par « zone » une partie du visage définie et assez réduite en surface, d'étendue comprise entre 1 cm2 et 100 cm2, mieux allant de 2 cm2 à 50 cm2.

Cette notion vaut ici pour tous traitements déposant un film sur la peau, coloré, masquant, ou non. Par exemple, la notion comprend l'application de film protecteur (avec filtres) ou traitant (avec actifs biologiques) ou tenseur.

Le système selon l'invention peut faire varier le film produit, produisant un effet de forte tenue ou de fort confort, et des intermédiaires. Ainsi, selon les réglages choisis, les propriétés de tenue/confort du film ne sont pas les mêmes.

### Système de distribution

Le système de distribution peut être constitué par un seul appareil fonctionnant de manière autonome, de préférence manipulable d'une main, ou par un appareil qui fonctionne en interagissant avec d'autres pièces ou appareils. Il peut s'agir par exemple de différentes interfaces de sorties qui sont montées sur le distributeur en fonction du type de maquillage à réaliser, comme cela sera précisé plus loin. Il peut s'agir également d'un système informatique qui échange des informations avec le distributeur pour le piloter, ce système informatique comportant par exemple un terminal portable tel qu'un téléphone intelligent, un photophone, une tablette, un ordinateur personnel, ou un terminal dédié.

De préférence, le distributeur est agencé pour mettre sous pression un ou plusieurs compartiments contenant le ou les produits de base, par le biais de dispositifs de dosage volumétrique, de préférence un moteur entraînant en déplacement un piston dans le compartiment correspondant.

Le distributeur peut être formé d'un boîtier et d'au moins deux ou trois compartiments, et de préférence d'autant de moteurs. Par exemple, la rotation des moteurs entraîne des vis sans fin, lesquelles poussent les pistons de chaque compartiment. L'avance des pistons est par exemple contrôlée par le nombre d'impulsions de commande envoyées aux moteurs et/ou par la durée de fonctionnement de ces derniers. Les moteurs peuvent être alimentés séquentiellement, ou de préférence simultanément.

Par exemple, les moteurs sont alimentés durant un cycle élémentaire de fonctionnement pendant une courte durée les uns après les autres, ou les uns en même temps que les autres, de façon à distribuer des microdoses correspondantes.

Les cycles élémentaires sont répétés, avec éventuellement un temps d'arrêt entre eux, qui laisse le temps aux produits de base de s'écouler hors des compartiments.

Les compartiments peuvent être définis par des cartouches qui se retirent lorsqu'elles sont vides. En variante, les compartiments sont présents à demeure et remplis à nouveau une fois vide.

Chaque cartouche peut être fermée par un bouchon démontable pour permettre le nettoyage de la cartouche.

De préférence, le boîtier du distributeur est de forme allongée selon un axe longitudinal, ce qui peut faciliter sa manipulation, et les cartouches sont disposées autour de cet axe, dans le boîtier.

De préférence, les cartouches sont mises en place par l'arrière, et le mélange est délivré par l'avant. La mise en place des cartouches peut s'effectuer individuellement, ou en variante les cartouches constituent un ensemble monobloc lors de leur mise en place.

Les cartouches peuvent comporter chacune un mécanisme de dosage volumétrique, comportant un piston mu par un mécanisme d'entraînement du distributeur, dans une direction s'accompagnant d'une diminution du volume intérieur contenant le produit de base et d'une expulsion du produit. Il peut être intéressant que les cartouches aient une région de leur paroi au moins qui soit transparente, afin de permettre de voir la couleur du produit contenu à l'intérieur.

Le mécanisme d'entraînement peut comporter un système de motorisation formé de moteurs couplés à des réducteurs, de forme allongée parallèlement à l'axe longitudinal du distributeur, et disposés entre les cartouches. Ce positionnement des moteurs et cartouches rend le distributeur particulièrement compact.

Le produit de base peut sortir de façon étanche de la cartouche correspondante puis circuler dans un canal prévu à cet effet dans le boîtier du distributeur, avant de sortir de celui-ci.

Les cartouches se terminent avantageusement par un embout réalisé de telle sorte que, une fois la cartouche insérée dans le boîtier du distributeur, l'extrémité de l'embout débouche au ras du boîtier. En variante, l'embout est suffisamment long pour dépasser du boîtier et connecter ainsi différentes interfaces de sortie susceptibles d'être rapportées sur le boîtier du distributeur.

Grâce au mécanisme d'entraînement comportant des moteurs pour faire avancer des pistons, il est possible de délivrer de façon précise des mélanges en de très petites quantités. Ainsi, le mécanisme d'entraînement peut délivrer les produits de base avec à un débit minimal inférieur ou égal à 50 µL/s, mieux inférieur ou égal à 20 µL/s, encore mieux inférieur ou égal 10 µL/s. De préférence, le mécanisme d'entraînement délivre des débits compris entre 20 et 100 µL/s, mieux compris entre 40 µL/s et 60 µL/s. Il est donc possible de réaliser facilement un mélange d'environ 10 mg. Un tel système de distribution est alors idéal pour réaliser de petites touches de maquillage, pour couvrir par exemple une zone de 1 cm², mieux une zone de 0, 5 cm².

Il est aussi possible de réaliser des plus grandes quantités de mélange comme celles nécessaires au maquillage d'une joue, ou d'un visage. Ces quantités restent néanmoins relativement faibles, par exemple une quantité comprise entre 100 et 500 mg, mieux comprise entre 150 et 250 mg.

De préférence, la cartouche comporte un embout de distribution par lequel s'effectue la sortie du produit, et cet embout de distribution est entraîné en rotation par le mécanisme d'entraînement pour déplacer le piston. L'embout peut comporter au moins un relief d'anti-rotation, mieux deux ergots d'anti-rotation diamétralement opposés.

L'embout peut porter un joint d'étanchéité, notamment un joint torique. Ainsi, au changement de cartouche, le joint est également changé, ce qui permet de s'affranchir de l'usure du joint.

Le distributeur peut comporter une carte électronique de pilotage du mécanisme d'entraînement motorisé, cette carte électronique étant traversée par le ou les embouts. Cela peut permettre de réaliser une carte s'étendant sensiblement sur toute la section du distributeur, donc de regrouper sur une seule carte tous les composants électroniques du distributeur, et ainsi de gagner en compacité et fiabilité. La carte peut s'étendre sensiblement perpendiculairement à l'axe longitudinal du boîtier. La carte peut porter un interrupteur de commande du fonctionnement du distributeur.

Le système de distribution peut être agencé pour fonctionner selon au moins deux modes de distribution.

Dans un premier mode, dit « continu », le mélange est distribué tant qu'une pression est exercée sur l'interrupteur de commande.

Dans un deuxième mode, dit « dose », une quantité prédéfinie du mélange est distribuée à chaque pression sur l'interrupteur.

Le ou les embouts peuvent déboucher à une extrémité du boîtier. Cela peut permettre de réduire le volume mort, comme cela sera détaillé dans la suite.

Le ou les embouts peuvent présenter à leur extrémité un système d'obturation pour éviter le sechage des produits dans le conduit, par exemple une membrane cicatrisante

La cartouche peut comporter une vis creuse sur laquelle le piston est vissé, le piston pouvant se déplacer axialement sur la vis lorsque celle-ci tourne ; le piston est empêché de tourner dans le corps de la cartouche. Par exemple, la friction du piston sur le corps de la cartouche peut suffire à l'empêcher de tourner quand la vis tourne.

Préférentiellement, la rotation est rendue impossible avec un corps de cartouche de section non circulaire et un piston non déformable.

Le couple des moteurs peut être déterminé électroniquement en fonction du courant absorbé, et servir par exemple à détecter une fin de course du piston. Une information concernant le couple peut être transmise à distance à un système informatique présentant une interface homme machine, pour permettre de surveiller le bon fonctionnement du distributeur.

Afin d'ajuster la teinte, le système de distribution selon l'invention doit permettre à l'utilisateur de faire varier le volume délivré provenant de chaque compartiment.

De façon préférée, le distributeur est piloté par un système informatique, qui est intégré au distributeur, ou externe à celui-ci, le distributeur étant alors apte à échanger avec le système informatique des informations par protocole sans fil ou câblé.

Le distributeur peut ainsi être piloté pour permettre l'ajustement de la teinte par distribution simultanée ou séquentielle, et contrôlée, de plusieurs produits de base de couleurs différentes.

La distribution des produits de base peut être continue ; si dans ce cas, les volumes de chacun des produits de base sont distribués d'une seule traite, simultanément ou successivement.

Dans le cas d'une distribution simultanée, il est utile de pouvoir ajuster les débits respectifs des différents produits de base pour que le mélange distribué corresponde à celui souhaité à chaque instant. Un tel mode de distribution peut convenir en particulier lors de la distribution du mélange par pulvérisation, à l'aide d'un aérographe. Pour ajuster les débits il est possible par exemple de jouer sur la vitesse de déplacement des pistons, par exemple, dans le cas d'un entrainement des pistons par une vis sans fin en faisant varier la vitesse de rotation des moteurs qui entrainent la vis. Les produits peuvent aussi être distribués de façon impulsionnelle avec un temps de distribution et un temps d'arrêt à chaque cycle. En jouant sur le rapport cyclique, il est possible d'agir sur le débit.

Tous les produits peuvent être délivrés simultanément lors du temps de distribution ; en variante, les cycles des différents produits sont déphasés, de sorte qu'un produit est distribué pendant un temps d'arrêt des autres produits.

Dans un mode particulier de l'invention, le mélange est délivré dans une cavité d'un contenant, pouvant fermer de façon hermétique ou non, par exemple en forme de coupelle, dans lequel on peut faire glisser un applicateur, en particulier un stylet ou une brosse. Un tel système de distribution est spécialement adapté aux liners, gloss et autres formules appliquées sans contact direct avec les mains. Ce contenant peut être amovible.

Par exemple, il sert de distributeur de rouge à lèvres et dispose d'un système de distribution, à vis par exemple. Lorsqu'il n'est pas amovible, le contenant peut être réalisé avec le corps du distributeur. Lorsqu'il est amovible, il peut constituer une interface de sortie parmi d'autres pouvant être montées sur le distributeur.

Les compartiments, et en particulier les cartouches, peuvent contenir tout ou partie du mécanisme d'entraînement, et par exemple le système de motorisation, ou mieux, une partie du système de motorisation, et ce, afin de réduire le nombre de pièces en mouvement dans le corps du distributeur à l'extérieur des cartouches. Par exemple, les cartouches comportent le rotor du moteur. Une fois les cartouches installées dans le corps du distributeur, les rotors sont mis en interaction avec les stators.

Le système de distribution est avantageusement agencé pour permettre de lancer des séquences préprogrammées dans lesquelles le mélange délivré par celui-ci est modifié de façon continue ou discontinue. Un mode « dégradé » permet par exemple passer progressivement d'un mélange A à un mélange B. Dans le cas d'une application par pulvérisation, notamment à l'aide d'un aérographe, cela permet de réaliser simplement les dégradés. Un mode « Alternatif » permet par exemple de passer rapidement d'un mélange

A à un mélange B plusieurs fois de suite. Dans le cas d'une application par pulvérisation, on peut ainsi réaliser un dépôt multicouche, avec des formulations différentes pour deux couches adjacentes superposées. Un autre mode permet par exemple de proposer plusieursmélanges successifs préprogrammés, le système informatique indiquant à chaque fois l'usage qu'il faut en faire à l'utilisateur, par exemple par affichage sur un écran.

Dans le cas d'une application manuelle, les mélanges sont distribués par exemple dans une coupelle. La personne se maquille à l'endroit recommandé avec un mélange correspondant prélevé dans la coupelle, puis nettoie éventuellement la coupelle et commande la délivrance d'un nouveau mélange ; l'opération est répétée autant de fois que nécessaire pour se maquiller complètement.

L'homogénisation des mélanges distribués peut se faire de différentes façons selon les cas d'utilisation. Dans le cas d'une application manuelle, elle peut se faire directement sur la zone d'application au moment de l'application ou dans la coupelle avant prélèvement ; dans le cas d'une application à l'aérographe, c'est la tuyère de l'aérographe qui sert de chambre de mélange ; dans le cas ou le mélange est distribué dans un contenanten vue d'une utilisation ultérieure, l'homogénéisation peut être faite à la main ou en faisant passer les produits distribués dans une chambre de mélange, située entre le distributeur et le contenant ou directement intégrée au contenant, comme détaillé par la suite.

Le produit peut être délivré par le système de distribution et utilisé extemporanément. En variante, le produit délivré par le système de distribution est conditionné et utilisé ultérieurement, par exemple à plusieurs reprises, avec par exemple un jour d'intervalle au moins entre deux utilisations.

Dans des exemples de mise en oeuvre préférés :
Le compartiment 1 comporte une composition C1
Le compartiment 2 comporte une composition C2

Le contact entre C1 et C2, à différents ratios, induit différentes qualités de tenue/confort de film.

La composition C1 comportede préférence une phase continue lipophile (émulsion inverse ou composition anhydre liquide) comprenant un polymère filmogène et un solvant volatil.

La composition C2 comporte de préférence une phase continue lipophile (émulsion inverse ou composition anhydre liquide) comprenant une huile non volatile.

### Polymère filmogène

Ce type de polymère est particulièrement avantageux dans la mesure où il permet d'augmenter la tenue de la matité dans le temps de manière significative.

Selon une forme particulièrement préférée de l'invention, les compositions selon l'invention comprennent au moins un polymère filmogène hydrophobe.

Par « polymère », on entend au sens de l'invention un composé correspondant à la répétition d'un ou plusieurs motifs (ces motifs étant issus de composés appelés monomères). Ce ou ces motifs sont répétés au moins deux fois et de préférence au moins 3 fois.

Au sens de la présente invention, on entend désigner par « polymère filmogène hydrophobe », un polymère filmogène dénué d'une affinité pour l'eau et à ce titre ne se prêtant pas une formulation à l'état de soluté dans un milieu aqueux. En particulier, par polymère hydrophobe, on entend un polymère ayant une solubilité dans l'eau à 25 C inférieure à 1% en poids.

Par polymère « filmogène », on entend un polymère apte à former à lui seul ou en présence d'un agent auxiliaire de filmification, un film macroscopiquement continu sur un support, notamment sur les matières kératiniques, et de préférence un film cohésif, et mieux encore un film dont la cohésion et les propriétés mécaniques sont telles que ledit film peut être isolable et manipulable isolément, par exemple lorsque ledit film est réalisé par coulage sur une surface antiadhérente comme une surface téflonnée ou siliconée.

En particulier, le polymère filmogène hydrophobe est un polymère choisi parmi le groupe comprenant :
- les polymères filmogènes solubles dans un milieu solvant organique, en particulier les polymères liposolubles ; cela signifie que le polymère est soluble ou miscible dans le milieu organique et va former une seule phase homogène lorsqu'il sera incorporé dans le milieu ;
- les polymères filmogènes dispersibles dans un milieu solvant organique, cela signifie que le polymère forme une phase insoluble dans le milieu organique, le polymère restant stable et/ou compatible une fois incorporé dans ce milieu. En particulier, de tels polymères peuvent se présenter sous la forme de dispersions non aqueuses de particules de polymères, de préférence des dispersions dans les huiles siliconées ou hydrocarbonées ; dans un mode de réalisation, les dispersions non aqueuses de polymère comprennent des particules polymères stabilisées sur leur surface par au moins un agent stabilisant ; ces dispersions non aqueuses sont souvent appelées « NAD (non-aqueous dispersions) » ;
- les polymères filmogènes sous forme de dispersions aqueuses de particules de polymère, cela signifie que le polymère forme une phase insoluble dans l'eau, le polymère restant stable et/ou compatible une fois incorporé dans l'eau, les particules de polymère pouvant être stabilisées sur leur surface par au moins un agent stabilisant. Ces particules de polymère sont souvent appelées « latex ».

Comme polymère filmogène hydrophobe, on peut citer notamment les homopolymères et les copolymères de composé à motif éthylénique, les polymères et copolymères acryliques, les polyuréthanes, les polyesters, les polyurées, les polymères cellulosiques comme la nitrocellulose, les polymères siliconés tels que les résines de silicone, les polyamides de silicone, les polymères à squelette organique non-siliconé greffé par des monomères contenant un polysiloxane, les polymères et copolymères polyamide, les polyisoprènes.

En particulier, le ou lesdits polymères filmogènes hydrophobes sont présents en tout ou partie, et de préférence uniquement, dans la phase huileuse gélifiée.

Comme polymères filmogènes hydrophobes convenant tout particulièrement à l'invention peuvent notamment être cités les polymères éthyléniques séquencés, les polymères vinyliques comprenant au moins un dérivé de dendrimère carbosiloxane et les résines siliconées (résine T, résine MQ).

### Solvant volatil

Par « solvant volatil », on entend, au sens de l'invention, toute huile susceptible de s'évaporer au contact de la peau en moins d'une heure, à température ambiante et pression atmosphérique.

L'huile volatile est un composé cosmétique volatil, liquide à température ambiante, ayant notamment une pression de vapeur non nulle, à température ambiante et pression atmosphérique, notamment ayant une pression de vapeur allant de 0,13 Pa à 40000 Pa (10⁻³ à 300 mm de Hg), en particulier allant de 1,3 Pa à 13000 Pa (0,01 à 100 mm de Hg), et plus particulièrement allant de 1,3 Pa à 1300 Pa (0,01 à 10 mm de Hg).

Les huiles volatiles peuvent être hydrocarbonées, ou siliconées.

On peut notamment citer parmi les huiles volatiles hydrocarbonées ayant de 8 à 16 atomes de carbone les alcanes ramifiés en C8-C16 comme les iso-alcanes (appelées aussi isoparaffines) en C8-C16, l'isododécane, l'isodécane, l'isohexadécane et par exemple les huiles vendues sous les noms commerciaux d'Isopars ou de Permetyls, les esters ramifiés en C8-C16 comme le néopentanoate d'iso-hexyle, et leurs mélanges. De préférence, l'huile volatile hydrocarbonée est choisie parmi les huiles volatiles hydrocarbonées ayant de 8 à 16 atomes de carbone et leurs mélanges, en particulier parmi l'isododécane, l'isodécane, l'isohexadécane, et est notamment l'isohexadécane.

On peut également citer les alcanes linéaires volatils comprenant de 8 à 16 atomes de carbone, en particulier de 10 à 15 atomes de carbone, et plus particulièrement de 11 à 13 atomes de carbone, par exemple tels que le n-dodécane (C12) et le n-tétradécane (C14) vendus par Sasol respectivement sous les références PARAFOL 12-97^{®} et PARAFOL 14-97^{®}, ainsi que leurs mélanges, le mélange undécane-tridécane, les mélanges de n-undécane (C11) et de n-tridécane (C13) obtenus aux exemples 1 et 2 de la demande WO 2008/155059 de la Société Cognis, et leurs mélanges.

Comme huiles volatiles siliconées, on peut citer les huiles volatiles siliconées linéaires telles que l'hexamethyldisiloxane, l'octamethyltrisiloxane, le decamethyltetrasiloxane, le tetradecamethylhexasiloxane, l'hexadecamethylheptasiloxane et le dodecaméthylpentasiloxane.

Comme huiles volatiles siliconées cycliques, on peut citer l'hexamethylcyclotrisiloxane, l'octamethylcylotetrasiloxane, le decamethylcyclopentasiloxane et le dodecamethylcyclohexasiloxane.

### Huile non volatile

Par « huile non volatile », on entend une huile restant sur la peau à température ambiante et pression atmosphérique au moins plusieurs heures et ayant notamment une pression de vapeur inférieure à 0,13 Pa (0,01 mm de Hg).

Ces huiles non volatiles peuvent être des huiles hydrocarbonées notamment d'origine animale ou végétale, des huiles siliconées, ou leurs mélanges. On entend par "huile hydrocarbonée", une huile contenant principalement des atomes d'hydrogène et de carbone et éventuellement des atomes d'oxygène, d'azote, de soufre et/ou de phosphore.

Comme huile hydrocarbonée non volatile, on peut notamment citer :
- les huiles hydrocarbonées d'origine animale,
- les huiles hydrocarbonées d'origine végétale telles que les triglycérides constitués d'esters d'acides gras et de glycérol dont les acides gras peuvent avoir des longueurs de chaînes variées de C4 à C24, ces dernières pouvant être linéaires ou ramifiées, saturées ou insaturées; ces huiles sont notamment des triglycérides d'acide heptanoïque ou d'acide octanoïque, ou bien encore les huiles de germe de blé, de tournesol, de pépins de raisin, de sésame, de maïs, d'abricot, de ricin, de karité, d'avocat, d'olive, de soja, d'amande douce, de palme, de colza, de coton, de noisette, de macadamia, de jojoba, de luzerne, de pavot, de potimarron, de sésame, de courge, de colza, de cassis, d'onagre, de millet, d'orge, de quinoa, de seigle, de carthame, de bancoulier, de passiflore, de rosier muscat ; le beurre de karité ; ou encore les triglycérides des acides caprylique/caprique,
- les éthers de synthèse ayant de 10 à 40 atomes de carbone ;
- les hydrocarbures linéaires ou ramifiés, d'origine minérale ou synthétique tels que la vaseline, les polydécènes, le polyisobutène hydrogéné, le squalane, les huiles de paraffine, et leurs mélanges,
- les esters de synthèse comme les huiles de formule R1COOR2 dans laquelle R1 représente le reste d'un acide gras linéaire ou ramifié comportant de 1 à 40 atomes de carbone et R2 représente une chaîne hydrocarbonée notamment ramifiée contenant de 1 à 40 atomes de carbone à condition que R1 + R2 soit ≥ 10, comme par exemple l'huile de Purcellin (octanoate de cétostéaryle), le myristate d'isopropyle, le palmitate d'isopropyle, les benzoates d'alcools en C12 à C15, le laurate d'hexyle, l'adipate de diisopropyle, l'isononanoate d'isononyle, le néopentanoate d'isodecyl, le palmitate de 2-éthyl-hexyle, l'isostéarate d'isostéaryle, le laurate de 2-hexyl-décyle, le palmitate de 2-octyl-décyle, le myristate de 2-octyl-dodécyle, des heptanoates, octanoates, décanoates ou ricinoléates d'alcools ou de polyalcools comme le dioctanoate de propylène glycol ; les esters hydroxylés comme le lactate d'isostéaryle, le malate de di-isostéaryle, le lactate de 2-octyl-dodécyle ; les esters de polyols et les esters du pentaérythritol,
- les alcools gras liquides à température ambiante à chaîne carbonée ramifiée et/ou insaturée ayant de 12 à 26 atomes de carbone comme l'octyl dodécanol, l'alcool isostéarylique, l'alcool oléique, le 2-hexyldécanol, le 2-butyloctanol, et le 2-undécylpentadécanol,
- les acides gras supérieurs tels que l'acide oléique, l'acide linoléique, l'acide linolénique et leurs mélanges.

Les huiles de silicone non volatiles utilisables dans la composition selon l'invention peuvent être les polydiméthylsiloxanes (PDMS) non volatiles, les polydiméthylsiloxanes comportant des groupements alkyle ou alcoxy pendants et/ou en bouts de chaîne siliconée, groupements ayant chacun de 2 à 24 atomes de carbone, les silicones phénylées comme les phényl triméthicones, les phényl diméthicones, les phényl triméthylsiloxy diphénylsiloxanes, les diphényl diméthicones, les diphényl méthyldiphényl trisiloxanes, et leurs mélanges.

De manière préférentielle, la phase huileuse comprend au moins une huile siliconée et encore plus préférentiellement choisie parmi
- les huiles volatiles siliconées cycliques ayant une viscosité à température ambiante inférieure à 8 cSt et ayant notamment de 4 à 7 atomes de silicium, ces silicones comportant éventuellement des groupes alkyle ou alcoxy ayant de 1 à 10 atomes de carbone, en particulier choisie parmi l'hexamethylcyclotrisiloxane, l'octamethylcylotetrasiloxane, le decamethylcyclopentasiloxane et le dodecamethylcyclohexasiloxane (cyclohexasiloxane), et leurs mélanges ;
- les polydiméthylsiloxanes (PDMS) volatiles ou non volatiles (Nom INCI : DIMETHICONE) ;
- les silicones phénylées ;
- les polydiméthylsiloxanes comprenant des groupes aliphatiques, en particulier alkyle, ou alcoxy, qui sont pendants et/ou à la fin de la chaîne silicone ; ces groupes comprenant chacun de 6 à 24 atomes de carbone, et plus particulièrement le caprylyl methicone tel que le produit commercial DOW CORNING FZ-3196^{®} par la société DOW CORNING ;
- leurs mélanges.

### Optimisation

Il est très intéressant d'avoir le moins de compartiments possible.

Ainsi, si on veut que le système puisse faire un réglage de couleur, il faudrait prévoir deux compartiments en plus des deux destinés au contrôle de la qualité du film. Ainsi, il faudrait prévoir :
- les compartiments 1 et 2 contiennent les ingrédients permettant de faire varier la qualité de tenue/confort du film et
- Et d'autres compartiments (3, 4 par exemple), pour y placer les ingrédients M permettant de faire varier la couleur.

Il est aussi possible de mettre les actifs M dans les compartiments 1 ou 2 ou 1 et 2.

Ainsi, on réduit le nombre de compartiments totaux à 3, voire 2, au lieu de 4.

Par exemple, on met les actifs M dans les produits qu'on place dans les compartiments 1 et 2. Dans le compartiment 1, M est mis à concentration importante et dans le compartiment 2, M est mis à concentration faible.

On met aussi les actifs produisant le film et réglant ses propriétés de tenue/confort dans les compartiments 1 et 2. A savoir, dans l'exemple, un polymère+solvant volatile dans le compartiment 2 et le solvant non volatile dans le compartiment 1.

Si on utilise le système avec un réglage majoritaire en 1, on obtient un produit concentré solvant non volatile et en actif M.

Le réglage majoritaire en 1 peut alors être utilisé sur des zones de fond de teint, où on désire une forte couvrance et un film confortable.

Le réglage majoritaire en 2 peut être utilisé sur les zones du visage tachées. La faible quantité d'actif coloré M ne permet pas d'obtenir un fort masquage mais ce masquage durera dans le temps, compensant alors le pouvoir masquant modéré. L'inconfort sur ces zones, de surface limitée, n'est pas considéré comme gênant.

Il est possible de mettre un actif M1 dans un produit destiné au compartiment 1 et un actif M2 dans un produit destiné au compartiment 2. Par exemple, M1 est un pigment jaune et M2 est un pigment rouge. On met aussi les actifs produisant le film et réglant ses propriétés de tenue/confort dans les compartiments 1 et 2. A savoir, dans l'exemple, un polymère + solvant volatil dans le compartiment 2 et l'huile non volatile dans le compartiment 1.

Le réglage majoritaire en 1 peut alors être utilisé sur des zones de fond de teint, où on désire une couleur de fond et un film confortable.

Le réglage majoritaire en 2 peut être utilisé sur les zones telles que les lèvres ou les jouées. On obtient une couleur plutôt rouge avec une forte tenue.

Il est possible de mettre un actif M1 dans un produit destiné au compartiment 1 et le même actif M1 dans un produit destiné au compartiment 2. Par exemple, M1 est un pigment jaune. On met aussi les actifs produisant le film et réglant ses propriétés de tenue/confort dans les compartiments 1 et 2. A savoir, dans l'exemple, un polymère + solvant volatil dans le compartiment 2 et l'huile non volatile dans le compartiment 1.

Le réglage majoritaire en 1 peut alors être utilisé sur des zones de fond de teint, où on désire une couleur de fond et un film confortable.

Le réglage majoritaire en 2 peut être utilisé sur les zones de contours, tels que le haut du front ou le nez. On obtient alors la même couleur que le reste du visage, mais avec une meilleure tenue, permettant alors de résister aux mouvements et frottements et mouvements des cheveux et/ou des lunettes.

Il est possible de mettre les actifs M dans un compartiment 3. Par exemple, on met un pigment jaune.

On met les actifs produisant la tenue/confort du film et réglant les propriétés de film dans les compartiments 1 et 2. A savoir, dans l'exemple, un polymère + solvant volatil dans le compartiment 2 et l'huile non volatile dans le compartiment 1.

L'invention selon ce dixième aspect n'est pas limitée aux actifs amenant des effets coloriels. On peut l'utiliser pour des actifs de soin tels que antiage, antioxydant, antiride, antitranspirant, antitache, photoprotection et hydratation.

On peut aussi mixer des actifs amenant des effets colorieles et des effets de soin.

### Utilisation pour le maquillage d'une ou plusieurs zones précises de peau

Le système de distribution permet de se maquiller jour après jour, en ne traitant que les zones à masquer. Pour ce faire, on délivre de petites doses de maquillage, qu'on applique précisément et séquentiellement sur les zones correspondantes. Chaque petite dose est réalisée selon le mélange qui convient à la zone.

Dans un mode préféré de mise en oeuvre de l'invention, le système de distribution attend qu'on le renseigne sur une zone à traiter puis délivre le mélange correspondant. Il peut faire appel à une table de correspondance préprogrammée pour cela, cette table résultant par exemple d'un procédé d'apprentissage tel que défini plus loin.

Dans un mode particulièrement intéressant, la programmation tient en compte la résistance et le confort qu'il doit atteindre pour obtenir les résultats optimums. En variante, le système de distribution renseigne la personne, tout en délivrant un mélange, sur la zone sur laquelle celle-ci doit appliquer le mélange. Ainsi, le système de distribution peut suivre un programme d'application où il délivre dans un ordre donné, les différentsmélanges à appliquer.

Dans un mode particulier de mise en oeuvre de l'invention, le système de distribution est renseigné sur les quantités à délivrer. Pour cela, il mémorise la correspondance entre la couleur, la zone du visage et la quantité nécessaire, ce qui permet de réduire les coûts et les pertes de produit, et de ne couvrir que faiblement la peau, évitant ainsi des effets d'occlusion. Ce faisant, il est possible d'utiliser des produits très couvrants et trop couvrants pour être appliqués sur l'ensemble du visage. Ainsi, il est possible d'obtenir des maquillages d'aspects naturels, voire indétectables.

Le système de distribution peut aussi permettre, en facilitant la distribution de petites quantités et leur usage rapide, de réduire la durée de conservation des produits, permettant ainsi de réduire les risques d'évolution et/ou les quantités de conservateurs à employer.

Le système de distribution est adapté à traiter les zones qu'on veut masquer, sans avoir à masquer l'ensemble du visage.

Lorsque l'utilisateur recherche la couleur à appliquer sur une zone du visage, il est intéressant de mémoriser la couleur qui va bien pour chaque zone, et le système de distribution est ainsi avantageusement agencé pour mémoriser cette couleur et la zone correspondante. Ainsi, en utilisant les informations mémorisées, à chaque utilisation, on peut obtenir la délivrance du même mélange pour chaque zone, ou, si l'on traite plusieurs zones, la même série de mélanges pour une même série de zones.

Le système de distribution peut aussi être agencé pour permettre de traiter une zone en faisant varier les couleurs application après application. Ainsi, la personne peut maquiller les lèvres avec différentes couleurs, qu'elle choisit au jour le jour selon sesgoûts. Cette approche est aussi adaptée aux paupières ou aux cils, ainsi qu'au maquillage du visage car la personne peut avoir envie de changer de couleur de teint. Par exemple, les jours de la semaine, la personne réalise un fond de teint couleur claire, et le weekend, un fond de teint de couleur hâlée, ou un maquillage des yeux d'une couleur un jour, et d'une autre couleur un autre jour.

Le système de distribution peut être agencé pour permettre à l'utilisateur de changer de couleur selon ses goûts du jour, du moment, de ses tenues, du climat. Ainsi, on prévoit avantageusement un système d'aide à la décision pour guider l'utilisateur dans ses choix de couleur.

On peut prévoir aussi un système d'aide pour équilibrer les couleurs sur un même visage et participer à la réalisation d'un maquillage global réussi.

Il peut être souhaitable que plusieurs personnes d'un même groupe, par exemple une famille, puissent utiliser le système de distribution, réduisant ainsi les coûts et minimisant la place occupée. Cette solution est spécialement adaptée aux voyages ou aux hôtels, campings, avions, campings cars, boutiques, écoles... Pour ce faire, on peut prévoir que le système de distribution puisse être renseigné sur la personne qui l'utilise, afind'accéder à des données personnelles préalablement mémorisées.

### Utilisation en continu pour un maquillage en dégradé

Dans cette application, le système de distribution, pendant qu'il délivre le produit, change la formulation du mélange. De plus, on déplace la sortie des produits de base ou du mélange relativement à un contenant ou un support définissant une surface d'application. Dans un mode particulier de mise en oeuvre de l'invention, le système de distribution est agencé pour calculer l'évolution du mélange en fonction de la couleur C1, d'une zone à traiter et de la couleur C2, d'une autre zone à traiter. Par exemple, sachant que le menton nécessite une couleur C1 et que la joue nécessite une couleur C2, le système de distribution peut faire varier la formulation du mélange pendant qu'il le délivre pouréaliser un dégradé de couleur entre ces deux couleurs. Cela permet par exemple de mieux masquer les imperfections du visage en faisant en sorte que le résultat final soit réaliste, ou de faire des dégradés de couleur à des fins d'embellissement. Le système de distribution peut encore être agencé de telle sorte que l'utilisateur puisse commander une variation de couleur du mélange distribué sans que les couleurs de départ et/ou d'arrivée n'aient été préalablement fixées. Pour ce faire, le système de distribution peut disposer d'un système de localisation, ou d'auto-localisation, et déduire d'une table de correspondance les couleurs C1 et C2 qu'il doit réaliser, et donc les évolutions de mélange qu'il doit réaliser.

Le système de distribution peut comporter une tête de sortie, en particulier dans le cas d'un aérographe, qui est mobile et pilotée. Cette option permet alors, sans bouger le reste du système de distribution, de réaliser des dégradés. Par exemple, on localise le système de distribution près de la joue, puis on déclenche un système de commande qui va piloter automatiquement la variation de la formulation du mélange et le mouvement de la tête de sortie, de façon à par exemple avoir le centre de la joue plus rouge que sa périphérie, avec un dégradé entre les deux.

Le système de distribution peut encore être utilisé pour réaliser des produits sur mesure, que l'on conserve pour réaliser plusieurs applications.

Il est aussi possible de réaliser des produits solides ou semi-solides.

### Fabrication de compacts « à façon » ou autres produits solides ou semi-solides

Le système de distribution peut être agencé pour permettre de choisir un mélange et de le délivrer dans un contenant tel qu'une coupelle. Le mélange comporte de préférence des composés qui sont tels que le mélange puisse prendre en masse.

Plus préférentiellement, on utilise des composés qui rendent la prise en masse spécialement rapide. Ces composés sont soit déposés dans le contenant avant ou après son remplissage avec les autres constituants, soit sont prévus dans les compartiments du distributeur avec les autres constituants des produits de base, soit sont compris dans le distributeur dans un compartiment spécialement prévu pour les contenir.

On peut ainsi distribuer des compositions spécifiques qui peuvent par réaction chimique, biochimique ou physicochimique, durcir de façon accélérée après évacuation.

Ces compositions sont spécialement adaptées à la réalisation des compacts, c'est-à-dire :
- Prennent en masse,
- donnent un matériau qui peut se déliter en cas de frottement, et sont préférentiellement colorées.

De préférence, ces compositions sont très riches en particules solides, avec par exemple plus de 10% en masse de particules solides par rapport à la masse totale de la composition, mieux plus de 20% en masse de particules solides par rapport à la masse totale de la composition, encore mieux plus de 30% en masse de particules solides par rapport à la masse totale de la composition, préférentiellement entre 10 et 40% en masse de particules solides par rapport à la masse totale de la composition. Ces compositions peuvent contenir des particules absorbantes, ou des composés réactifs, tels que ceux qui réagissent en contact avec l'air, par exemple du cyanoacrylate ou des alphasilanes, ou qui réagissent à la lumière, notamment UV.

Le contenant dans lequel le mélange est distribué peut comporter un composé A et les compositions distribuées un composé B, A et B étant choisis pour réagir entre eux et solidifier le mélange.

Dans un mode particulier de mise en oeuvre de l'invention, le système de distribution intègre un moyen de chauffage, par exemple à résistance électrique, pour la réalisation de rouges à lèvres ou autres produits cireux. Dans ce cas, les produits de base sont chauffés avant d'être délivrés.

Le système de distribution peut aussi comporter un moyen pour apporter de l'énergie, thermique et/ou lumineuse, après distribution du mélange dans un contenant, par exemple une résistance électrique ou une diode LED, notamment UV. Cette énergie peut accélérer la prise en masse du mélange distribué.

Préférentiellement, le mélange est homogénéisé avant la prise en masse.

### Réalisation de palettes de couleurs

Le système de distribution peut comporter un support, ayant plusieurs régions, et être agencé pour générer automatiquement plusieurs mélanges déposés dans lesdites régions, par exemple une série de couleurs adaptées à différentes parties du visage.

Le support peut définir plusieurs cavités pour recevoir les mélanges ou porter plusieurs contenants, par exemple sous forme de coupelles, éventuellement séparables du support.

Dans un cas particulier, le support prend la forme d'un visage avec des régions pour recevoir les mélanges pour des zones d'applications ciblées.

Le support être mobile, notamment rotatif, par rapport au corps du distributeur, et par exemple entraîné en déplacement par le distributeur, pour remplir successivement différents logements ou contenants.

### Distributeur à coupelle

Il existe un intérêt pour bénéficier d'un système de distribution capable de délivrer un mélange pouvant être facilement prélevé par l'utilisateur. Par ailleurs, dans le cas où les produits de base délivrés par le système de distribution ne sont pas déjà mélangés, il existe un besoin pour permettre à l'utilisateur d'effectuer aisément le mélange.

Le système de distribution peut comporter une coupelle, et un distributeur pour remplir la coupelle avec au moins un produit, la coupelle étant solidaire du distributeur au moins pendant son remplissage.

La coupelle est parfois encore appelée « creuset » et ce terme doit être compris avec une acceptation large.

Par « coupelle solidaire du distributeur » il faut comprendre que la coupelle est retenue, notamment immobilisée, au moins temporairement sur le distributeur, étant par exemple fixée sur celui-ci par des vis, par aimantation, par encliquetage, par verrouillage baïonnette, par serrage, ou réalisée avec une partie du corps du distributeur par moulage de matière. La coupelle lorsqu'elle est solidaire du distributeur permet de manipuler celui-ci d'une main, la coupelle restant en place sur le distributeur au cours des mouvements de ce dernier.

Le distributeur peut être proposé à l'utilisateur avec la coupelle déjà en place.

En variante, celle-ci est installée par l'utilisateur à la première utilisation du système de distribution.

De préférence la coupelle est moins profonde que large, ce qui facilite l'accès à celle-ci, et permet de prélever le produit, notamment le mélange avec un applicateur ou le doigt.

De préférence, la coupelle est séparable du distributeur, et constitue une interface de sortie qui peut être choisie parmi un ensemble d'interfaces de sortie pouvant être montées sur le distributeur, au choix de l'utilisateur en fonction du maquillage à réaliser, comme détaillé plus loin.

De préférence, le système de distribution comporte plusieurs orifices de remplissage en des produits de base différents, débouchant dans la coupelle. Ainsi, le mélange de ces produits peut avoir lieu dans la coupelle.

La coupelle a de préférence un fond de forme concave vers l'extérieur, ce qui peut faciliter son nettoyage par l'utilisateur entre deux utilisations.

De plus, cela peut faciliter le prélèvement du produit par l'utilisateur, et le mélange des produits de base.

De préférence, le distributeur permet de délivrer au moins deux produits de base dans la coupelle, dans des proportions réglables, et mieux au moins trois produits.

Dans un exemple de mise en oeuvre, le système de distribution comporte au moins deux coupelles pouvant être sélectivement alimentées par le distributeur. Cela peut permettre à l'utilisateur de remplir rapidement ces deux coupelles avec des mélanges dont les caractéristiques sont différentes. Cela peut faciliter les essais de matières colorées, et/ou permettre de préparer plusieurs mélanges de couleurs différentes destinées au maquillage de zones respectives du visage. Les coupelles peuvent être associées à des identifiants rappelant à l'utilisateur à quelle zone du visage un mélange contenu dans une coupelle donnée est destiné.

Les coupelles peuvent être mobiles relativement au distributeur, étant par exemple portées par un support mobile, tel qu'une tourelle mobile en rotation par rapport au distributeur, ou par un tiroir mobile en translation par rapport au distributeur.

Le système de distribution peut comporter un couvercle de fermeture de la coupelle. Ce couvercle de fermeture est de préférence transparent pour permettre à l'utilisateur de visualiser la couleur du mélange contenu à l'intérieur.

Lorsque la coupelle est séparable du distributeur, elle peut être introduite le cas échéant dans un boîtier permettant de la transporter plus facilement, ce boîtier pouvant comporter le cas échéant un miroir et/ou un applicateur. Le couvercle du boîtier peut dans ce cas servir de couvercle pour la coupelle.

Le volume de la coupelle peut être compris entre 2 et 1000 mm³, mieux entre 100 et 1000 mm³, encore mieux entre 250 et 750 mm³.

Le ou les produits de base délivrés dans la coupelle sont de préférence des fonds de teint, mais en variante il s'agit de produits de maquillage des lèvres ou des paupières.

La coupelle est de préférence de forme symétrique de révolution. En variante, elle est de contour polygonal ou autre. Son plus grand diamètre intérieur, ou celui du cercle inscrit dans le cas d'un contour non circulaire, est de préférence compris entre 2 et 100 mm, préférentiellement entre 5 et 40 mm. Sa profondeur est de préférence comprise entre 1 et 10 mm, mieux entre 3 et 8 mm. De préférence, la taille et la forme de la coupelle permettent soit une application directe du mélange sur la peau, soit une préhension au doigt ou avec un applicateur. La coupelle peut être réalisée avec un matériau élastiquement déformable, ce qui permet par exemple d'inverser la concavité du fond de la coupelle et de vider celle-ci plus facilement ou de l'utiliser pour appliquer le produit.

La coupelle peut être dépourvue de mélangeur; dans ce cas, les produits de base peuvent arriver du distributeur dans la coupelle à l'état non mélangé, par des orifices de distribution respectifs distincts. En variante, le distributeur intègre un mélangeur et les produits de base arrivent déjà mélangés dans la coupelle.

La coupelle peut aussi intégrer un mélangeur statique, comme détaillé plus loin, qui est alimenté par des orifices de remplissage distincts du distributeur, et qui délivre de préférence le mélange dans une cavité de la coupelle située au-dessus du mélangeur.

L'invention a encore pour objet un procédé de préparation d'un produit de maquillage, comportant l'étape consistant à remplir une coupelle d'un système de distribution tel que défini plus haut avec au moins un produit de base provenant du distributeur.

Plusieurs produits peuvent être délivrés dans le fond de la coupelle, puis mélangés à l'aide du doigt ou d'un applicateur, ou d'un mélangeur statique intégré à la coupelle.

La coupelle est de préférence remplie par le dessous. Il a été proposé dans le passé des systèmes de distribution à l'aide d'une sonotrode.

La coupelle selon l'invention n'est pas destinée à entrer en vibration pour distribuer le ou les produits amenés par le ou les canaux d'alimentation de celle-ci. Elle est différente d'une sonotrode. De préférence, la coupelle est réalisée en matière plastique.

### Mélangeur intégré à l'interface de sortie

Il existe un intérêt pour bénéficier d'un système de distribution capable de délivrer un mélange pouvant être facilement utilisé, notamment prélevé par l'utilisateur, sans nécessiter une action additionnelle de mélange de la part de celui-ci.

Le système de distribution peut comporter un distributeur ayant des canaux de sortie de produits de base et une interface de sortie séparable du distributeur, cette interface ayant un mélangeur statique, délivrant de préférence le mélange dans une cavité où il peut être prélevé.

Le mélangeur statique peut être situé sous la cavité précitée. Le système de distribution est alors particulièrement adapté à la création de compacts, en utilisant comme interfaces de sortie des coupelles à mélangeur statique intégré. Dans ce cas, on vient remplir la cavité de la coupelle en produit par le dessous. Après passage dans le mélangeur statique, les produits de base mélangés viennent recouvrir le mélangeur.

Il est possible d'utiliser plusieurs interfaces de sortie et de les remplir avec des mélanges respectifs différents, sans avoir à purger le mélangeur, ce qui réduit les pertes de produit. L'interface de sortie peut être à usage unique, le cas échéant.

De préférence, le mélangeur statique comporte une chambre centrale communiquant avec des canaux d'admission des produits de base. Cette chambre centrale peut communiquer avec une chambre périphérique comportant une série de cloisons qui agissent comme des déflecteurs pour le mélange, et créent un cisaillement de celui-ci.

La chambre périphérique peut comporter une cloison annulaire ajourée définissant des ajours par lesquels passe le mélange en circulant dans la chambre périphérique. Les chambres centrale et périphérique peuvent être fermées supérieurement par une paroi qui définit le fond de la cavité recevant le mélange.

Le fond de la chambre périphérique peut être de forme hélicoïdale autour de l'axe de la coupelle et de hauteur diminuant au fur et à mesure que l'on progresse vers la sortie. Cette dernière peut déboucher en avant d'une rampe de liaison entre le fond de la chambre périphérique et la paroi supérieure du mélangeur, cette rampe de liaison étant de préférence une portion d'hélice prolongeant l'hélice formée par la paroi de fond de la chambre périphérique.

De préférence la chambre périphérique comporte la cloison annulaire précitée et des cloisons radiales qui forcent le mélange à circuler alternativement entre des régions supérieure et inférieure de la chambre périphérique et entre des régions radialement intérieure et extérieure, le mélange circulant par exemple d'une région supérieure et radialement extérieure à une région inférieure et radialement extérieure en passant à travers la cloison annulaire précitée.

Le mélangeur peut comporter un corps extérieur dans lequel est reçue une pièce formant le coeur du mélangeur, le corps extérieur fermant radialement à l'extérieur la chambre périphérique et comportant un montant qui sépare les chambres centrale et périphérique.

Le corps extérieur du mélangeur et le coeur du mélangeur peuvent être chacun réalisés d'une seule pièce par moulage par injection.

### Volume mort réduit

Il existe un intérêt pour réduire les pertes de produit lors des changements de formulation du mélange, ainsi que pour permettre de faire varier le plus rapidement possible la couleur du mélange au cours de l'application, notamment en cas de couplage du distributeur avec un aérographe.

Le système de distribution peut comporter un distributeur recevant au moins deux cartouches ayant chacune un réservoir contenant un produit de base, ce dernier quittant la cartouche par un canal de sortie de la cartouche, ce canal de sortie débouchant à l'extérieur du distributeur ou à proximité de sa surface externe.

Le canal de sortie peut notamment déboucher dans une zone de prélèvement du mélange ou à proximité de celle-ci, notamment à moins de 5 mm, mieux à moins de 3 mm, mieux à moins de 1mm, mieux à fleur.

La section transversale du canal de sortie est par exemple comprise entre 1 et3 mm².

Ainsi, chaque produit de base provenant d'une cartouche peut quitter le distributeur sans se mélanger avec un produit de base d'une autre cartouche et l'on minimise le volume mort qui ne peut être prélevé et susceptible d'accroître l'inertie du système. Le produit est plus rapidement disponible sans avoir à circuler dans des canaux spécifiques du boîtier du distributeur, évitant une étape fastidieuse de purge si changement de cartouche.

L'extérieur du distributeur peut être la zone de prélèvement du produit, notamment lorsque le distributeur est réalisé avec une coupelle non prévue pour être enlevée, dans laquelle le mélange est distribué, ou une zone du distributeur destinée au montage d'une interface de sortie amovible, qui définit la zone de prélèvement. Cette interface de sortie peut comporter une coupelle telle que définie ci-dessus. Cette zone de montage correspond par exemple à l'extrémité du boîtier du distributeur en l'absence de l'interface de sortie. La zone de montage peut être sensiblement plane et perpendiculaire à l'axe longitudinal du boîtier du distributeur.

Le distributeur peut comporter trois cartouches de produits de base.

Le distributeur peut comporter des logements pour recevoir les cartouches, lesquelles sont de préférence reçues de façon amovible dans le distributeur. Ce dernier peut comporter des passages pour des conduits des cartouches définissant les canaux de sortie.

La longueur de ces conduits est de préférence telle que les conduits se situent légèrement en retrait du fond ou à affleurement de la cavité servant au prélèvement du produit, ou en variante légèrement en retrait ou à affleurement de la face d'extrémité du boîtier du distributeur définissant la zone de montage.

Ces conduits des cartouches peuvent être des embouts servant à entraîner en déplacement des pistons au sein des cartocuhes, comme détaillé plus haut.

### Interfaces de sortie multiples

Il existe un besoin pour pouvoir, à l'aide d'un même système de distribution, réaliser facilement des maquillages différents, et pouvoir si on le souhaite maquiller deszones aussi différentes que la peau, les lèvres ou les cils ou sourcils.

Le système de distribution peut comporter un ensemble comportant un distributeur d'au moins un produit cosmétique, notamment de maquillage, et au moins deux interfaces de sortie pouvant se monter chacune de façon amovible sur le distributeur, ces interfaces de sortie permettant de recevoir le ou les produits délivrés par le distributeur, étant de préférence choisies parmiles suivantes :
- interface de sortie comportant un contenant, notamment une coupelle permettant un prélèvement du produit au doigt ou à l'aide d'un applicateur,
- interface de sortie permettant de délivrer le produit à un système de pulvérisation, notamment un aérographe,
- interface de sortie comportant plusieurs régions de réception du produit, mobile par rapport au distributeur,
- interface de sortie permettant de délivrer le produit à un embout de distribution.

De préférence, l'ensemble comporte au moins trois desdites interfaces de sortie, mieux les quatre interfaces de sortie.

Le distributeur peut comporter au moins deux produits de base différents et permettre de délivrer ceux-ci dans des proportions variables, et de préférence, le distributeur comporte trois produits de base différents et permet de délivrer ceux-ci dans des proportions variables.

Chaque interface de sortie peut comporter une base permettant sa fixation sur le distributeur. Cette fixation peut s'effectuer à l'aide de vis par exemple, mais de préférence la base est agencée pour permettre un démontage et un remplacement d'une interface de sortie sans nécessité d'outil. Il s'agit par exemple d'une fixation quart de tour ou à l'aide d'une bague de verrouillage externe.

L'interface de sortie et/ou le boîtier du distributeur peuvent comporter des joints d'étanchéité permettant une communication étanche entre le boîtier du distributeur et l'interface de sortie. Le cas échéant, le distributeur est agencé pour reconnaître l'interface de sortie qui est montée dessus, par exemple grâce à identifiant de l'interface de sortie sous forme de reliefs spécifiques, lesquels sont détectés par le distributeur, ou d'une puce électronique reconnue par le distributeur. Cela peut permettre d'adapter le fonctionnement du distributeur à l'interface de sortie montée dessus. Le distributeur peut communiquer à un système informatique des informations sur l'interface de sortie qu'il porte, et le système informatique peut afficher en fonction de ces informations un écran spécifique et/ou lancer un programme spécifique de commande des paramètres de fonctionnement du distributeur, afin par exemple d'adapter la dose distribuée et/ou le débit à la nature de l'interface de sortie.

Plusieurs interfaces de sortie peuvent être proposées initialement à l'utilisateur avec un distributeur commun au sein d'un même emballage, par exemple un coffret ou une boîte de carton.

L'invention a encore pour objet un procédé de maquillage, comportant l'étape consistant à sélectionner une interface de sortie, à la monter sur le distributeur, et à délivrer le ou les produits contenus dans le distributeur dans celle-ci.

### Cartographie et apprentissage

Le terme « cartographie » s'entend ici comme un procédé d'indexation d'une couleur et d'une zone, avec enregistrement.

La cartographie peut concerner des applications sur des zones plus petites qu'un cm2. Toutefois, l'oeil nu a alors du mal à discerner si le résultat obtenu est adéquat, et il est préférable de substituer l'évaluation à l'oeil nu par une évaluation instrumentée, avec grossissement. L'application de petites quantités de matière colorée peut être faite au doigt, avec des outils classiques tels que des pinceaux, ou avec des applicateurs spécialisés.

La cartographie peut être générée lors d'une période d'apprentissage, pendant laquelle l'utilisateur effectue des tests avec des mélanges sur différentes zones du visage ; une fois renseignée, la cartographie peut ensuite être utilisée pour le maquillage de tous les jours. Cette période d'apprentissage peut tenir compte de la résistance et du confort. Ce faisant, l'utilisateur teste plusieurs réglages et renseigne le meilleur réglage obtenu.

Des interfaces graphiques spécifiques peuvent servir pendant la période d'apprentissage et pendant la période d'utilisation de la cartographie.

En particulier, le système de distribution peut être utilisé avec une interface graphique où l'opérateur voit le visage, qui est par exemple schématisé, figuratif ou précis comme une photograhie ou une simulation 3D. Dans ce cas, l'opérateur peut pointer une partie du visage sur l'écran pour voir apparaître et/ou délivrer la couleur adéquate.

L'interface graphique peut aussi laisser apparaître les autres zones du visage où l'utilisation de la même couleur est adéquate.

Pour réaliser la cartographie, l'opérateur applique une couleur puis réalise l'évaluation.

Les zones du visage peuvent être traitées les unes après les autres ; par exemple, on réalise l'exercice sur une partie de la joue, puis sur le nez, etc....

Une autre possibilité consiste à réaliser un mélange donné, et appliquer sur plusieurs zones ce même mélange. L'opérateur doit alors chercher la zone du visage pour laquelle la couleur est adaptée. Le mélange est ensuite indexé dans le système informatique en l'attribuant à la ou aux zones du visage pour lesquelles il convient.

Selon l'un de ses aspects, indépendamment ou en combinaison avec les autresaspects de celle-ci, et notamment ce qui précède, l'invention a pour objet un procédé d'apprentissage d'un système de distribution comportant un distributeur permettant de distribuer un mélange de couleur variable, et un système informatique permettant de sélectionner une couleur et de mémoriser des données, comportant les étapes consistant à :
a) Sélectionner au moins une couleur à l'aide d'une interface du système informatique,
b) délivrer à l'aide du distributeur au moins un mélange de la couleur sélectionnée,
c) évaluer le ou les mélanges distribués après application sur au moins une zone du visage,
d) mémoriser les caractéristiques d'au moins un mélange, notamment un mélange que l'utilisateur souhaite pouvoir rappeler, et d'au moins une zone sur laquelle il a été testé.

Cette mémorisation peut s'effectuer notamment en vue d'une distribution ultérieure de ce mélange pour maquiller ladite zone.

De préférence, le système informatique est agencé pour permettre à l'utilisateur de signaler si le résultat du test est satisfaisant ou non, voire de le renseigner sur la comparaison à un test effectué précédemment.

On peut aussi réaliser un mélange donné, et chercher la zone du visage pour laquelle il est adapté. On enregistre alors le mélange en l'attribuant à la ou aux zones du visage pour lesquelles il est adapté, dans une table de correspondance qui servira par la suite, pour, partant d'une zone de la peau, en déduire le mélange à utiliser.

On peut procéder de la même façon avec d'autres mélanges pour réaliser une cartographie de l'ensemble du visage et ainsi avoir une table de correspondance complète du visage.

On peut aussi réaliser un mélange donné, l'appliquer sur une zone donnée, puis faire varier le mélange jusqu'à obtenir le mélange le plus adapté. On enregistre alors le mélange en l'attribuant à la ou aux zones du visage pour lesquelles il convient, dans une table de correspondance qui servira par la suite, pour, partant d'une zone de la peau, en déduire le mélange à utiliser.

De préférence, le système informatique évalue et mémorise les quantités utilisées zone par zone. Un tel procédé, par « touches d'essai », permet d'identifier le ou les produits dont a besoin la personne qui veut se maquiller. Ainsi, le système de distribution peut servir sur les points de vente pour conseiller les personnes qui veulent se maquiller ou à la maison pour bien définir les produits à commander.

L'interface du système informatique comporte de préférence un écran tactile affichant la couleur du mélange lors de sa sélection.

L'interface peut afficher un visage et permettre de renseigner le système informatique en sélectionnant la zone sur le visage affiché.

Le système informatique est de préférence agencé pour permettre d'associer une zone, des paramètres de reconstitution du mélange, et la date de l'essai et/ou tout autre identifiant du mélange.

Le système informatique est de préférence également agencé pour permettre d'associer en outre à ladite zone, aux paramètres de reconstitution du mélange, et à la date ou à l'identifiant du mélange, au moins l'une des données suivantes : le nom de la zone, la période de l'année, le nom d'un évènement, un identifiant de l'utilisateur, l'âge de l'utilisateur.

Les étapes a) à c) peuvent être renouvelées au moins une fois avant la mémorisation des caractéristiques du mélange à l'étape d).

Le système informatique peut être agencé pour rechercher dans une base de données une référence de produit commerciale, sur la base des caractéristiques du mélange identifié comme convenant à au moins une zone donnée, et la communiquer à l'utilisateur.

La sélection à l'étape a) peut s'effectuer avec l'aide d'un système expert externe ou non au système informatique.

Le système expert peut analyser une image de l'utilisateur, pour proposer une couleur de mélange au moins sur la base de l'image analysée.

L'étape a) peut être précédée par la proposition d'une couleur et d'une zone à tester avec un mélange de cette couleur, par le système informatique à l'utilisateur.

Le système informatique peut être agencé pour permettre à l'utilisateur de lerenseigner sur l'appréciation du résultat du test de l'étape c) et pour générer uneproposition de modification du mélange à sélectionner lors du retour à l'étape a).

Le système informatique peut être agencé pour proposer au moins une couleur de mélange à l'étape a) en fonction d'une zone d'application renseignée par l'utilisateur.

Le système informatique peut être agencé pour proposer au moins une zone d'application à l'étape a), en fonction d'une couleur renseignée par l'utilisateur.

Le distributeur peut délivrer à l'étape b) au moins deux mélanges de couleurs différentes, de préférence séparés, en vue de leur application simultanée sur la zone de test.

Cela peut faire gagner du temps, et faciliter la comparaison des résultats.

L'invention a encore pour objet un procédé de maquillage à l'aide d'un système de distribution selon cet aspect de l'invention, dans lequel :
a) L'utilisateur adresse une requête concernant un besoin de maquillage au système informatique,
b) celui-ci génère en retour une proposition de couleur en vue du maquillage d'une zone associée, sur la base de l'apprentissage effectué préalablement, et
c) le système informatique pilote le distributeur pour la production du mélange de la couleur proposée, notamment si celle-ci est validée par l'utilisateur.

Un tel procédé peut utiliser une cartographie précédemment établie avec l'utilisateur.

L'invention a encore pour objet un produit programme d'ordinateur comportant des instructions de code permettant, lorsque exécutées dans un système informatique, d'amener le système informatique à :
- Permettre à l'utilisateur de sélectionner au moins une couleur et/ou une zone d'application, notamment à l'aide d'une interface telle qu'un écran tactile,
- piloter un distributeur de façon à délivrer un mélange de la couleur sélectionnée par l'utilisateur,
- permettre à l'utilisateur de déclencher la mémorisation de la couleur du mélange et d'une zone d'application associée, notamment en vue d'une distribution ultérieure du même mélange, notamment sur la même zone.

Le produit programme d'ordinateur peut comporter des instructions de code permettant, lorsque exécutées dans un système informatique, d'amener le systèmeinformatique à:
- Recevoir une requête de l'utilisateur concernant un besoin de maquillage, notamment à l'aide d'une interface telle qu'un écran tactile,
- proposer sur la base au moins de données générées par le procédé d'apprentissage tel que défini ci-dessus, au moins une couleur et/ou une zone d'application,
- piloter un distributeur pour la production du mélange de la couleur proposée, notamment si celle-ci est validée par l'utilisateur.

### Aide à distance

Il est souhaitable de pouvoir aider l'utilisateur à se maquiller, notamment à choisir les bons coloris.

L'invention a ainsi encore pour objet un procédé de maquillage comportant les étapes consistant à :
- permettre l'établissement d'une liaison vidéo, par exemple via internet, entre une caméra d'un premier site et un deuxième site,
- permettre au deuxième site de piloter directement ou indirectement un distributeur présent sur le premier site, ce distributeur permettant de faire varier la couleur d'un mélange distribué,
- permettre à une personne présente au premier site d'appliquer le mélange distribué et d'envoyer vers le deuxième site une image correspondante, pour recevoir en retour une information relative au résultat de maquillage.

Le deuxième site peut notamment comporter un écran de visualisation qui permet à un conseiller présent devant cet écran de voir le résultat du maquillage avec le produit distribué par le distributeur et de conseiller la personne qui s'est maquillée. Ce conseiller peut agir en retour sur le distributeur pour modifier la couleur du mélange et l'adapter au mieux au visage de la personne présente sur le premier site. Ainsi, cette personne gouverne le mélange délivré par le distributeur. La première personne peut se maquiller sous les yeux de la seconde. La seconde personne voit sur son écran le résultat de l'essai, et ainsi peut corriger le mélange qu'elle va commander à distance, jusqu'à obtenir le maquillage idéal.

Le cas échéant, l'acquisition vidéo peut être calibrée à l'aide d'une mire, ou avec le mélange distribué par le distributeur sur une surface de référence. Cela permet ensuite une visualisation plus fidèle du maquillage effectué sur le premier site.

De préférence, la liaison vidéo entre les deux sites est une liaison duplex.

Le premier site peut recevoir un tutoriel du deuxième site, le cas échéant.

Des identifiants des produits de base peuvent être communiqués au deuxième site ; cela peut permettre de connaître précisément la couleur de chacun des produits de base.

Le procédé peut comporter la mémorisation des paramètres de réglage du distributeur, une fois qu'un mélange donné est estimé satisfaisant. De préférence, cette mémorisation peut être commandée depuis le deuxième site. La mémorisation peut avoir lieu dans le système informatique présent sur le premier site et/ou sur un serveur externe.

Une variante peut consister à faire travailler une personne pour aider le maquillage de plusieurs personnes. Ce mode de mise en oeuvre permet le développement des « Make-Up Artists » et leur travail soit au sein d'un institut, soit via internet. Cela permet aussi le maquillage de personnes aux moyens limités, telles que les personnes malvoyantes, ou discernant mal les couleurs, ou âgées, ou manquant de confiance en elles.

### Pilotage par interface tactile

Il existe un besoin pour faciliter le pilotage du système de distribution et notamment le choix de la couleur du mélange distribué.

Selon un autre de ses aspects, indépendamment ou en combinaison avec ses autres aspects, et notamment avec ce qui précède, l'invention a ainsi pour objet un système de distribution comportant un distributeur et un système informatique de pilotage du distributeur, ce système informatique comportant un écran tactile sur lequel peut être affichée la couleur du mélange et un moyen de sélection déplaçable sur l'écran, pour faire varier la couleur du mélange distribué.

De préférence, l'écran affiche des couleurs extrêmes entre lesquelles la couleur du mélange peut être sélectionnée, en déplaçant le moyen de sélection entre ces couleurs extrêmes.

L'écran peut afficher une échelle de couleurs entre au moins deux couleurs, ou une surface, notamment de contour triangulaire, au sein de laquelle le moyen de sélectionpeut se déplacer. Cette surface peut localement faire apparaître la couleur du mélange en fonction par exemple de la distance à chacun des sommets, matérialisant chacun un produit de base pur.

Le système informaitque peut effectuer une partie des calculs nécessaires pour déterminer les fractions de chacun des produits de base conduisant à un mélange de la couleur recherchée.

Le système informatique peut être un téléphone intelligent, un photophone, une tablette, un ordinateur individuel. En variante, il est intégré au boîtier du distributeur.

Le système informatique peut disposer d'une caméra. Cette dernière peut être utilisée notamment pour l'acquisition d'une image de l'utilisateur et/ou du mélange.

Le système informatique peut être agencé pour afficher une image d'un visage, afin de faciliter le repérage des zones sur lesquelles le mélange doit être appliqué.

### Couplage du système de distribution à un moyen de pulvérisation

Le système de distribution peut comporter ou être connecté à un moyen de pulvérisation du mélange, de préférence un aérographe.

L'invention vise, selon un autre de ses aspects, à perfectionner encore les systèmes de distribution comportant un système de pulvérisation, de préférence un aérographe, et a pour objet, selon l'un de ses aspects, indépendamment ou en combinaison avec les autres aspects, et notamment ce qui précède, un ensemble comportant :
- Un moyen de pulvérisation, de préférence un aérographe comportant une chambre de prélèvement soumise à un courant d'air d'entraînement,
- un distributeur comportant au moins deux compartiments contenant des produits de base différents, les produits étant délivrés au moyen de pulvérisation de préférence par des orifices de distribution distincts.

Le distributeur peut comporter trois cartouches contenant des produits de maquillage de couleurs différentes.

L'aérographe peut comporter un stylet définissant la chambre de prélèvement, le stylet se fixant sur le distributeur ou sur une interface de sortie fixée au distributeur, ou faisant partie intégrante de cette interface de sortie.

Le système de distribution peut comporter un circuit de contrôle du fonctionnement du distributeur, permettant de faire varier la proportion des produits de base délivrés dans la chambre de prélèvement, au cours du fonctionnement de l'aérographe. Les proportions peuvent être modifiées en fonction du déplacement de l'aérographe relativement à la surface sur laquelle le mélange est pulvérisé. Ce déplacement peut être mécanisé, le cas échéant.

Ce circuit de contrôle peut comporter ou être constitué par un système informatique tel que défini plus haut.

Le boîtier du distributeur peut servir de poignée lors de la manipulation d l'ensemble pour délivrer le mélange.

Le distributeur peut comporter une caméra et/ou un ou plusieurs capteurs tels que des accéléromètres afin de localiser automatiquement la zone sur laquelle le mélange est appliquée, et pouvoir régler automatiquement la couleur en fonction de la position, le cas échéant.

L'invention a encore pour objet un procédé de maquillage à l'aide d'un ensemble tel que défini ci-dessus, dans lequel un mélange est pulvérisé sur la peau à l'aide du moyen de pulvérisation, notamment de l'aérographe.

La composition du mélange peut être modifiée au cours du déplacement de l'aérographe relativement à la peau. Un dégradé peut être réalisé.

Cet aspect de l'invention repose sur la constatation que le distributeur peut être utilisé pour alimenter le système de pulvérisation, notamment l'aérographe, tout en permettant au système de distribution d'être suffisamment réactif pour autoriser un changement de la couleur du mélange distribué en cours de maquillage du visage, notamment lorsque la zone à maquiller change.

Il peut être avantageux que la distribution des produits se fasse de façon itérative, notamment avec des temps de distribution non déphasés entre les différents produits.

Cela peut rendre plus facile de faire varier la composition du mélange distribué au cours du temps.

Le mélange peut être réalisé directement dans l'aérographe, sans pratiquement aucun volume mort gênant, permettant ainsi de changer le mélange pulvérisé en temps réel.

La dépression créée dans la chambre de prélèvement est suffisante pour entraîner les produits de base, sans pour autant gêner le dosage.

La dépression qui règne dans la chambre de prélèvement est par exemple comprise entre 10 mbar et 200 mbar, mieux entre 50 et 150 mbar, encore mieux entre 75 et 125 mbar.

La viscosité des produits de base mesurée à 1 atm et à 25°C avec un viscosimètre rotatif CONTRAVES TV équipé d'un mobile MS-r3 ou MS-r4 à la fréquence de 60 Hz, après 10 minutes de rotation du mobile, est par exemple comprise entre 0,05 Pa.S et 50 Pa.S

La section des canaux d'arrivée des produits de base dans la chambre est par exemple comprise entre 1 et 3 mm², mieux entre 2 et 3 mm².

L'alimention en produit s'effectue de préférence de façon continue.

Il est encore possible d'appliquer simultanément des produits de base non miscibles ou réactifs, comme un gel aqueux et un gel huileux, qui vont se déposer de façon pixellisée directement sur la peau, produisant une sorte de gel/gel in situ, des silicones réactives, ou des produits de coloration réagissant ensemble. Les ratios en produits de base peuvent être ajustés en fonction du résultat particulier souhaité. Par exemple, dans le cas des gels aqueux et gels huileux, on pourra faire varier le ratio correspondant au volume du premier produit de base sur le volume du deuxième produit de base entre 10/1 et 1/10, mieux entre 5/1 et 1/5.

### Système de localisation ou d'auto-localisation

Le système de distribution selon l'invention peut disposer d'un système de localisation ou d'auto-localisation.

On appelle système de localisation, un moyen de saisir, de la part de la personne, la zone qu'elle va traiter. Ceci peut être réalisé notamment par des systèmes laissant libre au moins une main. Ainsi, on peut utiliser une interface d'un système informatique telle qu'un écran tactile, un joystick, ou un système de reconnaissance vocale.

On appelle système d'auto-localisation, un moyen de saisir, sans que la personne n'intervienne, la zone à traiter. Ceci peut être réalisé par un ou plusieurs accéléromètres qui déduisent, par le fait des mouvements, les directions que la personne vise ou par une caméra et un système de reconnaissance d'image.

Des exemples de systèmes de distribution convenant à l'invention selon son dixième aspect sont représentés sur les figures qui ne seront pas décrites à nouveau.

### Exemple(dixième aspect de l'invention)

On réalise plusieurs produits de base (les proportions sont massiques).

La formule F2 est riche en filmogène et solvant volatil. Les formules F1 et F3 sont riches en huiles non volatiles (phenyl trimethicone et squalane) et de couleurs différentes.

| | | F1 | F2 | F3 |
|---|---|---|---|---|
| | | % massique | % massique | % massique |
| 1 | Dimethicone copolyol vendu sous la référence KF 6017 par la société Shin Etsu | 2 | 2 | 2 |
| | Cetyl PEG/PPG-10/1 dimethicone vendu sous la référence ABIL EM 90 par la société Goldschmidt | 0 | 1 | 0 |
| | Bis PEG/PPG-14/14 dimethicone + Cyclopentasiloxane vendu sous la référence ABIL EM 97 par la société Goldschmidt | 1 | 0 | 1 |
| | Cyclopentasiloxane | 17,65 | 0 | 17,65 |
| | Cyclohexasiloxane | 0 | 8,2 | 0 |
| | Isododecane | 0 | 1 | 0 |
| | Isohexadecane | 0 | 1,6 | 0 |
| | Phenyl trimethicone vendu sous la référence DC556 par la société Dow Corning | 2 | 0 | 2 |
| | Ethyl hexyl methoxycinnamate | 3 | 3 | 3 |
| | Squalane | 1 | 0 | 1 |
| 2 | Copolymère d'acrylate de butyle contenant des chaînes latérales silicones dendritiques: Tri((Trimethylsilo xy) silo xyethyldimet hylsiloxy)silylpropyl- méthacrylate dans l'isododécane (40/60) vendu sous la référence Dow Corning FA 4002 ID par Dow Corning | 0 | 10 | 0 |
| | Cyclopentasiloxane | 7 | 0 | 7 |
| 3 | Cyclohexasiloxane | 0 | 7,5 | 0 |
| | Oxyde de fer faune enrobé de stearoyl glutamate d'aluminium NAI-C33-9001-10 de la société Miyoshi Kasei | 1,25 | 1,25 | 1,65 |
| | Oxyde de fer rouge enrobé de stearoyl glutamate d'aluminium NAI-C33-8001-10 de la société Miyoshi Kasei | 0,5 | 0,5 | 0,3 |
| | Oxyde de fer noir enrobé de stearoyl glutamate d'aluminium NAI-C33-7001-10 de la société Miyoshi Kasei | 0,15 | 0,15 | 0,15 |
| | Dioxyde de titane (anatase) enrobé de stearoyl glutamate d'aluminium NAI-TAO-77891 de la société Miyoshi Kasei | 10,1 | 10,1 | 9,9 |
| 4 | Talc vendu sous la référence Micro Ace P3 par la société Nippon Talc | 0,5 | 0 | 0,5 |
| | Poudre de nylon 12 vendue sous la référence SP 500 par la société Toray Industries | 0,5 | 0 | 0,5 |
| | Poudre de nylon 12 vendue sous la référence Orgasol 2002 EXD NAT COS par la société Arkema | 0 | 3 | 0 |
| | Microsphére de Silice vendue sous la référence SB 700 par la société Miyoshi Kasei | 0 | 1 | 0 |
| | Perlite vendue sous la référence OPTIMAT 2550 OR par la société World Minerals | 0 | 0,2 | 0 |
| | Eau déminéralisée | 36,15 | 34,8 | 36,15 |
| | 1,3-Butylene glycol | 3 | 6 | 3 |
| | Sulfate de magnésium | 0,7 | 0,7 | 0,7 |
| | Solution de maltose hydrogénée | 0,5 | 0 | 0,5 |
| | Alcool éthylique 96° dénaturé | 13 | 8 | 13 |
| | TOTAL | 100 | 100 | 100 |

### Mode opératoire Formule F2

On pèse les constituants de la phase A1 dans le bêcher principal et l'on agite en maintenant à température ambiante sous Moritz (1000 tr/min). Puis on ajoute, à température ambiante, la phase A2 en agitant à l'aide d'un agitateur Moritz (1000 tr/min) jusqu'à homogénéisation. La phase A3 est préparée séparément en broyant trois fois à la tricylindre, le mélange de pigments et de cyclohexasiloxane. Cette phase A3 est ensuite ajoutée en maintenant l'agitation, ainsi que les phases A4 et A5.

La phase aqueuse B est aussi préparée séparément, en pesant dans un bêcher le butylène glycol et le sulfate de magnésium et en ajoutant de l'eau préalablement chauffée à 95°C. La phase aqueuse est placée sous agitation à l'aide d'un barreau magnétique jusqu'à homogénéisation. L'émulsion se fait à température ambiante : on verse la phase aqueuse B dans la phase grasse en augmentant progressivement la vitesse d'agitation (Moritz) jusqu'à 4000 trs/mn. On maintient l'agitation pendant 10 mn. Puis on ajoute enfin la phase C (éthanol). Le produit obtenu est placé sous agitation Rayneri (pâles) et agité pendant 10 mn entre 50 et 60 trs/mn.

### Mode opératoire formules F1 et F3

On pèse les constituants de la phase A1 dans le bêcher principal et l'on agite en maintenant à température ambiante sous Moritz (1000 tr/min). La phase A3 est préparée séparément en broyant trois fois à la tricylindre, le mélange de pigments et de cyclopentasiloxane. Cette phase A3 est ensuite ajoutée en maintenant l'agitation, ainsi que les charges (phase A4).

La phase aqueuse B est aussi préparée séparément, en pesant dans un bêcher la glycérine, le butylène glycol, le sulfate de magnésium, la solution de maltose hydrogénée et l'eau à température ambiante. La phase aqueuse B est placée sous agitation à l'aide d'un barreau magnétique jusqu'à homogénéisation. A4 B C L'émulsion se fait à température ambiante : on verse la phase aqueuse B dans la phase grasse en augmentant progressivement la vitesse d'agitation (Moritz) jusqu'à 4000 tours/mn. On maintient l'agitation pendant 7 mn. Puis on ajoute enfin la phase C (alcool éthylique) pendant les 3 dernières minutes de 5 l'émulsification.

Le système est testé avec :
- F1→Compartiment 1
- F2→Compartiment 2
- F3→Compartiment 3

### Test 1

### On réalise des mélanges (proportions volumiques) :

(On utilise le système pour délivrer des doses de 50 à 200 mg de produit. On étale alors les mélanges petit à petit, doses par doses).
- 50/40/10 pour maquiller les paumettes pour donner un effet rosé pour les pomettes avec une tenue moyenne et un confort modéré.
- 10/80/10 pour maquiller les zones tachées des pomettes, zones nécessitant une forte tenue.
- 20/80/0 pour maquiller les lèvres, avec une forte tenue.
- 30/20/50 pour maquiller les paupières avec un fort confort.

### Test 2

Ce jour, il fait froid mais, étant un jour de la semaine, la personne a peu d'occasions d'être dehors. Ainsi, elle réalise des mélanges :
(On utilise le système pour délivrer des doses de 50 à 200 mg de produit. On étale alors les mélanges petit à petit, doses par doses).
- (proportions volumiques) 50/40/10 pour maquiller les paumettes pour donner un effet rosé pour les pomettes avec une tenue moyenne et un confort modéré.
- 10/80/10 pour maquiller les zones tachées des pomettes, zones nécessitant une forte tenue.

Un autre jour, il fait froid et, étant un jour de week-end, la personne a plusieurs occasions d'être dehors. Ainsi, elle réalise des mélanges :
(On utilise le système pour délivrer des doses de 50 à 200 mg de produit. On étale alors les mélanges petit à petit, doses par doses).
- 50/30/10 pour maquiller les paumettes pour donner un effet rouge pour les pomettes avec une tenue moyenne et un confort modéré.
- 30/60/10 pour maquiller les zones tachées des pomettes, zones nécessitant une forte tenue.

Les résultats visuels sont proches entre les deux jours, mais le confort a été adapté, dans le cas du jour de week end. Certes, le confort a été amélioré au détriment de la tenue, mais grâce à la qualité des réglages, la tenue reste optimale.

L'invention n'est pas limitée à chacun des dix aspects qui viennent d'être décrits. En particulier, on peut combiner au sein de nouvelles varaiantes des caractérisitques issues de l'invention selon ses différents aspects.

### RESUME

i) .(premier aspect) Système de distribution d'un produit, comportant un distributeur recevant au moins deux cartouches ayant chacune un réservoir contenant un produit de base, un premier produit de base comportant au moins 0,1 % en masse de particules présentant un écart de densité d'au moins 0,5 g/cm3 avec le milieu qui les contient, notamment des particules présentant une densité supérieure ou égale à 2g/cm3, et un épaississant, le distributeur permettant de délivrer au moins deux produits de base dans des proportions réglables, la viscosité du premier produit de base étant supérieur supérieure à 2 Pa.s et de préférence supérieure ou égale à 4 Pa.s La viscosité des produits de base mesurée à 1 atm et à 25°C avec un viscosimètre rotatif CONTRAVES TV équipé d'un mobile MS-r3 ou MS-r4 à la fréquence de 60 Hz, après 10 minutes de rotation du mobile.
ii) Système selon i) un deuxième produit de base ayant une viscosité inférieure à celle du premier produit de base, notamment inférieure ou égale à 1 Pa.s et préférentiellement inférieure à 2 Pa.S
iii) Système selon ii), le deuxième produit de base ayant une viscosité inférieure ou égale à 2 Pa.s
iv) Système selon i) à iv) la densité desdites particules étant supérieure ou égale à 5g/cm3
v) Système selon i) à iv) les particules comportant l'un au moins des matériaux choisis dans la liste suivante : oxychlorure de bismuth, oxyde de cérium, oxyde de chrome, oxyde de zirconium, oxyde de fer, oxyde de titane, talc, carbonate de calcium, silice, nitrure de bore, carbure de tungstène
vi) Système selon i) à v) le deuxième produit étant dépourvu de particules de densité supérieure ou égale à 2g/cm3.
vii) Système selon i) à vi) comportant une troisième cartouche avec un troisième produit de base.
viii) Système selon vii), le troisième produit de base comportant un épaississant.
ix) Système selon i) à viii), l'épaississant est choisi parmi les composés saccharidiques de type gomme, tels que la gomme arabique, la gomme acacia, la gomme guar, la gomme gellane, la gomme keraya, la gomme de carraghénane, les composés cellulosiques tels que CMC, HMC, HPMC ,les polymères synthétiques tels que les polyacides acryliques ou méthacrylique tels que les carbomer (Carbopol), ou polyuréthanes, polyvinylacétate, polyvinylalcool, les émulsions épaisses inverses ou directes, des associations de solvants non aqueux avec des agents épaississants pour huile, des argiles telles que bentonite, attapulgite, des organochélateurs, des protéines telles que caséine ou collagène, des agents de rhéologie rhéofluidifiants ou thixotropes.
x) Système selon ix), l'épaississant étant choisi parmi les composés saccharidiques de type gomme et sa teneur massique étant comprise entre 0,1 % et 5 % dans le premier produit de base, mieux de 0,8 à 2,5 %.
xi) Système selon i) à x) les cartouches étant reçues de façon amovible dans le distributeur.
xii) Système selon i) à xi) chaque produit quittant la cartouche par un canal de sortie de la cartouche, le canal de sortie étant défini par un embout de distribution de la cartouche entrainé en rotation relative par rapport à un corps de la cartouche par un mécanisme d'entraînement du distributeur pour distribuer le produit de base contenu dans la cartouche.
xiii) Système selon i) à xiii) comportant un mécanisme d'homogénéisation du premier produit de base, notamment vibratoire.
xiv) (deuxième aspect)Système de distribution d'un produit, éventuellement selon l'un de i) à xiii), comportant un distributeur recevant au moins deux cartouches ayant chacune un réservoir contenant un produit de base, un premier produit de base comportant un épaississant, un deuxième produit de base comportant un agent de modulation de la viscosité de l'épaississant, le distributeur permettant de délivrer au moins ces deux produits de base dans des proportions réglables.
xv) Système selon xiv), et :
   Premier cas :
      Le premier produit comprend un actif épaississant sensible au pH, et une viscosité supérieure à 2 Pa.s et préférentiellement supérieure à 4 Pa.s, et le second produit contient un agent acide modifiant la viscosité de l'actif du premier produit lors du contact entre les deux produits. L'actif épaississant du premier produit est notamment un gélifiant à base de polymère ou copolymère acrylate ou méthacrylate tel qu'un Carbomer, utilisé entre 0,8 et 2,5% et le second produit est un agent acidifiant, tel qu'un acide minérale ou organique tel qu'acide citrique ou lactique, utilisé à 0,2% à 10%. La viscosité du second produit peut être inférieure ou supérieure ou égale à la viscosité du premier produit. De façon préférentielle, la viscosité du second produit est comprise entre 1 et 3Pa.s. Le pH du premier produit est supérieur à 6 et préférentiellement supérieur à 7. Le pH du second produit est inférieur à 6 et préférentiellement inférieur à 5
   Deuxième cas :
      Le premier produit présente une viscosité supérieure à 2 Pa.s et préférentiellement supérieure à 4Pa.s, et le second produit présente une viscosité faible, entre 0,01 et 2. Pa.s, de préférence, entre 0,1 et 0,5 Pa.s. Le premier produit et le second produit contiennent notamment un gélifiant à base de polymère ou copolymère acrylate ou méthacrylate tel qu'un Carbomer ou gélifiant à base polyosique, avec des concentrations en gélifiant (ensemble des gélifiants) de l'ordre de0,8% à 2,5% pour le premier et 0 à 2,5% pour le second.
   Troisième cas :
      Le premier produit contient un actif épaississant sensible au pH, et une viscosité inférieure à 2 Pa.s et préférentiellement inférieure à 1 Pa.s, et le second produit contient un agent alcalin modifiant la viscosité de l'actif du premier produit lors du contact entre les deux produits. L'actif épaississant du premier produit est notamment un épaississant à base de polymère ou copolymère acrylate ou méthacrylate tel qu'un Carbomer et le second produit est un agent alcalinisant, tel qu'une base minérale ou organique tel qu'une amine, de l'ammoniaque. La viscosité du second produit peut être inférieure ou supérieure ou égale à la viscosité du premier produit. De façon préférentielle, la viscosité du second produit est comprise entre 1 et 3Pa.s. La concentration en agent épaississant du premier produit peut varier de 0,8 à 5%. Le pH du premier produit est inférieur à 6 et préférentiellement inférieur à 5. Le pH du second produit est supérieur à 6 et préférentiellement inférieur à 7
xvi) Système selon xiv) à xv), l'épaississant ayant une viscosité dépendant du pH et l'agent de modulation de la viscosité étant un acide ou une base, notamment choisi parmi l'ammoniaque, les amines, la soude , l'acide citrique, l'acide lactique.
xvii) Système selon xiv) à xvi), l'agent de modulation de la viscosité étant un diluant, notamment l'eau, l'éthanol, une huile ou une composition de ces agentsfaiblement épaissie
xviii) ...
xix) ...
xx) Système selon xiv) à xix) le deuxième produit de base ayant une viscosité sensiblement égale à celle du premier produit de base.
xxi) Système selon xiv) à xx), comportant une troisième cartouche avec un troisième produit de base.
xxii) Système selon xxi), le troisième produit de base comportant un épaississant, notamment à une concentration différente de celle du premier produit de base.
xxiii) Système selon xiv) à xxii), l'épaississant est choisi parmi les composés saccharidiques de type gomme, tels que la gomme arabique, la gomme acacia, la gomme guar, la gomme gellane, la gomme keraya, la gomme de carraghénane, les composés cellulosiques tels que CMC, HMC, HPMC ,les polymères synthétiques tels que les polyacides acryliques ou méthacrylique tels que les carbomer (Carbopol), ou polyuréthanes, polyvinylacétate, polyvinylalcool, les émulsions épaisses inverses ou directes, des associations de solvants non aqueux avec des agents épaississants pour huile, des argiles telles que bentonite, attapulgite, des organochélateurs, des protéines telles que caséine ou collagène, des agents de rhéologie rhéofluidifiants ou thixotropes
xxiv) Système selon la revendication xxiii), l'épaississant étant un gel de carbopol, de préférence en milieu neutre ou alcalin, notamment à une teneur comprise entre 0,1 et 2,5 % en masse.
xxv) Système selon la revendication xxiii), l'épaississant étant choisi parmi les composés saccharidiques de type gomme et sa teneur massique étant comprise entre 0,2 % et 5 % dans le premier produit de base, mieux de 0,8 à 2,5 %.
xxvi) Système selon xiv) à xxv) les cartouches étant reçues de façon amovible dans le distributeur.
xxvii) Système selon xiv) à xxvi), chaque produit quittant la cartouche par un canal de sortie de la cartouche, le canal de sortie étant défini par un embout de distribution de la cartouche entrainé en rotation relative par rapport à un corps de la cartouche par un mécanisme d'entraînement du distributeur pour distribuer le produit de base contenu dans la cartouche.
xxviii) Système selon xiv) à xxvii), comportant un mécanisme d'homogénéisation du premier produit de base, notamment vibratoire.
xxix) Procédé d'application d'un produit cosmétique, de maquillage et/ou de soin, sur les matières kératiniques humaines, comportant le réglage du distributeur d'un système de distribution selon l'un des points précédents, en fonction de la zone à traiter, et la distribution du produit par prélèvement dans les cartouches des produits de base dans les proportions correspondant au réglage du distributeur.
xxx) Procédé selon xxix), le réglage s'effectuant de façon à avoir la viscosité la plus grande et l'application s'effectuant sur des taches de peau.
xxxi) Procédé selon xxix), le réglage s'effectuant de façon à avoir une viscosité intermédiaire entre les viscosités extrêmes pouvant être obtenues, et l'application s'effectuant sur des taches de peau ou des cernes.
xxxii) (troisième aspect)Système de distribution d'un produit, éventuellement selon l'un de i) à xxxi), comportant un distributeur recevant au moins deux cartouches contenant respectivement un premier produit de base et un deuxième produit de base, le distributeur permettant de délivrer au moins ces deux produits de base dans des proportions réglables pour former un film dont une propriété au moins varie en fonction du réglage.
xxxiii) Système selon la revendication xxxii), la propriété du film qui varie étant sa souplesse.
xxxiv) Système selon la revendication xxxii), la propriété du film qui varie étant son caractère huileux.
xxxv) Système selon la revendication xxxii), la propriété du film qui varie étant son caractère tenseur.
xxxvi) Système selon l'un de xxxii) à xxxv), les deux produits de base étant choisis pour réagir ensemble afin de former un film.
xxxvii) Système selon xxxvi) , le premier produit de base comportant une silicone réactive et le second produit de base un catalyseur provoquant la réticulation de la silicone réactive.
xxxviii) Système selon xxxvii), la silicone réactive comportant comportant un mélange de polyorganosiloxane téléchélique portant une fonction vinyl aux deux extrémités de chaîne et de polyhydrogénosiloxane.
xxxix) Système selon xxxvii), le catalyseur étant à base de platine.
xl) .Système selon xxxii) à xxxix), le premier produit de base comportant un polymère filmogène et le deuxième produit de base un composé huileux liquide à température ambiante.
xli) Système selon xl), le polymère filmogène présentant une température de transition vitreuse Tg supérieure ou égale à 30°C, mieux supérieure ou égale à 60°C.
xlii) Système selon xl) ou xli), le polymère filmogène étant choisi parmi les polymères ou copolymères vinyliques, notamment acryliques.
xliii) Système selon xl), le polymère filmogène étant liposoluble.
xliv) Système selon xxxii) à xxxv), les premier et deuxième produits de base contenant des polymères filmogènes respectifs différents.
xlv) Système selon xliv), le polymère filmogène du premier produit de base ayant une température de transition vitreuse Tg1 et le polymère du deuxième produit de base ayant une température de transition vitreuse Tg2 différente de Tg1, avec de préférence Tg1>60°C et Tg2<10°C.
xlvi) Système selon xlv), le premier produit de base comportant un polymère ou copolymère choisi parmi les polymères et copolymères méthacryliques, les polyamides, les alkycelluloses, les polymères et copolymères de la vinylpyrolidone, et les résines silicones.
xlvii) Système selon xlv), le deuxième produit de base comportant un polymère ou copolymère choisi parmi les polymères et copolymères acryliques, vinyliques et les polycondensats tels que les polyesters et les polyuréthanes.
xlviii) Système selon xxxii) à xxxv), le premier produit de base comportant un composé huileux de température de fusion Tf1>20°C et le deuxième produit de base comportant un composé huileux de température de fusion Tf2<20°C.
xlix) Procédé d'application d'un produit cosmétique, de maquillage et/ou de soin, sur les matières kératiniques humaines, à l'aide d'un système de distribution selon xxxii) à xlviii) comportant le réglage du distributeur en fonction de la zone à traiter, et la distribution du produit par prélèvement dans les cartouches des produits de base dans les proportions correspondant au réglage du distributeur.
   l) (quatrième aspect)Système de distribution d'un produit cosmétique, éventuellement selon l'un quelconque des points précédents, comportant un distributeur recevant au moins deux cartouches ayant chacune un réservoir contenant un produit de base, un premier produit de base comportant au moins un solvant organique, la cartouche le contenant comportant un corps réalisé dans l'une au moins des matières thermoplastiques choisies dans la liste comprenant les polyoléfines simples, le polyvinylchloride (PVC), les polyamides et polyamides semi-aromatiques, le polysulfure de phénylene (PPS), le polybismaléimide, les polyuréthannes, les polyesters, les polyépoxydes, le polyéther bloc amide, le polyacétal, le polyéthercétone, les polyetherimides (PEI), les polyimides, le polyamideimide (PAI), les FEP (ethylène propylène perfluoré) , PFA (Polyfluoroalkoxy), ECTFE (éthylène chloro trifluoro éthylène) , et ETFE (éthylène tétrafluoroéthylène), et leurs mélanges, et de préférence les polyoléfines simples, le polyvinylchloride (PVC), les polyamides et polyamides semi-aromatiques, le polysulfure de phénylene (PPS), le polybismaléimide, et leurs mélanges.
   li) Système selon l), la cartouche comportant le premier produit de base comportant outre le corps de la cartouche d'autres composants, notamment mobiles, exposés au premier produit de base, l'un au moins de ces composants, notamment mobile, et de préférence tous ces composants étant choisis dans ladite liste.
   Iii) Système selon l), la teneur totale en solvant(s) organique(s) du premier produit de base étant supérieure ou égale à la teneur totale en eau du premier produit de base.
   liii) Système selon l) à lii), la teneur totale en solvant(s) organique(s) du premier produit de base étant supérieure ou égale à 50% par rapport à l'ensemble des solvants.
   liv) Système selon l) à liii), les cartouches étant identiques, abstraction faite de leur contenu.
   Iv) Système selon l) à liv), l'une au moins des compositions comportant un parfum.
   Ivi) Système selon l) à lv), les cartouches étant reçues de façon amovible dans le distributeur.
   Ivii) Système selon l) à lvi), chaque produit quittant la cartouche par un canal de sortie de la cartouche, le canal de sortie étant défini par un embout de distribution de la cartouche entrainé en rotation relative par rapport à un corps de la cartouche par un mécanisme d'entraînement du distributeur pour distribuer le produit de base contenu dans la cartouche.
   Iviii) (cinquième aspect)Système de distribution d'un produit, éventuellement selon l'un quelconque des points i) à lvii), comportant un distributeur recevant au moins deux cartouches ayant chacune un réservoir et contenant respectivement des premier et deuxième produits de base, le premier produit de base comportant un pigment, le deuxième produit de base comportant une charge distincte du pigment du premier produit de base, le distributeur permettant de délivrer au moins ces deux produits de base dans des proportions réglables.
   lix) Système selon lviii), la taille D50 en volume de particule du pigment et de la charge étant comprise entre 100 nm et 1 mm.
   Ix) Système selon lviii), la taille D50 en volume du pigment allant de 100 nm à 25 microns, mieux de 200 nm à 10 microns.
   Ixi) Système selon lviii) à lx), le pigment étant choisi parmi les pigments minéraux, et de préférence les pigments minéraux modifiés hydrophobes, notamment ceux d'oxyde de fer ou d'oxyde de titane.
   Ixii) Système selon lviii) à lxi), le pigment comportant un enrobage comportant au moins un composé lipophile ou hydrophobe.
   Ixiii) Système selon lviii) à lxi), la charge étant choisie parmi le talc, le mica, la silice, le kaolin, les poudres de poly-β-alanine et de polyéthylène, les poudres de polymères de tétrafluoroéthylène, la lauroyl-lysine, l'amidon, le nitrure de bore, les microsphères creuses polymériques telles que celles de chlorure de polyvinylidène/acrylonitrile, de copolymères d'acide acrylique, les microbilles de résine de silicone, les particules de polyorganosiloxanes élastomères, le carbonate de calcium précipité, le carbonate et l'hydro-carbonate de magnésium, l'hydroxyapatite, le sulfate de barium, les oxydes d'aluminium, les poudres de polyuréthane, les charges composites, les microsphères de silice creuses, et les microcapsules de verre ou de céramique, les particules qui ont la forme de portions de sphères creuses, et leurs mélanges.
   Ixiv) Système selon lviii) à lxiii), le premier produit de base et le deuxième produit de base comportant une émulsion inverse.
   Ixv) Système selon lviii) à lxiii), la teneur massique en pigment dans le premier produit de base étant supérieure ou égale à 5% et préférentiellement supérieur à 10%
   Ixvi) Système selon lviii) à lxv), la teneur massique en charge dans le deuxième produit de base étant supérieure ou égale à 0,5% et de préférence à 1%.
   Ixvii) Système selon lviii) à lxvi), comportant une troisième cartouche avec un troisième produit de base.
   Ixviii) Système selon lviii) à lxvii), les cartouches étant reçues de façon amovible dans le distributeur.
   Ixix) Système selon lviii) à lxviii), chaque produit quittant la cartouche par un canal de sortie de la cartouche, le canal de sortie étant défini par un embout de distribution de la cartouche entrainé en rotation relative par rapport à un corps de la cartouche par un mécanisme d'entraînement du distributeur pour distribuer le produit de base contenu dans la cartouche.
   Ixx) Procédé d'application d'un produit cosmétique, de maquillage et/ou de soin, sur les matières kératiniques humaines, à l'aide d'un système de distribution selon lviii) à lxix) comportant le réglage du distributeur en fonction de la zone à traiter, et la distribution du produit par prélèvement dans les cartouches des produits de base dans les proportions correspondant au réglage du distributeur.
   Ixxi) (sixième aspect)Système de distribution d'un produit, éventuellement selon l'un quelconque des points i) à lxx),comportant un distributeur recevant au moins deux cartouches contenant respectivement des premier et deuxième produits de base, le premier produit de base comportant, notamment sous forme d'émulsion inverse, un filtre solaire organique et une huile, le deuxième produit de base comportant une huile, le distributeur permettant de délivrer au moins ces deux produits de base dans des proportions réglables, la concentration en filtre solaire dans le premier produit de base étant supérieure à celle du deuxième produit de base et la concentration en huile dans le deuxième produit de base étant supérieure à celle du premier produit de base.
   Ixxii) Système selon lxxi), la teneur massique du filtre solaire organique dans le premier produit de base étant supérieure ou égale à 2%, préférentiellement 4%
   Ixxiii) Système selon lxxi), la teneur massique en huile(s) dans le deuxième produit de base étant supérieure à 2%, préférentiellement 4%
   Ixxiv) Système selon lxxi), le deuxième produit de base étant dépourvu de filtre solaire organique.
   Ixxv) Système selon lxxi), le deuxième produit de base comportant un filtre solaire organique.
   Ixxvi) Système selon lxxi), l'un au moins des premier et deuxième produits de base contenant un agent de coloration.
   Ixxvii) Système selon lxxvi), l'agent de coloration étant choisi parmi les pigments, notamment les oxydes de fer.
   Ixxviii) Système selon lxxvi) ou lxxvii), chacun des premier et deuxième produits de base comportant un agent de coloration.
   Ixxix) Système selon lxxi) à lxxviii), l'un au moins des premier et deuxième produits de base contenant une charge incolore.
   Ixxx) Système selon lxxix), chacun des premier et deuxième produits de base comportant une charge incolore.
   Ixxxi) Système selon lxxi) à lxxx), comportant une troisième cartouche avec un troisième produit de base.
   Ixxxii) Système selon lxxxi) à lxxx), les cartouches étant reçues de façon amovible dans le distributeur.
   Ixxxiii) Système selon lxxxi) à lxxxii), chaque produit quittant la cartouche par un canal de sortie de la cartouche, le canal de sortie étant défini par un embout de distribution de la cartouche entrainé en rotation relative par rapport à un corps de la cartouche par un mécanisme d'entraînement du distributeur pour distribuer le produit de base contenu dans la cartouche.
   Ixxxiv) Procédé de réglage d'un système selon lxxxi) à lxxxiii), comportant le réglage du distributeur en fonction de la zone à traiter avec le produit et/ou l'intensité du rayonnement UV.
   Ixxxv) (septième aspect)Système de distribution d'un produit, éventuellement selon l'un quelconque des points précédents, comportant un distributeur recevant au moins deux cartouches ayant chacune un réservoir et contenant respectivement des premier et deuxième produits de base, le premier produit de base comportant l'un au moins d'une huile, d'un pigment interférentiel ou à reflet métallique, le deuxième produit de base comportant une charge matifiante, le distributeur permettant de délivrer au moins ces deux produits de base dans des proportions réglables.
   Ixxxvi) Système selon lxxxv), le premier produit de base contenant une huile.
   Ixxxvii) Système selon lxxxv) ou lxxxvi), le premier produit de base contenant un pigment interférentiel.
   Ixxxviii) Système selon lxxxv) à lxxxvii), le premier produit de base contenant un piment à reflet métallique.
   Ixxxix) Système selon lxxxv) à lxxxviii), la taille D50 en volume de particule du pigment et de la charge étant comprise entre 100 nm et 1 mm.
   xc) Système selon lxxxv) à lxxxix), la charge étant choisie parmi le talc, le mica, la silice, le kaolin, les poudres de poly-β-alanine et de polyéthylène, les poudres de polymères de tétrafluoroéthylène, la lauroyl-lysine, l'amidon, le nitrure de bore, les microsphères creuses polymériques telles que celles de chlorure de polyvinylidène/acrylonitrile, de copolymères d'acide acrylique, les microbilles de résine de silicone, les particules de polyorganosiloxanes élastomères, le carbonate de calcium précipité, le carbonate et l'hydro-carbonate de magnésium, l'hydroxyapatite, le sulfate de barium, les oxydes d'aluminium, les poudres de polyuréthane, les charges composites, les microsphères de silice creuses, et les microcapsules de verre ou de céramique, les particules qui ont la forme de portions de sphères creuses, et leurs mélanges.
   xci) Système selon lxxxv) à lxxxix), le premier produit de base et le deuxième produit de base comportant une émulsion inverse.
   xcii) Système selon lxxxv) à xci), la teneur massique en charge dans le deuxième produit de base étant supérieure ou égale à à 0,5% et de préférence à 1%.
   xciii) Système selon lxxxv) à xcii), comportant une troisième cartouche avec un troisième produit de base.
   xciv) Système selon lxxxv) à xciii), les cartouches étant reçues de façon amovible dans le distributeur.
   xcv) Système selon lxxxv) à xciv), chaque produit quittant la cartouche par un canal de sortie de la cartouche, le canal de sortie étant défini par un embout de distribution de la cartouche entrainé en rotation relative par rapport à un corps de la cartouche par un mécanisme d'entraînement du distributeur pour distribuer le produit de base contenu dans la cartouche.
   xcvi) Procédé d'application d'un produit cosmétique, de maquillage et/ou de soin, sur les matières kératiniques humaines, à l'aide d'un système de distribution selon lxxxv) à xcv), comportant le réglage du distributeur en fonction de la zone à traiter, et la distribution du produit par prélèvement dans les cartouches des produits de base dans les proportions correspondant au réglage du distributeur.
   xcvii) (huitième aspect)Système de distribution d'un produit, éventuellement selon l'un quelconque des points précédents, comportant un distributeur recevant au moins deux cartouches ayant chacune un réservoir et contenant respectivement des premier et deuxième produits de base, le premier produit de base comportant un agent hydratant, le deuxième produit de base comportant une charge, le distributeur permettant de délivrer au moins ces deux produits de base dans des proportions réglables.
   xcviii) Système selon xcvii), l'agent hydratant étant choisi parmi les polyols, l'urée et ses dérivés tels que notamment les hydroxyalkyle urée en particulier l'hydroxyéthylurée, l'acide hyaluronique, la glycine, la β-alanine, la taurine, la triméthyle glycine, et leurs mélanges
   xcix) Système selon xcvii) ou xcviii), la taille D50 en volume de particule de la charge étant comprise entre 100 nm et 1 mm.
   c) Système selon xcvii) à xcix), la charge étant choisie parmi le talc, le mica, la silice, le kaolin, les poudres de poly-β-alanine et de polyéthylène, les poudres de polymères de tétrafluoroéthylène, la lauroyl-lysine, l'amidon, le nitrure de bore, les microsphères creuses polymériques telles que celles de chlorure de polyvinylidène/acrylonitrile, de copolymères d'acide acrylique, les microbilles de résine de silicone, les particules de polyorganosiloxanes élastomères, le carbonate de calcium précipité, le carbonate et l'hydro-carbonate de magnésium, l'hydroxyapatite, le sulfate de barium, les oxydes d'aluminium, les poudres de polyuréthane, les charges composites, les microsphères de silice creuses, et les microcapsules de verre ou de céramique, les particules qui ont la forme de portions de sphères creuses, et leurs mélanges.
   ci) Système selon xcvii) à c), le premier produit de base et le deuxième produit de base comportant une émulsion inverse.
   cii) Système selon xcvii) à ci), la teneur massique en charge dans le deuxième produit de base étant supérieure ou à 0,5% et de préférence à 1%.
   ciii) Système selon xcvii) à cii), comportant une troisième cartouche avec un troisième produit de base.
   civ) Système selon xcvii) à ciii), les cartouches étant reçues de façon amovible dans le distributeur.
   cv) Système selon xcvii) à civ), chaque produit quittant la cartouche par un canal de sortie de la cartouche, le canal de sortie étant défini par un embout de distribution de la cartouche entrainé en rotation relative par rapport à un corps de la cartouche par un mécanisme d'entraînement du distributeur pour distribuer le produit de base contenu dans la cartouche.
   cvi) Procédé d'application d'un produit cosmétique, de maquillage et/ou de soin, sur les matières kératiniques humaines, à l'aide d'un système de distribution selon xcvii) à cv)comportant le réglage du distributeur en fonction de la zone à traiter, et la distribution du produit par prélèvement dans les cartouches des produits de base dans les proportions correspondant au réglage du distributeur.
   cvii) (neuvième aspect)Système de distribution d'un produit parfumé, éventuellement selon l'un quelconque des points précédents, comportant un distributeur recevant au moins deux cartouches contenant respectivement des premier et deuxième produits de base, le premier produit de base comportant une composition contenant au moins un alcool et au moins un dérivé cellulosique, le distributeur permettant de délivrer au moins ces deux produits de base dans des proportions réglables.
   cviii) Système selon cvii), chaque produit de base comportant une composition contenant au moins un alcool et au moins un dérivé cellulosique.
   cix) Système selon cvii) ou cviii), le premier produit de base ou chaque produit de base ayant une concentration massique en alcool par rapport au(x) autre(s) composé(s) non alcoolique(s) d'au moins 80/20 et mieux 90/10.
   ex) Système selon cvii) à cix), le premier produit de base ou chaque produit de base contenant au moins 50% en masse d'éthanol, mieux au moins 80% d'éthanol dans sa phase solvant.
   cxi) Système selon cvii) à cx), le premier produit de base ou chaque produit de base contenant au moins un composé odorant, de préférence choisi parmi les esters, carbonates, acides, anhydrides, aldéhydes, alcools, les composés aliphatiques sans fonctions autres qu'alcanes et alcènes, et leurs mélanges.
   cxii) Système selon la revendication cxi), le ou les composés odorants étant choisis parmi l'hexanal, l'octanal, le nonanal, le decanal, l'undecanal, le dodecanal, le tridecanal, le 2-méthyl décanal, 2-méthyl undecanal, le trans-2-hexenal, le cis-4-heptanal, le 2,6 diméthyl 5-hepten 1 al, le E-4-décenal, le 10 undecenal, le 2 dodecenal, le 1,1 diméthoxy 2,2,5 triméthyl 4 hexene, l'acide 2 méthyl 2 pentenoique, l'acide (S)-(+)-2-méthyl butanoïque, l'ethyl formate, cis-3- hexenyl formate, l'ethylacetate, le butylacetate, l'isoamyleacétate, l'hexylacétate, le 3,5,5 triméthyl hexylacétate, le trans 2 hexenyl acetate, le cis -3- hexenylacetate, l'ethylpropionate, l'ethylbutyrate, le butylbutyrate, l'isoamylbutyrate, l'hexylbutyrate, le cis 3 hexenylisobutyrate, l'ethylisovalérate, l'éthyl 2 méthylbutyrate, l'ethyl hexanoate, l'ethyl 2 méthylpentanoate, le 2 propenyl hexanoate, l'ethylheptanoate, le 2 propenyl heptanoate, l'ethyloctanoate, le methyl 2 nonenoate, l'ethyl 2 trans 4 cis decadienoate, le methyl 2 octynoate, le méthyl 2 nonynoate, l'ethyl3 octobutanoate, l'allyl amylglycolate, le Z 3 hexenylmethyl carbonate, le3-octanol ;le 2,6 diméthyl 2 heptanol, le trans 2 hexen-1 ol ; le 3 hexen-1-ol ; le 1-octen-3-ol ; le 9-decen-1-ol ; le 10 undecen-1-ol ; le 2-trans-6-cis Nonadien-1-ol ; le 4-methyl-3-decen-5-ol, le myrcène, l'ocimène, le béta Farnesene, le citral, le citral diethylacetate, le citronellal, le methoxydihydrocitronellal, le 2,6, 10 triméthyl 9 undecanal, l'acide cis-geranique, l'acide citronellique, le geranyl ester (formate, acétate, propionate, isobutyrate, isovalerate), le neryl acetate, le linalyl esters (formate, acétate, propionate, isobutyrate), les Citronellyl esters (formate, acétate, propionate, isobutyrate, isovalerate, tiglate) et les esters du myrcenol, le Géraniol, nérol, linalool, myrcenol, lavendulol, citronelol, trans trans farnesol, trans-nerolidol, tetrahydrogeraniol, tetrahydrolinalool, l'avendulol, le trans trans farnesol, le trans-nerolidol, le tetrahydrogeraniol, le tetrahydrolinalool, le limonène, le terpinene, le terpinolene, le phellandrene, le camphene, le 3-carène, le menthyl ester (acétate, lactate), les alpha-terpinyl esters (acétate), les Noryl esters (acétate), les Bornyl esters (acétate), les isobornyl esters (acétate), les cedryl ester (acétate), le 2,4-diméthyl-3-cyclohexene carboxaldehyde, le 4-(4-hydroxy-4-methylpentyl)-3 cyclohexene carboxaldehyde, le 1-(4 isopropycyclohexyl) ethanol, 2-méthyl-4-(2,2,3 trimethyl-3-cyclopentene-1-yl) butanol, 2-méthyl-4-(2,2,3 trimethyl-3-cyclopentene-1-yl) butenol, 3-méthyl-5-(2,2,3 trimethyl-3-cyclopentene-1-yl) 4-penten-2-ol, 3,3-diméthyl-5-(2,2,3 trimethyl-3-cyclopentene-1-yl) 4-penten-2-ol, l'Indole, le p-cymène, le diphénylméthane, le benzaldehyde, le phenylacetaldehyde, le phénylacetaldehyde dimethyl acetal, Dihydrocinnamaldehyde, le 2-phenylpropanal, le cyclamenaldehyde, le 2-methyl-3-(4-tert-butyl-phenyl) propanal, le cinnamaldehyde, l'heliotropine, le furfuraldehyde, les benzyl esters (acetate, propionate, isovalerate), les phenethyl ester (acetate, isobutyrate, isovalerate), l'alpha-trichloromethylbenzyl ester (acetate), le cinnamylacetate, le benzoate ester (acetate, hexyl, benzyl), le phenylacetate ethyl, le phenylacetate geranyl,le methylcinnamate, le benzylcinnamate, le phenylethylcinnamate, l'eugenol acetate, l'acide phenylacetique, l'alcool benzylique, 2 phenylethyl alcool, styrallyl alcool, , 2,2 dimethyl -3-(3-methylphenyl) propanol, alcool cinnamique, 3 methyl-5-phenylpentanol, thymol, anethole, isoeugenol, eugenol, anise alcool , raspeberry ketone, ethylmaltol, 2,6-dimethoxyphenol , 2-propyl phénol , 2-(methyl thio) phénol , ortho-guaiacol , 4-methyl guai anhydride abietic, l'anhydride citraconic.
   cxiii) Système selon cvii) à cxii), le dérivé cellulosique étant choisi parmi les dérivés de cellulose obtenus par réaction de cellulose alcalinisée avec de l'oxyde de propylène ou d'éthylène.
   cxiv) Système selon cvii) à cxiii), le dérivé cellulosique étant de l'hydroxypropylcellulose.
   cxv) Système selon cvii) à cxiv), la taille du dérivé cellulosique étant supérieure ou égale à 10000, mieux étant comprises entre environ 850000 et environ 1150000.
   cxvi) Système selon cvii) à cxv), la teneur massique totale en composé(s) cellulosique(s) variant entre 0,1 % et 20 %, mieux entre 0,5 % et 5%, le % étant exprimé par rapport à la masse du produit de base
   cxvii) Système selon cvii) à cxvi), comportant une troisième cartouche avec un troisième produit de base.
   cxviii) Système selon cvii) à cxvii), les cartouches étant reçues de façon amovible dans le distributeur.
   cxix) Système selon cvii) à cxviii), chaque produit quittant la cartouche par un canal de sortie de la cartouche, le canal de sortie étant défini par un embout de distribution de la cartouche entrainé en rotation relative par rapport à un corps de la cartouche par un mécanisme d'entraînement du distributeur pour distribuer le produit de base contenu dans la cartouche.
   cxx) Système selon cvii) à cxix), comportant une mémoire électronique pour enregistrer en association avec un produit distribué les proportions respectives en chacun des produits de base de ce produit, en vue de pouvoir automatiquement redistribuer ce produit ultérieurement.
   cxxi) Procédé pour générer un produit parfumé, à l'aide d'un système selon cvii) à cxx), dans lequel on sélectionne un ou plusieurs produits de base contenus dans des cartouches respectives du distributeur, et l'on distribue les produits de base sélectionnés dans des quantités choisies.
   cxxii) Procédé selon cxxi), dans lequel on mémorise en outre les proportions des différents produits de base composant le produit distribué.
   cxxiii) (dixième zaspect)Système de distribution d'un produit, éventuellement selon l'un quelconque des points précédents, comportant un distributeur recevant au moins deux cartouches contenant respectivement un premier produit de base et un deuxième produit de base, le distributeur permettant de délivrer au moins ces deux produits de base dans des proportions réglables, le premier produit de base comportant un solvant volatil et un polymère filmogène, le deuxième produit de base comportant une huile non volatile.
   cxxiv) Système selon cxxiii), le premier produit de base étant sous forme de composition anhydre liquide.
   cxxv) Système selon cxxiii) ou cxxiv) , le deuxième produit de base étant sous forme de composition liquide anhydre.
   cxxvi) Système selon cxxiii) à cxxv), le premier produit de base comportant une émulsion inverse.
   cxxvii) Système selon cxxiii) à cxxvi), le deuxième produit de base comportant une émulsion inverse.
   cxxviii) Système selon cxxiii) à cxxvii), le polymère filmogène étant hydrophobe.
   cxxix) Système selon cxxiii), la propriété du film qui varie étant sa souplesse.
   cxxx) Système selon cxxiii), la propriété du film qui varie étant son caractère huileux.
   cxxxi) Système selon cxxiii), la propriété du film qui varie étant son caractère tenseur.
   cxxxii) Système seloncxxiii) à cxxxi), les deux produits de base étant choisis pour réagir ensemble afin de former un film.
   cxxxiii) Système selon cxxxii), le premier produit de base comportant une silicone réactive et le second produit de base un catalyseur provoquant la réticulation de la silicone réactive.
   cxxxiv) Système selon cxxxiii), la silicone réactive comportant comportant un mélange de polyorganosiloxane téléchélique portant une fonction vinyl aux deux extrémités de chaîne et de polyhydrogénosiloxane.
   cxxxv) Système selon cxxxiii), le catalyseur étant à base de platine.
   cxxxvi) Système selon cxxxiii), le premier produit de base comportant un polymère filmogène et le deuxième produit de base un composé huileux liquide à température ambiante.
   cxxxvii) Système selon cxxxvi) le polymère filmogène présentant une température de transition vitreuse Tg supérieure ou égale à 30°C, mieux supérieure ou égale à 60°C.
   cxxxviii) Système seloncxxxvi) ou cxvii), le polymère filmogène étant choisi parmi les polymères ou copolymères vinyliques, notamment acryliques.
   cxxxix) Système seloncxxxvi) ou cxvii le polymère filmogène étant liposoluble.
   cxl) Système selon cxxiii) à cxxviii), les premier et deuxième produits de base contenant des polymères filmogènes respectifs différents.
   cxli) Système selon la revendication cxl), le polymère filmogène du premier produit de base ayant une température de transition vitreuse Tg1 et le polymère du deuxième produit de base ayant une température de transition vitreuse Tg2 différente de Tg1, avec de préférence Tg1>60°C et Tg2<10°C.
   cxlii) Système selon la revendication cxl), le premier produit de base comportant un polymère ou copolymère choisi parmi les polymères et copolymères méthacryliques, les polyamides, les alkycelluloses, les polymères et copolymères de la vinylpyrolidone, et les résines silicones.
   cxliii) Système selon la revendication cxl), le deuxième produit de base comportant un polymère ou copolymère choisi parmi les polymères et copolymères acryliques, vinyliques et les polycondensats tels que les polyesters et les polyuréthanes.
   cxliv) Système selon cxxiii) à cxxviii), le premier produit de base comportant un composé huileux de température de fusion Tf1>20°C et le deuxième produit de base comportant un composé huileux de température de fusion Tf2<20°C.
   cxlv) Procédé d'application d'un produit cosmétique, de maquillage et/ou de soin, sur les matières kératiniques humaines, à l'aide d'un système de distribution selon cxxiii) à cxliv), comportant le réglage du distributeur en fonction de la zone à traiter, et la distribution du produit par prélèvement dans les cartouches des produits de base dans les proportions correspondant au réglage du distributeur.

Dans l'énumération ci-dessus, selon ... à ... doit se comprendre comme selon l'un des points ... à ... ; par exemple selon i) à iii) signifie selon i), ii) ou iii)

## Revendications

1. Système (10) de distribution d'un produit, comportant un distributeur recevant au moins deux cartouches (30) ayant chacune un réservoir contenant un produit de base, un premier produit de base comportant au moins 0,1 % en masse de particules présentant un écart de densité, de préférence d'au moins 0,5 g/cm³, avec le milieu qui les contient, les particules présentant une densité supérieure ou égale à 2g/cm³, et un épaississant, le distributeur permettant de délivrer au moins deux produits de base dans des proportions réglables, la viscosité du premier produit de base étant supérieure à 2 Pa.s.

2. Système selon la revendication 1, la viscosité du premier produit de base étant supérieure ou égale à 4 Pa.s, plus préférentiellement comprise entre 4 Pa.s et 10 Pa.s, encore plus préférentiellement comprise entre 5 Pa.s et 8 Pa.s

3. Système selon l'une des revendications 1 et 2, le distributeur recevant une deuxième cartouche contenant un deuxième produit de base ayant une viscosité inférieure à celle du premier produit de base..

4. Système selon la revendication précédente, le deuxième produit de base ayant une viscosité inférieure ou égale à 1 Pa.s, plus préférentiellement inférieure ou égale à 0,8 Pa.s, encore plus préférentiellement comprise entre 0,6 Pa.s et 0,2 Pa.s.

5. Système selon l'une quelconque des revendications précédentes, la densité desdites particules étant supérieure ou égale à 5g/cm³, mieux supérieure ou égale à 6 g/cm³, encore mieux comprise entre 6 g/cm³ et 10 g/cm³, préférentiellement entre 7 g/cm³ et 8 g/cm³.

6. Système selon l'une quelconque des revendications précédentes, les particules comportant l'un au moins des matériaux choisis dans la liste suivante : oxychlorure de bismuth, oxyde de cérium, oxyde de chrome, oxyde de zirconium, oxyde de fer, oxyde de titane, talc, carbonate de calcium, silice, nitrure de bore, carbure de tungstène, étant de préférence choisi parmi l'oxychlorure de bismuth et l'oxyde de cérium.

7. Système selon l'une quelconque des revendications précédentes, le deuxième produit étant dépourvu de particules de densité supérieure ou égale à 7 g/cm³, mieux de densité supérieure ou égale à 6 g/cm³, encore mieux de densité supérieure ou égale à 5g/cm³, préférentiellement de densité supérieure ou égale à 2g/cm³.

8. Système selon l'une quelconque des revendications précédentes, comportant une troisième cartouche avec un troisième produit de base.

9. Système selon la revendication précédente, le troisième produit de base comportant un épaississant identique ou différent de celui du premier produit de base, préférentiellement identique de celui du premier produit de base.

10. Système selon l'une quelconque des revendications précédentes, l'épaississant du premier produit de base étant choisi parmi les composés saccharidiques de type gomme, tels que la gomme arabique, la gomme acacia, la gomme guar, la gomme gellane, la gomme keraya, la gomme de carraghénane, les composés cellulosiques tels que CMC, HMC, HPMC les polymères synthétiques tels que les polyacides acryliques ou méthacrylique tels que les carbomer (Carbopol), ou polyuréthanes, polyvinylacétate, polyvinylalcool, les émulsions épaisses inverses ou directes, des associations de solvants non aqueux avec des agents épaississants pour huile, des argiles telles que bentonite, attapulgite, des organochélateurs, des protéines telles que caséine ou collagène, des agents de rhéologie rhéofluidifiants ou thixotropes, étant de préférence choisi parmi les composés saccharidiques de type gomme, tels que la gomme arabique, la gomme acacia, la gomme guar, la gomme gellane, la gomme keraya, la gomme de carraghénane.

11. Système selon la revendication 10, l'épaississant du premier produit de base étant choisi parmi les composés saccharidiques de type gomme et sa teneur massique étant comprise entre 0,1 % et 5 par rapport à la masse totale du premier produit de base, mieux entre 0,8 à 2,5 % par rapport à la masse totale du premier produit de base, encore mieux entre 1,5 % et 2,3 % par rapport à la masse totale du premier produit de base.

12. Système selon l'une quelconque des revendications précédentes, les cartouches étant reçues de façon amovible dans le distributeur.

13. Système selon l'une quelconque des revendications, chaque produit quittant la cartouche par un canal de sortie de la cartouche, le canal de sortie étant défini par un embout de distribution (56) de la cartouche entrainé en rotation relative par rapport à un corps de la cartouche par un mécanisme d'entraînement du distributeur pour distribuer le produit de base contenu dans la cartouche.

14. Système selon l'une quelconque des revendications précédentes, comportant un mécanisme d'homogénéisation du premier produit de base, notamment vibratoire.
